# EUROPEAN PATENT SPECIFICATION

(11) **EP 2 458 006 B1**
(45) Date of publication and mention of the grant of the patent: **20.06.2018**
(21) Application number: 11189591.8
(22) Date of filing: 07.05.2007
(51) Int. Cl.: C12N 15/113, A61K 31/7088, A61K 31/712

(54) **Compounds and methods for modulating expression APOB**
Verbindungen und Verfahren zur Modulation der Expression von ApoB
Composés et procédés pour moduler l'expression d'APOB

(30) Priority: 05.05.2006 US 746631 P; 11.05.2006 US 747059 P; 23.06.2006 US 805660 P; 06.11.2006 US 864554 P; 27.01.2007 WO PCT/US2007/061183
(43) Date of publication of application: 30.05.2012
(62) Divisional of application: 07811873.4
(73) Proprietor: Ionis Pharmaceuticals, Inc., Carlsbad, CA 92010 (US)
(72) Inventor: Bhanot, Sanjay, Carlsbad, CA 92009 (US); Geary, Richard S., Carlsbad, CA 92010 (US); McKay, Robert, Poway, CA 92064 (US); Monia, Brett P., Encinitas, CA 92024 (US); Seth, Punit P., San Marcos, CA 92078 (US); Siwkowski, Andrew M., Carlsbad, CA 92009 (US); Swayze, Eric E., Encinitas, CA 92024 (US); Wancewicz, Edward, Poway, CA 92064 (US)
(74) Representative: Carpmaels & Ransford LLP

(56) References cited:
- WO-A2-2004/044181
- WO-A2-2004/046160
- FRIEDEN M ET AL: "Expanding the design horizon of antisense oligonucleotides with alpha-L-LNA", NUCLEIC ACIDS RESEARCH, OXFORD UNIVERSITY PRESS, SURREY, GB, vol. 31, no. 21, 1 November 2003 (2003-11-01), pages 6365-6372, XP002281376, ISSN: 0305-1048, DOI: 10.1093/NAR/GKG820

## Description

### BACKGROUND

Targeting disease-causing gene sequences was first suggested nearly 40 years ago (Belikova et al., Tet. Lett., 1967, 37, 3557-3562), and antisense activity was demonstrated in cell culture a decade later (Zamecnik et al., Proc. Natl. Acad. Sci. U.S.A., 1978, 75, 280-284). One advantage of antisense technology in the treatment of a disease or condition that stems from a disease-causing gene is that it is a direct genetic approach that has the ability to modulate expression of specific disease-causing genes.

Generally, the principle behind antisense technology is that an antisense compound hybridizes to a target nucleic acid and effects modulation of gene expression activity or function, such as transcription, translation or splicing. The modulation of gene expression can be achieved by, for example, target degradation or occupancy-based inhibition. An example of modulation of RNA target function by degradation is RNase H-based degradation of the target RNA upon hybridization with a DNA-like antisense compound. Another example of modulation of gene expression by target degradation is RNA interference (RNAi). RNAi is a form of antisense-mediated gene silencing involving the introduction of dsRNA leading to the sequence-specific reduction of targeted endogenous mRNA levels. Sequence-specificity makes antisense compounds extremely attractive as tools for target validation and gene functionalization, as well as research tools for identifying and characterizing nucleases and as therapeutics to selectively modulate the expression of genes involved in the pathogenesis of any one of a variety of diseases.

Antisense technology is an effective means for reducing the expression of one or more specific gene products and can therefore prove to be uniquely useful in a number of therapeutic, diagnostic, and research applications. Chemically modified nucleosides are routinely used for incorporation into antisense compounds to enhance one or more properties, such as nuclease resistance, pharmacokinetics or affinity for a target RNA.

Despite the expansion of knowledge since the discovery of antisense technology, there remains an unmet need for antisense compounds with greater efficacy, reduced toxicity and lower cost. Until the present disclosure, high-affinity modifications have not been employed in the design of short antisense compounds for reducing target RNA *in vivo.* This is because of concerns regarding the degree of target specificity that a sequence 15 nucleotides or shorter would have when employed to reduce target in a living system. Previous studies have described that greater specificity, and therefore greater potential for potency, is achieved by antisense compounds between 16 and 20 nucleobases in length.

The present disclosure describes incorporation of chemically-modified high-affinity nucleotides into antisense compounds allows for short antisense compounds about 8-16 nucleobases in length useful in the reduction of target RNAs in animals with increased potency and improved therapeutic index. Thus, provided herein are short antisense compounds comprising high-affinity nucleotide modifications useful for reducing a target RNA *in vivo.* Such short antisense compounds are effective at lower doses than previously described antisense compounds, allowing for a reduction in toxicity and cost of treatment.

### SUMMARY OF THE INVENTION

The invention provides a short antisense compound 10 to 14 monomers in length, comprising a 2'-deoxyribonucleotide gap region flanked on each side by a wing, wherein each wing independently comprises 1 to 3 high-affinity modified nucleotides which are sugar-modified nucleotides that comprise a bridge between the 4' and the 2' position of the sugar, and wherein the short antisense compound is targeted to a nucleic acid encoding ApoB, for use in treating an individual having hypercholesterolemia, familial hypercholesterolemia, heterozygous familial hypercholesterolemia, homozygous familial hypercholesterolemia, non-familial hypercholesterolemia, mixed dyslipidemia, atherosclerosis, a risk of developing atherosclerosis, coronary heart disease, a history of coronary heart disease, early onset coronary heart disease, one or more risk factors for coronary heart disease, type II diabetes, type II diabetes with dyslipidemia, dyslipidemia, hypertriglyceridemia, hyperlipidemia, hyperfattyacidemia, hepatic steatosis, non-alcoholic steatohepatitis, or non-alcoholic fatty liver disease.

The invention also provides a short antisense compound 10 to 14 monomers in length, comprising a 2'-deoxyribonucleotide gap region flanked on each side by a wing, wherein each wing independently comprises 1 to 3 high-affinity modified nucleotides which are sugar-modified nucleotides that comprise a bridge between the 4' and the 2' position of the sugar, wherein the short antisense compound is targeted to a nucleic acid encoding ApoB, and wherein:
(a) the short antisense compound is 10, 11, 12 or 13 monomers in length;
(b) the short antisense compound further comprises a conjugate group;
(c) the short antisense compound has phosphodiester linkages in the wings and phosphorothioate linkages in the gap; or
(d) the short antisense compound includes one mismatch, two mismatches or three mismatches to the target sequence.

Other aspects of the invention are set out in the appended claims.

### SUMMARY OF THE DISCLOSURE

Disclosed herein are short antisense compounds and methods of using said compounds to reduce target RNA expression in cells or tissues. In certain embodiments, disclosed herein is a method of reducing expression of a target in an animal, comprising administering to the animal a short antisense compound targeted to a nucleic acid of such target. In certain embodiments, shorts antisense compounds are oligonucleotide compounds. In certain embodiments short antisense oligonucleotides are about 8 to 16, preferably 9 to 15, more preferably 9 to 14, more preferably 10 to 14 nucleotides in length and comprises a gap region flanked on each side by a wing, wherein each wing independently consists of 1 to 3 nucleotides. Preferred motifs include but are not limited to wing - deoxy gap -wing motifs selected from 3-10-3, 2-10-3, 2-10-2, 1-10-1, 2-8-2, 1-8-1, 3-6-3 or 1-6-1. In a preferred embodiment, the short antisense oligonucleotide comprise at least one high-affinity modification. In a further embodiment, the high-affinity modification includes chemically-modified high-affinity nucleotides. In a preferred embodiment, each wing independently consists of 1 to 3 high-affinity modified nucleotides. In one embodiment the high affinity modified nucleotides are sugar-modified nucleotides.

In certain embodiments short antisense compounds exhibit greater uptake in the gut as compared to antisense compounds of greater length. Thus, also disclosed herein are methods of reducing a target in an animal, comprising orally administering the short antisense compounds of the present disclosure.

Further disclosed are methods of treating a metabolic disorder in an animal, comprising administering to an animal in need of such therapy a short antisense compound targeted to a nucleic acid involved in regulating glucose metabolism or clearance, lipid metabolism, cholesterol metabolism, or insulin signaling.

Also disclosed are methods of increasing insulin sensitivity, decreasing blood glucose or decreasing HbA_{1c} in an animal, comprising administering to said animal a short antisense compound targeted to a nucleic acid encoding a target involved in regulating glucose metabolism or clearance, lipid metabolism, cholesterol metabolism, or insulin signaling.

Further disclosed are methods of decreasing total serum cholesterol, serum LDL, serum VLDL, serum HDL, serum triglycerides, serum apolipoprotein(a) or free fatty acids in an animal, comprising administering to said animal a short antisense compound targeted to a nucleic acid encoding a target that is involved in regulating glucose metabolism or clearance, lipid metabolism, cholesterol metabolism, or insulin signaling, wherein said short antisense compound is 8 to 16 nucleotides in length and comprises a gap region flanked on each side by a wing, wherein each wing independently consists of 1 to 3 high-affinity modified nucleotides.

In certain embodiments, short antisense compounds further comprise a conjugate group. Conjugate groups include, but are not limited to, C₁₆ and cholesterol.

In certain embodiments short antisense compounds comprise at least one modified nucleobase, internucleoside linkage or sugar moiety. In certain embodiments, such modified internucleoside linkage is a phosphorothioate internucleoside linkage. In certain embodiments, each internucleoside linkage is a phosphorothioate internucleoside linkage.

In certain embodiments, short antisense compounds comprise at least one high affinity modification. In certain such embodiments, the high-affinity modification is a chemically-modified high-affinity nucleotide. In certain embodiments, chemically-modified high affinity nucleotides are sugar-modified nucleotides. In certain embodiments, at least one of the sugar-modified nucleotides comprises a bridge between the 4' and the 2' position of the sugar. Each of the sugar-modified nucleotides is, independently, in the β-D or α-L sugar conformation. In certain embodiments, each of said high-affinity modified nucleotides confers a ΔTₘ of at least 1 to 4 degrees per nucleotide. In certain embodiments, each of said sugar-modified nucleotides comprises a 2'-substituent group that is other than H or OH. Such sugar-modified nucleotides include those having a 4' to 2' bridged bicyclic sugar moiety. In certain embodiments, each of the 2'-substituent groups is, independently, alkoxy, substituted alkoxy, or halogen. In certain embodiments, each of the 2'-substituent groups is OCH₂CH₂OCH₃ (2'-MOE).

In certain embodiments, short antisense compounds have one or more sugar-modified nucleotides comprising a bridge between the 4' and 2' position of the sugar, wherein each of said bridges independently comprises from 2 to 4 linked groups independently selected from -[C(R₁)(R₂)]ₙ-, -C(R₁)=C(R₂)-, -C(R₁)=N-, -C(=NR₁)-, -C(=O)-, -C(=S)-, -O-, -Si(R₁)₂-, -S(=O)ₓ- and -N(R₁)-;
wherein
x is 0, 1, or 2;
n is 1, 2, 3, or 4;
   each R₁ and R₂ is, independently, H, a protecting group, hydroxyl, C₁-C₁₂ alkyl, substituted C₁-C₁₂ alkyl, C₂-C₁₂ alkenyl, substituted C₂-C₁₂ alkenyl, C_{2 The}-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
   each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

In one aspect, each of said bridges is, independently, -[C(R₁)(R₂)]ₙ-, -[C(R₁)(R₂)]ₙ-O-, -C(R₁R₂)-N(R₁)-O- or -C(R₁R₂)-O-N(R₁)-. In another aspect, each of said bridges is, independently, 4'-(CH₂)₃-2', 4'-(CH₂)₂-2', 4'-CH₂-O-2', 4'-(CH₂)₂-O-2', 4'-CH₂-O-N(R₁)-2' and 4'-CH₂-N(R₁)-O-2'- wherein each R₁ is, independently, H, a protecting group or C₁-C₁₂ alkyl.

In certain embodiments, disclosed herein are short antisense compounds useful in the reduction of targets and/or target RNAs associated with disease states in animals. In certain embodiments, disclosed are methods of using the short antisense compounds for reducing expression of a target RNA in an animal. In certain embodiments, disclosed herein is the use of a short antisense compound in the preparation of a medicament for the treatment of a metabolic disorder in an animal. In certain embodiments, disclosed herein is the use of a short antisense compound in the preparation of a medicament for increasing insulin sensitivity, decreasing blood glucose or decreasing HbA_{1c} in an animal. Also disclosed is the use of a short antisense compound in the preparation of a medicament for decreasing total serum cholesterol, serum LDL, serum VLDL, serum HDL, serum triglycerides, serum apolipoprotein(a) or free fatty acids in an animal.

In certain embodiments, short antisense compounds disclosed herein exhibit equal or increased potency with regard to target RNA knockdown as compared to longer parent antisense oligonucleotide at least 20 nucleotides in length. In certain embodiments, short antisense compounds exhibit a faster onset of action (target RNA reduction) as compared to the parent antisense oligonucleotide. In certain embodiments, increased potency is in the kidney. In certain embodiments, target RNA is predominately expressed in the kidney. In certain embodiments, increased potency is in the liver. In certain embodiments, target RNA is predominately expressed in the liver.

### DETAILED DESCRIPTION

It is to be understood that both the foregoing general description and the following detailed description are exemplary and explanatory only and are not restrictive of the invention, as claimed. Herein, the use of the singular includes the plural unless specifically stated otherwise. As used herein, the use of "or" means "and/or" unless stated otherwise. Furthermore, the use of the term "including" as well as other forms, such as "includes" and "included", is not limiting. Also, terms such as "element" or "component" encompass both elements and components comprising one unit and elements and components that comprise more than one subunit, unless specifically stated otherwise.

The section headings used herein are for organizational purposes only and are not to be construed as limiting the subject matter described.

### A. Definitions

Unless specific definitions are provided, the nomenclature utilized in connection with, and the procedures and techniques of, analytical chemistry, synthetic organic chemistry, and medicinal and pharmaceutical chemistry described herein are those well known and commonly used in the art. Standard techniques may be used for chemical synthesis, chemical analysis, pharmaceutical preparation, formulation and delivery, and treatment of subjects. Certain such techniques and procedures may be found for example in "Carbohydrate Modifications in Antisense Research" Edited by Sangvi and Cook, American Chemical Society, Washington D.C., 1994; and "Remington's Pharmaceutical Sciences," Mack Publishing Co., Easton, Pa., 18th edition, 1990.

Unless otherwise indicated, the following terms have the following meanings:
As used herein, the term "nucleoside" means a glycosylamine comprising a nucleobase and a sugar. Nucleosides includes, but are not limited to, naturally occurring nucleosides, abasic nucleosides, modified nucleosides, and nucleosides having mimetic bases and/or sugar groups.

As used herein, the term "nucleotide" refers to a glycosomine comprising a nucleobase and a sugur having a phosphate group covalently linked to the sugar. Nucleotides may be modified with any of a variety of substituents.

As used herein, the term "nucleobase" refers to the base portion of a nucleoside or nucleotide. A nucleobase may comprise any atom or group of atoms capable of hydrogen bonding to a base of another nucleic acid.

As used herein, the term "heterocyclic base moiety" refers to a nucleobase comprising a heterocycle.

As used herein, the term "deoxyribonucleotide" means a nucleotide having a hydrogen at the 2' position of the sugar portion of the nucleotide. Deoxyribonucleotides may be modified with any of a variety of substituents.

As used herein, the term "ribonucleotide" means a nucleotide having a hydroxy at the 2' position of the sugar portion of the nucleotide. Ribonucleotides may be modified with any of a variety of substituents.

As used herein, the term "oligomeric compound" refers to a polymeric structure comprising two or more sub-structures and capable of hybridizing to a region of a nucleic acid molecule. In certain embodiments, oligomeric compounds are oligonucleosides. In certain embodiments, oligomeric compounds are oligonucleotides. In certain embodiments, oligomeric compounds are antisense compounds. In certain embodiments, oligomeric compounds are antisense oligonucleotides. In certain embodiments, oligomeric compounds are short antisense compounds. In certain embodiments, oligomeric compounds are short antisense oligonucleotides. In certain embodiments, oligomeric compounds are chimeric oligonucleotides.

As used herein, the term "monomer" refers to a single unit of an oligomer. Monomers include, but are not limited to, nucleosides and nucleotides, whether naturally occuring or modified.

As used herein "oligonucleoside" refers to an oligonucleotide in which the internucleoside linkages do not contain a phosphorus atom.

As used herein, the term "oligonucleotide" refers to an oligomeric compound comprising a plurality of linked nucleotides. In certain embodiment, one or more nucleotides of an oligonucleotide is modified. In certain embodiments, an oligonucleotide comprises ribonucleic acid (RNA) or deoxyribonucleic acid (DNA). In certain embodiments, oligonucleotides are composed of naturally- and/or non-naturally-occurring nucleobases, sugars and covalent internucleotide linkages, and may further include non-nucleic acid conjugates.

As used herein "internucleotide linkage" refers to a covalent linkage between adjacent nucleotides.

As used herein, the term "monomeric linkage" refers to a covalent linkage between two monmers. Monomeric linkages include, but are not limited to internucleotide linkages and internucleoside linkages.

As used herein "naturally occuring internucleotide linkage" refers to a 3' to 5' phosphodiester linkage.

As used herein, the term "antisense compound" refers to an oligomeric compound that is at least partially complementary to a target nucleic acid molecule to which it hybridizes. In certain embodiments, an antisense compound modulates (increases or decreases) expression of a target nucleic acid. Antisense compounds include, but are not limited to, compounds that are oligonucleotides, oligonucleosides, oligonucleotide analogs, oligonucleotide mimetics, and chimeric combinations of these. Consequently, while all antisense compounds are oligomeric compounds, not all oligomeric compounds are antisense compounds.

As used herein, the term "antisense oligonucleotide" refers to an antisense compound that is an oligonucleotide.

As used herein, the term "parent antisense oligonucleotide" refers to an oligonucleotide 20 nucleotides in length having a deoxy gap region having ten 2'-deoxyribonucleotides, flanked by a first and a second wing region each having five 2'-O-(2-methoxyethyl) ribonucleotides (a 5-10-5 MOE gapmer) and comprising the sequence of the corresponding short antisense compound to which it is a parent.

As used herein, the term "short antisense compound" refers to an antisense compound about 8, 9, 10, 11, 12, 13, 14, 15 or 16 monomers in length. In certain embodiments, a short antisense compound has at least one high-affinity modification.

As used herein, the term "short antisense oligonucleotide" or refers to an antisense oligonucleotide about 8, 9, 10, 11, 12, 13, 14, 15 or 16 nucleotides in length. In certain embodiments, a short antisense oligonucleotide has at least one high-affinity modification.

As used herein, the term "short gapmer" refers to a short antisense oligonucleotide having a first and a second wing region each independently 1 to 3 nucleotides in length and a gap region 2 to 14 nucleobase in length.

As used herein, the term "motif' refers to the pattern of unmodified and modified nucleotides in a short antisense compound
As used herein, the term "chimeric antisense oligomer" refers to an antisense oligomeric compound, having at least one sugar, nucleobase or internucleoside linkage that is differentially modified as compared to at least on other sugar, nucleobase or internucleoside linkage within the same antisense oligomeric compound. The remainder of the sugars, nucleobases and internucleoside linkages can be independently modified or unmodified, the same or different.

As used herein, the term "chimeric antisense oligonucleotide" refers to an antisense oligonucleotide, having at least one sugar, nucleobase or internucleoside linkage that is differentially modified as compared to at least on other sugar, nucleobase or internucleoside linkage within the same antisense oligonucleotide. The remainder of the sugars, nucleobases and internucleoside linkages can be independently modified or unmodified, the same or different.

As used herein, the term "mixed-backbone antisense oligonucleotide" refers to an antisense oligonucleotide wherein at least one internucleoside linkage of the antisense oligonucleotide is different from at least one other internucleotide linkage of the antisense oligonucleotide.

As used herein, the term "target" refers to a protein, the modulation of which is desired.

As used herein, the term "target gene" refers to a gene encoding a target.

As used herein, the terms "target nucleic acid" and "nucleic acid molecule encoding a target" refer to any nucleic acid molecule the expression or activity of which is capable of being modulated by an antisense compound. Target nucleic acids include, but are not limited to, RNA (including, but not limited to pre-mRNA and mRNA or portions thereof) transcribed from DNA encoding a target, and also cDNA derived from such RNA, and miRNA. For example, the target nucleic acid can be a cellular gene (or mRNA transcribed from the gene) whose expression is associated with a particular disorder or disease state, or a nucleic acid molecule from an infectious agent.

As used herein, the term "targeting" or "targeted to" refers to the association of an antisense compound to a particular target nucleic acid molecule or a particular region of nucleotides within a target nucleic acid molecule.

As used herein, the term "5' target site" refers to the nucleotide of a target nucleic acid which is complementary to the 5'-most nucleotide of a particular antisense compound.

As used herein, the term "3' target site" refers to the nucleotide of a target nucleic acid which is complementary to the 3'-most nucleotide of a particular antisense compound.

As used herein, the term "target region," refers to a portion of a target nucleic acid to which one or more antisense compounds is complementary.

As used herein, the term "target segment" refers to a smaller or sub-portions of a region within a target nucleic acid.

As used herein, the term "nucleobase complementarity" refers to a nucleobase that is capable of base pairing with another nucleobase. For example, in DNA, adenine (A) is complementary to thymine (T). For example, in RNA, adenine (A) is complementary to uracil (U). In certain embodiments, complementary nucleobase refers to a nucleobase of an antisense compound that is capable of base pairing with a nucleobase of its target nucleic acid. For example, if a nucleobase at a certain position of an antisense compound is capable of hydrogen bonding with a nucleobase at a certain position of a target nucleic acid, then the position of hydrogen bonding between the oligonucleotide and the target nucleic acid is considered to be complementary at that nucleobase pair.

As used herein, the term "non-complementary nucleobase" refers to a pair of nucleobases that do not form hydrogen bonds with one another or otherwise support hybridization.

As used herein, the term "complementary" refers to the capacity of an oligomeric compound to hybridize to another oligomeric compound or nucleic acid through nucleobase complementarity. In certain embodiments, an antisense compound and its target are complementary to each other when a sufficient number of corresponding positions in each molecule are occupied by nucleobases that can bond with each other to allow stable association between the antisense compound and the target. One skilled in the art recognizes that the inclusion of mismatches is possible without eliminating the ability of the oligomeric compounds to remain in association. Therefore, described herein are antisense compounds that may comprise up to about 20% nucleotides that are mismatched (i.e., are not nucleobase complementary to the corresponding nucleotides of the target). Preferably the antisense compounds contain no more than about 15%, more preferably not more than about 10%, most preferably not more than 5% or no mismatches. The remaining nucleotides are nucleobase complementary or otherwise do not disrupt hybridization (e.g., universal bases). One of ordinary skill in the art would recognize the compounds provided herein are at least 80%, at least 85%, at least 90%, at least 95%, at least 96%, at least 97%, at least 98%, at least 99% or 100% complementary to a target nucleic acid.

As used herein, the term "mismatch" refers to a non-complementary nucleobase within a complementary oligomeric compound.

As used herein, "hybridization" means the pairing of complementary oligomeric compounds (e.g., an antisense compound and its target nucleic acid). While not limited to a particular mechanism, the most common mechanism of pairing involves hydrogen bonding, which may be Watson-Crick, Hoogsteen or reversed Hoogsteen hydrogen bonding, between complementary nucleoside or nucleotide bases (nucleobases). For example, the natural base adenine is nucleobase complementary to the natural nucleobases thymidine and uracil which pair through the formation of hydrogen bonds. The natural base guanine is nucleobase complementary to the natural bases cytosine and 5-methyl cytosine. Hybridization can occur under varying circumstances.

As used herein, the term "specifically hybridizes" refers to the ability of an oligomeric compound to hybridize to one nucleic acid site with greater affinity than it hybridizes to another nucleic acid site. In certain embodiments, an antisense oligonucleotide specifically hybridizes to more than one target site.

As used herein, "designing" or "designed to" refer to the process of designing an oligomeric compound that specifically hybridizes with a selected nucleic acid molecule.

As used herein, the term "modulation" refers to a perturbation of function or activity when compared to the level of the function or activity prior to modulation. For example, modulation includes the change, either an increase (stimulation or induction) or a decrease (inhibition or reduction) in gene expression. As further example, modulation of expression can include perturbing splice site selection of pre-mRNA processing.

As used herein, the term "expression" refers to all the functions and steps by which a gene's coded information is converted into structures present and operating in a cell. Such structures include, but are not limited to the products of transcription and translation.

As used herein, "variant" refers to an alternative RNA transcript that can be produced from the same genomic region of DNA. Variants include, but are not limited to "pre-mRNA variants" which are transcripts produced from the same genomic DNA that differ from other transcripts produced from the same genomic DNA in either their start or stop position and contain both intronic and exonic sequence. Variants also include, but are not limited to, those with alternate splice junctions, or alternate initiation and termination codons.

As used herein, "high-affinity modified monomer" refers to a monomer having at least one modified nucleobase, internucleoside linkage or sugar moiety, when compared to naturally occurring monomers, such that the modification increases the affinity of an antisense compound comprising the high-affinity modified monomer to its target nucleic acid. High-affinity modifications include, but are not limited to, monomers (e.g., nucleosides and nucleotides) comprising 2'-modifed sugars.

As used herein, the term "2'-modified" or "2'-substituted" means a sugar comprising substituent at the 2' position other than H or OH. 2'-modified monomers, include, but are not limited to, BNA's and monomers (e.g., nucleosides and nucleotides) with 2'- substituents, such as allyl, amino, azido, thio, O-allyl, O-C₁-C₁₀ alkyl, -OCF₃, O-(CH₂)₂-O-CH₃, 2'-O(CH₂)₂SCH₃, O-(CH₂)₂-O-N(Rₘ)(Rₙ), or O-CH₂-C(=O)-N(Rₘ)(Rₙ), where each Rₘ and Rₙ is, independently, H or substituted or unsubstituted C₁-C₁₀ alkyl. In certain embodiments, short antisense compounds comprise a 2'modified monomer that does not have the formula 2'-O(CH₂)ₙH, wherein n is one to six. In certain embodiments, short antisense compounds comprise a 2'modified monomer that does not have the formula 2'-OCH₃. In certain embodiments, short antisense compounds comprise a 2'modified monomer that does not have the formula or, in the alternative, 2'-O(CH₂)₂OCH₃.

As used herein, the term "bicyclic nucleic acid" or "BNA" or "bicyclic nucleoside" or "bicyclic nucleotide" refers to a nucleoside or nucleotide wherein the furanose portion of the nucleoside includes a bridge connecting two carbon atoms on the furanose ring, thereby forming a bicyclic ring system.

As used herein, unless otherwise indicated, the term "methyleneoxy BNA" alone refers to β-D-methyleneoxy BNA.

As used herein, the term "MOE" refers to a 2'-methoxyethyl substituent.

As used herein, the term "gapmer" refers to a chimeric oligomeric compound comprising a central region (a "gap") and a region on either side of the central region (the "wings"), wherein the gap comprises at least one modification that is different from that of each wing. Such modifications include nucleobase, monomeric linkage, and sugar modifications as well as the absence of modification (unmodified). Thus, in certain embodiments, the nucleotide linkages in each of the wings are different than the nucleotide linkages in the gap. In certain embodiments, each wing comprises nucleotides with high affinity modifications and the gap comprises nucleotides that do not comprise that modification. In certain embodiments the nucleotides in the gap and the nucleotides in the wings all comprise high affinity modifications, but the high affinity modifications in the gap are different than the high affinity modifications in the wings. In certain embodiments, the modifications in the wings are the same as one another. In certain embodiments, the modifications in the wings are different from each other. In certain embodiments, nucleotides in the gap are unmodified and nucleotides in the wings are modified. In certain embodiments, the modification(s) in each wing are the same. In certain embodiments, the modification(s) in one wing are different from the modification(s) in the other wing. In certain embodiments, short antisense compounds are gapmers having 2'-deoxynucleotides in the gap and nucleotides with high-affinity modifications in the wing.

As used herein, the term "prodrug" refers to a therapeutic agent that is prepared in an inactive form that is converted to an active form (i.e., drug) within the body or cells thereof by the action of endogenous enzymes or other chemicals and/or conditions.

As used herein, the term "pharmaceutically acceptable salts" refers to salts of active compounds that retain the desired biological activity of the active compound and do not impart undesired toxicological effects thereto.

As used herein, the term "cap structure" or "terminal cap moiety" refers to chemical modifications, which have been incorporated at either terminus of an antisense compound.

As used herein, the term "prevention" refers to delaying or forestalling the onset or development of a condition or disease for a period of time from hours to days, preferably weeks to months.

As used herein, the term "amelioration" refers to a lessening of at least one indicator of the severity of a condition or disease. The severity of indicators may be determined by subjective or objective measures which are known to those skilled in the art.

As used herein, the term "treatment" refers to administering a composition of the invention to effect an alteration or improvement of the disease or condition. Prevention, amelioration, and/or treatment may require administration of multiple doses at regular intervals, or prior to onset of the disease or condition to alter the course of the disease or condition. Moreover, a single agent may be used in a single individual for each prevention, amelioration, and treatment of a condition or disease sequentially, or concurrently.

As used herein, the term "pharmaceutical agent" refers to a substance provides a therapeutic benefit when administered to a subject.

As used herein, the term "therapeutically effective amount" refers to an amount of a pharmaceutical agent that provides a therapeutic benefit to an animal.

As used herein, "administering" means providing a pharmaceutical agent to an animal, and includes, but is not limited to administering by a medical professional and self-administering.

As used herein, the term "co-administration" refers to administration of two or more pharmaceutical agents to an animal. The two or more pharmaceutical agents may be in a single pharmaceutical composition, or may be in separate pharmaceutical compositions. Each of the two or more pharmaceutical agents may be administered through the same or different routes of administration. Co-administration encompasses administration in parallel or sequentially.

As used herein, the term "pharmaceutical composition" refers to a mixture of substances suitable for administering to an individual. For example, a pharmaceutical composition may comprise an antisense oligonucleotide and a sterile aqueous solution.

As used herein, the term "individual" refers to a human or non-human animal selected for treatment or therapy.

As used herein, the term "animal" refers to a human or non-human animal, including, but not limited to, mice, rats, rabbits, dogs, cats, pigs, and non-human primates, including, but not limited to, monkeys and chimpanzees.

As used herein, the term "subject" refers to an animal, including, but not limited to a human, to whom a pharmaceutical composition is administered.

As used herein, the term "duration" refers to the period of time during which an activity or event continues. In certain embodiments, the duration of treatment is the period of time during which doses of a pharmaceutical agent are administered.

As used herein, the term "parenteral administration," refers to administration through injection or infusion. Parenteral administration includes, but is not limited to, subcutaneous administration, intravenous administration, or intramuscular administration.

As used herein, the term "subcutaneous administration" refers to administration just below the skin. "Intravenous administration" means administration into a vein.

As used herein, the term "dose" refers to a specified quantity of a pharmaceutical agent provided in a single administration. In certain embodiments, a dose may be administered in two or more boluses, tablets, or injections. For example, in certain embodiments, where subcutaneous administration is desired, the desired dose requires a volume not easily accommodated by a single injection. In such embodiments, two or more injections may be used to achieve the desired dose. In certain embodiments, a dose may be administered in two or more injections to minimize injection site reaction in an individual.

As used herein, the term "dosage unit" refers to a form in which a pharmaceutical agent is provided. In certain embodiments, a dosage unit is a vial comprising lyophilized antisense oligonucleotide. In certain embodiments, a dosage unit is a vial comprising reconstituted antisense oligonucleotide.

As used herein, the term "pharmaceutical agent" refers to a substance provides a therapeutic benefit when administered to an individual. For example, in certain embodiments, an antisense oligonucleotide is a pharmaceutical agent.

As used herein, the term "active pharmaceutical ingredient" refers to the substance in a pharmaceutical composition that provides a desired effect.

As used herein, the term "therapeutically effective amount" refers to an amount of a pharmaceutical agent that provides a therapeutic benefit to an individual. In certain embodiments, a therapeutically effective amount of an antisense compound is the amount that needs to be administered to result in an observable benefit.

As used herein, the term "hypercholesterolemia" refers to a condition characterized by elevated serum cholesterol.

As used herein, the term "hyperlipidemia" refers to a condition characterized by elevated serum lipids.

As used herein, the term "hypertriglyceridemia" refers to a condition characterized by elevated triglyceride levels.

As used herein, the term "non-familial hypercholesterolemia" refers to a condition characterized by elevated cholesterol that is not the result of a single inherited gene mutation.

As used herein, the term "polygenic hypercholesterolemia" refers to a condition characterized by elevated cholesterol that results from the influence of a variety of genetic factors. In certain embodiments, polygenic hypercholesterolemia may be exacerbated by dietary intake of lipids.

As used herein, the term "familial hypercholesterolemia (FH)" refers to an autosomal dominant metabolic disorder characterized by a mutation in the LDL-receptor (LDL-R) gene, markedly elevated LDL-C and premature onset of atherosclerosis. A diagnosis of familial hypercholesterolemia is made when a individual meets one or more of the following criteria: genetic testing confirming 2 mutated LDL-receptor genes; genetic testing confirming one mutated LDL-receptor gene; document history of untreated serum LDL-cholesterol greater than 500 mg/dL; tendinous and/or cutaneous xanthoma prior to age 10 years; or, both parents have documented elevated serum LDL-cholesterol prior to lipid-lowering therapy consistent with heterozygous familial hypercholesterolemia.

As used herein, the term "homozygous familial hypercholesterolemia" or "HoFH" refers to a condition characterized by a mutation in both maternal and paternal LDL-R genes.

As used herein, the term "heterozygous familial hypercholesterolemia" or "HeFH" refers to a condition characterized by a mutation in either the maternal or paternal LDL-R gene.

As used herein, the term "mixed dyslipidemia" refers to a condition characterized by elevated serum cholesterol and elevated serum triglycerides.

As used herein, the term "diabetic dyslipidemia" or "Type II diabetes with dyslipidemia" refers to a condition characterized by Type II diabetes, reduced HDL-C, elevated serum triglycerides, and elevated small, dense LDL particles.

As used herein, the term "CHD risk equivalents," refers to indicators of clinical atherosclerotic disease that confer a high risk for coronary heart disease. For example, in certain embodiments, CHD risk equivalents include, without limitation, clinical coronary heart disease, symptomatic carotid artery disease, peripheral arterial disease, and/or abdominal aortic aneurysm.

As used herein, the term "non-alcoholic fatty liver disease (NAFLD)" refers to a condition characterized by fatty inflammation of the liver that is not due to excessive alcohol use (for example, alcohol consumption of over 20 g/day). In certain embodiments, NAFLD is related to insulin resistance and the metabolic syndrome.

As used herein, the term "non-alcoholic steatohepatitis (NASH)" refers to a condition characterized by inflammation and the accumulation of fat and fibrous tissue in the liver, that is not due to excessive alcohol use. NASH is an extreme form of NAFLD.

As used herein, the term "major risk factors" refers to factors that contribute to a high risk for a particular disease or condition. In certain embodiments, major risk factors for coronary heart disease include, without limitation, cigarette smoking, hypertension, low HDL-C, family history of coronary heart disease, and age.

As used herein, the term "CHD risk factors" refers to CHD risk equivalents and major risk factors.

As used herein, the term "coronary heart disease (CHD)" refers to a narrowing of the small blood vessels that supply blood and oxygen to the heart, which is often a result of atherosclerosis.

As used herein, the term "reduced coronary heart disease risk" refers to a reduction in the likelihood that a individual will develop coronary heart disease. In certain embodiments, a reduction in coronary heart disease risk is measured by an improvement in one or more CHD risk factors, for example, a decrease in LDL-C levels.

As used herein, the term "atherosclerosis" refers to a hardening of the arteries affecting large and medium-sized arteries and is characterized by the presence of fatty deposits. The fatty deposits are called "atheromas" or "plaques," which consist mainly of cholesterol and other fats, calcium and scar tissue, and damage the lining of arteries.

As used herein, the term "history of coronary heart disease" refers to the occurrence of clinically evident coronary heart disease in the medical history of a individual or a individual's family member.

As used herein, the term "Early onset coronary heart disease" refers to a diagnosis of coronary heart disease prior to age 50.

As used herein, the term "statin intolerant individual" refers to a individual who as a result of statin therapy experiences one or more of creatine kinase increases, liver function test abnormalities, muscle aches, or central nervous system side effects.

As used herein, the term "efficacy" refers to the ability to produce a desired effect. For example, efficacy of a lipid-lowering therapy may be reduction in the concentration of one or more of LDL-C, VLDL-C, IDL-C, non-HDL-C, ApoB, lipoprotein(a), or triglycerides.

As used herein, the term "acceptable safety profile" refers to a pattern of side effects that is within clinically acceptable limits.

As used herein, the term "side effects" refers to physiological responses attributable to a treatment other than desired effects. In certain embodiments, side effects include, without limitation, injection site reactions, liver function test abnormalities, renal function abnormalities, liver toxicity, renal toxicity, central nervous system abnormalities, and myopathies. For example, increased aminotransferase levels in serum may indicate liver toxicity or liver function abnormality. For example, increased bilirubin may indicate liver toxicity or liver function abnormality.

As used herein, the term "injection site reaction" refers to inflammation or abnormal redness of skin at a site of injection in an individual.

As used herein, the term "individual compliance" refers to adherence to a recommended or prescribed therapy by an individual.

As used herein, the term "lipid-lowering therapy" refers to a therapeutic regimen provided to a individual to reduce one or more lipids in a individual. In certain embodiments, a lipid-lowering therapy is provide to reduce one or more of ApoB, total cholesterol, LDL-C, VLDL-C, IDL-C, non-HDL-C, triglycerides, small dense LDL particles, and Lp(a) in an individual.

As used herein, the term "lipid-lowering agent" refers to a pharmaceutical agent provided to a individual to achieve a lowering of lipids in the individual. For example, in certain embodiments, a lipid-lowering agent is provided to an individual to reduce one or more of ApoB, LDL-C, total cholesterol, and triglycerides.

As used herein, the term "LDL-C target" refers to an LDL-C level that is desired following lipid-lowering therapy.

As used herein, the term "comply" refers to the adherence with a recommended therapy by an individual.

As used herein, the term "recommended therapy" refers to a therapeutic regimen recommended by a medical professional for the treatment, amelioration, or prevention of a disease.

As used herein, the term "low LDL-receptor activity" refers to LDL-receptor activity that is not sufficiently high to maintain clinically acceptable levels of LDL-C in the bloodstream.

As used herein, the term "cardiovascular outcome" refers to the occurrence of major adverse cardiovascular events.

As used herein, the term "improved cardiovascular outcome" refers to a reduction in the occurrence of major adverse cardiovascular events, or the risk thereof. Examples of major adverse cardiovascular events include, without limitation, death, reinfarction, stroke, cardiogenic shock, pulmonary edema, cardiac arrest, and atrial dysrhythmia.

As used herein, the term "surrogate markers of cardiovascular outcome" refers to indirect indicators of cardiovascular events, or the risk thereof. For example, surrogate markers of cardiovascular outcome include carotid intimal media thickness (CIMT). Another example of a surrogate marker of cardiovascular outcome includes atheroma size. Atheroma size may be determined by intravascular ultrasound (IVUS).

As used herein, the term "increased HDL-C" refers to an increase in serum HDL-C in an individual over time.

As used herein, the term "lipid-lowering" refers to a reduction in one or more serum lipids in an individual over time.

As used herein, the term "metabolic disorder" refers to a condition characterized by an alteration or disturbance in metabolic function. "Metabolic" and "metabolism" are terms well know in the art and generally include the whole range of biochemical processes that occur within a living organism. Metabolic disorders include, but are not limited to, hyperglycemia, prediabetes, diabetes (type I and type II), obesity, insulin resistance and metabolic syndrome.

As used herein, the term "metabolic syndrome" refers to a clustering of lipid and non-lipid cardiovascular risk factors of metabolic origin. It has been closely linked to the generalized metabolic disorder known as insulin resistance. The National Cholesterol Education Program (NCEP) Adult Treatment Panel III (ATPIII) established criteria for diagnosis of metabolic syndrome when three or more of five risk determinants are present. The five risk determinants are abdominal obesity defined as waist circumference of greater than 102 cm for men or greater than 88cm for women, triglyceride levels greater than or equal to 150 mg/dL, HDL cholesterol levels of less than 40 mg/dL for men and less than 50 mg/dL for women, blood pressure greater than or equal to 130/85 mm Hg and fasting glucose levels greater than or equal to 110 mg/dL. These determinants can be readily measured in clinical practice (JAMA, 2001, 285: 2486-2497).

The term "alkyl," as used herein, refers to a saturated straight or branched hydrocarbon radical containing up to twenty four carbon atoms. Examples of alkyl groups include, but are not limited to, methyl, ethyl, propyl, butyl, isopropyl, n-hexyl, octyl, decyl, dodecyl and the like. Alkyl groups typically include from 1 to about 24 carbon atoms, more typically from 1 to about 12 carbon atoms (C₁-C₁₂ alkyl) with from 1 to about 6 carbon atoms being more preferred. The term "lower alkyl" as used herein includes from 1 to about 6 carbon atoms. Alkyl groups as used herein may optionally include one or more further substituent groups.

The term "alkenyl," as used herein, refers to a straight or branched hydrocarbon chain radical containing up to twenty four carbon atoms and having at least one carbon-carbon double bond. Examples of alkenyl groups include, but are not limited to, ethenyl, propenyl, butenyl, 1-methyl-2-buten-1-yl, dienes such as 1,3-butadiene and the like. Alkenyl groups typically include from 2 to about 24 carbon atoms, more typically from 2 to about 12 carbon atoms with from 2 to about 6 carbon atoms being more preferred. Alkenyl groups as used herein may optionally include one or more further substituent groups.

The term "alkynyl," as used herein, refers to a straight or branched hydrocarbon radical containing up to twenty four carbon atoms and having at least one carbon-carbon triple bond. Examples of alkynyl groups include, but are not limited to, ethynyl, 1-propynyl, 1-butynyl, and the like. Alkynyl groups typically include from 2 to about 24 carbon atoms, more typically from 2 to about 12 carbon atoms with from 2 to about 6 carbon atoms being more preferred. Alkynyl groups as used herein may optionally include one or more further substitutent groups.

The term "aminoalkyl" as used herein, refers to an amino substituted alkyl radical. This term is meant to include C₁-C₁₂ alkyl groups having an amino substituent at any position and wherein the alkyl group attaches the aminoalkyl group to the parent molecule. The alkyl and/or amino portions of the aminoalkyl group can be further substituted with substituent groups.

The term "aliphatic," as used herein, refers to a straight or branched hydrocarbon radical containing up to twenty four carbon atoms wherein the saturation between any two carbon atoms is a single, double or triple bond. An aliphatic group preferably contains from 1 to about 24 carbon atoms, more typically from 1 to about 12 carbon atoms with from 1 to about 6 carbon atoms being more preferred. The straight or branched chain of an aliphatic group may be interrupted with one or more heteroatoms that include nitrogen, oxygen, sulfur and phosphorus. Such aliphatic groups interrupted by heteroatoms include without limitation polyalkoxys, such as polyalkylene glycols, polyamines, and polyimines. Aliphatic groups as used herein may optionally include further substitutent groups.

The term "alicyclic" or "alicyclyl" refers to a cyclic ring system wherein the ring is aliphatic. The ring system can comprise one or more rings wherein at least one ring is aliphatic. Preferred alicyclics include rings having from about 5 to about 9 carbon atoms in the ring. Alicyclic as used herein may optionally include further substitutent groups.

The term "alkoxy," as used herein, refers to a radical formed between an alkyl group and an oxygen atom wherein the oxygen atom is used to attach the alkoxy group to a parent molecule. Examples of alkoxy groups include, but are not limited to, methoxy, ethoxy, propoxy, isopropoxy, n-butoxy, sec-butoxy, tert-butoxy, n-pentoxy, neopentoxy, n-hexoxy and the like. Alkoxy groups as used herein may optionally include further substitutent groups.

The terms "halo" and "halogen," as used herein, refer to an atom selected from fluorine, chlorine, bromine and iodine.

The terms "aryl" and "aromatic," as used herein, refer to a mono- or polycyclic carbocyclic ring system radicals having one or more aromatic rings. Examples of aryl groups include, but are not limited to, phenyl, naphthyl, tetrahydronaphthyl, indanyl, idenyl and the like. Preferred aryl ring systems have from about 5 to about 20 carbon atoms in one or more rings. Aryl groups as used herein may optionally include further substitutent groups.

The terms "aralkyl" and "arylalkyl," as used herein, refer to a radical formed between an alkyl group and an aryl group wherein the alkyl group is used to attach the aralkyl group to a parent molecule. Examples include, but are not limited to, benzyl, phenethyl and the like. Aralkyl groups as used herein may optionally include further substitutent groups attached to the alkyl, the aryl or both groups that form the radical group.

The term "heterocyclic radical" as used herein, refers to a radical mono-, or poly-cyclic ring system that includes at least one heteroatom and is unsaturated, partially saturated or fully saturated, thereby including heteroaryl groups. Heterocyclic is also meant to include fused ring systems wherein one or more of the fused rings contain at least one heteroatom and the other rings can contain one or more heteroatoms or optionally contain no heteroatoms. A heterocyclic group typically includes at least one atom selected from sulfur, nitrogen or oxygen. Examples of heterocyclic groups include, [1,3]dioxolane, pyrrolidinyl, pyrazolinyl, pyrazolidinyl, imidazolinyl, imidazolidinyl, piperidinyl, piperazinyl, oxazolidinyl, isoxazolidinyl, morpholinyl, thiazolidinyl, isothiazolidinyl, quinoxalinyl, pyridazinonyl, tetrahydrofuryl and the like. Heterocyclic groups as used herein may optionally include further substitutent groups.

The terms "heteroaryl," and "heteroaromatic," as used herein, refer to a radical comprising a mono- or poly-cyclic aromatic ring, ring system or fused ring system wherein at least one of the rings is aromatic and includes one or more heteroatom. Heteroaryl is also meant to include fused ring systems including systems where one or more of the fused rings contain no heteroatoms. Heteroaryl groups typically include one ring atom selected from sulfur, nitrogen or oxygen. Examples of heteroaryl groups include, but are not limited to, pyridinyl, pyrazinyl, pyrimidinyl, pyrrolyl, pyrazolyl, imidazolyl, thiazolyl, oxazolyl, isooxazolyl, thiadiazolyl, oxadiazolyl, thiophenyl, furanyl, quinolinyl, isoquinolinyl, benzimidazolyl, benzooxazolyl, quinoxalinyl, and the like. Heteroaryl radicals can be attached to a parent molecule directly or through a linking moiety such as an aliphatic group or hetero atom. Heteroaryl groups as used herein may optionally include further substitutent groups.

The term "heteroarylalkyl," as used herein, refers to a heteroaryl group as previously defined having an alky radical that can attach the heteroarylalkyl group to a parent molecule. Examples include, but are not limited to, pyridinylmethyl, pyrimidinylethyl, napthyridinylpropyl and the like. Heteroarylalkyl groups as used herein may optionally include further substitutent groups on one or both of the heteroaryl or alkyl portions.

The term "mono or poly cyclic structure" as used herein includes all ring systems that are single or polycyclic having rings that are fused or linked and is meant to be inclusive of single and mixed ring systems individually selected from aliphatic, alicyclic, aryl, heteroaryl, aralkyl, arylalkyl, heterocyclic, heteroaryl, heteroaromatic, heteroarylalkyl. Such mono and poly cyclic structures can contain rings that are uniform or have varying degrees of saturation including fully saturated, partially saturated or fully unsaturated. Each ring can comprise ring atoms selected from C, N, O and S to give rise to heterocyclic rings as well as rings comprising only C ring atoms which can be present in a mixed motif such as for example benzimidazole wherein one ring has only carbon ring atoms and the fused ring has two nitrogen atoms. The mono or poly cyclic structures can be further substituted with substituent groups such as for example phthalimide which has two =O groups attached to one of the rings. In another aspect, mono or poly cyclic structures can be attached to a parent molecule directly through a ring atom, through a substituent group or a bifunctional linking moiety.

The term "acyl," as used herein, refers to a radical formed by removal of a hydroxyl group from an organic acid an d has the general formula -C(O)-X where X is typically aliphatic, alicyclic or aromatic. Examples include aliphatic carbonyls, aromatic carbonyls, aliphatic sulfonyls, aromatic sulfinyls, aliphatic sulfinyls, aromatic phosphates, aliphatic phosphates and the like. Acyl groups as used herein may optionally include further substitutent groups.

The term "hydrocarbyl includes groups comprising C, O and H. Included are straight, branched and cyclic groups having any degree of saturation. Such hydrocarbyl groups can include one or more heteroatoms selected from N, O and S and can be further mono or poly substituted with one or more substituent groups.

The terms "substituent" and "substituent group," as used herein, include groups that are typically added to other groups or parent compounds to enhance desired properties or give desired effects. Substituent groups can be protected or unprotected and can be added to one available site or to many available sites in a parent compound. Substituent groups may also be further substituted with other substituent groups and may be attached directly or via a linking group such as an alkyl or hydrocarbyl group to a parent compound. Such groups include without limitation, halogen, hydroxyl, alkyl, alkenyl, alkynyl, acyl (-C(O)Rₐₐ), carboxyl (-C(O)O-Rₐₐ), aliphatic groups, alicyclic groups, alkoxy, substituted oxo (-O-Rₐₐ), aryl, aralkyl, heterocyclic, heteroaryl, heteroarylalkyl, amino (-NR_{bb}R_{cc}), imino(=NR_{bb}), amido (-C(O)NR_{bb}R_{cc} or -N(R_{bb})C(O)Rₐₐ), azido (-N₃), nitro (-NO₂), cyano (-CN), carbamido (-OC(O)NR_{bb}R_{cc} or -N(R_{bb})C(O)ORₐₐ), ureido (-N(R_{bb})C(O)NR_{bb}R_{cc}), thioureido (-N(R_{bb})C(S)NR_{bb}R_{cc}), guanidinyl (-N(Rbb)C(=NRbb)NRbbRcc), amidinyl (-C(=NRbb)NRbbRcc or -N(R_{bb})C(NR_{bb})Rₐₐ), thiol (-SRbb), sulfinyl (-S(O)R_{bb}), sulfonyl (-S(O)₂R_{bb}), sulfonamidyl (-S(O)₂NR_{bb}R_{cc} or -N(R_{bb})S(O)₂R_{bb}) and conjugate groups. Wherein each Rₐₐ, R_{bb} and R_{cc} is, independently, H, an optionally linked chemical functional group or a further substituent group with a preferred list including without limitation H, alkyl, alkenyl, alkynyl, aliphatic, alkoxy, acyl, aryl, aralkyl, heteroaryl, alicyclic, heterocyclic and heteroarylalkyl.

### B. Certain Oligomeric Compounds

In certain embodiments, it is desirable to chemically modify oligomeric compounds, compared to naturally occuring oligomers, such as DNA or RNA. Certain such modifications alter the activity of the oligomeric compound. Certain such chemical modifications can alter activity by, for example: increasing affinity of an antisense compound for its target nucleic acid, increasing its resistance to one or more nucleases, and/or altering the pharmacokinetics or tissue distribution of the oligomeric compound. In certain instances, the use of chemistries that increase the affinity of an oligomeric compound for its target can allow for the use of shorter oligomeric compounds.

### 1. Certain monomers

In certain embodiment, oligomeric compounds comprise one or more modified monomer. In certain such embodiments, oligomeric compounds comprise one or more high affinity monomer. In certain embodiments, such high-affinity monomer is selected from monomers (e.g., nucleosides and nucleotides) comprising 2'-modifed sugars, including, but not limited to: BNA's and monomers (e.g., nucleosides and nucleotides) with 2'- substituents such as allyl, amino, azido, thio, O-allyl, O-C₁-C₁₀ alkyl, -OCF3, O-(CH₂)₂-O-CH₃, 2'-O(CH₂)₂SCH₃, O-(CH₂)₂-O-N(Rₘ)(Rₙ), or O-CH₂-C(=O)-N(Rₘ)(Rₙ), where each Rₘ and Rₙ is, independently, H or substituted or unsubstituted C₁-C₁₀ alkyl.

In certain embodiments, the oligomeric compounds including, but not limited to short antisense compounds of the present disclosure, comprise one or more high affinity monomers provided that the oligomeric compound does not comprise a nucleotide comprising a 2'-O(CH₂)ₙH, wherein n is one to six.

In certain embodiments, the oligomeric compounds including, but not limited to short antisense compounds of the present disclosure, comprise one or more high affinity monomer provided that the oligomeric compound does not comprise a nucleotide comprising a 2'-OCH₃ or a 2'-O(CH₂)₂OCH₃.

In certain embodiments, the oligomeric compounds including, but not limited to short antisense compounds of the present disclosure, comprise one or more high affinity monomer provided that the oligomeric compound does not comprise a α-L-Methyleneoxy (4'-CH₂-O-2') BNA.

In certain embodiments, the oligomeric compounds including, but not limited to short antisense compounds of the present disclosure, comprise one or more high affinity monomer provided that the oligomeric compound does not comprise a β-D-Methyleneoxy (4'-CH₂-O-2') BNA.

In certain embodiments, the oligomeric compounds including, but not limited to short antisense compounds of the present disclosure, comprise one or more high affinity monomer provided that the oligomeric compound does not comprise a α-L-Methyleneoxy (4'-CH₂-O-2') BNA or a β-D-Methyleneoxy (4'-CH₂-O-2') BNA.

### a. Certain Nucleobases

The naturally occurring base portion of a nucleoside is typically a heterocyclic base. The two most common classes of such heterocyclic bases are the purines and the pyrimidines. For those nucleosides that include a pentofuranosyl sugar, a phosphate group can be linked to the 2', 3' or 5' hydroxyl moiety of the sugar. In forming oligonucleotides, those phosphate groups covalently link adjacent nucleosides to one another to form a linear polymeric compound. Within oligonucleotides, the phosphate groups are commonly referred to as forming the internucleotide backbone of the oligonucleotide. The naturally occurring linkage or backbone of RNA and of DNA is a 3' to 5' phosphodiester linkage.

In addition to "unmodified" or "natural" nucleobases such as the purine nucleobases adenine (A) and guanine (G), and the pyrimidine nucleobases thymine (T), cytosine (C) and uracil (U), many modified nucleobases or nucleobase mimetics known to those skilled in the art are amenable with the compounds described herein. In certain embodiments, a modified nucleobase is a nucleobase that is fairly similar in structure to the parent nucleobase, such as for example a 7-deaza purine, a 5-methyl cytosine, or a G-clamp. In certain embodiments, nucleobase mimetic include more complicated structures, such as for example a tricyclic phenoxazine nucleobase mimetic. Methods for preparation of the above noted modified nucleobases are well known to those skilled in the art.

### b. Certain sugars

Oligomeric compounds described herein may comprise one or more monomer, including a nucleoside or nucleotide, having a modified sugar moiety. For example, the furanosyl sugar ring of a nucleoside can be modified in a number of ways including, but not limited to, addition of a substituent group, bridging of two non-geminal ring atoms to form a bicyclic nucleic acid (BNA).

In certain embodiments, oligomeric compounds comprise one or more monomers that is a BNA. In certain such embodiments, BNA s include, but are not limited to, (A) α-L-Methyleneoxy (4'-CH₂-O-2') BNA, (B) β-D-Methyleneoxy (4'-CH₂-O-2') BNA, (C) Ethyleneoxy (4'-(CH₂)₂-O-2') BNA , (D) Aminooxy (4'-CH₂-O-N(R)-2') BNA and (E) Oxyamino (4'-CH₂-N(R)-O-2') BNA, as depicted in Figure 1.

In certain embodimnents, BNA compounds include, but are not limited to, compounds having at least one bridge between the 4' and the 2' position of the sugar wherein each of the bridges independently comprises 1 or from 2 to 4 linked groups independently selected from -[C(R₁)(R₂)]ₙ-, -C(R₁)=C(R₂)-, -C(R₁)=N-, -C(=NR₁)-, -C(=O)-, -C(=S)-, -O-, -Si(R₁)₂-, -S(=O)ₓ- and -N(R₁)-;
wherein:
x is 0, 1, or 2;
n is 1, 2, 3, or 4;
each R₁ and R₂ is, independently, H, a protecting group, hydroxyl, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, heterocycle radical, substituted heterocycle radical, heteroaryl, substituted heteroaryl, C₅-C₇ alicyclic radical, substituted C₅-C₇ alicyclic radical, halogen, OJ₁, NJ₁J₂, SJ₁, N₃, COOJ₁, acyl (C(=O)-H), substituted acyl, CN, sulfonyl (S(=O)₂-J₁), or sulfoxyl (S(=O)-J₁); and
each J₁ and J₂ is, independently, H, C₁-C₁₂ alkyl, substituted C₁-C₁₂ alkyl, C₂-C₁₂ alkenyl, substituted C₂-C₁₂ alkenyl, C₂-C₁₂ alkynyl, substituted C₂-C₁₂ alkynyl, C₅-C₂₀ aryl, substituted C₅-C₂₀ aryl, acyl (C(=O)-H), substituted acyl, a heterocycle radical, a substituted heterocycle radical, C₁-C₁₂ aminoalkyl, substituted C₁-C₁₂ aminoalkyl or a protecting group.

In one embodiment, each of the bridges of the BNA compounds is, independently, -[C(R₁)(R₂)]ₙ-, -[C(R₁)(R₂)]ₙ-O-, -C(R₁R₂)-N(R₁)-O- or -C(R₁R₂)-O-N(R₁)-. In another embodiment, each of said bridges is, independently, 4'-CH₂-2', 4'-(CH₂)₂-2', 4'-(CH₂)₃-2', 4'-CH₂-O-2', 4'-(CH₂)₂-O-2', 4'-CH₂-ON(R₁)-2' and 4'-CH₂-N(R₁)-O-2'- wherein each R₁ is, independently, H, a protecting group or C₁-C₁₂ alkyl.

Certain BNA's have been prepared and disclosed in the patent literature as well as in scientific literature (Singh et al., Chem. Commun., 1998, 4, 455-456; Koshkin et al., Tetrahedron, 1998, 54, 3607-3630; Wahlestedt et al., Proc. Natl. Acad. Sci. U. S. A., 2000, 97, 5633-5638; Kumar et al., Bioorg. Med. Chem. Lett., 1998, 8, 2219-2222; WO 94/14226; WO 2005/021570; Singh et al., J. Org. Chem., 1998, 63, 10035-10039; Examples of issued US patents and published applications that disclose BNA s include, for example, U.S. Patent Nos. 7,053,207; 6,268,490; 6,770,748; 6,794,499; 7,034,133; and 6,525,191; and U.S. Pre-Grant Publication Nos. 2004-0171570; 2004-0219565; 2004-0014959; 2003-0207841; 2004-0143114; and 20030082807.

Also described herein are BNAs in which the 2'-hydroxyl group of the ribosyl sugar ring is linked to the 4' carbon atom of the sugar ring thereby forming a methyleneoxy (4'-CH₂-O-2') linkage to form the bicyclic sugar moiety (reviewed in Elayadi et al., Curr. Opinion Invens. Drugs, 2001, 2, 558-561; Braasch et al., Chem. Biol., 2001, 8 1-7; and Orum et al., Curr. Opinion Mol. Ther., 2001, 3, 239-243; see also U.S. Patents: 6,268,490 and 6,670,461). The linkage can be a methylene (-CH₂-) group bridging the 2' oxygen atom and the 4' carbon atom, for which the term methyleneoxy (4'-CH₂-O-2') BNA is used for the bicyclic moiety; in the case of an ethylene group in this position, the term ethyleneoxy (4'-CH₂CH₂-O-2') BNA is used (Singh et al., Chem. Commun., 1998, 4, 455-456: Morita et al., Bioorganic Medicinal Chemistry, 2003, 11, 2211-2226). Methyleneoxy (4'-CH₂-O-2') BNA and other bicyclic sugar analogs display very high duplex thermal stabilities with complementary DNA and RNA (Tm = +3 to +10° C), stability towards 3'-exonucleolytic degradation and good solubility properties. Potent and nontoxic antisense oligonucleotides compriseing BNAs have been described (Wahlestedt et al., Proc. Natl. Acad. Sci. U. S. A., 2000, 97, 5633-5638).

An isomer of methyleneoxy (4'-CH₂-O-2') BNA that has also been discussed is alpha-L-methyleneoxy (4'-CH₂-O-2') BNA which has been shown to have superior stability against a 3'-exonuclease. The alpha-L- methyleneoxy (4'-CH₂-O-2') BNA's were incorporated into antisense gapmers and chimeras that showed potent antisense activity (Frieden et al., Nucleic Acids Research, 2003, 21, 6365-6372).

The synthesis and preparation of the methyleneoxy (4'-CH₂-O-2') BNA monomers adenine, cytosine, guanine, 5-methyl-cytosine, thymine and uracil, along with their oligomerization, and nucleic acid recognition properties have been described (Koshkin et al., Tetrahedron, 1998, 54, 3607-3630). BNAs and preparation thereof are also described in WO 98/39352 and WO 99/14226.

Analogs of methyleneoxy (4'-CH₂-O-2') BNA, phosphorothioate- methyleneoxy (4'-CH₂-O-2') BNA and 2'-thio-BNAs, have also been prepared (Kumar et al., Bioorg. Med. Chem. Lett., 1998, 8, 2219-2222). Preparation of locked nucleoside analogs compriseing oligodeoxyribonucleotide duplexes as substrates for nucleic acid polymerases has also been described (Wengel et al., WO 99/14226). Furthermore, synthesis of 2'-amino-BNA, a novel comformationally restricted high-affinity oligonucleotide analog has been described in the art (Singh et al., J. Org. Chem., 1998, 63, 10035-10039). In addition, 2'-Amino- and 2'-methylamino-BNA's have been prepared and the thermal stability of their duplexes with complementary RNA and DNA strands has been previously reported.

Modified sugar moieties are well known and can be used to alter, typically increase, the affinity of the antisense compound for its target and/or increase nuclease resistance. A representative list of preferred modified sugars includes but is not limited to bicyclic modified sugars (BNA's), including methyleneoxy (4'-CH₂-O-2') BNA and ethyleneoxy (4'-(CH₂)₂-O-2' bridge) BNA ; substituted sugars, especially 2'-substituted sugars having a 2'-F, 2'-OCH₃ or a 2'-O(CH₂)₂-OCH₃ substituent group; and 4'-thio modified sugars. Sugars can also be replaced with sugar mimetic groups among others. Methods for the preparations of modified sugars are well known to those skilled in the art. Some representative patents and publications that teach the preparation of such modified sugars include, but are not limited to, U.S. Patents: 4,981,957; 5,118,800; 5,319,080; 5,359,044; 5,393,878; 5,446,137; 5,466,786; 5,514,785; 5,519,134; 5,567,811; 5,576,427; 5,591,722; 5,597,909; 5,610,300; 5,627,053; 5,639,873; 5,646,265; 5,658,873; 5,670,633; 5,792,747; 5,700,920; 6,531,584; and 6,600,032; and WO 2005/121371.

In certain embodiments, BNA's include bicyclic nucleoside having the formula: 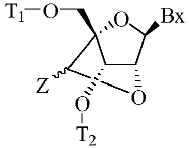 wherein:
Bx is a heterocyclic base moiety;
T₁ is H or a hydroxyl protecting group;
T₂ is H, a hydroxyl protecting group or a reactive phosphorus group;
Z is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, or substituted amide.

In one embodiment, each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂, NJ₃C(=X)NJ₁J₂ and CN, wherein each J₁, J₂ and J₃ is, independently, H or C₁-C₆ alkyl, and X is O, S or NJ₁.

In certain such embodiments, each of the substituted groups, is, independently, mono or poly substituted with substituent groups independently selected from halogen, oxo, hydroxyl, OJ₁, NJ₁J₂, SJ₁, N₃, OC(=X)J₁, and NJ₃C(=X)NJ₁J₂, wherein each J₁, J₂ and J₃ is, independently, H, C₁-C₆ alkyl, or substituted C₁-C₆ alkyl and X is O or NJ₁.

In certain embodiments, the Z group is C₁-C₆ alkyl substituted with one or more X^{x}, wherein each X^{x} is independently OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁. In another embodiment, the Z group is C₁-C₆ alkyl substituted with one or more X^{x}, wherein each X^{x} is independently halo (e.g., fluoro), hydroxyl, alkoxy (e.g., CH₃O-), substituted alkoxy or azido.

In certain embodiments, the Z group is -CH₂X^{x}, wherein X^{x} is OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁. In another embodiment, the Z group is -CH₂X^{x}, wherein X^{x} is halo (e.g., fluoro), hydroxyl, alkoxy (e.g., CH₃O-) or azido.

In certain such embodiments, the Z group is in the (*R*)-configuration:

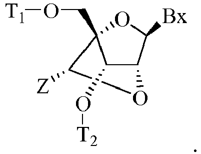

In certain such embodiments, the Z group is in the (*S*)-configuration:

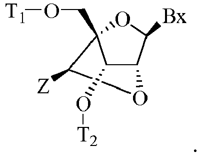

In certain embodiments, each T₁ and T₂ is a hydroxyl protecting group. A preferred list of hydroxyl protecting groups includes benzyl, benzoyl, 2,6-dichlorobenzyl, t-butyldimethylsilyl, t-butyldiphenylsilyl, mesylate, tosylate, dimethoxytrityl (DMT), 9-phenylxanthine-9-yl (Pixyl) and 9-(p-methoxyphenyl)xanthine-9-yl (MOX). In certain embodiments, T₁ is a hydroxyl protecting group selected from acetyl, benzyl, t-butyldimethylsilyl, t-butyldiphenylsilyl and dimethoxytrityl wherein a more preferred hydroxyl protecting group is T₁ is 4,4'-dimethoxytrityl.

In certain embodiments, T₂ is a reactive phosphorus group wherein preferred reactive phosphorus groups include diisopropylcyanoethoxy phosphoramidite and H-phosphonate. In certain embodiments T₁ is 4,4'-dimethoxytrityl and T₂ is diisopropylcyanoethoxy phosphoramidite.

In certain embodiments, oligomeric compounds have at least one monomer of the formula: 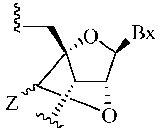 or of the formula: 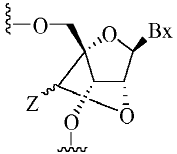 or of the formula: 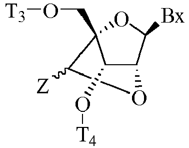 wherein
Bx is a heterocyclic base moiety;
T₃ is H, a hydroxyl protecting group, a linked conjugate group or an internucleoside linking group attached to a nucleoside, a nucleotide, an oligonucleoside, an oligonucleotide, a monomeric subunit or an oligomeric compound;
T₄ is H, a hydroxyl protecting group, a linked conjugate group or an internucleoside linking group attached to a nucleoside, a nucleotide, an oligonucleoside, an oligonucleotide, a monomeric subunit or an oligomeric compound;
wherein at least one of T₃ and T₄ is an internucleoside linking group attached to a nucleoside, a nucleotide, an oligonucleoside, an oligonucleotide, a monomeric subunit or an oligomeric compound; and
Z is C₁-C₆ alkyl, C₂-C₆ alkenyl, C₂-C₆ alkynyl, substituted C₁-C₆ alkyl, substituted C₂-C₆ alkenyl, substituted C₂-C₆ alkynyl, acyl, substituted acyl, or substituted amide.

In one embodiment, each of the substituted groups, is, independently, mono or poly substituted with optionally protected substituent groups independently selected from halogen, oxo, hydroxyl, OJ₁, NJ₁J₂, SJ₁, N₃, OC(=X)J₁, OC(=X)NJ₁J₂, NJ₃C(=X)NJ₁J₂ and CN, wherein each J₁, J₂ and J₃ is, independently, H or C₁-C₆ alkyl, and X is O, S or NJ₁.

In one embodiment, each of the substituted groups, is, independently, mono or poly substituted with substituent groups independently selected from halogen, oxo, hydroxyl, OJ₁, NJ₁J₂, SJ₁, N₃, OC(=X)J₁, and NJ₃C(=X)NJ₁J₂, wherein each J₁, J₂ and J₃ is, independently, H or C₁-C₆ alkyl, and X is O or NJ₁.

In certain such embodiments, at least one Z is C₁-C₆ alkyl or substituted C₁-C₆ alkyl. In certain embodiments, each Z is, independently, C₁-C₆ alkyl or substituted C₁-C₆ alkyl. In certain embodiments, at least one Z is C₁-C₆ alkyl. In certain embodiments, each Z is, independently, C₁-C₆ alkyl. In certain embodiments, at least one Z is methyl. In certain embodiments, each Z is methyl. In certain embodiments, at least one Z is ethyl. In certain embodiments, each Z is ethyl. In certain embodiments, at least one Z is substituted C₁-C₆ alkyl. In certain embodiments, each Z is, independently, substituted C₁-C₆ alkyl. In certain embodiments, at least one Z is substituted methyl. In certain embodiments, each Z is substituted methyl. In certain embodiments, at least one Z is substituted ethyl. In certain embodiments, each Z is substituted ethyl.

In certain embodiments, at least one substituent group is C₁-C₆ alkoxy (e.g., at least one Z is C₁-C₆ alkyl substituted with one or more C₁-C₆ alkoxy). In another embodiment, each substituent group is, independently, C₁-C₆ alkoxy (e.g., each Z is, independently, C₁-C₆ alkyl substituted with one or more C₁-C₆ alkoxy).

In certain embodiments, at least one C₁-C₆ alkoxy substituent group is CH₃O- (e.g., at least one Z is CH₃OCH₂-). In another embodiment, each C₁-C₆ alkoxy substituent group is CH₃O- (e.g., each Z is CH₃OCH₂-).

In certain embodiments, at least one substituent group is halogen (e.g., at least one Z is C₁-C₆ alkyl substituted with one or more halogen). In certain embodiments, each substituent group is, independently, halogen (e.g., each Z is, independently, C₁-C₆ alkyl substituted with one or more halogen). In certain embodiments, at least one halogen substituent group is fluoro (e.g., at least one Z is CH₂FCH₂-, CHF₂CH₂- or CF₃CH₂-). In certain embodiments, each halo substituent group is fluoro (e.g., each Z is, independently, CH₂FCH₂-, CHF₂CH₂- or CF₃CH₂-).

In certain embodiments, at least one substituent group is hydroxyl (e.g., at least one Z is C₁-C₆ alkyl substituted with one or more hydroxyl). In certain embodiments, each substituent group is, independently, hydroxyl (e.g., each Z is, independently, C₁-C₆ alkyl substituted with one or more hydroxyl). In certain embodiments, at least one Z is HOCH₂-. In another embodiment, each Z is HOCH₂-.

In certain embodiments, at least one Z is CH₃-, CH₃CH₂-, CH₂OCH₃-, CH₂F- or HOCH₂-. In certain embodiments, each Z is, independently, CH₃-, CH₃CH₂-, CH₂OCH₃-, CH₂F- or HOCH₂-.

In certain embodiments, at least one Z group is C₁-C₆ alkyl substituted with one or more X^{x}, wherein each X^{x} is, independently, OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁. In another embodiment, at least one Z group is C₁-C₆ alkyl substituted with one or more X^{x}, wherein each X^{x} is, independently, halo (e.g., fluoro), hydroxyl, alkoxy (e.g., CH₃O-) or azido.

In certain embodiments, each Z group is, independently, C₁-C₆ alkyl substituted with one or more X^{x}, wherein each X^{x} is independently OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁. In another embodiment, each Z group is, independently, C₁-C₆ alkyl substituted with one or more X^{x}, wherein each X^{x} is independently halo (e.g., fluoro), hydroxyl, alkoxy (e.g., CH₃O-) or azido.

In certain embodiments, at least one Z group is -CH₂X^{x}, wherein X^{x} is OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁ In certain embodiments, at least one Z group is -CH₂X^{x}, wherein X^{x} is halo (e.g., fluoro), hydroxyl, alkoxy (e.g., CH₃O-) or azido.

In certain embodiments, each Z group is, independently, -CH₂X^{x}, wherein each X^{x} is, independently, OJ₁, NJ₁J₂, SJ₁, N₃, OC(=X)J₁, OC(=X)NJ₁J₂, NJ₃C(=X)NJ₁J₂ or CN; wherein each J₁, J₂ and J₃ is, independently, H or C₁-C₆ alkyl, and X is O, S or NJ₁. In another embodiment, each Z group is, independently, -CH₂X^{x}, wherein each X^{x} is, independently, halo (e.g., fluoro), hydroxyl, alkoxy (e.g., CH₃O-) or azido.

In certain embodiments, at least one Z is CH₃-. In another embodiment, each Z is, CH₃-.

In certain embodiments, the Z group of at least one monomer is in the (R)- configuration represented by the formula: 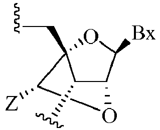 or the formula: 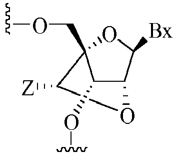 or the formula:

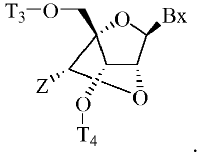

In certain embodiments, the Z group of each monomer of the formula is in the (R)- configuration.

In certain embodiments, the Z group of at least one monomer is in the (*S*)- configuration represented by the formula: 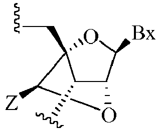 or the formula: 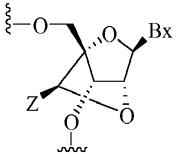 or the formula:

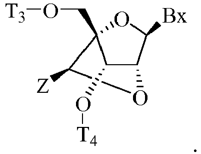

In certain embodiments, the Z group of each monomer of the formula is in the (*S*)- configuration.

In certain embodiments, T₃ is H or a hydroxyl protecting group. In certain embodiments, T₄ is H or a hydroxyl protecting group. In a further embodiment T₃ is an internucleoside linking group attached to a nucleoside, a nucleotide or a monomeric subunit. In certain embodiments, T₄ is an internucleoside linking group attached to a nucleoside, a nucleotide or a monomeric subunit. In certain embodiments, T₃ is an internucleoside linking group attached to an oligonucleoside or an oligonucleotide. In certain embodiments, T₄ is an internucleoside linking group attached to an oligonucleoside or an oligonucleotide. In certain embodiments, T₃ is an internucleoside linking group attached to an oligomeric compound. In certain embodiments, T₄ is an internucleoside linking group attached to an oligomeric compound. In In certain embodiments, at least one of T₃ and T₄ comprises an internucleoside linking group selected from phosphodiester or phosphorothioate.

In certain embodiments, oligomeric compounds have at least one region of at least two contiguous monomers of the formula: 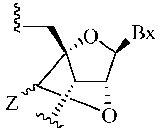 or of the formula: 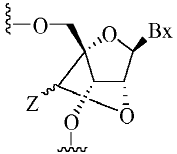 or of the formula: to

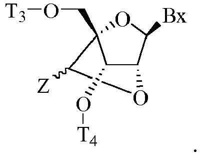

In certain embodiments, the oligomeric compound comprises at least two regions of at least two contiguous monomers of the above formula. In certain embodiments, the oligomeric compound comprises a gapped oligomeric compound. In certain embodiments, the oligmeric compound comprises at least one region of from about 8 to about 14 contiguous β-D-2'-deoxyribofuranosyl nucleosides. In certain embodiments, the oligomeric compound comprises at least one region of from about 9 to about 12 contiguous β-D-2'-deoxyribofuranosyl nucleosides.

In certain embodiments, monmers include sugar mimetics. In certain such embodiments, a mimetic is used in place of the sugar or sugar-internucleoside linkage combination, and the nucleobase is maintained for hybridization to a selected target. Representative examples of a sugar mimetics include, but are not limited to, cyclohexenyl or morpholino. Representative examples of a mimetic for a sugar-internucleoside linkage combination include, but are not limited to, peptide nucleic acids (PNA) and morpholino groups linked by uncharged achiral linkages. In some instances a mimetic is used in place of the nucleobase. Representative nucleobase mimetics are well known in the art and include, but are not limited to, tricyclic phenoxazine analogs and universal bases (Berger et al., Nuc Acid Res. 2000, 28:2911-14). Methods of synthesis of sugar, nucleoside and nucleobase mimetics are well known to those skilled in the art.

### 3. Monomeric Linkages

Described herein are linking groups that link monomers (including, but not limited to, modified and unmodified nucleosides and nucleotides) together, thereby forming an oligomeric compound. The two main classes of linking groups are defined by the presence or absence of a phosphorus atom. Representative phosphorus containing linkages include, but are not limited to, phosphodiesters (P=O), phosphotriesters, methylphosphonates, phosphoramidate, and phosphorothioates (P=S). Representative non-phosphorus containing linking groups include, but are not limited to, methylenemethylimino (-CH₂-N(CH₃)-O-CH₂-), thiodiester (-O-C(O)-S-), thionocarbamate (-O-C(O)(NH)-S-); siloxane (-O-Si(H)2-O-); and N,N'-dimethylhydrazine (-CH₂-N(CH₃)-N(CH₃)-). Oligomeric compounds having non-phosphorus linking groups are referred to as oligonucleosides. Modified linkages, compared to natural phosphodiester linkages, can be used to alter, typically increase, nuclease resistance of the oligomeric compound. In certain embodiments, linkages having a chiral atom can be prepared a racemic mixtures, as separate enantomers. Representative chiral linkages include, but are not limited to, alkylphosphonates and phosphorothioates. Methods of preparation of phosphorous-containing and non-phosphorous-containing linkages are well known to those skilled in the art.

The oligomeric compounds described herein contain one or more asymmetric centers and thus give rise to enantiomers, diastereomers, and other stereoisomeric configurations that may be defined, in terms of absolute stereochemistry, as (R) or (S), α or β such as for sugar anomers, or as (D) or (L) such as for amino acids et al. Included in the antisense compounds provided herein are all such possible isomers, as well as their racemic and optically pure forms.

### 4. Oligomeric Compounds

In certain embodiments, provided herein are oligomeric compounds having reactive phosphorus groups useful for forming linkages including for example phosphodiester and phosphorothioate internucleoside linkages. Methods of preparation and/or purification of precursors or oligomeric compounds are not a limitation of the compositions or methods disclosed herein. Methods for synthesis and purification of oligomeric compounds including DNA, RNA, oligonucleotides, oligonucleosides, and antisense compounds are well known to those skilled in the art.

Generally, oligomeric compounds comprise a plurality of monomeric subunits linked together by linking groups. Nonlimiting examples of oligomeric compounds include primers, probes, antisense compounds, antisense oligonucleotides, external guide sequence (EGS) oligonucleotides, alternate splicers, and siRNAs. As such, these compounds can be introduced in the form of single-stranded, double-stranded, circular, branched or hairpins and can contain structural elements such as internal or terminal bulges or loops. Oligomeric double-stranded compounds can be two strands hybridized to form double-stranded compounds or a single strand with sufficient self complementarity to allow for hybridization and formation of a fully or partially double-stranded compound.

In certain embodiments, disclosed herein are chimeric oligomeric compounds. In certain such embodiments, chimeric oligomeric compounds are chimeric oligonucleotides. In certain such embodiments, the chimeric oligonucleotides comprise differently modified nucleotides. In certain embodiments, chimeric oligonucleotides are mixed-backbone antisense oligonucleotides.

In general a chimeric oligomeric compound will have modified nucleosides that can be in isolated positions or grouped together in regions that will define a particular motif. Any combination of modifications and/or mimetic groups can comprise a chimeric oligomeric compound as described herein.

In certain embodiments, chimeric oligomeric compounds typically comprise at least one region modified so as to confer increased resistance to nuclease degradation, increased cellular uptake, and/or increased binding affinity for the target nucleic acid. In certain embodiments, an additional region of the oligomeric compound may serve as a substrate for enzymes capable of cleaving RNA:DNA or RNA:RNA hybrids. By way of example, RNase H is a cellular endonuclease that cleaves the RNA strand of an RNA:DNA duplex. Activation of RNase H, therefore, results in cleavage of the RNA target, thereby greatly enhancing the efficiency of inhibition of gene expression. Consequently, comparable results can often be obtained with shorter oligomeric compounds when chimeras are used, compared to for example phosphorothioate deoxyoligonucleotides hybridizing to the same target region. Cleavage of the RNA target can be routinely detected by gel electrophoresis and, if necessary, associated nucleic acid hybridization techniques known in the art.

In certain embodiments, chimeric oligomeric compounds are gapmers. In certain embodiments, chimeric compounds are short antisense compounds. In certain embodiments, short antisense compounds are gapmers. In certain such embodiments, a mixed-backbone antisense oligomer has one type of internucleotide linkages in one or both wings and a different type of internucleotide linkages in the gap. In certain such embodiments, the mixed-backbone antisense oligonucleotide has phosphodiester linkages in the wings and phosphorothioate linkages in the gap. In certain embodiments in which the internucleotide linkages in a wing is different from the internucleotide linkages in the gap, the internucleotide linkage bridging that wing and the gap is the same as the internucleotide linkage in the wing. In certain embodiments in which the internucleotide linkages in a wing is different from the internucleotide linkages in the gap, the internucleotide linkage bridging that wing and the gap is the same as the internucleotide linkage in the gap.

### C. Certain Short Antisense Compounds

Disclosed herein are short antisense compounds 8 to 16, preferably 9 to 15, more preferably 9 to 14, more preferably 10 to 14 nucleotides in length. In certain embodiments, short antisense compounds are 9 to 14 nucleotides in length. In certain embodiments, short antisense compounds are 10 to 14 nucleotides in length. In certain embodiments, such short antisense compounds are short antisense oligonucleotides.

In certain embodiments, short antisense compounds comprise one or more chemical modifications. In certain such embodiments, short antisense compounds comprise at least one modified nucleotide. In certain embodiments short antisense compounds comprise at least two or more modified nucleotides. In certain embodiments, short antisense compounds comprise at least one modified internucleotide linkage. In certain embodiments, short antisense compounds are mixed-backbone oligonucleotides. In certain embodiments, short antisense compounds are chimeric oligonucleotides. In certain embodiments, short antisense oligonucleotides are uniformly modified. In certain embodiments, short antisense oligonucleotides comprise modifications independently selected at each nucleobase and at each linkage.

In certain embodiments, short antisense compounds are short gapmers. In certain such embodiments, short gapmers comprise at least one high affinity modification in one or more wings of the compound. In certain embodiments, short antisense compounds comprise 1 to 3 high-affinity modifications in each wing. In certain embodiments, high affinity modifications of the short antisense compounds allow for a target affinity similar to, or even greater than, the target affinity of longer antisense compounds. In certain embodiments, the high-affinity modified nucleotides are sugar modified nucleotides. Such sugar modified nucleotides include those comprising a bridge between the 4' and 2' position of the sugar. Exemplary high affinity sugar modifications include, but are not limited to, BNA s and other 2'-modifications such as 2'-MOE. In an alternate embodiment of the disclosure, the high affinity modification is not a 2'-O-(CH₂)ₙH (n=1-6) sugar-modified nucleotide. In an additional alternate embodiment, the high affinity modified nucleotide is not a 2'-OCH₃ or a 2'-OCH₂CH₂OCH₃ nucleotide. In certain embodiments, the high-affinity modified nucleotides confer a ΔTₘ of at least 1, at least 1.5, at least 2, at least 2.5, at least 3.0, at least 3.5 or at least 4.0 degrees per nucleotide. Some high-affinity nucleotide modifications are known in the art to increase toxicity. As shown herein, short antisense compounds having a limited number (generally 2 to 6) of high affinity modifications exhibit little to no increase in toxicity but retain or increase affinity for the target RNA, while also significantly reducing expression of the RNA target. Short antisense compounds of the invention may optionally comprise a conjugate group, such as, for example, cholesterol or C₁₆.

### 1. Certain Wings

In certain embodiments, the short antisense compounds comprise a 5' wing and/or a 3' wing. In such embodiments, the features of the 3' wing and the features of the 5' wing are selected independently. Thus, in such embodiments, the number of monomers in the 5' wing and the number of monomers (length) in the 3' wing may be the same or may be different; the modifications, if any, in the 5' wing may be the same as the modifications, if any, in the 3' wing or such modifications, if any, may be different; and the monomeric linkages in the 5' wing and the monomeric linkages in the 3' wing may be the same or they may be different.

In certain embodiments a wing comprises one, two or three monomers (i.e. has a length of 1, 2, or 3). In certain embodiments, the monomers of a wing are modified. In certain such embodiments, the monomers of the wing are modified to increase affinity of the antisense compound for its target nucleic acid. In certain embodiments, the monomers of a wing are nucleosides or nucleotides. In certain such embodiments, the nucleosides or nucleotides of the wing comprise a 2' modification. In certain such embodiments, the monomers (nucleosides or nucleotides) of the wing are BNA's. In certain such embodiments, the monomers of the wing are selected from α-L-Methyleneoxy (4'-CH₂-O-2') BNA, β-D-Methyleneoxy (4'-CH₂-O-2') BNA , Ethyleneoxy (4'-(CH₂)₂-O-2') BNA , Aminooxy (4'-CH₂-O-N(R)-2') BNA and Oxyamino (4'-CH₂-N(R)-O-2') BNA. In certain embodiments, the monomers of a wing comprise a substituent at the 2' position selected from allyl, amino, azido, thio, O-allyl, O-C₁-C₁₀ alkyl, - OCF₃, O-(CH₂)₂-O-CH₃, 2'-O(CH₂)₂SCH₃, O-(CH₂)₂-O-N(Rₘ)(Rₙ), and O-CH₂-C(=O)-N(Rₘ)(Rₙ), where each Rₘ and Rₙ is, independently, H or substituted or unsubstituted C₁-C₁₀ alkyl. In certain embodiments, the monomers of a wing are 2'MOE nucleotides.

In certain embodiments, the monomeric linkages in a wing are naturally occurring internucleotide linkages. In certain embodiments, the monomeric linkages in a wing are non-naturally occurring internucleotide or internucleoside linkages. In certain such embodiments, the monomeric linkages in the wing are more resistant to one or more nucleases than naturally occurring internucleotide linkages. In certain such embodiments, the monomeric linkages in the wing are phosphorothioate linkages (P=S). In certain embodiments where a wing has more than one monomeric linkage, the monomeric linkages are the same as one another. In certain embodiments where a wing has more than one monomers linkage, the monomers linkages are different from each other.

One of ordinary skill in the art will recognize that the features and modifications discussed above may be used in any combination to prepare a wing. The table below provides non-limiting examples showing how one might prepare a wing by selecting a certain number of monomers, monomeric modifications (if any), and monomeric linkages both within the wing.

| Length | Monomer type/ modifications | monomeric linkages within wing |
|---|---|---|
| 1 | 2'MOE | None |
| 1 | BNA | None |
| 1 | Methyleneoxy BNA | None |
| 1 | ENA | None |
| 2 | 2'MOE | P=S |
| 2 | BNA | P=S |
| 2 | Methyleneoxy BNA | P=S |
| 2 | ENA | P=S |
| 2 | 2'MOE | P=O |
| 2 | BNA | P=O |
| 2 | Methyleneoxy BNA | P=O |
| 2 | ENA | P=O |
| 3 | 2'MOE | P=S |
| 3 | BNA | P=S |
| 3 | Methyleneoxy BNA | P=S |
| 3 | ENA | P=S |
| 3 | 2'MOE | P=O |
| 3 | BNA | P=O |
| 3 | Methyleneoxy BNA | P=O |
| 3 | ENA | P=O |

In certain embodiments in which a wing comprises two, three or four monomers, those two, three or four monomers all comprise the same modifications, if any. In certain embodiments in which a wing comprises two, three or four monomers, one or more of those two, three or four nucleobases comprises one or more modifications that is different from one or more of the modifications of one or more of the remaining monomers.

### 2. Certain Gaps

In certain embodiments, the short antisense compounds comprise a gap between the 5' wing and the 3' wing. In certain embodiments the gap comprises five, six, seven, eight, nine, ten, eleven, twelve, thirteen, or fourteen monomers. In certain embodiments, the monomers of the gap are unmodified deoxyribonucleotides. In certain embodiments, the monomers of the gap are unmodified ribonucleotides. In certain embodiments, gap modifications (if any) gap result in an antisense compound that, when bound to its target nucleic acid, supports cleavage by an RNase, including, but not limited to, RNase H.

In certain embodiments, the monomeric linkages in the gap are naturally occurring internucleotide linkages. In certain embodiments, the monomeric linkages in the gap are non-naturally occurring linkages. In certain such embodiments, the monomeric linkages in the gap are more resistant to one or more nuclease than naturally occurring internucleotide linkages. In certain such embodiments, the monomeric linkages in the gap are phosphorothioate linkages (P=S). In certain embodiments, the monomeric linkages in the gap are all the same as one another. In certain embodiments, the monomeric linkages within the gap are not all the same.

One of ordinary skill in the art will recognize that the features and modifications discussed above may be used in any combination to prepare a gap. The table below provides non-limiting examples showing how one might prepare a gap by selecting a certain number of monomers, monomeric modifications (if any), and monomeric linkages within the gap region.

| Length | Monomer type/ modifications | Monomeric linkages within gap |
|---|---|---|
| 5 | DNA | P=S |
| 6 | DNA | P=S |
| 7 | DNA | P=S |
| 8 | DNA | P=S |
| 9 | DNA | P=S |
| 10 | DNA | P=S |
| 11 | DNA | P=S |
| 12 | DNA | P=S |
| 13 | DNA | P=S |
| 14 | DNA | P=S |
| 6 | DNA | P=O |
| 7 | DNA | P=O |
| 8 | DNA | P=O |
| 9 | DNA | P=O |
| 10 | DNA | P=O |
| 11 | DNA | P=O |
| 12 | DNA | P=O |
| 8 | RNA | P=S |
| 9 | RNA | P=S |
| 10 | RNA | P=S |
| 11 | RNA | P=S |
| 12 | RNA | P=S |

### 3. Certain Gapped Antisense Oligomeric Compounds

One of ordinary skill in the art will recognize that the wings and the gaps discussed above may be selected and then combined in a variety of combinations to generate gapped oligomeric compounds, including, but not limited to, gapped antisense oligomeric compounds, and gapped antisense oligonucleotides. The features (length, modifications, linkages) of the 5' wing and the 3' wing may be selected independently of one another. The features of the gap include at least one difference in modification compared to the features of the 5' wing and at least one difference compared to the features of the 3' wing (i.e., there must be at least one difference in modification between neighboring regions to distinguish those neighboring regions from one another). The features of the gap may otherwise be selected independently.

In certain embodiments, the monomeric linkages within a wing and the monomeric linkages within the gap are the same. In certain embodiments, the monomeric linkages within a wing and the monomeric linkages within the gap are different. In certain such embodiments, the monomeric linkage bridging the wing and the gap are the same as the monomeric linkages in the wing. In certain embodiments, the monomeric linkage bridging the wing and the gap are the same as the monomeric linkages in the gap. In certain embodiments, short antisense compounds have uniform linkages throughout the compound. In certain such embodiments, all of the linkages are phosphorothioate (P=S) linkages.

One of ordinary skill in the art will recognize that the 3' wings, 5' wings, gaps, and linkages discussed above may be used in any combination to prepare a gapmer. The table below provides non-limiting examples showing how one might prepare a gapmer by selecting a certain 5' wing, a gap, a 3' wing and certain linkages bridging the gap and each wing.

| 5' Wing | | | 5'Bridge | Gap | | | 3' Bridge | 3' Wing | | |
|---|---|---|---|---|---|---|---|---|---|---|
| Length | Monomer | Link | Link | Length | Monomer | Link | Link | Length | Monomer | Link |
| 2 | MOE | P=S | P=S | 6 | DNA | P=S | P=S | 2 | MOE | P=S |
| 2 | BNA | P=S | P=O | 8 | DNA | P=O | P=S | 3 | BNA | P=S |
| 1 | MOE | None | P=S | 10 | DNA | P=S | P=S | 1 | MOE | P=S |
| 2 | MOE | P=S | P=S | 8 | RNA | P=S | P=S | 2 | MOE | P=S |
| 3 | Methyleneoxy BNA | P=S | P=S | 8 | RNA | P=S | P=S | 3 | MOE | P=S |
| 3 | DNA | P=O | P=O | 10 | RNA | P=S | P=O | 3 | 2'OH | P=O |
| 2 | 2-F | P=S | P=S | 5 | RNA | P=S | P=S | 2 | 2'-F | P=S |
| 1 | MOE | P=O | P=S | 5 | DNA | P=O | P=S | 4 | MOE | P=S |

In certain embodiments, the oligomeric compounds disclosed herein may comprise from about 8 to about 16, preferably 9 to 15, more preferably 9 to 14, more preferably 10 to 14 monomers (i.e. from about 8 to about 16 linked monomers). One of ordinary skill in the art will appreciate that this comprehends antisense compounds of 8, 9, 10, 11, 12, 13, 14, 15 or 16 nucleobases. In certain embodiments, oligomeric compounds are antisense compounds.

In certain embodiments, short antisense compounds are 8 nucleobases in length.

In certain embodiments, short antisense compounds are 9 nucleobases in length.

In certain embodiments, short antisense compounds are 10 nucleobases in length.

In certain embodiments, short antisense compounds are 11 nucleobases in length.

In certain embodiments, short antisense compounds are 12 nucleobases in length.

In certain embodiments, short antisense compounds are 13 nucleobases in length.

In certain embodiments, short antisense compounds are 14 nucleobases in length.

In certain embodiments, short antisense compounds are 15 nucleobases in length.

In certain embodiments, short antisense compounds are 16 nucleobases in length.

In certain embodiments, short antisense compounds are 8 monomers in length. In certain embodiments, short antisense compounds are 9 monomers in length. In certain embodiments, short antisense compounds are 10 monomers in length. In certain embodiments, short antisense compounds are 11 monomers in length. In certain embodiments, short antisense compounds are monomers in length. In certain embodiments, short antisense compounds are 13 monomers in length. In certain embodiments, short antisense compounds are 14 monomers in length. In certain embodiments, short antisense compounds are 15 monomers in length. In certain embodiments, short antisense compounds are 16 monomers in length. In certain embodiments, short antisense compounds comprise 9 to 15 monomers. In certain embodiments, short antisense compounds comprise 10 to 15 monomers. In certain embodiments, short antisense compounds comprise 12 to 14 monomers. In certain embodiments, short antisense compounds comprise 12 to 14 nucleotides or nucleosides.

One having skill in the art and informed by the short antisense compounds illustrated herein will be able, without undue experimentation, to identify further short antisense compounds.

In certain embodiments, short antisense compounds comprise a gap flanked by more than one wing on either or both sides. Thus, in certain embodiments, a short antisense compound comprises two or more 5' wings and two or more 3' wings. In certain embodiments, a short antisense compound comprises one 5' wing and two or more 3' wings. In certain embodiments, a short antisense compound comprises one 3' wing and two or more 5' wings. Certain such embodiments comprise, for example, the following regions: a first 5' wing - a bridge - a second 5' wing - a bridge - a gap - a bridge - a second 3' wing - a bridge - a first 3'wing. In such embodiments, each region has at least one difference in modification when compared to its neighboring region. Thus, in such embodiments, the second 5' wing and the second 3' wing each independently comprises one or more differences in modification compared to the gap and compared to the first 5' wing and the first 3' wing. In such embodiments, the modifications of the first 3' wing and first 5' wing may either or both be the same or different from the modifications of the gap, if any.

### 4. Certain Conjugate Groups

In one aspect, oligomeric compounds are modified by covalent attachment of one or more conjugate groups. In general, conjugate groups modify one or more properties of the attached oligomeric compound including but not limited to pharmacodynamic, pharmacokinetic, binding, absorption, cellular distribution, cellular uptake, charge and clearance. Conjugate groups are routinely used in the chemical arts and are linked directly or via an optional linking moiety or linking group to a parent compound such as an oligomeric compound. A preferred list of conjugate groups includes without limitation, intercalators, reporter molecules, polyamines, polyamides, polyethylene glycols, thioethers, polyethers, cholesterols, thiocholesterols, cholic acid moieties, folate, lipids, phospholipids, biotin, phenazine, phenanthridine, anthraquinone, adamantane, acridine, fluoresceins, rhodamines, coumarins and dyes.

Preferred conjugate groups amenable to the present invention include lipid moieties such as a cholesterol moiety (Letsinger et al., Proc. Natl. Acad. Sci. USA, 1989, 86, 6553); cholic acid (Manoharan et al., Bioorg. Med. Chem. Lett., 1994, 4, 1053); a thioether, e.g., hexyl-S-tritylthiol (Manoharan et al., Ann. N.Y. Acad. Sci., 1992, 660, 306; Manoharan et al., Bioorg. Med. Chem. Let., 1993, 3, 2765); a thiocholesterol (Oberhauser et al., Nucl. Acids Res., 1992, 20, 533); an aliphatic chain, e.g., dodecandiol or undecyl residues (Saison-Behmoaras et al., EMBO J., 1991, 10, 111; Kabanov et al., FEBS Lett., 1990, 259, 327; Svinarchuk et al., Biochimie, 1993, 75, 49); a phospholipid, e.g., di-hexadecyl-rac-glycerol or triethylammonium-1,2-di-O-hexadecyl-rac-glycero-3-H-phosphonate (Manoharan et al., Tetrahedron Lett., 1995, 36, 3651; Shea et al., Nucl. Acids Res., 1990, 18, 3777); a polyamine or a polyethylene glycol chain (Manoharan et al., Nucleosides & Nucleotides, 1995, 14, 969); adamantane acetic acid (Manoharan et al., Tetrahedron Lett., 1995, 36, 3651); a palmityl moiety (Mishra et al., Biochim. Biophys. Acta, 1995, 1264, 229); or an octadecylamine or hexylamino-carbonyl-oxycholesterol moiety (Crooke et al., J. Pharmacol. Exp. Ther., 1996, 277, 923).

Linking groups or bifunctional linking moieties such as those known in the art are amenable to the compounds provided herein. Linking groups are useful for attachment of chemical functional groups, conjugate groups, reporter groups and other groups to selective sites in a parent compound such as for example an oligomeric compound. In general a bifunctional linking moiety comprises a hydrocarbyl moiety having two functional groups. One of the functional groups is selected to bind to a parent molecule or compound of interest and the other is selected to bind essentially any selected group such as chemical functional group or a conjugate group. In some embodiments, the linker comprises a chain structure or an oligomer of repeating units such as ethylene glycol or amino acid units. Examples of functional groups that are routinely used in a bifunctional linking moiety include, but are not limited to, electrophiles for reacting with nucleophilic groups and nucleophiles for reacting with electrophilic groups. In some embodiments, bifunctional linking moieties include amino, hydroxyl, carboxylic acid, thiol, unsaturations (e.g., double or triple bonds), and the like. Some nonlimiting examples of bifunctional linking moieties include 8-amino-3,6-dioxaoctanoic acid (ADO), succinimidyl 4-(N-maleimidomethyl) cyclohexane-1-carboxylate (SMCC) and 6-aminohexanoic acid (AHEX or AHA). Other linking groups include, but are not limited to, substituted C₁-C₁₀ alkyl, substituted or unsubstituted C₂-C₁₀ alkenyl or substituted or unsubstituted C₂-C₁₀ alkynyl, wherein a nonlimiting list of preferred substituent groups includes hydroxyl, amino, alkoxy, carboxy, benzyl, phenyl, nitro, thiol, thioalkoxy, halogen, alkyl, aryl, alkenyl and alkynyl.

### 5. Synthesis, Purification and Analysis

Oligomerization of modified and unmodified nucleosides and nucleotides can be routinely performed according to literature procedures for DNA (Protocols for Oligonucleotides and Analogs, Ed. Agrawal (1993), Humana Press) and/or RNA (Scaringe, Methods (2001), 23, 206-217. Gait et al., Applications of Chemically synthesized RNA in RNA: Protein Interactions, Ed. Smith (1998), 1-36. Gallo et al., Tetrahedron (2001), 57, 5707-5713).

Oligomeric compounds provided herein can be conveniently and routinely made through the well-known technique of solid phase synthesis. Equipment for such synthesis is sold by several vendors including, for example, Applied Biosystems (Foster City, CA). Any other means for such synthesis known in the art may additionally or alternatively be employed. It is well known to use similar techniques to prepare oligonucleotides such as the phosphorothioates and alkylated derivatives. The invention is not limited by the method of antisense compound synthesis.

Methods of purification and analysis of oligomeric compounds are known to those skilled in the art. Analysis methods include capillary electrophoresis (CE) and electrospray-mass spectroscopy. Such synthesis and analysis methods can be performed in multi-well plates. The method of the disclosure is not limited by the method of oligomer purification.

### D. Antisense

Antisense mechanisms are all those involving the hybridization of a compound with target nucleic acid, wherein the outcome or effect of the hybridization is either target degradation or target occupancy with concomitant stalling of the cellular machinery involving, for example, transcription or splicing.

One type of antisense mechanism involving target degradation includes an RNase H. RNase H is a cellular endonuclease which cleaves the RNA strand of an RNA:DNA duplex. It is known in the art that single-stranded antisense compounds which are "DNA-like" elicit RNAse H activity in mammalian cells. Activation of RNase H, therefore, results in cleavage of the RNA target, thereby greatly enhancing the efficiency of DNA-like oligonucleotide-mediated inhibition of gene expression.

In certain embodiments, chemically-modified antisense compounds have a higher affinity for target RNAs than does non-modified DNA. In certain such embodiments, that higher affinity in turn provides increased potency allowing for the administration of lower doses of such compounds, reduced potential for toxicity and improvement in therapeutic index and decreased overall cost of therapy.

The present disclosure demonstrates that the incorporation of chemically-modified high-affinity nucleotides and nucleosides into antisense compounds allows for the design of short antisense compounds 8-16 nucleobases in length useful for the reduction of target RNAs and/or target proteins in cells, tissues, and animals, including, but not limited to, humans with increased potency and improved therapeutic index. Thus, in certain embodiments, disclosed herein are short antisense compounds comprising high-affinity nucleotide modifications useful for reducing a target RNA *in vivo.* Certain such short antisense compounds are effective at lower doses than previously described antisense compounds, allowing for a reduction in toxicity and cost of treatment. In addition, certain short antisense compounds have greater potential for oral dosing.

To address the need for more potent antisense compounds, disclosed herein are short antisense compounds (8-16, preferably 9 to 15, more preferably 9 to 14, more preferably 10 to 14 nucleotides in length) with increased activity *in vivo* relative to longer compounds. Certain short antisense compounds are gapmer compounds comprising high-affinity chemically-modified nucleotides on the 3' and 5' ends (wings) of the compound. In certain embodiments, the addition of high-affinity modified nucleotides allows antisense compounds to be active against, and specific for, their intended target RNA *in vivo* despite being shorter in length. Contemplated herein are short antisense compounds wherein each of the wings independently comprises 1 to 3 high-affinity modified nucleotides. In certain embodiments, the high-affinity modifications are sugar modifications. High-affinity modified nucleotides include, but are not limited to, BNA s or other 2'-modified nucleotides, such as 2'-MOE nucleotides. Also contemplated are short antisense compounds having at least one modified internucleotide linkage, such as a phosphorothioate internucleotide linkage. In certain embodiments, the short antisense compounds of the present invention can have all phosphorothioate internucleoside linkages. The short antisense compounds optionally comprise a conjugate group. As shown herein, short antisense compounds have greater affinity for target RNA than they have for DNA and are significantly more potent *in vivo* as shown by reduction of target mRNA as well as by amelioration of a variety of disease indications.

As used herein, an RNA which is involved in regulating glucose metabolism or clearance, lipid metabolism, cholesterol metabolism or insulin metabolism is any RNA involved in the biochemical pathways that regulate these processes. Such RNAs are well known in the art.

### 1. Modulation of Target Expression

In certain embodiments, a target is identified and antisense oligonucleotides are designed to modulate that target or its expression. In certain embodiments, designing an oligomeric compound to a target nucleic acid molecule can be a multistep process. Typically the process begins with the identification of a target protein, the activity of which is to be modulated, and then identifying the nucleic acid the expression of which yields the target protein. In certain embodiments, designing of an antisense compound results in an antisense compound that is hybridizable to the targeted nucleic acid molecule. In certain embodiments, the antisense compound is an antisense oligonucleotide or antisense oligonucleoside. In certain embodiments, an antisense compound and a target nucleic acid are complementary to one another. In certain such embodiments, an antisense compound is perfectly complementary to a target nucleic acid. In certain embodiments, an antisense compound includes one mismatch. In certain embodiments, an antisense compound includes two mismatches. In certain embodiments, an antisense compound includes three or more mismatches.

Modulation of expression of a target nucleic acid can be achieved through alteration of any number of nucleic acid functions. In certain embodiments, the functions of RNA to be modulated include, but are not limited to, translocation functions, which include, but are not limited to, translocation of the RNA to a site of protein translation, translocation of the RNA to sites within the cell which are distant from the site of RNA synthesis, and translation of protein from the RNA. RNA processing functions that can be modulated include, but are not limited to, splicing of the RNA to yield one or more RNA species, capping of the RNA, 3' maturation of the RNA and catalytic activity or complex formation involving the RNA which may be engaged in or facilitated by the RNA. Modulation of expression can result in the increased level of one or more nucleic acid species or the decreased level of one or more nucleic acid species, either temporally or by net steady state level. Thus, in one embodiment modulation of expression can mean increase or decrease in target RNA or protein levels. In another embodiment modulation of expression can mean an increase or decrease of one or more RNA splice products, or a change in the ratio of two or more splice products.

In certain embodiments, expression of a target gene is modulated using an oligomeric compound comprising from about 8 to about 16, preferably 9 to 15, more preferably 9 to 14, more preferably 10 to 14 monomers (i.e. from about 8 to about 16 linked monomers). One of ordinary skill in the art will appreciate that this comprehends methods of modulating expression of a target gene using one or more antisense compounds of 8, 9, 10, 11, 12, 13, 14, 15 or 16 nucleobases.

In certain embodiments, methods of modulating a target gene comprises use of a short antisense compound that is 8 nucleobases in length. In certain embodiments, methods of modulating a target gene comprises use of a short antisense compound that is 9 nucleobases in length. In certain embodiments, methods of modulating a target gene comprises use of a short antisense compound that is 8 nucleobases in length. In certain embodiments, methods of modulating a target gene comprises use of a short antisense compound that is 10 nucleobases in length. In certain embodiments, methods of modulating a target gene comprises use of a short antisense compound that is 10 nucleobases in length. In certain embodiments, methods of modulating a target gene comprises use of a short antisense compound that is 11 nucleobases in length. In certain embodiments, methods of modulating a target gene comprises use of a short antisense compound that is 12 nucleobases in length. In certain embodiments, methods of modulating a target gene comprises use of a short antisense compound that is 13 nucleobases in length. In certain embodiments, methods of modulating a target gene comprises use of a short antisense compound that is 14 nucleobases in length. In certain embodiments, methods of modulating a target gene comprises use of a short antisense compound that is 15 nucleobases in length. In certain embodiments, methods of modulating a target gene comprises use of a short antisense compound that is 16 nucleobases in length.

In certain embodiments, methods of modulating expression of a target gene comprises use of a short antisense compound comprising 9 to 15 monomers. In certain embodiments, methods of modulating expression of a target gene comprises use of a short antisense compound comprising 10 to 15 monomers. In certain embodiments, methods of modulating expression of a target gene comprises use of a short antisense compound comprising 12 to 14 monomers. In certain embodiments, methods of modulating expression of a target gene comprises use of a short antisense compound comprising 12 or 14 nucleotides or nucleosides.

### 2. Hybridization

In certain embodiments, antisense compounds specifically hybridize when there is a sufficient degree of complementarity to avoid non-specific binding of the antisense compound to non-target nucleic acid sequences under conditions in which specific binding is desired, i.e., under physiological conditions in the case of *in vivo* assays or therapeutic treatment, and under conditions in which assays are performed in the case of *in vitro* assays.

As used herein, "stringent hybridization conditions" or "stringent conditions" refers to conditions under which an antisense compound will hybridize to its target sequence, but to a minimal number of other sequences. Stringent conditions are sequence-dependent and will be different in different circumstances, and "stringent conditions" under which antisense compounds hybridize to a target sequence are determined by the nature and composition of the antisense compounds and the assays in which they are being investigated.

### 3. Complementarity

It is understood in the art that incorporation of nucleotide affinity modifications may allow for a greater number of mismatches compared to an unmodified compound. Similarly, certain oligonucleotide sequences may be more tolerant to mismatches than other oligonucleotide sequences. One of ordinary skill in the art is capable of determining an appropriate number of mismatches between oligonucleotides, or between an oligonucleotide and a target nucleic acid, such as by determining melting temperature (Tₘ). Tₘ or ΔTₘ can be calculated by techniques that are familiar to one of ordinary skill in the art. For example, techniques described in Freier et al. (Nucleic Acids Research, 1997, 25, 22: 4429-4443) allow one of ordinary skill in the art to evaluate nucleotide modifications for their ability to increase the melting temperature of an RNA:DNA duplex.

### 4. Identity

Antisense compounds, or a portion thereof, may have a defined percent identity to a SEQ ID NO, or a compound having a specific Isis number. As used herein, a sequence is identical to the sequence disclosed herein if it has the same nucleobase pairing ability. For example, an RNA which contains uracil in place of thymidine in the disclosed sequences of the compounds described herein would be considered identical as they both pair with adenine. This identity may be over the entire length of the oligomeric compound, or in a portion of the antisense compound (e.g., nucleobases 1-20 of a 27-mer may be compared to a 20-mer to determine percent identity of the oligomeric compound to the SEQ ID NO. It is understood by those skilled in the art that an antisense compound need not have an identical sequence to those described herein to function similarly to the antisense compound described herein. Shortened versions of antisense compounds taught herein, or non-identical versions of the antisense compounds taught herein, are also disclosed herein. Non-identical versions are those wherein each base does not have the same pairing activity as the antisense compounds disclosed herein. Bases do not have the same pairing activity by being shorter or having at least one abasic site. Alternatively, a non-identical version can include at least one base replaced with a different base with different pairing activity (e.g., G can be replaced by C, A, or T). Percent identity is calculated according to the number of bases that have identical base pairing corresponding to the SEQ ID NO or antisense compound to which it is being compared. The non-identical bases may be adjacent to each other, dispersed through out the oligonucleotide, or both.

For example, a 16-mer having the same sequence as nucleobases 2-17 of a 20-mer is 80% identical to the 20-mer. Alternatively, a 20-mer containing four nucleobases not identical to the 20-mer is also 80% identical to the 20-mer. A 14-mer having the same sequence as nucleobases 1-14 of an 18-mer is 78% identical to the 18-mer. Such calculations are well within the ability of those skilled in the art.

The percent identity is based on the percent of nucleobases in the original sequence present in a portion of the modified sequence. Therefore, a 30 nucleobase antisense compound comprising the full sequence of the complement of a 20 nucleobase active target segment would have a portion of 100% identity with the complement of the 20 nucleobase active target segment, while further comprising an additional 10 nucleobase portion. In the context of the instant description, the complement of an active target segment may constitute a single portion. In preferred embodiments, the oligonucleotides provided herein are at least 80%, at least 85%, at least 90%, at least 95%, at least 96%, at least 97%, at least 98%, at least 99% or 100% identical to at least a portion of the complement of the active target segments presented herein.

### E. Target Nucleic Acids, Regions and Segments

The target nucleic acid according to the invention is a nucleic acid molecule encoding ApoB. Nucleic acid molecules that encode ApoB include, without limitation, SEQ ID NO: 1 and SEQ ID NO: 2.

**Table 1: Certain Target Nucleic Acids of the disclosure**

| **Target** | **Species** | **GENBANK® Accession Number** | **SEQ ID NO** |
|---|---|---|---|
| ApoB | Human | NM_000384.1 | 1 |
| ApoB | Mouse | XM_137955.5 | 2 |
| SGLT2 | Human | NM_003041.1 | 3 |
| PCSK9 | Human | NM_174936.2 | 4 |
| SOD1 | Human | X02317.1 | 5 |
| CRP | Human | NM_000567.1 | 6 |
| GCCR | Mouse | BC031885.1 | 7 |
| GCCR | Human | Nucleotides 1 to 10600 of AC012634 | 8 |
| GCGR | Human | NM_000160.1 | 9 |
| DGAT2 | Human | NM_032564.2 | 10 |
| PTP1B | Human | NM_002827.2 | 11 |
| PTP1B | Human | Nucleotides 1417800 to 1425600 of NT_ 011362.9 | 12 |
| PTEN | Mouse | U92437.1 | 13 |
| PTEN | Human | NM_000314.4 | 14 |
| PTEN | Human | Nucleotides 8063255 to 8167140 of NT_ 033890.3 | 15 |

The targeting process usually includes determination of at least one target region, segment, or site within the target nucleic acid for the antisense interaction to occur such that the desired effect will result.

In certain embodiments, the 5'-most nucleotide of a target region is the 5' target site of a short antisense compound and the 3'-most nucleotide of a target region is the 3' target site of the same short antisense compound. In certain embodiments, the 5'-most nucleotide of a target region is the 5' target site of a short antisense compound and the 3'-most nucleotide of a target region is the 3' target site of a different short antisense compound. In certain embodiments, a target region comprises a nucleotide sequence within 10, 15, or 20 nucleotides of a 5' target site or a 3' target site.

In certain embodiments, a target region is a structurally defined region of the nucleic acid. For example, in certain such embodiments, a target region may encompass a 3' UTR, a 5' UTR, an exon, an intron, a coding region, a translation initiation region, translation termination region, or other defined nucleic acid region.

The locations on the target nucleic acid defined by having one or more active short antisense compounds targeted thereto are referred to as "active target segments." In certain embodiments, the target nucleic acid having one or more active short antisense compounds targeted thereto is a target RNA. When an active target segment is defined by multiple short antisense compounds, the compounds are preferably separated by no more than about 10 nucleotides on the target sequence, more preferably no more than about 5 nucleotides on the target sequence, even more preferably the short antisense compounds are contiguous, most preferably the short antisense compounds are overlapping. There may be substantial variation in activity (e.g., as defined by percent inhibition) of the short antisense compounds within an active target segment. Active short antisense compounds are those that modulate the expression of their target nucleic acid, including but not limited to a target RNA. Active short antisense compounds inhibit expression of their target RNA at least 10%, preferably 20%. In a preferred embodiment, at least about 50%, preferably about 70% of the short antisense compounds targeted to the active target segment modulate expression of their target RNA at least 40%. In a more preferred embodiment, the level of inhibition required to define an active short antisense compound is defined based on the results from the screen used to define the active target segments.

A suitable target segment is at least about an 8-nucleobase portion of a target region to which an active short antisense compound is targeted. Target segments can include DNA or RNA sequences that comprise at least the 8 consecutive nucleobases from the 5'-terminus of one of the illustrative target segments (the remaining nucleobases being a consecutive stretch of the same DNA or RNA beginning immediately upstream of the 5'-terminus of the target segment and continuing until the DNA or RNA comprises about 8 to about 16 nucleobases). Target segments are also represented by DNA or RNA sequences that comprise at least the 8 consecutive nucleobases from the 3'-terminus of one of the illustrative target segments (the remaining nucleobases being a consecutive stretch of the same DNA or RNA beginning immediately downstream of the 3'-terminus of the target segment and continuing until the DNA or RNA comprises about 8 to about 16 nucleobases). It is also understood that antisense target segments may be represented by DNA or RNA sequences that comprise at least 8 consecutive nucleobases from an internal portion of the sequence of an illustrative target segment, and may extend in either or both directions until the short antisense compound comprises about 8 to about 16 nucleobases. One having skill in the art armed with the target segments illustrated herein will be able, without undue experimentation, to identify further target segments.

Once one or more target regions, segments or sites have been identified, short antisense compounds are chosen which are sufficiently complementary to the target, i.e., hybridize sufficiently well and with sufficient specificity, to give the desired effect.

The short antisense compounds may also be targeted to regions of the target nucleobase sequence comprising any consecutive nucleobases 8 to 16 nucleobases in length along the target nucleic acid molecule.

Target segments 8-16 nucleobases in length comprising a stretch of at least eight (8) consecutive nucleobases selected from within the illustrative target segments are considered to be suitable for targeting as well. Thus, the short antisense compounds may also encompass 8-16 nucleobases within those segments identified herein as beginning at a particular 5' target site. Any segment of 8, 9, 10, 11, or more preferably 12, 13, 14, 15 or 16 contiguous nucleobases in a 50, preferably 25, more preferably 16 nucleobase perimeter around these regions are also considered to be suitable for targeting.

In a further embodiment, the "suitable target segments" identified herein may be employed in a screen for additional short antisense compounds that modulate the expression of a target nucleic acid. "Modulators" are those compounds that decrease or increase the expression of a target nucleic acid and which comprise at least an 8-nucleobase portion which is complementary to a target segment. The screening method comprises the steps of contacting a target segment of a nucleic acid with one or more candidate modulators, and selecting for one or more candidate modulators which decrease or increase the expression of a target nucleic acid. Once it is shown that the candidate modulator or modulators are capable of modulating (e.g. either decreasing or increasing) the expression of a target nucleic acid, the modulator may then be employed in further investigative studies of the function of the target, or for use as a research, diagnostic, or therapeutic agent in accordance with the present disclosure.

For all short antisense compounds discussed herein, sequence, monomer, monomeric modification, and monomeric linkage may each be selected independently. In certain embodiments, short antisense compounds are described by a motif. In such embodiments, any motif may be used with any sequence, whether or not the sequence and/or the motif is specifically disclosed herein. In certain embodiments, short antisense compounds comprise modifications that are not amenable to description by motif (for example, short antisense compounds comprising several different modifications and/or linkages at various positions throughout the compound). Such combinations may be incorporated for any sequence, whether or not it is disclosed herein. The sequence listing accompanying this filing provides certain nucleic acid sequences independent of chemical modification. Though that listing identifies each sequence as either "RNA" or "DNA" as required, in reality, those sequences may be modified with any combination of chemical modifications and/or motifs.

In certain embodiments, short antisense compounds comprise at least one high-affinity modified monomer. In certain embodiments, provided are short antisense compounds targeted to nucleic acid molecules encoding ApoB-100 (also known as APOB; Ag(x) antigen; apoB-48; apolipoprotein B; apolipoprotein B-100; apolipoprotein B-48).

### F. Certain Targets

In certain embodiments, short antisense compounds may be designed to modulate any target. In certain embodiments, the target is clinically relevant. In such embodiments, modulation of the target results in clinical benefit. Certain targets are preferentially expressed in the kidney. Certain targets are preferentially expressed in the liver. Certain targets are associated with a metabolic disorder. Certain targets are associated to a cardiovascular disorder. The target according to the invention is ApoB.

In certain embodiments, short antisense compounds exhibit liver and kidney-specific target RNA reduction *in vivo.* Such property renders those short antisense compounds particularly useful for inhibition of many target RNAs involved in metabolic and cardiovascular diseases. Thus, disclosed herein are methods of treating cardiovascular or metabolic disorders by contacting said kidney or liver tissues with short antisense compounds targeted to RNAs associated with said disorders. Thus, also disclosed are methods for ameliorating any of a variety of metabolic or cardiovascular disease indications with the short antisense compounds of the present disclosure.

### 1. ApoB

ApoB (also known as apolipoprotein B-100; ApoB-100, apolipoprotein B-48; ApoB-48 and Ag(x) antigen), is a large glycoprotein that serves an indispensable role in the assembly and secretion of lipids and in the transport and receptor-mediated uptake and delivery of distinct classes of lipoproteins. ApoB performs a variety of activities, from the absorption and processing of dietary lipids to the regulation of circulating lipoprotein levels (Davidson and Shelness, Annu. Rev. Nutr., 2000, 20, 169-193). This latter property underlies its relevance in terms of atherosclerosis susceptibility, which is highly correlated with the ambient concentration of ApoB-containing lipoproteins (Davidson and Shelness, Annu. Rev. Nutr., 2000, 20, 169-193). ApoB-100 is the major protein component of LDL-C and contains the domain required for interaction of this lipoprotein species with the LDL receptor. Elevated levels of LDL-C are a risk factor for cardiovascular disease, including atherosclerosis.

### Definitions

"ApoB" is the gene product or protein of which expression is to be modulated by administration of a short antisense compound.

"ApoB nucleic acid" means any nucleic acid encoding ApoB. For example, in certain embodiments, a ApoB nucleic acid includes, without limitation, a DNA sequence encoding ApoB, an RNA sequence transcribed from DNA encoding ApoB, and an mRNA sequence encoding ApoB.

"ApoB mRNA" means an mRNA encoding ApoB.

### ApoB Therapeutic Indications

In certain embodiments, the disclosed herein are methods of modulating the expression of ApoB in an individual comprising administering a short antisense compound targeted to an ApoB nucleic acid. In certain embodiments, disclosed herein are methods of treating an individual comprising administering one or more pharmaceutical compositions comprising a short antisense compound targeted to an ApoB nucleic acid. In certain embodiments, the individual has hypercholesterolemia, non-familial hypercholesterolemia, familial hypercholesterolemia, heterozygous familial hypercholesterolemia, homozygous familial hypercholesterolemia, mixed dyslipidemia, atherosclerosis, a risk of developing atherosclerosis, coronary heart disease, a history of coronary heart disease, early onset coronary heart disease, one or more risk factors for coronary heart disease, type II diabetes, type II diabetes with dyslipidemia, dyslipidemia, hypertriglyceridemia, hyperlipidemia, hyperfattyacidemia, hepatic steatosis, non-alcoholic steatohepatitis, or non-alcoholic fatty liver disease.

Guidelines for lipid-lowering therapy were established in 2001 by Adult Treatment Panel III (ATP III) of the National Cholesterol Education Program (NCEP), and updated in 2004 (Grundy et al., Circulation, 2004, 110, 227-239). The guidelines include obtaining a complete lipoprotein profile, typically after a 9 to 12 hour fast, for determination of LDL-C, total cholesterol, and HDL-C levels. According to the most recently established guidelines, LDL-C levels of 130-159 mg/dL, 160-189 mg/dL, and greater than or equal to 190 mg/dL are considered borderline high, high, and very high, respectively. Total cholesterol levels of 200-239 and greater than or equal to 240 mg/dL are considered borderline high and high, respectively. HDL-C levels of less than 40 mg/dL are considered low.

In certain embodiments, the individual has been identified as in need of lipid-lowering therapy. In certain such embodiments, the individual has been identified as in need of lipid-lowering therapy according to the guidelines established in 2001 by Adult Treatment Panel III (ATP III) of the National Cholesterol Education Program (NCEP), and updated in 2004 (Grundy et al., Circulation, 2004, 110, 227-239). In certain such embodiments, the individual in need of lipid-lowering therapy has LDL-C above 190 mg/dL. In certain such embodiments, the individual in need of lipid-lowering therapy has LDL-C above 160 mg/dL. In certain such embodiments, the individual in need of lipid-lowering therapy has LDL-C above 130 mg/dL. In certain such embodiments the individual in need of lipid-lowering therapy has LDL-C above 100 mg/dL. In certain such embodiments the individual in need of lipid-lowering therapy should maintain LDL-C below 160 mg/dL. In certain such embodiments the individual in need of lipid-lowering therapy should maintain LDL-C below 130 mg/dL. In certain such embodiments the individual in need of lipid-lowering therapy should maintain LDL-C below 100 mg/dL. In certain such embodiments the individual should maintain LDL-C below 70 mg/dL.

In certain embodiments disclosed herein are methods for reducing ApoB in an individual. In certain embodiments disclosed herein are methods for reducing ApoB-containing lipoprotein in an individual. In certain embodiments disclosed herein are methods for reducing LDL-C in an individual. In certain embodiments disclosed herein are methods for reducing VLDL-C in an individual. In certain embodiments disclosed herein are methods for reducing IDL-C in an individual. In certain embodiments disclosed herein are methods for reducing non-HDL-C in an individual. In certain embodiments disclosed herein are methods for reducing Lp(a) in an individual. In certain embodiments disclosed herein are methods for reducing serum triglyceride in an individual. In certain embodiments the disclosed herein are methods for reducing liver triglyceride in an individual. In certain embodiments disclosed herein are methods for reducing Ox-LDL-C in an individual. In certain embodiments disclosed herein are methods for reducing small LDL particles in an individual. In certain embodiments disclosed herein are methods for reducing small VLDL particles in an individual. In certain embodiments disclosed herein are methods for reducing phospholipids in an individual. In certain embodiments disclosed herein are methods for reducing oxidized phospholipids in an individual.

In certain embodiments disclosed herein are methods for reducing Ox-LDL-C concentration in a subject. In certain such embodiments, the reduction in ApoB, LDL-C, VLDL-C, IDL-C, total cholesterol, non-HDL-C, Lp(a), triglyerides, or Ox-LDL-C is, independently, selected from at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, and at least 100%. In certain such embodiments, the reduction in ApoB, LDL-C, VLDL-C, IDL-C, total cholesterol, non-HDL-C, Lp(a), triglyerides, or Ox-LDL-C is, independently, selected from at least 20%, at least 30%, at least 40%, at least 50%, at least 60%, and at least 70%. In certain such embodiments, the reduction in ApoB, LDL-C, VLDL-C, IDL-C, total cholesterol, non-HDL-C, Lp(a), triglyerides, or Ox-LDL-C is, independently, selected from at least 40%, at least 50%, at least 60%, and at least 70%.

In certain embodiments, disclosed herein are methods for raising HDL-C concentration in a subject.

In certain embodiments, the methods disclosed herein do not lower HDL-C. In certain embodiments, the methods disclosed herein do not result in accumulation of lipids in the liver. In certain embodiments, the methods disclosed herein do not cause hepatic steatosis.

In certain embodiments, disclosed herein are methods for lowering ApoB concentration in a subject while reducing side effects associated with treatment. In certain such embodiments, a side effect is liver toxicity. In certain such embodiments, a side effect is abnormal liver function. In certain such embodiments, a side effect is elevated alanine aminotransferase (ALT). In certain such embodiments, a side effect is elevated aspartate aminotransferase (AST).

In certain embodiments, disclosed herein are methods for lowering ApoB concentration in a subject who is not reaching target LDL-C levels as a result of lipid-lowering therapy. In certain such embodiments, a short antisense compound targeted to an ApoB nucleic acid is the only lipid-lowering agent administered to the subject. In certain such embodiments, the subject has not complied with recommended lipid-lowering therapy. In certain such embodiments, a pharmaceutical composition of the disclosure is co-administered with an additional different lipid-lowering therapy. In certain such embodiments, an additional lipid-lowering therapy is LDL-apheresis. In certain such embodiments, an additional lipid-lowering therapy is a statin. In certain such embodiments, an additional lipid-lowering therapy is ezetimibe.

In certain embodiments, disclosed herein are methods for lowering ApoB concentration in a statin-intolerant subject. In certain such embodiments, the subject has creatine kinase concentration increases as a result of statin administration. In certain such embodiments, the subject has liver function abnormalities as a result of statin administration. In certain such embodiments the subject has muscle aches as a result of statin administration. In certain such embodiments the subject has central nervous system side effects as a result of statin administration. In certain embodiments, the subject has not complied with recommended statin administration.

In certain embodiments, disclosed herein are methods for lowering liver triglycerides in a subject. In certain such embodiments, the subject has elevated liver triglycerides. In certain such embodiments, the subject has steatohepatitis. In certain such embodiments, the subject has steatosis. In certain such embodiments, liver triglyceride levels are measured by magnetic resonance imaging.

In certain embodiments, disclosed herein are methods for reducing coronary heart disease risk in a subject. In certain embodiments disclosed herein are methods for slowing the progression of atherosclerosis in a subject. In certain such embodiments disclosed herein are methods for stopping the progression of atherosclerosis in a subject. In certain such embodiments disclosed herein are methods for reducing the size and/or prevalence of atherosclerotic plaques in a subject. In certain embodiments the methods reduce a subject's risk of developing atherosclerosis.

In certain embodiments the methods improve the cardiovascular outcome in a subject. In certain such embodiments improved cardiovascular outcome is the reduction of the risk of developing coronary heart disease. In certain such embodiments, improved cardiovascular outcome is a reduction in the occurrence of one or more major cardiovascular events, which include, but are not limited to, death, myocardial infarction, reinfarction, stroke, cardiogenic shock, pulmonary edema, cardiac arrest, and atrial dysrhythmia. In certain such embodiments, the improved cardiovascular outcome is evidenced by improved carotid intimal media thickness. In certain such embodiments, improved carotid intimal media thickness is a decrease in thickness. In certain such embodiments, improved carotid intimal media thickness is a prevention an increase of intimal media thickness.

In certain embodiments a pharmaceutical composition comprising a short antisense compound targeted to an ApoB nucleic acid is for use in therapy. In certain embodiments, the therapy is the reduction of LDL-C, ApoB, VLDL-C, IDL-C, non-HDL-C, Lp(a), serum triglyceride, liver triglyceride, Ox-LDL-C, small LDL particles, small VLDL, phospholipids, or oxidized phospholipids in an individual. In certain embodiments, the therapy is the treatment of hypercholesterolemia, non-familial hypercholesterolemia, familial hypercholesterolemia, heterozygous familial hypercholesterolemia, homozygous familial hypercholesterolemia, mixed dyslipidemia, atherosclerosis, a risk of developing atherosclerosis, coronary heart disease, a history of coronary heart disease, early onset coronary heart disease, one or more risk factors for coronary heart disease, type II diabetes, type II diabetes with dyslipidemia, dyslipidemia, hypertriglyceridemia, hyperlipidemia, hyperfattyacidemia, hepatic steatosis, non-alcoholic steatohepatitis, or non-alcoholic fatty liver disease. In additional embodiments, the therapy is the reduction of CHD risk. In certain the therapy is prevention of atherosclerosis. In certain embodiments, the therapy is the prevention of coronary heart disease.

In certain embodiments a pharmaceutical composition comprising a short antisense compound targeted to an ApoB nucleic acid is used for the preparation of a medicament for reducing LDL-C, ApoB, VLDL-C, IDL-C, non-HDL-C, Lp(a), serum triglyceride, liver triglyceride, Ox-LDL-C, small LDL particles, small VLDL, phospholipids, or oxidized phospholipids in an individual. In certain embodiments pharmaceutical composition comprising a short antisense compound targeted to an ApoB nucleic acid is used for the preparation of a medicament for reducing coronary heart disease risk. In certain embodiments a short antisense compound targeted to an ApoB nucleic acid is used for the preparation of a medicament for the treatment of hypercholesterolemia, non-familial hypercholesterolemia, familial hypercholesterolemia, heterozygous familial hypercholesterolemia, homozygous familial hypercholesterolemia, mixed dyslipidemia, atherosclerosis, a risk of developing atherosclerosis, coronary heart disease, a history of coronary heart disease, early onset coronary heart disease, one or more risk factors for coronary heart disease, type II diabetes, type II diabetes with dyslipidemia, dyslipidemia, hypertriglyceridemia, hyperlipidemia, hyperfattyacidemia, hepatic steatosis, non-alcoholic steatohepatitis, or non-alcoholic fatty liver disease.

### ApoB Combination Therapies

In certain embodiments, one or more pharmaceutical compositions comprising a short antisense compound targeted to an ApoB nucleic acid are co-administered with one or more other pharmaceutical agents. In certain embodiments, such one or more other pharmaceutical agents are designed to treat the same disease or condition as the one or more pharmaceutical compositions of the present invention. In certain such embodiments, the one or more pharmaceutical agents are lipid-lowering agents. In certain embodiments, such one or more other pharmaceutical agents are designed to treat a different disease or condition as the one or more pharmaceutical compositions of the present invention. In certain embodiments, such one or more other pharmaceutical agents are designed to treat an undesired effect of one or more pharmaceutical compositions of the present invention. In certain embodiments, one or more pharmaceutical compositions of the present invention are co-administered with another pharmaceutical agent to treat an undesired effect of that other pharmaceutical agent. In certain embodiments, one or more pharmaceutical compositions of the present invention and one or more other pharmaceutical agents are administered at the same time. In certain embodiments, one or more pharmaceutical compositions of the present invention and one or more other pharmaceutical agents are administered at different times. In certain embodiments, one or more pharmaceutical compositions of the present invention and one or more other pharmaceutical agents are prepared together in a single formulation. In certain embodiments, one or more pharmaceutical compositions of the present invention and one or more other pharmaceutical agents are prepared separately.

In certain embodiments, pharmaceutical agents that may be co-administered with a pharmaceutical composition comprising a short antisense compound targeted to an ApoB nucleic acid include lipid-lowering agents. In certain such embodiments, pharmaceutical agents that may be co-administered with a pharmaceutical composition of the present invention include, but are not limited to atorvastatin, simvastatin, rosuvastatin, and ezetimibe. In certain such embodiments, the lipid-lowering agent is administered prior to administration of a pharmaceutical composition of the present invention. In certain such embodiments, the lipid-lowering agent is administered following administration of a pharmaceutical composition of the present invention. In certain such embodiments the lipid-lowering agent is administered at the same time as a pharmaceutical composition of the present invention. In certain such embodiments the dose of a co-administered lipid-lowering agent is the same as the dose that would be administered if the lipid-lowering agent was administered alone. In certain such embodiments the dose of a co-administered lipid-lowering agent is lower than the dose that would be administered if the lipid-lowering agent was administered alone. In certain such embodiments the dose of a co-administered lipid-lowering agent is greater than the dose that would be administered if the lipid-lowering agent was administered alone.

In certain embodiments, a co-administered lipid-lowering agent is a HMG-CoA reductase inhibitor. In certain such embodiments the HMG-CoA reductase inhibitor is a statin. In certain such embodiments the statin is selected from atorvastatin, simvastatin, pravastatin, fluvastatin, and rosuvastatin.

In certain embodiments, a co-administered lipid-lowering agent is a cholesterol absorption inhibitor. In certain such embodiments, cholesterol absorption inhibitor is ezetimibe.

In certain embodiments, a co-administered lipid-lowering agent is a co-formulated HMG-CoA reductase inhibitor and cholesterol absorption inhibitor. In certain such embodiments the co-formulated lipid-lowering agent is ezetimibe/simvastatin.

In certain embodiments, a co-administered lipid-lowering agent is a microsomal triglyceride transfer protein inhibitor (MTP inhibitor).

In certain embodiments, a co-administered pharmaceutical agent is a bile acid sequestrant. In certain such embodiments, the bile acid sequestrant is selected from cholestyramine, colestipol, and colesevelam.

In certain embodiments, a co-administered pharmaceutical agent is a nicotinic acid. In certain such embodiments, the nicotinic acid is selected from immediate release nicotinic acid, extended release nicotinic acid, and sustained release nicotinic acid.

In certain embodiments, a co-administered pharmaceutical agent is a fibric acid. In certain such embodiments, a fibric acid is selected from gemfibrozil, fenofibrate, clofibrate, bezafibrate, and ciprofibrate.

Further examples of pharmaceutical agents that may be co-administered with a pharmaceutical composition comprising a short antisense compound targeted to an ApoB nucleic acid include, but are not limited to, corticosteroids, including but not limited to prednisone; immunoglobulins, including, but not limited to intravenous immunoglobulin (IVIg); analgesics (e.g., acetaminophen); anti-inflammatory agents, including, but not limited to non-steroidal anti-inflammatory drugs (e.g., ibuprofen, COX-1 inhibitors, and COX-2, inhibitors); salicylates; antibiotics; antivirals; antifungal agents; antidiabetic agents (e.g., biguanides, glucosidase inhibitors, insulins, sulfonylureas, and thiazolidenediones); adrenergic modifiers; diuretics; hormones (e.g., anabolic steroids, androgen, estrogen, calcitonin, progestin, somatostan, and thyroid hormones); immunomodulators; muscle relaxants; antihistamines; osteoporosis agents (e.g., biphosphonates, calcitonin, and estrogens); prostaglandins, antineoplastic agents; psychotherapeutic agents; sedatives; poison oak or poison sumac products; antibodies; and vaccines.

In certain embodiments, a pharmaceutical composition comprising a short antisense compound targeted to an ApoB nucleic acid may be administered in conjunction with a lipid-lowering therapy. In certain such embodiments, a lipid-lowering therapy is therapeutic lifestyle change. In certain such embodiments, a lipid-lowering therapy is LDL apheresis.

In one embodiment, the antisense compounds provided herein can be used to lower the level of apolipoprotein B-containing lipoproteins in a human subject. As used herein, "apolipoprotein B-containing lipoprotein" refers to any lipoprotein that has apolipoprotein B as its protein component, and is understood to include LDL, VLDL, IDL, and lipoprotein(a). LDL, VLDL, IDL and lipoprotein(a) each contain one molecule of apolipoprotein B, thus a serum apolipoprotein B measurement reflects the total number of these lipoproteins. As is known in the art, each of the aforementioned lipoproteins is atherogenic. Thus, lowering one or more apolipoprotein B-containing lipoproteins in serum may provide a therapeutic benefit to a human subject. Small LDL particles are considered to be particularly atherogenic relative to large LDL particles, thus lowering small LDL particles can provide a therapeutic benefit to a human subject. Additional lipid parameters can also be determined in a subject. Reduction of total cholesterol:HDL ratio or LDL:HDL ratio is a clinically desirable improvement in cholesterol ratio. Similarly, it is clinically desirable to reduce serum triglycerides in humans who exhibit elevated lipid levels.

Other indications of cardiovascular disease that can be measured in a subject include serum LDL particle size; serum LDL cholesteryl ester concentration; serum LDL cholesteryl ester composition; the extent of polyunsaturation of serum LDL cholesteryl esters; and serum HDL cholesterol levels. As used herein, "serum LDL particle size" refers to the classification of serum LDL particle size, which may be very small, small, medium, or large, and is typically expressed in g/µmol. In the context of the present invention, "serum LDL cholesteryl ester concentration" means the amount of cholesteryl ester present in LDL particles, and is typically measured as mg/dL. In the context of the present invention, "serum LDL cholesteryl ester composition" is a measurement of the percentage of saturated, monounsaturated and polyunsaturated cholesteryl ester fatty acids present in serum LDL particles. "Polyunsaturation of serum LDL cholesteryl esters" means the percentage of polyunsaturated cholesteryl ester fatty acids in serum LDL particles.

Methods of obtaining serum or plasma samples for analysis and methods of preparation of the serum samples to allow for analysis are well known to those skilled in the art. With regard to measurements of lipoproteins, cholesterol, triglyceride and cholesteryl esters, the terms "serum" and "plasma" are herein used interchangeably.

In another embodiment, the antisense compounds provided herein can be used to treat metabolic disorders. A variety of biomarkers can be used for evaluating metabolic disease. For example, blood glucose levels can be determined by a physician or even by the patient using a commonly available test kit or glucometer (for example, the Ascensia ELITE™ kit, Ascensia (Bayer), Tarrytown NY, or Accucheck, Roche Diagnostics). Glycated hemoglobin (HbA_{1c}) can also be measured. HbA_{1c} is a stable minor hemoglobin variant formed *in vivo* via posttranslational modification by glucose, and it contains predominantly glycated NH₂-terminal β-chains. There is a strong correlation between levels of HbA_{1c} and the average blood glucose levels over the previous 3 months. Thus HbA_{1c} is often viewed as the "gold standard" for measuring sustained blood glucose control (Bunn, H.F. et al., 1978, Science. 200, 21-7). HbA_{1c} can be measured by ion-exchange HPLC or immunoassay; home blood collection and mailing kits for HbA_{1c} measurement are now widely available. Serum fructosamine is another measure of stable glucose control and can be measured by a colorimetric method (Cobas Integra, Roche Diagnostics).

### Certain Short Antisense Compounds Targeted to an ApoB Nucleic Acid

In certain embodiments, short antisense compounds are targeted to an ApoB nucleic acid having the sequence of GENBANK® Accession No. NM_000384.1, incorporated herein as SEQ ID NO: 1. In certain such embodiments, a short antisense compound targeted to SEQ ID NO: 1 is at least 90% complementary to SEQ ID NO: 1. In certain such embodiments, a short antisense compound targeted to SEQ ID NO: 1 is at least 95% complementary to SEQ ID NO: 1. In certain such embodiments, a short antisense compound targeted to SEQ ID NO: 1 is 100% complementary to SEQ ID NO: 1. In certain embodiments, a short antisense compound targeted to SEQ ID NO: 1 comprises a nucleotide sequence selected from the nucleotide sequences set forth in Table 2 and Table 3.

The nucleotide sequence set forth in each SEQ ID NO in Tables 2 and 3 is independent of any modification to a sugar moiety, a monomeric linkage, or a nucleobase. As such, short antisense compounds defined by a SEQ ID NO may comprise, independently, one or more modifications to a sugar moiety, an internucleoside linkage, or a nucleobase. Antisense compounds described by Isis Number (Isis NO.) indicate a combination of nucleobase sequence and one or more modifications to a sugar moiety, an internucleoside linkage, or a nucleobase.

Tables 2 and 3 illustrate examples of short antisense compounds targeted to SEQ ID NO: 1. Table 2 illustrates short antisense compounds that are 100% complementary to SEQ ID NO: 1. Table 3 illustrates short antisense compounds that have one or two mismatches with respect to SEQ ID NO: 1. The column labeled 'gapmer motif indicates the wing-gap-wing motif of each short antisense compounds. The gap segment comprises 2'-deoxynucleotides and each nucleotide of each wing segment comprises a 2'-modified sugar. The particular 2'-modified sugar is also indicated in the 'gapmer motif column. For example, '2-10-2 MOE' means a 2-10-2 gapmer motif, where a gap segment of ten 2'-deoxynucleotides is flanked by wing segments of two nucleotides, where the nucleotides of the wing segments are 2'-MOE nucleotides. Internucleoside linkages are phosphorothioate. The short antisense compounds comprise 5-methylcytidine in place of unmodified cytosine, unless "unmodified cytosine" is listed in the gapmer motif column, in which case the indicated cytosines are unmodified cytosines. For example, "5-mC in gap only" indicates that the gap segment has 5-methylcytosines, while the wing segments have unmodified cytosines.

**Table 2: Short Antisense Compounds targeted to SEQ ID NO: 1**

| **ISIS No** | **5' Target Site** | **3' Target Site** | **Sequence (5'-3')** | **Gapmer Motif** | **SEQ ID NO** |
|---|---|---|---|---|---|
| 372816 | 263 | 278 | CCGGAGGTGCTTGAAT | 3-10-3 MOE | 16 |
| 372894 | 264 | 277 | CGGAGGTGCTTGAA | 2-10-2 MOE | 17 |
| 372817 | 428 | 443 | GAAGCCATACACCTCT | 3-10-3 MOE | 18 |
| 372895 | 429 | 442 | AAGCCATACACCTC | 2-10-2 MOE | 19 |
| 372818 | 431 | 446 | GTTGAAGCCATACACC | 3-10-3 MOE | 20 |
| 372896 | 432 | 445 | TTGAAGCCATACAC | 2-10-2 MOE | 21 |
| 372819 | 438 | 453 | CCTCAGGGTTGAAGCC | 3-10-3 MOE | 22 |
| 372897 | 439 | 452 | CTCAGGGTTGAAGC | 2-10-2 MOE | 23 |
| 372820 | 443 | 458 | TTTGCCCTCAGGGTTG | 3-10-3 MOE | 24 |
| 372898 | 444 | 457 | TTGCCCTCAGGGTT | 2-10-2 MOE | 25 |
| 372821 | 468 | 483 | AGTTCTTGGTTTTCTT | 3-10-3 MOE | 26 |
| 372899 | 469 | 482 | GTTCTTGGTTTTCT | 2-10-2 MOE | 27 |
| 372822 | 587 | 602 | CCTCTTGATGTTCAGG | 3-10-3 MOE | 28 |
| 372900 | 588 | 601 | CTCTTGATGTTCAG | 2-10-2 MOE | 29 |
| 372823 | 592 | 607 | ATGCCCCTCTTGATGT | 3-10-3 MOE | 30 |
| 372901 | 593 | 606 | TGCCCCTCTTGATG | 2-10-2 MOE | 31 |
| 346583 | 715 | 728 | TGCCACATTGCCCT | 3-8-3 MOE | 32 |
| 346584 | 716 | 729 | TTGCCACATTGCCC | 3-8-3 MOE | 33 |
| 346585 | 717 | 730 | GTTGCCACATTGCC | 3-8-3 MOE | 34 |
| 346586 | 718 | 731 | TGTTGCCACATTGC | 3-8-3 MOE | 35 |
| 346587 | 719 | 732 | CTGTTGCCACATTG | 3-8-3 MOE | 36 |
| 346588 | 720 | 733 | TCTGTTGCCACATT | 3-8-3 MOE | 37 |
| 346589 | 721 | 734 | TTCTGTTGCCACAT | 3-8-3 MOE | 38 |
| 346590 | 722 | 735 | TTTCTGTTGCCACA | 3-8-3 MOE | 39 |
| 346591 | 723 | 736 | ATTTCTGTTGCCAC | 3-8-3 MOE | 40 |
| 372824 | 929 | 944 | GTAGGAGAAAGGCAGG | 3-10-3 MOE | 41 |
| 372902 | 930 | 943 | TAGGAGAAAGGCAG | 2-10-2 MOE | 42 |
| 372825 | 1256 | 1271 | GGCTTGTAAAGTGATG | 3-10-3 MOE | 43 |
| 372903 | 1257 | 1270 | GCTTGTAAAGTGAT | 2-10-2 MOE | 44 |
| 372826 | 1304 | 1319 | CCACTGGAGGATGTGA | 3-10-3 MOE | 45 |
| 372904 | 1305 | 1318 | CACTGGAGGATGTG | 2-10-2 MOE | 46 |
| 372829 | 2135 | 2150 | TTTCAGCATGCTTTCT | 3-10-3 MOE | 47 |
| 372907 | 2136 | 2149 | TTCAGCATGCTTTC | 2-10-2 MOE | 48 |
| 372832 | 2774 | 2789 | CATATTTGTCACAAAC | 3-10-3 MOE | 49 |
| 372910 | 2775 | 2788 | ATATTTGTCACAAA | 2-10-2 MOE | 50 |
| 372833 | 2779 | 2794 | ATGCCCATATTTGTCA | 3-10-3 MOE | 51 |
| 372911 | 2780 | 2793 | TGCCCATATTTGTC | 2-10-2 MOE | 52 |
| 372835 | 2961 | 2976 | TTTTGGTGGTAGAGAC | 3-10-3 MOE | 53 |
| 372913 | 2962 | 2975 | TTTGGTGGTAGAGA | 2-10-2 MOE | 54 |
| 346592 | 3248 | 3261 | TCTGCTTCGCACCT | 3-8-3 MOE | 55 |
| 346593 | 3249 | 3262 | GTCTGCTTCGCACC | 3-8-3 MOE | 56 |
| 346594 | 3250 | 3263 | AGTCTGCTTCGCAC | 3-8-3 MOE | 57 |
| 346595 | 3251 | 3264 | CAGTCTGCTTCGCA | 3-8-3 MOE | 58 |
| 346596 | 3252 | 3265 | TCAGTCTGCTTCGC | 3-8-3 MOE | 59 |
| 346597 | 3253 | 3266 | CTCAGTCTGCTTCG | 3-8-3 MOE | 60 |
| 346598 | 3254 | 3267 | CCTCAGTCTGCTTC | 3-8-3 MOE | 61 |
| 346599 | 3255 | 3268 | GCCTCAGTCTGCTT | 3-8-3 MOE | 62 |
| 346600 | 3256 | 3269 | AGCCTCAGTCTGCT | 3-8-3 MOE | 63 |
| 372836 | 3350 | 3365 | AACTCTGAGGATTGTT | 3-10-3 MOE | 64 |
| 372914 | 3351 | 3364 | ACTCTGAGGATTGT | 2-10-2 MOE | 65 |
| 372837 | 3355 | 3370 | TCATTAACTCTGAGGA | 3-10-3 MOE | 66 |
| 372915 | 3356 | 3369 | CATTAACTCTGAGG | 2-10-2 MOE | 67 |
| 372838 | 3360 | 3375 | ATTCATCATTAACTCT | 3-10-3 MOE | 68 |
| 372916 | 3361 | 3374 | TTCATCATTAACTC | 2-10-2 MOE | 69 |
| 372839 | 3409 | 3424 | TTGTTCTGAATGTCCA | 3-10-3 MOE | 70 |
| 387461 | 3409 | 3424 | TTGTTCTGAATGTCCA | 3-10-3 Methyleneoxy BNA Unmodified cytosines in gap | 70 |
| 380147 | 3409 | 3424 | TTGTTCTGAATGTCCA | 3-10-3 Methyleneoxy BNA | 70 |
| 372917 | 3410 | 3423 | TGTTCTGAATGTCC | 2-10-2 MOE | 73 |
| 372840 | 3573 | 3588 | CAGATGAGTCCATTTG | 3-10-3 MOE | 74 |
| 372918 | 3574 | 3587 | AGATGAGTCCATTT | 2-10-2 MOE | 75 |
| 372841 | 3701 | 3716 | ATCCACAGGGAAATTG | 3-10-3 MOE | 76 |
| 372919 | 3702 | 3715 | TCCACAGGGAAATT | 2-10-2 MOE | 77 |
| 372843 | 4219 | 4234 | CAGTTGTACAAGTTGC | 3-10-3 MOE | 78 |
| 372921 | 4220 | 4233 | AGTTGTACAAGTTG | 2-10-2 MOE | 79 |
| 372844 | 4301 | 4316 | CACAGAGTCAGCCTTC | 3-10-3 MOE | 80 |
| 372922 | 4302 | 4315 | ACAGAGTCAGCCTT | 2-10-2 MOE | 81 |
| 372845 | 4308 | 4323 | GGTCAACCACAGAGTC | 3-10-3 MOE | 82 |
| 372923 | 4309 | 4322 | GTCAACCACAGAGT | 2-10-2 MOE | 83 |
| 346601 | 5588 | 5601 | CAGCCACATGCAGC | 3-8-3 MOE | 84 |
| 346602 | 5589 | 5602 | CCAGCCACATGCAG | 3-8-3 MOE | 85 |
| 346603 | 5590 | 5603 | ACCAGCCACATGCA | 3-8-3 MOE | 86 |
| 346604 | 5591 | 5604 | TACCAGCCACATGC | 3-8-3 MOE | 87 |
| 346605 | 5592 | 5605 | TTACCAGCCACATG | 3-8-3 MOE | 88 |
| 346606 | 5593 | 5606 | GTTACCAGCCACAT | 3-8-3 MOE | 89 |
| 346607 | 5594 | 5607 | GGTTACCAGCCACA | 3-8-3 MOE | 90 |
| 346608 | 5595 | 5608 | AGGTTACCAGCCAC | 3-8-3 MOE | 91 |
| 346609 | 5596 | 5609 | TAGGTTACCAGCCA | 3-8-3 MOE | 92 |
| 372851 | 5924 | 5939 | AGGTTCTGCTTTCAAC | 3-10-3 MOE | 93 |
| 372929 | 5925 | 5938 | GGTTCTGCTTTCAA | 2-10-2 MOE | 94 |
| 372854 | 6664 | 6679 | TACTGATCAAATTGTA | 3-10-3 MOE | 95 |
| 372932 | 6665 | 6678 | ACTGATCAAATTGT | 2-10-2 MOE | 96 |
| 372855 | 6908 | 6923 | TTTTTCTTGTATCTGG | 3-10-3 MOE | 97 |
| 372933 | 6909 | 6922 | TTTTCTTGTATCTG | 2-10-2 MOE | 98 |
| 372856 | 7190 | 7205 | ATCCATTAAAACCTGG | 3-10-3 MOE | 99 |
| 372934 | 7191 | 7204 | TCCATTAAAACCTG | 2-10-2 MOE | 100 |
| 372858 | 7817 | 7832 | ATATTGCTCTGCAAAG | 3-10-3 MOE | 101 |
| 372936 | 7818 | 7831 | TATTGCTCTGCAAA | 2-10-2 MOE | 102 |
| 346610 | 7818 | 7831 | TATTGCTCTGCAAA | 3-8-3 MOE | 102 |
| 346611 | 7819 | 7832 | ATATTGCTCTGCAA | 3-8-3 MOE | 104 |
| 346612 | 7820 | 7833 | AATATTGCTCTGCA | 3-8-3 MOE | 105 |
| 346613 | 7821 | 7834 | GAATATTGCTCTGC | 3-8-3 MOE | 106 |
| 346614 | 7822 | 7835 | AGAATATTGCTCTG | 3-8-3 MOE | 107 |
| 346615 | 7823 | 7836 | TAGAATATTGCTCT | 3-8-3 MOE | 108 |
| 346616 | 7824 | 7837 | ATAGAATATTGCTC | 3-8-3 MOE | 109 |
| 346617 | 7825 | 7838 | GATAGAATATTGCT | 3-8-3 MOE | 110 |
| 346618 | 7826 | 7839 | GGATAGAATATTGC | 3-8-3 MOE | 111 |
| 372859 | 7995 | 8010 | ATGGAATCCTCAAATC | 3-10-3 MOE | 112 |
| 372937 | 7996 | 8009 | TGGAATCCTCAAAT | 2-10-2 MOE | 113 |
| 372861 | 8336 | 8351 | GAATTCTGGTATGTGA | 3-10-3 MOE | 114 |
| 372939 | 8337 | 8350 | AATTCTGGTATGTG | 2-10-2 MOE | 115 |
| 372862 | 8341 | 8356 | AGCTGGAATTCTGGTA | 3-10-3 MOE | 116 |
| 372940 | 8342 | 8355 | GCTGGAATTCTGGT | 2-10-2 MOE | 117 |
| 372863 | 8539 | 8554 | TGAAAATCAAAATTGA | 3-10-3 MOE | 118 |
| 372941 | 8540 | 8553 | GAAAATCAAAATTG | 2-10-2 MOE | 119 |
| 372871 | 9344 | 9359 | AAACAGTGCATAGTTA | 3-10-3 MOE | 120 |
| 372949 | 9345 | 9358 | AACAGTGCATAGTT | 2-10-2 MOE | 121 |
| 372872 | 9515 | 9530 | TTCAGGAATTGTTAAA | 3-10-3 MOE | 122 |
| 372950 | 9516 | 9529 | TCAGGAATTGTTAA | 2-10-2 MOE | 123 |
| 372875 | 9794 | 9809 | TTTTGTTTCATTATAG | 3-10-3 MOE | 124 |
| 372953 | 9795 | 9808 | TTTGTTTCATTATA | 2-10-2 MOE | 125 |
| 372877 | 10157 | 10172 | GATGACACTTGATTTA | 3-10-3 MOE | 126 |
| 372955 | 10158 | 10171 | ATGACACTTGATTT | 2-10-2 MOE | 127 |
| 372878 | 10161 | 10176 | GTGTGATGACACTTGA | 3-10-3 MOE | 128 |
| 372956 | 10162 | 10175 | TGTGATGACACTTG | 2-10-2 MOE | 129 |
| 372879 | 10167 | 10182 | TATTCAGTGTGATGAC | 3-10-3 MOE | 130 |
| 372957 | 10168 | 10181 | ATTCAGTGTGATGA | 2-10-2 MOE | 131 |
| 372880 | 10172 | 10187 | ATTGGTATTCAGTGTG | 3-10-3 MOE | 132 |
| 372958 | 10173 | 10186 | TTGGTATTCAGTGT | 2-10-2 MOE | 133 |
| 346619 | 10838 | 10851 | CCTCTAGCTGTAAG | 3-8-3 MOE | 134 |
| 346620 | 10839 | 10852 | CCCTCTAGCTGTAA | 3-8-3 MOE | 135 |
| 346621 | 10840 | 10853 | GCCCTCTAGCTGTA | 3-8-3 MOE | 136 |
| 346622 | 10841 | 10854 | GGCCCTCTAGCTGT | 3-8-3 MOE | 137 |
| 346623 | 10842 | 10855 | AGGCCCTCTAGCTG | 3-8-3 MOE | 138 |
| 346624 | 10843 | 10856 | GAGGCCCTCTAGCT | 3-8-3 MOE | 139 |
| 346625 | 10844 | 10857 | AGAGGCCCTCTAGC | 3-8-3 MOE | 140 |
| 346626 | 10845 | 10858 | AAGAGGCCCTCTAG | 3-8-3 MOE | 141 |
| 346627 | 10846 | 10859 | AAAGAGGCCCTCTA | 3-8-3 MOE | 142 |
| 372890 | 13689 | 13704 | GAATGGACAGGTCAAT | 3-10-3 MOE | 143 |
| 372968 | 13690 | 13703 | AATGGACAGGTCAA | 2-10-2 MOE | 144 |
| 372891 | 13694 | 13709 | GTTTTGAATGGACAGG | 3-10-3 MOE | 145 |
| 372969 | 13695 | 13708 | TTTTGAATGGACAG | 2-10-2 MOE | 146 |
| 372892 | 13699 | 13714 | TGGTAGTTTTGAATGG | 3-10-3 MOE | 147 |
| 372970 | 13700 | 13713 | GGTAGTTTTGAATG | 2-10-2 MOE | 148 |
| 346628 | 13907 | 13920 | TCACTGTATGGTTT | 3-8-3 MOE | 149 |
| 346629 | 13908 | 13921 | CTCACTGTATGGTT | 3-8-3 MOE | 150 |
| 346630 | 13909 | 13922 | GCTCACTGTATGGT | 3-8-3 MOE | 151 |
| 346631 | 13910 | 13923 | GGCTCACTGTATGG | 3-8-3 MOE | 152 |
| 346632 | 13911 | 13924 | TGGCTCACTGTATG | 3-8-3 MOE | 153 |
| 346633 | 13912 | 13925 | CTGGCTCACTGTAT | 3-8-3 MOE | 154 |
| 346634 | 13913 | 13926 | GCTGGCTCACTGTA | 3-8-3 MOE | 155 |
| 346635 | 13914 | 13927 | GGCTGGCTCACTGT | 3-8-3 MOE | 156 |
| 346636 | 13915 | 13928 | AGGCTGGCTCACTG | 3-8-3 MOE | 157 |
| 346637 | 13963 | 13976 | CAGGTCCAGTTCAT | 3-8-3 MOE | 158 |
| 346638 | 13964 | 13977 | GCAGGTCCAGTTCA | 3-8-3 MOE | 159 |
| 346639 | 13965 | 13978 | TGCAGGTCCAGTTC | 3-8-3 MOE | 160 |
| 346640 | 13966 | 13979 | GTGCAGGTCCAGTT | 3-8-3 MOE | 161 |
| 346641 | 13967 | 13980 | GGTGCAGGTCCAGT | 3-8-3 MOE | 162 |
| 346642 | 13968 | 13981 | TGGTGCAGGTCCAG | 3-8-3 MOE | 163 |
| 346643 | 13969 | 13982 | TTGGTGCAGGTCCA | 3-8-3 MOE | 164 |
| 346644 | 13970 | 13983 | TTTGGTGCAGGTCC | 3-8-3 MOE | 165 |
| 346645 | 13971 | 13984 | CTTTGGTGCAGGTC | 3-8-3 MOE | 166 |
| 346646 | 14051 | 14064 | TAACTCAGATCCTG | 3-8-3 MOE | 167 |
| 346647 | 14052 | 14065 | ATAACTCAGATCCT | 3-8-3 MOE | 168 |
| 346648 | 14053 | 14066 | AATAACTCAGATCC | 3-8-3 MOE | 169 |
| 346649 | 14054 | 14067 | AAATAACTCAGATC | 3-8-3 MOE | 170 |
| 346650 | 14055 | 14068 | AAAATAACTCAGAT | 3-8-3 MOE | 171 |
| 346651 | 14056 | 14069 | CAAAATAACTCAGA | 3-8-3 MOE | 172 |
| 346652 | 14057 | 14070 | GCAAAATAACTCAG | 3-8-3 MOE | 173 |
| 346653 | 14058 | 14071 | AGCAAAATAACTCA | 3-8-3 MOE | 174 |
| 346654 | 14059 | 14072 | TAGCAAAATAACTC | 3-8-3 MOE | 175 |

**Table 3: Short antisense compounds targeted to SEQ ID NO: 1 and having 1 or 2 mismatches**

| **Isis NO.** | **5' Target** | **3' Target** | **Sequence (5'-3')** | **Gapmer Motif** | **SEQ ID** |
|---|---|---|---|---|---|
| | **Site** | **Site** | | | **NO** |
| 372894 | 771 | 784 | CGGAGGTGCTTGAA | 2-10-2 MOE | 17 |
| 372905 | 1111 | 1124 | CAGGGCCTGGAGAG | 2-10-2 MOE | 176 |
| 346628 | 1493 | 1506 | TCACTGTATGGTTT | 3-8-3 MOE | 149 |
| 372828 | 2006 | 2021 | TCTGAAGTCCATGATC | 3-10-3 MOE | 177 |
| 372906 | 2007 | 2020 | CTGAAGTCCATGAT | 2-10-2 MOE | 178 |
| 372830 | 2382 | 2397 | TGGGCATGATTCCATT | 3-10-3 MOE | 179 |
| 372908 | 2383 | 2396 | GGGCATGATTCCAT | 2-10-2 MOE | 180 |
| 346616 | 3162 | 3175 | ATAGAATATTGCTC | 3-8-3 MOE | 109 |
| 346617 | 3163 | 3176 | GATAGAATATTGCT | 3-8-3 MOE | 110 |
| 372929 | 3513 | 3526 | GGTTCTGCTTTCAA | 2-10-2 MOE | 94 |
| 372946 | 3800 | 3813 | TGGAGCCCACGTGC | 2-10-2 MOE | 181 |
| 372904 | 4040 | 4053 | CACTGGAGGATGTG | 2-10-2 MOE | 46 |
| 372842 | 4084 | 4099 | TTGAAGTTGAGGGCTG | 3-10-3 MOE | 182 |
| 372920 | 4085 | 4098 | TGAAGTTGAGGGCT | 2-10-2 MOE | 183 |
| 346586 | 4778 | 4791 | TGTTGCCACATTGC | 3-8-3 MOE | 35 |
| 372847 | 5030 | 5045 | ACCAGTATTAATTTTG | 3-10-3 MOE | 184 |
| 372925 | 5031 | 5044 | CCAGTATTAATTTT | 2-10-2 MOE | 185 |
| 372848 | 5192 | 5207 | GTGTTCTTTGAAGCGG | 3-10-3 MOE | 186 |
| 372926 | 5193 | 5206 | TGTTCTTTGAAGCG | 2-10-2 MOE | 187 |
| 372953 | 5625 | 5638 | TTTGTTTCATTATA | 2-10-2 MOE | 125 |
| 372935 | 7585 | 7598 | AGTTACTTTGGTGT | 2-10-2 MOE | 188 |
| 372860 | 8255 | 8270 | TGGTACATGGAAGTCT | 3-10-3 MOE | 189 |
| 372938 | 8256 | 8269 | GGTACATGGAAGTC | 2-10-2 MOE | 190 |
| 391260 | 8256 | 8269 | GGTACATGGAAGTC | 2-10-2 MOE | 190 |
| 392068 | 8256 | 8269 | GGTACATGGAAGTC | 2-10-2 MOE | 190 |
| 387462 | 8256 | 8269 | GGTACATGGAAGTC | 2-10-2 Methyleneoxy BNA | 190 |
| 391872 | 8256 | 8269 | GGTACATGGAAGTC | 1-1-10-2 2'-(butylacetomido)-palmitamide Methyleneoxy BNA/Methyleneoxy BNA Unmodified cytosines in gap | 190 |
| 380148 | 8256 | 8269 | GGTACATGGAAGTC | 2-10-2 Methyleneoxy BNA | 190 |
| 391871 | 8256 | 8269 | GGTACATGGAAGTC | 1-1-10-2 2'-(butylacetomido)-palmitamide/MOE/MOE Unmodified cytosines in gap | 190 |
| 391755 | 8256 | 8269 | GGTACATGGAAGTC | 2-10-2 ENA mC in wing only | 190 |
| 398296 | 8256 | 8269 | GGTACATGGAAGTC | 2-10-2 (6'S)-6'-methyl-Methyleneoxy BNA Unmodified Cytosines | 190 |
| 372942 | 8455 | 8468 | TCCATGCCATATGT | 2-10-2 MOE | 200 |
| 372865 | 8888 | 8903 | CCCTGAAGAAGTCCAT | 3-10-3 MOE | 201 |
| 372943 | 8889 | 8902 | CCTGAAGAAGTCCA | 2-10-2 MOE | 202 |
| 372866 | 8908 | 8923 | GCCCAGTTCCATGACC | 3-10-3 MOE | 203 |
| 372944 | 8909 | 8922 | CCCAGTTCCATGAC | 2-10-2 MOE | 204 |
| 372867 | 9058 | 9073 | TTGAGGAAGCCAGATT | 3-10-3 MOE | 205 |
| 372945 | 9059 | 9072 | TGAGGAAGCCAGAT | 2-10-2 MOE | 206 |
| 372870 | 9261 | 9276 | TGGATGCAGTAATCTC | 3-10-3 MOE | 207 |
| 372948 | 9262 | 9275 | GGATGCAGTAATCT | 2-10-2 MOE | 208 |
| 372881 | 10185 | 10200 | TATAAAGTCCAGCATT | 3-10-3 MOE | 209 |
| 372959 | 10186 | 10199 | ATAAAGTCCAGCAT | 2-10-2 MOE | 210 |
| 372882 | 10445 | 10460 | AAGTTCCTGCTTGAAG | 3-10-3 MOE | 211 |
| 372960 | 10446 | 10459 | AGTTCCTGCTTGAA | 2-10-2 MOE | 212 |
| 372964 | 11451 | 11464 | AATGGTGAAGTACT | 2-10-2 MOE | 213 |
| 346612 | 13459 | 13472 | AATATTGCTCTGCA | 3-8-3 MOE | 105 |
| 346613 | 13460 | 13473 | GAATATTGCTCTGC | 3-8-3 MOE | 106 |

In certain embodiments, a target region is nucleotides 263-278 of SEQ ID NO: 1. In certain such embodiments, short antisense compounds targeted to nucleotides 263-278 of SEQ ID NO: 1 comprise a nucleotide sequence selected from SEQ ID NO: 16 or 17. In certain such embodiments, a short antisense compound targeted to nucleotides 263-278 of SEQ ID NO: 1 is selected from Isis NO. 372816 or 372894.

In certain embodiments, a target region is nucleotides 428-483 of SEQ ID NO: 1. In certain such embodiments, a short antisense compound targeted to nucleotides 428-483 of SEQ ID NO: 1 comprises a nucleotide sequence selected from SEQ ID NO 18, 19, 20, 21, 22, 23, 24, 25, 26, or 27. In certain such embodiments, a short antisense compound targeted to nucleotides 428-483 of SEQ ID NO: 1 is selected from Isis NO. 372817, 372895, 372818, 372896, 372819, 372897, 372820, 372898, 372821, or 372899.

In certain embodiments, a target region is nucleotides 428-458 of SEQ ID NO: 1. In certain such embodiments, a short antisense compound targeted to nucleotides 428-458 of SEQ ID NO: 1 comprises a nucleotide sequence selected from SEQ ID NO 18, 19, 20, 21, 22, 23, 24, or 25. In certain such embodiments, a short antisense compound targeted to nucleotides 428-458 of SEQ ID NO: 1 is selected from Isis NO. 372817, 372895, 372818, 372896, 372819, 372897, 372820, or 372898.

In certain embodiments, a target region is nucleotides 468-483 of SEQ ID NO: 1. In certain such embodiments, a short antisense compound targeted to nucleotides 468-483 of SEQ ID NO: 1 comprises a nucleotide sequence selected from SEQ ID NO 26 or 27. In certain such embodiments, a short antisense compound targeted to nucleotides 468-483 of SEQ ID NO: 1 is selected from Isis NO. 372821 or 372899.

In certain embodiments, a target region is nucleotides 587-607 of SEQ ID NO: 1. In certain such embodiments, a short antisense compound targeted to nucleotides 587-607 of SEQ ID NO: 1 comprises a nucleotide sequence selected from SEQ ID NO 28, 29, 30, or 31. In certain such embodiments, a short antisense compound targeted to nucleotides 587-607 of SEQ ID NO: 1 is selected from ISIS NO. 372822, 372900, 372823, or 372901.

In certain embodiments, a target region is nucleotides 715-736 of SEQ ID NO: 1. In certain such embodiments, a short antisense compound targeted to nucleotides 715-736 of SEQ ID NO: 1 comprises a nucleotide sequence selected from SEQ ID NO 32, 33, 34, 35, 36, 37, 38, 39, or 40. In certain such embodiments, a short antisense compound targeted to nucleotides 715-736 of SEQ ID NO: 1 is selected from Isis NO. 346583, 346584, 346585, 346586, 346587, 346588, 346589, 346590, or 346591.

In certain embodiments, a target region is nucleotides 929-944 of SEQ ID NO: 1. In certain such embodiments, a short antisense compound targeted to nucleotides 929-944 of SEQ ID NO: 1 comprises a nucleotide sequence selected from SEQ ID NO 41 or 42. In certain such embodiments, a short antisense compound targeted to nucleotides 929-944 of SEQ ID NO: 1 is selected from Isis NO. 372824 or 372902.

In certain embodiments, a target region is nucleotides 1256-1319 of SEQ ID NO: 1. In certain such embodiments, a short antisense compound targeted to nucleotides 1256-1319 of SEQ ID NO: 1 comprises a nucleotide sequence selected from SEQ ID NO 43, 44, 45, or 46. In certain such embodiments, a short antisense compound targeted to nucleotides 1256-1319 of SEQ ID NO: 1 is selected from Isis NO. 372825, 372903, 372826, or 372904.

In certain embodiments, a target region is nucleotides 1256-1271 of SEQ ID NO: 1. In certain such embodiments, a short antisense compound targeted to nucleotides 1256-1271 of SEQ ID NO: 1 comprises a nucleotide sequence selected from SEQ ID NO 43 or 44. In certain such embodiments, a short antisense compound targeted to nucleotides 1256-1271 of SEQ ID NO: 1 is selected from Isis NO. 372825 or 372903.

In certain embodiments, a target region is nucleotides 1304-1319 of SEQ ID NO: 1. In certain such embodiments, a short antisense compound targeted to nucleotides 1304-1319 of SEQ ID NO: 1 comprises a nucleotide sequence selected from SEQ ID NO 45 or 46. In certain such embodiments, a short antisense compound targeted to nucleotides 1304-1319 of SEQ ID NO: 1 is selected from Isis NO. 372826 or 372904.

In certain embodiments, a target region is nucleotides 2135-2150 of SEQ ID NO: 1. In certain such embodiments, a short antisense compound targeted to nucleotides 2135-2150 of SEQ ID NO: 1 comprises a nucleotide sequence selected from SEQ ID NO 47 or 48. In certain such embodiments, a short antisense compound targeted to nucleotides 2135-2150 of SEQ ID NO: 1 is selected from ISIS NO. 372829 or 372907.

In certain embodiments, a target region is nucleotides 2774-2794 of SEQ ID NO: 1. In certain such embodiments, a short antisense compound targeted to nucleotides 2774-2794 of SEQ ID NO: 1 comprises a nucleotide sequence selected from SEQ ID NO 49, 50, 51, or 52. In certain such embodiments, a short antisense compound targeted to nucleotides 2774-2794 of SEQ ID NO: 1 is selected from ISIS NO. 372832, 372910, 372833, or 372911.

In certain embodiments, a target region is nucleotides 2961-2976 of SEQ ID NO: 1. In certain such embodiments, a short antisense compound targeted to nucleotides 2961-2976 of SEQ ID NO: 1 comprises a nucleotide sequence selected from SEQ ID NO 53 or 54. In certain such embodiments, a short antisense compound targeted to nucleotides 2961-2976 of SEQ ID NO: 1 is selected from ISIS NO. 372835 or 372913.

In certain embodiments, a target region is nucleotides 3248-3269 of SEQ ID NO: 1. In certain such embodiments, a short antisense compound targeted to nucleotides 3248-3269 of SEQ ID NO: 1 comprises a nucleotide sequence selected from SEQ ID NO 55, 56, 57, 58, 59, 60, 61, 62, or 63. In certain such embodiments, a short antisense compound targeted to nucleotides 3248-3269 of SEQ ID NO: 1 is selected from ISIS NO. 346592, 346593, 346594, 346595, 346596, 346597, 346598, 346599, or 346600.

In certain embodiments, a target region is nucleotides 3350-3375 of SEQ ID NO: 1. In certain such embodiments, a short antisense compound targeted to nucleotides 3350-3375 of SEQ ID NO: 1 comprises a nucleotide sequence selected from SEQ ID NO 64, 65, 66, 67, 68, or 69. In certain such embodiments, a short antisense compound targeted to nucleotides 3350-3375 of SEQ ID NO: 1 is selected from ISIS NO. 372836, 372914, 372837, 372915, 372838, or 372916.

In certain embodiments, a target region is nucleotides 3409-3424 of SEQ ID NO: 1. In certain such embodiments, a short antisense compound targeted to nucleotides 3409-3424 of SEQ ID NO: 1 comprises a nucleotide sequence selected from SEQ ID NO 70 or 73. In certain such embodiments, a short antisense compound targeted to nucleotides 3409-3424 of SEQ ID NO: 1 is selected from ISIS NO. 372839,387461, 380147, or 372917.

In certain embodiments, a target region is nucleotides 3573-3588 of SEQ ID NO: 1. In certain such embodiments, a short antisense compound targeted to nucleotides 3573-3588 of SEQ ID NO: 1 comprises a nucleotide sequence selected from SEQ ID NO 74 or 75. In certain such embodiments, a short antisense compound targeted to nucleotides 3573-3588 of SEQ ID NO: 1 is selected from ISIS NO. 372840 or 372918.

In certain embodiments, a target region is nucleotides 3701-3716 of SEQ ID NO: 1. In certain such embodiments, a short antisense compound targeted to nucleotides 3701-3716 of SEQ ID NO: 1 comprises a nucleotide sequence selected from SEQ ID NO 76 or 77. In certain such embodiments, a short antisense compound targeted to nucleotides 3701-3716 of SEQ ID NO: 1 is selected from ISIS NO. 372841 or 372919.

In certain embodiments, a target region is nucleotides 4219-4234 of SEQ ID NO: 1. In certain such embodiments, a short antisense compound targeted to nucleotides 4219-4234 of SEQ ID NO: 1 comprises a nucleotide sequence selected from SEQ ID NO 78 or 79. In certain such embodiments, a short antisense compound targeted to nucleotides 4219-4234 of SEQ ID NO: 1 is selected from ISIS NO. 372843 or 372921.

In certain embodiments, a target region is nucleotides 4301-4323 of SEQ ID NO: 1. In certain such embodiments, a short antisense compound targeted to nucleotides 4301-4323 of SEQ ID NO: 1 comprises a nucleotide sequence selected from SEQ ID NO 80, 81, 82, or 83. In certain embodiments, a short antisense compound targeted to nucleotides 4301-4323 of SEQ ID NO: 1 is selected from ISIS NO. 372844, 372922, 372845, or 372923.

In certain embodiments, a target region is nucleotides 5588-5609 of SEQ ID NO: 1. In certain such embodiments, a short antisense compound targeted to nucleotides 5588-5609 of SEQ ID NO: 1 comprises a nucleotide sequence selected from SEQ ID NO 84, 85, 86, 87, 88, 89, 90, 91, or 92. In certain such embodiments, a short antisense compound targeted to nucleotides 5588-5609 of SEQ ID NO: 1 is selected from ISIS NO. 346601, 346602, 346603, 346604, 346605, 346606, 346607, 346608, or 346609.

In certain embodiments, a target region is nucleotides 5924-5939 of SEQ ID NO: 1. In certain such embodiments, a short antisense compound targeted to nucleotides 5924-5939 of SEQ ID NO: 1 comprises a nucleotide sequence selected from SEQ ID NO 93 or 94. In certain such embodiments, a short antisense compound targeted to nucleotides 5924-5939 of SEQ ID NO: 1 is selected from ISIS NO. 372851 or 372929.

In certain embodiments, a target region is nucleotides 6664-6679 of SEQ ID NO: 1. In certain such embodiments, a short antisense compound targeted to nucleotides 6664-6679 of SEQ ID NO: 1 comprises a nucleotide sequence selected from SEQ ID NO 95 or 96. In certain such embodiments, a short antisense compound targeted to nucleotides 6664-6679 of SEQ ID NO: 1 is selected from ISIS NO. 372854 or 372932.

In certain embodiments, a target region is nucleotides 6908-6923 of SEQ ID NO: 1. In certain such embodiments, a short antisense compound targeted to nucleotides 6908-6923 of SEQ ID NO: 1 comprises a nucleotide sequence selected from SEQ ID NO 97 or 98. In certain such embodiments, a short antisense compound targeted to nucleotides 6908-6923 of SEQ ID NO: 1 is selected from ISIS NO. 372855 or 372933.

In certain embodiments, a target region is nucleotides 7190-7205 of SEQ ID NO: 1. In certain such embodiments, a short antisense compound targeted to nucleotides 7190-7205 of SEQ ID NO: 1 comprises a nucleotide sequence selected from SEQ ID NO 99 or 100. In certain such embodiments, a short antisense compound targeted to nucleotides 7190-7205 of SEQ ID NO: 1 is selected from ISIS NO. 372856 or 372934.

In certain embodiments, a target region is nucleotides 7817-7839 of SEQ ID NO: 1. In certain such embodiments, a short antisense compound targeted to nucleotides 7817-7839 of SEQ ID NO: 1 comprises a nucleotide sequence selected from SEQ ID NO 101, 102, 104, 105, 106, 107, 108, 109, 110, or 111. In certain such embodiments, a short antisense compound targeted to nucleotides 7817-7839 of SEQ ID NO: 1 is selected from ISIS NO. 372858, 372936, 346610, 346611, 346612, 346613, 346614, 346615, 346616, 346617, or 346618.

In certain embodiments, a target region is nucleotides 7995-8010 of SEQ ID NO: 1. In certain such embodiments, a short antisense compound targeted to nucleotides 7995-8010 of SEQ ID NO: 1 comprises a nucleotide sequence selected from SEQ ID NO 112 or 113. In certain such embodiments, a short antisense compound targeted to nucleotides 7995-8010 of SEQ ID NO: 1 is selected from ISIS NO. 372859 or 372937.

In certain embodiments, a target region is nucleotides 8336-8356 of SEQ ID NO: 1. In certain such embodiments, a short antisense compound targeted to nucleotides 8336-8356 of SEQ ID NO: 1 comprises a nucleotide sequence selected from SEQ ID NO 114, 115, 116, or 117. In certain such embodiments, a short antisense compound targeted to nucleotides 8336-8356 of SEQ ID NO: 1 is selected from ISIS NO. 372861, 372939, 372862, or 372940.

In certain embodiments, a target region is nucleotides 8539-8554 of SEQ ID NO: 1. In certain such embodiments, a short antisense compound targeted to nucleotides 8539-8554 of SEQ ID NO: 1 comprises a nucleotide sequence selected from SEQ ID NO 118 or 119. In certain such embodiments, a short antisense compound targeted to nucleotides 8539-8554 of SEQ ID NO: 1 is selected from ISIS NO. 372863 or 372941.

In certain embodiments, a target region is nucleotides 9344-9359 of SEQ ID NO: 1. In certain such embodiments, a short antisense compound targeted to nucleotides 9344-9359 of SEQ ID NO: 1 comprises a nucleotide sequence selected from SEQ ID NO 120 or 121. In certain such embodiments, a short antisense compound targeted to nucleotides 9344-9359 of SEQ ID NO: 1 is selected from ISIS NO. 372871 or 372949.

In certain embodiments, a target region is nucleotides 9515-9530 of SEQ ID NO: 1. In certain such embodiments, a short antisense compound targeted to nucleotides 9515-9530 of SEQ ID NO: 1 comprises a nucleotide sequence selected from SEQ ID NO 122 or 123. In certain such embodiments, a short antisense compound targeted to nucleotides 9515-9530 of SEQ ID NO: 1 is selected from ISIS NO. 372872 or 372950.

In certain embodiments, a target region is nucleotides 9794-9809 of SEQ ID NO: 1. In certain such embodiments, a short antisense compound targeted to nucleotides 9794-9809 of SEQ ID NO: 1 comprises a nucleotide sequence selected from SEQ ID NO 124 or 125. In certain such embodiments, a short antisense compound targeted to nucleotides 9794-9809 of SEQ ID NO: 1 is selected from ISIS NO. 372875 or 372953.

In certain embodiments, a target region is nucleotides 10157-10187 of SEQ ID NO: 1. In certain such embodiments, a short antisense compound targeted to nucleotides 10157-10187 of SEQ ID NO: 1 comprises a nucleotide sequence selected from SEQ ID NO 126, 127, 128, 129, 130, 131, 132, or 133. In certain such embodiments, a short antisense compound targeted to nucleotides 10157-10187 of SEQ ID NO: 1 is selected from ISIS NO. 372877, 372955, 372878, 372956, 372879, 372957, 372880, or 372958.

In certain embodiments, a target region is nucleotides 10838-10859 of SEQ ID NO: 1. In certain such embodiments, a short antisense compound targeted to nucleotides 10838-10859 of SEQ ID NO: 1 comprises a nucleotide sequence selected from SEQ ID NO 134, 135, 136, 137, 138, 139, 140, 141, or 142. In certain such embodiments, a short antisense compound targeted to nucleotides 10838-10859 of SEQ ID NO: 1 is selected from ISIS NO. 346619, 346620, 346621, 346622, 346623, 346624, 346625, 346626, or 346627.

In certain embodiments, a target region is nucleotides 13689-13714 of SEQ ID NO: 1. In certain such embodiments, a short antisense compound targeted to nucleotides 13689-13714 of SEQ ID NO: 1 comprises a nucleotide sequence selected from SEQ ID NO 143, 144, 145, 146, 147, or 148. In certain such embodiments, a short antisense compound targeted to nucleotides 13689-13714 of SEQ ID NO: 1 is selected from ISIS NO. 372890, 372968, 372891, 372969, 372892, or 372970.

In certain embodiments, a target region is nucleotides 13907-13928 of SEQ ID NO: 1. In certain such embodiments, a short antisense compound targeted to nucleotides 13907-13928 of SEQ ID NO: 1 comprises a nucleotide sequence selected from SEQ ID NO 149, 150, 151, 152, 153, 154, 155, 156, or 157. In certain such embodiments, a short antisense compound targeted to nucleotides 13907-13928 of SEQ ID NO: 1 is selected from ISIS NO. 346628, 346629, 346630, 346631, 346632, 346633, 346634, 346635, or 346636.

In certain embodiments, a target region is nucleotides 13963-13984 of SEQ ID NO: 1. In certain such embodiments, a short antisense compound targeted to nucleotides 13963-13984 of SEQ ID NO: 1 comprises a nucleotide sequence selected from SEQ ID NO 158, 159, 160, 161, 162, 163, 164, 165, or 166. In certain such embodiments, a short antisense compound targeted to nucleotides 13963-13984 of SEQ ID NO: 1 is selected from ISIS NO. 346637, 346638, 346639, 346640, 346641, 346642, 346643, 346644, or 346645.

In certain embodiments, a target region is nucleotides 14051-14072 of SEQ ID NO: 1. In certain such embodiments, a short antisense compound targeted to nucleotides 14051-14072 of SEQ ID NO: 1 comprises a nucleotide sequence selected from SEQ ID NO 167, 168, 169, 170, 171, 172, 173, 174, or 175. In certain such embodiments, a short antisense compound targeted to nucleotides 14051-14072 of SEQ ID NO: 1 is selected from ISIS NO. 346646, 346647, 346648, 346649, 346650, 346651, 346652, 346653, or 346654.

In certain embodiments, short antisense compounds targeted to an ApoB nucleic acid are 8 to 16, preferably 9 to 15, more preferably 9 to 14, more preferably 10 to 14 nucleotides in length. In certain embodiments, short antisense compounds targeted to an ApoB nucleic acid are 9 to 14 nucleotides in length. In certain embodiments, short antisense compounds targeted to an ApoB nucleic acid are 10 to 14 nucleotides in length. In certain embodiments, such short antisense compounds are short antisense oligonucleotides.

In certain embodiments, short antisense compounds targeted to an ApoB nucleic acid are short gapmers. In certain such embodiments, short gapmers targeted to an ApoB nucleic acid comprise at least one high affinity modification in one or more wings of the compound. In certain embodiments, short antisense compounds targeted to an ApoB nucleic acid comprise 1 to 3 high-affinity modifications in each wing. In certain such embodiments, the nucleosides or nucleotides of the wing comprise a 2' modification. In certain such embodiments, the monomers of the wing are BNA's. In certain such embodiments, the monomers of the wing are selected from α-L-Methyleneoxy (4'-CH₂-O-2') BNA , β-D-Methyleneoxy (4'-CH₂-O-2') BNA, Ethyleneoxy (4'-(CH₂)₂-O-2') BNA , Aminooxy (4'-CH₂-O-N(R)-2') BNA and Oxyamino (4'-CH₂-N(R)-O-2') BNA. In certain embodiments, the monomers of a wing comprise a substituent at the 2' position selected from allyl, amino, azido, thio, O-allyl, O-C₁-C₁₀ alkyl, - OCF₃, O-(CH₂)₂-O-CH₃, 2'-O(CH₂)₂SCH₃, O-(CH₂)₂-O-N(Rₘ)(Rₙ), and O-CH₂-C(=O)-N(Rₘ)(Rₙ), where each Rₘ and Rₙ is, independently, H or substituted or unsubstituted C₁-C₁₀ alkyl. In certain embodiments, the monomers of a wing are 2'MOE nucleotides.

In certain embodiments, short antisense compounds targeted to an ApoB nucleic acid comprise a gap between the 5' wing and the 3' wing. In certain embodiments the gap comprises five, six, seven, eight, nine, ten, eleven, twelve, thirteen, or fourteen monomers. In certain embodiments, the monomers of the gap are unmodified deoxyribonucleotides. In certain embodiments, the monomers of the gap are unmodified ribonucleotides. In certain embodiments, gap modifications (if any) gap result in an antisense compound that, when bound to its target nucleic acid, supports cleavage by an RNase, including, but not limited to, RNase H.

In certain embodiments, short antisense compounds targeted to an ApoB nucleic acid have uniform monomeric linkages. In certain such embodiments, those linkages are all phosphorothioate linkages. In certain embodiments, the linkages are all phosphodiester linkages. In certain embodiments, short antisense compounds targeted to an ApoB nucleic acid have mixed backbones.

In certain embodiments, short antisense compounds targeted to an ApoB nucleic acid are 8 monomers in length. In certain embodiments, short antisense compounds targeted to an ApoB nucleic acid are 9 monomers in length. In certain embodiments, short antisense compounds targeted to an ApoB nucleic acid are 10 monomers in length. In certain embodiments, short antisense compounds targeted to an ApoB nucleic acid are 11 monomers in length. In certain embodiments, short antisense compounds targeted to an ApoB nucleic acid are monomers in length. In certain embodiments, short antisense compounds targeted to an ApoB nucleic acid are 13 monomers in length. In certain embodiments, short antisense compounds targeted to an ApoB nucleic acid are 14 monomers in length. In certain embodiments, short antisense compounds targeted to an ApoB nucleic acid are 15 monomers in length. In certain embodiments, short antisense compounds targeted to an ApoB nucleic acid are 16 monomers in length. In certain embodiments, short antisense compounds targeted to an ApoB nucleic acid comprise 9 to 15 monomers. In certain embodiments, short antisense compounds targeted to an ApoB nucleic acid comprise 10 to 15 monomers. In certain embodiments, short antisense compounds targeted to an ApoB nucleic acid comprise 12 to 14 monomers. In certain embodiments, short antisense compounds targeted to an ApoB nucleic acid comprise 12 to 14 nucleotides or nucleosides.

In certain embodiments, disclosed herein are methods of modulating expression of ApoB. In certain embodiments, such methods comprise use of one or more short antisense compound targeted to an ApoB nucleic acid, wherein the short antisense compound targeted to an ApoB nucleic acid is from about 8 to about 16, preferably 9 to 15, more preferably 9 to 14, more preferably 10 to 14 monomers (i.e. from about 8 to about 16 linked monomers). One of ordinary skill in the art will appreciate that this comprehends methods of modulating expression of ApoB using one or more short antisense compounds targeted to an ApoB nucleic acid of 8, 9, 10, 11, 12, 13, 14, 15 or 16 monomers.

In certain embodiments, methods of modulating ApoB comprise use of a short antisense compound targeted to an ApoB nucleic acid that is 8 monomers in length. In certain embodiments, methods of modulating ApoB comprise use of a short antisense compound targeted to an ApoB nucleic acid that is 9 monomers in length. In certain embodiments, methods of modulating ApoB comprise use of a short antisense compound targeted to an ApoB nucleic acid that is 10 monomers in length. In certain embodiments, methods of modulating ApoB comprise use of a short antisense compound targeted to an ApoB nucleic acid that is 11 monomers in length. In certain embodiments, methods of modulating ApoB comprise use of a short antisense compound targeted to an ApoB nucleic acid that is 12 monomers in length. In certain embodiments, methods of modulating ApoB comprise use of a short antisense compound targeted to an ApoB nucleic acid that is 13 monomers in length. In certain embodiments, methods of modulating ApoB comprise use of a short antisense compound targeted to an ApoB nucleic acid that is 14 monomers in length. In certain embodiments, methods of modulating ApoB comprise use of a short antisense compound targeted to an ApoB nucleic acid that is 15 monomers in length. In certain embodiments, methods of modulating ApoB comprise use of a short antisense compound targeted to an ApoB nucleic acid that is 16 monomers in length.

In certain embodiments, methods of modulating expression of ApoB comprise use of a short antisense compound targeted to an ApoB nucleic acid comprising 9 to 15 monomers. In certain embodiments, methods of modulating expression of ApoB comprise use of a short antisense compound targeted to an ApoB nucleic acid comprising 10 to 15 monomers. In certain embodiments, methods of modulating expression of ApoB comprise use of a short antisense compound targeted to an ApoB nucleic acid comprising 12 to 14 monomers. In certain embodiments, methods of modulating expression of ApoB comprise use of a short antisense compound targeted to an ApoB nucleic acid comprising 12 or 14 nucleotides or nucleosides.

In certain embodiments, short antisense compounds targeting a ApoB nucleic acid may have any one or more properties or characteristics of the short antisense compounds generally described herein. In certain embodiments, short antisense compounds targeting a ApoB nucleic acid have a motif (wing - deoxy gap -wing) selected from 1-12-1, 1-1-10-2, 2-10-1-1, 3-10-3, 2-10-3, 2-10-2, 1-10-1,1-10-2, 3-8-3, 2-8-2, 1-8-1, 3-6-3 or 1-6-1, more preferably 1-10-1, 2-10-2, 3-10-3, and 1-9-2.

### Salts, prodrugs and bioequivalents

The antisense compounds provided herein comprise any pharmaceutically acceptable salts, esters, or salts of such esters, or any other functional chemical equivalent which, upon administration to an animal including a human, is capable of providing (directly or indirectly) the biologically active metabolite or residue thereof. Accordingly, for example, the disclosure is also drawn to prodrugs and pharmaceutically acceptable salts of the antisense compounds, pharmaceutically acceptable salts of such prodrugs, and other bioequivalents.

The term "prodrug" indicates a therapeutic agent that is prepared in an inactive or less active form that is converted to an active form (i.e., drug) within the body or cells thereof by the action of endogenous enzymes, chemicals, and/or conditions. In particular, prodrug versions of the oligonucleotides are prepared as SATE ((S-acetyl-2-thioethyl) phosphate) derivatives according to the methods disclosed in WO 93/24510 or WO 94/26764. Prodrugs can also include antisense compounds wherein one or both ends comprise nucleobases that are cleaved (e.g., by incorporating phosphodiester backbone linkages at the ends) to produce the active compound. In certain embodiments, one or more non-drug moieties is cleaved from a prodrug to yield the active form. In certain such embodiments, such non-drug moieties is not a nucleotide or oligonucleotide.

The term "pharmaceutically acceptable salts" refers to physiologically and pharmaceutically acceptable salts of the compounds described herein: i.e., salts that retain the desired biological activity of the parent compound and do not impart undesired toxicological effects thereto. Sodium salts of antisense oligonucleotides are useful and are well accepted for therapeutic administration to humans.

In certain embodiments, salts, including, but not limited to sodium salts, of double stranded nucleic acids (including but not limited to dsRNA compounds) are also provided.

### G. Certain Pharmaceutical Compositions

In certain embodiments, pharmaceutical compositions of the present invention comprise one or more short antisense compound and one or more excipients. In certain such embodiments, excipients are selected from water, salt solutions, alcohol, polyethylene glycols, gelatin, lactose, amylase, magnesium stearate, talc, silicic acid, viscous paraffin, hydroxymethylcellulose and polyvinylpyrrolidone.

In certain embodiments, a pharmaceutical composition of the present invention is prepared using known techniques, including, but not limited to mixing, dissolving, granulating, dragee-making, levigating, emulsifying, encapsulating, entrapping or tabletting processes.

In certain embodiments, a pharmaceutical composition of the present invention is a liquid (e.g., a suspension, elixir and/or solution). In certain of such embodiments, a liquid pharmaceutical composition is prepared using ingredients known in the art, including, but not limited to, water, glycols, oils, alcohols, flavoring agents, preservatives, and coloring agents.

In certain embodiments, a pharmaceutical composition of the present invention is a solid (e.g., a powder, tablet, and/or capsule). In certain of such embodiments, a solid pharmaceutical composition comprising one or more oligonucleotides is prepared using ingredients known in the art, including, but not limited to, starches, sugars, diluents, granulating agents, lubricants, binders, and disintegrating agents.

In certain embodiments, a pharmaceutical composition of the present invention is formulated as a depot preparation. Certain such depot preparations are typically longer acting than non-depot preparations. In certain embodiments, such preparations are administered by implantation (for example subcutaneously or intramuscularly) or by intramuscular injection. In certain embodiments, depot preparations are prepared using suitable polymeric or hydrophobic materials (for example an emulsion in an acceptable oil) or ion exchange resins, or as sparingly soluble derivatives, for example, as a sparingly soluble salt.

In certain embodiments, a pharmaceutical composition of the present invention comprises a delivery system. Examples of delivery systems include, but are not limited to, liposomes and emulsions. Certain delivery systems are useful for preparing certain pharmaceutical compositions including those comprising hydrophobic compounds. In certain embodiments, certain organic solvents such as dimethylsulfoxide are used.

In certain embodiments, a pharmaceutical composition of the present invention comprises one or more tissue-specific delivery molecules designed to deliver the one or more pharmaceutical agents of the present invention to specific tissues or cell types. For example, in certain embodiments, pharmaceutical compositions include liposomes coated with a tissue-specific antibody.

In certain embodiments, a pharmaceutical composition of the present invention comprises a co-solvent system. Certain of such co-solvent systems comprise, for example, benzyl alcohol, a nonpolar surfactant, a water-miscible organic polymer, and an aqueous phase. In certain embodiments, such co-solvent systems are used for hydrophobic compounds. A non-limiting example of such a co-solvent system is the VPD co-solvent system, which is a solution of absolute ethanol comprising 3% w/v benzyl alcohol, 8% w/v of the nonpolar surfactant Polysorbate 80.TM., and 65% w/v polyethylene glycol 300. The proportions of such co-solvent systems may be varied considerably without significantly altering their solubility and toxicity characteristics. Furthermore, the identity of co-solvent components may be varied: for example, other surfactants may be used instead of Polysorbate 80.TM.; the fraction size of polyethylene glycol may be varied; other biocompatible polymers may replace polyethylene glycol, e.g., polyvinyl pyrrolidone; and other sugars or polysaccharides may substitute for dextrose.

In certain embodiments, a pharmaceutical composition of the present invention comprises a sustained-release system. A non-limiting example of such a sustained-release system is a semi-permeable matrix of solid hydrophobic polymers. In certain embodiments, sustained-release systems may, depending on their chemical nature, release pharmaceutical agents over a period of hours, days, weeks or months.

In certain embodiments, a pharmaceutical composition of the present invention is prepared for oral administration. In certain of such embodiments, a pharmaceutical composition is formulated by combining one or more oligonucleotides with one or more pharmaceutically acceptable carriers. Certain of such carriers enable pharmaceutical compositions to be formulated as tablets, pills, dragees, capsules, liquids, gels, syrups, slurries, suspensions and the like, for oral ingestion by a subject. In certain embodiments, pharmaceutical compositions for oral use are obtained by mixing oligonucleotide and one or more solid excipient. Suitable excipients include, but are not limited to, fillers, such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethylcellulose, sodium carboxymethylcellulose, and/or polyvinylpyrrolidone (PVP). In certain embodiments, such a mixture is optionally ground and auxiliaries are optionally added. In certain embodiments, pharmaceutical compositions are formed to obtain tablets or dragee cores. In certain embodiments, disintegrating agents (e.g., cross-linked polyvinyl pyrrolidone, agar, or alginic acid or a salt thereof, such as sodium alginate) are added.

In certain embodiments, dragee cores are provided with coatings. In certain such embodiments, concentrated sugar solutions may be used, which may optionally comprise gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to tablets or dragee coatings.

In certain embodiments, pharmaceutical compositions for oral administration are push-fit capsules made of gelatin. Certain of such push-fit capsules comprise one or more pharmaceutical agents of the present invention in admixture with one or more filler such as lactose, binders such as starches, and/or lubricants such as talc or magnesium stearate and, optionally, stabilizers. In certain embodiments, pharmaceutical compositions for oral administration are soft, sealed capsules made of gelatin and a plasticizer, such as glycerol or sorbitol. In certain soft capsules, one or more pharmaceutical agents of the present invention are be dissolved or suspended in suitable liquids, such as fatty oils, liquid paraffin, or liquid polyethylene glycols. In addition, stabilizers may be added.

In certain embodiments, pharmaceutical compositions are prepared for buccal administration. Certain of such pharmaceutical compositions are tablets or lozenges formulated in conventional manner.

In certain embodiments, a pharmaceutical composition is prepared for administration by injection (e.g., intravenous, subcutaneous, intramuscular, etc.). In certain of such embodiments, a pharmaceutical composition comprises a carrier and is formulated in aqueous solution, such as water or physiologically compatible buffers such as Hanks's solution, Ringer's solution, or physiological saline buffer. In certain embodiments, other ingredients are included (e.g., ingredients that aid in solubility or serve as preservatives). In certain embodiments, injectable suspensions are prepared using appropriate liquid carriers, suspending agents and the like. Certain pharmaceutical compositions for injection are presented in unit dosage form, e.g., in ampoules or in multi-dose containers. Certain pharmaceutical compositions for injection are suspensions, solutions or emulsions in oily or aqueous vehicles, and may comprise formulatory agents such as suspending, stabilizing and/or dispersing agents. Certain solvents suitable for use in pharmaceutical compositions for injection include, but are not limited to, lipophilic solvents and fatty oils, such as sesame oil, synthetic fatty acid esters, such as ethyl oleate or triglycerides, and liposomes. Aqueous injection suspensions may comprise substances that increase the viscosity of the suspension, such as sodium carboxymethyl cellulose, sorbitol, or dextran. Optionally, such suspensions may also comprise suitable stabilizers or agents that increase the solubility of the pharmaceutical agents to allow for the preparation of highly concentrated solutions.

In certain embodiments, a pharmaceutical composition is prepared for transmucosal administration. In certain of such embodiments penetrants appropriate to the barrier to be permeated are used in the formulation. Such penetrants are generally known in the art.

In certain embodiments, a pharmaceutical composition is prepared for administration by inhalation. Certain of such pharmaceutical compositions for inhalation are prepared in the form of an aerosol spray in a pressurized pack or a nebulizer. Certain of such pharmaceutical compositions comprise a propellant, e.g., dichlorodifluoromethane, trichlorofluoromethane, dichlorotetrafluoroethane, carbon dioxide or other suitable gas. In certain embodiments using a pressurized aerosol, the dosage unit may be determined with a valve that delivers a metered amount. In certain embodiments, capsules and cartridges for use in an inhaler or insufflator may be formulated. Certain of such formulations comprise a powder mixture of a pharmaceutical agent of the invention and a suitable powder base such as lactose or starch.

In certain embodiments, a pharmaceutical composition is prepared for rectal administration, such as a suppositories or retention enema. Certain of such pharmaceutical compositions comprise known ingredients, such as cocoa butter and/or other glycerides.

In certain embodiments, a pharmaceutical composition is prepared for topical administration. Certain of such pharmaceutical compositions comprise bland moisturizing bases, such as ointments or creams. Exemplary suitable ointment bases include, but are not limited to, petrolatum, petrolatum plus volatile silicones, lanolin and water in oil emulsions such as Eucerin.TM., available from Beiersdorf (Cincinnati, Ohio). Exemplary suitable cream bases include, but are not limited to, Nivea.TM. Cream, available from Beiersdorf (Cincinnati, Ohio), cold cream (USP), Purpose Cream.TM., available from Johnson & Johnson (New Brunswick, N.J.), hydrophilic ointment (USP) and Lubriderm.TM., available from Pfizer (Morris Plains, N.J.).

In certain embodiments, a pharmaceutical composition of the present invention comprises an oligonucleotide in a therapeutically effective amount. In certain embodiments, the therapeutically effective amount is sufficient to prevent, alleviate or ameliorate symptoms of a disease or to prolong the survival of the subject being treated. Determination of a therapeutically effective amount is well within the capability of those skilled in the art.

In certain embodiments, one or more short antisense compound of the present invention is formulated as a prodrug. In certain embodiments, upon in vivo administration, a prodrug is chemically converted to the biologically, pharmaceutically or therapeutically more active form of the short antisense compound. In certain embodiments, prodrugs are useful because they are easier to administer than the corresponding active form. For example, in certain instances, a prodrug may be more bioavailable (e.g., through oral administration) than is the corresponding active form. In certain instances, a prodrug may have improved solubility compared to the corresponding active form. In certain embodiments, prodrugs are less water soluble than the corresponding active form. In certain instances, such prodrugs possess superior transmittal across cell membranes, where water solubility is detrimental to mobility. In certain embodiments, a prodrug is an ester. In certain such embodiments, the ester is metabolically hydrolyzed to carboxylic acid upon administration. In certain instances the carboxylic acid containing compound is the corresponding active form. In certain embodiments, a prodrug comprises a short peptide (polyaminoacid) bound to an acid group. In certain of such embodiments, the peptide is cleaved upon administration to form the corresponding active form.

In certain embodiments, a prodrug is produced by modifying a pharmaceutically active compound such that the active compound will be regenerated upon in vivo administration. The prodrug can be designed to alter the metabolic stability or the transport characteristics of a drug, to mask side effects or toxicity, to improve the flavor of a drug or to alter other characteristics or properties of a drug. By virtue of knowledge of pharmacodynamic processes and drug metabolism in vivo, those of skill in this art, once a pharmaceutically active compound is known, can design prodrugs of the compound (see, e.g., Nogrady (1985) Medicinal Chemistry A Biochemical Approach, Oxford University Press, New York, pages 388-392).

In certain embodiments, a pharmaceutical composition comprising one or more pharmaceutical agents of the present invention is useful for treating a conditions or disorders in a mammalian, and particularly in a human, subject. Suitable administration routes include, but are not limited to, oral, rectal, transmucosal, intestinal, enteral, topical, suppository, through inhalation, intrathecal, intraventricular, intraperitoneal, intranasal, intraocular and parenteral (e.g., intravenous, intramuscular, intramedullary, and subcutaneous). In certain embodiments, pharmaceutical intrathecals are administered to achieve local rather than systemic exposures. For example, pharmaceutical compositions may be injected directly in the area of desired effect (e.g., in the renal or cardiac area).

In certain embodiments, short antisense compounds, compared to their parent oligonucleotides, make them particularly suited to oral administration. In certain embodiments, short antisense compounds are better suited for oral administration than their parent oligonucleotides because they have increased potency compared to those parent oligonucleotides. In certain embodiments, short antisense compounds are better suited for oral administration than their parent oligonucleotides because they have better stability, availability or solubility properties compared to those parent oligonucleotides.

In a further aspect, a pharmaceutical agent is sterile lyophilized oligonucleotide that is reconstituted with a suitable diluent, e.g., sterile water for injection. The reconstituted product is administered as a subcutaneous injection or as an intravenous infusion after dilution into saline. The lyophilized drug product consists of the oligonucleotide which has been prepared in water for injection, adjusted to pH 7.0-9.0 with acid or base during preparation, and then lyophilized. The lyophilized oligonucleotide may be 25-800 mg of the oligonucleotide. It is understood that this encompasses 25, 50, 75, 100, 125, 150, 175, 200, 225, 250, 275, 300, 325, 350, 375, 425, 450, 475, 500, 525, 550, 575, 600, 625, 650, 675, 700, 725, 750, 775, and 800 mg of lyophilized oligonucleotide. The lyophilized drug product may be packaged in a 2 mL Type I, clear glass vial (ammonium sulfate-treated), stoppered with a bromobutyl rubber closure and sealed with an aluminum FLIP-OFF® overseal.

The compositions of the present invention may additionally comprise other adjunct components conventionally found in pharmaceutical compositions, at their art-established usage levels. Thus, for example, the compositions may comprise additional, compatible, pharmaceutically-active materials such as, for example, antipruritics, astringents, local anesthetics or anti-inflammatory agents, or may comprise additional materials useful in physically formulating various dosage forms of the compositions of the present invention, such as dyes, flavoring agents, preservatives, antioxidants, opacifiers, thickening agents and stabilizers. However, such materials, when added, should not unduly interfere with the biological activities of the components of the compositions of the present invention. The formulations can be sterilized and, if desired, mixed with auxiliary agents, e.g., lubricants, preservatives, stabilizers, wetting agents, emulsifiers, salts for influencing osmotic pressure, buffers, colorings, flavorings and/or aromatic substances and the like which do not deleteriously interact with the oligonucleotide(s) of the formulation. The antisense compounds provided herein may also be admixed, encapsulated, conjugated or otherwise associated with other molecules, molecule structures or mixtures of compounds.

Also described herein are pharmaceutical compositions and formulations which include the antisense compounds provided herein. The pharmaceutical compositions may be administered in a number of ways depending upon whether local or systemic treatment is desired and upon the area to be treated. In a preferred embodiment, administration is topical to the surface of the respiratory tract, particularly pulmonary, e.g., by nebulization, inhalation, or insufflation of powders or aerosols, by mouth and/or nose.

The pharmaceutical formulations described herein, which may conveniently be presented in unit dosage form, may be prepared according to conventional techniques well known in the pharmaceutical industry. Such techniques include the step of bringing into association the active ingredients with the pharmaceutical carrier(s) or excipient(s). In general, the formulations are prepared by uniformly and intimately bringing into association the active ingredients with liquid carriers, finely divided solid carriers, or both, and then, if necessary, shaping the product (e.g., into a specific particle size for delivery). In a preferred embodiment, the pharmaceutical formulations are prepared for pulmonary administration in an appropriate solvent, e.g., water or normal saline, possibly in a sterile formulation, with carriers or other agents to allow for the formation of droplets of the desired diameter for delivery using inhalers, nasal delivery devices, nebulizers, and other devices for pulmonary delivery. Alternatively, the pharmaceutical formulations may be formulated as dry powders for use in dry powder inhalers.

A "pharmaceutical carrier" or "excipient" can be a pharmaceutically acceptable solvent, suspending agent or any other pharmacologically inert vehicle for delivering one or more nucleic acids to an individual and are known in the art. The excipient may be liquid or solid and is selected, with the planned manner of administration in mind, so as to provide for the desired bulk, consistency, etc., when combined with a nucleic acid and the other components of a given pharmaceutical composition.

### H. Certain Therapeutic Uses

In certain embodiments, antisense compounds are used to modulate the expression of a target gene in an animal, such as a human. In certain embodiments, such compounds can be used to treat metabolic disorders or modulate one or more disease indications. For example, the methods comprise the step of administering to said animal in need of therapy for a disease or condition associated with a target gene an effective amount of an antisense compound that modulates expression of the target gene. Antisense compounds provided herein which effectively modulate expression of a target RNA or protein products of expression are considered active antisense compounds. Active antisense compounds also include compounds which effectively modulate one or more of a number of disease indications, including metabolic and cardiovascular disease indications, examples of which are described below.

Modulation of expression of a target gene can be measured in a bodily fluid, which may or may not contain cells; tissue; or organ of the animal. Methods of obtaining samples for analysis, such as body fluids (e.g., sputum, serum, urine), tissues (e.g., biopsy), or organs, and methods of preparation of the samples to allow for analysis are well known to those skilled in the art. Methods for analysis of RNA and protein levels are discussed above and are well known to those skilled in the art. The effects of treatment can be assessed by measuring biomarkers, or disease indications, associated with the target gene expression in the aforementioned fluids, tissues or organs, collected from an animal contacted with one or more compounds described herein, by routine clinical methods known in the art. These biomarkers include but are not limited to: liver transaminases, bilirubin, albumin, blood urea nitrogen, creatine and other markers of kidney and liver function; interleukins, tumor necrosis factors, intracellular adhesion molecules, C-reactive protein, chemokines, cytokines, and other markers of inflammation.
The antisense compounds provided herein can be utilized in pharmaceutical compositions by adding an effective amount of a compound to a suitable pharmaceutically acceptable diluent or carrier. Acceptable carriers and diluents are well known to those skilled in the art. Selection of a diluent or carrier is based on a number of factors, including, but not limited to, the solubility of the compound and the route of administration. Such considerations are well understood by those skilled in the art. In one aspect, the antisense compounds described herein inhibit expression of a target gene. The compounds can also be used in the manufacture of a medicament for the treatment of diseases and disorders related to a target gene.

Methods whereby bodily fluids, organs or tissues are contacted with an effective amount of one or more of the antisense compounds or compositions provided herein are also contemplated. Bodily fluids, organs or tissues can be contacted with one or more of the compounds resulting in modulation of target gene expression in the cells of bodily fluids, organs or tissues. An effective amount can be determined by monitoring the modulatory effect of the antisense compound or compounds or compositions on target nucleic acids or their products by methods routine to the skilled artisan.

### Co-administration

In certain embodiments, two or more antisense compounds are co-administered. In certain embodiments, pharmaceutical compositions include one or more antisense compounds, particularly oligonucleotides, targeted to a first nucleic acid and one or more antisense compounds targeted to a second nucleic acid target. One or more of those antisense compounds may be a short antisense compound. In certain embodiments, pharmaceutical compositions include two or more antisense compounds targeted to different regions of the same nucleic acid target. One or more of such antisense compounds may be a short antisense compound. Two or more combined compounds may be used together or sequentially.

In certain embodiments, one or more pharmaceutical compositions are co-administered with one or more other pharmaceutical agents. In certain embodiments, such one or more other pharmaceutical agents are designed to treat the same disease or condition as the one or more pharmaceutical compositions of the present invention. In certain embodiments, such one or more other pharmaceutical agents are designed to treat a different disease or condition as the one or more pharmaceutical compositions of the present invention. In certain embodiments, such one or more other pharmaceutical agents are designed to treat an undesired effect of one or more pharmaceutical compositions of the present invention. In certain embodiments, one or more pharmaceutical compositions of the present invention are co-administered with another pharmaceutical agent to treat an undesired effect of that other pharmaceutical agent. In certain embodiments, one or more pharmaceutical compositions of the present invention and one or more other pharmaceutical agents are administered at the same time. In certain embodiments, one or more pharmaceutical compositions of the present invention and one or more other pharmaceutical agents are administered at different times. In certain embodiments, one or more pharmaceutical compositions of the present invention and one or more other pharmaceutical agents are prepared together in a single formulation. In certain embodiments, one or more pharmaceutical compositions of the present invention and one or more other pharmaceutical agents are prepared separately.

In certain embodiments, pharmaceutical agents that may be co-administered with a pharmaceutical composition of the present invention include lipid-lowering agents. In certain such embodiments, pharmaceutical agents that may be co-administered with a pharmaceutical composition of the present invention include, but are not limited to atorvastatin, simvastatin, rosuvastatin, and ezetimibe. In certain such embodiments, the lipid-lowering agent is administered prior to administration of a pharmaceutical composition of the present invention. In certain such embodiments, the lipid-lowering agent is administered following administration of a pharmaceutical composition of the present invention. In certain such embodiments the lipid-lowering agent is administered at the same time as a pharmaceutical composition of the present invention. In certain such embodiments the dose of a co-administered lipid-lowering agent is the same as the dose that would be administered if the lipid-lowering agent was administered alone. In certain such embodiments the dose of a co-administered lipid-lowering agent is lower than the dose that would be administered if the lipid-lowering agent was administered alone. In certain such embodiments the dose of a co-administered lipid-lowering agent is greater than the dose that would be administered if the lipid-lowering agent was administered alone.

In certain embodiments, a co-administered lipid-lowering agent is a HMG-CoA reductase inhibitor. In certain such embodiments the HMG-CoA reductase inhibitor is a statin. In certain such embodiments the statin is selected from atorvastatin, simvastatin, pravastatin, fluvastatin, and rosuvastatin. In certain embodiments, a co-administered lipid-lowering agent is a cholesterol absorption inhibitor. In certain such embodiments, cholesterol absorption inhibitor is ezetimibe. In certain embodiments, a co-administered lipid-lowering agent is a co-formulated HMG-CoA reductase inhibitor and cholesterol absorption inhibitor. In certain such embodiments the co-formulated lipid-lowering agent is ezetimibe/simvastatin. In certain embodiments, a co-administered lipid-lowering agent is a microsomal triglyceride transfer protein inhibitor.

In certain embodiments, a co-administered pharmaceutical agent is a bile acid sequestrant. In certain such embodiments, the bile acid sequestrant is selected from cholestyramine, colestipol, and colesevelam.

In certain embodiments, a co-administered pharmaceutical agent is a nicotinic acid. In certain such embodiments, the nicotinic acid is selected from immediate release nicotinic acid, extended release nicotinic acid, and sustained release nicotinic acid.

In certain embodiments, a co-administered pharmaceutical agent is a fibric acid. In certain such embodiments, a fibric acid is selected from gemfibrozil, fenofibrate, clofibrate, bezafibrate, and ciprofibrate.

Further examples of pharmaceutical agents that may be co-administered with a pharmaceutical composition of the present invention include, but are not limited to, corticosteroids, including but not limited to prednisone; immunoglobulins, including, but not limited to intravenous immunoglobulin (IVIg); analgesics (e.g., acetaminophen); anti-inflammatory agents, including, but not limited to non-steroidal anti-inflammatory drugs (e.g., ibuprofen, COX-1 inhibitors, and COX-2, inhibitors); salicylates; antibiotics; antivirals; antifungal agents; antidiabetic agents (e.g., biguanides, glucosidase inhibitors, insulins, sulfonylureas, and thiazolidenediones); adrenergic modifiers; diuretics; hormones (e.g., anabolic steroids, androgen, estrogen, calcitonin, progestin, somatostan, and thyroid hormones); immunomodulators; muscle relaxants; antihistamines; osteoporosis agents (e.g., biphosphonates, calcitonin, and estrogens); prostaglandins, antineoplastic agents; psychotherapeutic agents; sedatives; poison oak or poison sumac products; antibodies; and vaccines.

In certain embodiments, the pharmaceutical compositions of the present invention may be administered in conjuction with a lipid-lowering therapy. In certain such embodiments, a lipid-lowering therapy is therapeutic lifestyle change. In certain such embodiments, a lipid-lowering therapy is LDL apheresis.

### I. Kits, Research Reagents and Diagnostics

The antisense compounds provided herein can be utilized for diagnostics, and as research reagents and kits. Furthermore, antisense compounds, which are able to inhibit gene expression or modulate gene expression with specificity, are often used by those of ordinary skill to elucidate the function of particular genes or to distinguish between functions of various members of a biological pathway.

For use in kits and diagnostics, the antisense compounds described herein, either alone or in combination with other compounds or therapeutics, can be used as tools in differential and/or combinatorial analyses to elucidate expression patterns of a portion or the entire complement of genes expressed within cells and tissues. Methods of gene expression analysis are well known to those skilled in the art.

### J. Certain Advantages of Short Antisense Compounds

In certain embodiments, short antisense compounds have advantages when compared to their parent oligonucleotides. For example, in certain embodiments, short antisense compounds have greater affinity for a target nucleic acid than their parent oligonucleotide. In certain embodiments, short antisense compounds have greater potency in vitro than their parent oligonucleotide. In certain such embodiments, that increased in vitro potency is not entirely explained by increased affinity. In certain embodiments, such increased in vitro potency may be attributable to increased ability of short antisense compounds to penetrate cells and/or increased ability to access target nucleic acids in a cell. In certain embodiments, short antisense compounds have greater potency in vivo than their parent oligonucleotides. In certain embodiments, such greater in vivo potency is not attributable to increased in vitro potency or increased affinity. In certain embodiments, short antisense compounds have even greater in vivo potency compared to their parent oligonucleotides than would be predicted based on in vitro potencies or on affinities. In certain embodiments, such increased in vivo potency may be attributable to increased bioavailability, better penetration into the cell, better access to target nucleic acid once in the cell, or other factors.

In certain embodiments, one would expect short antisense compounds to be less specific for their target nucleic acid compared to their parent oligonucleotides. In certain such embodiments, one would expect increased side-effects, including potential for toxic effects, from short antisense compounds. In certain embodiments, such additional side-effects are not observed. In certain embodiments, non-target nucleic acids to which a particular short antisense compound may bind are not available to the short antisense compound. In such embodiments, side-effects, including toxicity, are less problematic than would be predicted.

In certain embodiments, because they are smaller, short antisense compounds are less likely to bind proteins. In certain such embodiments, such less binding of proteins results in lower toxicity, since protein binding may have undesired consequences. In certain embodiments, such less binding of proteins results in greater potency, since it leaves more antisense compound available for therapeutic effect. In certain embodiments, less binding of proteins results in decreased drug-drug interaction toxicity.

### Example 1

### Cell culture and treatment with short antisense compounds

The effect of short antisense compounds on target nucleic acid expression can be tested in any one of a number of cultured or primary cell lines. Cells lines can be obtained from publicly available sources, such as the American Type Culture Collection (Manassas, VA). Cells are cultured according to methods well known to those of ordinary skill in the art.

When cells reached appropriate confluency, they were treated with oligonucleotide using LIPOFECTIN® as described. When cells reached 65-75% confluency, they were treated with oligonucleotide. Oligonucleotide was mixed with LIPOFECTIN® Invitrogen Life Technologies, Carlsbad, CA) in Opti-MEM®-1 reduced serum medium (Invitrogen Life Technologies, Carlsbad, CA) to achieve the desired concentration of oligonucleotide and a LIPOFECTIN® concentration of 2.5 or 3 µg/mL per 100 nM oligonucleotide. This transfection mixture was incubated at room temperature for approximately 0.5 hours. For cells grown in 96-well plates, wells were washed once with 100 µL OPTI-MEM®-1 and then treated with 130 µL of the transfection mixture. Cells grown in 24-well plates or other standard tissue culture plates were treated similarly, using appropriate volumes of medium and oligonucleotide. Cells were treated and data were obtained in duplicate or triplicate. After approximately 4-7 hours of treatment at 37°C, the medium containing the transfection mixture was replaced with fresh culture medium. Cells were harvested 16-24 hours after oligonucleotide treatment.

Control oligonucleotides are used to determine the optimal oligomeric compound concentration for a particular cell line. Furthermore, when oligomeric compounds are tested in oligomeric compound screening experiments or phenotypic assays, control oligonucleotides are tested in parallel.

The concentration of oligonucleotide used varies from cell line to cell line. To determine the optimal oligonucleotide concentration for a particular cell line, the cells are treated with a positive control oligonucleotide at a range of concentrations. The concentration of positive control oligonucleotide that results in 80% inhibition of the target mRNA is then utilized as the screening concentration for new oligonucleotides in subsequent experiments for that cell line. If 80% inhibition is not achieved, the lowest concentration of positive control oligonucleotide that results in 60% inhibition of the target mRNA is then utilized as the oligonucleotide screening concentration in subsequent experiments for that cell line. If 60% inhibition is not achieved, that particular cell line is deemed as unsuitable for oligonucleotide transfection experiments. The concentrations of antisense oligonucleotides used herein are from 50 nM to 300 nM when the antisense oligonucleotide is transfected using a liposome reagent and 1 nM to 40 nM when the antisense oligonucleotide is transfected by electroporation.

### Example 2: Real-time Quantitative PCR Analysis of Target mRNA Levels

Quantitation of target mRNA levels was accomplished by real-time quantitative PCR using the ABI PRISM® 7600, 7700, or 7900 Sequence Detection System (PE-Applied Biosystems, Foster City, CA) according to manufacturer's instructions.

Prior to quantitative PCR analysis, primer-probe sets specific to the target gene being measured were evaluated for their ability to be "multiplexed" with a GAPDH amplification reaction. After isolation the RNA is subjected to sequential reverse transcriptase (RT) reaction and real-time PCR, both of which are performed in the same well. RT and PCR reagents were obtained from Invitrogen Life Technologies (Carlsbad, CA). RT, real-time PCR was carried out in the same by adding 20 µL PCR cocktail (2.5x PCR buffer minus MgCl₂, 6.6 mM MgCl₂, 375 µM each of dATP, dCTP, dCTP and dGTP, 375 nM each of forward primer and reverse primer, 125 nM of probe, 4 Units RNAse inhibitor, 1.25 Units PLATINUM® Taq, 5 Units MuLV reverse transcriptase, and 2.5x ROX dye) to 96-well plates containing 30 µL total RNA solution (20-200 ng). The RT reaction was carried out by incubation for 30 minutes at 48°C. Following a 10 minute incubation at 95°C to activate the PLATINUM® Taq, 40 cycles of a two-step PCR protocol were carried out: 95°C for 15 seconds (denaturation) followed by 60°C for 1.5 minutes (annealing/extension).

Gene target quantities obtained by RT, real-time PCR were normalized using either the expression level of GAPDH, a gene whose expression is constant, or by quantifying total RNA using RiboGreen® (Molecular Probes, Inc. Eugene, OR). GAPDH expression was quantified by RT, real-time PCR, by being run simultaneously with the target, multiplexing, or separately. Total RNA was quantified using RiboGreen® RNA quantification reagent (Molecular Probes, Inc. Eugene, OR).

170 µL of RiboGreen® working reagent (RiboGreen® reagent diluted 1:350 in 10mM Tris-HCl, 1 mM EDTA, pH 7.5) was pipetted into a 96-well plate containing 30 µL purified cellular RNA. The plate was read in a CytoFluor® 4000 (PE Applied Biosystems) with excitation at 485nm and emission at 530nm.

The GAPDH PCR probes have JOE covalently linked to the 5' end and TAMRA or MGB covalently linked to the 3' end, where JOE is the fluorescent reporter dye and TAMRA or MGB is the quencher dye. In some cell types, primers and probe designed to a GAPDH sequence from a different species are used to measure GAPDH expression. For example, a human GAPDH primer and probe set is used to measure GAPDH expression in monkey-derived cells and cell lines.

Probes and primers for use in real-time PCR were designed to hybridize to target nucleic acids using routine methods. For example, PrimerExpress® (Applied Biosystems, Foster City, CA) software is routinely used to design probes and primers for use in real-time PCR. Examples of primer and probe sequences and the target nucleic acids to which they hybridize are presented in Table 4. The target-specific PCR probes have FAM covalently linked to the 5' end and TAMRA or MGB covalently linked to the 3' end, where FAM is the fluorescent dye and TAMRA or MGB is the quencher dye.

**Table 4**

| **Target-specific primers and probes for use in real-time PCR** | | | | |
|---|---|---|---|---|
| **Target Name** | **Species** | **Sequence Description** | **Sequence (5' to 3')** | **SEQ ID NO** |
| ApoB | Mouse | Forward Primer | CGTGGGCTCCAGCATTCTA | 1524 |
| ApoB | Mouse | Reverse Primer | AGTCATTTCTGCCTTTGCGTC | 1525 |
| ApoB | Mouse | Probe | CCAATGGTCGGGCACTGCTCAA | 1526 |
| ApoB | Mouse | Forward Primer | GAAAATAGACTTCCTGAATAACTATGCATT | 1527 |
| ApoB | Mouse | Reverse Primer | ACTCGCTTGCCAGCTTGC | 1528 |
| ApoB | Mouse | Probe | TTTCTGAGTCCCCGTGCCCAACA | 1529 |
| GCGR | Mouse | Forward Primer | TGAGCCTTGCCACCTTCTCT | 1530 |
| GCGR | Mouse | Reverse Primer | GCGCACCCCAGCCAA | 1531 |
| GCGR | Mouse | Probe | AGAGGAGCTTCTTTTCCCTCTACCTGGGC | 1532 |
| GCGR | Mouse | Forward Primer | ATTTCCTGCCCCTGGTACCT | 1533 |
| GCGR | Mouse | Reverse Primer | CGGGCCCACACCTCTTG | 1534 |
| GCGR | Mouse | Probe | CCACAAAGTGCAGCACCGCCTAGTGT | 1535 |
| PTEN | Mouse | Forward Primer | GCCACAGGCTCCCAGACAT | 1536 |
| PTEN | Mouse | Reverse Primer | TCCATCCTCTTGATATCTCCTTTTG | 1537 |
| PTEN | Mouse | Probe | ACAGCCATCATCAAAGAGATCGTTAGCAGAA | 1538 |
| PTEN | Mouse | Forward Primer | ATGACAATCATGTTGCAGCAATTC | 1539 |
| PTEN | Mouse | Reverse Primer | CGATGCAATAAATATGCACAAATCA | 1540 |
| PTEN | Mouse | Probe | CTGTAAAGCTGGAAAGGGACGGACTGGT | 1541 |

### Example 3: Short Antisense Compounds Targeted to an ApoB nucleic acid and having 2'-MOE or methyleneoxy (4'-CH₂-O-2') BNA modifications

Six-week old male Balb/c mice (Jackson Laboratory, Bar Harbor, ME) were injected intraperitoneally (i.p.) with antisense compounds targeted to ApoB, at a frequency of twice per week for three weeks. Antisense compound doses included 2.4, 1.2, 0.6, 0.3 and 0.15 µmol/kg. For antisense compounds 14 nucleotides in length, these doses equate to approximately 12, 6, 3, 1.5 or 0.75 mg/kg, respectively. Shown in Table 5 are the sequences and motifs of the antisense compounds used in this study. The antisense compounds are either 20 or 14 nucleotides in length and have a central "gap" region consisting of ten 2'-deoxynucleotides flanked by wings having 2'-O-methoxyethyl (2'-MOE) or BNA modified "wings." For example, the 2-10-2 MOE gapmer motif indicates an antisense compound with a gap of ten nucleotides flanked by 2 nucleotide wings with 2'-MOE modifications. Bolded residues indicate 2'-O-methoxyethyl moieties and italicized residues indicate methyleneoxy (4'-CH₂-O-2') BNAs. The internucleoside linkages of each compound are phosphorothioate throughout. All cytosine residues of ISIS 147764 and ISIS 372938 are replaced by 5-methyl cytosines. For ISIS 387462, only the cytosine residue in the wing of the compound is replaced by 5-methyl cytosine. ApoB antisense compounds are targeted to publicly available ApoB-100 sequences, including Genbank Accession No. XM_137955.5 (SEQ ID NO: 2).

**Table 5: Antisense Compounds Targeted to an ApoB nucleic acid**

| **ISIS NO** | **Target SEQ ID NO** | **5' Target Site** | **Sequence (5'-3')** | **Gapmer Motif** | **SEQ ID NO** |
|---|---|---|---|---|---|
| 147764 | 2 | 8865 | **GTCCC**TGAAGATGTC**AATGC** | 5-10-5 MOE | 1561 |
| 372938 | 2 | 8235 | **GG**TACATGGAAG**TC** | 2-10-2 MOE | 190 |
| 387462 | 2 | 8235 | GGTACATGGAAGTC | 2-10-2 methyleneoxy (4'-CH₂-O-2') BNA | 190 |

Forty-eight hours following the final injection, mice were sacrificed to evaluate transaminases (Table 6); liver and kidney weight (Table 7); triglyceride, LDL, HDL and free fatty acid levels (Table 8); target mRNA level in liver (Table 9); target protein level in plasma; and oligonucleotide tissue concentration (Table 0). These endpoints were determined using methods described herein and well known to those of ordinary skill in the art.

**Table 6: ALT and AST Levels (IU/L)**

| **ISIS NO** | **Dose µmol/kg** | **ALT** | **AST** |
|---|---|---|---|
| Saline | N/A | 27.8 | 46.3 |
| 147764 | 2.4 | 29.5 | 64.0 |
| 372938 | 2.4 | 26.0 | 49.0 |
| 372938 | 1.2 | 24.8 | 49.5 |
| 372938 | 0.6 | 28.0 | 79.3 |
| 372938 | 0.3 | 28.3 | 60.0 |
| 372938 | 0.15 | 28.3 | 50.3 |
| 387462 | 2.4 | 41.3 | 84.0 |
| 387462 | 1.2 | 35.3 | 63.5 |
| 387462 | 0.6 | 32.0 | 77.3 |
| 387462 | 0.3 | 27.8 | 55.0 |
| 387462 | 0.15 | 29.3 | 68.3 |

**Table 7: Liver and Kidney Weight (% of saline control)**

| **ISIS NO** | **Dose µmol/kg** | **Liver** | **Kidney** |
|---|---|---|---|
| Saline | N/A | 100 | 100 |
| 147764 | 2.4 | 102 | 105 |
| 372938 | 2.4 | 100 | 100 |
| 372938 | 1.2 | 90 | 101 |
| 372938 | 0.6 | 96 | 112 |
| 372938 | 0.3 | 91 | 107 |
| 372938 | 0.15 | 96 | 98 |
| 387462 | 2.4 | 116 | 90 |
| 387462 | 1.2 | 113 | 90 |
| 387462 | 0.6 | 106 | 97 |
| 387462 | 0.3 | 101 | 126 |
| 387462 | 0.15 | 95 | 100 |

Total body weight and food consumption did not differ significantly between saline-treated or oligonucleotide-treated animals. Glucose levels also were similar among all treatment groups.

**Table 8: Triglyceride (TRIG), Total Cholesterol (CHOL), HDL, LDL and Free Fatty Acid (FFA) Levels**

| **ISIS NO** | **Dose µmol/kg** | **TRIG (mg/dL)** | **CHOL (mg/dL)** | **HDL (mg/dL)** | **LDL (mg/dL)** | **FFA (mg/dL)** |
|---|---|---|---|---|---|---|
| Saline | N/A | 167 | 107 | 81.8 | 11.0 | 1.76 |
| 147764 | 2.4 | 167 | 107 | 81.3 | 10.3 | 1.29 |
| 372938 | 2.4 | 153 | 104 | 79.0 | 10.3 | 1.28 |
| 372938 | 1.2 | 136 | 101 | 77.8 | 9.5 | 1.70 |
| 372938 | 0.6 | 184 | 110 | 83.3 | 10.8 | 1.66 |
| 372938 | 0.3 | 138 | 109 | 84.3 | 11.0 | 1.53 |
| 372938 | 0.15 | 151 | 106 | 82.8 | 10.8 | 1.57 |
| 387462 | 2.4 | 49 | 14 | 9.0 | 1.5 | 0.74 |
| 387462 | 1.2 | 71 | 23 | 16.5 | 2.0 | 0.76 |
| 387462 | 0.6 | 150 | 55 | 39.3 | 3.7 | 1.43 |
| 387462 | 0.3 | 136 | 92 | 72.8 | 7.5 | 1.14 |
| 387462 | 0.15 | 163 | 104 | 81.5 | 9.3 | 1.47 |

**Table 9: % ApoB mRNA Level (relative to saline control)**

| **ISIS NO** | **2.4 µmol/kg** | **1.2 µmol/kg** | **0.6 µmol/kg** | **0.3 µmol/kg** | **0.15 µmol/kg** |
|---|---|---|---|---|---|
| 147764 | 57.7 | ND | ND | ND | ND |
| 372938 | 77.0 | 90.0 | 87.3 | 92.6 | 93.1 |
| 387462 | 1.5 | 8.5 | 27.4 | 58.9 | 75.8 |

Treatment with ISIS 387462 resulted in a significant and dose-dependent decrease in triglycerides, total cholesterol, HDL, LDL and free fatty acids. In accordance with these phenotypic findings, treatment with ISIS 387462 also led to a dose-dependent reduction in ApoB mRNA (Table 9) and protein (not shown) levels in mouse plasma. To determine whether the observed increase in efficiency with the methyleneoxy (4'-CH₂-O-2') BNA gapmer is due to an increase in oligonucleotide accumulation, full-length and total oligonucleotide concentration in the liver and kidney were determined.

**Table 10: Full-length and Total Antisense Compound Tissue Concentration (µM) Relative to ApoB mRNA level (% of saline control)**

| **ISIS NO** | **Dose µmol/kg** | **Kidney Full-Length** | **Liver Full-Length** | **Kidney Total** | **Liver Total** | **ApoB mRNA** |
|---|---|---|---|---|---|---|
| 147764 | 2.4 | 28.6 | 22.9 | 33.5 | 31.3 | 58 |
| 372938 | 2.4 | 32.0 | 5.49 | 34.0 | 7.76 | 77 |
| 387462 | 2.4 | 37.2 | 5.69 | 38.9 | 7.31 | 1.5 |
| 387462 | 1.2 | 29.8 | 3.71 | 31.3 | 4.91 | 8.5 |
| 387462 | 0.6 | 18.9 | 1.97 | 20.0 | 2.57 | 27 |
| 387462 | 0.3 | 9.11 | 0.73 | 9.49 | 0.78 | 59 |
| 387462 | 0.15 | 6.97 | 0.19 | 7.43 | 0.24 | 76 |

Levels of the 2-10-2 methyleneoxy (4'-CH₂-O-2') BNA gapmer were similar to the 5-10-5 and 2-10-2 MOE gapmers in the kidney, but significantly reduced in the liver. The EC₅₀ for ISIS 387462 in the liver was determined by comparing oligonucleotide concentration in the liver to inhibition of ApoB mRNA. The approximate EC₅₀ for ISIS 387462 is 1 µM. In contrast, an effective 5-10-5 MOE gapmer compound typically has an EC₅₀ of approximately 15 µM in the liver.

Taken together, these results demonstrate that the ApoB short gapmer having methyleneoxy (4'-CH₂-O-2') in the wings is a potent inhibitor of target mRNA expression and can effectively lower triglycerides, cholesterol and free fatty acids. The potency of the short antisense compound does not appear to be a result of increased tissue accumulation since similar levels of the compound were observed in kidney and reduced levels were found in the liver, relative to the 5-10-5 MOE gapmer. In addition, the methyleneoxy (4'-CH₂-O-2') BNA gapmer exhibited little to no adverse side effects.

### Example 4. Single Dose Administration Dose Response Study with Short Antisense Compounds Targeting ApoB and PTEN nucleic acids

Six-week old male Balb/c mice (Jackson Laboratory, Bar Harbor, ME) were administered a single intraperitoneal (i.p.) injection of antisense compound at a dose of 8, 4, 2 or 1 µmol/kg. Each dose group consisted of four animals. Shown in Table 11 are the sequence, wing chemistry and motif of each antisense compound used in this study. Italicized residues indicate methyleneoxy (4'-CH₂-O-2') BNA modifications, underlined residues indicate *N*-methyl-oxyamino (4'-CH₂-N(CH₃)-O-2') BNA modifications, and boxed residues indicate α-L-methyleneoxy (4'-CH₂-O-2') BNA modifications. All antisense compounds comprise phosphorothioate linkages at each position. Each cytosine of ISIS 116847 and the cytosine residues in the methyleneoxy (4'-CH₂-O-2') BNA wings of ISIS 392063 are replaced with 5-methylcytosines, while the thymidine residues in the methyleneoxy (4'-CH₂-O-2') BNA wings of ISIS 392745 are replaced with 5-methyl thymidines. PTEN antisense compounds target published PTEN nucleic acid, including Genbank Accession No. U92437.1 (SEQ ID NO: 13). ApoB antisense compounds target published ApoB nucleic acid, including Genbank Accession No. XM_137955.5 (SEQ ID NO: 2).

**Table 11: Short Antisense Compounds Targeted to ApoB and PTEN Nucleic Acids**

| **ISIS NO** | **Target** | **Target Seq ID** | **5' Target Site** | **SEQUENCE** | **Gapmer** | **SEQ ID NO** |
|---|---|---|---|---|---|---|
| 387462 | ApoB | 19 | 8235 | *GG*TACATGGAAG*TC* | 2-10-2 Methyleneoxy BNA | 193 |
| 392063 | PTEN | 29 | 3099 | *CT*TAGCACTGGC*CT* | 2-10-2 Methyleneoxy BNA | 1226 |
| 396565 | PTEN | 29 | 3099 | CUTAGCACTGGCCU | 2-10-2 N-Me-oxyamino BNA | 1226 |
| 396006 | PTEN | 29 | 3099 | 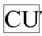 TAGCACTGGC 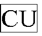 | 2-10-2 α-L-methyleneoxy BNA | 1226 |

**Table 12: %ApoB and PTEN mRNA Reduction (relative to saline control)**

| **ISIS NO** | **Dose (µmol/kg)** | **%ApoB mRNA Reduction (relative to saline)** | **%PTEN mRNA Reduction (relative to saline)** |
|---|---|---|---|
| 387462 | 8 | 0.62 | 92.8 |
| | 4 | 6.55 | 103 |
| | 2 | 18.6 | 105 |
| | 1 | 42.0 | 98.0 |
| 392063 | 8 | 126 | 6.79 |
| | 4 | 111 | 18.1 |
| | 2 | 112 | 42.4 |
| | 1 | 114 | 62.3 |
| 396565 | 8 | 116 | 23.8 |
| | 4 | 1.04 | 46.6 |
| | 2 | 94.4 | 76.1 |
| | 1 | 115 | 89.5 |
| 396006 | 8 | 94.3 | 62.9 |
| | 4 | 101 | 18.2 |
| | 2 | 79.7 | 52.4 |
| | 1 | 111 | 82.4 |

As shown in Table 12, each short antisense compound having Methyleneoxy BNA modifications demonstrated a dose-dependent reduction in target mRNA levels. Notably, the short antisense compound with N-methyl-oxyamino BNA wings (ISIS 396565) also demonstrated dose-dependent reduction in PTEN expression similar to both the β-D-methyleneoxy BNA and α-L-methyleneoxy BNA short antisense compounds. Next, estimated ED₅₀ concentrations for each antisense were calculated using Graphpad Prism. The results are shown below in Table 12.

**Table 13: Estimated ED₅₀ Concentrations**

| **Wing Chemistry** | **ISIS NO** | **ED₅₀ (µmole/kg)** | **ED₅₀ (mg/kg)** |
|---|---|---|---|
| Methyleneoxy BNA | 387462 | 0.8 | 3.9 |
| Methyleneoxy BNA | 392063 | 1.5 | 7 |
| N-Me-oxyamino BNA | 396565 | 3.8 | 17.4 |
| α-L-methyleneoxy BNA | 396006 | 2.1 | 9.3 |

### Example 5: Antisense inhibition of ApoB by short antisense compounds

The short antisense compounds shown in Table 14 were tested for their effects in vivo. Six-week old male Balb/c mice (Jackson Laboratory, Bar Harbor, ME) were administered intraperitoneal doses of 3.2, 1, 0.32, or .1 umol/kg, twice per week for three weeks. A 5-10-5 MOE gapmer was used for a control treatment. Mice were sacrificed approximately 48 hours following the final dose. Liver tissue was collected for RNA isolation, and blood was collected for serum chemistry analyses. ApoB mRNA levels were measured by real-time PCR as described herein. ApoB mRNA levels were normalized to RNA levels as determined by RIBOGREEN, and are presented in Table 15 as percent inhibition relative to ApoB mRNA levels in saline-treated control animals.

**Table 14: Short Antisense Compounds Targeting an ApoB nucleic acid**

| **ISIS NO** | **Sequence (5'-3')** | **Gapmer Motif** | **SEQ ID NO** |
|---|---|---|---|
| 387462 | GGTACATGGAAGTC | 2-10-2 Methyleneoxy BNA | 190 |
| 398296 | GGTACATGGAAGTC | 2-10-2 6'-(*S*)-methyl Methyleneoxy BNA | 190 |

**Table 15: Antisense inhibition of ApoB by Short Antisense Compounds Comprising BNA**

| Isis No | Dose (umol/kg) | % Inhib |
|---|---|---|
| 379818 | 1 | 56 |
| 387462 | 0.1 | 33 |
| | 0.32 | 57 |
| | 1 | 93 |
| | 3.2 | 99 |
| 398296 | 0.1 | 17 |
| | 0.32 | 35 |
| | 1 | 80 |
| | 3.2 | 98 |

Table 15 shows that ApoB mRNA levels were reduced in a dose-dependent manner following treatment with short antisense compounds having a 2-10-2 gapmer motif and BNA modifications in the wings. At the 1 umol/kg dose, ApoB inhibition by the short antisense compounds was greater than observed with a 5-10-5 MOE gapmer at an equivalent dose. Cholesterol was reduced at the 1 and 3.2 umol/kg doses of short antisense compound.

The short antisense compounds exhibited little to no adverse side effects, as judged by organ and body weights, serum transaminases, bilirubin, blood urea nitrogen, and creatinine.

### Example 6: Single dose administration of short antisense compounds comprising BNA modifications

Six-week old male Balb/c mice (Jackson Laboratory, Bar Harbor, ME) were administered a single intraperitoneal injection of short antisense compound at a dose of 8, 4, 2 or 1 µmol/kg. The short antisense compounds tested were ISIS 387462 and ISIS 398296. Each dose group consisted of four animals. A 5-10-5 MOE gapmer was used for a control treatment. Mice were sacrificed approximately 48 hours following the final dose. Liver tissue was collected for RNA isolation, and blood was collected for serum chemistry analyses. ApoB mRNA levels were measured by real-time PCR as described herein. ApoB mRNA levels were normalized to RNA levels as determined by RIBOGREEN, and are presented in Table 16 as percent inhibition relative to ApoB mRNA levels in saline-treated control animals.

**Table 16: Antisense inhibition of ApoB by Short Antisense Compounds Comprising BNA**

| Isis No | Dose (umol/kg) | % Inhib |
|---|---|---|
| 379818 | 8 | 77 |
| 387462 | 8 | 99 |
| | 4 | 93 |
| | 2 | 81 |
| | 1 | 58 |
| 398296 | 8 | 97 |
| | 4 | 81 |
| | 2 | 54 |
| | 1 | 19 |

Table 16 shows that ApoB mRNA levels were reduced in a dose-dependent manner following a single administration of short antisense compounds having a 2-10-2 gapmer motif and BNA modifications in the wings. At the 8 umol/kg dose, ApoB inhibition by the short antisense compounds was greater than observed with a 5-10-5 MOE gapmer at an equivalent dose. The ED₅₀ of ISIS 387462 was 3.9 mg/kg, and the ED₅₀ of ISIS 398296 was 8.7 mg/kg. Cholesterol was also reduced in a dose-dependent manner. Triglycerides were reduced at the highest dose.

The short antisense compounds exhibited little to no adverse side effects, as judged by organ and body weights, serum transaminases, bilirubin, blood urea nitrogen, and creatinine.

In a similar single dose administration study, ISIS 392748, having SEQ ID NO: 1226, a 2-10-2 gapmer motif, where the nucleotides of the wings comprise (6'R)-6'-methyl methyleneoxy BNA modifications, reduced PTEN mRNA in a dose-dependent manner. Additionally, ISIS 392749, having SEQ ID NO: 1226, a 2-10-2 gapmer motif, where the nucleotides of the wings comprise (6'S)-6'-methyl methyleneoxy BNA modifications, reduced PTEN mRNA in a dose-dependent manner. A short antisense compound having 2-10-2 gapmer motifs, the sequence of SEQ ID NO: 1226, and 6-(S)-CH2-O-CH3-BNA modifications also reduced PTEN mRNA in a similar *in vivo* study. A short antisense compound having 2-10-2 gapmer motifs, the sequence of SEQ ID NO: 1226, and 6-(R)-CH2-O-CH3-BNA modifications also reduced PTEN mRNA in a similar *in vivo* study.

### Example 7: Antisense inhibition of ApoB by short antisense compounds comprising palmitic acid conjugates

Six-week old male Balb/c mice (Jackson Laboratory, Bar Harbor, ME) were administered a single intraperitoneal injection of antisense compound at a dose of 2.5, 1.0. 0.4, and 0.16 umol/kg. Each dose group consisted of four animals. The compounds tested are shown in Table 17. A 5-10-5 MOE gapmer was used for a control treatment. Mice were sacrificed approximately 48 hours following the final dose. Liver tissue was collected for RNA isolation, and blood was collected for serum chemistry analyses. ApoB mRNA levels were measured by real-time PCR as described herein. ApoB mRNA levels were normalized to RNA levels as determined by RIBOGREEN, and are presented in Table 18 as percent inhibition relative to ApoB mRNA levels in saline-treated control animals.

**Table 17: Short antisense compounds comprising palmitic conjugates**

| **ISIS NO** | **Sequence (5'-3')** | **Gapmer Motif** | **SEQ ID NO** |
|---|---|---|---|
| 387462 | GGTACATGGAAGTC | 2-10-2 Methyleneoxy BNA | 190 |
| 391871 | GGTACATGGAAGTC | 1-1-10-2 2'-(butylacetomido)-palmitamide/MOE/MOE Unmodified cytosines in gap (i.e., 2-10-2 MOE with 2'-(butylacetomido)-palmitamide substituted at 5' nucleotide | 190 |
| 391872 | GGTACATGGAAGTC | 1-1-10-2 2'-(butylacetomido)-palmitamide Methyleneoxy BNA/Methyleneoxy BNA Unmodified cytosines in gap (i.e., 2-10-2 methyleneoxy BNA with 2'-(butylacetomido)-palmitamide substituted at 5' nucleotide) | 190 |

**Table 18: Antisense inhibition by short antisense compounds comprising palmitic acid conjugates**

| Isis No | Dose (umol/kg) | % Inhib |
|---|---|---|
| 5-10-5 | 2.5 | 54 |
| 387462 | 2.5 | 99 |
| | 1.0 | 91 |
| | 0.4 | 65 |
| | 0.16 | 16 |
| 391871 | 2.5 | 49 |
| | 1.0 | 18 |
| | 0.4 | 5 |
| | 0.16 | 0 |
| 391872 | 2.5 | 99 |
| | 1.0 | 92 |
| | 0.4 | 50 |
| | 0.16 | 18 |

Table 18 shows that ApoB mRNA levels were reduced in a dose-dependent manner following treatment with short antisense compounds having a palmitic acid (C16) conjugate. At the 2.5 umol/kg dose, ApoB inhibition by the short antisense compounds was greater than observed with a 5-10-5 MOE gapmer at an equivalent dose. In this study, the estimated ED₅₀s were 1.5 mg/kg for ISIS 387462, 13.1 mg/kg for ISIS 391871, and 1.9 mg/kg for ISIS 391872. The estimated ED₅₀ for the 5-10-5 MOE gapmer was 17.4 mg/kg. Triglycerides were reduced at the 2.5 and 1.0 mg/kg doses of ISIS 387462 and ISIS 391872. ISIS 387462 and ISIS 391872 markedly reduced total cholesterol, HDL-C and LDL-C in a dose-dependent manner; reduction in LDL-C was so marked that it fell below the limit of detection. Overall, the short antisense compounds exhibited little to no adverse effects.

## Claims

1. A short antisense compound 10 to 14 monomers in length, comprising a 2'-deoxyribonucleotide gap region flanked on each side by a wing, wherein each wing independently comprises 1 to 3 high-affinity modified nucleotides which are sugar-modified nucleotides that comprise a bridge between the 4' and the 2' position of the sugar, and wherein the short antisense compound is targeted to a nucleic acid encoding ApoB,
for use in treating an individual having hypercholesterolemia, familial hypercholesterolemia, heterozygous familial hypercholesterolemia, homozygous familial hypercholesterolemia, non-familial hypercholesterolemia, mixed dyslipidemia, atherosclerosis, a risk of developing atherosclerosis, coronary heart disease, a history of coronary heart disease, early onset coronary heart disease, one or more risk factors for coronary heart disease, type II diabetes, type II diabetes with dyslipidemia, dyslipidemia, hypertriglyceridemia, hyperlipidemia, hyperfattyacidemia, hepatic steatosis, non-alcoholic steatohepatitis, or non-alcoholic fatty liver disease.

2. The compound for use according to claim 1, wherein administration of the short antisense compound decreases total serum cholesterol, ApoB, serum LDL, serum VLDL, serum triglycerides, serum apolipoprotein(a) and/or free fatty acids in the animal.

3. A short antisense compound 10 to 14 monomers in length, comprising a 2'-deoxyribonucleotide gap region flanked on each side by a wing, wherein each wing independently comprises 1 to 3 high-affinity modified nucleotides which are sugar-modified nucleotides that comprise a bridge between the 4' and the 2' position of the sugar, wherein the short antisense compound is targeted to a nucleic acid encoding ApoB, and wherein:
(a) the short antisense compound is 10, 11, 12 or 13 monomers in length;
(b) the short antisense compound further comprises a conjugate group;
(c) the short antisense compound has phosphodiester linkages in the wings and phosphorothioate linkages in the gap; or
(d) the short antisense compound includes one mismatch, two mismatches or three mismatches to the target sequence.

4. The short antisense compound of claim 1 or claim 3, wherein the conformation of each of said sugar-modified nucleotides is, independently, β-D or α-L.

5. The short antisense compound of claim 1 or claim 3, wherein each of said bridges is, independently, 4'-CH₂-2', 4'-(CH₂)₂-2', 4'-CH₂-O-2', 4'-(CH₂)₂-O-2', 4'-CH₂-O-N(R₁)-2' or 4'-CH₂-N(R₁)-O-2'- and wherein each R₁ is, independently, H, a protecting group or C₁-C₁₂ alkyl.

6. The short antisense compound of claim 5, wherein the bridge is 4'-CH₂-O-2'.

7. The short antisense compound of claim 1 or claim 3, wherein the bridge is 4'-CH(CH₃)-O-2'.

8. The short antisense compound of any preceding claim, wherein at least one monomeric linkage is a modified monomeric linkage, such as a phosphorothioate linkage, or wherein each monomeric linkage is a phosphorothioate internucleoside linkage.

9. The short antisense compound of claim 3(b), wherein the conjugate group is selected from the list consisting of: cholesterol, cholic acid, a thiocholesterol, a C₁₆ alkyl, a phospholipid, a polyamine chain, a polyethylene glycol chain, adamantane acetic acid, a palmityl moiety, an octadecylamine moiety, and a hexylamino-carbonyl-oxycholesterol moiety.

10. The short antisense compound of any of claims 1-9 that is 10-13 monomers in length, 10-12 monomers in length, 10-11 monomers in length.

11. The short antisense compound of any of claims 1-9 that is 11 monomers in length.

12. The short antisense compound of any of claims 1-9 that is 12 monomers in length.

13. The short antisense compound of any of claims 1-9 that is 13 monomers in length.

14. The short antisense compound of any of claims 1-9 that is 14 monomers in length.

15. The short antisense compound of any of claims 1-9, having a motif selected from:
(a) 1-12-1; 2-10-2; 1-10-1; 1-10-2; 3-8-3; 2-8-2; 1-8-1; and 3-6-3 wherein the first number represents the number of monomers in the 5'-wing, the second number represents the number of monomers in the gap, and the third number represents the number of monomers in the 3' wing;
(b) 1-1-10-2, 1-1-8-2, 1-1-6-3, and 1-2-8-2, wherein the first number represents the number of monomers in a first 5' wing, the second number represents the number of monomers in a second 5' wing, the third number represents the number of monomers in the gap, and the fourth number represents the number of monomers in the 3' wing;
(c) 2-10-1-1, 2-8-1-1, 3-6-1-1,and 2-8-2-1, wherein the first number represents the number of monomers in the 5' wing, the second number represents the number of monomers in the gap, the third number represents the number of monomers in a first 3' wing, and the fourth number represents the number of monomers in a second 3' wing; and
(d) 1-1-8-1-1; 2-1-6-1-1; and 1-2-8-2-1, wherein the first number represents the number of monomers in a first 5' wing, the second number represents the number of monomers in a second 5' wing, the third number represents the number of monomers in the gap, the fourth number represents the number of monomers in a first 3' wing and the fifth number represents the number of monomers in a second 3' wing.

## Patentansprüche

1. Kurze Antisense-Verbindung mit einer Länge von 10 bis 14 Monomeren, umfassend einen auf jeder Seite von einem Flügel flankierten 2'-Desoxyribonukleotid-Gap-Bereich, wobei die Flügel jeweils unabhängig 1 bis 3 hochaffine modifizierte Nukleotide umfassen, bei denen es sich um zuckermodifizierte Nukleotide handelt, die eine Brücke zwischen der 4'- und der 2'-Position des Zuckers umfassen, und wobei die kurze Antisense-Verbindung auf eine für ApoB codierende Nukleinsäure gerichtet ist,
zur Verwendung bei der Behandlung eines Individuums mit Hypercholesterinämie, familiärer Hypercholesterinämie, heterozygoter familiärer Hypercholesterinämie, homozygoter familiärer Hypercholesterinämie, nichtfamiliärer Hypercholesterinämie, gemischter Dyslipidämie, Atherosklerose, einer Gefahr der Entwicklung von Atherosklerose, koronarer Herzkrankheit, einer Geschichte von koronarer Herzkrankheit, früh auftretender koronarer Herzkrankheit, einem oder mehreren Risikofaktoren für koronare Herzkrankheit, Typ-II-Diabetes, Typ-II-Diabetes mit Dyslipidämie, Dyslipidämie, Hypertriglyceridämie, Hyperlipidämie, Hyper-Fettsäure-Azidämie, Steatosis hepatis, nichtalkoholischer Steatohepatitis oder nichtalkoholischer Fettleberkrankheit.

2. Verbindung zur Verwendung gemäß Anspruch 1, wobei Verabreichung der kurzen Antisense-Verbindung zu einer Abnahme von Gesamtserumcholesterin, ApoB, Serum-LDL, Serum-VLDL, Serumtriglyceriden, Serum-Apolipoprotein(a) und/oder freien Fettsäuren beim Tier führt.

3. Kurze Antisense-Verbindung mit einer Länge von 10 bis 14 Monomeren, umfassend einen auf jeder Seite von einem Flügel flankierten 2'-Desoxyribonukleotid-Gap-Bereich, wobei die Flügel jeweils unabhängig 1 bis 3 hochaffine modifizierte Nukleotide umfassen, bei denen es sich um zuckermodifizierte Nukleotide handelt, die eine Brücke zwischen der 4'- und der 2'-Position des Zuckers umfassen, wobei die kurze Antisense-Verbindung auf eine für ApoB codierende Nukleinsäure gerichtet ist, und wobei:
(a) die kurze Antisense-Verbindung eine Länge von 10, 11, 12 oder 13 Monomeren aufweist;
(b) die kurze Antisense-Verbindung ferner eine Konjugatgruppe umfasst;
(c) die kurze Antisense-Verbindung Phosphodiesterverknüpfungen in den Flügeln und Phosphorothioatverknüpfungen im Gap-Bereich aufweist; oder
(d) die kurze Antisense-Verbindung eine Fehlpaarung, zwei Fehlpaarungen oder drei Fehlpaarungen mit der Zielsequenz enthält.

4. Kurze Antisense-Verbindung nach Anspruch 1 oder Anspruch 3, wobei die zuckermodifizierten Nukleotide jeweils unabhängig in der β-D- oder α-L-Konformation vorliegen.

5. Kurze Antisense-Verbindung nach Anspruch 1 oder Anspruch 3, wobei es sich bei den Brücken jeweils unabhängig um 4'-CH₂-2', 4'-(CH₂)₂-2', 4'-CH₂-O-2', 4'-(CH₂)₂-O-2, 4'-CH₂-O-N(R₁)-2' oder 4'-CH₂-N(R₁)-O-2'- handelt und wobei R₁ jeweils unabhängig für H, eine Schutzgruppe oder C₁-C₁₂-Alkyl steht.

6. Kurze Antisense-Verbindung nach Anspruch 5, wobei es sich bei der Brücke um 4'-CH₂-O-2' handelt.

7. Kurze Antisense-Verbindung nach Anspruch 1 oder Anspruch 3, wobei es sich bei der Brücke um 4'-CH(CH₃)-O-2' handelt.

8. Kurze Antisense-Verbindung nach einem vorhergehenden Anspruch, wobei es sich bei wenigstens einer Monomerverknüpfung um eine modifizierte Monomerverknüpfung wie eine Phosphorothioatverknüpfung handelt oder wobei es sich bei der Monomerverknüpfung jeweils um eine Phosphorothioat-Internukleosidverknüpfung handelt.

9. Kurze Antisense-Verbindung nach Anspruch 3(b), wobei die Konjugatgruppe aus der Liste bestehend aus Cholesterin, Cholsäure, einem Thiocholesterin, einem C₁₆-Alkyl, einem Phospholipid, einer Polyaminkette, einer Polyethylenglykolkette, Adamantanessigsäure, einer Palmitylgruppierung, einer Octadecylamingruppierung und einer Hexylaminocarbonyloxycholesterin-Gruppierung ausgewählt ist.

10. Kurze Antisense-Verbindung nach einem der Ansprüche 1-9, die eine Länge von 10-13 Monomeren, eine Länge von 10-12 Monomeren, eine Länge von 10-11 Monomeren aufweist.

11. Kurze Antisense-Verbindung nach einem der Ansprüche 1-9, die eine Länge von 11 Monomeren aufweist.

12. Kurze Antisense-Verbindung nach einem der Ansprüche 1-9, die eine Länge von 12 Monomeren aufweist.

13. Kurze Antisense-Verbindung nach einem der Ansprüche 1-9, die eine Länge von 13 Monomeren aufweist.

14. Kurze Antisense-Verbindung nach einem der Ansprüche 1-9, die eine Länge von 14 Monomeren aufweist.

15. Kurze Antisense-Verbindung nach einem der Ansprüche 1-9, mit einem Motiv ausgewählt aus:
(a) 1-12-1; 2-10-2; 1-10-1; 1-10-2; 3-8-3; 2-8-2; 1-8-1; und 3-6-3, wobei die erste Zahl die Anzahl der Monomeren im 5'-Flügel, die zweite Zahl die Anzahl der Monomeren im Gap-Bereich und die dritte Zahl die Anzahl der Monomeren im 3'-Flügel repräsentiert;
(b) 1-1-10-2; 1-1-8-2; 1-1-6-3; und 1-2-8-2, wobei die erste Zahl die Anzahl der Monomeren in einem ersten 5'-Flügel, die zweite Zahl die Anzahl der Monomeren in einem zweiten 5'-Flügel, die dritte Zahl die Anzahl der Monomeren im Gap-Bereich und die vierte Zahl die Anzahl der Monomeren im 3'-Flügel repräsentiert;
(c) 2-10-1-1; 2-8-1-1; 3-6-1-1; und 2-8-2-1, wobei die erste Zahl die Anzahl der Monomeren im 5'-Flügel, die zweite Zahl die Anzahl der Monomeren im Gap-Bereich, die dritte Zahl die Anzahl der Monomeren in einem ersten 3'-Flügel und die vierte Zahl die Anzahl der Monomeren in einem zweiten 3'-Flügel repräsentiert; und
(d) 1-1-8-1-1; 2-1-6-1-1; und 1-2-8-2-1, wobei die erste Zahl die Anzahl der Monomeren in einem ersten 5'-Flügel, die zweite Zahl die Anzahl der Monomeren in einem zweiten 5'-Flügel, die dritte Zahl die Anzahl der Monomeren im Gap-Bereich, die vierte Zahl die Anzahl der Monomeren in einem ersten 3'-Flügel und die fünfte Zahl die Anzahl der Monomeren in einem zweiten 3'-Flügel repräsentiert.

## Revendications

1. Composé antisens court de 10 à 14 monomères de longueur, comprenant une région de brèche de 2'-désoxyribonucléotide flanquée de chaque côté par une aile, chaque aile comprenant indépendamment de 1 à 3 nucléotides modifiés à affinité élevée qui sont des nucléotides modifiés par glucide qui comprennent un pont entre la position 4' et 2' du glucide, et le composé antisens court étant ciblé vers un acide nucléique codant pour ApoB,
pour utilisation dans le traitement d'une personne présentant une hypercholestérolémie, une hypercholestérolémie familiale, une hypercholestérolémie familiale hétérozygote, une hypercholestérolémie familiale homozygote, une hypercholestérolémie non familiale, une dyslipidémie mixte, une athérosclérose, un risque de développement d'athérosclérose, une maladie coronarienne, un antécédent de maladie coronarienne, une maladie coronarienne à apparition précoce, un ou plusieurs facteurs de risque pour une maladie coronarienne, un diabète de type II, un diabète de type II avec dyslipidémie, une dyslipidémie, une hypertriglycéridémie, une hyperlipidémie, une hyperglycémie, une stéatose hépatique, une stéatose hépatique non alcoolique, ou une hépatite graisseuse non alcoolique.

2. Composé pour utilisation selon la revendication 1, l'administration du composé antisens court diminuant le cholestérol sérique total, l'ApoB, les β-lipoprotéines sériques, les préβ-lipoprotéines sériques, les triglycérides sériques, l'apolipoprotéine(a) sérique et/ou les acides gras libres chez l'animal.

3. Composé antisens court de 10 à 14 monomères de longueur, comprenant une région de brèche de 2'-désoxyribonucléotide flanquée de chaque côté par une aile, chaque aile comprenant indépendamment de 1 à 3 nucléotides modifiés à affinité élevée qui sont des nucléotides modifiés par glucide qui comprennent un pont entre la position 4' et 2' du glucide, et le composé antisens court étant ciblé vers un acide nucléique codant pour ApoB, et
(a) le composé antisens court est de 10, 11, 12 ou 13 monomères de longueur ;
(b) le composé antisens court comprend en outre un groupe conjugué ;
(c) le composé antisens court a des lieurs phosphodiester dans les ailes et des lieurs phosphorothioate dans la brèche ; ou
(d) le composé antisens court comprend un mésappariement, deux mésappariements ou trois mésappariements par rapport à la séquence cible.

4. Composé antisens court de la revendication 1 ou la revendication 3, la conformation de chacun desdits nucléotides modifiés par glucide étant, indépendamment, β-D ou α-L.

5. Composé antisens court de la revendication 1 ou la revendication 3, chacun desdits ponts étant, indépendamment, 4'-CH₂-2', 4'-(CH₂)₂-2', 4'-CH₂-O-2', 4'-(CH₂)₂-O-2', 4'-CH₂-O-N(R₁)-2' ou 4'-CH₂-N(R₁)-O-2'- et chaque R₁ étant, indépendamment, H, un groupe protecteur ou alkyle en C₁-C₁₂.

6. Composé antisens court de la revendication 5, le pont étant 4'-CH₂-O-2'.

7. Composé antisens court de la revendication 1 ou la revendication 3, le pont étant 4'-CH(CH₃)-O-2'.

8. Composé antisens court de l'une quelconque des revendications précédentes, au moins un lieur monomère étant un lieur monomère modifié, tel qu'un lieur phosphorothioate, ou chaque lieur monomère étant un lieur internucléosidique phosphorothioate.

9. Composé antisens court de la revendication 3(b), le groupe conjugué étant sélectionné dans la liste constituée : du cholestérol, de l'acide cholique, d'un thiocholestérol, d'un alkyle en C₁₆, d'un phospholipide, d'une chaîne polyamine, d'une chaîne polyéthylèneglycol, d'un acide adamantane-acétique, d'un fragment palmityle, d'un fragment octadécylamine et d'un fragment hexylamino-carbonyl-oxycholestérol.

10. Composé antisens court de l'une quelconque des revendications 1 à 9 qui est de 10 à 13 monomères de longueur, de 10 à 12 monomères de longueur, de 10 à 11 monomères de longueur.

11. Composé antisens court de l'une quelconque des revendications 1 à 9 qui est de 11 monomères de longueur.

12. Composé antisens court de l'une quelconque des revendications 1 à 9 qui est de 12 monomères de longueur.

13. Composé antisens court de l'une quelconque des revendications 1 à 9 qui est de 13 monomères de longueur.

14. Composé antisens court de l'une quelconque des revendications 1 à 9 qui est de 14 monomères de longueur.

15. Composé antisens court de l'une quelconque des revendications 1 à 9, ayant un motif choisi parmi :
(a) 1-12-1 ; 2-10-2 ; 1-10-1 ; 1-10-2 ; 3-8-3 ; 2-8-2 ; 1-8-1 ; et 3-6-3 où le premier nombre représente le nombre de monomères dans l'aile 5', le deuxième nombre représente le nombre de monomères dans la brèche, et le troisième nombre représente le nombre de monomères dans l'aile 3' ;
(b) 1-1-10-2, 1-1-8-2, 1-1-6-3 et 1-2-8-2, où le premier nombre représente le nombre de monomères dans une première aile 5', le deuxième nombre représente le nombre de monomères dans une seconde aile 5', le troisième nombre représente le nombre de monomères dans la brèche, et le quatrième nombre représente le nombre de monomères dans l'aile 3' ;
(c) 2-10-1-1, 2-8-1-1, 3-6-1-1 et 2-8-2-1, où le premier nombre représente le nombre de monomères dans l'aile 5', le deuxième nombre représente le nombre de monomères dans la brèche, le troisième nombre représente le nombre de monomères dans une première aile 3', et le quatrième nombre représente le nombre de monomères dans une seconde aile 3' ; et
(d) 1-1-8-1-1, 2-1-6-1-1 et 1-2-8-2-1, où le premier nombre représente le nombre de monomères dans une première aile 5', le deuxième nombre représente le nombre de monomères dans une seconde aile 5', le troisième nombre représente le nombre de monomères dans la brèche, le quatrième nombre représente le nombre de monomères dans une première aile 3', et le cinquième nombre représente le nombre de monomères dans une seconde aile 3'.


### SEQUENCE LISTING

<110> ISIS PHARMACEUTICALS, INC.
<120> COMPOUNDS AND METHODS FOR MODULATING EXPRESSION OF APOB
<130> P058491EP
<150> 60/746,631
   <151> 2006-05-05
<150> 60/747,059
   <151> 2006-05-11
<150> 60/805,660
   <151> 2006-06-23
<150> 60/864,554
   <151> 2006-11-06
<150> PCT/US2007/061183
   <151> 2007-01-27
<150> PCT/US2007/068403
   <151> 2007-05-07
<150> EP07811873.4
   <151> 2007-05-07
<160> 1576
<170> FastSEQ for Windows Version 4.0
<210> 1
   <211> 14121
   <212> DNA
   <213> H. sapiens
<400> 1
<210> 2
   <211> 13928
   <212> DNA
   <213> Mus musculus
<400> 2
   atcagtgcct gcagtggatc aagtacctgc ctgagctccg cctccgaaga ccctgtagag 60
   caagcagcag gggctaggcc cgtggccagg ccacagccag gaagccaccc caccatccat 120
   ccgccatggg cccacgaaag cctgccctgc ggacgccgtt actgctgctg ttcctgctac 180
   tgttcttgga caccagcgtc tgggctcaag atgaagtcct ggaaaactta agcttcagct 240
   gtccaaaaga tgcaactcga ttcaagcacc tccgaaagta cgtgtacaac tatgaagctg 300
   aaagttccag cggtgtccag ggcacagctg actccagaag cgccaccaag atcaactgta 360
   aggtagagct ggaggtcccc caaatctgtg gtttcatcat gaggaccaac cagtgtaccc 420
   ttaaagaggt gtatggcttc aaccctgagg gcaaggcctt gatgaagaaa accaagaact 480
   ctgaagagtt tgcagctgcc atgtccaggt acgaactcaa gctggccatt cctgaaggga 540
   aacaaattgt tctttaccct gacaaggatg aacctaaata tatcctgaac atcaagaggg 600
   gcatcatctc tgctcttctg gttcccccag agacagaaga ggaccaacaa gagttgttcc 660
   tggataccgt gtatggaaac tgctcaactc aggttaccgt gaattcaaga aagggaaccg 720
   taccaacaga aatgtccaca gagagaaacc tgcagcaatg tgacggcttc cagcccatca 780
   gtacaagtgt cagccctctc gctctcatca aaggcctggt ccaccccttg tcaactctta 840
   tcagcagcag ccaaacttgc cagtacaccc tggatcctaa gaggaagcat gtgtctgaag 900
   ctgtctgtga tgagcagcat cttttcctgc ctttctccta caagaataag tatgggatca 960
   tgacacgtgt tacacagaaa ctgagtcttg aagacacacc taagatcaac agtcgcttct 1020
   tcagtgaagg taccaaccgg atgggtctgg cctttgagag caccaagtcc acgtcatccc 1080
   caaagcaggc tgatgctgtt ttgaagaccc ttcaagaact gaaaaaattg tccatctcag 1140
   agcagaatgc tcagagagca aatctcttca ataaactggt tactgagctg agaggcctca 1200
   ctggtgaagc aatcacatcc ctcttgccac agctgattga agtgtccagc cccatcactt 1260
   tacaagcctt ggttcagtgt ggacagccac agtgctatac tcacatcctc cagtggctga 1320
   aaactgagaa ggctcacccc ctcctggttg acattgtcac ctacctgatg gctctgatcc 1380
   caaatccctc aacacagagg ctgcaggaaa tctttaatac tgccaaggag cagcagagcc 1440
   gagccactct gtatgcactg agccacgcag ttaacagcta ttttgatgtg gaccattcaa 1500
   ggagcccagt tctgcaggat atcgctggtt acctgttgaa acagatcgac aatgaatgca 1560
   cgggcaatga agaccacacc ttcttgattc tgagggtcat tggaaatatg ggaagaacca 1620
   tggaacaagt aatgccagcc ctcaagtcct cagtcctgag ctgtgtacga agtacaaaac 1680
   catctctgct gattcagaaa gctgctctcc aggccctgag gaagatggaa ctggaagatg 1740
   aggtccggac gatccttttt gatacatttg taaatggtgt cgctcccgtg gagaagagac 1800
   tggctgccta tctcttgctg atgaagaacc cttcctcatc agatattaac aaaattgccc 1860
   aacttctcca atgggaacag agtgagcagg tgaagaactt cgtggcatct cacattgcca 1920
   acatcttgaa ctcggaagaa ctgtatgtcc aagatctgaa agttttgatc aaaaatgctc 1980
   tggagaattc tcaatttcca acgatcatgg acttcagaaa attttcccga aactatcaga 2040
   tttccaaatc tgcttctctc ccaatgttcg acccagtctc agtcaaaata gaagggaatc 2100
   ttatatttga tccaagcagt tatcttccca gagaaagctt gctgaaaaca accctcacag 2160
   tctttggact tgcttcactt gatctctttg agattggttt agaaggaaaa gggtttgagc 2220
   caacactaga agctcttttt ggtaagcaag gattcttccc agacagtgtc aacaaggctt 2280
   tgtattgggt caatggccga gttccagatg gtgtctccaa ggtcttggtg gaccactttg 2340
   gctatactac agatggcaag catgaacagg acatggtgaa tggaatcatg cccattgtgg 2400
   acaagttgat caaagatctg aaatctaaag aaattcctga agccagggcc tatctccgca 2460
   tcctaggaaa agagctaagc tttgtcagac tccaagacct ccaagtcctg gggaagctgt 2520
   tgctgagtgg tgcacaaact ttgcagggaa tcccccagat ggttgtacag gccatcagag 2580
   aagggtcaaa gaatgacttg tttctccact acatcttcat ggacaatgcc tttgagctcc 2640
   ccactggagc agggttacag ctgcaagtgt cctcgtctgg agtcttcacc cccgggatca 2700
   aggctggtgt aagactggaa ttagccaaca tacaggcaga gctagtggca aagccctctg 2760
   tgtccttgga gtttgtgaca aatatgggca tcatcatccc agacttcgct aagagcagtg 2820
   tccagatgaa caccaacttc ttccacgagt caggcctgga ggcgcgagtg gccctgaagg 2880
   ctgggcagct gaaggtcatc attccttctc caaagaggcc agtcaagctg ttcagtggca 2940
   gcaacacact gcatctggtc tctaccacca aaacagaagt gatcccacct ctggttgaga 3000
   acaggcagtc ctggtcaact tgcaagcctc tcttcactgg aatgaactac tgtaccacag 3060
   gagcttactc caacgccagc tccacggagt ctgcctctta ctacccactg acaggggaca 3120
   caaggtatga gctggagctg aggcccacgg gagaagtgga gcagtattct gccactgcaa 3180
   cctatgaact cctaaaagag gacaagtctt tggttgacac attgaagttc ctagttcaag 3240
   cagaaggagt gcagcagtct gaagctactg tactgttcaa atataatcgg agaagcagga 3300
   ccttatctag tgaagtccta attccagggt ttgatgtcaa cttcgggaca atactaagag 3360
   ttaatgatga atctgctaag gacaaaaaca cttacaaact catcctggac attcagaaca 3420
   agaaaatcac tgaggtctct ctcgtgggcc acttgagtta tgataaaaag ggagatggca 3480
   agatcaaagg tgttgtttcc ataccacgtt tgcaagcaga agccaggagt gaggtccaca 3540
   cccactggtc ctccaccaaa ctgctcttcc aaatggactc atctgctaca gcttacggct 3600
   caacaatttc caagagagtg acatggcgtt acgataatga gataatagaa tttgattgga 3660
   acacgggaac caatgtggat accaaaaaag tggcctccaa tttccctgtg gatctttccc 3720
   attatcctag aatgttgcat gagtatgcca atggtctcct ggatcacaga gtccctcaaa 3780
   cagatgtgac ttttcgggac atgggttcca aattaattgt tgcaacaaac acatggcttc 3840
   agatggcaac caggggtctt ccttaccccc aaactctaca ggatcacctc aatagcctct 3900
   cagagttgaa cctcctgaaa atgggactgt ctgacttcca tattccagac aacctcttcc 3960
   taaagactga tggcagagtc aaatacacaa tgaacaggaa caaaataaac attgacatcc 4020
   ctttgccttt gggtggcaag tcttcaaaag acctcaagat gccagagagt gtgaggacac 4080
   cagccctcaa cttcaagtct gtgggattcc atctgccatc tcgagaggtc caggtcccca 4140
   cttttacaat ccccaagaca catcagcttc aagtgcctct cttgggtgtt ctagaccttt 4200
   ccacaaatgt ctacagcaat ttgtacaact ggtcagcctc ctacactggt ggcaacacca 4260
   gcagagacca cttcagcctt caggctcagt accgcatgaa gactgactct gtggttgacc 4320
   tgttttccta cagtgtgcaa ggatctggag aaacaacata tgacagcaag aacacattta 4380
   cattgtcctg tgatggatct ctacaccata aatttctaga ctcaaaattc aaagtcagcc 4440
   acgtagaaaa atttggaaac agcccagtct caaaaggttt actaacattt gaaacatcta 4500
   gtgccttggg accacagatg tctgctactg ttcacctaga ctcaaaaaag aaacaacatc 4560
   tatacgtcaa agatatcaag gttgatggac agttcagagc ttcttcattt tatgctcaag 4620
   gcaaatatgg cctgtcttgt gagagagatg ttacaactgg ccagctgagc ggcgaatcca 4680
   acatgagatt taactccacc tacttccagg gcaccaacca gatcgtggga atgtaccagg 4740
   atggagccct gtccatcacc tccacttctg acctgcaaga tggcatattc aagaacacag 4800
   cttccttgaa atatgaaaac tatgagctga ctctgaaatc tgatagcagt gggcagtatg 4860
   agaacttcgc tgcttccaac aagctggatg tgaccttctc tacgcaaagt gcactgctgc 4920
   gttctgaaca ccaggccaat tacaagtccc tgaggcttgt cacccttctt tcaggatccc 4980
   tcacttccca gggtgtagaa ttaaatgctg acatcttggg cacagacaaa attaatactg 5040
   gtgctcacaa ggcaacacta aagattgcac gtgatggact atcaaccagt gcgaccacca 5100
   acttgaagta cagccccctg ctgctggaga atgagttgaa tgcagagctt gggctctctg 5160
   gggcatccat gaaattatca acaaacggcc gcttcaaaga acaccatgca aaattcagtc 5220
   ttgatgggag agctgccctc acagaggtgt cactggggag catttaccag gccatgattc 5280
   tgggtgcaga cagcaaaaac atcttcaact tcaaactcag ccgagaaggg ctgaggctgt 5340
   ccaatgattt gatgggctcc tatgctgaga tgaaacttga ccacacacac agtctgaaca 5400
   ttgcaggtct ctcactggac ttcttctcaa aaatggacaa tatttacagt ggagacaagt 5460
   tctataagca gaattttaac ttacagctac agccctattc tttcataact actttaagca 5520
   acgacctgag atatggtgct ctagatttga ccaacaatgg aaggtttcgg ctggagccac 5580
   tgaagctgaa tgtgggtggc aactttaaag gaacctatca aaataatgag ctgaaacata 5640
   tctataccat atcttatact gacctggtag tagcaagtta cagagcagac actgtggcta 5700
   aggttcaggg tgtcgaattc agccataggc taaatgcaga cattgaagga ctgacttcct 5760
   ctgttgatgt cactaccagc tacaattcag atccactgca ttttaacaat gttttccact 5820
   tttctctggc accttttacc ttgggcatcg acacacatac aagtggtgat gggaaactgt 5880
   ccttctgggg agaacacact gggcagctat atagtaagtt tctgttgaaa gcagaacctc 5940
   tggcacttat tgtctctcat gactacaaag gatccacaag ccacagtctc ccgtacgaga 6000
   gcagcatcag cacggctctt gaacacacag tcagtgcctt gctgacgcca gctgagcaga 6060
   caagcacctg gaaattcaag accaaactga atgacaaagt atacagccag gactttgaag 6120
   cctacaacac taaagacaaa atcggtgttg agcttagtgg acgggctgac ctctctgggc 6180
   tgtattctcc aattaaacta ccgtttttct acagtgagcc tgtcaatgtc cttaatggct 6240
   tagaggtaaa tgatgctgtt gacaagcccc aagaattcac aattattgct gtggtgaagt 6300
   acgataagaa ccaggatgtt cacaccatca acctcccatt cttcaaaagc ctgccagact 6360
   atttggagag aaatcgaaga ggaatgataa gtctactgga agccatgcga ggggaattgc 6420
   aacgcctcag tgttgatcag tttgtgagga aatacagagc ggccctgagc agacttcctc 6480
   agcagattca tcattatctg aatgcatctg actgggagag acaagtagct ggtgccaagg 6540
   aaaaaataac ttctttcatg gaaaattata gaattacaga taatgatgta ctaattgcca 6600
   tagatagtgc caaaatcaac ttcaatgaaa aactctctca acttgagaca tacgcgatac 6660
   aatttgatca gtatattaaa gataattatg atccacatga cttaaaaaga actattgctg 6720
   agattattga tcgaatcatt gaaaagttaa aaattcttga tgaacagtat catatccgtg 6780
   taaatctagc aaaatcaatc cataatctct atttatttgt tgaaaacgtt gatcttaacc 6840
   aagtcagtag tagtaacacc tcttggatcc aaaatgtgga ttccaattat caagtcagaa 6900
   tccaaattca agaaaaacta cagcagctca ggacacaaat tcagaatata gacattcagc 6960
   agcttgctgc agaggtaaaa cgacagatgg acgctattga tgtcacaatg catttagatc 7020
   aattgagaac tgcaattcta ttccaaagaa taagtgacat tattgaccgt gtcaaatact 7080
   ttgttatgaa tcttattgaa gattttaaag taactgagaa aatcaatact tttagagtta 7140
   tagtccgtga gctaattgag aaatatgaag tagaccaaca catccaggtt ttaatggata 7200
   aatcagtaga gttggcccac agatatagcc tgagcgagcc tcttcagaaa ctcagtaatg 7260
   tgctacagcg aattgagata aaagattact atgagaaatt ggttgggttt attgatgata 7320
   ctgttgagtg gcttaaagca ttgtctttca aaaataccat tgaagaacta aatagattga 7380
   ctgacatgtt ggtgaagaag ttgaaagcat ttgattatca ccagtttgta gacaaaacca 7440
   acagcaaaat ccgtgagatg actcagagaa tcaatgctga aatccaagct ctcaaacttc 7500
   cacaaaaaat ggaagcatta aaactgttgg tagaagactt caaaaccaca gtctccaatt 7560
   ccctggaaag actcaaggac accaaagtaa ctgtggtcat tgattggctg caggatattt 7620
   tgactcaaat gaaagaccat ttccaagata ctctggaaga tgtaagagac cgaatttatc 7680
   aaatggacat tcagagggaa ctggagcact tcttgtctct ggtaaaccaa gtttacagta 7740
   cactggtcac ctatatgtct gactggtgga ctctgactgc taaaaacata acagactttg 7800
   cagagcaata ttccatccaa aactgggctg agagtataaa agtactggtg gaacaaggat 7860
   tcatagttcc tgaaatgcaa acatttctgt ggaccatgcc tgcttttgag gtcagtctcc 7920
   gtgctctcca agaaggtaac tttcagaccc ctgtctttat agtccccttg acagatttga 7980
   ggattccatc aattcggata aactttaaaa tgttaaagaa tataaaaatc ccattgagat 8040
   tttccactcc agaattcact cttctcaaca ccttccatgt ccattccttt acaattgact 8100
   tgctggaaat aaaagcaaag atcattagaa ctatcgacca aattttgagc agtgagctac 8160
   agtggcctct tccagaaatg tatttgagag acctggatgt agtgaacatt cctcttgcaa 8220
   gactgactct gccagacttc catgtaccag aaatcacaat tccagaattc acaatcccaa 8280
   atgtcaatct caaagattta cacgttcctg atcttcacat accagaattc caacttcctc 8340
   acctctcaca tacaattgaa atacctgctt ttggcaaact gcatagcatc cttaagatcc 8400
   aatctcctct ctttatatta gatgctaatg ccaacataca gaatgtaaca acttcaggga 8460
   acaaagcaga gattgtggct tctgtcactg ctaaaggaga gtcccaattt gaagctctca 8520
   attttgattt tcaagcacaa gctcaattcc tggagttaaa tcctcatcct ccagtcctga 8580
   aggaatccat gaacttctcc agtaagcatg tgagaatgga gcatgagggt gagatagtat 8640
   ttgatggaaa ggccattgag gggaaatcag acacagtcgc aagtttacac acagagaaaa 8700
   atgaagtaga gtttaataat ggtatgactg tcaaagtaaa caatcagctc acccttgaca 8760
   gtcacacaaa gtacttccac aagttgagtg ttcctaggct ggacttctcc agtaaggctt 8820
   ctcttaataa tgaaatcaag acactattag aagctggaca tgtggcattg acatcttcag 8880
   ggacagggtc atggaactgg gcctgtccca acttctcgga tgaaggcata cattcgtccc 8940
   aaattagctt tactgtggat ggtcccattg cttttgttgg actatccaat aacataaatg 9000
   gcaaacactt acgggtcatc caaaaactga cttatgaatc tggcttcctc aactattcta 9060
   agtttgaagt tgagtcaaaa gttgaatctc agcacgtggg ctccagcatt ctaacagcca 9120
   atggtcgggc actgctcaag gacgcaaagg cagaaatgac tggtgagcac aatgccaact 9180
   taaatggaaa agttattgga actttgaaaa attctctctt cttttcagca caaccatttg 9240
   agattactgc atccacaaat aatgaaggaa atttgaaagt gggttttcca ctaaagctga 9300
   ctgggaaaat agacttcctg aataactatg cattgtttct gagtccccgt gcccaacaag 9360
   caagctggca agcgagtacc agattcaatc agtacaaata caatcaaaac ttttctgcta 9420
   taaacaatga acacaacata gaagccagta taggaatgaa tggagatgcc aacctggatt 9480
   tcttaaacat acctttaaca attcctgaaa ttaacttgcc ttacacggag ttcaaaactc 9540
   ccttactgaa ggatttctcc atatgggaag aaacaggctt gaaagaattt ttgaagacaa 9600
   caaagcaatc atttgatttg agtgtaaagg ctcaatataa aaagaacagt gacaagcatt 9660
   ccattgttgt ccctctgggt atgttttatg aatttattct caacaatgtc aattcgtggg 9720
   acagaaaatt tgagaaagtc agaaacaatg ctttacattt tcttaccacc tcctataatg 9780
   aagcaaaaat taaggttgat aagtacaaaa ctgaaaattc ccttaatcag ccctctggga 9840
   cctttcaaaa tcatggctac actatcccag ttgtcaacat tgaagtatct ccatttgctg 9900
   tagagacact ggcttccagc catgtgatcc ccacagcaat aagcaccccc agtgtcacaa 9960
   tccctggtcc taacatcatg gtgccttcat acaagttagt gctgccaccc ctggagttgc 10020
   cagttttcca tggtcctggg aatctattca agtttttcct cccagatttc aagggattca 10080
   acactattga caatatttat attccagcca tgggcaactt tacctatgac ttttctttta 10140
   aatcaagtgt catcacactg aataccaatg ctggacttta taaccaatca gatatcgttg 10200
   cccatttcct ttcttcctct tcatttgtca ctgacgccct gcagtacaaa ttagagggaa 10260
   catcacgtct gatgcgaaaa aggggattga aactagccac agctgtctct ctaactaaca 10320
   aatttgtaaa gggcagtcat gacagcacca ttagtttaac caagaaaaac atggaagcat 10380
   cagtgagaac aactgccaac ctccatgctc ccatattctc aatgaacttc aagcaggaac 10440
   ttaatggaaa taccaagtca aaacccactg tttcatcatc cattgaacta aactatgact 10500
   tcaattcctc aaagctgcac tctactgcaa caggaggcat tgatcacaag ttcagcttag 10560
   aaagtctcac ttcctacttt tccattgagt cattcaccaa aggaaatatc aagagttcct 10620
   tcctttctca ggaatattca ggaagtgttg ccaatgaagc caatgtatat ctgaattcca 10680
   agggtactcg gtcttcagtg aggctacaag gagcttccaa agttgatggt atctggaacg 10740
   ttgaagtagg agaaaatttt gctggagaag ccaccctcca acgcatctac accacatggg 10800
   agcacaatat gaaaaaccat ttgcaggtat atagctactt cttcacaaaa ggaaagcaaa 10860
   catgcagagc tactttggag ctctccccat ggaccatgtc aaccttgcta caggttcatg 10920
   tgagtcaact cagttccctc cttgacctcc atcactttga ccaggaagtg atcctaaaag 10980
   ctaacactaa gaaccagaag atcagctgga aaggtggggt ccaggttgaa tcacgggttc 11040
   ttcagcacaa tgcacagttc tccaatgacc aagaagaaat acggcttgac cttgcaggat 11100
   ccttagacgg acagctgtgg gaccttgaag ctatcttttt accagtatat ggcaagagct 11160
   tgcaggaact cctacaaatg gatggaaagc gacagtatct tcaagcttca acttctcttc 11220
   tatataccaa aaaccctaat ggctatctcc tctcactccc cgtgcaagaa ctggctgata 11280
   gatttattat accagggata aaactaaatg acttcagtgg agtaaaaatc tataagaagt 11340
   taagtacttc accatttgcc ctcaacctaa caatgctccc caaagtaaaa ttccctggga 11400
   ttgatctgtt aacacagtac tctacaccag agggctcctc tgtccctatt tttgaggcaa 11460
   ctatacctga aattcattta actgtatccc agtttacact tccaaagagc cttccagttg 11520
   gcaacacagt ctttgatctg aataagttgg ccaacatgat tgccgatgtt gacctgccta 11580
   gtgtcaccct gcctgagcag actattgtaa tcccaccctt ggagttctct gtacctgctg 11640
   ggatttttat tcctttcttt ggagaactga ctgcacgtgc tgggatggct tctcccctgt 11700
   ataatgtcac ttggagcgct ggttggaaaa ccaaagcaga tcatgttgaa acgttcctag 11760
   attccatgtg cacttcaacc ttgcagtttc tggagtatgc tttaaaagtt gtagaaacac 11820
   acaaaattga agaagatctg ttaacctata atatcaaagg aacacttcaa cactgtgact 11880
   tcaatgtgga gtataatgaa gatggtctat ttaaaggact ttgggactgg cagggagagg 11940
   ctcacctgga catcaccagc ccagcactga ctgactttca tctgtactac aaagaagaca 12000
   agacaagtct gtctgcctca gcagcctcct cgaccatcgg cactgtgggt ctggattcga 12060
   gcacagatga ccagagtgtg gagctgaatg tctacttcca cccacagtcc cctccagaga 12120
   agaaactcag catattcaaa actgagtgga ggtacaagga gtctgatggt gaaaggtaca 12180
   tcaaaattaa ttgggaagaa gaggcagctt ccagattgct aggctcccta aaaagcaatg 12240
   tgcccaaggc ttctaaggct atttatgatt atgccaataa gtaccacctg gaatacgttt 12300
   cttcagaact aagaaaaagt ctacaggtca atgctgaaca tgccagaagg atggttgatg 12360
   aaatgaacat gagtttccag agagtagccc gtgataccta ccagaatctc tatgaggaga 12420
   tgttggctca gaagagcctg agcatccctg agaatctcaa gaagagggtg ttagacagta 12480
   tagtacatgt tactcagaag taccacatgg cagtcatgtg gctgatggac tcattcattc 12540
   attttctgaa attcaataga gtccagttcc cagggtacgc tggaacatat actgtggacg 12600
   aactctacac tatagtcatg aaggaaacca agaagtcact gtctcagctg tttaatgggt 12660
   taggaaacct actttcctac gttcaaaacc aagtagagaa atcaagatta atcaatgaca 12720
   taacatttaa atgtcctttt ttctcaaaac cttgtaaact aaaagatctc atattgattt 12780
   tcagggagga gttaaacatt ttatcaaaca taggccaaca ggatatcaag tttacaacaa 12840
   tactaagtag tcttcagggc tttttggaga gagttttaga catcatagaa gaacaaatta 12900
   aatgcctaaa ggacaatgaa tctacttgtg ttgctgacca tatcaacatg gttttcaaaa 12960
   tacaggtccc atatgctttt aaatccctaa gagaagacat atactttgtc ctcggtgagt 13020
   tcaatgactt tcttcaatcc atacttcagg aggggtccta caagctacag caggtccatc 13080
   agtatatgaa ggcccttcgt gaagagtatt ttgatccgag catggttggg tggacagtga 13140
   aatattatga aatagaagaa aatatggttg agctgatcaa gaccctttta gtttccttta 13200
   gggatgtcta ctctgaatat agtgtgacag ctgctgattt tgcttccaaa atgtcaactc 13260
   aagttgaaca atttgtgtcc agggatatca gagagtatct tagcatgctt actgatataa 13320
   atggaaagtg gatggaaaag attgcagagc tttctattgt ggcaaaggaa acaatgaaaa 13380
   gctgggtcac tgccgtggcc aaaataatgt ctgattaccc ccagcagttc cactccaatc 13440
   tgcaggattt ttcagaccaa ctctctagct actatgaaaa atttgttggt gagtccacaa 13500
   gattgattga cctgtccatt caaaactacc acgtgtttct cagatacatc accgagttac 13560
   tgagaaagct gcaggtggcc acagccaata atgtgagccc ctatataaag cttgctcaag 13620
   gagagctgat gatcaccttc tgattcatct actaacaaat tcaaattaaa ccttcacata 13680
   gtaggagact ttgtagacta ctataaagac catcctgagc cagacctgca gtcaacagca 13740
   agagcaagaa gcacatagga actatacctg caaccaagct ggcataagaa ccaagacctt 13800
   caaagcagcc tgaactcaag atgacatatt ttacaagtta gagtaaagtc aagagctgag 13860
   ttgttttgtc caactcagga tggagggagg gagggaaggg gaaataaata aatacttcct 13920
   tattgtgc 13928
<210> 3
   <211> 2273
   <212> DNA
   <213> H. sapiens
<400> 3
   gggggcagat cctggggaga atggaggagc acacagaggc aggctcggca ccagagatgg 60
   gggcccagaa ggccctgatt gacaatcctg ctgacatcct agtcattgct gcatatttcc 120
   tgctggtcat tggcgttggc ttgtggtcca tgtgcagaac caacagaggc actgtgggcg 180
   gctacttcct ggcaggacgc agcatggtgt ggtggccggt tggggcctct ctcttcgcca 240
   gcaacatcgg cagtggccac tttgtgggcc tggcagggac tggcgctgca agtggcttgg 300
   ctgttgctgg attcgagtgg aatgcgctct tcgtggtgct gctactgggc tggctgtttg 360
   cacccgtgta cctgacagcg ggggtcatca cgatgccaca gtacctgcgc aagcgcttcg 420
   gcggccgccg catccgcctc tacctgtctg tgctctccct tttcctgtac atcttcacca 480
   agatctcagt ggacatgttc tccggagctg tattcatcca gcaggctctg ggctggaaca 540
   tctatgcctc cgtcatcgcg cttctgggca tcaccatgat ttacacggtg acaggagggc 600
   tggccgcgct gatgtacacg gacacggtac agaccttcgt cattctgggg ggcgcctgca 660
   tcctcatggg ttacgccttc cacgaggtgg gcgggtattc gggtctcttc gacaaatacc 720
   tgggagcagc gacttcgctg acggtgtccg aggatccagc cgtgggaaac atctccagct 780
   tctgctatcg accccggccc gactcctacc acctgctccg gcaccccgtg accggggatc 840
   tgccgtggcc cgcgctgctc ctcggactca caatcgtctc gggctggtac tggtgcagcg 900
   accaggtcat cgtgcagcgc tgcctggccg ggaagagcct gacccacatc aaggcgggct 960
   gcatcctgtg tgggtacctg aagctgacgc ccatgtttct catggtcatg ccaggcatga 1020
   tcagccgcat tctgtaccca gacgaggtgg cgtgcgtggt gcctgaggtg tgcaggcgcg 1080
   tgtgcggcac ggaggtgggc tgctccaaca tcgcctaccc gcggctcgtc gtgaagctca 1140
   tgcccaacgg tctgcgcgga ctcatgctgg cggtcatgct ggccgcgctc atgtcctcgc 1200
   tggcctccat cttcaacagc agcagcacgc tcttcaccat ggacatctac acgcgcctgc 1260
   ggccacgcgc cggcgaccgc gagctgctgc tggtgggacg gctctgggtg gtgttcatcg 1320
   tggtagtgtc ggtggcctgg cttcccgtgg tgcaggcggc acagggcggg cagctcttcg 1380
   attacatcca ggcagtctct agctacctgg caccgcccgt gtccgccgtc ttcgtgctgg 1440
   cgctcttcgt gccgcgcgtt aatgagcagg gcgccttctg gggactcatc gggggcctgc 1500
   tgatgggcct ggcacgcctg attcccgagt tctccttcgg ctcgggcagc tgtgtgcagc 1560
   cctcggcgtg cccagctttc ctctgcggcg tgcactacct ctacttcgcc attgtgctgt 1620
   tcttctgctc tggcctcctc accctcacgg tctccctgtg caccgcgccc atccccagaa 1680
   agcacctcca ccgcctggtc ttcagtctcc ggcatagcaa ggaggaacgg gaggacctgg 1740
   atgctgatga gcagcaaggc tcctcactcc ctgtacagaa tgggtgccca gagagtgcca 1800
   tggagatgaa tgagccccag gccccggcac caagcctctt ccgccagtgc ctgctctggt 1860
   tttgtggaat gagcagaggt ggggtgggca gtcctccgcc ccttacccag gaggaggcag 1920
   cggcagcagc caggcggctg gaggacatca gcgaggaccc gagctgggcc cgtgtggtca 1980
   acctcaatgc cctgctcatg atggcagtgg ccgtgttcct ctggggcttc tatgcctaag 2040
   accaactgcg ttggacacca taagccacag cctcacagga agtgggggtg aggagcctgc 2100
   ggtgctcccc agaaaagggg aaggggcagt ggggtgagaa ggtcctggct ccccttctcc 2160
   cggccttcct ctgcctgggg cccactgcat ctgattggca gtcacttccc atgagggcct 2220
   ggcccacccg ctgcagttgc cctaaggaaa aataaagctg cctttcccct gta        2273
<210> 4
   <211> 3636
   <212> DNA
   <213> H. sapiens
<400> 4
   cagcgacgtc gaggcgctca tggttgcagg cgggcgccgc cgttcagttc agggtctgag 60
   cctggaggag tgagccaggc agtgagactg gctcgggcgg gccgggacgc gtcgttgcag 120
   cagcggctcc cagctcccag ccaggattcc gcgcgcccct tcacgcgccc tgctcctgaa 180
   cttcagctcc tgcacagtcc tccccaccgc aaggctcaag gcgccgccgg cgtggaccgc 240
   gcacggcctc taggtctcct cgccaggaca gcaacctctc ccctggccct catgggcacc 300
   gtcagctcca ggcggtcctg gtggccgctg ccactgctgc tgctgctgct gctgctcctg 360
   ggtcccgcgg gcgcccgtgc gcaggaggac gaggacggcg actacgagga gctggtgcta 420
   gccttgcgtt ccgaggagga cggcctggcc gaagcacccg agcacggaac cacagccacc 480
   ttccaccgct gcgccaagga tccgtggagg ttgcctggca cctacgtggt ggtgctgaag 540
   gaggagaccc acctctcgca gtcagagcgc actgcccgcc gcctgcaggc ccaggctgcc 600
   cgccggggat acctcaccaa gatcctgcat gtcttccatg gccttcttcc tggcttcctg 660
   gtgaagatga gtggcgacct gctggagctg gccttgaagt tgccccatgt cgactacatc 720
   gaggaggact cctctgtctt tgcccagagc atcccgtgga acctggagcg gattacccct 780
   ccacggtacc gggcggatga ataccagccc cccgacggag gcagcctggt ggaggtgtat 840
   ctcctagaca ccagcataca gagtgaccac cgggaaatcg agggcagggt catggtcacc 900
   gacttcgaga atgtgcccga ggaggacggg acccgcttcc acagacaggc cagcaagtgt 960
   gacagtcatg gcacccacct ggcaggggtg gtcagcggcc gggatgccgg cgtggccaag 1020
   ggtgccagca tgcgcagcct gcgcgtgctc aactgccaag ggaagggcac ggttagcggc 1080
   accctcatag gcctggagtt tattcggaaa agccagctgg tccagcctgt ggggccactg 1140
   gtggtgctgc tgcccctggc gggtgggtac agccgcgtcc tcaacgccgc ctgccagcgc 1200
   ctggcgaggg ctggggtcgt gctggtcacc gctgccggca acttccggga cgatgcctgc 1260
   ctctactccc cagcctcagc tcccgaggtc atcacagttg gggccaccaa tgcccaagac 1320
   cagccggtga ccctggggac tttggggacc aactttggcc gctgtgtgga cctctttgcc 1380
   ccaggggagg acatcattgg tgcctccagc gactgcagca cctgctttgt gtcacagagt 1440
   gggacatcac aggctgctgc ccacgtggct ggcattgcag ccatgatgct gtctgccgag 1500
   ccggagctca ccctggccga gttgaggcag agactgatcc acttctctgc caaagatgtc 1560
   atcaatgagg cctggttccc tgaggaccag cgggtactga cccccaacct ggtggccgcc 1620
   ctgcccccca gcacccatgg ggcaggttgg cagctgtttt gcaggactgt atggtcagca 1680
   cactcggggc ctacacggat ggccacagcc gtcgcccgct gcgccccaga tgaggagctg 1740
   ctgagctgct ccagtttctc caggagtggg aagcggcggg gcgagcgcat ggaggcccaa 1800
   gggggcaagc tggtctgccg ggcccacaac gcttttgggg gtgagggtgt ctacgccatt 1860
   gccaggtgct gcctgctacc ccaggccaac tgcagcgtcc acacagctcc accagctgag 1920
   gccagcatgg ggacccgtgt ccactgccac caacagggcc acgtcctcac aggctgcagc 1980
   tcccactggg aggtggagga ccttggcacc cacaagccgc ctgtgctgag gccacgaggt 2040
   cagcccaacc agtgcgtggg ccacagggag gccagcatcc acgcttcctg ctgccatgcc 2100
   ccaggtctgg aatgcaaagt caaggagcat ggaatcccgg cccctcagga gcaggtgacc 2160
   gtggcctgcg aggagggctg gaccctgact ggctgcagtg ccctccctgg gacctcccac 2220
   gtcctggggg cctacgccgt agacaacacg tgtgtagtca ggagccggga cgtcagcact 2280
   acaggcagca ccagcgaagg ggccgtgaca gccgttgcca tctgctgccg gagccggcac 2340
   ctggcgcagg cctcccagga gctccagtga cagccccatc ccaggatggg tgtctgggga 2400
   gggtcaaggg ctggggctga gctttaaaat ggttccgact tgtccctctc tcagccctcc 2460
   atggcctggc acgaggggat ggggatgctt ccgcctttcc ggggctgctg gcctggccct 2520
   tgagtggggc agcctccttg cctggaactc actcactctg ggtgcctcct ccccaggtgg 2580
   aggtgccagg aagctccctc cctcactgtg gggcatttca ccattcaaac aggtcgagct 2640
   gtgctcgggt gctgccagct gctcccaatg tgccgatgtc cgtgggcaga atgactttta 2700
   ttgagctctt gttccgtgcc aggcattcaa tcctcaggtc tccaccaagg aggcaggatt 2760
   cttcccatgg ataggggagg gggcggtagg ggctgcaggg acaaacatcg ttggggggtg 2820
   agtgtgaaag gtgctgatgg ccctcatctc cagctaactg tggagaagcc cctgggggct 2880
   ccctgattaa tggaggctta gctttctgga tggcatctag ccagaggctg gagacaggtg 2940
   cgcccctggt ggtcacaggc tgtgccttgg tttcctgagc cacctttact ctgctctatg 3000
   ccaggctgtg ctagcaacac ccaaaggtgg cctgcgggga gccatcacct aggactgact 3060
   cggcagtgtg cagtggtgca tgcactgtct cagccaaccc gctccactac ccggcagggt 3120
   acacattcgc acccctactt cacagaggaa gaaacctgga accagagggg gcgtgcctgc 3180
   caagctcaca cagcaggaac tgagccagaa acgcagattg ggctggctct gaagccaagc 3240
   ctcttcttac ttcacccggc tgggctcctc atttttacgg gtaacagtga ggctgggaag 3300
   gggaacacag accaggaagc tcggtgagtg atggcagaac gatgcctgca ggcatggaac 3360
   tttttccgtt atcacccagg cctgattcac tggcctggcg gagatgcttc taaggcatgg 3420
   tcgggggaga gggccaacaa ctgtccctcc ttgagcacca gccccaccca agcaagcaga 3480
   catttatctt ttgggtctgt cctctctgtt gcctttttac agccaacttt tctagacctg 3540
   ttttgctttt gtaacttgaa gatatttatt ctgggttttg tagcattttt attaatatgg 3600
   tgacttttta aaataaaaac aaacaaacgt tgtcct                            3636
<210> 5
   <211> 874
   <212> DNA
   <213> H. sapiens
<400> 5
   ctgcagcgtc tggggtttcc gttgcagtcc tcggaaccag gacctcggcg tggcctagcg 60
   agttatggcg acgaaggccg tgtgcgtgct gaagggcgac ggcccagtgc agggcatcat 120
   caatttcgag cagaaggaaa gtaatggacc agtgaaggtg tggggaagca ttaaaggact 180
   gactgaaggc ctgcatggat tccatgttca tgagtttgga gataatacag caggctgtac 240
   cagtgcaggt cctcacttta atcctctatc cagaaaacac ggtgggccaa aggatgaaga 300
   gaggcatgtt ggagacttgg gcaatgtgac tgctgacaaa gatggtgtgg ccgatgtgtc 360
   tattgaagat tctgtgatct cactctcagg agaccattgc atcattggcc gcacactggt 420
   ggtccatgaa aaagcagatg acttgggcaa aggtggaaat gaagaaagta caaagacagg 480
   aaacgctgga agtcgtttgg cttgtggtgt aattgggatc gcccaataaa cattcccttg 540
   gatgtagtct gaggcccctt aactcatctg ttatcctgct agctgtagaa atgtatcctg 600
   ataaacatta aacactgtaa tcttaaaagt gtaattgtgt gactttttca gagttgcttt 660
   aaagtacctg tagtgagaaa ctgatttatg atcacttgga agatttgtat agttttataa 720
   aactcagtta aaatgtctgt ttcaatgacc tgtattttgc cagacttaaa tcacagatgg 780
   gtattaaact tgtcagaatt tctttgtcat tcaagcctgt gaataaaaac cctgtatggc 840
   acttattatg aggctattaa aagaatccaa attc                             874
<210> 6
   <211> 1631
   <212> DNA
   <213> H. sapiens
<400> 6
   ggacttctag cccctgaact ttcagccgaa tacatctttt ccaaaggagt gaattcaggc 60
   ccttgtatca ctggcagcag gacgtgacca tggagaagct gttgtgtttc ttggtcttga 120
   ccagcctctc tcatgctttt ggccagacag acatgtcgag gaaggctttt gtgtttccca 180
   aagagtcgga tacttcctat gtatccctca aagcaccgtt aacgaagcct ctcaaagcct 240
   tcactgtgtg cctccacttc tacacggaac tgtcctcgac ccggggtaca gtattttctc 300
   gtatgccacc aagagacaag acaatgagat tcttcatatt ttggtctaag gatataggat 360
   acagttttac agtgggtggg tctgaaatat tattcgaggt tcctgaagtc acagtagctc 420
   cagtacacat ttgtacaagc tgggagtccg cctcagggat cgtggagttc tgggtagatg 480
   ggaagcccag ggtgaggaag agtctgaaga agggatacac tgtgggggca gaagcaagca 540
   tcatcttggg gcaggagcag gattccttcg gtgggaactt tgaaggaagc cagtccctgg 600
   tgggagacat tggaaatgtg aacatgtggg actttgtgct gtcaccagat gagattaaca 660
   ccatctatct tggcgggccc ttcagtccta atgtcctgaa ctggcgggca ctgaagtatg 720
   aagtgcaagg cgaagtgttc accaaacccc agctgtggcc ctgaggccca gctgtgggtc 780
   ctgaaggtac ctcccggttt tttacaccgc atgggcccca cgtctctgtc tctggtacct 840
   cccgcttttt tacactgcat ggttcccacg tctctgtctc tgggcctttg ttcccctata 900
   tgcattgcag gcctgctcca ccctcctcag cgcctgagaa tggaggtaaa gtgtctggtc 960
   tgggagctcg ttaactatgc tgggaaacgg tccaaaagaa tcagaatttg aggtgttttg 1020
   ttttcatttt tatttcaagt tggacagatc ttggagataa tttcttacct cacatagatg 1080
   agaaaactaa cacccagaaa ggagaaatga tgttataaaa aactcataag gcaagagctg 1140
   agaaggaagc gctgatcttc tatttaattc cccacccatg acccccagaa agcaggagca 1200
   ttgcccacat tcacagggct cttcagtatc agaatcagga cactggccag gtgtctggtt 1260
   tgggtccaga gtgctcatca tcatgtcata gaactgctgg gcccaggtct cctgaaatgg 1320
   gaagcccagc aataccacgc agtccctcca ctttctcaaa gcacactgga aaggccatta 1380
   gaattgcccc agcagagcag atctgctttt tttccagagc aaaatgaagc actaggtata 1440
   aatatgttgt tactgccaag aacttaaatg actggttttt gtttgcttgc agtgctttct 1500
   taattttatg gctcttctgg gaaactcctc cccttttcca cacgaacctt gtggggctgt 1560
   gaattctttc ttcatccccg cattcccaat atacccaggc cacaagagtg gacgtgaaca 1620
   caggtgccgt g                                                      1631
<210> 7
   <211> 1898
   <212> DNA
   <213> Mus musculus
<400> 7
   cggacgcgtg ggcgaggacc gcgaggagcg cagccctagc cccggcgact gagcacacct 60
   gaggagaggt gcacacactc tgaggaccta ggtgtgcaac ctctgccaga tgtggggcgt 120
   ggctacccag aggcatgccc ctcacccagc tccactgtcc ccacctgctg ctgctgctgt 180
   tggtgctgtc atgtctgcca gaggcaccct ctgcccaggt aatggacttt ttgtttgaga 240
   agtggaagct ctatagtgac caatgccacc acaacctaag cctgctgccc ccacctactg 300
   agctggtctg taacagaacc ttcgacaagt actcctgctg gcctgacacc cctcccaaca 360
   ccactgccaa catttcctgc ccctggtacc taccttggta ccacaaagtg cagcaccgcc 420
   tagtgttcaa gaggtgtggg cccgatgggc agtgggttcg agggccacgg gggcagccgt 480
   ggcgcaacgc ctcccaatgt cagttggatg atgaagagat cgaggtccag aagggggtgg 540
   ccaagatgta tagcagccag caggtgatgt acaccgtggg ctacagtctg tccctggggg 600
   ccttgctcct tgcgctggtc atcctgctgg gcctcaggaa gctgcactgc acccgaaact 660
   acatccatgg gaacctgttt gcgtcctttg tgctcaaggc tggctctgtg ttggtcatcg 720
   attggctgct gaagacacgg tacagccaga agattggcga tgacctcagt gtgagcgtct 780
   ggctcagtga cggggcgatg gccggctgca gagtggccac agtgatcatg cagtacggca 840
   tcatagccaa ctattgctgg ttgctggtag agggcgtgta cctgtacagc ctgctgagcc 900
   ttgccacctt ctctgagagg agcttctttt ccctctacct gggcattggc tggggtgcgc 960
   ccctgctgtt tgtcatcccc tgggtggtgg tcaagtgtct gtttgagaat gttcagtgct 1020
   ggaccagcaa tgacaacatg ggattctggt ggatcctgcg tattcctgtc ttcctggcct 1080
   tactgatcaa ttttttcatc tttgtccaca tcattcacct tcttgtggcc aagctgcgtg 1140
   cccatcagat gcactatgct gactataagt tccggctggc caggtccacg ctgaccctca 1200
   tccctctgct gggggtccac gaggtggtct ttgcctttgt gactgacgag catgcccaag 1260
   gcaccctgcg ctccaccaag ctcttttttg acctgttcct cagctccttc cagggtctgc 1320
   tggtggctgt tctctactgt ttcctcaaca aggaggtgca ggcagagctg atgcggcgtt 1380
   ggaggcaatg gcaagaaggc aaagctcttc aggaggaaag gttggccagc agccatggca 1440
   gccacatggc cccagcaggg ccttgtcatg gtgatccctg tgagaaactt cagcttatga 1500
   gtgcaggcag cagcagtggg actggctgtg tgccctctat ggagacctcg ctggccagta 1560
   gtctcccaag gttggctgac agccccacct gaatctccac tggagcctag ccaggctgcg 1620
   ttcagaaagg gcctcagagg acaacccaga gccagatgcc cggccaaggc tgaagagaca 1680
   aagcagcaag acagcagctt gtactgtgca cactccccta acctgtccta gcctggcaca 1740
   ggccacagtg acagagtagg ggttggatat gatggagaag ccatgttatc tatgaactct 1800
   gagtgttccc atgtgtgttg acatggtccc tgtacccaga tatgtccttc agtaaaaagc 1860
   tcgagtggga aaaaaaaaaa aaaaaaaaaa aaaaaaaa                          1898
<210> 8
   <211> 106000
   <212> DNA
   <213> H. sapiens
<400> 8
   aagcttatgg tttatgggtg ttacattcaa gacatttgta ggacacattc taaaatgcca 60
   tccaatttca ggctctttcc agcagaaact gtggaatatt tttccgttca ttcagcattt 120
   acttagtgcc tgctctgcca ggaattgaag agaaagccca aagacaggca gaccttacct 180
   gagaggtagt gaactgacca ggatgactgt gggcagtaga cttgtttccc aaactagcct 240
   caccatttct gtatttgcat atacgaggaa aggattagat atagggattc atgtcagcat 300
   acaccccagg gacatttgtt tttagtgaaa ggtgccagtc ttcatccctg tacccagtac 360
   acaaaccacg aagaagtatg ctcccgtcat tgtcaaagaa tcatagaatt ccaaatggag 420
   ctagttttga tatccagatc tcacttcata tgaggaaact aggtccagta ttgtgagtaa 480
   gaattaggac tcttcagatt ccctgggtat gaatctgact aacaactgtg tgaacttgac 540
   caaattcata accctgtaaa ctctgtttcc tcacttttaa aatgggcaca acaaagtgat 600
   gcatgtaaac tgcatagcac agtgtctggc acttaaaaag cactcctgaa gttattttta 660
   gtgatgtgtt ttaagattag acaactcctt aatgccaaag gtttttactt gagaactctg 720
   tctgtgtgcc atactacacg ctgttcataa gataagcctt tttcattaat tgatctcaaa 780
   ctggcttcat tatgatctta actttatttc agttttattt ttaaaattta tttttaattt 840
   ttatgggtat atagtaggca tatatattta tggggtacag gtcatgtttt aatgcaagca 900
   tgcaattgtg ggggtgatat ataattgact ggggtgagat atctcattgt agttttgatt 960
   tgcatttctc tgatgattaa ggatgttgaa catttcttca tacacctgtt ggccatttgt 1020
   atgtcttttg agaaatgtct attcagatct tttgtccatt ttttaagttg gattgtttga 1080
   ttttttcctg ttgtctgaac tctttatata ttctagttat taatcccttc tcagatgggt 1140
   agcttgcaaa tattttcttc cattttgtgg gttgcttctt tgttgtttcc gttgctgtgc 1200
   agaagttttt tagcttgatg tgatcccatt tgtccatttt tgcattggtt gcctgtgcat 1260
   ttgaggtatt actaaagaaa tctttgccca taccagtgtc ctggagagct tcccaaatgt 1320
   tttcttttag tatcctagtt tcaggtctta gatttagggc tttagtccat ttttatttga 1380
   tttttatatg tggtgagaga taggggtcta gtttcattct gcctatggat atccagtttt 1440
   cccagcacca tttattgaag agactgtcct ttccctagtg tatgttcttg gcacctttgc 1500
   tgaaaatgag ttcactgtag gtgtatgaat ttgtttctgg gttctctagg tctgtgtatc 1560
   tgtttttatg ctagaactat gttgtttggg ttattatagt tttgtagcat aatttgaagt 1620
   cagataatgt aattcctcca gttttatttt ttttgttcag gatggctttg gctattccgg 1680
   ggcttttgtg gttccatata aatcctatga tttttttttt ctatttctgt gaagaatgtc 1740
   attgatattt attaataaag attgcattga atctgtagat tgctttgggt agtatggaca 1800
   ttttaacaat attgattctt ccaatccatg agcatggact atctttcttt ttttgtgtgt 1860
   cctcttcaat atttttcctc agtgttttat tgttttcatt gtagagctct ttcacttctt 1920
   tcgttgagtt tattcctagg tgttttattt tatctgtagc tattgtaaat gagattactt 1980
   tctgatttct tttttagatt gtcctctgtt ggcatctaga aatgccacag atttttgtat 2040
   gttgattttg tatcctgtaa ctgtactgaa tttatctgtt ctaatatttt tttggtggag 2100
   tctttaggct tttccaataa gatcatacag tctgcaaaca agaataattt gacttcttcc 2160
   attccatttt ggattccctt tatatctttc tcttgtctga ttactctagg taggtcttcc 2220
   agtacttcca gttgaataac agtgggcact cttgtcttgt tgtagatctt agaagaaagg 2280
   ctttcagttt ttccccattc agtatgatac tagctgtcag tttgttgcag atggcataac 2340
   tttcaaacta attgattata gttaggaagt ggatacttta acttgtggta ccattatcag 2400
   atttatattt cggccataag cttgaagagg agctgaaaaa tgcatatgtg atgcatatgc 2460
   ttcctatttg gctctcttct cccacccccc tgccctataa tccacacaag ttcctctctc 2520
   agtcactcat caactacttg aacctctgag gaacttgggg ttaaggtaaa ttagaataaa 2580
   actgtctgaa gaagagcaag cctttcatgt cttgagaaat tcttggggtt ttagaaataa 2640
   cttcattgct ttttttctcc agttactttg gcttcttctt aaagagaata ctaacacttt 2700
   gaacgtcata atactaaggt tctgcctctt caaataaaga ctttaaaaaa aaatggtttt 2760
   tgtatgattc agtgtgaatt aaatcccaca gtgtaaagga ctttactttc ttaatgtaga 2820
   ttttcaaata cacaattact gatgtttata agtagattta ttacaccaaa gcacctagca 2880
   aattcttgaa tggatcaggt cttatttttc agtcttactt tgcaaattta agccaaataa 2940
   ttaaggattt gttaaatatt tgtcttaata tcaagctttt gcatatcggg gccctctttt 3000
   ataagcttta taagcaatct tttgttttct ctgcttgctc aaagtagcta tgtttgttgt 3060
   atctgttagt atttgctcta taacaaacat actgggtgcc ttcccactta gatttggcaa 3120
   ttatcactcc tgtaaatgag atattacata agataggaaa aagaacagta tctttccaag 3180
   aagaatagta tccttccata ttaacagttt agagctgact gcttttaaaa tttagtggct 3240
   ttaaaataac aaccatttat tattcttcat gagtctacaa atgaggtggg cagttctgct 3300
   gatctggcca agctgaactt atctcagctg ggcacattca gcgtatctgc tgtcagttgg 3360
   ctggttggct gtagcaatga atggtgaaag taggctgccc ttaacttttt cacacagtag 3420
   cattagagtt acaaaagaac cagcagaacc atgcaaaact ctttaagacc taggcttgga 3480
   acaactatat ttctaccaca ttctattggt caaagcaaat cacggggcta gtctagattc 3540
   aagtgggtgg aggagctgca attacactgc aaaggagtgt gactgtaggg agaggtgttt 3600
   ttttattttt atttttttgc gatttgtcac agtagttgta ggaatcaggt gtatttaaaa 3660
   ttctgatcct tctgtgatat ccgaattgtt catgaacctt gcctctggtg gaaaggcaga 3720
   atcattgtga cagaaggata aaatcttgga atttagagac taacaaaggt tcagattcca 3780
   gctccatcac ttatttctgc aatcctgcag aagttaatct tcctgatagg cattcagtaa 3840
   tgattgattc acctgaacct cagattcttt atgtatttta aagaaagggc taggtaaatg 3900
   caaagcactt atgtaactgc ttttattatt gcaaacctgg ctcccacact ccattcaagg 3960
   tgtaagactc agtgtcttcc ttgaattaaa aaggaagaga aagtgtgtta gggaaaggaa 4020
   gagaaatatt tgactaattg tggccccaat aaagtgacca ctcactgggg gtattttcct 4080
   gtaagaaaag aatggttgag gctcagagtt aagagataca aatccaaaag tctccttggg 4140
   gtaggattcc ctgtgattca tgggttgaga ggtgtaacat tagacacagt cccagtctag 4200
   attttttttt taaagaattg tggtccatcc tatacacact gggtgcctta atactatatg 4260
   tggcaattat cactcctata aatcaggttt tacataagat aggaaaaaga acagtatcat 4320
   tccacattaa caattgaaag atgactgctt ttaaaaaatt aaaagggcca tatagaaata 4380
   aaatcacata aatttcttgt gttaaacata gttgtcatat tggatgagga ctaaacacct 4440
   aaattcatcc aactagtagt aatagaaaag atgaaacaca cacacagtaa aactagatta 4500
   atttaattta tacaaagggc cagatatctc agaattcaga cagtcagaga tgttgactag 4560
   agttaatgcc tcttttagga gaggtaccag gtaagtgttc tcaaagaact ggaaactgag 4620
   accaccacct ctggcattat ctgtttgtga acacaagcaa gtctgaattt ttccgcacca 4680
   tagctacctt tcatgtaagc ttcttttctt agaagaaaag aaggtaacat ttgggtgtaa 4740
   ttttttatta agggtgaaat ttagtgtaga gagtaaaggc atttggcata gaagccctta 4800
   gttttttttg tttttaagtt gaactgccag cctttatgga ttgcagtctt cgctgttttg 4860
   attgacattt cccaattcat tttgtattat ttattttttt aagagacagg gtctcactct 4920
   gttacccagg ctggagtgca atggggcaaa cttggatcac tgcagccttg aactcctggg 4980
   ctcaagcaat cctcccacct cagcctccca agtagcttgg actataggtg tgcaccacca 5040
   tccttggcta attttttaaa tcttttgtag agacagggta gtgctctgtt gcccaggctg 5100
   gtctcacatt cctggcctca gttgatgctc tggtctcagc cttccaaaat gctgggatta 5160
   caagtgtgag ccactgcacc tggcccccaa tttcatcctt tacaaagact actttcaacc 5220
   ataaatcaac ggaaacttca gctccctcag acatatttgg gatccaagga tattttccca 5280
   aatgattaat gctaatttca tatcaataca tttttgcaaa acctacaaaa atggactagt 5340
   aaagaaagac tcttaatttg ggaaagacag ttacttggag agaagagaaa cttaagaggc 5400
   aggtcgagtt cagtgttcag aaatgagagg atcataaaga gatagccata aaaatgtttc 5460
   tccctatatt gcctgctgat agggtgtatc agtgaaggtc ttactaagga ccttgtacct 5520
   tttcagcgct gcactgcgtg ctcataggga ggaaagataa atcatgtgtt ttttctgacc 5580
   tcaaaggagc ctgtatctgg ctagagagac atgatgcaga cacatgaaat aattaagaaa 5640
   caattaactg tagcaggtgc tgaagaatat accaggaggt cagagaatgg tagagctagt 5700
   gtgggcgaag gtatagccca gagcatcatc agatgattct tccttatgca aattcacatc 5760
   tcctctgggt caagtatcat cctggcatgc agcagctcca taggtaatgc cctaaggcta 5820
   gcctgaggca agttgcaaaa gccatcatat tgagtcatgg cctttttttg tgtgggggga 5880
   ggggaatggc atccccttcc tgtctgccaa atcaaggaat acagtgccct cctaaacctg 5940
   ctttgtttta gtggattgtt aaaaagaagt gaatgaattt atgcttcatt agggaaaggt 6000
   tacagtggaa tactgaggag taaggggtat ttctatttaa caaatgacat aacttgaagg 6060
   aatgaaatca taaggatgga atttcaggca ttaataaaaa gctgatgaga gatactttga 6120
   gacaaaagag ccttcccagt gtaaccgaga tcacagcacc tacttcacat acacaggaaa 6180
   ccagtcctat ctgtctctcc catagagcag tagctgcctt gtttttcctc cctcctccat 6240
   cattcattct aaatctccag tcctccaccg caccttatcc aaaccctgat acccttaagt 6300
   cacagatggt gaatcagtca aaagtagtat taaaaactag tggtacacag ctacacctgg 6360
   aatgcagtaa gaaaaatacg gatttctgta catcatcttc cctccctgct cttaccccca 6420
   tttaagagtt acagggtcag aacccaagag tgtgagtttt tgaaagtccc taaaaatttt 6480
   ggatgatcac ctacatttag aaccactgca ctaagaagga caacaaatat gccaataaat 6540
   tctgttgcca aggaggtgat tatgcaagct ggaaccctga taacatgagg agaatcccac 6600
   aatagccaaa tagtccatgt actagttaca ttataataaa gccaaaagca gcaggcctac 6660
   ctgactttct cctgaggtct atcatgagct tagagagaag gaacgtggac atacagaggt 6720
   agctctagat ggagaagggc actaggtgtc atggaaagaa tcatgtgcaa gaagtaaaga 6780
   ggtgctctga atgtcctagc cctgcttagg tgtctgtgtc ctcacatgag aatttatcca 6840
   cagttctttc ccgctgtaac aatctttggt tccaactgca tttgtgagac agcaaaaagc 6900
   tatggtccag tctccttcca ttgtatcatc tcatcaatgt atttctccca ctacccttgt 6960
   gtgaaataca aacttttttg gcttattgtg attatgcaag gtgtatgcca actttttttt 7020
   ttctccacat ctttcagctt tctgatgggt aaaaattttc cttattttgc tttagaaaaa 7080
   ttctcattgg catagatcta atttcaggga gcctcccttg aaagctaaat aacattgaga 7140
   attcatgaaa atataatgta gagcattatg cctgttagca tattagttta aatagaagtg 7200
   gttcatgaaa atttttgaaa tgccagaccc tgtcctgtgt tttgtattct cccaaatact 7260
   catccagata ctgttcagaa tgtaacatga ttattttgaa ataaagattt tcccctagtt 7320
   tttaaaaaag ttactttata cattaaccct tatgttcctc tttgatcaat ttttccagta 7380
   gtgtaaacag tcttcaggga agtagatttc ttacagaaat tgtcaagtgg ctctctgctg 7440
   ttagcatggt tactaatctt ttggttactt ttcatatttt ttatactttc tggaagtgga 7500
   caacttactt gtaaataaaa gtgcataatt tgtattaaaa atttttagta acaatctaat 7560
   ttgtaaaata gatgtgagca gcatgaatgt gtgtgatatg cgtacatacg aattatgtct 7620
   cttaaaaatg tatcacagac atctttccgt gtccaaacaa atctacctca ttctttctaa 7680
   tagccatatg ggtataccat aatatattta actaggcccc tattaaaaga attttgactc 7740
   ttttgtagct actatagtgt tgcagtgtgt atctgtgtat gtatctttgt gtgtgtatct 7800
   ttgtacgagt gtacatatat tttccccttg gctatttcag attttttttt aggtttaaat 7860
   cttaggaaag gttttgaaat tgtcttaagt attttcagaa gcattaaatc atggtttttt 7920
   tacatttttc ttttagaagt tttatgtcat ctctatgagt agctttcagt aatttgttct 7980
   gcataaaatt cccgaaaact tccatttaaa aataggtggc atgactagac tttctcagcc 8040
   gaaagagtga ggtcccagga aggattttgg agaagctgtg ttcaaatata gctgctgacc 8100
   tgatgtctgc ctagagtctg gcaaggtgat gtgttgaatc tagtgtctgc ctgcatgcca 8160
   gcatcccttt actgatgaga tttgtggttt tcatcacttc atggtaatca tcccaagtta 8220
   taagatggag tctctagaaa atcagtagag tatgaaggcc caagtaaaat acatgtgagt 8280
   gcatgtatgt gtgcatacaa attacttctc ttaaaaacgt atcctgggca tttaaagaat 8340
   gaggacctcc gaaggatttt gtggaagctg tgttcaagta cagctgctga gcgtatgtca 8400
   gcctggagcc tggcaaggtg aagtgttgaa tctagtgtct ttttgactca ctgttttttt 8460
   tgactcactg tgctttgaag cccttgtcat ttgggctcat aaaatagatt tctgtatact 8520
   gtctctcctc cctgccctcg cccccattta aaagtatagt ggcagaaccc aagaatcaga 8580
   gttactaaaa actctctaga aaatttggat gatcacccac ctgatcatgt cttttttact 8640
   cactatgttt tttttttttt tgagacagag tctcgctctg tcgcccaggc tggagtgcag 8700
   tggcatgatc ttggctcact gcaagctccg cctccctggt tcacgccatt ctcctgcctc 8760
   agcctcccat gtagctggga ctacagggcc tgccaccgcg cccggctaat tttttgtatt 8820
   tttagtagag tcggggtttc actgtgttag ccaggatggt cccgatctcc tgacctcgtg 8880
   atccacccgc ctcggcctcc caaagtgctg ggattacagg cgtgagccac cacacccggc 8940
   cctttactca ctatgttttt aagcccttgt tttcatttgc tccactgtaa aacattcccc 9000
   aagccaatct ggagctgagg caaattttta acaatttaaa atctggggaa tataaatatt 9060
   ggataatgat catcctgaaa aaacaatgaa ggtagtagca taatacttta tatatcaata 9120
   aaatggcaaa ataagacagt tgttgaagga cagaaagagt aactgaagtt aggagcttat 9180
   cttaacacat tttttgtgtc ataccatagg catcatattt tttaaatttt ttttatttca 9240
   tacacatagg aaaatatatg tgtgtaagaa ataataaaca cctctttgta cctaccaccc 9300
   aacttaagga acagctcatt gctattccct ttggtgctcg ctggatgccc tttcccagtc 9360
   acatccccct cccttcccat ctgcaggact atactagtaa attttgtatt ttttgcatta 9420
   ttttgctttg ttttatgatt ttactaccta tctacatatc cctaaataat acattattta 9480
   gtttcatatg ttttaacttt atgttgtgga atcacattaa atgtagtctt ttttttttat 9540
   attatacttt aagttctagg gtacatgtgc acaacgtgca ggtttgttac gtaggtatac 9600
   atgcgccatg ttggtttgct gcacccatca actcgtcatt tacactgggt atttctccta 9660
   atgctatccc tcccctagcc ccccaccccc cgataaatgt agtctttata acttgttttt 9720
   ttaactcaac attgtttgta agattcatcc atgtaagctg aagctttttt atagagatct 9780
   ttgttaagcc ttttaatgaa tacagtacat acatttctct gttcccctgt tagtggacac 9840
   ttggattgtt tccagagttt tgctgttttg aacaacgctg ctgtgaaaat gtctcctgaa 9900
   acacatttat aagagttttt ttttccccaa gggaattata cctagaaatt gaataactag 9960
   atcacaaggc atacacatct acaacttctg ctaggtaatg ccaaattgtt tccaaggagc 10020
   gttagaagtg ttctcatcaa cttttactag tgctagtctt ttacatttgt ggcagtatgg 10080
   tgggtgtgaa atatttatgt ttagtttttc ttggtgccat ttaataattt ttataaaaaa 10140
   tatttagaag tcaaggcagt tttttgtttt tgtttttatt ttttgcttgt tttgttttaa 10200
   tgcagacatt gagattacga cttggaataa acattggttg caaagttcct aaaaggaaaa 10260
   ctttttttgg tattctggag cttttctggt actgaataaa ataagtatgt taaattatgc 10320
   atgtgtagtt tagaagtcag agcaataatt gtgattgttg aacagaatgg cagtaaaaag 10380
   tttctaaacg attgtactgt acaagggaca cttgttgtgg gtcagtttta gcctccccaa 10440
   cttttatgtt aaaagttgca acaaggttta agggcttatg tttgataggc cagatggtga 10500
   ccagctgtga taaaacacag ggaacccttg caaaggattt caaaatttat gcagtagtcc 10560
   gccttatctg cagttttgct ttccaaggtt tcagttaccc gcagtcaact gtgttctgaa 10620
   aatattaagt gaaaaattac agaaataaag aatcgaagag ttttaaattt tatgcttccc 10680
   acccatccca cctgggatgt gaatcattcc tttgttcagc atctccatgc tgtaggtgct 10740
   gcctgcccct tagtcacttg gtagccatcc aggttatcag attgactctt ctagtattac 10800
   aacacttggc ttcaagtaat ccttatttta cttcatagtg gccccaaagt gcaggagtgg 10860
   tgatcctggc aattcagata tgtcaaagag aagctgtaaa ttgcttccct taagtgaaag 10920
   atgaaaattc tagacttata tataaagaaa agaaatcata tgctgagact gctaagatct 10980
   atgataagaa tgaatctttt atacatgaaa ttgtgaagaa tgaaaaagaa atgcgtgctg 11040
   gttttgctgt catatctcag actgcaaaag tttgcagcca atgtgtatga taagtgctta 11100
   gttaaaagga aaaaggcatt taaggtaagt atatatagtg tttggtacta cctgtgattt 11160
   caggcatcca ttgggggtct cctgagtata aggggagact actcttttag tgttaaatga 11220
   acactaagga acagagatgg ggaagaggtt ggagaagatt agttcagcag tttgagtata 11280
   ggtaaacagt tgtttgagaa agaagaaaaa tgtgattagt attttacctt agcaatagtg 11340
   gcatagataa tgataaatta tagtcacaca gaactcttag tatttacaga acgttcacat 11400
   ttgtgatccc atttaacaat aactctgaaa gaaaggtatc atctaccact gctttattga 11460
   taaagatata aaaggtaaga gagatgaaac atattggcca atgataccca tctggtaaga 11520
   gacagggatg gggtgggacc ccaaggctct tctcgccaag cccacggttt ttttgcttta 11580
   tacttttttg cctcctgatc accatggctg cagtttctac tgtggacaat gtctgtcagc 11640
   aagcattgat cccctgcctt cagcactctt acgtcttagc aaggactgga aagaaaaagc 11700
   caggagttta cagtctgctg gagcaacaga aaagaatgat atgaaatatg aagagaccaa 11760
   aatgatttat aataaggtgc tagactatgt agtaaaaatc tgctttagct gtaagtcaaa 11820
   agcaagagca gtcttttcag aatggaatag aaatgttgga attaaaggaa ttttcaaagt 11880
   tgtgaatttt tttcaagata aacatgtttt attttggtaa ttatggtatt actaatttga 11940
   taaccttcag ggagccacct aatattatag aagatgtaca tataatgaca aaagcaaaca 12000
   ttttattttt aaggaccaca atctaatcta aaacaaaatt tccccctttt ctggtctttg 12060
   gttaattaag gacttattta aatatcaaag aaagacacat agaaaacatt tagtatattt 12120
   ctatactttt attaatgtcc tccatacctt acacagatac ttgacttggc tatggtctag 12180
   ataatccatg aaaatttaaa ggacagattt taacaacttt atgctaaatt gatagatctc 12240
   taggatcaga ttgccatcac tctcagatgc gaagcttcca accacttata ggttcctgat 12300
   atcttgcttt tatacagacc taatttctct tcctttaaac tttcttttcc tcagttgcta 12360
   tttgattgaa atattgagtc attaaaaatt tccaagtggg aatttttgtg tttcttcatc 12420
   tatcatgaag ctgctcaaat aagtaggtgt ttgaatagga gtagaaacag taataggctg 12480
   aagccagacc aatacagctt cagctaaatg ccgaccttgc taaagtctgg gaggaccggt 12540
   gtggtattct acaatgtaca agtctgtagc cggtgccctt aatatgttgg cttcatgtct 12600
   catgactctc ttctgtaaat atgcagttta aaaaatacaa gttattctgc tgtagaagat 12660
   acatttgcaa aattgatgta tcccctctaa gtaaagttgg ctaaacaata aggacatatt 12720
   tataattaat gaatttgaga agaatgctga cgatatgcat tattctttga agttaacatt 12780
   tttcaggtcc taaataaaca aaaagtaggt tacttctgtc tggagtgtat gcaaggggta 12840
   ccatcttgtc cttggttcct ggctgctatt ccaaggtgct ataaagtcag ctaaagagag 12900
   caatcataat acattgatag catccctcaa tgtgtttctg agctacttga gaatcttatt 12960
   tttgaatagg tagcaggaaa ccatctttgc agggcagcat gggcaaaggg attggaggga 13020
   ctattattat aaagatccac tgaactgctt cagtatcata atatcttaaa ctaaaggact 13080
   ggaaagagcc agattccaat ttaatctgct cttctatgaa ttcttagctg ggttcattta 13140
   aaaagaaaaa acttgaagat tgcaagattt tgaagacatc ttaaaatagg tgaactccaa 13200
   ggtgcacttt aaacttgaga ctgataactg aatactcctt caccttttga tctgatattg 13260
   tcaaaatgaa tgaggactta gtgctctagt aagtttggaa cagaatgata ttaatttatt 13320
   ttctcatgat tgattctttt ttgcttttta atagattaaa cttcaccgta gaacagtttc 13380
   tcaacctctg gactattgac atttttgatt ggataattct ttgctgtcag ggctgttctg 13440
   tgtgttgcag gatagttagc aacatccctg acaatcacaa atgttacttt ctgtctctat 13500
   ggatttgcct attctggaca tttcgtataa atagaatcat atatatgtgg cttcttgtac 13560
   ctggcttatt tcacttaaca tgttttcaag gttcatccat attgtagcat gtaacagcac 13620
   ttcattttct ttttatggct gagtaatatt ctgttatgtg gatatactac catattttgt 13680
   ctatccactc cttagctgat ggtcttttag gttgtgtcca ttctttggct attataaata 13740
   atgctgttaa gaacattcat atacaagttt ctgtgtagac atatatcttt atttctcttg 13800
   tgtggatacc taggagtaga attactggat catatgataa ctctatgtgt taccttttga 13860
   ggaactgcca aacatttttc tacagtggct gtatcatttt acactcccat cagcaatgta 13920
   taagaattcc aatttctctg tccttgccta tatttattaa ctgtcttttc ttattagcca 13980
   actgctgtgg ttcgaatgtt tgtcccctcc aaaactcatg ttggaacata atccccaatg 14040
   tggcagtatt gagatgtgag gcctttaaga agtgcttggg tcatcagagg tctgccctca 14100
   tgaataggct aatccattca tgagttaatg tactaatggg ttatcactgg attgggacta 14160
   gtggctttat aagaagagga agagaactaa tctagtaagc tcagccttct cactatgtga 14220
   ttgctgccct gtgtcacctt gggactctgc agagagtcct ccagcagcaa gaagttcttc 14280
   atcagctgtg gccccttgac cttggacttc ccagcctcca gaaatgtaag aaatccattt 14340
   ctttttttta ataaattaca cagtctcacg tattcagtta taccaacaga acacagacta 14400
   agacaccatc ctattgggta tgggtatctc attgtgtttt ttatttgtgt ctcccaaatg 14460
   actaacgatg ttgaacatct tttcatctgc tttttggaca tttgtgtatt ttctttgaag 14520
   aaatgtcttt aacattcttt gcccatttta aaattaggtt gtctttttat tgttgagttg 14580
   tcggtgtgtg tgtgtgtgtg tgtgtgtgtg tgtgtatcta gaatatatgt gtatgtatat 14640
   atgcagatat attctaaaca ctagaccctt atgaaatata taatttgagg acaatttctc 14700
   ccatttaaaa ggccatcttt tcacttcttg atagtgtcat ttgactcaca agtttttaat 14760
   ttttatgaag tccaatttat tttttaattc tttgtttttg gcactgtatc tttaaaaagt 14820
   tgcctgatct aaggtcaaac tgattttcac ctatgttttc atctaagaat tatagtttta 14880
   gctcttacat ttaggccttt gatccatttt gaattaattt gtgtatatgg tgtgaagtag 14940
   ggctctaact tattcttttg tgtaatgata cctagttgtc ccagcaccat ttgttgaaaa 15000
   gattattctt tccccattga atggtcttga taccttgttg aaatcaactg accataaata 15060
   tataggctta ttcctggact cacaattcta tgagtctgta tgtctaatct tatgccagta 15120
   ccacactgtt ttgattatta catctttgta caaagttttg aaattgggaa atgtgagtct 15180
   tccaactttg ttctttttta agattacttt gcctatattc cgtgttcgtt gcaaactcat 15240
   atgaatttta aatcaactct ccatttctgg aagaaaaaaa gaggcaattg aagttcagat 15300
   agggattgca ttgaacctgt agatcagttt ggggaatatt gccatcataa caattagtag 15360
   gtcttccaac ccatgaatac aagacttctt tccatttctg tagatattta gtttctttca 15420
   ttaatatttt gtagttttca atataaaagt cttgtacttc gattaaattt attcttgaat 15480
   attttgggtt ttgatgcttt tatgaatttg ttttcttaat ttcactttaa gattgttcat 15540
   tgctactgat tagtaatgca actgattttt gtgtgttgat ttttgtatcc tgcaacctag 15600
   ctgaaatcat tgattagcat aatagagtat ttaatagatt taggatttct atatataaga 15660
   tcatgtcatc tgcaattaga gataatttta cttcttccct ttcaatctgg acatttttta 15720
   cttctttttc ttgcctagtt gccctagcta gaacctccag tgcagtgttg aatagcagtg 15780
   gtgagaatga gcatctttgt gttggtcttc atcttgtggg gaaacctttc agtttaagtg 15840
   tgttgttgtg gggttttcat agttgtcctt tatcagattg agaatgttcc tttctgttcc 15900
   tagtttgttg agtgttttct ttttgattgt tttaatcagg aaagggcatt agattttgtc 15960
   aaatgctttt tctgcagcta ttgagatttt tgtgtgtttt tctggtcttt tatggtttat 16020
   cacattaatt gattttcata tgtcaaacaa accctgtgtt cttgggtttc atctcacttg 16080
   gttatggttt ataatccttt ttatatactt gtagattcag tttgccagta ttttgttgag 16140
   gatgcttgca tttatattta taagggatat tggtctgttg tagctgacca gtaagtatag 16200
   taagctgtat agtttactaa gtgttccctc tgttttgggg gagactttga gaagaaggat 16260
   tgttggtaat tgttctttaa acatttggta aaattcacta gtgaagccat ctggggtctt 16320
   ctttggaagt tttttgatta ctaacttaat gtctttactt gtttgttata agtccattca 16380
   gatttttttc tccttgagtc atttttgaca gttggttgag gaatttgttc atttcatgta 16440
   gttatctaat tggttagtgt ataattattc atagtattcc tttataatct tatttttttg 16500
   ctgtaaggtc agtcataatg ttcactcttt catttcggat tctggtaatt taagagtctt 16560
   ctctcctttt ttttcttggt cagtctagct aaagtaaagt tttgtccgtt ttcaggggaa 16620
   cagctttttt tttttttttg aggcagaatt tccatcttgt cacccagtct agagtgcagt 16680
   ggtgcaatct cggctcattg cagcctccgc ttcccgggtt caagagattc tcctgcctca 16740
   gcttgccaag tagctgggat tacaagcgcc caccaccacg cctggctaat tttttatatt 16800
   tttagtagag acggggtttc accatgttgg gcaggctggt ctcgaactcc tgacctcagg 16860
   tgatctgcct gccttggcct cccaaagtgc tgggattaca ggtgtgagct accgtgccca 16920
   acccagcttt ggttattttt gttgacctac tctattgttt ttctcttctc tatttcactt 16980
   atttctacac tggtctttat tattttcttc cttatgcttg ctttggactt agttcttctt 17040
   tttctagtct cttaaggtgg ataattaagt tcctgatttg aattcttact tctttgtaag 17100
   gtggtcatgt actgctatga atttccttct cagaaatgta tatgctttca ctgcatccct 17160
   taagatttgg tatgttgtat ttttgttttc atttgtctca aggtatagtc ttctgatttc 17220
   cattgtgatt tcttccccct ctaacccgtt tattatttag gaacttgttg atttccacat 17280
   acctgtgaac tttccagatt tccttctttg ttaattctca gtgtcattcc attctggtcc 17340
   gagaacatac tttgtatgat ttctatcttt taaaatttat ttggcttgtc ttatgaccta 17400
   atacattgtc tatcctggag gatgtttcat gtacacttga gaagaatgtg tattctgctt 17460
   ttgttgggta gagtgtttga caggtgtgtt ggtacatagt tctgttcaaa tctgtttcct 17520
   tgcagatttc tatctagttg ttctgtctat tggaagtagg atattgaaat ctccaactaa 17580
   tattgctgaa ttgtttattg ttttcttcag ttctgtcact ttttgcttta tatattttga 17640
   aattctattg ttaggtacaa gtaagtttat gattattata tcttcttgat agattgattc 17700
   ttttatcatt atacagtgcc ctataagaac aatttttatc ttaagtctat tggtctatat 17760
   tagtatagcc acttcagctt tcttttgttt actgtttgca tggaatattt tcttctttta 17820
   ctttctattt gtgttcttga gtctaaggtg aatctctgta gatagcaatt ggatctgcca 17880
   atctttgctt tttatttggg gagtttaaac cattgacatt taatgtaatt attgatgagg 17940
   aagattactt ctgatatttt gccatttgtt tcctttattt tgtgtctctt gttcttaaat 18000
   tcttccatta ctaccttctt tcttttgtat tacatatttt ctagtgtaac gattttaatt 18060
   tctttgtcat ttcttttgtt gtatgttttt agttattttc ttagtggttg ccacggagat 18120
   tttattgtca ttttaacagc ctaggttggg cacagtggct catgcctgta atcccagcac 18180
   tttgggagac tgaggcagga ggatagcttg agtccaggag ttcaagacca gcctgggcaa 18240
   cttactgaga tactgtctct acaaaaaaat acaaaaatta gccaggcatg gtggtgtgtg 18300
   cctgtagtcc cagatgcttg agaggctgag ttgggaggat agcttgagcc caggaggttg 18360
   aggctgcagt gaactttgat cacaccactg cactccagcc tgggtaccag ggcaaaacta 18420
   gcccaaagaa atgaaggaaa aaaaaaatct aatttagatt aatatcaact caacttcaac 18480
   agtgtataaa aactttgcct ctgtatacct cttctgcttc cactctgtgc tgttattgtc 18540
   atagattttc atctttctac actgtgtgtt tatcaatgta gatttaaaaa tattgcttag 18600
   tagttgtctt tagaatccga tacggagaaa aggagatata aacaaaagat gcatttttac 18660
   tgtcttgtat gtttacttat gtaattccct ttcctgatgt tgtatttcta aaggcaaagt 18720
   agggttattg tgagtgtcct tttgtttcaa cctgaaagac tccttttagc atgtgttgga 18780
   gatatgctaa tgatggactc tcacagtttt tgttatctgg gaatgtgtta atttatcctt 18840
   catttttgaa ggatagtgtt ggcaggatac agaattcttg gttgacatgt aattctttca 18900
   gcattatgaa tatgtcatcg tactgtcttc tgacctccat ggtttctgat aaggaatcaa 18960
   ctgttaatct tattgaggat cacttgtttg taatgacttg cttgtcgtgc tgctttcaag 19020
   attcattctt tgcctttagc ttttggtagt ttgattgtga tgcatttagg tgtgtacttt 19080
   attagtctgt tctacttgga gtttgttgag ctttgtagat gtatttcatc agatgtgtca 19140
   agttcttttg ccactatttt ttttttaaat aatctttttg cccctttccg ctccttctgt 19200
   cactctgatt atttgtgtgt tgctttgttt ggtggtgtcc cagaagtctc tgagactctg 19260
   tccagttttt tcctccccat tcttttttct ttcacttcct cagactggat gatctcaatt 19320
   tgacctatct tcgagttcat ggattttctc ttctccaagt gacatctgtg agatgaattt 19380
   ttttctagag aatttttcat ttcagttatt ctacttcaaa atttctcttt ggttcagttt 19440
   tatcattgct atctttatat tattctcagt ttaatgagat actgttttat actttccttt 19500
   agttctttag acatagttta tgtcactgaa tatatttaaa atagctgatt ttaagtcttt 19560
   tttttttatt tttttggaga tggagtctcg ctctgtcacc caggctggag tgcagtggca 19620
   cgatctcagc tcactgcaag ctccacctcc tgggttcacg caatgatttt aagtctttgt 19680
   ctatgaagtc tagtatctgg gcttcctcag gcatagtttc tgtttctttt ctttcttttc 19740
   ctgtgtactt cgtttctttg tataccttgt aattgttgtt gttaactgga cattttgaat 19800
   attatagtgt aacaactctg gcagtcagac tgtctcccct ccccagtatt tgttgttggt 19860
   gagtattgta gatgtttgtt tagtgacttt tcatggctaa ttctgtaaat tttatattct 19920
   ttgaagattg tgggcaccct gaagtctctg tttgttagtt tagtggtcac ctaataatta 19980
   acagagattt cattaaatgc ctagaagcaa aatatcttcc agtctttgcc catggcctct 20040
   gtgtatgcat tagggcaggc cttgaactct tacccaggga gtttacaacc ctgccttagc 20100
   ctttactacc agcttctgca gagcattaag gtcaacaggt ggtgagagtt tggagcctac 20160
   tccatctttc ctgagcgtat acacagccct actcatgcat gtggccctct agatttccag 20220
   gaatatgttg gaccctttca aagcccttac agactcccca gcttttcctc tcaatcttta 20280
   gactagtgtg ttgttttctt caacagttat ctgtcaggca gcagcaaatt aagagattag 20340
   cataaatgtt ttcaactcct ccacccgtca tgtgccccag ggaagcacta agccagttct 20400
   aagttaggca aaataaagac aatccttttg aggtggtctt ccatggagtc accagacagg 20460
   taaaccaaat aattaattac aagtctttgg ctggatacag tggctcacac ctgtaatccc 20520
   ggcactttgg gaggctgagg caggtggatc acaaggtcag gagattgaga ccatcctggc 20580
   taacacggtg aaaccctgtc tctactaaaa aatacgaaaa aataggtggc tgtggtggcg 20640
   ggcgcctgta gtcccagcta ctcgggaggc tgaggcagga gaatggaatg aacccaggag 20700
   gtggagcttg ccgtgagccg agatcacact actgcactcc agcctgggtg acagagcaag 20760
   actccgtctc aacaaaaaaa aaaaaaaaca agtcttcatg aaagaggtcc attctgctgt 20820
   ctttcatacc aggaatgtgg aatgtggact gttattttca tggctactgc taagctagga 20880
   atcaagggat agatggggac tgggtaaaac accacagagt ttgctgttct taccaagaat 20940
   aagctgggga agagggttgt ttttgttttt cagtaaaaat tccctgggct gcttcaagcc 21000
   gttgattaat tttcaggttc cgaaaaagtt cagtttgaca gtttttgccc tttttatttg 21060
   cttttatgga tatgtagaac ttgagttctt ttttccacca gttttgctga cattgtttta 21120
   aaagcacttt ttgtaaaacc caaatgttgt ctctctcaag gctagccaat aattaaaaat 21180
   actgttactc ccctttgatt ttggaaatga attcgtattg accaaaattc aatactagag 21240
   gtctttcaag ctgttttacc atttatctaa actttagaat ctaatgattc ctgtacattg 21300
   tctagcatac tggtggtcct caattgtcat aagttcaact ttggaacaaa tgaacttttt 21360
   gtgtgcaagt ttccaattgt ttggaaatta cattgatgcc ccctccatca aactgttatt 21420
   cgtgggacat ctaggaattt cttacagcag ctgacaaata tttcaagtca gtgcctggta 21480
   gtactgtcca ccaggcaaca gcttcagtag tagagcgatc tttatctata aggcagtgtt 21540
   tgagcaattg tttattagtg ttttcctaac tactcagaag aactatcagg ggttatagag 21600
   gtagctcaga gagttgggtg caagtagaga aatccacccg gcttgcatta cacatcttat 21660
   ttctagagaa gctttccttt gaagaaagag ttctaaggtt taaaaaatta ccttgaatgc 21720
   cacttatatt gcattttaat tttattttag agaaatcaat ggaaagtaga aaaattaagg 21780
   cactgatact agtgttaaga atgttggtta aagcttctgg caattaattt tttatttcct 21840
   tttttaattt tattaaaatt taacaatttt cagtttatgc tgtaatccag accaaggttt 21900
   caatctaatg aagttaatgc cagtgttgct gctacctatt ttgtctttag tcattcagcc 21960
   atgcttccta cttatactga ataagctagc ttaatctaac aatcaaaaaa gaaagctgtt 22020
   gcctaagtta agaaaaacag tttgaactgt tttcaaacta aatacccagt agactctcta 22080
   gttgttgaca ggagaatgct taattcagaa ttgtcctgca gtagatcatt ttatctcatt 22140
   cctgttcttc tataggatag cttatttgtt tgaaattgta tttaatatgt tgtgattttt 22200
   gtgtgcttgt ttctattttt cactggatag actcaagata aaacctggta ccctgcagtg 22260
   tagctatcag tttatagcag aggaaattta cattagaact tggctgtgta tttacatgta 22320
   tctaacttgg aggtcactct gcttactgtt gatatatcag tcatattaga tgagtcccta 22380
   atgagatacc agaaaccccg gaaacatcat taggtggaac agtgtcctta atgctttatt 22440
   aagtgttata ggtaagacaa agcctagtac tatttgtggc atcaaggtta ggtgtttaaa 22500
   gacctgtatt cttctattgt catgttgaaa ttgttccctt gatgtagcaa tagaaaattt 22560
   tagattaggc ttaagttaat cagcaaacaa agataaaagt ctgatactat cctaaatatt 22620
   ttgtgtttct aaataattta acagtgatcc aattagctac tcctgtagaa atgtaattga 22680
   taaacttttc actctctttt aaattgccat cttgaatttt acctgttttt taaagctgtc 22740
   tcaagtcctc tctaaaaaaa ggcagtcatt tataaattta gaaaagcttg atagcacaga 22800
   aagtcacaga aaaatgtaaa catagtttaa aactgaattg tatacaagcc actagaagta 22860
   cttttattaa gtttacaaat attagtagag tggaactcat gcatttaata tgtttgaaac 22920
   ttttgatcaa atactgtgct atgaaaaaca ttttagataa ttattcttta atcatgtgtg 22980
   tgtaaaatgt ggcttttttt gacaaccaag tagcttttct gtgtgccaaa ctgtgacttt 23040
   aaaattttaa agtactcaac agagtaaaca aaccacaaat accacttaaa ctgtacacat 23100
   ttgcacatgc atttcctata aatagtacat gggtttcaag tcttcacttt tgaaattcag 23160
   aaatgggttt tttctccttc cagtagaaat aaaaacttga tttattttat ttatttattt 23220
   attttatttt tgagacggag tctcgttctg tggcccaggc tatggtgcag gagggtgatc 23280
   tcagctcact gcaacctctg cctcctgggt tcaagtgatt ctcctgcctc agcctgccga 23340
   gtagctggga ttacaggtgc ctgccaccat gcccagctaa tttttgtatt tttagtagag 23400
   atggggtttc tccatgttgg gcaggctggt ctcgaactcc tggcctcagg tgatctgtct 23460
   gtctcagcct tccaaagtgc tggggattac aggtgtgagc caccgcatcc agctaaaaac 23520
   ttgattttta aaaatccaaa tcgaagacag aattgtgtat tttagtacat ttattagcag 23580
   ccttgacgct ataccatatg gctgtttatc atttaaacag cttgtaaaag caaacacttc 23640
   aggattcatg agtggcagaa ggactgagta ctttgggaaa taagagagaa cttttgttga 23700
   ggatggttga ggaagagtcc aagacaataa taggcagaat aagcaaaaat ctagagactc 23760
   attgtaggca ctcaagtatg tatttgttag aatgaatggc tgaacttggt atattgagga 23820
   acactgagaa agccatactg actggaagat agttcctaca agaaactggt gagacatatg 23880
   ttacagtcta gattttggtg agccttgtta aagtttgggc tttattttta tacggggaga 23940
   aagtttcaca ggggtttgga aatgaggctt ggagctgtta atggggacac agtgaggttt 24000
   tagggtagtg gctttcaaac tgtttaaatc caaactttga tgataaccct gacataacta 24060
   ttgtttataa cttccatttc agttgtattg gttttatcaa aacatcttca ttgatcttac 24120
   tgattgcttc ctatgcagat taatattata aatttgaatg tacaaaggaa gctttagcag 24180
   taaaatagca acttttatct gtcttacgta ttggaggttc tgcataagat ttaatttttt 24240
   ttttttttga aatggagttt tgctcttgtt cacggggctg gagtgcaatg gtgtgatctc 24300
   ggctcaccac aacctctgcc tcccgggttt aagtgattct cctggctcag cctcccaagt 24360
   agctgggatt acaggcatgt gccaccatgc ccggctaatt ttgaatttta gtagagacgg 24420
   ggtttctcca tgttggtcag gctggtctcg aactcctgac ctcaggtgat ccgcctgcct 24480
   cagcctccca aagtgctggg attacaggcg tgagccaccg cgcccggcca agatttaatt 24540
   ttttaaaaga aaatattttg ctaagggttt ggaaactctt gttttagcaa gaatggatta 24600
   agactgatta aaactaaagg caaagaggag gctcttatgt ttggaattct ttgctaatat 24660
   ttacacaata taattctctc cacaaatatt taatggtacc agatattaga tggttataat 24720
   ggcaaaagtg ttcaaaggat gctatcatat tcatgattca tgatcaaaat gaacattata 24780
   aggctatccc tcttcagaat taaatacgtt actcctgtgg aaaacttgct tttaatgtag 24840
   aagttgtccc agagcctttc ttcctttctc atgtcctctt atgtccactg ctgagctaac 24900
   atgggtctca ctgaatgatt aagaaaaaac atcttaggtg gggagttctg tatatagtaa 24960
   atgtttaatt tattggggtg gtgaacggga agtgctgctg gcaagagagg atgggaagag 25020
   aaatctaccc aaatccttac ccgctttaca gaacataaac ttcctattca gtagtacaca 25080
   ataacttaac gatcaaggca tcttaacttt tctgttttca gatgaaagaa ctatcgtttg 25140
   gcttgatcaa gtatttagta tttattcgtt cactcaagtg cttacgtttt tttgttatct 25200
   cagggtttta cgttagttat taaccaaaag aactagtttt agttctggaa gtctaaaata 25260
   tataagagaa ggtgaggagt aataagagaa gatgaaggga gactttcgga atggcctatg 25320
   aacttctagt aactatacca ccttaaaata gacaaattac aatgcagtta tgaagatatg 25380
   tatttttcag tgaagacaac taaaatgttt gcacagaatt ttctttttta ttgagtgtta 25440
   gaaattctat tttggagata ctaccttgca caacataaaa agaaaaagtg agtgtggaat 25500
   ctaggaatct acgtggctct aggaaatttt ttaagtgtgg aaactgaagg agagcaagag 25560
   aaagggagca tggcattccc ctgtttgtag ttcatgaggt gggtttaaat tgccttttgc 25620
   caatgcagct gcacactgag gattacagaa ttctttttaa atgtttgtag aattattttt 25680
   cacttattag gtaaaacgtg tattttttga ttttctccaa tttcagcttt ctcatgttgc 25740
   tatgctcaat tttgtatacc atatatagtt ttgttaaatt gacaaagtgg tgttttttgt 25800
   tcttcttttt cccattggtt aaaatttaaa gagaaagtgg aagctagaaa tttatctaaa 25860
   aaatgtaact ttccctgtaa ttattaaagt atcaaatcta aatttgaatt ttctttgtgc 25920
   ataatctttt ttcaagctat ttaccatgtt gacaaacttg ctttcctgtg gcaaatacac 25980
   tagcaatacg ttataaatat gtaactttca acctatttac agttgatgct tttttagccc 26040
   tttggattta aaatacaagc actgaagagg tgaggaagta ccactgctgc ctcagcatta 26100
   tttcgaaatt ctgtttataa actatacaat ttccaaggtc atgaatccag cacctttcca 26160
   ggtactaact attgggacaa agatagaatt tgattttatt tatttaccta ttgactgaag 26220
   tctaacttaa atcttgcacc tagtaagatc ttagaaataa cgtgtgtact ctgacctgta 26280
   aactaatcct agtattctgt gtgtatattc tttctcattt gggctcttaa aaggaaaagt 26340
   aacgtacatc tgatgatcat tagcactgag ctttttcagc aaaaagtata tgtttataaa 26400
   gaagtatagg ataatttagt aatttaataa tgtgacaaca tttgcgtgtg tttttttttt 26460
   tgagaaatac aaattgtgag aaacagaaaa gtaaaagaag cagcagcaga aatatcacta 26520
   taggatcaaa agattgcagg aaccaaaact ccaaaattat tgggcataat gtactaaaaa 26580
   cagggcagtg gaggaaaggg acagtccaga ctagctctga gggtccaaag aaagtattaa 26640
   atattgttac tggagtgatt tgctctgcta tttgggcttg ggaattaagt gaaattgttg 26700
   atatactaga cagatacttc ccacccattt ttctcttgat aatcagggtt cattttttct 26760
   attttctatt tctctggatg ctccatttct taatattaat attaatatta agctctcagt 26820
   ctttatgcta aaaattggtt atttaaaaca atttaaatca acttcagtct aattggctta 26880
   agttcaaatc cattttaaga tcgatattgt gtcctttaaa aattttattt aaaagatatt 26940
   taaactgatg agaggatact acccattcca ctgataaact attactgtaa gtttgtctat 27000
   tgagggctag ttatttggtt taaaaatgct gagattatgg aaagtggatt ggaatatttt 27060
   ggagcaatat taaaaacagt atctgtaaca atttaataaa cttataaatt cctctttctc 27120
   tgttgatcta tcttgaaaag acactctatg tctctaggca ttccttctct gtggtgtgat 27180
   tggtagacag ggagtaaaca acttactgta aatgggcacc atgccagttg gcttcaggca 27240
   gcatcaagct tgtgactcac agtcagggtt aggaaaatgc cttttaactt gtttgtctct 27300
   gcctctttta aacattaaag gcacaactgt actaattatt aagtatttca taaggtcttt 27360
   tagggcttat aagatctttt aggaatggcc tggaagttat tagtactgtt tcattgaatc 27420
   tgaatacctt taacatgata atgagaagtt tttaaagggt ggttttatag ttaaacggaa 27480
   tttctcaaat tggcttgctc cttatgttga tttatttagg atcacatttg ggagtttctc 27540
   tgccctactt tcaatgtatt taatttactg accatcacta tttgggggga aaatgttata 27600
   tgatatttag aaaccaagag ttttggagtt tttcccccat tagatgtatt tatttattta 27660
   tttattattt tttaaagaca gggtcttgct ctgtcaccca ggctggagca cagtggcatg 27720
   atcctagctc actgtattct tgaactcctg ggctcagact gtcctcccac ctcagcccaa 27780
   gtggctaagt atcaagtaag aatcacctgg caaattccaa ggctgtatac cagatttcct 27840
   aaattagaat tttggggttg ggtatctgaa ttttagtaaa gccctccaaa tgtttctggt 27900
   attgcttcta agaacaattg ataacataat agctgtggcc attatagggg tattctgtca 27960
   tatttagata taggcatacc ttgttttatt gtacttccca aatattgcgt gtttattttg 28020
   ttttgtttca cttacaaatt gaaggtttgt ggcaacccta tattaagcga gtctgtcagt 28080
   gccatttttc caacagcttg tgctcatttt gtgtctctgt gtcacatttt ggtaattctc 28140
   tcaatatatc aaactttttc atcatttttg tatctgttac gaccagtgat cagtgatctt 28200
   tgattttttc tttttttttt tttttttgag acggactttt gctctgtcac ccaggctgga 28260
   gtgcagtggt tcaatcttgg ctcacagcaa cctctgcctc ccaggttcaa gcaatcctcc 28320
   tgcctcagcc tccccagtag ccgggcctac aggcgtgtgc caccacgcct ggctaatttt 28380
   tgtattttta gtagagatgg ggattcccca tgttggccag gctggtctcg aactcctgac 28440
   ctcaggtgat ccgctcacct tggcctccca aagtgctggg attaccgtgc cagcctgatg 28500
   ttactatttt aattgttttc aggcaccata aacctcacct gtataaggca ccgtacttaa 28560
   ttggtaaata ttgcgcatga tctgactgct cttccaactg gccattccct gtctgtctcc 28620
   ctcttcctgg gactctcaaa tccctgagag acaataatat taaaattaag ctaattaata 28680
   accctacagt ggcctctaag tgttgaagtg aaagagttgc atgtctctca ctttaaataa 28740
   aaagctagaa gtggctaaac ttagtgagga aggcacatca aaagccaaga caggccaaaa 28800
   gcaaggactc ttgtactaaa cagctaaatt gtgaatgcaa aggaaaagct cttgaaggaa 28860
   ataactagtg ctactccagc aaacatgtga atgatcagaa agtgaaacag ccttcttgct 28920
   gatacgaaga aagttttagt ggtctggaca gaagatcaaa ccattcacaa cattccttta 28980
   agccaaagct taactctctt caattctatg aaggctgtga gaggtgagaa agctgcagaa 29040
   gaaaaattgg aagctagcag aggtcggttg atgaggttta gggaaagaag ccagcgctgt 29100
   aacataaaag tgtaaggtga agcagcaagt gctgatacag aaactgcagc aagttatgta 29160
   gaagatctag ctaagattac taaataatag attttccatg tagatgaaaa agccttttgt 29220
   tggaagaaga tgccatctag gactttcata gctagaaagg agtcaatgtc tggcttcaga 29280
   ggacaggctg acattcttgt taggggctaa tgtagttggt gactttaagt tgaagccagg 29340
   tctcatttac cactccaaaa atccgaagac ccttaagact tatgcttaat ctactctgct 29400
   tgtactctag aaatgaaaca acaaagcctg gatgacagca catctgttta tagtatgctt 29460
   cactgaatat tttaaggcca ctgtaaagac ctgttcaact gctcagaaaa aaatgattac 29520
   tttcaaaata ttgctgttca ttgacagtgc acctgggctc acccaagagc tctaatggaa 29580
   ttgtacaaca agatggatgt tgttctcatg cctgccaaca catcatccat ttgtagccca 29640
   tgaatcaggg agtgatttca agtttcaaat cagtacattt tgtaaggcta tagctgctat 29700
   agacagtgat tgctctggtg gacctgggca aagtaaatca aaaaccttct gaaaaggatt 29760
   ggccattcta gatgctatta agaatttgtg attcgcagga ggaggtcaaa ggatcaacat 29820
   tagtagcagt ttgaaagaag ttgattccaa cagttataga tgaatttgag gggttcaaca 29880
   cttcagttta ggaagtcact gcagatgtgg tagaaacagc aagagaacta gaattagaag 29940
   tggagcccga aaatgtgacg gaattgctgc aatctcatga gaaaacgtga atggatgagg 30000
   agttgcttct tatggacaaa tgagcaaata aatttttttc ttgagatgga atctactcct 30060
   ggtgaagatt ctgtgaacct tgttgaaata acaacaaagg atttagaata ttacataaac 30120
   ttaattggta aagcagcagc atggtttgag tggattcatt ccagttttga aagagtttct 30180
   actgtgggta aaatgctatc aaacagcatc tcgtgctaca aagaaatctt ttatgaaaag 30240
   aaaagtgaaa cttcattgtt gtctacttta agaaattgcc acagccaccc caccttcagc 30300
   aaccacctct ctgatcagtc agcaggcatc aacactgaag caagaccctc cacaaggaaa 30360
   aagattacaa ctcactgaaa gttcaaatga ttgttagcat ttttaagcaa tattttaaga 30420
   ttaaggtaaa tacattttta aagacacaat gctattgcac acttaataga ctacagtata 30480
   gtataaatat aacttttata tgtagtggga aaccaaaaaa ttcgtctgac ttgctttgtt 30540
   gcaatattca ctttattgtg gtctagaacc gaacctgaaa tatctcagag gtatgcctgt 30600
   attaatatta ttttgcaagt aaaaaaccca gcatataaaa aaaacgtaga atatgttgag 30660
   agttcagtaa tatggatgaa aatgtttttc tctaactgaa gaacatgata aattataatt 30720
   agggaaggat ataaaccaag aaaatatgtc tgagatagcc aattcttgca gttcataata 30780
   tgaaaactca ttataccaat ctcagtaaga atacttttaa tagctgttat ttctttggga 30840
   tatagaattt ataaagtaca cagtaatctt cttatgatca atcctaggat cactttacaa 30900
   ccacttaccc catattacaa tgtagtacca agacaagcag accaaattat agaaggacaa 30960
   agtttttgct aagcatattt tgtcatcagc ataccgcatt gtgtgtgcat gcatgtgtgt 31020
   gtttgtgcat gtgtgtgatt gtataaaata ttagaaagcc accccagaaa agttaaatga 31080
   ctaggaatgt tgtgaaggga ttaagctacc cctaaaatta tataacaaaa ctctcttcat 31140
   ctattattag gtcatcttta gaacatcttc tcttaaattt gttataggtc tctctcatct 31200
   gtttggatta aaattggtct gaaagcctaa aatggctttt tacctatata attatttccc 31260
   aactagcttg tagtataggt gcaaagctat cacacttgct aggttagtga agtatgtaaa 31320
   aactaccatc tttcaattag gaaccatcgg atagcttcta caggattgct ggggagaacc 31380
   tttataaaga aagttatatc tttataaatt ttttgtcatt ttacttagct gagaatataa 31440
   aataagttag ctaataatag agtagaaatg ttttctgtaa cagattaata ttgatcaaat 31500
   gtgttattaa atgctaaaac accatttttt ttctctgtaa gccatgtgtt tcatgccaca 31560
   acacaaaagg gacaattgtc tgtgttttat gacagttctg ttctgtcaga tgctgtttgt 31620
   tcattttggt gaataaatga agagagccct ggacacatct ttttttcctc aacaaaagag 31680
   gaaaattatt cttgtctgta tgtctataat cctgactctt tgaatggctt taattttttt 31740
   aaagtcagca tttttttata aagataggtg tttggaatgt gggcgatatg gctggacagt 31800
   tagattggga ccaaataatg gaaggctttg aacatcatgc taagaggttt gggttttact 31860
   ctgaaggcag tagagaacca ttatgttttt aagccaggat tgacttgttc taagctgtac 31920
   cttagaaata ttactctggc agttgtacat aggatgagct gtatgttgct ttgttttgtt 31980
   tggggagaca gttctcgaag agagactaca tacgaaggca gttatatgag tcattactaa 32040
   aggtctggca agaagtagta aaagcattaa ctggagtggt agcagtaggg aaggaaataa 32100
   aaggatagat gtgggagtca tttggaaagt atgaggcaat tcattgacct tacagaatca 32160
   ctggttttct gcttccactc cattcacatt gacctttcca aggttatcag tgacctgctt 32220
   gtccttaaat tcagtgggca ctttccagta acctactgtt ggcaccagcc ctgtgctaga 32280
   caccaggatc ctgtttgtaa aggcatctgc cagtggtttc tgtgacacaa ttctgtttct 32340
   agttttcctc cttctacttc tctagcctct tggcaagttc ttctttcaga gtttctcaga 32400
   gctttgtgct aggccctctt ctcattttct ccttctctaa gtgatcccat ccttttctgt 32460
   tgcttcagtt accatttgtc cttatgcaaa ggacagccat atctactgta tctccagctc 32520
   agatgtatct ctttgcctcc tgacccatat ttccaactat ctaactgggt atcttttctt 32580
   ggatgagtta taggtctctc aaacacaaca tgtccagaat aattcattga cttattctaa 32640
   ggcctgcttc ctctttctcc tgtagtccct atctcaggaa atatatggtg ctatcaaccc 32700
   caaagcagaa atctggacat aatccctaac tacccttttc ccctctctgt gcacataatt 32760
   tcagtcatta ggcctcatag attggactaa ataaatacct cgcaaaccct tctacttata 32820
   ttcttaactg ctcctacctt aagccaggct accataattt tgtagctgga tgactgcatc 32880
   atcatcttga ctggctccct tgtcatcttc aatctatatt ctatactgca gctagagctt 32940
   tcaaacataa acatgtgatc agattagtcc cctctttaga acaccctagg gttctcactg 33000
   tcctgagtac agtctaaggg tttaccatgg cttacagggt cttttatgat ttggtgagct 33060
   ttttattgta taacctttct aaactgcctt tacttccctc tttcttggct ctgtgtcttt 33120
   gcataatgct gttccctata cttcacctca cgtctaacct tcatctcctt ttcacttctc 33180
   ctcttcctcc aaaatccagc tgaatatcac attgtcatgc aggcccattc ttgatctccc 33240
   acgtttgggt tagatatccc tcttcagtac catcaccgca ccaggtgtgt cccctatcct 33300
   agcatttgcc tcattgtatt acaactactg tgtactcgtc tctacagctc ctgctagtct 33360
   aaaagttttg ggagagcaaa ggttcatgtt tgtgtttttc actgtggtat accccagtgc 33420
   ctagtatatg ataagctctc aaaatatttg ttagatgtat gaagaaatga gaaagagaac 33480
   aggaagaggg taagtttcaa gactaggaaa caaggctatg aaagctgcag gaaagcagca 33540
   ggttaaaacc tagaagaaga gtttgtttta ggaaatactg tgttttaaac cactataact 33600
   gaagcaaaaa cccaaggcct gggtgtggat agagtccact atctgataac agtggatact 33660
   gatgcatggc agagttggag aggaagagag ccagattcca aaacagaagg ggtaaagtct 33720
   tctaagaaga tagattatag taagaaggat taggggatag aaatatgagc ctgttccact 33780
   catagatctc aaacatgaaa tgatgagtca tcatgaagag agtaggcaat tgtccagtga 33840
   agaaggggat gctaaccctt cttaaccttg aatctctcag gtagaagcag ttagagaagg 33900
   aacagccatc atcagatagt gttgtaagga aaatgatatc cttggggaaa cctgcatttt 33960
   ggtaaagcaa agcaactaag aaagaatata ctaccactgt ttaacaatcg ccacaaaaag 34020
   acagtaggat catctttgac ccccctcatc ctttctcagg aacttggagg actaagaaga 34080
   gagaaatctg tagaagaggc ttctctctct gatcctccct ccacttcagt tttaccacat 34140
   gtaatgcaac aataattaag aatttgtgta aaatttcacc aggttggcat gcatggagag 34200
   aaaaattatt cagatgtttt cctttgtcaa taatacaagg agcatttgta gggaaaaata 34260
   tttacaaata cagtaagacc tattctcttt ctatatttat gggaaaattt taagttgtgc 34320
   ccttgtttca tgtgtgtttc tatttaaaga taccatactt aatatatatt gttgattcat 34380
   taacattgaa ctcatggcta acagcactat aaatcatgtc tgatcaaaac ttatgataca 34440
   tgtactttct tcgtaaggta catcatagtc ttctcgtaca tgggaactct aggtagtact 34500
   tcaggactat gcatagaggc cattttaaac agcaaaattc ccaacaaaaa gcacaaaact 34560
   caaaaaatgt gccactaaat ttaccatgaa aaggacactt gtttacagtt tgagagctaa 34620
   aacaagaagg tggcgtgtca cttcgtttga cttcagctgg gaacatgcat atcagtcgac 34680
   tcaaattttt tgctattctg tgcttatcca cgaatcgata ggaaagcaag tgtggatttg 34740
   ggggttacaa ataaaatgta gcaaatgtgt aaacttgcag atgtggaatc tacaagtagt 34800
   tagaatcaac tatgttagtc tgatcattaa atcagttttt taaagtacta ttgtaacacc 34860
   ttataacctg ccccattcac tgagtgttgt agtttatagt ttcattgggc attttcagta 34920
   gttttatctg aagtcacatt tcaaattttg taattgaagc tccaaagtat gctaccggaa 34980
   acacgagctg atgctgtgag acaaaatcaa caggtaatcc accatcacaa ctgtgggcta 35040
   gaatgctcaa gaaaccttgg aggcccagag agctgagatg aatactgaag aatcataggc 35100
   aggtttactc tgtcaagctg cctgtatttt gagggtgtag tcctcaaacc aaaaagacac 35160
   caaatgaaca aactcagatg gcctcactgg ggaacagaga ttgaaagctg acactggaat 35220
   gtgtacttaa aaaaatgaga gcccgttttg gaaaggcaga ctgggcacag aatgtggaga 35280
   gctatatttg ctaactgaag aaatttagac tttatcctct acaaaacaaa gctattggtt 35340
   tttgaaggtt gcataaaagc tgcattttag cagcatatat tttggtagag ctgttacctg 35400
   cctgaaaaca tcaatgtcat ttcacacaaa tgatacttat cccttggtgt ttgatctaaa 35460
   tttctacaat gagaatgtga ttttatagtc tttactgggg aaggaagtag gtttttcagg 35520
   ccgaaattct tgtgtagcaa aaattaacac ttaagttagc ccttggcaat ctccagttct 35580
   ataatggtaa aatggatttc ccagaaagtc actctctatc cctttgaata gacattagaa 35640
   ataacatgta ctttaagtgg gatttacaga ggaagggggc ctttaattct ttactagtgt 35700
   gatgccctgt aaaaaaataa ctaacattag agttgaggcc tagaaatagc agcactgggt 35760
   taaagtctgt tttcaagtgc aagtttttct ttttattcgt gtgtgtgtgt gtctgtgtgt 35820
   gtttcacata gaaggaggaa atgccaattt cagttcttac aaatattaat gactgcaact 35880
   tataaaaatg ttacagacta tattcttccc ttttgtaaca gatgagaaga ttttgaaatt 35940
   tagtctctac tttttagttt ggtaagacaa tttgaataaa ctgcaataat tgcaaaagaa 36000
   ttctgaatat ttgaacattt gacattttct atgtcaaata tacatttctt gtactatata 36060
   aacattctag aaaagagaga caggcaggga ggaaagtgct cattaaaaag agcttcaccc 36120
   tctctgaaaa gggatttcct ttacagtgct gtgtactaaa gcctgtgttg taaatcagaa 36180
   agcactgagc acacatgttg ctgctttggt agcatcagaa gtcgattttc attagcctta 36240
   taccattcac tatttctgcc aagcaatctt aaattataaa agaatcttat ttgattttgt 36300
   gattctcttg ttttctgctc ataaagaaaa tatcctaaat tgaacaatgg catgctacgt 36360
   ttttagtttt taagacagct aatgtgtaaa aagacattta aagtatagtt gtgttaagtt 36420
   tttgaagttt acagttgttt caattttgct gctatacttt gttaacatat tttaggaata 36480
   tttcatttta gtcacaacta ggatataaac attattttgg tggcgatctc cttgtaatca 36540
   cgacatcaac caaatttggg aaattttgat ttgttagatt tataaatttt acagtaacac 36600
   aaaagtctaa tttcctatat attttcaagg cccctatacc tttgtcaaaa taaagtatca 36660
   atgaaaaatg aaaaaatcat aaactatgtt caggccaaac tgatactgac tttgttaaaa 36720
   ggctagatag aaatctgttt tcctcttctg ttacatctcc tcttctggag accactctgt 36780
   gtggactgaa ggtttgagat cctaggacct aggctagaac agattaggag attgtgctgt 36840
   atgttaagtg gcagatacca tggaattcta agcctgttac gaaggaggag aagaagaggc 36900
   acaatgaccc tgacacagcc cctgggttga ccacagcaga tatctcactt gagcaagtag 36960
   atatcatctc aattgcttgc tgattatctc taacttgtca gtaacttact ttgataacct 37020
   agatttagga gtctgacagc atgcagtgta tgcctcataa taatctgctg tttatgaaag 37080
   tcataacatt gtatgtttag cataatggtg aagagcctgc catctggaat ggtctactta 37140
   tttgggatcc acatacagta agctctcact taacatcatc agtaggttct tggaaactgt 37200
   gaccttaagc aaaacaacct ctaatgaaac caattttacc acaggctaat tgatataaac 37260
   aagagttaag ttcctgtggc atatttctgg tcacaaaaac atcactaaac ttctaaataa 37320
   agacccaaaa cacttataat attaaccact gaaataaatg tgagctatat atatacattt 37380
   aagaataata aaaacaaaaa ataattattt acccaatttt tggtgaacca gtgagtgata 37440
   gtgatcatag tgatggtgga tgaaatcaag gaataaatat ttgcaaagtg aaaattgtaa 37500
   gaagcacccc ctgtcaccac atagctcaga aataataatt agggcaggct tgctgagcat 37560
   ttttaaactg cactgtttat tgtcatgcat ttgaatgatt atcgcagact ttatgaattt 37620
   tcattttata ataatttgta ggccaggcac agtggctcac gtctgtaatc ccggcacttt 37680
   gggaggccaa ggcaggcggg tcactggagg tcaggagttc aacaccagcc tgaccaacat 37740
   ggggaatccc catctctact aaaaatacaa aaattagcca ggtgtggtgg tacacacctg 37800
   taatcccagc tatttgggag gctgaggcag gagaattgct tgaacctggg aggtggaggt 37860
   tgcagtaagc cgagattgtg cccctgcact ccggcctggt gacagagcta gactctgtct 37920
   caaaaaacaa taataataat ttgtattcat tcattttcca atgtgttcat tccagttcag 37980
   ggtccagggg gcctgcagct tatactcata gctcagagca actgacccta tagacaggac 38040
   gccaccccat tgtagggtgc actcaaatgc acactcacac tcaaactggg acccttcaga 38100
   catgccagtt accgtatcac acacagcttc gggatgtggg aggaaagcga agtatctgga 38160
   gaaaaactac acagacatgg gaagaacgag ccaactctac acagacagtg gccctggaca 38220
   gagctgggca ggcatcagtt ttttttcttt tttttgtggg gggtgagggt ggggcatgga 38280
   gtcttactct gtcacccagg ctggattgca gtgcagtggt gtgatctcag ctcactacaa 38340
   cctccacctc ccgggttcaa gagtttctcc tgcctcagcc tcccaagtag ctgggattac 38400
   aggcgcccgc caccacacct ggctaatttt tgtattttta gtagagacaa ggtttcacca 38460
   tgttggccaa gctggtctgg aactcctgac ctcaggtgat ccacccgcct tggcctccca 38520
   aagtgatggg attacaggcg tgagctaccg cgcccagtca gcatcatttt ttttttctca 38580
   tcaacgttaa aacaatgttg aacaaaacat tattcaaaga cctgccgtat ggctattttc 38640
   tagttgtgtg actttctttg ggaaagttag caaccctttc tgagcttaaa tgtcctcatt 38700
   cataaaatgg ggctagtaat aatgcataag gtttttgtaa gaattagaat taataaagta 38760
   cttagaccat aataactaat tagtattagt tgttgtcttt gctattattt tgatgtggtg 38820
   gttgtttggt ttcacctgtg tactatcagg acatgctgaa ataaaattta agaattggct 38880
   ttataatatt agaaaagcaa acttttgtac gatatgggta tgaaaaattg ttgggagtct 38940
   actttttctc tcttacctaa tttgtcttag tctttttaaa gcttagattt tccaaatgag 39000
   ccatagcaaa atataatgtt taaaaatgtt taaattctaa gcactatgtc atagttaaat 39060
   aacttaaagg tgctacatct tatacagtcc aaaaggaaca taattagtaa aattctacaa 39120
   tttagaaaaa aaaatagctg acagtgactg atttataaaa gtaaaatatc ttttgttaat 39180
   actaatattc tttttataaa ttaattgatg acaaaaaatt gagtgaatga gatttgcagt 39240
   tcatttatct atgatgctgg tttatttaat ctctataatt tgctgtattt gaaagagcat 39300
   agtgatagag gtcatgataa aatctaggcc cagtgccaca agtaaatccc tgtaggaact 39360
   ctcaaggttt tgatttcatc tctgaatggg aataacacct tccaagaata ttatgaagat 39420
   taaaaagtta cgtatcataa atacacacag agtaacaata ctgggaatat tgcaacttgt 39480
   aagaaagagg aagcatatgg catattctga tggttaggga tatggactct gtagctggga 39540
   tgcctgaaag agaactctga ctccactaat ggctagttat atgaaattgt gcagataatt 39600
   taacttctct gagtttgcat ttttctttgt ctatataatg gggataataa tagtacctac 39660
   ctcacacata gtgttaattt ctattagtgg ttctcattaa gatagtattg ttgttcatcc 39720
   ctggttgtta gccatcatgt atctgagtta gagagtcatt gattttagaa agtcccgagg 39780
   agactatcag gtcaagcaac ctgcctcctg ctagacaatt agctttatcc atgagttacc 39840
   aaagagggag ccgaaaccca gggaagctga aagagctgtt gattgtcacc ctgtgagttg 39900
   gtgatagaaa gatatctgga atcccagtag ttgcccattt cctagttctg ggctctgcat 39960
   tgcactagaa tactgtgcca ttctaaatat gaaaaggcag tatgaccatt gtgcttgtca 40020
   ctttccattc cctagatgct atcttatatt tgtccttatg aaatttaacc tgtgactttc 40080
   agatcactta gaaccttggt tggacagtgt tttctagtgt tatttagtat atttttttgt 40140
   catcttctgt tgtctttggg ttcccctaaa agagctatac tctgggtgcc aggaaacttc 40200
   acacatgact gtcttctctt cctcgacttc cctctctact tacctttcca gctcgtagca 40260
   aatcagaaga cttctctgac acctctctat gtctaaaggt cctttgatat tctcacatgg 40320
   cggcatgaat cacagtgtat tttaactggc cttttccttg tgtgtctcct acaatgagct 40380
   gttgaagctt catgaaaaca caatctgttt tactcagggc agttataatt ccaattacaa 40440
   agcacatttc ctggctcctg gctaggaact cgatcatttt tcgatgcttc cttgctcagg 40500
   actttctgat tccttcttaa aacattttgg ggcatctcct tctcctggtt tttggaaaca 40560
   tattctcata ctgctatgaa ggtttttact gacatttcca acttctctta aattgattca 40620
   gcaaatgttt ttccataata aatgtcattg atatgtcatc aatatggaga gcaacaacag 40680
   aatgcattga gtaaactcct cccctggagg tctgagaatc tagattccag ttctcacaga 40740
   gccaccacct tggtgacctt ggacagtaga ccttctaagc ctcagtttcc ttatccctta 40800
   agtggggata ttaatagaac ccattctcag agatgttgcc aagattaaaa taaccaagat 40860
   aattcctgta gatgatttgg catagtgcct gccacgtact aagcaagagt tagcctccgt 40920
   cattatagta tgatcataaa aaatgaacag actaaacgaa gtaaccagaa ggaaagaaat 40980
   tttaattctt aaaatgtaat agtttcttgg tttttttttt tctgtgaaac acctgcatgg 41040
   cacctttttg ttattcatac tgttttgact gtggctgtcg tagattcttg ttgaaagtct 41100
   gagagactga gacttgtcat tttgaacatg gcatcagtgg aacagcttat gattcaataa 41160
   ttgcatcatc ctggacaagc accagtagaa gtgagtcagg acatgtgata aaaagacatt 41220
   cattttgccc ctcctccctc tctgtatttt ctttgctata aaattattga tgttaagccc 41280
   atagtactaa tatttcagtt caattcataa taaaatttga gggcatttga atatattatc 41340
   tgttgtaaat tataatttta tatttgacca cagagtattt gaagtgggtc ttttctttcc 41400
   ccaaaattct attttaataa ctaaaaaata ttcttaggag aagtattatt taagaacagg 41460
   tttatattaa ataacatcat ttcactttca actttctggt ggtcaaaaaa tatgctaata 41520
   ctaattagga tatgatacac atgttctgtt agaacagttt tggcagttag aagacttctc 41580
   ttcttgtgtt tgaaagggat gttacttggg gtagttatga gccatgtatc cagatgtcct 41640
   gaaaggacca gtggtagatg tatttctatt tttgtctttt cttttttctt tctggcattc 41700
   tagttgctga gtgactgact tttgttttca gctcttctca caatcaccat tgttctaata 41760
   actttgctta aatagaatgt ctccttttgc tataagccat ggggccattt accgttaatt 41820
   ttttaaagta ctgaaatgag aacctcataa attaaagaac actcctgatt ctgagttagc 41880
   agatcctact aagccttttg cagatggaaa tttcctttaa attggtttgt tttcctttaa 41940
   cattccatta tcctattgtt cattctttgg agctgtgatt tgtttaatat atttcaggct 42000
   tcttaataaa tcaagtcatg taagttatta tttggatcat ttcgaaacta caacagctta 42060
   tcaaacctct gaaagaagaa ttttgtgttt gcccacagac tgaagaactg attcagtttt 42120
   attggctgag ctaccttcat tattcatatt taattcctgg tactgagggt gggaggaggg 42180
   agaggagcag aaaagataca actattgggt actgggccta atatctgggt gatgaaataa 42240
   tatgtacaac aagcccccgt gacatgtgtt tacctattta acgaaccctc acatgtatcc 42300
   ccaagcctaa aagtttaaaa atatatattt ggtaaatcaa ttgatgtgtt ttaaaaaata 42360
   tcgccttttg gccgggtgtg gtggcccatg tctgtaaccc cagcactttg ggaggccaag 42420
   ccgggcggat cacgaggtca ggagttcaag accagcctgg ccaacatggt gaaaccctgt 42480
   ctctactaaa aatacaaaaa atagctgggc gtggtggcgc gcacctgtaa tcccagctac 42540
   tcgggaggct gaggcagggg aatctcttca acccaggagg cggaggttgc agtgagccaa 42600
   gattgtgcca ttggactcca gcctgggcga cagagcgaga ctctgtctca aaaaaaaaaa 42660
   aaaaaaaaaa aatcatcttt aaagagataa ctaacccttc cccagaaggc agggccaaag 42720
   tctaaggttc ttccaggtcc tttgtattcc ctataaattt tagagtcagc ctgtcaattt 42780
   ctatacacac acaaaaaaaa gcctgctggg attatgattg gtattgcatt gaaattaaat 42840
   caatttgggt ataagagact tcaatttggg gattgagtct atattgagtc ttccaatcca 42900
   ggaacactgt atatctctcc atttagtcag atatttagtt tatttcaaca atattttcag 42960
   atctttagtt cctttcagca atattttctc atttttcctg taaagctctt gcacatcttt 43020
   tgtcccatat ctattgtgta tatgtgtttt gctagttatt aaattatatt aatataaatt 43080
   ttattttcca attgtttgtc gcatatatag aatgttttaa aaatattgtg tcctgtgacc 43140
   atgctaaatt aactaattct agtcattatg tcttcattat ctttctcttg aattttcatt 43200
   gtcttcccct tctgggactc cattcatatg taaggccatt tgatactgtc tctcaggtcc 43260
   atgaagttct gttaattttt cttcattctt ctttttctct gtgttcttca actgaatgaa 43320
   tgccattaat aatttggtat gtaatggctc acttaaactt ccttttgttt ttaagatatt 43380
   tctactctca gctgtgtctg gaatccttta gtccggagcc ccaccaaccc tcagcctaga 43440
   aggaaggagg agaaggatag ggtgaaagga aggggagagc ttctagcttc aggacagaga 43500
   tcagaacaaa caacagagca gtcatcttgg ataaggaaac ttccctcaaa cctattactt 43560
   atatcctcag aaataagaaa aataatgcat ttatcaaatt aaaggatttt gaaaaaggga 43620
   acattcagag aataaaacta aactcttgaa agttaaaagg atgataacat aaatgaaaag 43680
   ctcagttgaa ggattgaaag ataaaagtaa gaaaatatcc cagaaataag agcaaaaaga 43740
   cagcaatgta aaatagggga gaagataaga gaattagaga accagcttag gagttctaga 43800
   aagagaaaat gtagacaaca aaaggtaaga aatcatcaaa gactggagta ggggaggtca 43860
   tgctatctgt ttctttttct attttttatt ttgagttaca ttttttttac tgtgaaacaa 43920
   gcatatgtac atgagaatga acaaaacaaa tatgcagtca tgtattgctt aacaacagag 43980
   ataggttctg agaaatgcat cattaggcga tgtcatcatt gtgcagacat catagagtga 44040
   acttacacaa atctgaatgg tatgtcctac agtacacctg gaccatatgg tatagctgtt 44100
   gcttccaggc cacaaactta cagcatgtta ctgtactgaa cactgcaggc acctctaata 44160
   catcggtaag tatttatgta tctaaacata gaaaaggtac aataaaaata caatataaaa 44220
   gaggaaaaaa atagtacacc tgtataggtg cttactgtga atagggcttc caggattgga 44280
   agttgctgtg agtcattgag tagtgagtga atgtgaaggc ctaggacatt tattatatga 44340
   agtctactgt agtgtaaact ctgtagactt aggctacact aaatttatag aaaaattttc 44400
   ttcaataata aattaacctt agcctactgt aactttttta ctttgtaaac ttttaatttt 44460
   tttaacattt tgactccttt ttagtaacac ttagcttaaa acacacacat tgtacagctg 44520
   taaagaaaat tttatgtcct tcttctgtaa gcttttttcc atttttaaga tgtttttatt 44580
   tttaaaactg ttactaaaaa ctaatacaca aacacacaca ttaacctagg cctatacaaa 44640
   gtcagtgtca tcagtgttca accttcacat gttatcccac tggaaggcct tcaggggcaa 44700
   taacaaacac agagctgtcg ttttctgtga taacagtgcc tttttctgat atacctactg 44760
   aaagacctgg ctgagagtgt ttgacagtta acaaaaaaaa aaaaaggaca agaagtacac 44820
   tctaaaataa tgaaaaaagt ataatacagt aaatacataa accaccaaca tagtcattta 44880
   ttatcattat cgagtattat gtactgtaca cagttgtatt tgctgtactt ttctataact 44940
   ggtagcacgg taggtttgtt tataccagca tcaccacaaa cataagcatg gtgttgtatt 45000
   acaatgcaca gctgcagcta agtgatagga ctttttcagc tccattataa ttttatggga 45060
   ccatcactat aaatgctgtc catcattgac tgaaatttat gtcgtgcatg accatacata 45120
   caatttaatg aaaaataata ataataaagc tagcagtgtg taattaccaa ccagggcaag 45180
   aaatagaata ttgccaatac cttggaggcc tccagtatga ccatataagt ttacaaatcc 45240
   tattttgttc ctcctcccca gaggtaacca ctgccctgac aaatgtgatc gttgttttct 45300
   tgtttttctt actacctata taaacatcct taaacaatat aactcagttt gtatattttg 45360
   aattccatgt taatagaata tcatatgtat atgaatttta tgtgaataga atattatata 45420
   tgtcattttg catcttgctt ttttcattca acattgtagg attcattcat gttgtagtgt 45480
   acagctgtcg tttattcatt gctgtataga attatatcct cagagataag atatatggat 45540
   gtttataaat cattccacta ttatgaacat ttgactagtt tgtagttttt atttaaccaa 45600
   aaaaatgctg ctgccaacat tcttacacat tttactgtat atgcacatta atttatttac 45660
   aagtataaat ttctttttga atacatatct attgatggag ttgctacatc ataggacatt 45720
   cttgtctttg actttactgg ataataccaa actgtcttcc aaaatgatta catccttaaa 45780
   ctcaggacac atcttattgt caaatgttta atttttgtca gtctgatggg tatgtaagtt 45840
   attttattgt cgttttaatt tgcatttccc tgattactaa ttaagctgag taacttttca 45900
   tatgtttatt ggccatttgg agttcctgta ttgtaaagta taagtttttt tgtccatttt 45960
   tctagttttc tgtcctttta gttgaaatcc aaatttgcct aaatctgtta ttctctgagc 46020
   acaagtaact tgggatgctt tcctttagat ttagcctaat tctttatcat tttgtcagct 46080
   tgatggtgct tttaaggaga tatatatgtg tgtgtgcgca cacatgtgcg tgtgtgtata 46140
   tatatatatg tatatgtatg tatgtatttt ttgagacagg gtctcactct gtcacccagg 46200
   ctggagtgca gcggcacagt cttggctcac tgcagcctcc acctcctggg ttcaagcttt 46260
   tccctgtctc agcaacccga gtagctagga ttacaggtat gccaccatac ccgctaattt 46320
   ttgtatttaa tagaaacagg gtttcgccat gttgacaggc tggacttgaa ctcctcactt 46380
   gaactcctca cgtcaagtga tctgcctgct ttagcctccc aaagtgctgg gattacaggc 46440
   atgagctacc gcgcctggcc tggatatttt ttaaaaatat tttttatcta gcactttggt 46500
   ttttggcagg caggttggca ctcatagtct gacctaccat ttctataaaa agaaacctgt 46560
   aaatgttctt aaacagactt tgaaccagtc ttcctgattt tgaaccccta cctttacccc 46620
   cagtttttga gcctttcaga attttttttc ataataatta ggttgcttct tagctttccc 46680
   cactggtgac ttaacagatc ttaggaagcc aacaatcctt gtccatctgc tttctgtctt 46740
   gtgaactgtt gctggtattg tctcttctct ttattcttag aggtgtatgc ttttaaaaac 46800
   atatactggg tttgagaggg agctgaaata aaagcatgtg ttaaatatac catctttaac 46860
   cagaactaca tttgactggt cattttattt tcaagctcac atacacttca aacagagata 46920
   tggctaaagg aattatcatg tgaacaacag ccagggctct gaacatcaca gattatatca 46980
   tcatacttga aatatttgaa attttgattc aaaatgagag ctttatagct atgtcctcaa 47040
   tggactaagt gtttaagtac ttaacatcca aaacattctt actaatcaag agaagacaaa 47100
   caccccaaca gagaaatagg caaattttat caatagccag ttcaccagat ttgttttctg 47160
   ttagaagcga atatggggaa atacatgtgt ccatgttttg cctacttttc ctggagcagg 47220
   taaggagagg cagtttaagg atccatgtga taaaccctaa agttgtccat cggctttcca 47280
   gtcccttcta ggaatttaac ttagggaaat aatcagacat ttgcaaaggt gtgtacagtg 47340
   gtatttataa tagtgaaaaa ccaaagaatg accaataacg ggagaatgga agttacagcc 47400
   aaatacttta caactactaa agaatcatgt aaaatatcta ttgacatagg agttttatca 47460
   aaatgtgaag tatacagatg aatagtacca cacataaaaa gcaaggtgca aattagccat 47520
   ttatattgtt atccccaaaa taaatagatg cagttttttt aaaagatgca ggctatatat 47580
   ggaagtgttt gctggttttc tgtcaaaaga atggcgactt tattttctaa tttaaacttt 47640
   ttgctgtttt ctaaattgtc taaatagtta tagtttttat aatgtaaaag tatcttccaa 47700
   tttagcttca tttgacaaat taccttttca ttctatctag ctatgtaatt ctaaatgaat 47760
   ttacagcagt aatcttagag cagatgaatt tacaacaata atcttagagt agactacgga 47820
   ttagatgtaa aaacatgagt tgggctttat ggttacagag agttttcctc agtgtgggga 47880
   tcatagctgt attgagttta ttcagttttc ctttcccaca tgaatgaaaa atggggccag 47940
   cctacaactg gaagggcctc ggcatgtacc actgtactgt gtatgatgtg atttcttgat 48000
   gctagtaggg agagaatcaa attgcctcct attcaaacca agacccacaa atagcgtcaa 48060
   ccagtcattt cagctactcc ctgcagtgtc aagaaggtgt gaacccctca tgttctctat 48120
   tgcataccct tgtctaattc agtgtttctt cttcttttca ggttttggct ttatgctaca 48180
   tttcagaaat cataataacc ttttctggta ttattttatt ctttttcgca ctgtgagaaa 48240
   aattaaactt tcaagtggat gcttcttata aactatttat acccttttgc tcccttttgg 48300
   gaggcaggga cagggacaga gttcctcctc aggctaacta agaaaactta ctgcttccaa 48360
   tgtaatttaa aagatctccc tctttctatt gctctctgta ctcttaattc tttttttttt 48420
   ttttcacagc agagacaagt gaacatttat ttttatgcct ttcttcctat gtgtatttca 48480
   agtctttatc aaaacaaggc cccaggactc tccagattca attatgtcct tgggcttggt 48540
   cgactgctgt aggagtctca gggagccttc tacaaatgct agagtgactc atttaccaac 48600
   attaaaccct aggatacatg caacaaagca ggactccttc ctccatggaa tgtgccgatt 48660
   tcagatgaca cagcacccaa tgtagaaaac gctggaattt ttccttggaa ctagactgtg 48720
   atgagaggtg cttgacatga acataagcta ctgtcttttc tttttttttg agacagagtt 48780
   tcgcttgttg cccaggctgg agtgcaatgg cgtgatctca gctcactgca acttccacct 48840
   cccaggttca agcgattctc ctgcctcagc ctcctgagta gctgggatta caggcacgtg 48900
   ccaccatgcc cggctaattt ttgtattttt agtagagatg gcatttctcc atgttggtca 48960
   ggctggtctc gaactcccaa cctcaggtga tctgcctgcc tcagcctccc aaagtgttgg 49020
   gattacaggc atgagccacc acgaccggcc agctactgtc ttttctttga cccttccttt 49080
   ccagtttttg aagataaagc aggaaataat cttctctgaa gatacttgat aaaaattccc 49140
   aaaacaacaa aacgcatgct tccacttcac tgataaaaaa tttaccgcag tttgtcacct 49200
   aagagtatga caacagcaat aaaaagtaat ttcaaaaagt taagatttct tcagcaaaat 49260
   agatgattca catcttcaag tcctttttga aatcagttat taatattatt ctttccccat 49320
   ttccatctga atgactgcag caatagtttt ttgtttgttt gtttgtttgt ttgtttgttt 49380
   tttgagatgg agtctcgctc tgtcgcccag ctggagtgca ctggcgcaat cttggctcac 49440
   tgcagtctct gcctcctggg ttcaagcgat tttcctgcct tagcctctcg agtagctggg 49500
   actacaggca cgtgccacca cacccagctc atttttgtat ttttagtaga gacagggttt 49560
   caccatgttg gccaggatgg tctcaatctc ctgacctcat ggtctgcccg ccttggcctc 49620
   ccaaagtgct gggattacag gcgtgagcca ccgcgcccgg ccagcaatac agtttttagt 49680
   tactcgacat ctttaagcct ataactctta ggctatgcat agccccatgt cctaatcagg 49740
   cattcactga tcccagcagg tctccatcta tttgtaccag cctcctcttt cctcccaatc 49800
   tcaaggttac tcttaaatac tagtaaatgc aaaaagaact tgtaaagtgg caaggcatgg 49860
   cctatcaaaa gtcagcccaa gggcagtttt cagccctgcc tcacctgggt ctagttcagc 49920
   tgacggatga gctgattgat gcgttcaccc cgatagccag gtgtgcccat ctccttgagg 49980
   aagcccactc ttatttttgg tagcatgatg ggccactgag aggtggaaag ggcgcaagaa 50040
   ccatgagatc tcctggaaat gcttccctgg gaaggcaatt tcatgaatga ggtcttccaa 50100
   gcaaatgaag ccaaacttcc ccaggtgctc ctcaatcact gtgttgtctg tcagagggat 50160
   ggtcttattc ttgaccttgg cttgtccacg tttcaaaatg agttctcgga cagacttcag 50220
   atttggaaat ccccaggtca cataaggttc cactatatgc agcattttta gattctaggg 50280
   ggtaactttt acaaagatac cactaaaaat tttctttagg cgaagtcttg cagtggttct 50340
   ctgcacccgt aaactcacgc catcaatcct ttcgatgcgt acaacaaagg ccaaggaatg 50400
   tttatctggc aattccaagg catgaggttt cacttctagt cgtctgagac gcaccttgtc 50460
   acgtttctgc cgccaggaat catgtaggaa tgattccagt cgcttaaacc tgagcccttt 50520
   tcctttcttc tgtcttgctc tgccatcttt ctagtggtgc agctactcaa ttcttttttt 50580
   aattataatt tttattttaa gttccagggt acatgtgcag gatttgcagg ttacataggt 50640
   aaacatgtgg ccatggtggt ttgctgtacc tatcaactca tcaggtatta agcccggcat 50700
   gcgttagcta tttttcctaa tgctgtcccg cccccccacc caacgggccc cagttacact 50760
   cttaatcctt atagctcaga tgttatgatc cacagtgtgg ttcttacaga aagttatgga 50820
   ttaaaaaaaa aaaaaaacac tcaaagtgcc cgaactttct taaaataatc ctggtacagc 50880
   taaactcatg cactgactgt ccacctaata tttaacagtc tgtgttgtga tatattgttt 50940
   taatgttctg aatgcttgtc agctttcagt attgaagatg tgaatcattt atcagcaatg 51000
   acacatttag tctaaggttg tcagctattt atgctacaaa ttaatgactt gtccttaaaa 51060
   tatcaatttt gtgattcatg ttttggcagg tggttagatg ttttgtgttc taattttaaa 51120
   ctatggataa aggttttgtc ataatcattg ttttattggt tccttttctc ccctgcccac 51180
   tccccaaaaa accctgcaat tcttttttgt taaactttta ttttaggttc agaggtacat 51240
   gtgcaggttt gttatatagg caaattttgt gccacagggg tttgctgtac agattatttc 51300
   atcacccagg aaataaacac agtacttgat ggataggttt ttagtcttca ttctcttccc 51360
   accctcaagt aggccccagt gtctgtcctt cccttctttg tgtccctgtg tactcaatgt 51420
   ttagttccta gttataactg agaagaacat gtggtatttg gttttctatt cctgtgttag 51480
   tttgcttagg ataatggctg ccagctccat ccatgttgcc gcaaaggaca tgatttcatt 51540
   ctttttatcg ctgtgtagaa ttccatggtg tatatgtacc acattttctt tatgcagtct 51600
   tctgttgatg ggcttttagg ttgattctat gtctttgcta ttgtgagtag tactgcagtg 51660
   aacatacaca tgcatgcgtc tttatggtag aatcatttat attcctctgg gtatataccc 51720
   agtgatggga ttgctgggtc gaatggtagt tctgttttaa gttctttgag aaatcatcaa 51780
   actgctttcc acaatggctg gattaattta cacttccacc aggagtgtat aagcatttcc 51840
   ctttctctgc aacctcacca ggatctatta ttttctgact ttttaataat agctgttctg 51900
   actggtgtga gatggtatcc cagcaccatt tattgaatag ggagtccttt ccccattact 51960
   tgtttttgtt gactttgttg aagattggat ggttttaagt gtgtggtctt atttctgggc 52020
   tctattctgt tgcattggtc tatgtgtctg ttttgtacca ataccatgct gttttggtta 52080
   ctttagcctt gtagtagttt gaagtcgggt aatacggtgc ctccagcttt gttcttttgg 52140
   cttaggattg ctttggctat ttgtgccctt ttttgattct atatgaattt taaaatagtt 52200
   tttttctaat tctgtgatga atgtcattgg tattttgaga gcaatagcac tgaacccgct 52260
   aattgctttg ggcagtatgg cgattttaac aatatcgatt ctttctatcc cctgcaattc 52320
   tttgttgttg tatttaacta tttttacttg tgaagttttt tcagggatga ttttgttgaa 52380
   agtgacaact ctaaaaatta tgttggtaat taaaatttta agtaatgact tttattttca 52440
   gagattccac ttctcttaga ctttggagct gttaacagca gtgtccaatc tgcagtggta 52500
   ctcagcagtt tctgtttcct gcatgcagaa ctgcttatat gaaaacacag ttttaaaaat 52560
   gctttcttat ggctgacatt cacattctta ttccttttga ttcttttcaa gagggatttg 52620
   gtttgttaaa attaattttt gcaatacttt tatgaagata caaactctga caaagctttt 52680
   aaaacaagtt tgagagaata cagtattgat ttcacttgta aatctgacga ttattttaga 52740
   aaaaaggaaa atattattta ctattatttt gcttataaat gtttatcaat tttaaagctt 52800
   ccacattgca catctcccac tacaacagta gctaccattt attctttctc aaaaaaagtg 52860
   ctaagtgtgc ccttgaaatt tttacattgt gcagaatatc cctaaaattt taaaacaaaa 52920
   attacatcat cacttgcttt aaatgtttct tctttattta acatacagtt tctaaaatgt 52980
   tagcaaatag cattttagaa gagacacgtt acttttctaa tgaatgttct aaaatgaacc 53040
   acagtaacct atacttactt agactgtgaa aaacaaaact tatattctat tgttaaattt 53100
   tcaaaagtga aactacacga tagtttactt ggcacatcac tctgttattg tgaattgaca 53160
   aatgtatatg tagacaaata tgtgaaaatc agagtacata tacattatat gcagcaccac 53220
   aatacatttt ttagtatgtt ttgactgata tttaattata taatttacca agaggatctc 53280
   accagaatgt agaaaagtat tgaattttag aacaattcac atatttaaaa aaaatgtagt 53340
   cagccctttt atctgtatct ggagaatgca gggtaaagga ataatacatg agtattggta 53400
   tttaaaaaaa ggtgttaatt tcttacctat gatacctgtt actttgggta tcatttaacc 53460
   tttatttctg tgaaatagag gagttctaac atcctctaat tattataata ttgttctaat 53520
   ttaatctatc ttaatctgtg atacagtttg aaaaccaagc ttttactatt ggcatgtgca 53580
   aaaaaataaa gcagcagtag acttggaatc ttgaatgcaa atttagattt tgcctcttaa 53640
   taaatgtata atatagtgtt ctgggaccaa ttctctaaca tttctgagtc ctagtttctg 53700
   catctgtcaa atgggattag agatacctac tttcaggatg tgatatggtt tggctctgtg 53760
   tccccaccca aatcttatct tgaattgtaa tccccatata ttgagggagg gacctggtgt 53820
   gaggtgtttg gatcatggaa gtgatttcct ccatgctgtt cttgtgatag tgtgggagat 53880
   cgcaaaacat ctgatggttt aaatatggca gtttcccctg tgctttctct ctctcctgcc 53940
   accatgtaag actttccttg cttcctcttt gccttctgcc gtgattgtat gtttcttgag 54000
   gcctccccag ctatgcagaa ctatgagtaa attaaacctc ccttataaat tacccagtct 54060
   cagatattct ttatagtagt gtaaaaactg actaatacag agaattggta ctggcagggt 54120
   tgggtactgc tataaagata atctgaaaat gcggaagtga ctttggaact gggtaacagg 54180
   cagtggttag aacagtttgg agggctcaga agaaaactgg aagatatagg aaagtttgga 54240
   acgtcgtaga gacttgtttt gaatactttt gaccaaaatg ctgatagtga cgtggacaat 54300
   gaagtccagg ctgaaatggt cccagagatg aggaacttat tgggaactgg agcaaaggtt 54360
   atttttgcta tgctttagca aaaagactgg cagcatttta cccctgccct agagaactga 54420
   tgaactttga gatgatttag ggtatttggc agaagaaaat ttctaagcag caaagcatcc 54480
   tagtggtgac ttggctgatt ctgaaagcgt tcagtcatgt gcattcacga agatatggtc 54540
   tgaaattgga acttaggttt agaagtgaag cagaacataa aggtttggaa aatttgcagc 54600
   ctgaccatgt agtagaaaag aaaaccccat tttctgggga ggaattcaag ccagctgcag 54660
   aaatctgaat aagtaacaag gagtaataag taataataag taaaaagtaa taagtaataa 54720
   gtaacaagga gccaaatgtt aataaccaag acaatggaga aaatgtctcc agggcatggc 54780
   agagatcttc ggggcagccc ctcccatcac aggcctgaga actaggaggg aaaaatggtt 54840
   tcctgctcag ggccttgctg ctctgtacag cctcacgaca tggtgccctg catccctgat 54900
   gctccagctc cagctgtggc tgtaaggggc caagttacag ctcgcaccat tgcttcagag 54960
   ggtgcaagcc ccaagctttg gcagctttca cgtggtgttg ggcctgcagg tgcgcagaag 55020
   acaagagttg aggtttggga acctgtgcct atatttaaga ggatgtatag aaacgcctgg 55080
   atgtccaggc agaagtctgc catggaggca gagccttcat ggagaacctc tgctagggca 55140
   atgcggaagg gaaatatggg gttggatccc tcatacagag tccccactgg ggcactacct 55200
   agtggagctg tgagaagagg gcctctgtcc tccaggcccc agaaaggtag attcaccgac 55260
   agtttgcagt atacgtctgg aaaagccaca gaatgccagc ctgtgaaagc cacaggggta 55320
   ccctgctgag ccacaggggc ggagctgccc aagggtatga aagcccaccc cttacttcag 55380
   tgtgccctga atgtgagaca tggagtcaaa ggagattttg gagcttttag atttaagggc 55440
   tgcccagctg ggtttcagat ttcatggggc ctgtggccct tggtttgacc agtttctccc 55500
   atttggaaca ggaacattta cccaatgcct gttccctcat tgtatcttgg aagtaactaa 55560
   cttgcttttg attttatagg ctcatacgtg gaagggactt gccatgtctc agatgagact 55620
   ttggtcttgg acttttgagt taatgctgta ataagacttt gggggactgt tgtgaaggca 55680
   taattggttt taaaatgtaa aaagacatgg gatttgagag ggagcaagtg caaaataata 55740
   tggtttggct ctgtgtcccc acccaaatct aatcttgaat tgtaacccgc atgttttggg 55800
   ggagggacct ggtgggaggc agttggatca tgggggggtt ttttccatgc tgttcttgtg 55860
   atagggagtt ctcaggagag ttgatggttt aaatgtggca gtttcccttg tgctctttct 55920
   ctctcctgct gccaggtgag acgtgtcttg cttcccctgc cccttccacc atgatcataa 55980
   gtttcctgag gcctccccag ccatgcagaa ctgtgagtca attaaacctc ctttccgtat 56040
   aaattaccca gtctcagata gtatctttat agcagtgtca gaatggacta atacaggata 56100
   gtaatgaaga ttacagaata tgtagatgaa gaagtgctaa gtaaatagca gctattatta 56160
   tgtagtcaaa ttgaatgtat acattgtggt acttcagtgt cctttaaatt gaataactag 56220
   aaatttgttg gctttctcaa tctgctcaca tcagatgaca tgttaattta tgcctatact 56280
   tttttctagt taatagatat aaatctattc actcaacttc tattgacaga actggtagtg 56340
   tggcaagaca tctcatttct agttaaggct gtataatatt aagttcattt tacttaaatt 56400
   aactatggtt tgggaaatgc ttttcatgtc atcatgtatg cccaatttga tactttagtg 56460
   ggacagtata tttcagaaaa aaacaaatgc ttccccaaaa attccagggt tgaatacatt 56520
   agtcagacat ataacaatgt acttcagagt tcctctaagg gcaaaaatcg tggtatgaat 56580
   atacaaaaca ctcctattta tacttttgta tttttgaaat gtagtcttca tgttaattta 56640
   gcatttcaat gaccagcatg acattatctt aataatttgg aatgccaata tgttcattta 56700
   agacttaata tagtaagtat ctaaagaaaa aaatggaagt gactgaatgc ttttgtatct 56760
   cttaattata atttgtgctc cattgtgata tgaaggatag aaggggcagg atagatagaa 56820
   aacagaaatt aactttgatg tttaacctta ccttaagact gtctgttaag tgacccacat 56880
   aatcttaaaa aactctgtca agcttaatgg atgctactct gcaggcccct gccaggcaac 56940
   agtcacaagg ttatgaggtg catagatttt ggaattaggc agagctgaat tcagatccag 57000
   gtgttgcctt ataatgcgac tttgggcaaa taaaaggccc aatttttgta ttcttatctg 57060
   taaaatggac tcagtaaaaa ttatttgaga taatttattt gtgtactgta cctaggcatg 57120
   cagcttgaca cacagaatta caagtcagta gtttccagta tgattattat tgtgaaagag 57180
   atattttgtt tcacctactg aaaacttttt tcagtcttaa attttttatc taactggctg 57240
   tattgcagat gtctgctata taacttttat ataattttaa aaactatttc ttttctcctt 57300
   gatcttctag ggtaaggttt accaatgttt tcattattta ctaaatatag cagcccccac 57360
   cccttattca tggaggatag gttccaaaac ccctagtgta tgcttgaaac cacagaccac 57420
   agataatccc aaatcctata tgtatattgt ttttcctata catacatacc tatggttaat 57480
   gtttaaccta ctaattagga agagtaaaag agtaatagta actaataata aaataaaaca 57540
   attgtaacaa tattccagca tcactattct tgtgctttag ggccaccatt aagtaaaata 57600
   agggttactt gaacacaagc actgtgatac tgtggcagtc caactggtaa cagagatagt 57660
   gatgcggttt ggctgtgtcc tcaccagaat ctcaacgtga attgtatctc ccagaattcc 57720
   tatgtgttgt gggagggacc cagggggagc taattgaatc acagggtctg gtctttccct 57780
   tgctattctc gtgatagtta ataagtctca catgatctga tgggtttatc aggggtttcc 57840
   ccttttgcct cttcctcatt tttcttttgc caccaccatg taagaagtac cttttgcctc 57900
   ccgccatgat tctgaggcct ccccagccct gtggaactct aagtccaatt aaacctcttt 57960
   ttgttcccag ttttgggtgt gtctttatca caagcatgaa aatggactaa tacagtaaat 58020
   tggtaccagt agagtgggtg ttgctgaaaa gatacccaaa aatgtggaag cgactttgga 58080
   actttggagg actcagaaga agacgggaaa atgtgggaaa gttaggaacc tcctagagac 58140
   atgttgaatg gctttgacca acatgctgat agtgatatga acaataagat ccaggctgag 58200
   gtggtctcag atggatatta ggaacttttt gggaactgga gcaaaggtta ctatgttatg 58260
   ttttagcaaa aagactggca gcattttgcc tctgccctag agatttgtgg aactttgaac 58320
   ttgagagaga tgatttaggg tatctggtgg aagaaatttc taagcagcaa agcactcaaa 58380
   aggtgacttc ggtgctgtta aaagcattct gttttaaaag ggaaacagca taaaacttca 58440
   gaaaatttgc agcctgacaa tgcagttgaa aagagaaacc cattttttga gaagaaatta 58500
   aagctggctg cagatatttg cataagtagc aaggagccta atgttaatcc ccaagaccat 58560
   ggggaaaatg tctccatggc catgtcagag accttcacag cagcccttcc catcacaggc 58620
   ccagagaccc aggaggaaaa agtggtttcg tgggccaggc ccacggtcct catgctatgt 58680
   gtaggctagg gactttgtgc cctgtgtccc agctgctcca gctgtggctg aaaggagcca 58740
   atatagagct caggctgtga cttcagaggg tggaggcccc aagccttggc agcttccaca 58800
   tggtgctgag cctgtgggta cacagaagtc aagaattgag gtttgggaac ctctgcctag 58860
   attttagaag acgtatggaa acacctagat gcccaggcag aagtattact gcagggcagg 58920
   gctgtcatgg agaacctttg ctagggcagt gcagaaggga aatgtgggat tggagccctc 58980
   acacagaatc cctactgggg cactgcccag tggagctgtg ggaagagagc cgtcatcctc 59040
   cagaccccag aatggtagat ccaccaacaa cttgcaccat gtacctggaa aagccacaga 59100
   cactcaatgc cagcctgtga aagcagccgg gaggtaggct gcaaagtcac aggggcggag 59160
   ctgcccaaga ccatgggaat ccatcttttg catcagcatg acctggatat gagacctgga 59220
   gtcaaaggag atcattttgg ggctttaaaa tttgactaac tcactggatt tcagacttgc 59280
   atgggccccg taaccccttt gttttggcca atttctccca tttggaacag ctgtatttaa 59340
   cctgtgacac ccccctaccc cctgcccccc atccctccgg cccttgtatc tggaagtaac 59400
   tagcttgctt ttgattttat aggctcatag gcagaagaga cttactagcc ttgtctcaga 59460
   tgagactttg gactgtggac ttctgggtta atactgaaat aagctaagac tttgggggac 59520
   tattgggaag gcatgattgg ttttgaaatg tgaggacatg agatttggag gggccagggg 59580
   tggaatgata tggtttggct gtgtccccac cctaatctca acttgaattg tatgtcccag 59640
   aattcccatg tgttgtggga gggacccggg ggtgggggtg cagtaattga atcatggggg 59700
   ctggtctttc ctgtgctatt ctcatgatag tgaataagac tgacgagatc tcatgggttt 59760
   atcaggggtt tccaaaactt ttgcctcttc ctcatttttc tcttgccacc accatgtaag 59820
   aagtaccttt cacctcctgc catgattctg aggcttcccc agccatgtgg aactgtaagt 59880
   ccaattaaac ctctttttct tcccagtttt aggtatatct ttatcagcag tgtgaaaaca 59940
   actaatacag atggctagta agggactaac cggcagggag cgtctccagt gtggatatgc 60000
   tggacaaagg gatgattcac gttccagggc ataagatttc attactcaga attgcacaga 60060
   atttaaaact tattaattat ttctggaatt ttccacttaa tgttttcaaa ctgtggttga 60120
   ctgcaggtac ctgaaactgt caaaagtgaa accacagata agtggggagt cctgtaccta 60180
   agattattcc tttaaattgt ttcagtggat atgtagggac ctgagtgtga agtgagagca 60240
   gcagcatcaa aacctgaggg aaatccagat agcaaaagaa acttgtctag tatactggca 60300
   tgacagagaa accaaaaagt tctcaagtta atgtgagaat ctaagaatta aagaattaag 60360
   cctttgcctt tgagggaagg aaaggggtaa tgtggcttta aatcaggttg agattggttc 60420
   tgagggttcc ttttccttcc tttatattga tatgaatata gacacaactg ttctgcattt 60480
   ccatttgttt ttataaatgt ctttttagga tttaggaact gctaattatg caatatgaga 60540
   tatctgttag tttgaggaac atttgaaaat ttggtcaaat gacacagatc gtcacacagt 60600
   tttaagacaa atgtttttac ctatttgacc tagtctggca atccctattt gggcaaaaat 60660
   cttcatttgc aggtcatgat tggaggcagg cacagaaaaa aaattgccac cttttttgca 60720
   ttatgtcatc aagacatcaa acttcagcct acaaagtaga aagtgttatt tctcaagttg 60780
   aaggcctgga tatacctcag cttctcagtt ctgacacttt atcatagtgg aaaatgaaga 60840
   agattgctta agaacactga tgttggtgtc agaaagacct gggtttgaac cctgacttta 60900
   ctagttactt agatcacttt aggcaactca acttttctaa atcttgtttc ttcatctgta 60960
   aatgctgaaa atagtaccca cctcttaggt ctgtggagag gattaaatga gataatctat 61020
   acaaagaaag agcttgcata atagtgccaa gtaatggtga ggttatacct gtattctgat 61080
   tataatctca taaatattta ccatgttagc tgtctcagag ttcttttgca aaacagataa 61140
   agatagaaag tataaataag aaaaataagt gaacatatac tgaactttgt acaagatgct 61200
   ggcgatatgg agagacccaa gacatgggcc ctacctaaaa gagattattg atagaaacag 61260
   gatacatata catcaaaagg taacatagga tcatctgtgc aaagtgctat atggcagtgt 61320
   tttaggaagt ctagaagctg tcatggatca ggaataccat ggtggacact tcaggcaggg 61380
   aaaacagatc ttagcaaaag ctactcctat cataggtact tgataaatat ttgtagaatc 61440
   caggatccct gtagtgataa agaaactaca tggattatgt aggggagtga taagacatat 61500
   gactggaaaa ataaaaagac caaattatgg accatactga gcttgtacta taaacagtgg 61560
   aggagccctt cagattttta atcatgttga gaaaagagtt ttagcagtgt gtgggggata 61620
   gaatggaaag agaagccagt gccagaagga ctacttagta tcaaccattg cagtggttaa 61680
   agcaagaggt gagagaaggc atgcattaga atggcagcgg tcagagtgga tgggaaggaa 61740
   taggtcctga catagtgtta cagggagtaa taaataggat gtggaagatg ggttagaatt 61800
   ggcaaaatct ctgcatgtaa gtctgggtta ctaaatatag tgagagaaat tcaaatctct 61860
   ctttaagaat cgaataaaat atttagaaat aagttactgt tgtatttgag gtgaacacaa 61920
   atggcatttc aaagatgctc gagatacctt gttggaaaaa gtcaataact gcactattgt 61980
   ctccaacatg ttcttgcctt ctctgaagac atcatgttcc taattctgaa ttatgaacca 62040
   tctattatcc ttgtatgctc ttatgtgtga ggaaccataa ggtgggaaca aaatccggtc 62100
   ttcattctag aaataactat gcgatcaaaa agtttttagt ctttcttctt accatactgg 62160
   ttcttggtat tctgtttacc attcaatgta ctattattgc ttctgcttaa aactcacatc 62220
   ccctaatgca agcctgagca aacagaactg ataacacaca gcctgagaag ggagtgcttg 62280
   gggtctcaag acttattctg tttttctcca tctttgacac ttggtttgaa gagcaaagaa 62340
   ggatacagct gttaggaagt aagttaccca aacacagtga ccaaactgga ttaattcttc 62400
   caatgagaaa gaaatacatt atttctgtga gacagattag actttaagta gcatagataa 62460
   catgattata ttctctctac aaataaatac acaggaccta agaaaccctt tacagatcca 62520
   agtgttttcc tctccacttt tccatcccca aacccatctt gcaagatatg gccagcttat 62580
   ttggagttaa ttaaatcaag accttcgttt tacagacagg gaaaccaagc ccagagacac 62640
   tgagtagtag gccactggtg tcttagaggt ctgaaaaatc ctttactgaa cattctcttg 62700
   atctattaat gtataggttt tgttgctgta accctctccc caagaggagt gaatataaat 62760
   gatgcagagt ttggatgaac tatcttaata agaacctaaa gttgaaacca atgcaaacct 62820
   ctctcaataa atgcaaagca aagagaataa tcagtctttc tttggcttgt taaataagat 62880
   aaaatgtgtt ctgctaaaac catttaacag aaatattgtg aaaggtttcc cctaaagcat 62940
   ttttctattt gatttgaaaa ctattccata gcttattatc aaacaaatca gtaatccttt 63000
   agctaatgca gagataaatg ggcagtcaga aaatataatc acctggtgtg tgcagctgag 63060
   tatttacatt tttcctaatg aacaaagata agaaaagtgc aggtgacttt aatgtgtaaa 63120
   aactaccttt tagtgctagc gctagaggga aaaagaaatt actggctcaa gccaatcctg 63180
   tacttgataa ctaagccgta tagtccatgg cttggcttca gttctgtttt gaatctcttt 63240
   ttggacttgt cttgaatgga ctgtttaggg ctgcttcagt agtgcagttg ttgcattttt 63300
   aagcatagtt taggttttaa aatgtttctg gtcccttttt ttttttcttt tccactttat 63360
   gttgcttaaa gctttatggc caggttttct catcctcagc attattgaca tttgaagctg 63420
   gatacttctt tgtggtgggg gctgtcctgt gccttgtagg ctggttagca gcatccctcg 63480
   cctcttctca cttagatgcc aatagcattt ccccaaccgt gataaccaaa agtgttttca 63540
   gacactgcca aatgtctcct agagagcaaa attgctctct gttgagaact actgtgttac 63600
   ggtgtttgga caaaaactga caagccaatg ggaatattct attggtagtt gtaaaaaatt 63660
   aatccagtta tagcagctgt atttctggaa tttttttcca tattaacact tgctttctga 63720
   ggtgataata tctttgtttt ttttctccca aatagatttc ttgcattaca ctgaaaaatt 63780
   gctgattaat tcacttaaat tgaagactaa gccaatcatg tcatttgggt aatagtttac 63840
   caactctgcc cctttctctg tcagggaagc ctctaattta gtaagcgata ctgtatcctt 63900
   ttgtcaggta cattaccatt cctattagca atagggcaat tgagattgag aaagattaaa 63960
   aggtcaccaa gctattacat tgtagaatta ggttatgaat tgtagcctat ctggtttaga 64020
   atctttacct tactagtctc cataacaaca attcttccag tgtggtccat ggggccctgg 64080
   gagtctcccc ttaaagggca gactattttc acagtaacac gtactttatt tgccatttca 64140
   ttatgtcagc atttgcaata atggtacaaa agcaaagatg agtaaaactg ttggcatctt 64200
   agtatacagt agttactgta ttcactgtca tgcacttaaa atctttgaag aagcaaaaaa 64260
   attattaatt acattaaatt tcaaccctta aatacatgtg gtctttctca tgtcagtgtg 64320
   acaaaatgag aaggtgcata atccacttat atcgcatata gcatttgata gttgtctcaa 64380
   agaaaagtgt ataagattaa actgtgagtt aacctacttt ttttcatgga gtaccatgag 64440
   agataaactc tggttttcag ccttgggtat ttggcgatgt tttcccaaaa atgactgaag 64500
   taaacttagc actttaagga aaacaactta aagtatttgt tgccaattga taaaatatag 64560
   gtttcaagca aaaatcagaa tttttgaaga cttgtatctg ccactgtgag cttgacaaat 64620
   gtgactcttt tatattacat aatgaactat gtcaacattt gaaagatctg cataactcag 64680
   tgaaccagta ttttccagat gactaatgca tgataataca aaatcatgca tgggtaaaag 64740
   atacattcaa agtgcaagat agactgacat atttcaatgt aacaatcaaa agttcattga 64800
   taacagtttt ggattccaca ttgcaatact aaaaccttta aaaaacgaaa ttgtccaatt 64860
   ttggtgtagt aatcagaaaa ggcaatctat aattacctga acttaagttt ctggaggacc 64920
   attaaccttc tacaggctca tggggaagac tgtagcactt ctctttccct aagatcctcc 64980
   agaaaggaag aaggtaatcc ttgggggtag ggtagagacc tattgtgtga tgatcaccaa 65040
   gtatgtaaca atgctttata taactctaat atatataatc cacacaaacc ccctaaaatg 65100
   gcactaataa gggaatggac tcaaagaagt taagtcagct agccactgtc acagctatta 65160
   gagcactgga actaggattt gaacccagat ttgtctgtat gtaaagctga ttctcttcgt 65220
   aatagtactg agacacaaga ggcggctaca aaatattctg gtactccatc ctagaccaga 65280
   gtttcaaggt tcgttatcat ttgtagcatg atactggatc ctcacagtgc ttgcctttca 65340
   ttcaggtgcc aggaaacgtc tgcctgaatg aatgggtgta atttacctgc acattttaca 65400
   tgcttctcta ggtgtgtgat taactcataa tccatccatg actttcaccc ataatcctcc 65460
   ttgtagcaat tgctttgctt gcaacaaaac taagtagaca tatctagctt tatgcatggt 65520
   tttctctctc tgaactctaa cataaactca gcctcaggaa ttattcggtt tctactacat 65580
   ttgccattct gattgggaac caccagcatt caggtattca cctggaacaa ggcattttgt 65640
   tccaagggtt cctcacttaa aagcaagcac cctagcaata gttcataatg gaacttctta 65700
   acattctcag aatgtttggc acagctgtga gtgaacacac attgagcaat caataactat 65760
   tacagataat gatgccctta agaccaggat attttagctt tcccattcaa agggggtgaa 65820
   atatgcactc ttactatggt atacttttgg ttccttctgc catgtatcct taataaaaga 65880
   tgtcaattcc atatggtttt ctcttgagtt ctaaccattt tgttgtaccc tagccctttt 65940
   aacaatatca aacttgcaac tgaataccat ttagcattca tccatttttt ccaatggtgt 66000
   tcattatagg ctatcttact cctcctattt gtatgacaaa aattggcttt tttcaccgat 66060
   gtctatggta catctggcag ctttccatgt actcagttct tatctgatgt agcccagaac 66120
   gactgcctga agggatgcca aaagcctgat tgaggttcca aattttcagc tactgtacta 66180
   tcaatccatt tgttcatttt tactttccct tgtcatctgt agcttacagt tgagtggcct 66240
   gaacatgttt tgcatacatt gtaatatcta agaatttggg aatacggtcc taggatttag 66300
   acttaatact accttccatt tatataatac ttactcataa aatcttcagt gttcctgaaa 66360
   aagaaaaagg aacatgtatt gagtgcctgc tagaagcagg aacttgtagt agattttcta 66420
   tgtgttacct tattttcaca acacacacac aggtgatatc cttcccagtt tactgatgag 66480
   gaaactcagg ggtcaaagta gtagatacct acccaaggta acagaagctg tgaagtggta 66540
   cagctgggat ctaaaatatg tcagcttcac cgtagatagg ctccctgatg aaccacctgc 66600
   cacggcccgt atgaccgcat ccaggggtga tgatgtcatt ttcacagggt tattgagagc 66660
   taaaactacg aagtactata aactattatt taaaatataa atacatacta tatatgcata 66720
   tgtgtgtata tataattaat ggggtaaaca ttacagaata ctgtcctaac ctttaaacaa 66780
   tgcactcgtt ttctgtaaac taatatacaa acaactgttt ggtccctaaa aatagatgtc 66840
   aggtgacaga gactggctga gcaagaatag gagtatcttc agaatagaag ccagaggagt 66900
   ttttgcttcc ccaacacatt gtcgcaccat tcactgttcc aggaccttcc tacttctctg 66960
   gaaaactctg gcccaaagca gctcctctac attagtcaca agtttccatt aatcaggggt 67020
   ggcctgtgcc ggacctacag cagagtcatt tcaggttatt ctgttacagg ctttcgacgt 67080
   gtagtcagtc cactcgccca aatctagcag ggaatgaatg ccttgtaata cggaagcatc 67140
   tacaaattct tcttaacagt gttcagagaa caatgtgaaa ccctggggcc ttttcccaga 67200
   attagggtgg tgggaatgct gtcctattga ctaagcctgt taggtaagca ggcagttggc 67260
   aagattcagg aagcttcatt tgaagataga atttagggcg atcgtttgga tttactggct 67320
   taattactta aggtaacatt tataaaagaa attgtcattc cattattatt accttttaac 67380
   ttttattcct aaacggaaca ttagcaacaa actacattac ttgataaatg taatttctaa 67440
   ccagattgat aactagaaaa aaattttaag ttactttgct ctgtgaatta gtttaaacat 67500
   atttgtaatt gagacttact actgttattg gctgaaataa ataaaagcaa gagataataa 67560
   agaataacag agacaacgaa cacccaattt aagtttattt ctaagttcca tcttttttag 67620
   agaaaaggca aattaagaaa agtttagaga gaggtactag tatatttatg aacttgtata 67680
   gatgataagc aaaacggact ttaatatgta gaattccaga atcaacaggt tgccagcatc 67740
   catgtttttg aagatttgct taagaacaca accaaaaatg gaatgggcag tctctaatta 67800
   caagcagaag gctacaaaat cattttagct gcataataca gttttggttc taaagtcagc 67860
   acgtaagagg aaaattcctt aggaaaatac aacattgaaa accattgtgt catgtaatat 67920
   gaaatgcaat aattaatttt tcctccagta atagaaagat cactgtttca ttggtttata 67980
   aaaatatatc tttatcatta aatgtggcaa aatgttaaga cttggtgaat attggtgaaa 68040
   agtatatatc cattgtacaa ttctttccaa ttttttttga gattgaaaat ttttaaaaca 68100
   acaaattatc ttttaaacag ctaataatca ctagacctgc actctttgtg gtgagactat 68160
   gaaaaatgtt agagacctag taagagaagc agattcacat ttctgtcttc ttcttcaagc 68220
   caaacagtca tagagtggag tgggcagaat ggaactcact tttgaaagcc tagtgctttg 68280
   tccaatctta ctgcaagcca gacaggaagg ttatagaaaa tgtttctgga tcagtcttct 68340
   ctgagtcata tgaaattgtg gtttcagcca agatgacatt aggaattaga gacatgggac 68400
   aaaaacttta agattgtaaa aaaattttga ctctagtagg aaacatgggt agaattgtaa 68460
   tgacacttga ttgaatttta aaagatgcct gtataagatc ttaaaattag gaaaaaaatt 68520
   atggcctaag caattaaagg cataggaggc atctttttgg gatgatggaa atatcctctc 68580
   tcctgattgt gatagtagtt acatgaatat tcatttaaca aaaaccataa attatagact 68640
   tagaaaacag taaatgttac tgtatgtgac accttaataa acgtgattat aaaaataaat 68700
   cctaagcatc taaaaaaaaa aaaaaaaaga agaagaagtg aaccagaacc acaccattct 68760
   attttggaga cacttcaaaa gaaatgacct cattcttaat tttgtttaaa gaagaatata 68820
   acatgatttg aatatattta gctaggatat tttagtgcct gctagcactt gaagccagag 68880
   ttcactgtga gcattctgac tatgaagtga gaagctaaga gaactgtatt ttgatattcc 68940
   tttgacagtt aaatcataac actgttcttc cccttcttta gccccagcat gagaccagat 69000
   gtaagctctc ctccatccag ctcctcaaca gcaacaacag gaccacctcc caaactctgc 69060
   ctggtgtgct ctgatgaagc ttcaggatgt cattatggag tcttaacttg tggaagctgt 69120
   aaagttttct tcaaaagagc agtggaaggt agtgtgtgtt ttgaagagtt tatttttcct 69180
   ctacttggtt ttcatttctc agggtggatt ttgaaatttc cattatatgc aaagcccatg 69240
   aaaggctaaa tatcagttaa gaggggagag gagggtggct cctaggtcct ctaatgggca 69300
   ggaaagtatt taaaacgaca atacaaaaag atctagaata aaatagaaaa gtacaagttg 69360
   atgtctggga gtttggtcag ggagcataag gtaacactat aagaaagtgc tatcatatga 69420
   aatgatggtg ttaagtttgg gcataacata atgttcattg tattagaaac atgggcttta 69480
   acttccataa gctaataggt ttcaaagtca ccaactttac tggcctggca aaaatgagtc 69540
   acagtgagaa ctgtgacagg aaaaaaaaaa gatattcatt tcatttctta ttcatttttt 69600
   ttttctatta agccagggca ctgtgctaag tggtataaat accaataaga cctgatcctt 69660
   accctctggg aagtcacact ccactgaagt gaaagatgag ttaacaatga caaggtacag 69720
   agattataat atagatgagg gagagagaaa ctcggcctga ggaggtcagg aaaggtattt 69780
   tagagaaact gatttcacta tataaatgtt gtattaacac aaatcttact ttgttatgga 69840
   ttcagactgc tgacagggca acagcattat ctccctaaag aatgagaaat tcattccata 69900
   gcaaatttat tagaagagag tctaaaatgt cctaatacta ccagtgactc ctctaggaaa 69960
   aaaattgtca tataatttag ttatttctaa agcagtttga aagtagcttg gcctaaagct 70020
   ctgattatat taatttttta aagaaacaat tattcattca ctgtatgagg attattatta 70080
   tttgtctcat gttgtgtttg catatccatg agagttagat gagtcatttt cttttgtttt 70140
   actttttaat acattagcaa attataaaat tactcatatt acaccacaaa gattacaagg 70200
   atggcagctt tggccagtgt agtagtccca cctattgatt agagtcaaaa gtaaagccca 70260
   gccctgcttt gtgcattgct cctaataaag tggatgttac ttaacacata cgcagaagac 70320
   agaagcgtct tcgtgtcctc actttactcc tcactttctt aactgcttaa gtatttccac 70380
   gatataaatg cagtgataat aataatacgg acagtccctg acttaacgat ttttcaactt 70440
   ttatgatggt gggaaagtga tacgcattca gtatggctcc tcgacttaca atggggttgc 70500
   ctccagataa acccattgtg aattgaaaat atcttacact tagcactcca ttcttaatac 70560
   ctgctagaat tatagattat ccctcaaaat tggcatagta taatatgggt atcagcaagt 70620
   tgttgcactt tattcagagc tttacactag gcaggggtgg gctttacttt tgactctaat 70680
   caataggtgg gactactaca ctagccaaag ctggcatcct tgtggtctct gtggagtaac 70740
   gtgagtagca ttataattta catcccccat aacaaatgat ccaagagagt atgtgatcaa 70800
   tgcagcagaa ctattgtctt ttattatctg atttcacatg taacatgcca tcacttctgc 70860
   catattttat tggccacaca gaccaatctt ggtaaaggac ggaaagggac tgcacaagac 70920
   catgcattca aggaggcaga gatcactggg ggccatcttg ggaggctggc taccacaccc 70980
   accataaata gaaaaccaga attatttgcc aaaaatagac tttaaccaca aaaatgaata 71040
   ccatataaac aaaacaaagt cacaaaattt cagctgactt gaagactcat ctttctatta 71100
   gttagaaagg gaatttacca agtagtagaa gacacaggaa ctccaaaata agatatctca 71160
   ttgtcttatc agaagggttg acaggaaaat gggctgggca ctgtggctca aggaaaatgg 71220
   gctgtgcact gtggctcaca cctattatcc cagcaatttg ggaggccaag atgggaggat 71280
   tgcttgaggc ctggagtttg agaccagcct gagcaacata acgagacccc gtctctacag 71340
   gaaaaaaaaa aaaaaaaaaa cgttatccag gcatcgcacc tgtagtctca gctactcagg 71400
   aagctaaagc aggagattca ggctgcaaag agctatgaca caccactgta ctccagccta 71460
   ggcaacgtag caagaacttg tctaaaaata aataaataaa tgagtcaagg aatgaatgaa 71520
   tggattgaca ggaaatgact attagttgta cgtggccatg tgttatgaaa tagtgaatac 71580
   tagttaaaac tcctcatttt atagataagg aacagataga tagacttgtc caacttcatg 71640
   ctaataacca caaagggcta tttttaactt atgaaggtac attgcctctg atcctatagc 71700
   tcagagtctt agctgtgcac aagacatacc tgggataaag aaatcaagat tggcgtaatg 71760
   tgcacatcct gacatttcag ttggatataa acaaaacttt ggaatttttc atttttagca 71820
   gtgggtgatt ttttttcttt ttttcttcca gtaactgtag gacagtgatt tagagattcc 71880
   ttatagggta taactttttt gtattataac cacttcatca atagatgtat ctgttgatcg 71940
   tacttttgat ttatagggga tagaattggg ttagtgcttc cattttctgt ccaagtaaag 72000
   aagctaggat atttatagag tacaaaaaga aattgaaaca gctggtacag atatttggca 72060
   ttggagagca gctctgaaca aaggtgaatt atagtctagt ggtcaatttt gtggcctgtt 72120
   ctttacaaag aattgaacct gatacagtta accatctacc ccaaactatt atttgtttaa 72180
   aacacaatct attggctggg cgtggtggct catgcctgta atcccagcac atcgggaggc 72240
   cgaggcgggt ggatcacgag gtcctgagat cgagacaatc ctagccaaca tggtgaaacc 72300
   ctgtctctac taaaaatata aaaattagcc aggcgtggtg gcgtgcacct gtaatcccag 72360
   ctactcggga gtctgagcca ggagaattgc ttgaacctgg gaggcagagg ttgcagtgag 72420
   gtcatgccac tacactacta cactcccagc ctgggcgaca gagcgagact ccatctcaaa 72480
   aaaataaaaa taaaaaaaca taatctatca aactgtgtaa aacacagttt atcaaaaaag 72540
   tagttaccct tggtgggtac tggctggaat tgggcagaaa gggggcctgt tggggtactg 72600
   ttctgtttct tgatctgaga gctgattaca taaaggttct tggtttgtaa aaatttatta 72660
   aatggttcac tgatttgtgt acttttttta tatgtgaata ctgcaataag gttttttatt 72720
   gcactgtttt cagtttgttg aacagaaaaa gggagactct ttttgttgtt tttgacctct 72780
   cgacctcata atggcaatgt aggcaagaac attccctcaa ggcaatacct gtgggtgtct 72840
   tggttatatt ccaccggaaa caaagacaga ggctgtcctt ataaaatatg tttgaagacc 72900
   tgtgaaactt taatagtgcc ttttattcca tataggacag cacaattacc tatgtgctgg 72960
   aaggaatgat tgcatcatcg ataaaattcg aagaaaaaac tgcccagcat gccgctatcg 73020
   aaaatgtctt caggctggaa tgaacctgga aggtaatata aatatctgaa agcaattgtt 73080
   tgtctctgta gcttataaaa atttatcatt ttacttttga agatacacgt aagcagatgt 73140
   aattaatgta gtcagttcag tatatatatg cttgactagc ataatgttac tgcccaataa 73200
   aaatgggaaa tttttttcat gaatatgtca tattgtttgt ttatccacca gttcttctta 73260
   cacacactga attcagtaca gccagactat atacaaagaa aggaaattat gtaataatga 73320
   aacttacaca acatgcagca actttattat tcttactcct tttttcagcc tcaaaactat 73380
   tccctagggt tggaaatgtt tctgtatcag acatatttac atgtccattt ttctgtttgc 73440
   cttttaaaag catacctttt acttggagat ctgtgtttta ttacagatct tcaagcgggg 73500
   ggtggtggga aaaaaaaaac ctcaaggaag aactggatgg gttttgtttt ggttttcaag 73560
   taaagaagaa acctgggccg ggtgcagtgg ctcacgcctg taatccccga agtttgtgag 73620
   aatccttctg tctagttttt atgtgaagat attacctttt ccaccgtagg cctcaaagcg 73680
   ctccaaatat ccacttgcag attctataaa atgagtgttt gaaaaactgc tcaatcaaaa 73740
   gaaacgttca actccatgac ctgaatgcac acaacagtga gaagtttctg agaaagtttc 73800
   ttggtctccc cgcactttgg gagaccaagg caggcggatc acgaggtcaa gagatcaaga 73860
   tcatcctggc taacatggtg aaaccctgtc tctgctaaaa acacaaaaat tagcggagcg 73920
   tggtggtgtc acctgtagtc ccagctactc aggaggctga ggcaggagaa tcacttgaac 73980
   ccgggaggca gaggttgcag tgagccgaga tcacaccact gtactccagc ttggcgacag 74040
   agcaagactc cgtcttggaa aaaaaaaaaa aaaaagaaac ctgaaactag ttataagtta 74100
   gagtttcata tccctgttta tataacaagt tgtataatta acactgatct cagcattaaa 74160
   aaattttcct ctgaaaaaag tttggaattc tgctgtggtt gaaattgcaa gttctgtgaa 74220
   ggtagtggtg atctcataac acatatgctt agtatttatt gtgaaattag cacttttatt 74280
   caacaaatat gcaccaacaa ggcagtcact aggtataaaa tgaataaaat agtgcctgta 74340
   ttcaagtagt ttatctgcta gttaggttgc agagtcagtc acaaaatagc gtggcacacc 74400
   atagagggca tagggccaca ggaacaagag gaaggtcacc taattctgtc ttggaagtca 74460
   aggaagaagt aacattgaat tttaaatcta taagctgagt aggaattaga tagatgaaaa 74520
   ataagggcag agacatgatc agatttgtat tttacaaaga ctaatcttac atggagagac 74580
   caattaagtg aatatggcag tcctccagat aagagatggc agtactgaga gagaatggaa 74640
   accatgtggt tccttttatg attatgatga ttattattat tttagagaca gagtctaact 74700
   cttgtcaccc aggctggagt gcagtgacat gaacatggct cactgcagcc ttgaactcct 74760
   agactcaagc catcttccca cccagtaggg ctacggatgt acactaccat gcccagctga 74820
   ttttttttta atttttgttt taattttttg tagagacaaa ggggtcttgc tatgttccca 74880
   ggctggtgtc taactcctgg ccttaagtga tcctcccaac gtggcctccc aaagtgctgg 74940
   tattacaggt gtgagccact gcaactgacc tatgtggttc ttttgatagg agagactaat 75000
   tgttggtgct atctagcaca cactgtgtgt agacatcttg ttaaatagaa aatagattta 75060
   tgggtatgac tatgaagagt ctaattcccc aaaccacaca cacaactcta tctacgtttg 75120
   accaggctat ttaaacttaa ctgcagagtg tcagcatgtt aaacattgat ttacataaaa 75180
   tgatagctgc ccactttctt gtaaatgtta taaaaactgc agagattaac taaaaaatgc 75240
   acacagaagt ttgctttcag ttccacaagg gtagtttatt tttgttataa aaacagtatt 75300
   ccccactttc ttagatacca gatctctgcc cagattttac ccagtttcat cttgctgctc 75360
   tctaatctcc tatgtatgta atatactttg accatttaaa tatgtattaa gacacttgag 75420
   tttttagtgc cctttggttt attttctccg gtcccaatta tctctaatct tcattttttc 75480
   attttaccta ttttatattt cgaaataggt tttgaatgaa gctcaaagga caaacccaaa 75540
   taaaattctg tcgtatctct aatatattgt ggttgcttac ccagtaacat ttttaggtgc 75600
   ttttctgaat acatataaag tttaagatct ttggagtttt aagtatataa tgtttttctg 75660
   ggcaatttct ccctatccaa actatgaggg ccttctttca tcaaaagaaa aaagatatat 75720
   caactacaaa gtaatgattt tgatggacta ggctacgaaa tctgtccatt ttttcctcct 75780
   tcttacagtt taatagcaat tgcagtgccc tttgccctta ctgtactaga agacgacccc 75840
   aggcagtgac tgacatctga tttttctatt aattatacca tcactgccat ttccagttga 75900
   atcttttgtt ggacatcaga aatttttctt acatgaataa aatttaagca tacggttggg 75960
   cgcggtggct catgcctgta atcccagcac tttgggaggc ctaggcaggt ggatcacgag 76020
   gtcaggagat cgagactatc ctggctaaca cggtgaaacc ccgtctctac taaaaataca 76080
   aaaaattagc caggcgtggt ggcgggcgcc tgtagtccca gctactcggg aggctgaggc 76140
   aggagaatgg catcagccca ggagttggag cttgcagtga gccaagatcg cgccactgca 76200
   ctccagcctg ggcgacagag cgagactccg tctcaaaaaa aaaaaaaaaa aaaaaaaaaa 76260
   aaaaaaaaaa atttaagcat acaatttagg ctgcagtttc tcaaaatatt gtattaaaaa 76320
   taaccaatta tatgctttta tagtcagtat aacgtatcca gttagtgtag aaattggcat 76380
   ttgttgaaaa ctactacatg ttagtctttg atatacattc ttctactttt tggaccctga 76440
   ttattaaaaa cacctttgaa tagggccatg atttacttta tatccatttt tatactacat 76500
   agtggaagaa aattctgatt tgttatttcc tactatgata tgtaccgtgt ggcacatatc 76560
   atataaatga tccaattcta cttgtagatg aattgaaaga aaggcttaaa aaagttctta 76620
   gggtttgtgt gtgtggtttc actgtaaaac tatcattttt gtattgaact aacctcagta 76680
   tacataaaat ctttatttgg cctggtatgt acgtatgcca ggaatctttg gcagacccta 76740
   acacttacaa tacagatgag ccatgtgttt cacacttttt ttttaacaac cttcagaaat 76800
   attctcttgt tcatcagagt gcttccccta agccaagcag tttcgatgat agccccagaa 76860
   taactttgcc caagtctctc cataaatgta acttaggact ccaagtggtg tatttttata 76920
   ctcttgcccc ataccaagta aatctcaaga tttattttaa gggagtggcc ttcactgctt 76980
   aaagggccta gcatttaaga acagataaga tttttaatgg tgatcctaaa tgtttttttt 77040
   taaaaaactt gcttgttttt ctcttgaaac taaatgtttt tattcacttc attttaagat 77100
   atattgtaat caatccaaag tatggcttta tttttagtat aaacagtcaa atgaagctta 77160
   gtcttgtggc attgtcagat ttataaccaa atattactga aactaatttt tttaagttca 77220
   aaaacccaat ctagtagttt ctctcttatt ttcaactttt attttagatt ctaggggtac 77280
   atgtacaggt ttgttactaa gatacattgt gtgatgccgg tgtttggagt atgattgaac 77340
   ctttcatcta ggaagtaagc acagtaccta acaggtgctt tttaacctgt gcctcccttc 77400
   ctctatcccc cctcttgtat ttcccagtgt ctgttcccat ctttatgtct atgtgtactc 77460
   aatgtttagc tcccatttat aaatgagaac atggtatttg tttttctgca ttagttcatg 77520
   taggatactg gccgcctgct acatccatgt tgctgcaaag gacgtgattt cattcttttt 77580
   gtggccacat agtattccat ggcatataaa taccacattt tctttatcca gtccactgtt 77640
   gatgggcacc tgggttggtt ccatgtcttt gctattgcaa accatgctgc agtgaacata 77700
   tgggtacatg tgtctttttg atagaatgat ttatttttct ttgggtatat tcccagcaat 77760
   aggattgcta ggttgaatgg tagttaaact cttaattctt tgaagaatct ccaaacttct 77820
   ttccacagtg gtgtcattgt ggttttgact tgcatttctc tgatgattaa caatcagcat 77880
   ttttccatat gtttgttggc cacacgtatg tctttttttg agaagtgtct gttcatgtcc 77940
   tttgcccatt tttaatgggg ttgtttttgc ttgttaattt aagttccata taaactctgg 78000
   atattagggc tttgtcagat gcatagtttg caaatatttt ctcccattct gtagattgtg 78060
   atagtttctc ttgatttgca gaaactcttt agttaggtcc cattgtcaat ttttgttttt 78120
   gttgcagttt cttttgggga ttagtcataa attctttccc aaggccaatg tcgagaaggt 78180
   tatttcctag gttttcttct aggattttca tagtttgagg tcttacattt acatctttaa 78240
   tccaccttac taatttttat atggcagtag gtaggggtcc agtttcattc ttctgcacat 78300
   ggatagccag ttatcccagc accattaatg gaatagggag tcttttccct atggcttatt 78360
   tttatcaact ttgtgtagat tacatggctg taggtgtgtg tctttatttc tggactctat 78420
   tctgtaccat tgtgtgtggt ttttttttac cagtaccatg ctgtttcggt tactatagcc 78480
   tgtagtatag tttgatttgg ggtaatgtga tgttgccaac tttgttcttt ttgcttagga 78540
   ttgctttggc tatttggggc attttttggt tccataggaa ttttagaatg ctttttgcta 78600
   attctgtgaa aaatgacatt gtagtttgat aggaatagtg ttgaatctat aaattgcttt 78660
   gggtagtatg accattttaa ctatactgat tctaccagtc catgagcatg gaatgttatt 78720
   ccatttgttt gtgtcatctt tgatttcttt cagcagtgtt ttgtagttct ccttgtaaaa 78780
   attttaaact aacttagatg cattcctagg tattttactc tttttgtgac tgttacaaat 78840
   gggattgcat tcttgatttg gctctcagct tgaacattac tggtgtatag aaatgctact 78900
   gatttttgta cattgatttt aaatcctgaa cctttaccaa agttgtttat cagctccagg 78960
   agccttttgg cagagtcttc agggttttct aggtatagaa tcataagtga aaagagatcg 79020
   tttgattatt tattttccta tttggaagcc ttttatctct ttctcttacc tgattgttct 79080
   gactaggatt tccagtacta tgttaaattg gaatggtgac attgggcatc cttgtcttat 79140
   tgcattaagg ggaatgcttc cagcttttgc ccatttggta tgatgttggc tgttggtttg 79200
   tcatacaggg ctctttatta ctttgaggta tgttccttca atacctagtt tggtgaaggt 79260
   ttttatcatg aagagatgct ggattttatc gcaacttttt ctgcatctat tgagatgatc 79320
   attatttttt ttgttatgtg gtgaatcaca tttattgatt tgcatatgtt gaacgagcct 79380
   tgcatcccag aaataaagcc tacttgattg tggtgaatta actttttgat gtgcagctgg 79440
   attcagtttg ctagtgtttt gttgaagatt tttgtatctg tgttcatcag ggatattggc 79500
   ctgtagtttt gttgttgttg ttgtttctct accaggtttt ggtattagaa tgatgtttcc 79560
   cttgtagaat aagttaggga tgaggccctc tttctagatt gcttttttag aatagtttta 79620
   gtaggattag taccagctct tctttgtata tctggtagaa tttggctgtg aatccatctg 79680
   gtcaagggct ttttttaatt ggtaggtttt ttattattga ttcaatttca gaactcgtta 79740
   ttggtctgtt cagaatttca gtttcttcct ggttcaatct aggcaggttg tgtgtttcca 79800
   tttccacata catacttact ccaaataatg gctttatata tacgggggtc agctgaaaac 79860
   aaaaatgata ctttcatagt aaactccacc cgccccccca cccacataca cacacacata 79920
   aaccctagat tttttaaagc ctttgttcca atttatccat ttcctctaga ttgtctactt 79980
   tgtgtgcata gaggtgcttg taatagtgtg aagatctttt tcacttctgt ggaatctctt 80040
   gtaatgtcat cttttacatt ttttattgtg cttatttggg tcttcactct ttttttcttt 80100
   gttaatcttg ctagtggtct atcaatcttg tttatccttt caagtaacca acttttataa 80160
   actaggtttt aagctaatta agatttctct actttcatta agaaggaagt agtgttacca 80220
   cagactcatg aacacttctg tggagctcct gtattgactg ctaatcaact atatgctcca 80280
   atgggtcagg aatttatata aagttgtatt aactaagttg ctttaaaata gtgattgctt 80340
   aactaaatga ttcagttcag ttaactcctt cctgaagata ttttgaaaaa ttaattagta 80400
   ttatttcttg ctctagtcag tacagcacag ttgggttcaa ttgtactttc tgagctgtat 80460
   tgaaaaacat cagttttctc atttagaact atatataagt agtgagaaat taattacaaa 80520
   ctgagtcata gaaaatgttt ttttttaatc ctccagcttg ttactctttc ttccttgttc 80580
   taatgtggag taaagaaata tgcattccaa accatttaaa gttatgacta attgaggctg 80640
   tcaaagtact gtttcagtgt attgatttgg cacatgtgtg ttctctttta cattgtcaac 80700
   aaaagtacat tttatgattt tggatcaaga tttcactgag atacttctgg ttgtttaaag 80760
   agtttcttta tgtattggtg tctttccttt ttaaaatttt atcactcctc tattaagttg 80820
   tgatatccaa atttaaaata ttctaaaaac atgttctcct gcaagttgag gtaatgatag 80880
   ttgttatgtg gtacttacta taatatatgc caggaactgt tctaagcatt ttacatattt 80940
   aattctcaca acaaccctat gaggtaggga ctaatattgt cctcatttta cagaagggga 81000
   aatgaagagt cagggagtaa cttgcacaga tatccagcta caacatggca gaaccaggac 81060
   ttaaatccaa atatgctgat ttcaggtttc tgccctttag tcctatatca tactgtgcct 81120
   ccaagagagc atggtaaact aattagcatg gttctatcat gattctgttt ctattttgaa 81180
   ctattaataa aaatttttgc aattctcagt taccccattt agtatagaac acaataagaa 81240
   tggaaccatt ctattctaac attgtacatt gagatatcgt tcccaccacc atatctgtcc 81300
   tccatagact atatggtgtg tcattttaag gacagaggat ctaaaaatga tttttaaagg 81360
   tgatttacat ttactcttcc ctttgcaaaa tggtttgcat ccctaataat ttagacaagt 81420
   acatttcttc gtgatataaa ttacatttct tgcctttccc tggaattctg agtactttcc 81480
   ctctgagaga acaatgtaat tcttatttat ttagtcacta aaataacttc aggagtatga 81540
   ataagtctac taaaaagtct acaggatcca tgttgtagtt tgagtagatg gttccatacc 81600
   aagtcaaggt aaaagataat ttatatataa tatgaaaatg gctgctttag gtttatagag 81660
   taatcaatat aaatcttcct tataaaaggg aaatttccca cttataattt atgtaatgta 81720
   aagtttttca tttcatcttc ccaaatgttt ttagtcccac gcagtattta tgttagtacc 81780
   tatgtaaagg tgaaaagtga attttttcta ctggtagaac taatactatt tttagcatgt 81840
   aatctgctgt catcttccta tctttataag tggctttgaa caagtgtaaa tagtgtaatt 81900
   ctcttcatta tatatactac catgatttag attaatctta aaccacagtt tgtaatccgt 81960
   tactccaagc ttagattttt ttttcagttt atagtaagag taatttgcct tatataacca 82020
   atgaaattgt tgcatttaga gtgaaagtga gataaaaaaa taatttatag aagaatttac 82080
   aaaagttatt tactcagatt gttttaacat accgttataa tactttgtat aaggaataac 82140
   tctaatgaag tttctggcct atttgtaggc aaaattaatt gggaataggt tcctctggat 82200
   cttttgcttt cagaaaaaaa aaagtttttt ctccttttcc atgtcacttt atcataattg 82260
   ctaaataaaa tatttctccc atcttaatag ttttagaaag taaaaatact tcttgaataa 82320
   actgtgtagc gcagaccttc ccattacagt tcatttctat gtattttttt aaatacccac 82380
   agctcgaaaa acaaagaaaa aaataaaagg aattcagcag gccactacag gagtctcaca 82440
   agaaacctct gaaaatcctg gtaacaaaac aatagttcct gcaacgttac cacaactcac 82500
   ccctaccctg gtgtcactgt tggaggttat tgaacctgaa gtgttatatg caggatatga 82560
   tagctctgtt ccagactcaa cttggaggat catgactacg ctcaacatgt taggagggcg 82620
   gcaagtgatt gcagcagtga aatgggcaaa ggcaatacca ggtaagatgc aaaacataaa 82680
   agagcaacta tataaacctt tgtgttttct tcagcaaaaa cactttggct tttatatcat 82740
   cgtgagccca tggcttatct tgtttctctt agttctgggg actatgaagg ggagagtcag 82800
   gtgaatacag gtgataggga gtttataata aaacatttac attactccct gcttttcaaa 82860
   tcattatgca caggatggta atttcacata ggatgatgta atatcagaat tcaagttaca 82920
   agactcactc aaaactcctt ttacactgaa gtttggggaa agaaaatgtt tttagttaat 82980
   tccatttgtt ttccttcatt gtgccacttt taaaaatcag gttgtttgta agattggtaa 83040
   acatcaagta tgttgattgt caaaatttgt actaaagtag aatgatttta acccttcact 83100
   aaatgaaatg ctacacattg aatgtaattt taaagataat tttaaataaa agttacccta 83160
   ttggaatttg gtgtggaatg gcagaggtca atgttagtgt cagctctgac tttaaagaca 83220
   gggaattgac aagcctgtgt tcacgcaaat agttagggag agagcaagaa agtaacctga 83280
   cctcctgtca tccttgtttt attaaggggg aaagaggtgt gaatagcagg gcaaatgttt 83340
   tgcttaactc attgattaat acctcaagcc aagattcttt tctgtttttt aaaatcaata 83400
   cataatagtt gtacatattt actgtacata tttatattta gggggtacat gtaataattt 83460
   aataaaagca tacaacgtgt aaggatcaaa tcagagtaac tgggatatcc atcacctcaa 83520
   acatttgttt ggggaacatt ccaaatcttc tcttttagct attttgaaat ataaagtaaa 83580
   ttattgttaa ctatagtcat cctgttgtgc tactgaacac taaaacttat ttcttctaac 83640
   tgtatttttg cacccgtcaa ccattcccgc ttcatcccca tcaccactat ctttcccggt 83700
   cactggtaac cgccaagcca agaattttgg ctattttact atttagttca tgtttactta 83760
   agcagacaga ggtgacaaaa ctggcttttt ttttttttac attaaaagct attaaaaagc 83820
   acctaggggg ctgggtgcga tggctcacgc ctgtaatccc agcacttggg aagcccaggt 83880
   gggtggatca gttgaggtca ggagttcgag accagcctgg ccagcatagc aaaaccccat 83940
   ctctactaaa attacaaaaa ttagccgggc atggtggtat gaatctgtat tcctagctac 84000
   ttgggaggct ggcactgaga atcacttgaa cccgggaggc ggaggttgca gtgagccgag 84060
   atggcaccat tgcactccag cctgggtgac agagcaagac tttgtctcaa ttaaaaaaaa 84120
   aaaaaaaaaa aaaaaacaca agagggtttg tgagtcttaa agtgtcagat gacagaagaa 84180
   aactgtgtct acctagtatt taatttccat tttctgttag gggtgccctt gttttgacag 84240
   ggctaattga tctcattgct ccttggcaat tcccacagag atgatcttct gaagagtgtt 84300
   gcctcatacc tttatttctc ttaattcagg tttcaggaac ttacatctgg atgaccaaat 84360
   gaccctactg cagtactcct ggatgtttct tatggcattt gctctggggt ggagatcata 84420
   tagacaatca agtgcaaacc tgctgtgttt tgctcctgat ctgattatta atgagtaagt 84480
   tgtatgtgtg tcattttccc tgtattcata gggtatcttt aaccagctga tgttttcctg 84540
   attgactgct attgtgataa ttcaggactg aaacaatcct actaggtatc taggatctag 84600
   gcaaactgga aatagagtta tgagtgcttg gggcaggaca agtgtaatgt aaagcaaatg 84660
   tacatgtggc attattactg tcccaggaca tgtttgagga tatttaacag catatctgag 84720
   gttagtaaag tctgtcgcaa gcaacaagga atcttactgt gatatcattt acataaccct 84780
   attccagaaa gaaaaaggag catggtaaaa ctcatgtgga ttcagtgggg acaattgtag 84840
   atgaggatat ctaggctgat ggggtgggac atatggaccc agacacaaga ggtatctctt 84900
   tgcatggcaa ggctcaccca gtgtctgtgg tttaagaata tgggaacaaa tttgttttgt 84960
   ttaactgaga gaagaccaag cctttaagat tttataaatc agctattctc ttatcctcta 85020
   agcttattcc tgtgtctgcg aaatacttca ggtgtccatt tccccttacc tcattgcagt 85080
   tgtttcctca ctcgttttct ccctccagtg taacgttcat catgttggct aatgtttgct 85140
   tcctcaagca cagtctgact gcatcacata tctccccagt acacagattg tcttcagtat 85200
   cttcccactg accctccagt acatattctg catgatttca gactttccag aatctgacct 85260
   cacttcctct cccattgttt tccttcacac actcttcatt cccatccatc ctttccagca 85320
   tactcttaga ctcttggtgt tcacatcacc agatacacag cagagaagtc acatcctagt 85380
   tactctcact ttctaccttg tattactact tttcgtaccc ctagcttatt gctattagta 85440
   caatgtaaac agggagttca cacacacata cccctggtct aagaagaata aaaaatgaag 85500
   gagatttctg tttgtataga aaacagaagt caccttgact tttattgcca aaaagaggac 85560
   tgttcaaact actgcatcac aatgtaacaa gattaggtag ttggatccaa ttttaaatta 85620
   actggtaaat atatttagtt tctggggaaa ctgaagacat tattactcat cataatccta 85680
   ccatgctgtt taaaaaatac catgttggca gtatttgttt tttagtcact ttctaatatg 85740
   taatttgaag gcatttaagt ggaattaaaa gcataaacag atttgtatga aacaccaact 85800
   tatcctggtt tataaaacta acctaattta gggtttttat tattagggca ttcagattta 85860
   gctttaagca gtcacagcaa aatctaatca tgccacatac attccttaca taaagtggga 85920
   tttataattt tttttcctca acagatttac attagtttca ttttcattaa gggatatgta 85980
   cttcctattc ttgtgttctc atgctgctgc ctaaaagatg ggcagtcctc cacctttttc 86040
   ttttcttttt tttttttttt tttttttgag acgagtctta ctctgtcacc caggctcaag 86100
   tgcagtggtg tgatcttggc tcatggcaac ctctgcctcc agggttcaag tgattctctg 86160
   cctcagcctc ccgaatagct gggattacag gcgcactcca ccacacttgg ctaatttttt 86220
   gtatttttag tagagacggg gttttgccat attggccagg ctggtcttga actcctgacc 86280
   tcaagtgatc cacccacttt ggcatcccaa agtgctggga ttacaggtgt gagccaccgc 86340
   acccagccct ccaccctttt ttcttagccc actatgtttc catactgctc tggtgtctgt 86400
   gacaggcaga tattgcatat cagaaagtat gcattcaagt tctgaccctc tatagagctg 86460
   tcaaacagtc tctcatggtt gcccttaggt cagaacgttg tgggggaaaa aaaaattgtt 86520
   gtttttacag ccaacaagaa tgagttttta cttattctac tacactataa ctttgttgaa 86580
   attttcagtt atatgagtat aaccatgtac aagaaactaa aggaaaaaaa ggtgcctccc 86640
   agaaaaggag tgctttacct actattaagg actagggagg tgcctcttcg gtaagagcag 86700
   attttaaatt tgaagagcct ctgatcactt tggcagcata taagtcatgt ctaatttatt 86760
   ttatataaag gaataaacca catattcagt agagaaaaat aataaccttt ctgttgttaa 86820
   gtccaagacg actttctgtc agaaacttaa aaaaaaaaaa aaatcttgaa gcattttaaa 86880
   agctgtgaac tgggcccagt ttcaggctct tagtgtcatt tcacaagtca ggaaacttta 86940
   gagacctatt tgaaaatcat aggtatgtaa tgacttcaga atcataagca agaattggtt 87000
   tagtaccttt agtttaaaga atattaaggc atatgcctgt cagaggcaga ttttgagcat 87060
   cagaagtcta gaatcaagtt ctaggtctcg ccctctgcat aactgtgaac agtgtcacac 87120
   atttttgtct ttaggatgga ctgctgtgaa aaaatttacc tttaaaaatc aagtgtgtag 87180
   gacctaaaac tgtcgtctaa ttgaccgtat tcaaatgata aaccttgatt taaatgagca 87240
   actagtaata agttctataa gaattctaac actttaatta aataataaaa taatacatgg 87300
   catgcatgat agaaaataat atctccactg ttacattaga ttattcatta gtctatttaa 87360
   acagccaaga tgcaggaagt ttaaggaaag ttctccaaaa ttctgatttt atagggaatt 87420
   agcaataata ttattgcagt agttgttttt ctttatgagt tcatagtttt gcaaaacaaa 87480
   acaaaaatgt gctttttggg gggaagtagc agtatttcta actaataccc tgctatttat 87540
   ctttcacagg cagagaatga ctctaccctg catgtacgac caatgtaaac acatgctgta 87600
   tgtttcctct gagttacaca ggcttcaggt atcttatgaa gagtatctct gtatgaaaac 87660
   cttactgctt ctctcttcag gttggtagaa caccttttca ccttatgtca aaagcatgaa 87720
   atatgaaggc ctagaaacaa aggttaattt atatacatag tactaataat tataccaagt 87780
   ctactattat ttcctactag tcagatgatt tttatgaatg taaaatatta gaaaggcaca 87840
   gtaagtgaca ccaagattaa taagacaaat aggtatggca gaaacagaga ggtatatgag 87900
   ctgcataggg atctctgttg ataagaatct gtgtagactt ttttctcctt ccttcctttg 87960
   atctttgatc atgggaagac atggaaaaag aaagctaact acagtgattt tgtctactac 88020
   actgttattt ggttaaaaat tttagtttcc taatgagtat tagcatgtat gagaaattat 88080
   gggagaaaaa ggcgcatcct agaaaaggtg tgcttaatta ctattgggga ttggttaaca 88140
   tagcatggga gctggattgt cagagattca ttatctagaa aatggcaaca agagtttata 88200
   aaacgaactt ctgtgagatt actttttagc tagcaaagac aaagatgtcc ttcagtaggt 88260
   gaagtgataa actatgatac atccagatga tggaatacta ttgaggacta aaaagaaata 88320
   agctgtcaag ccatgaaaac acatggaggg acgttaaatg catattacta agtgaaaaaa 88380
   gctaatctga aagggctaca tactgtgtga ttctaactat ataacattcc ataaaaggca 88440
   aaactgtgaa gacagcaaaa aaaaatcagc ggttgccagg gtttagaagg aagggaggga 88500
   taaatgtgca gagcacagag gatttttagg gcagtgaaaa tacttcgtat gatactacaa 88560
   tggtggaaac atgtcattat acatttatcc aaacccaaag aatgtccacc accaagagtg 88620
   aaccctcaac tatggacttt gggtgatgat gtgtgggaca ggaggtatat gaaaaatctc 88680
   tgtaccttcc tcccaatttt gctgtgaact taaaactgct ctaaaaaaag tcttttttaa 88740
   aaaaagctct atgaactagt tggtattata aaccttaggc catttcaagt aaaaattaca 88800
   tatcaatgtt tattaaatac tgagttaata gctgaatacc tctttcatat acaaataagt 88860
   acatttgcaa ttttttaaaa agtcttaatt ccattagtaa ctgtggtttc atagttgcca 88920
   aataactgta agctatggat gttgcacaag actgtgattt tatttaatca tttcatatct 88980
   atttaaacat ttccaaagcg cacattcatc ttaatgtttt cacactattt ttgctcaaca 89040
   aaaagttatt ttatgttaat ggatataaga agtattaata atatttcagt caaggcaaga 89100
   gaacccgata aagatcattg ctagagacgt ttaatgttac ctgtagcggt acacttgtta 89160
   aagaagtgat taagcagtta cataaaattc tgatcatagc tttgattgat accatgaagg 89220
   tataattcag tgcctggata ctaacaactt tacttgttta aaaaaaaaaa aaaaagaatg 89280
   gtttcaattg tatacatccc agactaattg agctatatga tttttttcat tgtaaataat 89340
   atcacgagtt cttcttgtta aaaaataata gaatcataag gatggaaata tataccttaa 89400
   gatatagact tctactatga tagactactg gaataggtat ataacctccc accaaaaatg 89460
   ctagactaaa aaaattaaga actaagtgaa ggcaggaacc tacagagata agtggaactc 89520
   aagccaactt gctctttgac ggcatttgta gaacctggta aattagtaag tttagtaagt 89580
   tggggttttt ttaagtttat aatctttttt aaaatgattt caataggttt ttggggaaca 89640
   ggtagtggta ggttacatga ataagttctt tagtggtgat ttctgggatt ttggtgcacc 89700
   catcacccga gcagtgtaca ctgtacccaa tgtgtagtct ttcatccctc atcccctccc 89760
   caaccctagt ccacaaagtc cataatatca ttctcatgcc tttgcatctt catagtttag 89820
   ctcccactta gaagtgagaa catgcaatat ttggtttccc attcctgggt tacttcactt 89880
   acaataatgg tttccagttc catccaggtt gctgcaaatg ccattatttt gttccttttt 89940
   gtggctgagt agtattccat ggtatatata taccacattt tctttatcca ctcgttgatt 90000
   gatgggcatt tggactggtt ctgtatattt agtaagttta aaaacaaggg atggaaatat 90060
   aaatgcagtt gaaaaggcag tggatggatc taaaagcaga agaatacaat tgtttttaat 90120
   gattgtgtat atgtttgtgt atataaacca caagggaaat ctgtaggtac tgaaaatcac 90180
   aacaggaaaa tggcaacaaa gctatagaaa ctggaaaagc aatgactttt cttagatccc 90240
   tcagagaatg gaggtcatag gacaaaccac cacttcaaaa tctagaagaa tagacaaata 90300
   cagagaaaca gccaagatca gcttactggg aaaagatgcc actgaagcca ggaagactat 90360
   ggcaatttgg gaaaagatgc cactgaagtc aggaagacta tggcaatttt gatgaattgc 90420
   tggaggctga gtgaggacta gcttcagagt taaaaactcc cagggaccca gtcttagtgg 90480
   gggtttcctg caatttcttg ggtttacccc acaaaatttc taacttccag aaactccaca 90540
   aggttcttat ggtgaagatg caagaaaaat tccctccttt ttctggtagg agtagaggga 90600
   aggtaaaatt tggaaatacg tagcagagtg ttcacaacaa aaggcctgcc ctgtaaggaa 90660
   aactaattca acaggccctt atgtgacctg ggggaaaggc aaatagagga ttctagccct 90720
   tccttagcct tcttgtctca tttctgaaag tcacagccca gggattcaga cccactaaaa 90780
   aaaactgaga tttaatcata aagattaaaa aacaattccc ctccccctcc ccaacacctt 90840
   accaccatat aaacagggct ccaggataaa ataacagtgg attacaactg agagagctgc 90900
   aagacacaag ctgtttaagg agctcttagg aaacccaaaa acaacagaag aaaaagtaaa 90960
   taaaaacaag gaaactagag gaaactgaag cctccagtac ctacaattat ggcaaacatt 91020
   aaatacagcc cagctcctag ccagattagc atgaaacctc acactaaaag tctaattact 91080
   tcagttttga tatatcaatc atgtccagct ttcagcaaaa aaactacaag gcatgctaaa 91140
   aggcaagaaa aacccacggt ctgaagagac aaaacaagca tcagaagcag tcctcagata 91200
   tgacacaaat atttcaatta tcagataggg aatttacaat acctatgatt agtaggttaa 91260
   aggctccaat ggaaaaaagt agacaacatg caagaagtga tgtacgcaga gagatggaaa 91320
   ctctaaaaat aaatgctaag gaatgctgta aggaaatgca gaatgatgtt gatgggctca 91380
   tcagtagact gagcacagcc aagcaaagag tcagtgagct tgaagataga taggtcaaag 91440
   gaaattcccc caaactcaaa tgcaatataa acatagtaga cattaatcca gctgtatcag 91500
   taattacttt aaatttgaat gctctaagta caccaatcag ctattttttt aactaggagg 91560
   tgaaaataaa gtttgccacc agatgctcac taaaaaatta ttagaggata tacttaggct 91620
   aaagaaaagt aatcccagcc aggcgtggtg gctcacaccg gtaatcccaa cactttggga 91680
   ggctgaggca ggcagatcac agagtcaaga gatcaagacc atcctggctt acgtggtaaa 91740
   accccatctc tactagaaat acaaaactta gctgggggtg gtggtgcgcg cctgtagtcc 91800
   cagctactca ggaggctgag gcaggagaat cacttgaacc tgggacgtag aggttgcaga 91860
   gagccaagat agcaccactg cactccagcc tagtgacaga gggagactcc atcttagaaa 91920
   aaaaataata aaagtaatcc catctttaag aaggactgaa gaataacaaa agtggtaaat 91980
   aatatagata catttaaact gacatttact atgtatataa aataacaaca gtaacaattt 92040
   ccttgagggc taaaaagtag aactaaagta agtttcaagg atgacaacta gaaatagggt 92100
   atgcagggta tgcaaagtac caaaccattg gggaagagaa atacctaaga aaaacaatcc 92160
   aaaagaatga aagacatgag aggagggaga aaaaaatgca taaacaaggg catgataaca 92220
   ggaagtaaca gataaggtac attagtacag ctaaattcaa acacatcagt agtttagttt 92280
   cattaaatat agagatgggg ccaggtgtag tggctcacac ctataatccc agcactttgg 92340
   gaggctgtgg gcagatcact tgaggtcagg agttcgagac cagcctgacc aacatggcga 92400
   aaccccgact ctactacaac tataaaaagc cgggtgtggt ggtgcatgcc tgttatccta 92460
   gctactcggg aggctgaggc acaagaatca tttgaacctg ggagatggag gttgcagtga 92520
   gccaagatcg tgccactctt ctccaaactg ggtgacagag ggacactgtc tcaaaaataa 92580
   aataaatgta gagatggact gaatgctcca agctaatctg acaggatttt agaaataatc 92640
   caaatttatg ctatttaaaa aaagctatat ctgaataaag atattgaaag gctgaagtaa 92700
   aaggatctac tttgcatagt ataacccaag acatggccaa ctttttctgt aaagggccag 92760
   atggtaaatg ttgttagctt tgcacagtct ctgtcacagc tactaaactc tgcccttgtg 92820
   gcaggaacat agtcattgac ggtactcaaa tagaacaggc atggctgtgt tccaataaaa 92880
   ctttatttac aaatacaggc tgcaagtagg atttggccca taggccaaag tttgctggcc 92940
   cctatattga ccaaaacaaa accgaaggag ctacattatt accaagcaaa atagatgtta 93000
   aggcaaaata ctccttaaag catttgttca ggaaaaataa ttgtaaatat atagtttcaa 93060
   attacataat acaaaaattc atagaacaag aatacttaga taaatctagt aaaaataatg 93120
   agattttact atacctttct tacaaattaa gcagacaaaa aaataaggat atggatgtac 93180
   atttcatctc tcttgggtca atactgaggt gtgagatcac tgggacatag gttgagtgtg 93240
   tgtttaaatt tatttttaaa attgccaaac ttttccgcaa ttgttaacat ttaccagaaa 93300
   tgtatgagac ttcttaagat ccattctata tcctcctcag tacttggtac tgtcagcctc 93360
   tttcatcgta ggtatactga tgattaaaaa tattaagcat cttttcatgg gcttattggc 93420
   cacctatatt tcttatttgg tattgtgcct cttttaatct tttgcccatt ttttaactgg 93480
   gttttaagaa ttgttcaaat attctcaatg tggccctttg ttaaatatat gttttgcatg 93540
   ttttctttaa gtggattaca tttacagttt tcttaaaaaa atgtagagat gagcaaaagt 93600
   gtataatttt gaagaaagct tcgtgtcttt gtttactaag aaagttttgc ttaatccagg 93660
   gttaaaaaga ttttctacta tttgttttct tatagaaatt ctgtagtttc agctcacatg 93720
   cttaagtata tgatgcaagg taagggacaa ggttcatttt cttccccaaa atccatatct 93780
   ggttgctcca gaacttgact ctcttttccc tattgagtta cttggcaatt ttgtagaaaa 93840
   tcagttgttt gtatatgtgt gggtctactt tcagactctt tttcttaccc aacgatctgt 93900
   atttcttacc caatgatctg tatgcctata ttcatattga taacaccctg tcttgattac 93960
   tgttgcatta cagtaaatct tgaaatttgg taatatgaat tctccaaatc tgttgttctt 94020
   ttccaaactg ttgttttgga tattctagtt tccttgcatt tccacttcct tttttttttt 94080
   tttttttgag atggagtctc actattgttg cccaggctgg agtgcagtgg catgatcttg 94140
   gctcatcgca gcctcagcct ccccagcagt gggattgcag gcacccacca tcatgcttgg 94200
   ctaatttttg tatttttagt agagacgggg tttcgccatg ttggccaggc tggtctcaaa 94260
   ccctgacctc aggtgatcca cccacctcgg cctcccaaag tgctgggatt acaggcatga 94320
   gccactgtgc ctggtcttcc acgtattttt taattagctt gacaatctct accaaaaagt 94380
   cttttggggc tgggtgtggt agttcatgcc tgtaattcca ccactttgag aggccaaggc 94440
   aggcagatcg cttaagccca ggagtttgag accagcctgg gcaaaatgtc gaaaccctgt 94500
   cactacacaa aatagaaaaa attagccagg catggtagct tgtgcctgta gtcccagcta 94560
   cccaggaggc tgaggaggga ggtcaaggct gcagtgagcc atgatcatgc cagtgcactc 94620
   tagcctgggc aacagagtga gactctgtct caaaaacaca gtctgataga atttttatta 94680
   ggatagcctt gaatctatag atccatttga aaataattaa catcttaaat ttccaatttc 94740
   tggccgggcg ctatggctca cgcctgtaat tccagcacgt tgggaggccg aggtgggcag 94800
   atcatcaagt caggagttcg agaccagcct gaccaacatg gtgaaaccct gtctctacta 94860
   aaaatacaaa aaaattagcc aggcgtggtg gcacatgcct gtagtcccag ctactcagga 94920
   ggctgaggca ggagaatcgc ttgaatctgg gaggcagagg ttgcagtaag ccgagattgt 94980
   gccactgtac tccagcctgg gcaacagagt gaggctccgt ctccaaaaaa aaaaaaaaaa 95040
   attccagttg ttgagaaaga ataggaattc cagctttgga ggagtgggga gaccatcaaa 95100
   tcctctttcc aaaaatacta ctaaaatact actgagcaga gtatagttcc acaaatagtc 95160
   ttctgtaaag agactcacag tacatatttg tctttgtagg ccatatagtc cctgttgcaa 95220
   tttctcaatt ctacagctat aacaggaaag cagctatata cagtatgtga atgcttgtgt 95280
   tctaatacaa atttatttgc aaaatcagga aaatggcttg aaatggttta agatctagtt 95340
   ttctgactag atcatggtat ataatctttt ccatatatat tttgaatttg gtttgctaat 95400
   attttgctga tcatttttat atctctcttt atgaaggatg ctgatctaca actttctttt 95460
   cttgtgatat ctttttctgg ctttgctacc agggtagtac tagcctctta aaatgagttg 95520
   agaagtattt tctgttttct taaagagttt atagagtatt gatcttattt attctttaaa 95580
   tatttgatac atgttaccag tgaagccatc tgggtctgtg ttttctttca gggaagattt 95640
   ttaattattt gcttatttgt tatatagatc tattcagaat ttatattttt ccttgacata 95700
   gttttgtaat ttgtgtgttt ctatgaaatg agccattttg tctgagttgt ctaacttggg 95760
   cataaagttg tttgtaatcc tttaagtttt gtaggatcca tagaggtgtc ccctccatta 95820
   tagattttca taatttgtgc ctgatcatct ttttttcatg gtcagtctag ttaaaaattt 95880
   atcaattttg ttggtcttta caaagaacca atttttagtt tcattgaaat ttttagtttc 95940
   attgatttcc tctttttgtt tcctatgtca ttgattatta tttcttcttt tctgcttgct 96000
   tttcatttaa tttgttcctc tttttctagt ttaaggtaga agcttccatt gttagttgaa 96060
   gaccttattt tcttatatag atgtttaaag ctatacattt tttgtatatt ttcattcatt 96120
   tcattttcta atgtccttca tgattttttt cattgaccca tgtgtattgc ttaattttta 96180
   tatatttggg gattttccat atctcttcct attcatttct aatttaattc cactgaggta 96240
   ggaggtacat tgaaggactc taatattgaa tgactccaat aagtcttctg agactttttt 96300
   aggcacttgc atatggtcta tcctgagtgt tccatgagtg cttgaaaaaa aacttactgt 96360
   gctcttgtta agtagagttt tatgaacgtc agttaggtca agttgattga tagactaatt 96420
   caagttttct gtatctttgc tgattttctg tctagttgtt ctagatccta caactttgtc 96480
   tacatccttg ccagagcttg gtatggtttt tttattatcg ctatcctaga gagtatgtag 96540
   ttgacccttg tgacttgcca tgcatttaat gactgcccat gttcatagca gcattattca 96600
   taatagcaaa aaaaactttt atcatatgct tttgtgcctc aagatcatat atttttcgtt 96660
   tttagtcact aatatggtat aatggtataa tatactgttt aatttctgag taattgacta 96720
   gcctttcatt ccggggataa atcctatttg gttatgatat agtatccttt ttacatatag 96780
   ctgaattcat tgtactaaaa ttttggtatt tttgcatcta aatccatgag ggatatattc 96840
   tatagctttg gtgttatgat aatatggtat tatttctttc ttaaacgttt ggtaaaactc 96900
   agcagtgaag ctgtcttggt ttgtttggag ccttttttgt agaaaggttt tcaagtacaa 96960
   gttcatcaaa tgtttactga taatatgttt attcttgagt gagctttgtt ggtttacatc 97020
   tttgaaggaa tttaactgtt tccttcaaat gttgaattta ttggtataaa gttaagttat 97080
   tcataatatt cccataatat ccttctaatg gctccagtat ctctagtgtt attccctttc 97140
   attcccgaca ttggtattta atatattctt gctttttttt ttttttttta atcagtctgg 97200
   ctaaaagttt ttcagtttta ccaatgtttt catagaacca gcttggtctt gattttgttg 97260
   ttgtttatgc atgttcttag ttattcgttt ctactcttta tcctttccat ttttcttgtg 97320
   tttagggtag aagcatatat aattaattga gacctttctt ttctaatcaa agcttttaat 97380
   gctgtaaatt ttctaagcac tgtcttcatt gcatcccaca cattttgata tgctgtgttt 97440
   tcagtactag agatttttaa ttttatgata ccttatttaa tcatgatgcc ttatttaatc 97500
   tatagcttat taaatgtcaa attctaaaca tttgggtttt tctccagata tgtttgttac 97560
   tgacttctat tttaatctca tttttgtcag acagcattca ttgtatgact taatcctcct 97620
   aaatgtattc agacttgttt tatgttctag attaatgttc tgtgtatact tgaaaagaat 97680
   gcaagttctt gggtagactg tttcagaaat gtcagtcaaa tttaagtctt gtttattctt 97740
   attgattctg agacaaaggt gtttataatg ttagatttgt ctgctatatc tctgacattg 97800
   ccaaatatcc ccttggaggc aaaatctccc cctccctttt gagaaccact gatctatgta 97860
   gccttttttc tgggactaat ttagccttgc ttctgagatg tggcccctag gtctctactg 97920
   aatgcccggc atatttaatt agatctttct ttcctctatg gcctcaaggg atttcaccct 97980
   aagtatgcac aaatttttat tcagccgaag actgtacaga tttctggagg cctttctttg 98040
   tgtacctcct tcgtttccag tagtctgacc cataaattgt acagatttct ggaggccttt 98100
   ctttgtgtac ctccttcgtt tccagtagtc tgacccataa attaaagctg ctttagcctc 98160
   cccaaacttc aatctctttc tcctcaaccc agcaagattg ctagaccctg ggttcccttt 98220
   cccttcactg cagtatgata attactttca agcacaaagg tttagaatta agatttctta 98280
   ctcctgggct aggtatggct taccgtattt gtttctcttt tcctagggat cataatcatg 98340
   tattgcttgt tgtccagttt tccagtagga ggggaattcc aggctgtact tacttcctgc 98400
   agccaaaaga ggaagtaatg ttagtgattt caatattaaa acattaaaaa aaaatttaag 98460
   atggatgaaa ttcttttata tgcatattga attgggcttc accatagtta tttttagaat 98520
   taggactaac cggcagggaa aaaaactata cggcagggaa aaaaactata agccatcgct 98580
   gttttacaat tttgcaataa ttagattttc tgtagtatag taatgtgtaa aattaaccca 98640
   ttgttaatat agaatgccgt tatcactcct gattaagcgg tcttcatttt catgttaata 98700
   ctgatgtctt gtaatgcttt ctggaatcaa acattttcat acatattcat tagtctaatt 98760
   ctaatcataa tccaatgaaa aagagcagga aagatgctca aggaggttat attcaagtcc 98820
   acatggcaag taagaaataa gactactcgg ctgggcatgg tgacttactg cctgaaatcc 98880
   cagcactttg ggaggccaag gtgagcggaa ttgcttgaac ctgggaggcg gaagtggcag 98940
   tgagctgaga tcatgccaat gcactccagc ctaggcaaca cagcaagact ctgtctcggg 99000
   aaaaaaataa taataataag acttctagaa gctcctaaat ccatagcttt tcctctatac 99060
   cagcatcttc taaaaatgtc agcagcagtg aagtttcagt ttgggaaata atgcatttcc 99120
   cctctctgga gagtgcacag ttatatctcc aagaagtact gaaattcaga agtctgccta 99180
   atatgtatta aacatttagc ttttctcaaa ctttgaccac caaatccttt gtctcgctct 99240
   aactatagtt aacacagaat cagtgttccc aggagcacac tgtgaaaaat gtagcactct 99300
   acaaaagtcc taatctccac aggattaagt gaaaccatga ttaaccctct gttccttgtc 99360
   cttattagta ccattttctg aagagtaatg tatcccccca aaacttttat actagtttca 99420
   ctaaccagaa tccatgtaca taaggaagga cagatatttg ctccctacta agacatatct 99480
   attagctaca ttaaaaaaag tattgcatgc cgattttaaa gttataatta actggtgata 99540
   tcacagatat tccaagatat aattgctgga ataaacactg ttgttgaagc cttctatcta 99600
   tctcagtact agaattaaac tcaagtgcag aatggcagac aaagttaact aaaaatcact 99660
   gtattatttc atttggtcct ccaaatagct ttgtgagcta aggaggagaa ggtgtatcat 99720
   caccacttcc attttataga tgagaaatca agtgatttac tcaaggttaa gtcctccaat 99780
   tctttgttat cctgcatttt ctcttggctg tagtttaatt aataatccta agaaaatgct 99840
   tatattttag agtgcagtaa gagtacataa acaatgttaa atgcccatct tgcatgtata 99900
   aaaagttata gcaagaaatc tggctgggaa tggtggctca cacctgtaat cctggcactt 99960
   tgggaggccg aggcaggagg attgcttgag cccaggagtt taagaccagc ctgggcaaca 100020
   tagggagatc ctgtctctac aaaaaaattt agccagacac agtggcttgt gtcctagcta 100080
   ctcaggaggc tgaggtggga ggatcacttg agccaaggag gtcaaggctc cagtgagcta 100140
   tgattatgcc actcagacat ggtggcttgt gcctacagtc ctagctactc aggaggctga 100200
   ggtgggagga tcacttgagc caaggaggtc aaggctccag tgagctatga ttatgccact 100260
   gcactccagc ctggatgaca cagtgagacc ctatctatct caaaaaaaaa aaaaaaagaa 100320
   aagaaaagaa aaagaaaatc ctttaactga cttcatctta accttttagt tcctaaggat 100380
   ggtctgaaga gccaagagct atttgatgaa attagaatga cctacatcaa agagctagga 100440
   aaagccattg tcaagaggga aggaaactcc agccagaact ggcagcggtt ttatcaactg 100500
   acaaaactct tggattctat gcatgaagta agtgtcaaac ataaagccaa atataagagt 100560
   tttctgggac aaagtatgtt ttgattagtg aatataatta tataccagca gcgcccccac 100620
   ccccgccccc agtttgtgga tgttggtgat agcttgagtt caacttatga acttcagttt 100680
   tgtagacatt tttcctaagg ccaattatga aatatccttt cacctagtca tgtgtatata 100740
   aaatcaccat gttattacag aatttagtaa cactgttttt aaaaagtatg attaatccat 100800
   taaattagaa taatgcaccc ttcatatatt atggtactac agtgattcat gaaataattc 100860
   tatataattc tacatacaat caaagaaata taaaatgtgt tttgtacgga agtgcttatt 100920
   tttcatctgg ggaattccag tgagattggt atattctagg ccagataatt ttttcaaaat 100980
   agaggacaac aaacatgaga tgttcccact gaccaatttg gaagcctgat cattaccata 101040
   tcttctcttg caggtggttg aaaatctcct taactattgc ttccaaacat ttttggataa 101100
   gaccatgagt attgaattcc ccgagatgtt agctgaaatc atcaccaatc agataccaaa 101160
   atattcaaac ggaaatatca aaaaacttct gtttcatcaa aagtgactgc cttaataaga 101220
   atggttgcct taaagaaagt cgaattaata gcttttattg tataaactat cagtttgtcc 101280
   tgtagaggtt ttgttgtttt atttttattt gttttcatct gttgttttgt tttaaatacg 101340
   cactacatgt ggtttataga gggccaagac ttggcaacag aagcagttga gtcgtcatca 101400
   cttttcagtg atgggagagt agatggtgaa atttattagt taatatatcc cagaaattag 101460
   aaaccttaat atgtggacgt aatctccaca gtcaaagaag gatggcacct aaaccaccag 101520
   tgcccaaagt ctgtgtgatg aactttctct tcatactttt tttcacagtt ggctggatga 101580
   aattttctag actttctgtt ggtgtatccc ccccctgtat agttaggata gcatttttga 101640
   tttatgcatg gaaacctgaa aaaaagttta caagtgtata tcagaaaagg gaagttgtgc 101700
   cttttatagc tattactgtc tggttttaac aatttccttt atatttagtg aactacgctt 101760
   gctcattttt tcttacataa ttttttattc aagttattgt acagctgttt aagatgggca 101820
   gctagttcgt agctttccca aataaactct aaacattaat caatcatctg tgtgaaaatg 101880
   ggttggtgct tctaacctga tggcacttag ctatcagaag accacaaaaa ttgactcaaa 101940
   tctccagtat tcttgtcaaa aaaaaaaaaa aaaaagctca tattttgtat atatctgctt 102000
   cagtggagaa ttatataggt tgtgcaaatt aacagtccta actggtatag agcacctagt 102060
   ccagtgacct gctgggtaaa ctgtggatga tggttgcaaa agactaattt aaaaaataac 102120
   taccaagagg ccctgtctgt acctaacgcc ctatttttgc aatggctata tggcaagaaa 102180
   gctggtaaac tatttgtctt tcaggacctt ttgaagtagt ttgtataact tcttaaaagt 102240
   tgtgattcca gataaccagc tgtaacacag ctgagagact tttaatcaga caaagtaatt 102300
   cctctcacta aactttaccc aaaaactaaa tctctaatat ggcaaaaatg gctagacacc 102360
   cattttcaca ttcccatctg tcaccaattg gttaatcttt cctgatggta caggaaagct 102420
   cagctactga tttttgtgat ttagaactgt atgtcagaca tccatgtttg taaaactaca 102480
   catccctaat gtgtgccata gagtttaaca caagtcctgt gaatttcttc actgttgaaa 102540
   attattttaa acaaaataga agctgtagta gccctttctg tgtgcacctt accaactttc 102600
   tgtaaactca aaacttaaca tatttactaa gccacaagaa atttgatttc tattcaaggt 102660
   ggccaaatta tttgtgtaat agaaaactga aaatctaata ttaaaaatat ggaacttcta 102720
   atatattttt atatttagtt atagtttcag atatatatca tattggtatt cactaatctg 102780
   ggaagggaag ggctactgca gctttacatg caatttatta aaatgattgt aaaatagctt 102840
   gtatagtgta aaataagaat gatttttaga tgagattgtt ttatcatgac atgttatata 102900
   ttttttgtag gggtcaaaga aatgctgatg gataacctat atgatttata gtttgtacat 102960
   gcattcatac aggcagcgat ggtctcagaa accaaacagt ttgctctagg ggaagaggga 103020
   gatggagact ggtcctgtgt gcagtgaagg ttgctgaggc tctgacccag tgagattaca 103080
   gaggaagtta tcctctgcct cccattctga ccacccttct cattccaaca gtgagtctgt 103140
   cagcgcaggt ttagtttact caatctcccc ttgcactaaa gtatgtaaag tatgtaaaca 103200
   ggagacagga aggtggtgct tacatcctta aaggcaccat ctaatagcgg gttactttca 103260
   catacagccc tcccccagca gttgaatgac aacagaagct tcagaagttt ggcaatagtt 103320
   tgcatagagg taccagcaat atgtaaatag tgcagaatct cataggttgc caataataca 103380
   ctaattcctt tctatcctac aacaagagtt tatttccaaa taaaatgagg acatgttttt 103440
   gttttctttg aatgcttttt gaatgttatt tgttattttc agtattttgg agaaattatt 103500
   taataaaaaa acaatcattt gctttttgaa tgctctctaa aagggaatgt aatattttaa 103560
   gatggtgtgt aacccggctg gataaatttt tggtgcctaa gaaaactgct tgaatattct 103620
   tatcaatgac agtgttaagt ttcaaaaaga gcttctaaaa cgtagattat cattccttta 103680
   tagaatgtta tgtggttaaa accagaaagc acatctcaca cattaatctg attttcatcc 103740
   caacaatctt ggcgctcaaa aaatagaact caatgagaaa aagaagatta tgtgcacttc 103800
   gttgtcaata ataagtcaac tgatgctcat cgacaactat aggaggcttt tcattaaatg 103860
   ggaaaagaag ctgtgccctt ttaggatacg tgggggaaaa gaaagtcatc ttaattatgt 103920
   ttaattgtgg atttaagtgc tatatggtgg tgctgtttga aagcagattt atttcctatg 103980
   tatgtgttat ctggccatcc caacccaaac tgttgaagtt tgtagtaact tcagtgagag 104040
   ttggttactc acaacaaatc ctgaaaagta tttttagtgt ttgtaggtat tctgtgggat 104100
   actatacaag cagaactgag gcacttagga cataacactt ttggggtata tatatccaaa 104160
   tgcctaaaac tatgggagga aaccttggcc accccaaaag gaaaactaac atgatttgtg 104220
   tctatgaagt gctggataat tagcatggga tgagctctgg gcatgccatg aaggaaagcc 104280
   acgctccctt cagaattcag aggcagggag caattccagt ttcacctaag tctcataatt 104340
   ttagttccct tttaaaaacc ctgaaaacta catcaccatg gaatgaaaaa tattgttata 104400
   caatacattg atctgtcaaa cttccagaac catggtagcc ttcagtgaga tttccatctt 104460
   ggctggtcac tccctgactg tagctgtagg tgaatgtgtt tttttgtgtg tgtgtctggt 104520
   tttagtgtca gaagggaaat aaaagtgtaa ggaggacact ttaaaccctt tgggtggagt 104580
   ttcgtaattt cccagactat tttcaagcaa cctggtccac ccaggattag tgaccaggtt 104640
   ttcaggaaag gatttgcttc tctctagaaa atgtctgaaa ggattttatt ttctgatgaa 104700
   aggctgtatg aaaataccct cctcaaataa cttgcttaac tacatataga ttcaagtgtg 104760
   tcaatattct attttgtata ttaaatgcta tataatgggg acaaatctat attatactgt 104820
   gtatggcatt attaagaagc tttttcatta ttttttatca cagtaatttt aaaatgtgta 104880
   aaaattaaaa ccagtgactc ctgtttaaaa ataaaagttg tagtttttta ttcatgctga 104940
   ataataatct gtagttaaaa aaaaagtgtc tttttaccta cgcagtgaaa tgtcagactg 105000
   taaaaccttg tgtggaaatg tttaactttt attttttcat ttaaatttgc tgttctggta 105060
   ttaccaaacc acacatttgt accgaattgg cagtaaatgt tagccattta cagcaatgcc 105120
   aaatatggag aaacatcata ataaaaaaat ctgctttttc attatgtgac tccaacatgc 105180
   ttttgtagaa cttgtacagt tccgattgtc caatctgatt tttgtttact gaaagtagag 105240
   ttacccctgc ttcaggaacc ttaagataat atggtgggca tttaaatgtc agtgtggcaa 105300
   tgttcgcctg ctaatatggc atagattcaa aataagctta accctggtgc caaagacctg 105360
   aagattatcc catccatgcc tcaaatggtt gtgtgccaat tactgcaaag ggtactaagg 105420
   gaaggagaaa ttcactcctg aggctgcttc aaatgtatgt ctttatcaca aaagatgaca 105480
   ttttatgtaa gctaatgtta tctagtcaaa attcttagct tattttaaaa tcaactcttc 105540
   aagaaaagga ataaacattt aatataaata tcatagcagt attgcacata gaatagaaag 105600
   gtcgggcagg gtagtggaag tcagctattc tatacaatcc attcggtatt ttccaaaaca 105660
   tttgatgttc aggccatatc caggaactgg atgacctaac aaacttctct gagtaccttt 105720
   ttttccacaa gagatctcca tcactaagaa aaaaagcatt gtgatttaaa agccaaattt 105780
   gccttatcca tcatcatgtg caccaagtat ttgctacctg cctactatat aatattgaag 105840
   atacaacgtg aataagaaaa atactattgc taccctcaat cagagtatgt gattggaaaa 105900
   gtgtataaca aacctttccc agtgtcttca ggtataatgc agagatacca gatacggcat 105960
   caatgtgtat acacattatg gctgtaccat tcactttaag                       106000
<210> 9
   <211> 2034
   <212> DNA
   <213> H. sapiens
<400> 9
   ggatctggca gcgccgcgaa gacgagcggt caccggcgcc cgacccgagc gcgcccagag 60
   gacggcgggg agccaagccg acccccgagc agcgccgcgc gggccctgag gctcaaaggg 120
   gcagcttcag gggaggacac cccactggcc aggacgcccc aggctctgct gctctgccac 180
   tcagctgccc tcggaggagc gtacacacac accaggactg cattgcccca gtgtgcagcc 240
   cctgccagat gtgggaggca gctagctgcc cagaggcatg cccccctgcc agccacagcg 300
   acccctgctg ctgttgctgc tgctgctggc ctgccagcca caggtcccct ccgctcaggt 360
   gatggacttc ctgtttgaga agtggaagct ctacggtgac cagtgtcacc acaacctgag 420
   cctgctgccc cctcccacgg agctggtgtg caacagaacc ttcgacaagt attcctgctg 480
   gccggacacc cccgccaata ccacggccaa catctcctgc ccctggtacc tgccttggca 540
   ccacaaagtg caacaccgct tcgtgttcaa gagatgcggg cccgacggtc agtgggtgcg 600
   tggaccccgg gggcagcctt ggcgtgatgc ctcccagtgc cagatggatg gcgaggagat 660
   tgaggtccag aaggaggtgg ccaagatgta cagcagcttc caggtgatgt acacagtggg 720
   ctacagcctg tccctggggg ccctgctcct cgccttggcc atcctggggg gcctcagcaa 780
   gctgcactgc acccgcaatg ccatccacgc gaatctgttt gcgtccttcg tgctgaaagc 840
   cagctccgtg ctggtcattg atgggctgct caggacccgc tacagccaga aaattggcga 900
   cgacctcagt gtcagcacct ggctcagtga tggagcggtg gctggctgcc gtgtggccgc 960
   ggtgttcatg caatatggca tcgtggccaa ctactgctgg ctgctggtgg agggcctgta 1020
   cctgcacaac ctgctgggcc tggccaccct ccccgagagg agcttcttca gcctctacct 1080
   gggcatcggc tggggtgccc ccatgctgtt cgtcgtcccc tgggcagtgg tcaagtgtct 1140
   gttcgagaac gtccagtgct ggaccagcaa tgacaacatg ggcttctggt ggatcctgcg 1200
   gttccccgtc ttcctggcca tcctgatcaa cttcttcatc ttcgtccgca tcgttcagct 1260
   gctcgtggcc aagctgcggg cacggcagat gcaccacaca gactacaagt tccggctggc 1320
   caagtccacg ctgaccctca tccctctgct gggcgtccac gaagtggtct ttgccttcgt 1380
   gacggacgag cacgcccagg gcaccctgcg ctccgccaag ctcttcttcg acctcttcct 1440
   cagctccttc cagggcctgc tggtggctgt cctctactgc ttcctcaaca aggaggtgca 1500
   gtcggagctg cggcggcgtt ggcaccgctg gcgcctgggc aaagtgctat gggaggagcg 1560
   gaacaccagc aaccacaggg cctcatcttc gcccggccac ggccctccca gcaaggagct 1620
   gcagtttggg aggggtggtg gcagccagga ttcatctgcg gagaccccct tggctggtgg 1680
   cctccctaga ttggctgaga gccccttctg aaccctgctg ggaccccagc tagggctgga 1740
   ctctggcacc cagaggcgtc gctggacaac ccagaactgg acgcccagct gaggctgggg 1800
   gcgggggagc caacagcagc ccccacctac cccccacccc cagtgtggct gtctgcgaga 1860
   ttgggcctcc tctccctgca cctgccttgt ccctggtgca gaggtgagca gaggagtcca 1920
   gggcgggagt gggggctgtg ccgtgaactg cgtgccagtg tccccacgta tgtcggcacg 1980
   tcccatgtgc atggaaatgt cctccaacaa taaagagctc aagtggtcac cgtg 2034
<210> 10
   <211> 2439
   <212> DNA
   <213> H. sapiens
<400> 10
   ctccgggaac gccagcgccg cggctgccgc ctctgctggg gtctaggctg tttctctcgc 60
   gccaccactg gccgccggcc gcagctccag gtgtcctagc cgcccagcct cgacgccgtc 120
   ccgggacccc tgtgctctgc gcgaagccct ggccccgggg gccggggcat gggccagggg 180
   cgcggggtga agcggcttcc cgcggggccg tgactgggcg ggcttcagcc atgaagaccc 240
   tcatagccgc ctactccggg gtcctgcgcg gcgagcgtca ggccgaggct gaccggagcc 300
   agcgctctca cggaggacct gcgctgtcgc gcgaggggtc tgggagatgg ggcactggat 360
   ccagcatcct ctccgccctc caggacctct tctctgtcac ctggctcaat aggtccaagg 420
   tggaaaagca gctacaggtc atctcagtgc tccagtgggt cctgtccttc cttgtactgg 480
   gagtggcctg cagtgccatc ctcatgtaca tattctgcac tgattgctgg ctcatcgctg 540
   tgctctactt cacttggctg gtgtttgact ggaacacacc caagaaaggt ggcaggaggt 600
   cacagtgggt ccgaaactgg gctgtgtggc gctactttcg agactacttt cccatccagc 660
   tggtgaagac acacaacctg ctgaccacca ggaactatat ctttggatac cacccccatg 720
   gtatcatggg cctgggtgcc ttctgcaact tcagcacaga ggccacagaa gtgagcaaga 780
   agttcccagg catacggcct tacctggcta cactggcagg caacttccga atgcctgtgt 840
   tgagggagta cctgatgtct ggaggtatct gccctgtcag ccgggacacc atagactatt 900
   tgctttcaaa gaatgggagt ggcaatgcta tcatcatcgt ggtcgggggt gcggctgagt 960
   ctctgagctc catgcctggc aagaatgcag tcaccctgcg gaaccgcaag ggctttgtga 1020
   aactggccct gcgtcatgga gctgacctgg ttcccatcta ctcctttgga gagaatgaag 1080
   tgtacaagca ggtgatcttc gaggagggct cctggggccg atgggtccag aagaagttcc 1140
   agaaatacat tggtttcgcc ccatgcatct tccatggtcg aggcctcttc tcctccgaca 1200
   cctgggggct ggtgccctac tccaagccca tcaccactgt tgtgggagag cccatcacca 1260
   tccccaagct ggagcaccca acccagcaag acatcgacct gtaccacacc atgtacatgg 1320
   aggccctggt gaagctcttc gacaagcaca agaccaagtt cggcctcccg gagactgagg 1380
   tcctggaggt gaactgagcc agccttcggg gccaactccc tggaggaacc agctgcaaat 1440
   cacttttttg ctctgtaaat ttggaagtgt catgggtgtc tgtgggttat ttaaaagaaa 1500
   ttataacaat tttgctaaac cattacaatg ttaggtcttt tttaagaagg aaaaagtcag 1560
   tatttcaagt tctttcactt ccagcttgcc ctgttctagg tggtggctaa atctgggcct 1620
   aatctgggtg gctcagctaa cctctcttct tcccttcctg aagtgacaaa ggaaactcag 1680
   tcttcttggg gaagaaggat tgccattagt gacttggacc agttagatga ttcacttttt 1740
   gcccctaggg atgagaggcg aaagccactt ctcatacaag cccctttatt gccactaccc 1800
   cacgctcgtc tagtcctgaa actgcaggac cagtttctct gccaagggga ggagttggag 1860
   agcacagttg ccccgttgtg tgagggcagt agtaggcatc tggaatgctc cagtttgatc 1920
   tcccttctgc cacccctacc tcacccctag tcactcatat cggagcctgg actggcctcc 1980
   aggatgagga tgggggtggc aatgacaccc tgcaggggaa aggactgccc cccatgcacc 2040
   attgcaggga ggatgccgcc accatgagct aggtggagta actggttttt cttgggtggc 2100
   tgatgacatg gatgcagcac agactcagcc ttggcctgga gcacatgctt actggtggcc 2160
   tcagtttacc ttccccagat cctagattct ggatgtgagg aagagatccc tcttcagaag 2220
   gggcctggcc ttctgagcag cagattagtt ccaaagcagg tggcccccga acccaagcct 2280
   cacttttctg tgccttcctg agggggttgg gccggggagg aaacccaacc ctctcctgtg 2340
   tgttctgtta tctcttgatg agatcattgc accatgtcag acttttgtat atgccttgaa 2400
   aataaatgaa agtgagaatc caaaaaaaaa aaaaaaaaa                        2439
<210> 11
   <211> 3318
   <212> DNA
   <213> H. sapiens
<400> 11
   gtgatgcgta gttccggctg ccggttgaca tgaagaagca gcagcggcta gggcggcggt 60
   agctgcaggg gtcggggatt gcagcgggcc tcggggctaa gagcgcgacg cggcctagag 120
   cggcagacgg cgcagtgggc cgagaaggag gcgcagcagc cgccctggcc cgtcatggag 180
   atggaaaagg agttcgagca gatcgacaag tccgggagct gggcggccat ttaccaggat 240
   atccgacatg aagccagtga cttcccatgt agagtggcca agcttcctaa gaacaaaaac 300
   cgaaataggt acagagacgt cagtcccttt gaccatagtc ggattaaact acatcaagaa 360
   gataatgact atatcaacgc tagtttgata aaaatggaag aagcccaaag gagttacatt 420
   cttacccagg gccctttgcc taacacatgc ggtcactttt gggagatggt gtgggagcag 480
   aaaagcaggg gtgtcgtcat gctcaacaga gtgatggaga aaggttcgtt aaaatgcgca 540
   caatactggc cacaaaaaga agaaaaagag atgatctttg aagacacaaa tttgaaatta 600
   acattgatct ctgaagatat caagtcatat tatacagtgc gacagctaga attggaaaac 660
   cttacaaccc aagaaactcg agagatctta catttccact ataccacatg gcctgacttt 720
   ggagtccctg aatcaccagc ctcattcttg aactttcttt tcaaagtccg agagtcaggg 780
   tcactcagcc cggagcacgg gcccgttgtg gtgcactgca gtgcaggcat cggcaggtct 840
   ggaaccttct gtctggctga tacctgcctc ttgctgatgg acaagaggaa agacccttct 900
   tccgttgata tcaagaaagt gctgttagaa atgaggaagt ttcggatggg gctgatccag 960
   acagccgacc agctgcgctt ctcctacctg gctgtgatcg aaggtgccaa attcatcatg 1020
   ggggactctt ccgtgcagga tcagtggaag gagctttccc acgaggacct ggagccccca 1080
   cccgagcata tccccccacc tccccggcca cccaaacgaa tcctggagcc acacaatggg 1140
   aaatgcaggg agttcttccc aaatcaccag tgggtgaagg aagagaccca ggaggataaa 1200
   gactgcccca tcaaggaaga aaaaggaagc cccttaaatg ccgcacccta cggcatcgaa 1260
   agcatgagtc aagacactga agttagaagt cgggtcgtgg ggggaagtct tcgaggtgcc 1320
   caggctgcct ccccagccaa aggggagccg tcactgcccg agaaggacga ggaccatgca 1380
   ctgagttact ggaagccctt cctggtcaac atgtgcgtgg ctacggtcct cacggccggc 1440
   gcttacctct gctacaggtt cctgttcaac agcaacacat agcctgaccc tcctccactc 1500
   cacctccacc cactgtccgc ctctgcccgc agagcccacg cccgactagc aggcatgccg 1560
   cggtaggtaa gggccgccgg accgcgtaga gagccgggcc ccggacggac gttggttctg 1620
   cactaaaacc catcttcccc ggatgtgtgt ctcacccctc atccttttac tttttgcccc 1680
   ttccactttg agtaccaaat ccacaagcca ttttttgagg agagtgaaag agagtaccat 1740
   gctggcggcg cagagggaag gggcctacac ccgtcttggg gctcgcccca cccagggctc 1800
   cctcctggag catcccaggc gggcggcacg ccaacagccc cccccttgaa tctgcaggga 1860
   gcaactctcc actccatatt tatttaaaca attttttccc caaaggcatc catagtgcac 1920
   tagcattttc ttgaaccaat aatgtattaa aattttttga tgtcagcctt gcatcaaggg 1980
   ctttatcaaa aagtacaata ataaatcctc aggtagtact gggaatggaa ggctttgcca 2040
   tgggcctgct gcgtcagacc agtactggga aggaggacgg ttgtaagcag ttgttattta 2100
   gtgatattgt gggtaacgtg agaagataga acaatgctat aatatataat gaacacgtgg 2160
   gtatttaata agaaacatga tgtgagatta ctttgtcccg cttattctcc tccctgttat 2220
   ctgctagatc tagttctcaa tcactgctcc cccgtgtgta ttagaatgca tgtaaggtct 2280
   tcttgtgtcc tgatgaaaaa tatgtgcttg aaatgagaaa ctttgatctc tgcttactaa 2340
   tgtgccccat gtccaagtcc aacctgcctg tgcatgacct gatcattaca tggctgtggt 2400
   tcctaagcct gttgctgaag tcattgtcgc tcagcaatag ggtgcagttt tccaggaata 2460
   ggcatttgcc taattcctgg catgacactc tagtgacttc ctggtgaggc ccagcctgtc 2520
   ctggtacagc agggtcttgc tgtaactcag acattccaag ggtatgggaa gccatattca 2580
   cacctcacgc tctggacatg atttagggaa gcagggacac cccccgcccc ccacctttgg 2640
   gatcagcctc cgccattcca agtcaacact cttcttgagc agaccgtgat ttggaagaga 2700
   ggcacctgct ggaaaccaca cttcttgaaa cagcctgggt gacggtcctt taggcagcct 2760
   gccgccgtct ctgtcccggt tcaccttgcc gagagaggcg cgtctgcccc accctcaaac 2820
   cctgtggggc ctgatggtgc tcacgactct tcctgcaaag ggaactgaag acctccacat 2880
   taagtggctt tttaacatga aaaacacggc agctgtagct cccgagctac tctcttgcca 2940
   gcattttcac attttgcctt tctcgtggta gaagccagta cagagaaatt ctgtggtggg 3000
   aacattcgag gtgtcaccct gcagagctat ggtgaggtgt ggataaggct taggtgccag 3060
   gctgtaagca ttctgagctg ggcttgttgt ttttaagtcc tgtatatgta tgtagtagtt 3120
   tgggtgtgta tatatagtag catttcaaaa tggacgtact ggtttaacct cctatccttg 3180
   gagagcagct ggctctccac cttgttacac attatgttag agaggtagcg agctgctctg 3240
   ctatatgcct taagccaata tttactcatc aggtcattat tttttacaat ggccatggaa 3300
   taaaccattt ttacaaaa                                                3318
<210> 12
   <211> 78001
   <212> DNA
   <213> H. sapiens
<400> 12
   cagattccgg gtctcatccc tagaccaaag atatctgaat catttggcga tgggtccagg 60
   aacctgcatt attcaacaca cttcccaggt gaccgtaaat gtcaaaagac aaaattacaa 120
   caaacttaaa catcttaatt ggcttcattc atgattctag aatcgggcaa gacttcattc 180
   ctcaaaatag cacaagtgtc ccaatgagct gagcagagga ggttggtttt atagacagaa 240
   aagggctgaa aaaagcagaa acaaagaaca aaaagcagat tggtcatttc aaagttactt 300
   tccttgtaag gcaggaacag ggaaacagaa caagagagaa ataactgatt ggtcgcatcg 360
   ggttacttca ggttactttt tgttgtaagg attaaagcaa agggaacttc attatgttga 420
   ttaaaacggt ctgcttggga aatcagggtg tgtatctctc ttctgatttt gtgaaaggtt 480
   atcagtctga tgatgtagaa ctttagcatg agtgactcca ttttgatttt tagtctagtc 540
   tgttgagacc ctaatgccag aacttttttt gtttgtttgt ttcgctcttg ttgcccaggc 600
   tggagtgcag tggagctatc ttggctcact gcaacctctg cctccagggt tcaagcgatt 660
   ctcctgcctc agcctcccaa gtagctggga ttacaggcat gcgccaccac gcccggctaa 720
   tttttttttt tttttttttt tttagcagaa accgagtttc accatgttgg tcaggctggt 780
   ctcggactct tgacctcagg tgatccacct gcgtcggtct cccaaagtgc tgggattaca 840
   ggtgtgagcc accacaccca gccctttttt ttcaagacag ggtctctctc tgccacccaa 900
   ggtggagtgc agtggcgcca aaacagctca ctgcagcttc cacctcctgg gctcaggtga 960
   tcttcctgcc tcagcctccc cagcagctgg gccccaccac accggctaat tttttaactt 1020
   ttagtagtga cgaggtctga ttctgttacc caggctggtc tggaactcct ggcctcaaga 1080
   catccgcctg cctctgcctc ccaaagtgct gggattacag atgtaagcca ccgcgcctgg 1140
   gctcctatga tttttattta acataatgca ccatggaatt tgtgctctgc ttagttcagt 1200
   ctgagcagga gttccttgat acttcgggaa acactgaaaa tcattccatc cccatccatt 1260
   cattcctgca gcacccaagt ggaaattctg cgtttcagac agggacacta cccttagaga 1320
   gcagtgggct tccccagcag cgtagtgaaa catgatactc ctgagtttca tgaaaaaagg 1380
   gcagacatct ggccagagct gggaggcagg aaatagagca cggtgccctc ctcccatact 1440
   ccagcttgga ttactgaggc tggggcccag gccctgcagg aaaggaggtg catgactact 1500
   ttaaggccac tcactctgtg actcaacggg ccgggtcggg gctggaactc aatgccctcc 1560
   cgggcctgga gagcccacgc gccgtgggcg gggctcccgg ggtcgcctag gcaacaggcg 1620
   cgcgccgcgc ccgagcccag agccccaaag cggaggaggg aacgcgcgct attagatatc 1680
   tcgcggtgct ggggccactt cccctagcac cgcccccggc tcctccccgc ggaagtgctt 1740
   gtcgaaattc tcgatcgctg attggtcctt ctgcttcagg ggcggagccc ctggcaggcg 1800
   tgatgcgtag ttccggctgc cggttgacat gaagaagcag cagcggctag ggcggcggta 1860
   gctgcagggg tcggggattg cagcgggcct cggggctaag agcgcgacgc ggcctagagc 1920
   ggcagacggc gcagtgggcc gagaaggagg cgcagcagcc gccctggccc gtcatggaga 1980
   tggaaaagga gttcgagcag atcgacaagt ccgggagctg ggcggccatt taccaggtgc 2040
   gggagcgccc cggagcgtgg cgggcccttc gcttaggccg cttgaacatc ccctcagacc 2100
   tccaggcccc agactccctc tgggtcttgc cctctgcctc gctcctactg cttgaggatt 2160
   cgatgggaca gcgacgcact gcgtcccccc accctttgtc cccggggcgg gcgtgtttct 2220
   cgccgcagcg tcggagcccc cttcgatccc ccacctccct tctgttctcc agctcgggtg 2280
   atctctcaag ccgggggacc gccggtctgt gctctcaacg cgaatccctc gcaccccgac 2340
   cccgccccct gcctgtccac tctttgtccc ctggggtgat ttagcacccc cactatttcc 2400
   ttttctggag tggaccacct cagactctct tcctttgtct ccctggggga aaaggttact 2460
   ccccccgtcc ctccttcaca tttcctttcc cctagtctca gtgtgcgtcg agtcccagag 2520
   atgacagtcc cctttcccct ttctgttcat tcatttattg gataggagtt ggcaagctta 2580
   ttctgtgcta ggcaccgctt aggcattgga ggtggtgttt gctaatcagg acaggcaaga 2640
   tcctagcctt agtggggcct agagtcgaat agggcaatca aacacaaaag caaataattt 2700
   cagatagtga caggtgctgt gaagagaacg acttcctaac ggggtacagg gtgactgcat 2760
   agaaggccgg ctgtcttaga gaaggggatc agggaaggcc tgtcaaagga ggagacattt 2820
   gctttgtgag ctgaaccaag aggagcagaa agccgtgaga atatggggct aaagaacctt 2880
   ctagccagga ggcctgcggt acccactcca ttggggccat gatattattc tttcaggcag 2940
   ggactcagga aggttaacgt tttaaccctc tctaaaatag catctttcct caatgagcag 3000
   cttagtcttt ggtcgtggca gagatgacct tgtcttagga gtcatctcct tgtgtgttaa 3060
   aaagttagga aaggagggtt tctcatatat ctataaaaca agtagttaaa aacacaaaga 3120
   gctcttcctt tcacaagcag ctgaataaga tacatactcc caattaaatg tcattgcggg 3180
   ggttgttaag attaactaaa accacacttg cacagtatct taaataagcg atatacagaa 3240
   tagagagatt ttgttacttg tgtaaaggga gacagcagat gattctgttt tcagcttata 3300
   ggctcaaaag gcaaattgtg agatccatca gctgtagtat taaaatctat tttgagctcc 3360
   gcttagaaag gaaaaaaggt ttaagcagtt ctttggtatg cttgactaac aaaagccttt 3420
   ttttttggca gccttgattt tcatgtggat ttacatcaag cttatttgac aggattcttt 3480
   ttatttggac tgtagtgtgt atattagttt ctgctagact aatatttcta accactgtaa 3540
   tctatatact aataagtatg attgatcagt atataaaatt tgtatgccat atctggtctc 3600
   tgaattagct gaatgaattc cataagggac tttgagactg tgtagacaaa ttttctgcat 3660
   cagtttaatg cagtagagtc taaaatgtct ttaaatgaaa attgttggtc tgaagtgttg 3720
   gagttgatta tgatacaccc catcacagtg gaagcattgt ggagagaagt cttttccact 3780
   gaaattgact gagttgacaa caagaaatac gtattgtaac ttagttctta gttgaatttt 3840
   atttcttaca attttaagcc agagtgggtt gacctgtcac ccaagcatgg ttaaaattgt 3900
   attcagcatg caactagcat ggagtgtgtc agtcttcaat tcatttcctt cattgttctt 3960
   aagtttttct gccacaatta aaccccacaa gttagtcaag gtgttgagat tttcactgct 4020
   tcttaatgga ttgccacatt ccctgaggta gtttcttttg gtcttagaga attgtcaggg 4080
   ccagcttttc tcacctccac tgtatggata tttttctttt ctaagatctt gaaatcagaa 4140
   gcttttctcc taagtgtaaa agtagctctt tgtcatacaa ctgtagcgtt ttctgaaaca 4200
   gagttcagat gaccttgagt ctaaagtggc taactttcca aggtgtgtat cgctttacca 4260
   aaaccattat ttttcaagga ttcaaagaat gtgtttacaa ttgatagaaa atggaagttt 4320
   aaaaaaatta atactttata gcatgttgaa atgagggcag ccttatacaa agtcatactt 4380
   tgagcttgcc tagcctattg tgatcagaga ataatgtaat ttttgcttac aacttggtaa 4440
   gcaggtcagt tattctaact tattttctga ttagaacaaa aagatgtaaa aacttgaaaa 4500
   ctattgggaa aagaacaaag agtgaagagg acttttgagt gctgaggaat gtggcagctt 4560
   ggaaaacaaa ctttttaggc agagattctt tgctaggtca gtttgataaa gtgagcataa 4620
   ccgtattttt aatctttaat gctaatgaat agcatagatg ctaataagca tctaggtcta 4680
   taaaaagtca gctttgatag tgtatataga tggctttaaa cattgttttc tagcatttaa 4740
   acactttcaa atcatccggt tgcttgattg ggcctagctg tctaagagga gagaatgagc 4800
   ccagatgagg aaaagagatt gattttactg agctagaatg agaggagaga gggttgagtg 4860
   aatgaaaaga atagctcatg tgctcccctc catctgtagt ttaagagggg ttgggtccgg 4920
   tgttttgctt gttttctcgt ctgtaaattc tttgattctc tgacaccact cactatattt 4980
   cattgtgaat gatttgattg tttcagataa aggggactgc aataatacct tgtgacatga 5040
   aggcaagatt tattcatgtt agaggcaggc tttgtaaaat gggccactct tccaattgac 5100
   atttgttttt atagctgttt tcattatgaa atacaatcta atgcctgact aggttaaaac 5160
   catgttgtaa caatagttca ctaaaattcc ttactgatat acagcttatg ttgttatatt 5220
   ccaaaaagat gaatattaaa atttgccaat aatgtttatt taaatactat tttcttcaga 5280
   ggaaaaaaaa ctattttatg caaaggagaa agatctatac actatgactc acttcactta 5340
   aaaaaaaaaa gactaacgga aatgacatgg agagactggg aagttctagt catcttgagt 5400
   gacccattag atctaaatgt tcttgtttag ccctggtttg agtgaactaa atttaggtgt 5460
   ctgatcagta ctttggaaat ggtgtaaatg cctttgtaat tgtctggact gatattagat 5520
   taactgggag cacaagtaga aatagtgaag gaaagaactt tttgctattg ttatttgaca 5580
   tcactggcat atttatagga atactttggt gtttttggaa gtaagtaaac caaccagtgg 5640
   ttctaaaaag tcagctgggg gataatggta atgccgctgt ttcttagctg caagttatct 5700
   gccgttactt ctcctccatt ttgcatttta tcttgaatag ctcctcaaaa cctattaaaa 5760
   tacctggtat tgaataatgt aattgaatgt gtactgaatt tcacagtgga aatgaataag 5820
   aaatttcctg tggaggtttt ttgacttagc tactgaaata acggcctttt gttgtgtgat 5880
   tctttccctt ttctctttgt taaagaaaac tgtcttgtga tcttgtagat tacagaatcc 5940
   ttttggcaat ttctgttcct agcactgctt tttctttctt tctttctttt aaatagaaat 6000
   ggggttttgc tgtgttgccc aggttggtct tgaactcctg gcttcaagcg atcctcccac 6060
   cttggcctcc tgaagttggg attgcaggcg tgagcaggta ctttttctga ggcctgcctg 6120
   agcctatata tattttgcac aatttggcat tcctccctac agtgtttatg ctgatttgtt 6180
   tctggtaaca actaatactg gcaaatcggc tgggcatgtt actttatgct gcccatattc 6240
   aggaaaattg gaattctagc tgggtcattg ttcccagatg atgtagtttg gcaccagcca 6300
   ttccatgttc acattttgag tatccaggag ggctggggac tttggagtag ttggtgattc 6360
   cctctgccac atttcactgg ttggtcacta tggcatcctt tccaccacac tagtagtcta 6420
   ggttctcaga tgttgcttat gagcctgcaa tggtttctag tttcacactg cagaaatgag 6480
   tgaagccggt tacccgttaa tatggtccca tcatcactag agtaattcat tgttctaaaa 6540
   ccagatctga gtctctcact cctctgcaac tacttctgat tctttcataa cacttgtaaa 6600
   gtccaaactc ctctttagca tggcagccag cttccagtcc ttccctccta tgtggcttcc 6660
   attctagcca gacaagaaag ggcagcgttc tccaaactca tcctcgccct tcattcctct 6720
   ataccattgc tgagcacttt gttgaggatg cctctcccgt tcaatctagc ttgcatcttc 6780
   cagctcgaat gtgtgcttcc ttgcaccaga gttttgttcc gtcacctgtg tgttttcata 6840
   caagctggca catatctctt ctaaagccct gctgtcattg tagctgcgtc tttacaaaca 6900
   tttttttttt aaatttttat aaagtcaagg tctcactata ttgcccaggc tggtctcaaa 6960
   ctcctgggct caagtgatcc tcctgccttg gcctcccaga gtgctgggat tataggtatg 7020
   agacactgtg cccagctgta gctgctactt tatatcccag gtctatctcc aatggagccc 7080
   aagcttcctg aggccacctg ttgtatcttt ctcattcatc ttgaagtcct ctgctcctgg 7140
   cacagagtag gtacctaaca agagttggga ttgaattgat ggtcagtact ttgctagcct 7200
   gatggtataa agatgtacaa aacatgttcc tggctcccac tctagggggg caatgatgga 7260
   aacaaataga ttagcccaca ttagtaccaa tagtagaggt cactctggga gaaggccccc 7320
   accacatttt gagtcatggc ctaatgaggt aatttagtat tgcctgctgc agtggctttg 7380
   gaagaaaggc tggcattctt agccagtaga agctgatacc actgatttgt ttcacagaag 7440
   ctttaaatat aacaataaat ttgtgcttgg cctacggtga actttacagg caacttggag 7500
   gtaatatgtt tgtctctcta agaattgttg aattcctctt ccctcatccc tcctgactgg 7560
   ttctcacaag cctagcgggc ctttgcatgt ggttggttca taaaatactt tttgattttg 7620
   ggatataaaa tatagttctc cataaaataa cgactgttac caagtctttg attttttttt 7680
   tcaaactata aatggtaatg acattctttg gcctttgatc agaccaccct taggggcaag 7740
   agagtagttt catgttttgc tttttctagt gtcccctgtg tctgggtata gttgcagtct 7800
   cagctgtcat actaacagtg ctgagtgagt cccttacttt ctttgggttt tggtttctcc 7860
   cttgtaaaaa tgatcctgga ctaactgatc attaagttca ggtcaagtaa taaaaatcct 7920
   taatgtactc acaaatacaa tttaatgttc ctgaataatc cttgtaaaaa ctgcagcagt 7980
   tactcagttt tgtaaggtgt ggttgggtac tattaggctc aaaagtttat aggagctttg 8040
   tgagtatagt taacaactca aaagaatggg gtgttttttc ccgaggggca tgaaatgttt 8100
   ttgataaata gagttcattt gacttggtaa tgtggaaaat gagtagccct gacacgtacg 8160
   ctatgctttt gcagtttttc tctcaagtag caattgggtg gcttttcctg taaaagatag 8220
   aggaactgat tcttgagaat ttacgaaagc ttcaacccta actaggtatg caaagaatag 8280
   ttgcccttta tgttgtaatt ttaggaagaa acctacatct ggtctaagtt tcatttgaat 8340
   aatatgatag tttacacatc tgccatattt gagaagaaag tacctaagtc tccagcattt 8400
   tagaaataat gctttacttt gtgtagaaat ggtctttaga gtttaatagc tgctgccctc 8460
   tcctttttca aagcagcttg acataatcat gagtatcttg ctgacagctt gtaaattttg 8520
   attgtatgaa aactgaaaat aagaccattt cacatggaag attccctcct gccctgaaac 8580
   agccaaagaa aactgtagcc atcaaatcta ttgatctctg ggctttggta caagtcacac 8640
   tactacaaat aaaataatac caagtactta taaatgattt tcagtccttt taaagtttat 8700
   ttttttaata ttttttttga gatggggtct tgctgtgtcg tccaggctgg agtgcagtgg 8760
   cacaatcttg gctcactgca acctccacct cctgggctca agtgatcctc ccacctcagg 8820
   ctcccaagta gctgagacta caggcatgtg ccatcacgcc cagctaattt ttgtattttt 8880
   ttggagtaga gatgggattt tgctgtgttg cccaggctgg tcttgaactc ctgggcttaa 8940
   gccatctgtc tgcctcaggc tcccaaagtg ttgggattac aggtgtgagc cactgtgccc 9000
   ggcccagccc tttttttaag agaaaaacgt atgacatcgt tcgatttact gagtgcttat 9060
   ggttttacta aggcagtaag gttttatgga taccctatgg taattagata gaattagtgc 9120
   tctgaagtca gctctgtaat atggactcag agtaaacatg gcaaagggac acttaaggtc 9180
   tgcattttct ctgggaaata aacgtattct ttactactct gaatctagtg ctgggaaatt 9240
   ctaaatcctt cttgaggatt aaccacttga agtaaagttt tgggtcccaa gtaggcttgt 9300
   gtccctgtct ccttctcttt acttttcaga tgtttcttcc tagagactga ggtatatttt 9360
   acttttacag atgaagaagg aagcctcggc tgtgtttgtg gcttttgtgg gtgagcaaca 9420
   tcacttgcaa agataagatg agcatagcaa aactaggctt tcaaaataat ttttaaaaat 9480
   ttcttagtga ttagaaaagg aaaactcttc ccttgtctct gttaagaaac gtttttcgac 9540
   ttttttcctt tcttaatgga tcttttattg gcacttctct tccttttgca gaatcttact 9600
   taaaagtcac tacgttacat tacagcaaac agcttagcta atttttatcc agatgggccc 9660
   cggttacagg attgtacact attgcgaatt tcttacagga aagtgaacat caagtaatta 9720
   ttccaaatag agttctctta agaacgtgag ttacttaaaa atgtctaagg atgaagtcac 9780
   ttctgaatat aacttcactc aagagaacaa ataagcaaac tgcatttagc ataacatggt 9840
   aaattagctt taactctcct tgatgtttga acatttgtcg ctgttaacta ctgtttcact 9900
   tttcaaatag tcagggctta gtttgcttct gtaaggataa agggaaaata cgccttcact 9960
   gagtcataaa tatttttgtg gctaactttt gcacagagaa aagaggcctc taagaaggta 10020
   cccagtgaat ttttttttcg gggcagggag agaatatgtc attttttggt ttgttgttgt 10080
   tgttgtcatt gttttgcttt gttgttttta ctctgaactg aactgtatct tgacagcact 10140
   tttgaattaa gagcattact cttattgttc tctactacct ggacgccacc tccctgttgc 10200
   catagtgtta aggatcatgc tccgaggtgg ggtgaggcag aatggggcca agatcagaaa 10260
   gttacattaa gctacatcag gtttatacaa gcataaaacc aaatttttgg agcagtcccc 10320
   agaatacaac ctggtttagc cacacctaaa ggttgctctt gaatattcct tgagaatcca 10380
   catccctaga atgctgggtt tcaatgggcc ctttatgtac ctatcatggt gtcatttctg 10440
   agcatttcta aatattcctt catgtcttac tgacagtttt tcttgaataa atcttaggaa 10500
   tattagtgcc attatcagta ttttgtttgg tctgttcaca ccacaaataa ctacccaggt 10560
   ctgctacttg cccctatttc tctacctgct aatgaaaatg cttttgaaag tttgagtaac 10620
   agtattggag tgtgcacagt ggtattggta ggttctgtac tcatccttaa ccacttgttt 10680
   tcatcctttg tgagcttgaa gtttctccaa aaaatttatc acaaaactta tcagacatag 10740
   ttaatacact cagagagaga atcactgaaa aagtagatgt agtttaacaa acccagtgcc 10800
   ttttttttac ccatgaatac atatttgtca actaaacctc attttgcaac ttgttccact 10860
   actcgaatgg taacaaactt ttggtttccc aatagatttg gaagatgttg cttttgaaag 10920
   taggaaatag atggctttag aagatggaag aatattttgt ttgaagtggg agcgtggtat 10980
   gtccttagct gtctgtgaaa tgcagctgaa gatgggtgtg ggccttcatc tgcatttccc 11040
   atcttcagtt tgaggaggta gttacccttc taaccactta agaactgcat ggtacatgct 11100
   gttttattta cagggcaaaa ctgtgctccc gtagtttccc tggtgcttgc cttcacgtta 11160
   acacagtgtc atcgtttggc agtgtttatg tgccagggtc catgttagaa ggaggaaagg 11220
   tatagcgaag ttaaagggtg cagttggcct cccaccttta gttttgtaag tgcctttaaa 11280
   gtttgatttt tgtaggttga tcataaggaa gtgataagta tgttaggtta tttgtggttt 11340
   gagctaattt tagtctcttt ttacagcttg ctttgtatcc tttgccatta aaacatgctt 11400
   tctagaaaga caacttttga atgtaggaca cagtctatat tctatacttg gctacatttc 11460
   aaaaaatatt ttctcagtac tttggaagtt ggacagttgg aagcatagtg acagtattta 11520
   aaaatctttg attccggccg ggcatggtgg ctcacgcctg taatcccagc actttgggag 11580
   gccgaggtgg gtggatcact tgaggtccgg agttcaggac cagcctgacc aacatggtga 11640
   aaccctgtct ctactaaaaa tacaaaatta gccgagcgtg gtggtacatg cctgtaatcc 11700
   cagctactca ggaggctgag gcaggagaat cgcttgaatc tgggaggcgg aggttgcatt 11760
   gagccgagat cataccattg cactacagcc tgggggacaa gagtgaaact ctgtctcaaa 11820
   aaaaaaaaaa aaattaagtg atttctttgc tttgtgacac ttctactttt ccagcaagta 11880
   aattatattc tttcatacag gtatgaaatt cttgttccaa gctagtggtt aaaaaggcac 11940
   agttgatatt agaggatttg taaaagatta tgaccacgcc tgcaatgtac tgaagcaagg 12000
   ctttgctggg ctgtgtatag gaaaccttcc ccagcctgtg cccttgcttg atagaacatt 12060
   ttgctcctaa gggtaggtgc ctgtatctgt ctccagtact ggttagtttc acacagaaca 12120
   gttgtgtttc agagctttag tctcaagctg ccctgctccc ctgaagcagc caccctgagc 12180
   atgtgcactc acaggagggg acatgtgagg tcatggaaga agacgactca ggaagaagaa 12240
   gacttgggtt tgggttctga ctctgccttt gactgttgtg ggattttgag gagttgcata 12300
   caggatctgt aaaatgtagt cattagacta gactagacag ccatatagca ttacctagat 12360
   gtaactttct acaaagacat ggtcacagga gaagaccaga gggtggggtg atctttctgg 12420
   aaaaattggg gcttcatgcc ttactcatgc tagatatggt agcattatat ggctgtgcct 12480
   gatcccccta atctaaaagt gggacagaac tttaaaattt catattaact caaattaaaa 12540
   cttgaaaaaa acccattatt tccttaaaaa taataaaatg ccctgtgggg gcataagtca 12600
   cattatattt taaaattcct gaatgccaca tggatgaatg tagttccttt tgaaattctt 12660
   cttttgtcta aagaggaatg ttggattttg taattggact aaaaaatctt ccatttgaga 12720
   gagaaacagt ctgctgcatg ttctaccctt gttcaggata aaacccacta atagctaaca 12780
   tttattgaat tctgtgttgt gcctcaggca ctgtgcaaag tcctttacat gcaatgctgt 12840
   ttattatata ctgtcaattg gtctataaca gcaggaaatg tttcaggagg acaatgaggt 12900
   cccagaccct cagtcttctc ctgtgtcctg gattcagctt cacaatagca ctatggcagt 12960
   gtggccactg cttcagcttc cacatacatg gctgtgaaga gagacagggg attgtgctaa 13020
   gcctccccga tttattagga cataggagga gagagtttgt agtttttgac ctttgcctag 13080
   ttttctaacc tctttcctag atgtcacaaa ttggccaccc acagtcatat tttgcttgct 13140
   tcacgcaatg ctttttaaaa aagagaagag tttaatttgt gccattgttt ataaatgaat 13200
   caggagaaat gacatgcaac tctggattct ggcctctctt gaaaaatctg aaaatcacac 13260
   cgtctgagct tacactggca gtggtctgct ggactgaggg acacaactcc ttttggatgt 13320
   acatgtgtgc gttgcagagt ttaccacagt cccacagtgg gtcacactgt ccttgtcggt 13380
   gtacactacc tagcacttga gtttgcaacc cctaccccaa gctgagtttt ctcgtcaagc 13440
   ttgatgttaa tgttatgtga tgcttggcct tgtaggtatt tggtatatta tcgttagata 13500
   aaattgaagc aaagggctaa agggttggtg gcctgaggga gtgcccttga cagtaaagtc 13560
   taggataaaa tcattggcca ggtactcctt cccttcccgc ccttcctctt ttctctttat 13620
   cctcagcctc cttctgctat tttgaggaag ttagaagcca ccaccatttt ttcccacctc 13680
   aggcaactga gtgtggctgt atttctgtcc catgttcagt tatttccagg aactattttt 13740
   gatgaccaac ttgaagttac attgggtggg cctaatgggg gctgataaaa gaatgaggtg 13800
   accaaatatg cttgcactga gacggctacg aagtaaggtt tttaatgact tgctttgtga 13860
   cttggtcagg agtgatacca tttgtcatgt gtccaacttc atgactaaat ggttgctcta 13920
   ccttatcctc atagctataa taaaataaaa taaatacata cattgcaggg aggaatgtat 13980
   cttgttaaag gtctctccct tttagcaaca aaagtacata ttatgttgta gaacatgctt 14040
   tttctttgat ccttcttgaa cacctattac tctatagagg tatgttgtgt atggcaaatt 14100
   agaacaagca atagataagg atgattcttt accattataa cccagtcaag gtctttgtcc 14160
   taagttttgt acctttctcc agagggaaag gtatttgtat ttatttattt atttttgagg 14220
   cagagttttg ctcttgttgc ccaggctggg gtgcaatggc acgatctcag ctcactgtaa 14280
   catccgcctc ccgagttcaa gtgattctcc tgcctcagcc tcccgagtag ctgggattac 14340
   aggtgcctgc cacgatgccc ggctaatttt tttttttttt tttgtatttt tagtagagat 14400
   ggggtttcat catgttggcc aggctggtct tgaactcctg acctcaggtg atccatccac 14460
   ctcggcctcc caaagtgttg ggattacagg catcagccac tgcctccggc caggtatttg 14520
   tatttttagt ctctatgcct taccgtctca gatcaggagg atttggtgat ttatcgaatg 14580
   tgggggaagg ggaagaagag gaaacgggag gaatgttcca gattagggaa atagctagat 14640
   ggaagatgca gcccctcatc aaggtgggga cacaggaaaa ggaacgtgtg caaagaagat 14700
   ggtgatctgg ttgtgaccat gttgttagag gacgtccagg gaagcatctg gtaggtggtg 14760
   gggtgtttaa atatagaaca ttcggagaat gctccgaagc ttcagagaac ccttcccaaa 14820
   aggacaaaac cagctcagtg ttttagcact ccgggatcat atggcatgac agcatggctg 14880
   ctttatactt ttttgtgtat gtgaaattaa aaccaaccac tcaggaccaa tttctctgaa 14940
   gctttttgtc aatctttcat ttgcttttct cgtctagatt gtaagctcct tgcagccagt 15000
   gtctgttgat tcagtcattc aaaaaataat acatgaacag ctactaggta ccaggctctg 15060
   tgctgggcag ttgggatatg tggtgaggaa gacaaacttg gtccctgccc ttaggaagtt 15120
   cagtagtcca gcagacaaag tggctgaata aagataatct cagttcacag tgataagagc 15180
   tcttacaggc ctaggctcca ggtgctgtgg ggatgctcag gaaaaggtat ctaattggga 15240
   ttgggagcag gcaaaacaaa taaaggatag tgtataaagg taatatctag ttgaagttct 15300
   gaagggcaag gaggagtgag cctgtatatt ctctgagtct ctccctaatc tgggattgac 15360
   ttcttgtccg tctctgttca tattaagtgt cacctaggct tgaaagggtg agatcatatt 15420
   tcacttcctt cctctttggt cttaaccttt ctctgctacc ccctcacaca atgcatatgc 15480
   attattctct tattgtatat atttttcctc tcttcctttt catgtttcct ctgccattac 15540
   ttttaacctc gactgccata tggcctctaa acgcttccag aagggtagcc tagtggaggt 15600
   tattccatca tggccttgag ctcatgcgac cagatagtga aggcatctgt gtaggtgtct 15660
   tctccaggag ggtgatattt gtttcattgt aaattttgta gccctagaac accaacaaca 15720
   gtgcacagta attagtaggc aggcagtaca ggattcattg aagtgaagtg ataactttta 15780
   tccaagtatg tatgcagata atctttgatt tgtacaaaaa aaattatatt ttaatatgta 15840
   aagatttttt aaaagaatct tcaagtttta gccttcccac taggaatata ttgaaaacat 15900
   gtgcctagtt cactgacttg cagctgccac tatgagaata aaggtctcat ttagttgttg 15960
   tgaattttaa gggatatttt caatgatgtt ggctggttta tcccattatg tggtcttttt 16020
   tttttttttt tttttttttt gaggtggagt ctcgctctgt cacccaggct ggagtgcagt 16080
   ggcgcaatct cgactcactg caacctccgc ctcccgggtt caagcgattc tgctgtctca 16140
   gcctcctaag tagctgggat tacaggcgcc tgccactacg cccagctaat tttggtattt 16200
   tttggtagag aagggtttca ccatgttggt caggctggtc tcgaactcct gacctcatga 16260
   tccactcact tcagcctccc aaagtgctgg gattacaggc gtgagccacc atgcccagcc 16320
   tatgtgctct tattagcaat tctcagtaca cagatagctt tgagtgattc tttcaagtca 16380
   agtaccttat taaaaaactc aagtgtactg ataattatct tacttttaaa tggctaagtg 16440
   ataagactga atttttaggt actgtaacac ttcagattac agattctgat atttttatgg 16500
   ttatttatat ttatttattt ttgagatgga gttttgctct tgctgcctag gctggagtgc 16560
   aatggcacga tctcggctca ctgcaacctc cgcctcccag gttcaagcga ttctcctgcc 16620
   tcagcctcct gagtagctgg gattacagtc acccgccact acagccggct aatttttgtt 16680
   atttttaata gagacaatgt ttcaccatgt tggccagggt ggtctcgcac ttctgacctc 16740
   tggcgatccg cccgcctcgg cctcccaaag tgctgggatt acaggcgtga gccaccgcac 16800
   ctggcctggt tacttaaatt taaatacaaa aattatgttg attaattctg aatgatttcc 16860
   tgattgctcc ccgtttacca ttcacacatt tattaaattc ttcgcttgcc atatagaagc 16920
   agtctctctg ccatatatgc catatagata acagaactag ctgtctgcaa accactgaaa 16980
   ttgtgaaaac atctcccctt ttttcctgtt tctaattcta gctatgagga ttatatacag 17040
   aagtagtcct ggatttgatt tttttttttt tttgatgatt gttttttgat agttgttgac 17100
   tacaaatcat ttaaacgtct gaaaggggaa aggttttcct taaaaatgga tgacaaagga 17160
   gaataaaaag gtattttgac tatttttttg aatgatgagt tttttttttc tctttcttgt 17220
   tttcttttgg agtcatttat gtgtcactga gtggatacca tggaacatgt ggcagaagta 17280
   gatatatggg gtaaaagaac catagttcat aagctccttg acagaatcac tgaagtgtag 17340
   ccgttatatg gccactgtcg cagggggagg cagcagtttt gaagaagggg atgagtaata 17400
   atgagtgata aaaaggcatc ctggatagaa gaccaaactc tgcagaagac cccagtttga 17460
   ttatgctttt gttttctgat ttgcggagga gagtgaaaat gcctgagggg tgcgggggag 17520
   cacatagggt gtatgtgtgt gtgtgtgcgc gtgcagattc tctctttcac tgtatgtatt 17580
   tgtatgcatg tatgtatctt aggacttaag ctttctagtc aataaattgc catagtgggg 17640
   aattgcttaa ttgcttgcct tctgttgttg tatttaattt aattttattt ttaatgattt 17700
   ttttggtggg gtacagggtc ttaactatgt tgtccaggct ggtcttgaac tcctaaactc 17760
   aagtgatcct cccgcctcgg gctcccaaaa tgctgggatt acaggtgtga gccaccatgc 17820
   ccagcttagt tgtattttaa atgggcctgt ttgcagcatt ccctactccc cttagtttac 17880
   ctggctcaca acctgtcttt ccatatcaag gcttctgtca cccctggccc atgtcagtgc 17940
   atttgggcag cccacccagc atcatcacct catgtcccag ggaacttcct gttcctctct 18000
   tccagctatt tccttccctg gcagttgaga tagtctctac ctttgaccta ctgttaagct 18060
   cagaccttct gctctctagt tacagcctct gtgctgccag attccctcgc tcagttgctt 18120
   tctctagttt gggttttctc ctttattcag atttccagct gtttctctcc tccccccacc 18180
   gcagcctcct cacttccctc cttatgcatc tgagactgtg gtcagtcact ttagatgctg 18240
   cctctccact gtacttgtgt ccatcttctt acctaccacc tctagccctg gagcaggctc 18300
   ttcccctgtc tttgtcttcc tgggcccagg ctcctaagcg ctgctggaaa aaaaatcccc 18360
   cagtattgag cccctagaaa tccagtcttt aatcccaaat ctgtctcccc cagcatctgg 18420
   ccatcagatc taaagcttac ctgccatcct ttccacctca tttctctcac aggggaaaag 18480
   gagcctttgc tcctagagtc tgcgctcctg accccttccc atctcacctg ttcaaggcat 18540
   cttgcaataa ggggttggtg actctcgagg aatggatccc aggccctccc tattatcatc 18600
   ttatgtatgc cagttcaacg ttctcagctt cctccagccg agacggcccc tccagccact 18660
   gctttatact ctccttctct ggttgaaatt tttgaagtaa ataggtcact ctgcccatcg 18720
   ttcatcttcc agtcactctg tgtgtttatc ttccagggaa gtgaggctct atgctaccaa 18780
   gccactgaaa taattttttt ttttttccag actgagtctt gctctgtcac ccaggctgga 18840
   gtgcagtgcc gcagtcttgg ctcactgcaa cctctgcctc ccggcttcag gcgattctcc 18900
   tgccccagcc tcctgagtag ctgggattac aggtgcctgt catcacgcct ggctaatttt 18960
   ttgtattttt ggtagagatg gggcttcacc atgttggcca ggcttgttgg catgttgacc 19020
   atgttggcca ggctagcctc aagtgatcca cccgtcagcc tcccaaagtg ctgagattac 19080
   aggtgtgagc caccgcacct ggcctgaaat aattcttgac aagatctgct tccttgttac 19140
   taatacagtg gatattttgc atcctaattt taatgcagtt cagtgtggta gacctgtatt 19200
   tgcatattga atattccctt ccctgtttta ataactctat tttttccttt tcttttatat 19260
   ctcctgcttc tctagctagt cctagacctt actcatcggt gtcttctctg tttgttcctc 19320
   aacttgagga gttcctacag ggtttaccca atctgctgct ttcatttagc ccttttgttc 19380
   tttttgagcc atctcattca ctcacccagg atgtagcatc ggcccttgaa ttcagtgtgc 19440
   acacatacac tgtgcactat gggacagcct tcagaggcac tttgttcctg aaattgtggt 19500
   ggtctttgcc tctcatggag ccttgcatat gctgtttcct ctgcctggaa tatcctacct 19560
   tttacttaac tgattctcgt tcttctttcc agtcacattt tgtacatttc ttctgggaag 19620
   ctttctctga tttccccttt ccacaggtcc aagttaactg ccttgtctag gtcctcccat 19680
   ggccctctga aggcctcctt tcatagcacc atgtctgagt atactgtaat aacacgcatt 19740
   gctctgtaat agcctgttta cttacctatt gccaagtaat ctatcaagtc ttataaaggg 19800
   cggggctgct tttgttctag tcatttgtat ctcttagtac ccaatatagt gtttggcata 19860
   tagaaaatac ccaacaaggc cagtcgcagt ggctcatacc tgtaatccga gcactttggt 19920
   aggctgaggt gggcggatca cttgaggtca ggagtttgag accagcctgg ccaacatggt 19980
   gaaaccctgt ctctactaaa aatacaaaaa ttagccaggc gtggtggcgg gtgcctgtag 20040
   tcccagctac ttgggaggct gaggcaggag aatcacttga actggggagg tggaggttgc 20100
   agtgagctga gatcactcca ctgcactcca gcctgggtga cagagtgaga ctccatctta 20160
   aaaaaaaaaa agactccatc ttaaaaaaaa aaaaaaagaa aaaagaaaga aaatacccaa 20220
   taagtagttc ctgaatgaat agatgagaat gctgtttaga aggttcatga attggaaacc 20280
   gtgattgcta gggaggcttt gagttgatgg tattgtgttg aaccatgtgt tacccaggat 20340
   caatttagat tttacacttt gttttctctg ttccttttta tagtaatttt ctgtatgtgg 20400
   tgttttcccc ccatgagatt gtataccatt tctcagcgag aactgtgtgt aatgcttggt 20460
   ggctccctca tggtgccttg catggaattg gacttcgttt cagtggatct gatcccagtt 20520
   atgttaatgc tcgatggagc taagtcttat ctcgaagcag tccatgtctt catcagctgg 20580
   ccctgcctcc atgccctgca cagaccatgc cactctggag aggtagtttc cctgtggctt 20640
   attagtctta tgttccagtg tgctggccaa gtatgagaga catcagtggt atgagagagt 20700
   ctctctcatt caaacttcgt aggttttgta gctgggactg accagtgctg acaggaaata 20760
   gaggcattta ttaaaagcca gagatttttc aagttgcagg aagcaaagct cttgttagct 20820
   atgattttgt ggtgggtttg gtagtccaat ataaaagtaa aaactggatg acaatgggag 20880
   gagcatgctt gggtctccaa agttagatca tttttcctaa gtaatttgtc tttaaacttt 20940
   tactggtttg gaatttcctg agattttgat cttgccagaa agtttatagc aaaagttctg 21000
   agcagatgac acttttgcgt ctgaaaccaa atcattgttt ttgtttttaa cttttttctt 21060
   aatatattat ccttagttca gccctgaaga ttattctgtt atttgtggat ctcaactttc 21120
   cccccatctc ctggatcttt gtgaaatgaa tggtattaat tgaatagaga aggaagatat 21180
   aaacataaac ttagtcaaaa acttgttctt gactaggcaa gttgggcttt atagctttga 21240
   gctgatgaca tgtctattct tgtgaaaaag ggatttttag tgttggtttg gcttcttgtt 21300
   atatttgatt tattattatt atcattatca ttatttttga gacagagtct tgctctgtcg 21360
   cccaggctgg agtgcagtgg ctcaatctcg gctcagtgca acctccgcct cccaggttca 21420
   agcgattctc gtgcctcagc ctctggagta gctgggatta caggcgggtg ccactacacc 21480
   tggctaatat ttgtattttt agtagagaca ggtttcacca tgttggctag gctggtcttg 21540
   aactcctgac ctcaggtgat ccacctgcct tggcctccca aagtgctggg attacaggcc 21600
   ttagccactg tgcctggctg attttttttt tttttttttt tttaggtttg ttttaactgg 21660
   aactttacgt gaatgtaatt gaatttagaa taaaagcact taatttcaca gtgtgcagtg 21720
   aactttctgt tacttatttt aacagtaaaa ccccttgcag taaatgactt ggagcaaaga 21780
   ttgctttttt aaaaaatgtt ttaatttgtt tttcttttct tgagatggag tcttgctctg 21840
   tcaccaggct ggagtatggt ggcgcgatct tggctcactg cagcctcccc gcctcctagg 21900
   ttcaagcgaa tctcctgcct cagcctcctg agtagctggg actacaggca catgccacca 21960
   tgcccagcta atttttgtat ttttagtaga gacagggttt caccatgttg gtcaggatgg 22020
   tcttgatctc ttgaccccgt gatccaccct cctcggcctc ccaaagtgct gggattacaa 22080
   ctgctgggat tacaagtgct gggattacaa gcgtgagcca ccacgcctgg ccaatttttt 22140
   tttttttttt ctttttgaga cagagtttca ctctgtcacc caggctggag tgcagtgtca 22200
   cagtcaaaac tcactggcag ccttaacctc ctgggctcga atgatcctcc tgcctcagcc 22260
   tcccaagtaa ctgagactac aggcatgtac cactgtgccc agctaattgt ttttttattt 22320
   tttatttttt gtagggacag ggtctcgcta ttttgcccag gctagtctac aactcttggg 22380
   ctcaagcagt cctcctgcct tgacctccca aaatgttggg attacaggga caagccactg 22440
   cacctggcca aggattgttt tttaagtgaa ctgagaccca gccttattag tggtcccaga 22500
   gcagacctgg gacctgaagg gaaccctttt cttctggtcc agcgtctttc ctctgatggg 22560
   ctactttcct ggagcctttg attgcctgtc atcagagtaa ctgagtttga acagagtagg 22620
   tagttcctct ccagaccacc acactcacca gctttcattc tgcttctctc gtttagactg 22680
   tggttctgaa tcctcagttc tatttactga gtgtttttaa acataaaaat gccttttaat 22740
   gagattgaag gccagaggtg ggacagttga ggacaaagta gaaataaaac cttcaaggcg 22800
   gggttgttgg tgggagtctt tttttgtttg tttgtttttt gagactgagt ctcgctctgt 22860
   cacccaggct ggagtgcagt ggcacaatct cagctcactg caacctccgc ctcccgagtt 22920
   caagctattc tcctgcctca gcctccttag tagctgggat ttcaggctcc cgccaccatg 22980
   cccagctaat ttttgtattt ttggtagaaa cggggtttca ccatgttggc caggctggtc 23040
   tcaaactcct gacctcaggt gatctgtctg cctcagcctc ccaaagtgct gggattacag 23100
   gcgtgagcca ctgtgcctgg cagggagtct tatagaagct gtcgtggaca atgtgggaag 23160
   tagtgagcct ttgtattcca gtatgctggg ctccactgtg cttgctctgg cccccggtcg 23220
   ctctctgtgt gttattgagt ccccatccac ggccatactc ttcgtcctgc ttctctcctt 23280
   accatcctct ccccgctagt ggtaccacgg ctaccactag caattactga catgtgggat 23340
   cttagggcta cttccctata aggctgcagg gcatgtggtg ttggctacgc gcatggtaac 23400
   catggtagcc ctgtggttct ccacatgtgc gccttgtgac ctgggattgg ctgcagacta 23460
   gtaataaact gcgtcttctg gtatggaatc tgtctgtagt tgtactttct acctctgtat 23520
   ttaaggggag atctgtaacc taccaatgcc agttgaagag gatggatgat agagatgtta 23580
   acaaacagct gaaaaactaa ctacaatggc ctgcaaaata gaacagcagg tttttgtggc 23640
   aaaactttgt gtccatgagt ttgtttttta aatatcctca tataatctgt tttaaatcga 23700
   gaggctttgg gtaaaagcca tggctagtct tacatgtcat ggagtaccta gcttgtgagg 23760
   ttcacagttt attatttaca gagtgtcccc ttaaatcttc tttgggtcgg ttcagcgaat 23820
   gttgctcaga tggacttttt tggctgacat agagtcaaaa tggtaatcaa gcatgaaagt 23880
   acagacagtc cttaacgcac aaatgtgtca tgcttgaaaa gttggaaagt tggttctctg 23940
   gagctctgat tgtattgtcc tgtagaatcc gtgttgtgaa tggtggttaa atcccaaatg 24000
   agtccgtaga acctatataa tctgcaatat acctgcagta ttccaattaa tatgtaattc 24060
   ccccatagaa ctatgttaat gatttgtatg tatggtattt aatattatac ataataatga 24120
   ttgtatgaat aaaaaacatt ctgggctcca tgtggatgat ggggtgtgtg tgtgtgtctg 24180
   tctatgtgtg ggtgggtgtg tgttcataga tcccttttcc tgcaatcctg gcactggaat 24240
   tggttttatc atttccaatt aagtttcatt cccatgaatt ttggagtaca gactgggtcc 24300
   aggtatgcag ggcatagatt agagccctga gaaataggat taggctggaa ttgctgggtt 24360
   ggagatcagt agcttccagg aacacttttt gggcctggct gtcttcatta tccccttttg 24420
   ttttctcctg gggtctgcag gtattgccct gttttgttcc tctaatatca cttttttttt 24480
   ttttctgctt ttgaccaggg tttttgcctc tggtctacaa ctgaatatcc tatcagactc 24540
   tcctgatttt gaaataaata tatagttttt ttgaggtgtt ctagcgaatt tctaaatcta 24600
   aatgttgtgg cagagttatt acatactaat tttgctatga gaggttgtag aatcccagat 24660
   gactaatctt gtaaaccata cacgcatttc catctaattc tccattgtat atcatgttgc 24720
   agaaaataac agcctctaga gtttacattg cctcctttga ctatatttct tatttaagat 24780
   tagttttcag ataagacctt ttcatggcag tacataactg tacagagggc ttccaacttg 24840
   tcttgggagc tctcatctct gggagacatc acattaccca ctgccccctg ccccccgccc 24900
   ccagcctgga tgcactcagc ctgtacccca tttctgtcct cagccaaaca ctgctgaaat 24960
   gcaagagctt tcaattgcta gccagtgaag atgcagacta agggatttcc atgtagaagc 25020
   ccgctctttt cagctggctc gtcgagagct ggaggcccct tgcttgttca catgaggctt 25080
   tttgtccctg acttggtggc tgctgtttca cttctcagca gaaagggaca cccttgcccc 25140
   cccccagaaa ggaagatttg atgtaccact tccgaaaggt tcagtcgggc atcactgtaa 25200
   ccaagaagat aggtcaggtg aggctggagg tggaacaggg ctgctcgcta gaactccaga 25260
   ttgttccaca agtgccttct ggcagagaat gatggaagct tccgtgattt ttttttctcc 25320
   ttaatagtta tgagcacaga agaggagcag attgtctggc tatagaagct gtcttatttt 25380
   ttatttttgt ttttgagatg gagtctttct ctcttgccca ggctaaagtg caatggcgcg 25440
   atctcggctc actgcaacct ccgcctcccg agttcaagcg attctcctgc ctcagcctcc 25500
   tgagtagctg ggaattacag gcatgcgcca ccatgccaga ctgatttttg tattagagac 25560
   agggtttcac catgttggtc agtctggttt cgaactcctg acctcaagat ctgcccacct 25620
   cagcctccca aagtgttggg attacaggtg ttagccactg cacccggccg aagctgtcat 25680
   attaaatagc actttctgct tttagcaaat ttaatccaaa tgagacttta gattttcttg 25740
   ctctgactta ccagcagttc cttgaaacac atttaattat ttttgccaga aaatcactca 25800
   agcacttacg ccattttttt accgtgaaaa tatgctgcat tattttaaaa tatattagaa 25860
   gtcagtaacc ataagatttt atatgttttc taatgtattc tgtaagcttt ctgctgcttt 25920
   tgtttggaag gtgtattttg taacgtagag gactgcttta tctgcttgta agcttgattt 25980
   ttgtttttac tgtaattttt ttttcttttg ctgtattgag aaatacattg agtaattata 26040
   aagtcagtgg catgtttata agttaatatt tgtatctatt ccttagttac tctaactcaa 26100
   aacctaaagt aatcttcaac tctaatttac tctgacatcc agttgactgc caagtcctcc 26160
   aacttaatcc ttatcctttt ttttttaaag agatgcagtc ttgctttgtc acccaggctg 26220
   gagtgcagtg gtgcaatcat agcttactgt aacctcaaat tcctgggctc aaatgatcct 26280
   cccacgtcag cctctggagt agctggggct acaggctctt gctaccatgc ccagctaact 26340
   ttttattttt attttttata gagacagagt ctcactgttg ctcaggctgg ccttgaactc 26400
   ctgccttcag gcggaactcc tgccttcagg cggtcctcct gcattggcct cccaaagtgc 26460
   tggaattaca ggcccaattt tattcttggg atgtatgtct gaaactcttt ccttcacttc 26520
   cttcccaagc cttagttcag gcccttctca tctgtggtct tcaaagtcgc cttcagctgg 26580
   ttcaggtcct tcctttctgc tgtatctttc atgggaggac atgttatgta tcactgtcct 26640
   acttgaaaac ttccattccc cattgatgag ggtgttacct ccagattcct aacacaggtg 26700
   ctgaaggcat gcctggataa aggcactccc ttgatctcct ggccaggtcc ccgtacacct 26760
   gcagcgcatg ctccacattc tgtctttact gatgctgtgt cttctgcctg cggagccacc 26820
   caccattcta ttcacagccc ctgcctcagc ggagcacgtg cctccctctt cctacactga 26880
   gctgtccttt ctattgaatc ccctcttttt tgtagtatgg gaaatatttt attatgaata 26940
   ctcttttctc tgttgcctcc gtgaccacgt taactttgcc ctaattcgcc ttaggactcc 27000
   atctgcttag gggaaagtta ggatttggtt acagaaagca agctgctaga aagaacagtg 27060
   tttagcttct gacaggcaaa ataggatttt gcaacatgct tttccttttt aatgcttaga 27120
   cattttatat gaattaatat ttttatttgg ttgcttatac attactttct ttttagctag 27180
   aatgtgaacc ctataggaac atggggattg cctttcacat ctttgtatcc tcagtaccta 27240
   atgttcagtc accctgtggt cttgtgtcgt atatacattt agccttcctt aattaaacca 27300
   tatgtactgg tccccgtccc ccacccccaa atagagagaa agaaattcct tgaatactac 27360
   attgccagta tcaaaccaca ccttgatatc ctctggggaa agggaggtat cagttgaaaa 27420
   gagaaaagag gttaaaatct aggcattaaa atgtgtaagg cttagatgct ggcaatttaa 27480
   ggtatgtttt cctgaggtta attttgattg tgtgcaaatt ttacctcata tctaactgta 27540
   ggatttagtc accacataag atgggatacc tccataaatc cttcagaaat gtttgtgaaa 27600
   ttaaataaag ccttattgaa gactcagctc ttgagagtca tctacctacc taacagttat 27660
   tcttgaacag aagagtctta cttttcccta taaggcagtg tgatagccat ctgtatattc 27720
   atataattta tgttggcgct tacttcattt aaaaatgtat tccgtgaatg cagttgccag 27780
   gcggtgtgct gatcagaaac gtgtaccaat ggcctctttt ataattataa gaggaagacc 27840
   aacctgaaac agtcacacaa atgattaatt ttaattgtgg aggagtgctg ggaaagaaaa 27900
   ataaaagatg caatgcaagt gtttacaaag gagctttgag cttgtttgaa gtggtccttg 27960
   ggcacttaag caaggcttaa agaatgatgt gattagaagt ggcttagcaa ttctaaagaa 28020
   cacagggaag gcgtgtggcc agaacattgg tccctagagc acatcgcctc ctgacatacc 28080
   atttccttaa gttaatgttt taccactata cataggccct cccctttgtt tacccagatt 28140
   tttttaattt taaggatgtt tttaataact tagaatcctg taatttgttg aacagtcctg 28200
   tattcccttt acttatattc cttgagattt tataaaatat tttttacatg tcccaagtct 28260
   tgattatatc tttttacctc ttgttaagaa atacttactt ttctattttt atgctatatt 28320
   tcatgtttac tgtagaaaac aaaaaaagta aaatttttct ttattcctat cactgcagct 28380
   tataagcact ctaaacattt tgatctatat tttgccaatc atatatttta gttaaaattg 28440
   ttgttgacat aattgtagat tcctgtgcag ttgaaagaaa taatacagag ctgagcgcgg 28500
   tggctcacgc ctgtaatccc agcactttgg gaggccgagg caggcagatc atgaggtcag 28560
   gagtttgaga ccagactggc caacatggcg aaaccctgtc tctactaaaa atacaaaaat 28620
   tagctgggtg tggtggcggg cacctgtaat cccagctagt tgggaggctg aggcaggaga 28680
   atcgtttgaa ctccggaggc agaggttgca gtgagccgag atggtaccat tccactccag 28740
   cctgggcaac aagagcaaga ctgcatctca aaaataataa taataataat aaataaactt 28800
   taaaaataaa acagagagat cccatgtgcg ctttgcctag ttcccccatc cactgcccat 28860
   aacattttgc agaactgcag tacagtatca caaccacaat actgacattg atacagtctg 28920
   ctcatcttat tcatatttcc ccagtgttac tcgtatccac gtgtgtatgc attgtgtttt 28980
   caatactctt ttattataaa gctgttttta atgtgattca attctaggtt gttttgttct 29040
   gccctcaaaa agcattccct ctcctaatca tatctccgtc atacccttgt atgttttctt 29100
   taaacctgtt ttaagaaagc agctacctgt aagagaaatg agattgaaaa cagaattgcc 29160
   aatctgcttg tactttataa gcctgttgat tgtttagata cggtttagcc agtttatagt 29220
   taccctgggt gctgaaaggt atgctggatg atacctaacc aacagagaac cattgaatgc 29280
   cgttcaaaat ggactgaagc atcagcaatg tctgaaaaag gcctgacagt aatgtacatg 29340
   tcaaatggcc cgtaatttaa gcagagtaga gtaagtagaa gaataaacat ggggaaagtt 29400
   ccagcaacag aggaggcttt gagcttttgc tcttcatctt gagtggatgt tgttctcagg 29460
   tggtaatagg ccatcgagct ttctccactg gctgcctctc tggggaacaa ataaccgaaa 29520
   agatactcag caccctggtt ggtacatagg tggtcagttg atttatactt cctggttttc 29580
   agtgttgctt gaattttcta aatggaaaca cagtaccttt ataatcagaa aacaatcccg 29640
   agttttgatt tgagggtgtt gtaaaaagtt aaaaaaaaaa aaacagaaat gtgaaaagga 29700
   agttgtgtta gagtatttgg agttgagaaa gcatgaaaag gacagaagag aagctggttg 29760
   tcaggttgca tggggtagct acaagcacac tgaccagaaa gtcagctgga aaaaaaatgt 29820
   agaaacagga gataaaacgg ccaaggggct atacaagcaa acagcaagga cctgagaaga 29880
   aaaactagtt aggtgtgact gtcagagtga tgtgtacagt gtgatccttt ctgtgtaaaa 29940
   acaagcagta agaattcgct gtttacgttt gcgtgtgttt ggagaagagt ggggaagagt 30000
   aggcactgcc agactgtgaa cactggttag gttattgtta tatctttgta ttatatacac 30060
   tggacatgtt atttgtataa tatgagaaga aattttataa atcattaaat cttttggcat 30120
   ttaggaacat ttgtgttttc taatagttgc ttctatacta ttatctttat tatatgccct 30180
   tcatcttctc agtgtttggc tgttgttgtg attccctttt gtgagcagtg ttgaagttag 30240
   ctaatattca tttcttctcc cttctttcac cctcctccag agtctgattt gaagtattcc 30300
   tagctgctac ctataaaagc aataagcaag attgttttac ttttcacaaa ctcgtcctgt 30360
   tctgtgcctc tgcctcggac atagctgtag tatagagtgt tgtctccctt acatccttct 30420
   atcttagacc tactagtaaa tattaatgct cactctaagt tcttctcaat tctttttttt 30480
   tttttttttt ttttttttga gaaagagttt cgctcttgtt gcccaggctg gagtgcaacg 30540
   gcacgatttc ggctcaccgc aacctccacc ttctgggttt aagcgactct cctgcctcag 30600
   cctcctgagt agctgggatt acagtcacgt gccaccaccc ctggcaaatt ttgtattttt 30660
   agtagagaca aggtttcttc catgttggcc aggctggtct caaactcccg acctcaggtg 30720
   atccacctgc ctcagccttc caaagtgctg ggattccagg cgtgagccac cgcgcccagc 30780
   ctcttctctc aattcttcct gaagctcttt ctgcactaga ttcctcagga agggcttgtg 30840
   ggaacaatct tctgtgaatc aacagtacat attcataata gtttgtcagc agcctattat 30900
   tttaaggcca tttggtctgt atataaaaat gtttggatca cattttcttt ctttaaggta 30960
   aatatgttat tctgttgtct tctggtataa agcattgctg taaatgtttg acagtctaat 31020
   tatcttttgc ttataagtga cttagggttt tttgtctatg tgcccaaagg attttttccc 31080
   tctttctctc tttttttttt tttttttttt tttttaaaca gacaggatct caccctgttg 31140
   cccaggcttt agtgcagtga ggcagtcaga gcttactgaa gttttgaact cctgggcttg 31200
   aggaacaaag gattttttta accttttaat tcaaagtctc atcatttatg caaccatgtc 31260
   ttggtgttgg ctgttttggg ttgttctccc tcaaaaatcc atgtgctctt tcaatatgta 31320
   gttttaaatc tttttttttt aatttcagga aaatcttgaa ttagagtttt ccgtttttcg 31380
   tctggtacat tgcttgggtt tccttcttca ggaactcagc ctgttatgtg tatgtttgat 31440
   cttctttgcc tgtcgtctgt ttctttcact tcctctcact tttttaaact tcatttatta 31500
   aaaaaaaatt tttttttcga gacagagttt cgctcttgtt gcccaggctg gagtgcaatg 31560
   gcgtgatctc ggctcactgc aacctccgcc tcccaggttc aagtgattct cctgcctcag 31620
   tctcccaagt agctgggatt acaggcatgc gccaccacgc ccagctaatt ttttgtattt 31680
   ttagtagaga cagggtttct ccatgttggt caggctggtc ttgaactcct gacctcgtga 31740
   tctgcccgcc tcagcctccc aaagtgctgg gattacaggc gtgagccact gtgcccagcc 31800
   ttattaaaaa ttttaaaaac atacatttaa acttaacaga aaaattatga gagagaaggg 31860
   ggtggtgcca ggctttttta aacaaccagc tcttacatga actcatagag tgataactca 31920
   ttaccatgag gacggcatca agccgttcat gaaggatctg gccccgtgac ccagacacct 31980
   cctactaggt ccatttttaa cattggggat cacatttcaa cgtgagattt ggagggggca 32040
   aaactacaaa ccatgtcact cagggattgg aggagcaagt accacctata ctttggactc 32100
   aggtagaaag gcaaaatatc caggaaataa gctgctaccg tccagggttc agcagaggtg 32160
   cccatcagcc tgccaagtac tcaagagtcc agcctctagg gagctaatca tcatggtgag 32220
   ctcttcgagg cacagggagc tgggaagaca gtgcttgcca cccctgcctg aatagtgttt 32280
   gcacagagag ttctgttgtg tcttgattgg gtcctcctgc cactgggaat gctgtggatt 32340
   atactaggtc tctatctggc ttgtttcagg gctccatgtg aaaaccttct tgatatccta 32400
   gccatccacc tgctcagtcc ctagtttgca aggaggctgt ggggagccta gattctgtgt 32460
   cagatagaat gtactacatt ccgtctcagg aatgtaccac atcagaaaac agtgcgacct 32520
   gcaggagaag tagaggtgaa gaggcacatt cttccgagaa atgtttctct caacacccag 32580
   cattccctgg atatcagcag gaaattactc actgctagaa aatgccccat gagccttctg 32640
   ttaaggaggt caagggagag aacagagaaa gttctcaaag ttgacttggt cactggtact 32700
   ttcttatgcg gttcttattt tgtttgccat cgtcatcatc atgctatgtc tattttctca 32760
   atccaaatcc actgctttca ccttggttct ttctgaccgg tttggcacac tcattcagta 32820
   aatccttatg gagagcccaa tgtctgcata attgtgctgt gctgatgacc aagctagacc 32880
   tacgagtgtc ggctcctttg agatgtacgg gacagctctt ctgtcatctc ttctgggaag 32940
   cctctccagg cttggtgaac agtggcaaga tgtttaacag ttgtacatgt gtcccatgtt 33000
   cctttctaag agcctgggca aaccagaccc ggtcgcaggt catcgtagta tggcgtgagc 33060
   ttcctctctc ctttctgacc ttttgtgtga tggcaagaac ctgcagagtg acacaagcag 33120
   caggcttctg aggttgctct agcctcagaa tggccgtccc ttctccaccc tggccctcat 33180
   tgctgaggtt tcctttgaag caacagtgcc ggaacagact aggggaagca gcttggacat 33240
   agctgtatga tttattacca cccattgagg ccaaccaaag tcggcaagga gaggtagcag 33300
   gtcagtggtg cctggaagct tcctctttcc tttgcaccag atgtgactgc tctgcaatta 33360
   ctcctaaatt tgctactctc gtttttacta gccaaccttg atgtttttcc cttcttcctg 33420
   tagaatagac ttcccctctg atcagtactt tctactcaac actatttgtg gccacagtgg 33480
   gaactcattg aggacaggga ccatgacatt actacctgac ccatcaacac ttggcataac 33540
   ttgaaatgca aggacaaaaa ttggctgcaa gtacaatgtg gtcttcactc tgaaggtgat 33600
   ccttaaaact tggctttggc atcatattgc cttaatatac ctaggggatt gggtaaaacc 33660
   agttacttta aaagagtttt acaattctgg ccttctagct atcttgtctt cttaaacaag 33720
   agcacaagat gaatgtatct tagtgaaatt ttatatggtt tgctttgagt aatcttgcga 33780
   agattgattt ttagcacagt aggaaagaca cattctaata gtgatttttt tccccgagtt 33840
   tatgtactgc tgttgcatga aaatctgact agatttaatg ttcctaaagt tctttgttca 33900
   tcctgatttt tgcaggtcct agggaaagct ttgttttcct cttaacctaa cttagatgtt 33960
   gtcatttcat gagctttgga ggaagagtgt atagccaatt gtgtaatgtc tttaaaggat 34020
   attatctctg caatagttgt ttataaggcc taagttattc atgtaataat agtggccccg 34080
   gatctgtttc tagcaatagg tatatggatt ttggttccta tatagttgta gttgtggctt 34140
   tgagatattg agcaagccct tttaagaaag gatttggcat ccctcagcct tcaaaagctt 34200
   ctcaaaattg atcatatgtt attagcaaag gtttactgcc tgcttccatt gtatagacaa 34260
   tttatttttt atgtattccg ttctaagaag gcagatgacc aaaagatctt gcatctgttg 34320
   cccaaggctt gtgactagag aggaaagaga taagaatact tttttaaaat cccattttac 34380
   taaatatgtt gaggaagtgg taagatatat taatttgttg agatttttct gttatgccta 34440
   ttatatgaaa taggtactct gaacatggct tcttaattaa atatatttga taaaatacaa 34500
   cttgcttccc ctggagttta gaagtcagat aactgccatg gagagctatg ctttctttgt 34560
   tttaaagatc tgcttatgaa catgataaac aggaacaatt taatgttttc aatattttct 34620
   tgtattttac tgcaagttta tacacaacat aaatatgggg gaagggggaa atgtttatac 34680
   cagagccatc ctgcccattc tttccttaca gaaggacaaa ggagcagtat ttattttaac 34740
   tacaaaaata ctattgtagg ttttaaaaat tccgtatatt ttgatatctt gtgttcctct 34800
   tgacctttaa tttgctaaat agttgcaaag aatgaaggta acctgcatca tcttcttaaa 34860
   aaccaactct atctaattat aatagtttgt ctatctctga aaaatagtga tgtgttcatt 34920
   ctgaaatcag aactaccgga tgcagctgca ttttgttact atttgaattt cgggagaggg 34980
   aggaggatgc agcctttcga gctgctgaaa tacacaaaca caaagaagac accaagcata 35040
   gtagaactgt gttaagctga ccaagccaga agaagcacct attctcagca tagtatgaga 35100
   cgtaaaggca atataatggg catagttgaa gatggtagaa ggaaaataga ctctgatggt 35160
   ttaatgttaa atgctttttt taaaaaagtg gtattccaat atcgaagaag aagactttct 35220
   acttttagaa gcaataaagg aaattgcaga ggaaagggtc aataggttgg aatacataaa 35280
   aattaaaaac ttttaaactt tttttttttg agacagagtc tcactctgtc acccaggctg 35340
   gagtgcaatg gtgcaatctc ggctcgctac aacctccgct tcctgagttc aagcaattct 35400
   cctgcctcag cctcccgagt agctgggatt acaggcatgg gccaccactc ctggctaata 35460
   tttgtatttt tagtagagac agggtttcac catgttgtcc aggctgatct caaactcctg 35520
   acctcgtgat ccgcctgcct cggcctccca aagtgctggg attacaggca tgagccaccg 35580
   cgcctggact aaattgtttc agtattaatt ttttttaaaa caagatctta ctgttgccca 35640
   ggctgaagta cagtggccca atcatggcta actgcagcct tgacttctgg gcctcaaggg 35700
   atcctcccac ctcagcgtcc cgagtagctg ggaccacaga catgtaccac cacacccagc 35760
   tacttgtttt atttttattt ttgtagagat gaggtttcac catgttgccc aggctggtct 35820
   cgaactcctg ggcccaagca atcctcctcc cttggcctcc caaagtgctg gtattacagg 35880
   tgtaagccat tgcgccctgc ctgatttttt aaatgtgcaa acagataagt tggaaaagtg 35940
   atttccaata aagataaaga gttgatggtt ttaaaatacg taaagagctt atatgaatga 36000
   gaaaaacact aacattccaa aagattagaa ggcaaaggac agaaagaaac aaatcactat 36060
   gtctgggaag ggacatgaag gagcaggttc ccactgggcc agcggggctc aaacccactg 36120
   gggacgtccg agagactgca agggccatgc cttcacattg ccgtacctga gaagcaagga 36180
   gctggggtat ttatctcttt cacactttgg gaggctgagg tgggcggatc acctgaggtc 36240
   aggagttcga gactagcctg gccaacacag tgaaaccccg tctctactaa aactagaaat 36300
   aattagctgg gtgtggtggc acacacctgt aatcccagct acttggaagg ctgaggcatg 36360
   agaattgctt gagcccagga ggtagaggct gcagtgagca taaattgcac cactgcactc 36420
   cagcctgggt gaaactctgt ctcaaaaagt aataataatc atgataaata aaataacatt 36480
   agattgttag cagaagtagc cacaggtttc tcccacctct ctgcaagttg ctgagtgtga 36540
   ttcccatcaa gaggtacaat gtctttttat ttttatttta tttattttat ttatattgcc 36600
   tatgttgtct aggctggttc caaactcctg agctcaagtg atccttctac gtcagccccc 36660
   caaagtgttg ggattacagg catcagccac tgcacctggc ccagatactt tttcttgagt 36720
   aggaatttcg agtcaccctg aacattgcat gccttcgtag tggggaagac aataggaaac 36780
   cacaggctgt aggctaaaat gggttgtgtt tcttgtaacg tcatgacaag gcataaccca 36840
   tcttggcata gtaaatagta agcactcact gaactgatga ttttaaatct ttgctgttta 36900
   ttcagcaata tcctaaatta gcgctatgtt agtggagttg catctccctc atggattagt 36960
   ctgaaaaaga tgagaaatct gtatgtagac caagttatcc ttaaactgct cataatgtat 37020
   gatgcacgtg gttttacgtg tacagcctgg taccattgtt cttaggcaca tttcagtgcc 37080
   agaactctta atacccagga agaagcaaaa agaaagatgg aggtgcagct agaggttgtg 37140
   gcctttgaac gattcattct gccttaataa gagtggtctg gctgagctcg gtggctcaca 37200
   cctgtaatcc cagcactttg ggaggccaag gcaggcagat cgcttgagcc caggagttca 37260
   agaccagccc aggcagcata gcgagacccc ccctcccccc gtctctacaa aaaaatagaa 37320
   acaatgagcc aggcatggtg gaacgtagtg cgtggtgcct gtagtctcag ctacccagtt 37380
   ggctgaggtg ggaggatcac ctgagcccta gaagtcgagg cttcagtgag cccttattgt 37440
   gccactgcac tccactctag gtgacagagc gagacaggtc ctgtctcgaa aagaaagaag 37500
   aagaattaaa aaaagtgatt agatcccttg tgtttgggac acttgttggc agcagggatg 37560
   gtagcgttta tgagggttgc atgtaacatc gcctagctca gacatctgtt tgactgtctt 37620
   cccccctgaa gcgcaggctc tgtgagggca ggtcttttgt ctttcttgtt aatcttcata 37680
   tgcttagtgc ttgccacata gttgatgctc agtcgatatt tggatgaatt gaagggatta 37740
   atgcattgaa tctgaacctt gctttcttaa tgcatatggg gagttctttg gaaagccaca 37800
   cagaggagct tggttgcctg cttcctctct tccccagatt gtctttttat tgttgtggct 37860
   tcactgaagc actctcactt caaataattt tgggcattgg tcgtatttta ttctttgttc 37920
   cttcttcatc cttacccctc agatggtatg tagaaaagta cactacatct agaaagtact 37980
   ttataaactc atttggttga taataataca tatgcctttt ccttggtcct ggtagcagaa 38040
   tcttgtgcca ctcttggaat acaaacgaaa ttcttaacca aagccagttt cattttgatg 38100
   ttctattttc ctcccattca cactccaaat tgtgcaccaa agtatcatcc tagttttgtg 38160
   aggatggttc tccatacttc agggtaggag tatcatgtgg attcctatga tacctttctc 38220
   cctgggacca tggagggcag cagctggtga ttgatagtct gattcccggt gaggaaagct 38280
   gtgagccttc cacttgcaga tgtctgccaa ctacatgtgt ccttagtcaa ctgtaccact 38340
   gtcctccggc aaacagcaga agcccagggc ctgaagttct taagctgtca ttatggaaag 38400
   cagaaggtaa acaaaacaga agtgaaagta gatttaattt tttagactgt tctcttacag 38460
   gaatggtttt gtggttctca gcattttaaa aaaaatagtg gttccaatat gttttattga 38520
   catcaattac tgtaagtctg attcattttc tgcctattga tttctaccca aggtgaaatt 38580
   catgacattt aacagaaagc ataagtgatt ttttaaaagc agacactatt agggacggta 38640
   aaaataagat ttaaagtcgg gacacttgaa aaagcaattt ttataccttt ggtaacgatt 38700
   ctattctgat tctttgtata aataatataa acaaaggctc tagaagctta ctataatgaa 38760
   gttggtgtgc tgtttctaaa ttctggttta aggcccaaat tcattttatc tgcattaact 38820
   tttttttttt tgagagtctc gctctgtcac ccaggctaga gtgcaatggt atgatctcgg 38880
   ctcactgcaa cctctgcctc ccgggttcaa gcgattctcc tgcctcagcc tcccgagtag 38940
   ctgggattat aggtgtgcgc caccacgccc ggctaatttt tgtattttta gtagagacgg 39000
   ggtttcacta tgctggtcag gctggtctca aactcctgac cttgtgatcc gcctgcctcg 39060
   gcctcccaaa gtgctgggat tacaggcgtg agccactgca cccggccgtg ttaaaatttt 39120
   tcagtggtag accactatgt caatatgttg ctttcactga caacagtatt ttcttaaaga 39180
   taggataccc catttctaga tgaatctcat tctagctgga aaataatttt tcagttctga 39240
   aactacatca ggcctcaggg aatcaaaact agctattagc cacacacata taaagtggct 39300
   ttgctttata aacgatttag ggtcaccatc aatgacaatg gtcccttttt attgtatttt 39360
   taagagtttc ttatcttaaa tggctgcata actgtagagt tttaaaaaaa ttaagtaaat 39420
   gaccatgtta atgctctatt aagcttccaa acaatattgt aatttacttt gaagattttt 39480
   ttttattctc aacatcctgc agcttgaccg tttgcctccg tgtctcagtg ctgcttattt 39540
   tgaggtgtgg actggagtcc atctgtcccc cttgcctctg aactgctccg ttttgtgttt 39600
   cgtaattctt catgctgcat cctgggcgca tttctctgta gtagctttca atttgctcat 39660
   gctttgactg ggcttagtct agcgtttatc ctatctctta aggtttttta aaaaattttc 39720
   atgattattc atttatttcc aggatttctc atttcttcag tcacatctcc ttgttctggt 39780
   tttacttctt cctgttttta ttcataacat cttttttata cacgattcct tcatgtattt 39840
   ctaatcttaa gtatatttaa ttgcttattt gattcttttt tttttttatt gagacagggt 39900
   cttactctgc caccaggccg gagtgcagtg acatagtcat agctcactgc agcctcaact 39960
   acttggactc aagcgacctt cccacctcag cctcccaggt agctaggaat acaggtgtga 40020
   gagccgccac acccagctga tttgtcttac tatgttgccc aggctggtct tgaattcctg 40080
   ggctcatgtg atctgccctt cttggcctcc tgaagtgctg agattatagg tgtgaaccac 40140
   tgcacctggc caagtatgtt tatttattta ttctaatttg agagggagtc tcgctctgtc 40200
   gtgcccaggc tgtagtgcag tggcacaatc ccagctcact gcaacctctg cctcctgggt 40260
   tcatgcgatt ctcttgcctc agcctcctga gtacctgggg ttacagttgc gtgccaccac 40320
   acctagctaa tttttgtgtt tttagtacag gcggggtttt accctgttgg ccaggctggt 40380
   cttgaacttg tgacctgaag tgatccgccc gccttggcct cccaaagtgc tgggattaca 40440
   ggcatgagcc accacgcttg gcccaagtat gtttattttt aaagtcccca acaagctata 40500
   caataaattg catatggaat ggatttttgt tctagttgat ttgttggtta tcatttgtag 40560
   aactaactag ttgtcttctg tgtttgatac cttgcttcta ggtcattttg agttgggagc 40620
   cttttgtttt gtttttattc tcatgctgtt tttgagccta gctgtgcctt tatggttttc 40680
   tctaaattta attgaccatt gttttatatt tggagcagtg ggtgtacatc agagtgtgaa 40740
   agcagcccca ccctctccac cagaaggtct ccatgccagt ttcacgaagc atttttcatg 40800
   ccctcattcc tgcccttatc ccttgatttg tggggagttt gtaaagcagt tgattgtttt 40860
   ttttccacgt agttttccaa gtgcacataa ttgttctgtt agtgacttgt agctccatta 40920
   tctattaacc ttgccccaga ccactgtaca agcggaccca acgcttcctc cagctgtggc 40980
   agggacagtt acttggtatc ctgctgcctt ttcaatgctg accagttttg ccccttcctc 41040
   ccctcaaccc ctgtctttca ttcaactatc accaaaccaa aagattctgg tttgcttttt 41100
   agtatgtgtt cttattcagt acatagtcat tttaaaattt aaaccaaaac agacttggta 41160
   ctgattagct taattttaag ctttttcttt attattaaac agtgtagttt atcttagcat 41220
   ttcatattaa gtatatgatt tatttcatat tgcttatatg aatgtacaca taaatataat 41280
   aaaaatattt tcctaaggtt tttgtagtaa attatatcgt ttcattaact ttcatatata 41340
   gcattgcttt tgacctggaa gacattgaac ctctgatgat ttgtatattc ctcggagtat 41400
   actttgttac atagaaattt tctcatttat aatgagattt gtgattaaca aaatttgttc 41460
   aacatgcatt actttgaaga tctggtttct aaaattttat gctagttacc ccaccccccc 41520
   ttctatatat atctccctat tcagcgacta ctgcaagagt tccaggaaat gtacactgtg 41580
   tgttcactta ctgcatttta aatcattgcc tttactatat ttctgcattt cccttcaatc 41640
   tagctctgtc tgtacatttc tgaaagccag tagcttccct gaagaaccag gtaacaaccc 41700
   gaacaatcaa attagataac catttgtaga atggaggttc cgggagatct tagaagatgt 41760
   gatgggtgct aagggacttt gtagttccct gaagttccag tgagtaaaag gtacccttgg 41820
   aattttttat tccttcagac ttttaaaaca gagatcactt tcaaaaatta ctctttctgc 41880
   tttgaatcca tgttttagta actattttga cactgtttgg tcagaaggct gtgtgggtca 41940
   actgcaaata aataaaataa atgtgatttc agtaatttcc attttgtaac aagtaattga 42000
   gaaaatagga ttggatcaga tatttgctta tacacattcc ctttcaggag cacttctgtt 42060
   ctataaagaa tgttggtata ttgttaagga cacttcaagc tttgggaacc tttgaagtat 42120
   ccattgattc agttaacaaa attatgttga gtgcctaccc tgggcctggg cctgtgttag 42180
   gaggggacac taagatgaga gtccaaagca cttcttctca gactcctggc tgctaatggg 42240
   ttgctgcctc tacttcttca cttagcagat agctttaaaa tgagtaatgc attttaccat 42300
   ggagcccgta agagacattc acccagttgt ggaccgagga gaagggtgtt aaacccagat 42360
   tgtgatgttt cacttgatga agtgcttaat ataaacatgg aaatatttcc gcaaggataa 42420
   actggctttt atgcctgtgt gttttcagga gaaatagaaa tctctaatca aatattgcca 42480
   gcttttcacc caagtttgac tttttgccta attgagtttg ggaggtgtct gaataatgga 42540
   taatgagctt tcctgaataa atataaaaat taattaactc caggctctaa ttcattctgt 42600
   taccagagtt ttgtaagcat gttacccctt tgtgttcatt gggagatcat ctgttacctt 42660
   cttaaatgag tggggaagga tgggaaatga ggaagagcta taaaaactat tcaggtgaag 42720
   aaggtttctg cccctccttg ccccttttaa aatctccagc tcagcagatg ctttgtttaa 42780
   acttgatcaa gtgcttgtga atcttcctag cctagctaaa tcataacttt ggaaggactt 42840
   gcttttttct ctcatgacaa tggtttacca cagaaatgat tcagatcact ttgtgtgcct 42900
   gatgcctatg taaaatgata cagtgaaatg gaaaccattt acctgtaagc tttgggcaca 42960
   cccaagcctg cttcaggagc acatgatcag gcgtgcactc tgggagagcc gtacacattt 43020
   gacatctatg atgtgtggcg ttttattcta tcacatttct gaaatctaca ctaagagaaa 43080
   ggaggctctt aaaaaaccac tgaggtgtgg actgggggaa ggagagatcc gtaaagaacc 43140
   tgtttgttac ctgttgatac tatttcccat tggtaaaatt tctaatttag tgtgatccag 43200
   ccctgaaatg ctgaggcaca cactgaatga ctcctgacat ctttagtgtt tttgttcagg 43260
   ggactcttct gggaatctgt ttcatggcaa gtttattatt cccttttggt ttggctcatc 43320
   agtttaccca gcagtcatct taatcggttt taaaggcttt tattttattt tgttttctct 43380
   gtggaaattt tacacattca gtagattaga agtagttatt taatctttgg ttagcataat 43440
   aaaagatctt ctagggacat tttttgcttg cagtggaagg ctagttaaat gtgttcatta 43500
   gtcatgaatc tgctttttct atagctgttg gaaacgtagc tcccctgtga tacagttgta 43560
   gaatacagaa atctcgtttt gctgttacgg tacggtagtc tacttacttt cttccaaacc 43620
   attaatgtta tagttacctt taattgcgta ggtcctatca cccctcaatt ttaagactct 43680
   aagcctggca ttttatctta caaaatgaaa tataaagact tgtactcaga gtatgtgtgt 43740
   gttttccata taccattcta aagtagagaa agatgaggga ttcgccagaa actgatttct 43800
   aataaattat ccagaaactg accccttctc acctcttctg ttactgtcac tgtggtttca 43860
   gccacagcat cctttgctgc attgttacct tagtttcctg actgtatcct tccttacacc 43920
   attgatccct gcaatcccat ctgcgcgtag cagccagaag ggatccactt actgctgtga 43980
   tcagaaatcc tcagccaggt gcagtggctc atgcctgtaa tctcagcact atgggaggct 44040
   gagactggag aattatttga gcccaggagt ttgagaccag cctcaaactg ggtaatataa 44100
   tgagacctca tctctacaaa caggaaaaaa aaaatttttt tttttttttt aactagccag 44160
   gtatagtgct aatatacctg ttctgggatc cagcatgctc tccctgacct gcagcttcat 44220
   ctccaccact ttgcccctca ctcccaccac aatggctttc ttctcttcct cagacatgcc 44280
   gtgcgtcctc ctacctggaa tattcccctc caaacattcc catggctcac tccctcacct 44340
   tcatcagatc tctgttccag tgtcactttt actggaaggt cttttgtgac catcctactt 44400
   attataaaaa aataatctgc ccaaccttct ccttttattt cctctacttg atttttcaat 44460
   ttagtactta tcagctgaca tatattttgt ctctctgtct ctctctgtct ctcatagaag 44520
   gtaaattcta taaaggaagg aatttttatg tttggttctt tgctgtagct ccaatattca 44580
   aaacagtgcc tgacacacag taggcccttt atatttgttg aataaatgtt gacactctga 44640
   tatctaattt ttgtctggtg actaatacga aaactataga gtgataataa aagcattacc 44700
   ttagtagact ggaaagggat gagcgctagg atgaactttc tgcctggcga tcttgctgaa 44760
   tttaggaggc agattggggt tcaaaggagg ctgaaatggc taggatttgc agagcagggt 44820
   actaaggatg agcaggctat gacagaaaga actccagaaa tctgcaaagg gatcaccttg 44880
   agtctggctg gatacagtgt acactttgta gggtgtctct tcatgagctt ggataaagaa 44940
   caactgttgg ggagtggata attcccagca ctcattcaag cttgcatcgg ccagaacgga 45000
   gagagacaga cctctgtaat acgtaggata tttggtagaa acattcaacc gaaaaccatc 45060
   agatatgcaa aaagtaataa taataagtaa acaatgtgat gcatagctag aagaaaaatc 45120
   agacattaga agcaagccca gaaatgacag atgataaatt agcagataag gacattaaaa 45180
   cagctattat aaataactta gcagatttaa agaaaaacaa cataatgagg ataatggaag 45240
   aaaaacaacc gaataccatt tctaaagaag aaaaatacaa tatctgaaat gagaatttag 45300
   ctggatagga ttaatagttt aggcactgca gaagaaaaaa acagcatcta tatgagaata 45360
   tacccaaggg aagtacagag aggaaaaaaa tgtggattgg ggggtgcctc agtgacatat 45420
   ggaacaatat taaacaagtc tgcccccaaa atacttgaag gaataaggtt caagtttttt 45480
   ccaggtttaa tgaaaactat aagcctacag attcaagcat ttcaacaaac cttcagcaaa 45540
   ataaacaaaa ccacagtagg cctggcacac tgtctcatgc ctgcaatccc agcactttgg 45600
   gagcctgagt caggaggatt gcttgagatc tgcttgggca acatagccag accctgtctc 45660
   tacaaaaaat aaaatgaaat aaattagctg gatgtggagg tccacacctg taactctagc 45720
   tagcctggag gctaagaagg gaggattgcc tgagcccagt agttcaaggc tggagtgagc 45780
   taggactgca tcactgcact ccagcctagg caacagcaag accacatctc tctctctctc 45840
   tctctctctc tctcaaaagg cagtgaaata acgacttatt tggggaaaaa ataaaggcag 45900
   agaatttgtt gccagcagac tagcataaaa aaaaggaagt ccttgaaaca gaagagaaat 45960
   gataaaagat ggaaatttgg atatatacta aagaatgagg attgctaaaa gtgacataca 46020
   tagataaata tgaaatatat ttttatttta aaatttattt aaagcaaaaa taaaaataca 46080
   tcatatttat aacatagaaa taaaaaatgt atgataatag cataaaggat aagtggacaa 46140
   atgctgttgt cgtatttttg gtaaaatgca ctattatttg aaagtagacc atcgtgaatt 46200
   cgatgcatat tgtaaaccaa atagaacact aaaaaatgaa aataaagaga tatggctaat 46260
   gtgccaatgg tggagataag atagatgcaa aaaaagaaaa acattcaaaa gaaggcagag 46320
   acagaggaaa aaaggaccaa agatcaaatg agtcaaatag aaagcagcta aactagcaat 46380
   atggcagatt taaatctagc catgtcaata gttatattaa atgtaaatgt tctaaatacc 46440
   tgaattaaag gatgaagatt gtcagattag attgaaaaag catgacccaa ctacatgctg 46500
   tctgtaagaa attagaaaaa gaacaaatta aatccaaagt aagaagaaag gaaatagagt 46560
   agaagttagt gaagtataaa acaaagagca aagaaaatca attaaatgaa aagctggttc 46620
   tttgtaaaga tcagtaaaat tgataaattt ctagctaaac tggccaagaa aaaagaaaag 46680
   acatacaaat taacagtatc aggaagaaaa acagagaatt caaaggagtg taatgcaaac 46740
   tttatgctag taaatgcaat aagttagatg gtatggaaaa aaatgtgaac aatacaaagc 46800
   agactgtggt tgcctttggt ggcagtagcg gggtgggagt ggaaggttga attgactgga 46860
   accagaagca caagtgaact ttttggggtg atggaaatgt tttgtatctt ggttgcattg 46920
   atagttaaat ggttgtagac attgcttaaa actcactgaa cacttaagtg ggtatgtttt 46980
   attatttgta aaatatacct caaaagcagt tttaaaaatg tattcaagta catacttaag 47040
   atctttgcat tttactctga gtatacctta attttaaaat ctgtttttta aaaagtatta 47100
   tgtagatacc ttttattttc ccaatgtctt tattaaatga catctccacg ttttgcttct 47160
   tacctctatt tttttttttt tatttctctg tctctcaggc atgcacacac acacaccaaa 47220
   aaaagtacat atgcataatc cttttggctg aataaaatca gttgcaactg ttatttcggc 47280
   ccttatttgc tccgggtaaa tattcgttag ctgagtggtt tatctgtatc agatatttct 47340
   tacatcttca tccagtcaca ccagctggac tgaccagatt gtttttcact tcaagggcag 47400
   aatttgtact cactgctgaa tgcttccaaa tgatacgtag aataacaaat ttaagactta 47460
   gatttttact ttttcaggtc tttttttttt tttctgtgct gtatagcatt tccctgaaag 47520
   cttaatctca tctgtaagtg atgcagtgga tgtgttacta ttggattaat ttatttactc 47580
   ttaggtaggt ttgtaatctg tcatcatgct gttgtttttt tgtgtgggtt tgtttttggt 47640
   tttgagacag ggtctcactc tgctgcccag gctggagagg ctagagtgca gtgatgtgtt 47700
   tatgggtcac tgcagattca atctcctggg ctcaagtgat cttcctgcct caaccccttg 47760
   tgtagatgga agcacaggtg cacgccacca cacccggcta tttttttaaa tgtattgtag 47820
   agacgaggca tcattttttt gcccaaggct gatcttgaac tcctgggctc aaacaatcct 47880
   cccacctcgg ctcccaaagt gctgggatta cagatgtgaa ccaccactcg agctccatca 47940
   ttctgttatt agttgttctc tagtatgagt caaaaactct tacctgccct tttacagttt 48000
   tataaataag taagcagaat agcagaatgt ggacattttt taaatccaaa ttgaatatgc 48060
   acatgactca aggagtcaaa tagtaccgta atcggtttat gataaaatcc agtggtttgg 48120
   ctgggtgtcg tggctcacac ttgtaatccc agcaccttgg gaggctgagg caggtggatc 48180
   acctgaagtc aggagtttga gaccagtctg acctacatgg tgaaactact aaaatacaaa 48240
   attagctggg catggtggtg catgcctgta atcccagcta cttgggaggc tgaggcagga 48300
   gaattgcttc aacccgggag gcagaggttg tggtgagccg atatcgcatt atttcagaac 48360
   aattttccac aagatcagtg agtgctgtcc aatagacata taatacaacc cacatacatg 48420
   actttacatt ttcttgtagc catagtagaa aaggtcaaaa gaagcagatg aaattaatag 48480
   cctgggcaac aagagcaaaa ccccatcttt taaaaaataa aataaaatat ggtggtttgc 48540
   tgtccccacc tcagaccatt tctctggtct ttctcattga ccaccactcc caatctttgt 48600
   tctgctgatt gattacagct tgtatatatc tccatatttc taagcaaaat gtttatcttt 48660
   tttaaattta taaattcttt ttattatttt tcagagacag ggtcttaact ctgtcgccca 48720
   ggctggagta cagtggcacc atcgtagctc actgtagcct cgaactcctg ggctcaagca 48780
   gtcttcctgc ctccgcctct caggtagctg agactacgct acaggcacat accaccatgc 48840
   ccagctcaaa atgtttatct tttgatacat tattcgagac cattattaag gtggatgatt 48900
   tagttttctt aaacagccat cccctttctt ttcctcccct ctgcttcacc gcccccattt 48960
   tcccaatgtt ttaccttttg gttaaatcag tactcattgt ttacattatt tgcctctgca 49020
   catagtcaca gatagtattg tactgtactg tactgtgttt cttttttaaa cattatttct 49080
   gttgttaata attgactttt taattttttt cctattttgt tttttaaaga gatggggtct 49140
   tactatattg cccaggctag agttcagtgg ctcttcgcgg gcatgatccc actgctgatc 49200
   agtacaggaa tttccacctg ctccatttcc aacctggacc agttcacccc ttcttaggca 49260
   acctggtggt cccccattcc cgggaggtca gcatattgat gccaaactta gtgcggacac 49320
   ccgatcggca taacgcatgc agcccaggac tcctgggctc aagcagtcct cccgggctca 49380
   agcagtcctc ccacctaagc ctcccgcgta gctgagacta cagacacttg ccaccacacc 49440
   aggttaattt ttgtgttttt tgtagaggtg gggttttgcc atgttgtcca gactcatctc 49500
   aaacttctca gctcaagtga gcctcctgcc tcagcttccc aagtagctgg gattatagac 49560
   gcatgccacc acaccccatg ataattgcct ttttttttaa tttgcataat tttctttgta 49620
   gcttttgcta atgttcccat atcttcttat agccttacag aatgattttc cacaagatca 49680
   gtgagtgctg tccagtagac atataataca acccacatac atgattttac ctttttttgt 49740
   agccatagta aaaaaggtca aaagaagcag atgaaattaa tagtatcttt tacttaaccc 49800
   agttcattca aaatgttatt tcaataaatg gtcaatattt aaaatacttg agatattttg 49860
   cttttattta tttcttttgt tactaagtct tcaaaatcca atgtgtattt tacacttaca 49920
   gaacatctct ttttagactg gccacatgta gctcagggtt actgtattgg acagagtggt 49980
   ttcagtttca agtttttcct tggagacatc ctacttgaaa tttccattct ccatgtatct 50040
   gggtggttgg tctatagact tgccactcac agctgtcatc ttgagacttt ctttgctttt 50100
   cttctctatt ggatattcag tttcctggat ttcaggtctt ctcattttcc tctagtagtt 50160
   ttgttaggtc atggttggta tggcatggtt gggatagcgt gttcacacag ctatctcgtg 50220
   agtcatactc ctccaatcca gcctgctcgc ttcccgtgtc tgtcatgtag ttgtcaccct 50280
   gctatctctc cctccagttt ttgcagaaat ttcctttgtc ttcactcttg gtcttcctct 50340
   cccatccccc atgtatccta tatctttctc tttcttggtt tatttcatca ctcaggtgga 50400
   aaagatgctc cagtggatta ctgggaaaag gggagcatgg gatgataaag gtattgagac 50460
   cttacacgtc agggaatttt tttttttttt tttttttgag acggagtttt gctcttgtcc 50520
   aggttggagt gcagtggcgc caactcggct cactgcaacc tccacctcct gggttcaagt 50580
   gattctcctg cctcagcctc ctgagtagct gggattacag gtgcccgcca ccacgcccag 50640
   ctaatttttt gtatttttaa tagagacgag gtttcactgt gttggccagg ctggtcttga 50700
   actcctactt caggcaatcc acccacctcg gaatgttttt attgtccctt ctcatttcat 50760
   gactgctggg ctaggtatag aattccagaa tcattgttct tagaatctcg aaggcattgc 50820
   ttcattgctg gccagctttc agtgttcttg caaagtctga agctgtgcta atcacctcat 50880
   cctttgaaag tgaactgttt tttcttccca gaaacttaca gaacattctc tttgtccgca 50940
   gaattctggg attgcaatta ctgtgcctta gaatgggtct gtttttatca ttatgaagag 51000
   tactggatgg gtcgggaggt tttcttgaat tacttcttga tgttttcttt ccttgtattt 51060
   ttttgtttgc taattttcta tttttttttc ttggtttact ttcttgggca gggggatttc 51120
   ttctacttat atttgattct tcagttgagc ttgtcatttt tgctatcttg tttttaagtt 51180
   tcgagagaca tctttgtttt atataacatt ctgttcttaa tacatagatg caagatcttt 51240
   tctttctgag tatattaata tgtatttgaa atctttctat tctctgcagt ttgtttcccc 51300
   caagggtttt tttttttttc tggtttttgt tttttgtttt tatgttagag actttcctgt 51360
   tatatctggt catcagtggt acctgcatgt ggtggagagt aggggcttat tggagtatga 51420
   gaaccttgag caggtgtaag gagcctgtca acactgcgct ggcctcaggg cctctaggga 51480
   ggctgccagt tgtgcattct gaggatacct tttggttgtg ccttttgtct ggtcagatta 51540
   tctagagatg ctctgcctcc tacctggagg agaagggtct agctgccagc ggtgtgagtg 51600
   tctcttgggg aaaaggactc gagttcctgg tgtttggctt gtgtatggcc gcttacccca 51660
   tttttggtgg agcgctcaca tcttccactg tgccaacagt cttgctgcag ttcatagacc 51720
   ttctggttta catttttcca gaaagtatgt ctttagattt ctgcagaagt ctgaggagca 51780
   tggaaggagc ttggggaatg agatggcaat ccaggtcttc ccagatggct ctacctttat 51840
   cccctgcagg gaatcccact cctccttcct gactgggagc acagccagag ccttgggagg 51900
   aatctggagt ggaaatctcg ggcggtctgg ctttcttact gttcacttgt aattttgctt 51960
   tctcacaact gccaaccact aatcagcctg atttccagct tccagaattc tattgctgtt 52020
   gtctgctctc ctattcccac cgtaggggat ggggctgtct tttttttttt ttttaatttt 52080
   ggtaaaacat acaaaacata aagtgttcca ttttagccat ttttaggtac acagttcagt 52140
   ggcagtaagt acattcacgt tgtgtgtatt tgttttttta gtaataaaca atataaaatt 52200
   ttttaagtaa taaaacacaa ataaaagatt gtttaatgtg attatcgtgg aattttaggt 52260
   gtgatcagga gccatggtgt agtcttctgt tgaaacaggg tgataggatt tgtttaccac 52320
   ctcctaggaa agcagttgga tagtttgttg gcataaaagt acattttatc tatttttaat 52380
   aatcgtagct ttatagaaat tgcagttgga actcccaggc ctggcattca aggctctctg 52440
   agatctgggc tacccaccca tgtcctccag ccgtctgtcg cacctcctac tgcccactca 52500
   ctgttcctgg catgagatgt gatctccagc ccccatgcct ttgctgtgca gggtgttcca 52560
   gagtgaattg tccctcctgt ctgtctctct gccctcttcc tcgtctttcc atcttcctgc 52620
   cccacatcac tgcctcctac ccaaggcctg tgctcattcc tcctcggttt tcccccatgg 52680
   cctggtacat acctctgaat tatcaccttg catttcccat attgcccggc tctctttgat 52740
   gtctgtttct ttgctgggtc ttcctcagtg tctgacggtc agttaaatgt ctttattctt 52800
   ttttgtagga tatccgacat gaagccagtg acttcccatg tagagtggcc aagcttccta 52860
   agaacaaaaa ccgaaatagg tacagagacg tcagtccctg taagtatcca cgtggccggt 52920
   accagtcttg ctcttccttt gctgcaggcc tttttagtca agactccttt cgcctcaggg 52980
   tttagtataa taataaatca atgtagcaga ggtttatgac gcgattgttt cctatagtaa 53040
   aggcattaga gacttatagt aatagctcat ttttccacca ttatagaagg gctcaggttt 53100
   cagtttctgg aaaattcagt gaagttcaaa gcacttttct taagctttga ctgtttttgt 53160
   gatgaatcat tttcctacca gctgaagcag agtatagcag gcataataaa accttttctg 53220
   gatgactcag cagcagcgtc attagggcat gagcactgtg ttccgctgta atgaagcccc 53280
   gcacaggcat tcggggtggg cactgtcgtc ccctgcgctg aatatgcaag gcagctctgt 53340
   ctggagtccc caccgcctcc acccccgcca acctcatcat ttttctccct ctttcctgct 53400
   gttagttctt cctaggattg tcagtgtgcc tgctggcctg tggcagccct gtccgccttc 53460
   tgagtgattg gctgtcagtc tgccggtagc tgaaaagtaa ataacttaac atgttagaat 53520
   ttgcataaag taaggaaaac tggagctgag tacaggactt gaactgcgcc atctcctcta 53580
   ggccacagag gcctttttga cccccttcca ggtctttaga cattgtcagg cagtgagggg 53640
   tcgtagctgc cagtgtctcc atggtagcgt gctctgccag ggatgcagaa gattctccag 53700
   tcattcctcc agtgggcact tcctgcaggt cctgtgccca tggctgggag tggtggctgt 53760
   cattgttctc tgccagaagg gttagcagtg catcctgacc tgacttatgt ggcgcccaga 53820
   ttcctggaag gggtctaaaa atggacctag acttggtgta gaacgtgtgc ctcttggcct 53880
   gccaccatgg ttccctgcct ggttttgtgt gtcagctctg ccgcttaaga actgagtggc 53940
   ttcgggcaag ttgttctctc tcataggagt gtgtgaagat gaagcaacat aagctgctta 54000
   gcccagcgcc cagtacctca cgcagacata agtgctcagt aaatgttgtc tgtggtgggg 54060
   atggttgtca ccaacatctg aagtgcactt ctaggtcatc aggtgacatg attggcgcca 54120
   acacatggta ctcttgattt agcacatctc agctgaggca cctcattgat atttgtttaa 54180
   aaacaaaaac aaaaaacctt ggtgattctg ctgtgaagtc ctggccagaa acctccagac 54240
   cgctgatcaa cacgcaacag aaccatcacc gttcacctct ttgacatggt gccaggatac 54300
   cctggatctc tagcttttgc tatagttgct ctaattaggg aataatcttg tctttaatat 54360
   tcctttgcta cattttttaa catttcttat ctaaatggtt ttatgaatca gttttacaga 54420
   gaaaaaaaac cagtatttaa aatattcttc caggggctgg tccaagtaca gtagtgttta 54480
   caactatgtg atcacaacca gttacagatt tctttgttcc ttctccatcc ccactgcttt 54540
   acttgactag ccaaaaaaaa aaaaaaaaaa agttattcca gggaaacaat tctccaactt 54600
   tttcactccc aatctcactc ctcttatctt cctcccgtac tcctatcctc ctcccgtact 54660
   cctatcctcc tcccctactc ctatcctcca gtagaaacag tcatttgctg tgaaggttat 54720
   gggggagaat gagtcaaggt agaaggtcac ctgctgccca gctcacagtg ctgctggtga 54780
   tgacagcagt ccacagttac aggcacttgc tgaacgaggg gctctgtata cacctcagct 54840
   cattgactct tcccacaacc ctcttgtcac ctaccattta gcaaatgaaa aaaccaaggc 54900
   tctgaggtga gttgtttgcc cagagtcacc cagtgctgtt tgaacccact cacataacca 54960
   accaatacca ttatgtaatt tttgaggtct tttatctctg tgatccactt aaaaattatc 55020
   caagtatctt tatttgtact aagcctccat aatgagaaac agtgttccag atggtggcta 55080
   gttttcaaag acatctctct ttggaattct tctttagaac aaaaagcccc agaccactta 55140
   tccccattca tatccccttt ggacctaggg agaaggtact atttataggt gatcacctga 55200
   gtttattgtc ccttgtgctg tgccagaaat aaaggtcccc acctgctctt attagctcta 55260
   ctaacaggat aaggaaagtg gccctcagag agctactgct tttgtgacaa acaaatgata 55320
   caagaaaaaa aaagtggctt tttaatttta gtgacctggg gcaggacttc caaatgaaag 55380
   tttatttcta aaaactaaaa ggtaaattta atatactttc agtgtttggg cttaaattct 55440
   ctttcaagtg tctttgtgat atgctctgaa ttttaaaaat ttagaatcat tgaagttcat 55500
   tatacttgaa ctttaaaaaa aaaaaacaaa aacctcgtat aaaggtcaag gtatgacttc 55560
   atgctgctgt gtacttaggt catttaatct tcaaaccact ggatagaggt taggttgaag 55620
   ttcgatctta aatcctacct actgtagctc attgtaccag caacagctgt agggactagg 55680
   tggaattcat ggtgggtttt gttccctttt aaagattgaa gccaccatat tttctgccct 55740
   ctaaaagttt atgtcagcca ggcatgggtg gctcacactt gtaatcccag cactttgggg 55800
   aggctgaggt gggtggatca cttgaggcca ggagttcgag accagcctgg ccaacatggt 55860
   gaaaccccat ctctactaaa aatagaaaaa ttaggtgagc atggtggcct gcgcctgtaa 55920
   tcccagctac tcgggaggct gaggcaggag aaacatttga atccgggaga tggaggctgc 55980
   agtgagctga gaacatgcca ctgcactcca gcctgggtga cagagtgaga ctcttgactc 56040
   aaaaaaaaaa gttatgcatc agagaacaga tcctttgatg ccctcctctg ccctgaaagg 56100
   tttttggggg agagtaataa gtatcacaac aagatatgac ctgagaacag atttcccaga 56160
   taggacatga tccatgtttt aatatggctt actgctgttg cttcatagtg tgaagcttca 56220
   gacacttctg aaaacccttt cagaaaatcc cagtcgcccc atactgatga ctaatctcaa 56280
   ctaaaacagg gcttcagcca gtgtgaatgc cactaatgcc accaactcac ctttgctttt 56340
   ctgtagggtg tgcacctgta tgtacacatt cagcttttcc gggattaacc tctgagttct 56400
   ggtttgtctt tcagttgacc atagtcggat taaactacat caagaagata atgactatat 56460
   caacgctagt ttgataaaaa tggaagaagc ccaaaggagt tacattctta cccaggtaag 56520
   cagattgtct gaattttcta tttaatgtca atttaagagt ttgagagtgc tgttatccac 56580
   acctcaaata aaatctgcca catcctttag aaggtcagga tttcagcata ccaaaaagca 56640
   gcaaggaagg gggaaaaatc atccttcaaa ggttcagttt ggttataagg aacgctaatc 56700
   ttttctggga agcataagat gacattgctg gaaatgagag cttatagaaa acaacattaa 56760
   aatgccagag ttgcctgtgt ggtctgttgg cagagacagc agagccatgg ctggaggagg 56820
   gtctgtacct gtgttgcttc cagaagtatt tgtcgtagag cacttgtgat ggcaaatcta 56880
   agaacgttag cagtagacca ggaatctctg tccagagcca ttcagagtag ctcagcatgg 56940
   ttctcattct ttggccagaa gaaaggcatc attggatcat gtgaacaagc atgaaaaatg 57000
   acttaaaatt tctgttggct tttggcatct ttatggaaac aaaatcctga aagtggttta 57060
   ataattgagc ctcttgtaaa acactcagtg gcatgtgacc aaaagggtat ctgggaaaga 57120
   ggataaaaag agtttctttt taattaatct tctcaagtct taacttgtta cctgtaagtt 57180
   ggtctaaaaa gactgggttt cttattttgt ttttcatcat aatttttgtt tctcattcca 57240
   tgtcagcttt cagtcttata tggctttagg ccacagggcg attttgaaca tttgtaattt 57300
   tgcttaataa ttaggaaatt aaaattctgg ggaagacaga atgctctatg aagaaaggct 57360
   gctttgagca aggagctagg tcagggcgcg ttcaactgag gcctttcttc actgcctttt 57420
   tgtcttgtcc cagttcctcc ccatttatga ctaaaatcag cccagatgct tctcgtcatc 57480
   tgggatgcag agcatcagcc cagctgtgtt cagtcctatg gggccattga gtaagttctt 57540
   ggtgcatgga tacagggcag gcctttacca ggccctgagc ccctggtcct cccagcacct 57600
   ctggggtatt taggggaggc tgatggggga gggggttgat aaggcgggag atgtctgggg 57660
   atgaggttga ggcaaaagtg acttcttgag gactttgctt tttggagaag tcaaatttcc 57720
   tacttcttga tttcagccct tcaactctgg tatggagtca ggaagccctt taaatacctg 57780
   ttgtcgggtg tatcatgtca agtgttgcat tagcaaatga ccatgtatcc ttgtgctact 57840
   gtcctgccta ccccgcatcc tagcgcttcc ttgggacatg agaagctctg tctggtttgt 57900
   gaggtggcac tggggatgtt gagaaactgt ttacacagtt tccctttgcc ctggggattt 57960
   actaaaggag tcgaggcagc ctgaccccaa agcatcaccc ctggacacta tgaccgaaac 58020
   atttccccag tgcccaaacc aagaacaccc ttcccatttt tttttcagtg gtgttcatta 58080
   tgtaataata caagtctctc ttctcatttt ttaaaagtca gaagtacaga agagcagaga 58140
   ataatgtcca aggggccctc cttcacctcc cccgtgcagt gtcagctaag tgtggtgcgt 58200
   gtccttgcag atcttagggg attgtgatcc ttcagaccat tctaaactgg ggtggtgctg 58260
   ggagttaggg aaggcatgaa gggagtagtg gagagctgca gtgactgggg tcttcatgcc 58320
   agggtggaga atgcaaggcc caggtggcca gccatgtgcc acgggatttc tggctgccaa 58380
   gagctgttta tctgttcact ggggagggaa gagttaaatg tggtctgctt ttctccgagt 58440
   cccttcagca cagggagtgc tgacttgtct tgttcaggta gtaagttcaa gatgagctca 58500
   ggaaagaaag tgagaggaca ctgagggcta gtggttgagc caagtgtgat gggacttaaa 58560
   gggagaagat ttaaagaata aggagcttat gggccgggga cggtggctta cgcctgtaat 58620
   cccagcactt tgggaggctg aagcaggtgg atcacttggg tcaggagttc gaggccagcc 58680
   tggccaacat ggtgaaaccc cgtctctact aaaaatacag aaattagctg ggtgtggtag 58740
   tgtgcacctg taatcccagc tacttgggag gctgagacag gagaatcgct tgagcccagg 58800
   aggcagaggt tgcagtgagc caggattgcg cccctgtact ccagcctggg tgatggagcg 58860
   agactctgcc tcaaaaaaaa ttaaaaaaaa ataaagaggt taggtgaaaa tagatgagaa 58920
   tggaaaccat gagaagaagt gatgctggcc aaggacatga caggttctga tgtggaggtg 58980
   ataggcaatg tctcttccag ccactgctaa taattgagac aaactcaagg cattcatacc 59040
   ctgtgtccag taaacatctg tgcccattgc caggtgagct ggattgaaat gggccagctg 59100
   ctcagcagac accctcatgc cccagtgact ctgttcccct tgggccacct cattgaccat 59160
   ttatgtttct acatctccta agtttgttgg gccaaggatg gaggctgtct gccgtcaggg 59220
   tcctcattgc tgatggtagg aatagttgct gatgtttcat tggatgttgc tgtattctag 59280
   ggactgtgct aagtacttta tagaaatgaa catacttcat tttcacagtt ttatgaatag 59340
   ggactattat tagtcaagta agcgatgggg aaactggggc agggagcgat gaagtgactt 59400
   gcgcaaggtc acaagatgat gtgattggaa ccaagagaag tgttgtggtt ggccacgccc 59460
   ccacactgcc tctcatctgc accaaggagt tttgtcccat agcccaaggg ccttggggac 59520
   gaatctcagt ggaggccctt agcgggcctg cctgagccag aaagcagaat cggcattttt 59580
   ctgtccttgg ttggcccagc cctgaactga gatgcggaaa tcgcctttcg ctgcctggta 59640
   gaaaatggag ctgcagttac tgaccaccag gcagagagag gtgggtccct gtcccagcct 59700
   cagccaccac tctgcctaag ctgtggggac tgagggcgct gtcgttagct gactgcagaa 59760
   ggtgagcaca cgctgtagca tgttatgttt cagatgtcac atgttgtgtt attgtgtctt 59820
   tgcagggccc tttgcctaac acatgcggtc acttttggga gatggtgtgg gagcagaaaa 59880
   gcaggggtgt cgtcatgctc aacagagtga tggagaaagg ttcggtaagt ctcggcttca 59940
   tttgctgtgt atgtgatcat gcataccact ccatatagtt accattttcg tccagatttt 60000
   taaattattt ttcttgcctt tgtatttcct ttacgtagta tttttattta aaaaaattaa 60060
   aacagcagca tataaatgca tgttggttgt caaccagtta atgaagtgaa taaaagggag 60120
   gaggcggaag aactgcacgg acctcttcgc ccccgccttc tcctgtgtgg tgcgtgtggc 60180
   gctccgccca cctgtgctgc ctgtgcggct ctcatcacag tgtggagttg tgtgtggagt 60240
   tatggagacc tgcttttatc ttgaaaagca agttcttagt gcatcttcat ggtgtctgat 60300
   tttttggctg gtgagagtgt ggctacctct gcggagctgt gggagcggct gactagatga 60360
   gatttgcctc cattcagtac ctagactctt gccctgccac acctcttcgg agtgagcatt 60420
   gacttcagga tgtgtgtcat tctaagttcc tgcaactttt caaacacccc tcgggctagc 60480
   gtgtggctgc acggtgtcca tttgtgcagg ccaccactcc tcttgcatct gggtctagcc 60540
   acctctcctt cttgacttac catagttcat tttgtaccat gctttcagaa tgagctttct 60600
   caaatccaag tctcaccacg gttcttccca gctgaaaacc cttgtgcggt tccctttgcc 60660
   tcacaggata atacatggtg tggcttacgg aaccctgcag gtctggccct aggcccctgg 60720
   acacagacct ctcaccactc ttggaacttt agccaggaca aagttttctg tttttagttt 60780
   cttaccatgt tctctgggcc gaggagtccc agtgcccacg ttcatcccac ttgcaggcac 60840
   ccctggacgg ctgcccccag ctccccaact gcctgcattc tcccctgccc tcctcactct 60900
   gttggaatag ctgagaatag ccgatttctg ggcagccggc ctcctgtgta gactgtcctg 60960
   tgtagactgt cctgtgtaga ttgtctgtgt agactgtcct gtgtagattg tctgtgtaga 61020
   ctgtcctgtg tagactgtcc atgtaaactg tcctgtgtag attgtctgtg tagactgtcc 61080
   tgtgtagact gtcctgtgta gattgtctgt gtagactgtc tctgtagacc gtcgtgtata 61140
   gactgtcctg tgtagactgt ctctgtagac cgtcgtgtat agactgtcct gtgtagactg 61200
   tctctgtaga ccgtcctgtg tagattgtct gtgtagacca tcctgtgtag accatcccat 61260
   ttagaccatc tgcctgtgca ggcgcaggcc agtgttcagc agggccacag gctcctcggc 61320
   ctccctgccc tcgctgctcc ccaacactgc caaccctgct gcggggtcca ggaggagatg 61380
   ggctgaggat cgtggagacc agcaggagcg tgtggcccag gagcagggaa ctgggtgtcc 61440
   ttgggccttg ccaggtccag gctcagctag gacacggctc tcacagctgt cctggttgcc 61500
   tccggccaca gaagaaggtg agggctccag agaggccacc tttccaaaaa aagcacagtc 61560
   atggccctag aatgtaaaaa atccaagtgt taagaaggaa cacatcaaag gaaacttcag 61620
   cagtgaaaac ttgaagcatt aaccacgaag cctctgcctc caccacacac aaagaaacgg 61680
   ctttagttac tcgcagaaag tcttcctctt aggacagcgc gtgtttaaaa tcataggggt 61740
   ttggtttgtt ttgttttggg gttggggttt tttgggggtt ttttaccctt gcctactttt 61800
   taaaaaatga aagtgtttat ttgcccaaca ataacagaca gggagcttgc ctaagtgttc 61860
   tgttgatgat ataatgtatc ttgtcttaga aaaaaacttt ttcagtgaaa ggtggttttt 61920
   aaattttttc ttccctcctt agtagcttga ttagtaaaat gtgaagttac aaatgtgaag 61980
   caaaccccca cccttcacca ctagtcagca attttgagta aagaaacaaa gcatcaggtg 62040
   ctcacagcac acactgtctt agagggaagg ggaagcctgg tggcctgtgg aagccttcag 62100
   catagctcca tctgcaggct tctgaccctc agcactactg acacttgggc tggatcattg 62160
   tctgctaggg atccgggcag ggagtggctg tgctgggcgc tgtaggaagt ttagcagcat 62220
   ctctggcctc tatccaccag atgccagtag caccccctcc ccagatgtgg cagtcagatg 62280
   tgtttctgtc tagactccag actttgtcca acgtcccctg gtaggccaaa ttgcccccgg 62340
   ttgagaacca ccgctctaga tggtattgag ggttgggaat tttaaatcaa gacatttatt 62400
   cagaaattac cagatatagt agcatttgct tcttatttat ttctttgttg ctaagtgttt 62460
   ggcaaaacct ctttgctgtg agcacaaggt ttgctttagc aattgttgtc acattacagc 62520
   aaggagtggt gtccagcgct gtagttatgt atttgagcag tgtccagtgc tgtagttatg 62580
   tgttccagcc tcaccaggcc ctgtgcttca ttgtctccca ctcaagactg accacaaatg 62640
   gcccacagat ccactgtgac aacctttccc tttgggttac tgtggtggca tcgagaacat 62700
   ggctggttgg ctttgctgta gtttactgtg ataactgtgc cagcagtccc tgctttcctt 62760
   tgttaagtat cccattccac tggaggatta cttgggcgtg cagattggca tgaaaagcaa 62820
   tgtatggttt gagattgtta aagtttcttt gggatcaaca ttttcaattc tgtatcagca 62880
   ttatccctcc cagagggctg gctgggagaa atcatgagaa gttacagtat cttatttgct 62940
   cagctaatct aattataaat gatccacaca gcttgtggta aaaccagctt ttggggagtt 63000
   ttcatttaat gcatacttgt cttctgattt ccttccttca ccaaatagtg taggatgctc 63060
   cctcttattt ttggcaaaca tgcctgttat cttttgggac cctgggcttc ctggaaacca 63120
   gttatgcaga agatgattgt gtgtgttaga ctggggtcat ccagatggct agagttctca 63180
   ctggttctgt ttaaggattg actttagaca cctcagtgta ggctgcacca tggcgtaagg 63240
   gttgggattg ttgtttagaa gggggaagta agcaaggtga gtttaattgg ccattgcaga 63300
   atctcacccg tatctccctc ctgaaatcct cactaaagct gccgtttgct ttcaggtgct 63360
   ttcatgcaca agacactgca ttttgtatca cagggtccat ataattcatt tttctctcgt 63420
   acttagttct ctgtgttaag aattacttac ttagttctct gtgttaataa tttttggcga 63480
   aaccaaatta cccgtcacag ggttactgta gatgtctttc ataggttttc caaacaccac 63540
   ttgcccactt gtttgggaag gccccaagga ctgtttaaca tctgccttca tggtggaaac 63600
   agcaactatg agagatgcta gcatgttggc actgccatgt tcctctggta ccagcccaag 63660
   ataggactca atttgaggcc tggtgaagta ctgtgttcta ataaaaatcc atctactttt 63720
   catggccgta tatatcaatg taatagggta actggaaatg tgatcttgtg ccttttaaaa 63780
   attttgtgtg tttaaaacaa aaatttctat tggaaatgac agagcatagc ttgttgctgt 63840
   agacacctga gagtccttaa aaataaatat tgggttattg acacttagtt gcatgacaga 63900
   attcctcact tgtacagttc caaagtctta gtctttaccc agattacaga gggttattaa 63960
   gcattaggtt tggttttgaa agtgagtgct tgctgtctgg aggtgagctt taagactcgt 64020
   ctgccctgct tatgagatga ggaagggtgg cctcttcctc ctgcatttct gttcttcgct 64080
   tccttctctg tctgctcact ctgtggaatg cccaccccag cacgggtggg gtggaacctg 64140
   tcagatcagt ctcttgtttc tggggtcttg aggcattata agatctagtt gttagaagtg 64200
   tgggattaat tcatcttttc acattcttct aagttcctgc ttttagctgc cacacccact 64260
   ttggctaagt gggggtcttg ccatgtaatt agcgcctcca tgccaagtgg cagaattgct 64320
   tcaatggtga cagattgtcc ccattcaaga gttcactttt ggcaactcat cattgatcca 64380
   ggaaggtgac atggatgaaa ctggctaaga cttcagacag gcttgtgtcc agactcttga 64440
   gaaagctctg ttggcttctg gtctggcact gtgaagtttg ctgtgatgct ggcaccacaa 64500
   cctggtgttt cctaatttgt ttctcccaca ttttgctttg gttttgtctt ttgggcagct 64560
   tccagctcca gtagagcagg accaataggc atttgtggtt ctatattcac cctcctcacg 64620
   tgcttcctgg ctcctcattg cccccagatg atgccacagg tccctgggcc tgctgccagt 64680
   cgtctgtgat ctgggcctct gctggcccct tctccagctg ctcttttcag cctcttattt 64740
   gcagtcactg cctaggaaat cctagtcatc cttcaaaacc tgcctcttgc acagagcttt 64800
   ctctgatctc tcttttctgt aaccttggct gacctgaaac atttccctct tctgaattcc 64860
   tgctgcatgt ccgtagcatt tccccctcag ccctccccca tagtccacct tgtcactgct 64920
   gggcacagca gtgtcttctg acagacagct ggccctgaag tggttccctt cacccacacc 64980
   atcctttgcc ccagaggagg tattgagtgg gtcagtgcac gtgaactgcc agtgtcattt 65040
   gccaaagagc tgttgacaca cgctgacatt tcttttgctg aaaatcataa gggctttgag 65100
   cttccctctg tccaggcaca tggtcaggct gacccggtag ctctgcccct gctgacctgc 65160
   catttttgtc cacaacagtt atccatgagc agaaacattt gtgtaactga ggcagaaact 65220
   tagttcaagt aaaatgtcac taaattcgag tcagtttttg tcttagaccc taaatgaaac 65280
   caaattttca taaattttct tgttttaaag aaaaatttaa tgagctacat ttaaactgag 65340
   aacatcagat agtgtctgag attatcaaaa tagaacatca aaagtatttt tctgaatgaa 65400
   ctgaaccaaa ccagaatgaa agggcaagcc ctggggagcc tgtctccaag ccttctctga 65460
   aagggagtct gtatttggtg ataactgctc agcctctcca aagggcctca cctgctgtct 65520
   ctcccagttt tatttttaat tgcctgtgag ttttctgtgc agggtaaggc acctacattc 65580
   tatgccagca gcctgatcag gtcctgggta atgtttgaaa tggctacaca gaggagtttc 65640
   aaagcctttt gttcaatctg gcttcacctc gtagacggtg agaaagcgtc agagccctgc 65700
   aggatcccgt tgccacgttt gaccggggag ccgatgggtt tggaagtctg agccctgtct 65760
   gcacaacctg ccccggtcag cagcttcgtg cccccacccc catctcccca tgaggcaggc 65820
   atctgtgctg accatggctt ccatgttcag aaacccccag gcctttgagt tatcatgaag 65880
   cttgtgggat gtgctccaag cctcctgcca tagaaaaact gccatattgc tcacaataat 65940
   tcactattat ttgtttcccc agttaaaatg cgcacaatac tggccacaaa aagaagaaaa 66000
   agagatgatc tttgaagaca caaatttgaa attaacattg atctctgaag atatcaagtc 66060
   atattataca gtgcgacagc tagaattgga aaaccttaca gtgagtatag cacacacttc 66120
   agcacttcag gcggctactg gttcacatgc ctcttccttt atcccttggg tgatattacc 66180
   taatgtcagt gttcctggct tttgtatacc ccgagcaaga tgtggtttgg gcactgtggt 66240
   gagcggagct tacttgtgta cctaccaagt gcccagggag ggtggaggcc acagtgctct 66300
   ctctgacctt taacaacagt taacaccagt tcttagggaa aggagagttt cttacccaaa 66360
   agactggttc ctgcttgtgc agctgcagag ggactggagc ggcagcctgc aagtcccagt 66420
   gaagcatgct gccttctttg tggtcctcag tcttcgagtc tgaagagagg gaagaagggg 66480
   tataggggct cactccagtt tcatagctag tgaaagtttt ctgggccagg tcttgggttt 66540
   ttttgttgtg ggaagagttt ataacaccag ctacttgctt ggtaaaagtt ggtcttggaa 66600
   catggcaagg cattgtggca agcagcactg ccgctgaacg cgctgctcct ggggctttgg 66660
   aataattccc ctggatccgt aacttggggg tgttcatgtc attctgggga acagtggagg 66720
   gagtgcgcgg cagcacctgg gggcaccagt gaagagtggc cagccaccaa cctctagaac 66780
   ctaactgggg tcgaatcctg gccccacctt actagctcat cacagtgtct ccgtttcctc 66840
   ttctgtcaaa ctcaggtttt gcgagggttc tgggaggtcc tatacgggaa gggttagcag 66900
   ttaccatggg tgtgtagcac gggctttatc tgaagggaag gtggagccgt agggagacca 66960
   tgtggagtgg ggctccaggg ctgtgtgggt ggggagggat ctgcttctgg gttaccccat 67020
   gcctcccctt ctcaagtact actttttaat catcatggct cctgccattc atttcatagt 67080
   tgatgtaagc caggtgcggt ggctcacgtc tttaatccca gcacttgggg aggctgaggc 67140
   caggaggatc actcgaggcc aggagttcaa gaccagcttg ggcaacatag tgagaccccc 67200
   gtctctacaa aaaaacaaaa acagttagtc agacatcgtg gtgctcccct atagtccagc 67260
   tactcaggag gctgaggcag gaggattgct tgtgcccggg agttcaaggc tgcagtgagc 67320
   tatgcttgca ccactgcact ctagcctggg tgacagagca agaccctgtc tcaaaaataa 67380
   ataaataaaa aaaatagtag aagtaagatc tagaatgtag cacaggttac caggacgtag 67440
   gcaaggggtt cgggctgcct ggctcttgag gatggtagca gtgcagctga tgtgagtgct 67500
   ttctgccctc tggtggtgac cgcgccggag tcaccagccc tgccatagcc ctgatggggc 67560
   agagggttct gagtacggtg gatggaggtg ctttctggaa gattctcagg agtaacatgg 67620
   gcagtgtgtt ggaatgtgct agaggattta tgcagtagcc ttttaaaaga atgcttttta 67680
   gcatttgcaa gcctgacatt aagagtgact tctgggaaac tatttgcttg ttgagggaaa 67740
   ctgaatttca acagagcaga agagctgtgc gctttttgct tggcagagtg aatacagcca 67800
   gctcagaggt tttgatgtta ggatctgttt gctccaacag actttgtttt taaaaggctt 67860
   ttctcagcca tagctgtctg ttctagcaca aggctggaat gagttccttg tgaaagaggt 67920
   gagcaggtgt gagggagggt gtcagtgggc ggtaacccac accttcaagg attaaaggaa 67980
   aacttgcatt tggcatgctt gcttcttatt caattttaaa atacatttta acggccgggc 68040
   acggtggcta acacctgtaa tcccagcact ttgaggggct gaggtgggtg gttcacgagg 68100
   ccaggggttc aagaccagcc tggccaagat ggtgaaaccc catctctact aaaaatacaa 68160
   aaaaaaaaaa aattagccgg gcgtggtggc gggcacctgt aatcccagct actcgggagg 68220
   ctgaggcaga gaattgcttg aacccaggag gcggaggttg cattgagccg agatcatgcc 68280
   actgcattcc agcctgggcg gcagagcaag actctgtctc aaaataataa taataatttt 68340
   ttaaaaatac attttaagtc cttttcttcc ccacctgcct ccacccacca aatagaagag 68400
   gtatttcttc ttctttaatg tcattaaggt tatatggata ccattttcta gagaggaaag 68460
   aatgatggaa ttgcctagtg tgagtctagc aattatccta acatacacaa atttctcctt 68520
   gttctgtgcc aagatactgt atttaatatt taatgaacat taaatattat ttactagtgt 68580
   atttaatggc tgaggcaggg ttaaatatgt attattttca tcccagcaga gttgggggag 68640
   gtcctagtaa ctatgccatg agctctgtga gggtgaggtg gtgtctttgc ccccgcctcc 68700
   ctggcacagt gactggcaca tgattggcat agtgtggaca ttcgtcaagt gaaggaaggc 68760
   atcatgagca gatctctggc ctgaatcctt ctgccatcag ctgctcgcca ggtggccctg 68820
   gcactgggcc acagggaaac tctccaggct ggtatggttc ctgtctgtgg ctgtcttccc 68880
   gggcccatgt taggagactt tcacttccag agccctttcc ctctcagggc cttgcttacc 68940
   aagtgactgg ttcccattta ctaggagctc ttaggtcatt gaagatgttg cgtactcccc 69000
   ccagtgaggg ctgccttttg atcacagccg ccagaagcct caaggaagga gcagagctgg 69060
   aaacagacgc caggccattg cttctgttcc tctggggcag acccagccac ggaagagaca 69120
   ttctgggaca agggctgggg tccacctttc aaacgtgtct gcagcaggct ctcagcatgg 69180
   actctctgcc tccaaacatc cacctcctca tcggaaaatg gatgggagtg cctgcctgga 69240
   gcagctggtg ggagagcgca gcgccagcac gtaggacaca ctcggttcat gggctgatgc 69300
   cgttcgcatt gactgcctct tcagctgggt gttgagccac accttggagt caccagtctt 69360
   tggagaccaa gtctgctact tttttctcta aagtgacaat cctctgaaac ctccagatca 69420
   tcttgaagcc cccgtctgaa agttgcccag agccagtgcc tcacctgctg ttccttgttc 69480
   actttttcac gggaggcctt gcagggcttt atgacaagat tttatgggtg gctgcccagc 69540
   atcattgtga ctcgtgagac agagagaaac cagttgtaac catgtagaca gtggaagtga 69600
   tagggagaaa agaggtgagg ggactcttca atccgaaggg aaatgaagtc taagcaggcg 69660
   caccctgcag gttcagtgtc aagcccaggg cctggcccca gggtgtggta tttgttgact 69720
   gggtgtgtgg accctgggag aaagtctgag aatgaatgtt cctcttagag gtagagagtg 69780
   gaaggtgact ctgtgtgtac ttggaattag tgatttctgt acagatgatt cttttagaat 69840
   catcatgagt atttttctct ttcagaccca agaaactcga gagatcttac atttccacta 69900
   taccacatgg cctgactttg gagtccctga atcaccagcc tcattcttga actttctttt 69960
   caaagtccga gagtcagggt cactcagccc ggagcacggg cccgttgtgg tgcactgcag 70020
   tgcaggcatc ggcaggtctg gaaccttctg tctggctgat acctgcctct tgctggtaag 70080
   gaggccctcg cgggtgccct ggggagctcc tctacctgct ctgctgtgat gttttttcct 70140
   aagtagaaac tgaagcgctc ctcttccaaa atacagagac tcactgtgtt agtctgtttt 70200
   tgcgttacta ataaaggcgt acctgagact cggtaatttg taaagaaaag aggtttaact 70260
   ggctcccggt tctgcaggct gtacaagcat ggcaccagca tctgctcggc tcctggggag 70320
   gcctcaggga gcttccagtc atggtggaag gtgaagggga gcaggagcaa gagatggggg 70380
   aggtcccaga ctcttaacca gctctcttgt gaatgcattg cctcagggag ggcaccaagc 70440
   ctttcatgag ggacctgtcc ccctgaccca gacacctccc acccagcccc acctccaaca 70500
   ctagggatca catttcagca tgagattggg aggggacaga catctaacgg tgttattaac 70560
   gttgcccttg agaattggac ctggctgact tatatctcct ctctggcttt cagatggaca 70620
   agaggaaaga cccttcttcc gttgatatca agaaagtgct gttagaaatg aggaagtttc 70680
   ggatggggct gatccagaca gccgaccagc tgcgcttctc ctacctggct gtgatcgaag 70740
   gtgccaaatt catcatgggg gactcttccg tgcaggtcag cattgccttt gtttgaatcc 70800
   aggtgtgacc attttaactt ttttgtcttt gaaggaggct gtcagttgta aaagttcaaa 70860
   caccgtctgg tgtcagggga aatagctacc cttcatgttt aaaatagcta gaaagttgtc 70920
   aaaatgttca ccatgttgca ctttgtgcct ttgaagtgct cacatagaga gcattgatag 70980
   gaagacgaga ctttattttc aaaagatttc atcttccaag tacatggctg cagccctgag 71040
   aggccgagag cccctcgcca agccgtcacc tctgctcatg caaagggatt tcctgacaaa 71100
   ccagccgaag tgaacactaa taggacttcc tcttgctgct ctttcaagga tcagtggaag 71160
   gagctttccc acgaggacct ggagccccca cccgagcata tccccccacc tccccggcca 71220
   cccaaacgaa tcctggagcc acacaatggg aaatgcaggg agttcttccc aaatcaccag 71280
   tgggtgaagg aagagaccca ggaggataaa gactgcccca tcaaggaaga aaaaggaagc 71340
   cccttaaatg ccgcacccta cggcatcgaa aggtaatatg attgggtccc agcttgttgg 71400
   ggtgagggga aatgactttc tgttctagaa acacacgctg gtactgaaac cctgtggatg 71460
   cagcctcctg ttggcaagca gcgcttccgc atccttgggg aacagggcgc gtggaccaca 71520
   gccactccac tcctggctgc tggaggtccg gtattgggca cagggtggcc gcaggacatg 71580
   agccacttct gtgggcttct agtgccacct tgtggtgctt gttggaatga ggggctcgga 71640
   gccaccgagt agggtttttc tgccccccct gacgacagcg ccctccccca ggtttccgga 71700
   cagtcctgaa atgtgatgtc caggcttgag tgccctcagt ccccacagtg gtcctttggg 71760
   gaatgtaacc ttttttatgt ggtcttgatt aaatcccatt ttacttcctt gcaggttaac 71820
   aaccattatt gagtacctat tgatatgtgt ggtgtactga gttaactaga acatgtcccc 71880
   tggtctgtgt tctagaccat cttgctggga aaaaggcaga cccaaagcat attttggtgg 71940
   gggcccatgg acagtgatgt gatagaggtg tccgctgagg tggtcaggga aggctgcttg 72000
   cagtaggtgg ccgtgcacgg aaagtttgca gaatgagcag gtgttagttc cagctggaga 72060
   tgactgccgg ctgtgccctt ggtacctgct ttctggaggg aagttttaag acgtgtgcat 72120
   acttgaccca gcagttgtat acatggagaa atttactttg cagcaactct caaaacaagc 72180
   gtgtaaagat gtgtataggt agttgtgttt gttgtggcat tgtttgtagt agtgaaaaat 72240
   tagagacagg ccaatgatat aaccagggac ctgatcaatt atgttctctc ccggtgttgg 72300
   gatattctgt agctcttaaa gaatgagatc tgggtgtact gatgtggcca gacattgcaa 72360
   ttgcagtaca tgagaaggca aatcatacag tagtgtgtac accagtgagt cctccagcca 72420
   gataaatcct cacagtgacc agtcgcccag gcaccttgtg aaccctaccc tgggtgtggg 72480
   tgctatctga agtacctggg ggagggggtg acaagtggac ttcaggctga tgtgggccct 72540
   ggcctggccc tccctccaag cagagggggc tggctcgctg gaaggttaac atcatccaac 72600
   tctgtctaca cgtggcttgt tttttcctag aattcctgcc acaatagcag catccttgcc 72660
   attcattttc tccaaagtga gtaacccatc tctgccctct gattcctcag catgagtcaa 72720
   gacactgaag ttagaagtcg ggtcgtgggg ggaagtcttc gaggtgccca ggctgcctcc 72780
   ccagccaaag gggagccgtc actgcccgag aaggacgagg accatgcact gagttactgg 72840
   aagcccttcc tggtcaacat gtgcgtggct acggtcctca cggccggcgc ttacctctgc 72900
   tacagggtat gtttccactg acagacgcgc tggcgagatg ctcgtgtgca gagagcactg 72960
   gccgctagcc cgatggtagg attcagttct gtggtgcatc tgagccagtc tcagaagaaa 73020
   cagatcaaag gtttttaaag tctggaactg tggaagggct aacaagagaa ttaaggatcg 73080
   atgcactggg gttttaagga gccctctggt cccaagaata taagagtcta atctcagggc 73140
   cttaacctat tcaggagtaa gtagagaaaa tgccaaatac gtctgtttct ctctctcttt 73200
   ttttttttat tcctttgttt ttggaaaaaa atagagttac aacacattgt tgtttttaac 73260
   ctttataaaa agcagctttt tgttatttct ggaacaaaaa aaaacaaagt aggcacttat 73320
   gaaactttct cataccctta ggtgatgtaa tcagccatat aatttatatt tgatttccca 73380
   gggaaggaat cccaaacttt tacgaatgta aactcccttg gagaagaggg ttaggacgct 73440
   gttgcgctca agcccccctc agctgtgtgc acactgagcc aggacagggt ctttgagctt 73500
   tcccactata agaagaacag caacaaaagg ccgtctagaa aaacagaacc tgcctctgct 73560
   tctgctcagg gtgtccccgc tgggtttcca ttgtcctttc tccattgctc cctcctgtga 73620
   cagccatctt gctcatgtac cagccctcat caccccatcc ccataaatgg gtgtcctcga 73680
   ggcctctgcc tgggggtcag aggtcaccac agggtggcca ttggcatgtc aacccgctgt 73740
   taattcagag aagtgggctc cacctcattg ggagaagtgc catttcagca gaaattcaca 73800
   cgttagacgt gtgttgctgt taagtaaggg gaagagagag gactagcctc agagctctgg 73860
   ccatggaaat gacctcctaa gactttttcg tggttttaaa tattttacct ctttccaggt 73920
   ggcatctgag tacatcagat ggttttgcaa aatgcaaaca attttttcct tggggatgat 73980
   ttttggggag agggggctac tgtaaaaaat aaaaccaaaa ccccctttgc tccctcggag 74040
   gttgaagttg ccggggggtg tggccggggt catgcatgag gcgacagctc tgcaggtgcg 74100
   ggtctgggct catctgaact gtttggtttc attccagttc ctgttcaaca gcaacacata 74160
   gcctgaccct cctccactcc acctccaccc actgtccgcc tctgcccgca gagcccacgc 74220
   ccgactagca ggcatgccgc ggtaggtaag ggccgccgga ccgcgtagag agccgggccc 74280
   cggacggacg ttggttctgc actaaaaccc atcttccccg gatgtgtgtc tcacccctca 74340
   tccttttact ttttgcccct tccactttga gtaccaaatc cacaagccat tttttgagga 74400
   gagtgaaaga gagtaccatg ctggcggcgc agagggaagg ggcctacacc cgtcttgggg 74460
   ctcgccccac ccagggctcc ctcctggagc atcccaggcg ggcggcacgc caacagcccc 74520
   ccccttgaat ctgcagggag caactctcca ctccatattt atttaaacaa ttttttcccc 74580
   aaaggcatcc atagtgcact agcattttct tgaaccaata atgtattaaa attttttgat 74640
   gtcagccttg catcaagggc tttatcaaaa agtacaataa taaatcctca ggtagtactg 74700
   ggaatggaag gctttgccat gggcctgctg cgtcagacca gtactgggaa ggaggacggt 74760
   tgtaagcagt tgttatttag tgatattgtg ggtaacgtga gaagatagaa caatgctata 74820
   atatataatg aacacgtggg tatttaataa gaaacatgat gtgagattac tttgtcccgc 74880
   ttattctcct ccctgttatc tgctagatct agttctcaat cactgctccc ccgtgtgtat 74940
   tagaatgcat gtaaggtctt cttgtgtcct gatgaaaaat atgtgcttga aatgagaaac 75000
   tttgatctct gcttactaat gtgccccatg tccaagtcca acctgcctgt gcatgacctg 75060
   atcattacat ggctgtggtt cctaagcctg ttgctgaagt cattgtcgct cagcaatagg 75120
   gtgcagtttt ccaggaatag gcatttgcct aattcctggc atgacactct agtgacttcc 75180
   tggtgaggcc cagcctgtcc tggtacagca gggtcttgct gtaactcaga cattccaagg 75240
   gtatgggaag ccatattcac acctcacgct ctggacatga tttagggaag cagggacacc 75300
   ccccgccccc cacctttggg atcagcctcc gccattccaa gtcaacactc ttcttgagca 75360
   gaccgtgatt tggaagagag gcacctgctg gaaaccacac ttcttgaaac agcctgggtg 75420
   acggtccttt aggcagcctg ccgccgtctc tgtcccggtt caccttgccg agagaggcgc 75480
   gtctgcccca ccctcaaacc ctgtggggcc tgatggtgct cacgactctt cctgcaaagg 75540
   gaactgaaga cctccacatt aagtggcttt ttaacatgaa aaacacggca gctgtagctc 75600
   ccgagctact ctcttgccag cattttcaca ttttgccttt ctcgtggtag aagccagtac 75660
   agagaaattc tgtggtggga acattcgagg tgtcaccctg cagagctatg gtgaggtgtg 75720
   gataaggctt aggtgccagg ctgtaagcat tctgagctgg gcttgttgtt tttaagtcct 75780
   gtatatgtat gtagtagttt gggtgtgtat atatagtagc atttcaaaat ggacgtactg 75840
   gtttaacctc ctatccttgg agagcagctg gctctccacc ttgttacaca ttatgttaga 75900
   gaggtagcga gctgctctgc tatatgcctt aagccaatat ttactcatca ggtcattatt 75960
   ttttacaatg gccatggaat aaaccatttt tacaaaaata aaaacaaaaa aagcaaggtg 76020
   ttttggtata ataccttttc aggtgtgtgt ggatacgtgg ctgcatgacc gggtgggtgg 76080
   gggggagtgt ctcagggtct tctgtgacct cacagaactg tcagactgta cagttttcca 76140
   acttgccata ttcatgatgg gtttgcattt tagctgcaac aataaaattt ttttctaaag 76200
   aacatgaatt tggggtgctt cccatttttt tctttgctta atagagctaa accaggatga 76260
   gtaactcctg tttctttcta tccctgctga tgtgaaacag atgttgtcaa tcagctgggg 76320
   ttagagtttt ccacttctaa gaattaacct cagcatccct gcattgccag caccctcagg 76380
   ctggagcgct ttccttgact gtgagcttgt tgaacacctt aggcctcagc ccatttcctt 76440
   cccaaattga cgctttgcct gtgtagggcc ctcagataac ttaacaaact taccagtgtt 76500
   gtttgaagaa cagtgttttg agttgtaatc tcaaaaccat atcccttacc caattacctg 76560
   taagacacaa tggttaccac atctcagtac gtaaagtcca cttgatatag aattgactta 76620
   gaaataagac agattagtat agtttttcat ttgtgtacaa aattaaacaa tgtaaattcc 76680
   ccccaaagtg atttttttga ctttttgaag taattttgga cttgcaaaat gttgccaaaa 76740
   tagtacgaag agttccccag taccctcgaa gtttcctcga ctgtttcaaa gctggctgca 76800
   ggcccaggct catgagactg ggaagaggac aggctgtggt catgtggacc cacaggggcc 76860
   tggggctgca gaagtcagtg tggcttccac catttcaggt ataaaaaagg gcatctaagc 76920
   tttcaagaag agggaggatg ctctagggca gcggtcccca accttttctg gcaccaggaa 76980
   ccggttccat ggaagacaat tttttcacag gcctgggggt ggtgagggat ggttttggga 77040
   tagaaacttc cacctcagat catcaggcat cagattctca taaggagctt gcaacctgat 77100
   ctcttgcaca cattcagttc acaatagggt tcacgctcct aagagaacct gatgctgcag 77160
   ctgatctaac aggagatgga gctcaggtgg tcatgctcag tcgctcgcca ctcacctcct 77220
   gccatgcagt ccagttccta acaggcctca gaccagtacc ggtctgtggc ctgggggttg 77280
   aggacccctg ctctaggctg gtactgctga tgcttaaaaa gagagggttt gccagaaatc 77340
   agatgggaca aaagggcaaa ggccgtgcca cagagtgccc atatagggga gagcacgcct 77400
   ggagccttcg agagcatgca gagaagcctg gagactgcat ttaccggagc tgctgcctga 77460
   ggccaccctc caagtgtccc cacagcgcac cacaagacca caggagtgac ctcctcactg 77520
   gcaggtattt ggggaaacaa ctgctgtcta ctcttttggg taaaaagtga aacaccaata 77580
   gtttaattga aatttcagaa aattgaacat atgaacaagg caaataaata ctaagtaagt 77640
   taaaaacaca aaatatgtcc aggaagtatc gatgagaatg ttcaagttaa agttctccaa 77700
   tgccattgct acagcaacct caaaccctag gttctctctg cactattaac acagacatct 77760
   caggacatgg tttgcttttt tttaagactt aaataggaaa ctaatttttc tttctttaaa 77820
   gcaattgcgt tcttcagtga actctttctt taggccagtt gatggcttct tagcagttta 77880
   ttgacgagat cctagggtag cttccgaagc tgggttgatt gattgcattt gggtgcggat 77940
   ggccaaagtg agtggcccta ctgcctgtgc tgctcagggc tcctgggctg atgtggtggc 78000
   t 78001
<210> 13
   <211> 2160
   <212> DNA
   <213> Mus musculus
<400> 13
   ggcgccctgc tctcccggcg gggcggcgga gggggcgggc tggccggcgc acggtgatgt 60
   ggcgggactc tttgtgcact gcggcaggat acgcgcttgg gcgtcgggac gcggctgcgc 120
   tcagctctct cctctcggaa gctgcagcca tgatggaagt ttgagagttg agccgctgtg 180
   aggccaggcc cggcgcaggc gagggagatg agagacggcg gcggccacgg cccagagccc 240
   ctctcagcgc ctgtgagcag ccgcgggggc agcgccctcg gggagccggc cgggcggcgg 300
   cggcggcagc ggcggcgggc ctcgcctcct cgtcgtctgt tctaaccggg cagcttctga 360
   gcagcttcgg agagagacgg tggaagaagc cgtgggctcg agcgggagcc ggcgcaggct 420
   cggcggctgc acctcccgct cctggagcgg gggggagaag cggcggcggc ggccgcggct 480
   ccggggaggg ggtcggagtc gcctgtcacc attgccaggg ctgggaacgc cggagagttg 540
   ctctctcccc ttctcctgcc tccaacacgg cggcggcggc ggcggcacgt ccagggaccc 600
   gggccggtgt taagcctccc gtccgccgcc gccgcacccc ccctggcccg ggctccggag 660
   gccgccggag gaggcagccg ctgcgaggat tatccgtctt ctccccattc cgctgcctcg 720
   gctgccaggc ctctggctgc tgaggagaag caggcccagt ctctgcaacc atccagcagc 780
   cgccgcagca gccattaccc ggctgcggtc cagggccaag cggcagcaga gcgaggggca 840
   tcagcgaccg ccaagtccag agccatttcc atcctgcaga agaagcctcg ccaccagcag 900
   cttctgccat ctctctcctc ctttttcttc agccacaggc tcccagacat gacagccatc 960
   atcaaagaga tcgttagcag aaacaaaagg agatatcaag aggatggatt cgacttagac 1020
   ttgacctata tttatccaaa tattattgct atgggatttc ctgcagaaag acttgaaggt 1080
   gtatacagga acaatattga tgatgtagta aggtttttgg attcaaagca taaaaaccat 1140
   tacaagatat acaatctatg tgctgagaga cattatgaca ccgccaaatt taactgcaga 1200
   gttgcacagt atccttttga agaccataac ccaccacagc tagaacttat caaacccttc 1260
   tgtgaagatc ttgaccaatg gctaagtgaa gatgacaatc atgttgcagc aattcactgt 1320
   aaagctggaa agggacggac tggtgtaatg atttgtgcat atttattgca tcggggcaaa 1380
   tttttaaagg cacaagaggc cctagatttt tatggggaag taaggaccag agacaaaaag 1440
   ggagtcacaa ttcccagtca gaggcgctat gtatattatt atagctacct gctaaaaaat 1500
   cacctggatt acagacccgt ggcactgctg tttcacaaga tgatgtttga aactattcca 1560
   atgttcagtg gcggaacttg caatcctcag tttgtggtct gccagctaaa ggtgaagata 1620
   tattcctcca attcaggacc cacgcggcgg gaggacaagt tcatgtactt tgagttccct 1680
   cagccattgc ctgtgtgtgg tgatatcaaa gtagagttct tccacaaaca gaacaagatg 1740
   ctcaaaaagg acaaaatgtt tcacttttgg gtaaatacgt tcttcatacc aggaccagag 1800
   gaaacctcag aaaaagtgga aaatggaagt ctttgtgatc aggaaatcga tagcatttgc 1860
   agtatagagc gtgcagataa tgacaaggag tatcttgtac tcaccctaac aaaaaacgat 1920
   cttgacaaag caaacaaaga caaggccaac cgatacttct ctccaaattt taaggtgaaa 1980
   ctatacttta caaaaacagt agaggagcca tcaaatccag aggctagcag ttcaacttct 2040
   gtgactccag atgttagtga caatgaacct gatcattata gatattctga caccactgac 2100
   tctgatccag agaatgaacc ttttgatgaa gatcagcatt cacaaattac aaaagtctga 2160
<210> 14
   <211> 5572
   <212> DNA
   <213> H. sapiens
<400> 14
   cctcccctcg cccggcgcgg tcccgtccgc ctctcgctcg cctcccgcct cccctcggtc 60
   ttccgaggcg cccgggctcc cggcgcggcg gcggaggggg cgggcaggcc ggcgggcggt 120
   gatgtggcgg gactctttat gcgctgcggc aggatacgcg ctcggcgctg ggacgcgact 180
   gcgctcagtt ctctcctctc ggaagctgca gccatgatgg aagtttgaga gttgagccgc 240
   tgtgaggcga ggccgggctc aggcgaggga gatgagagac ggcggcggcc gcggcccgga 300
   gcccctctca gcgcctgtga gcagccgcgg gggcagcgcc ctcggggagc cggccggcct 360
   gcggcggcgg cagcggcggc gtttctcgcc tcctcttcgt cttttctaac cgtgcagcct 420
   cttcctcggc ttctcctgaa agggaaggtg gaagccgtgg gctcgggcgg gagccggctg 480
   aggcgcggcg gcggcggcgg cacctcccgc tcctggagcg ggggggagaa gcggcggcgg 540
   cggcggccgc ggcggctgca gctccaggga gggggtctga gtcgcctgtc accatttcca 600
   gggctgggaa cgccggagag ttggtctctc cccttctact gcctccaaca cggcggcggc 660
   ggcggcggca catccaggga cccgggccgg ttttaaacct cccgtccgcc gccgccgcac 720
   cccccgtggc ccgggctccg gaggccgccg gcggaggcag ccgttcggag gattattcgt 780
   cttctcccca ttccgctgcc gccgctgcca ggcctctggc tgctgaggag aagcaggccc 840
   agtcgctgca accatccagc agccgccgca gcagccatta cccggctgcg gtccagagcc 900
   aagcggcggc agagcgaggg gcatcagcta ccgccaagtc cagagccatt tccatcctgc 960
   agaagaagcc ccgccaccag cagcttctgc catctctctc ctcctttttc ttcagccaca 1020
   ggctcccaga catgacagcc atcatcaaag agatcgttag cagaaacaaa aggagatatc 1080
   aagaggatgg attcgactta gacttgacct atatttatcc aaacattatt gctatgggat 1140
   ttcctgcaga aagacttgaa ggcgtataca ggaacaatat tgatgatgta gtaaggtttt 1200
   tggattcaaa gcataaaaac cattacaaga tatacaatct ttgtgctgaa agacattatg 1260
   acaccgccaa atttaattgc agagttgcac aatatccttt tgaagaccat aacccaccac 1320
   agctagaact tatcaaaccc ttttgtgaag atcttgacca atggctaagt gaagatgaca 1380
   atcatgttgc agcaattcac tgtaaagctg gaaagggacg aactggtgta atgatatgtg 1440
   catatttatt acatcggggc aaatttttaa aggcacaaga ggccctagat ttctatgggg 1500
   aagtaaggac cagagacaaa aagggagtaa ctattcccag tcagaggcgc tatgtgtatt 1560
   attatagcta cctgttaaag aatcatctgg attatagacc agtggcactg ttgtttcaca 1620
   agatgatgtt tgaaactatt ccaatgttca gtggcggaac ttgcaatcct cagtttgtgg 1680
   tctgccagct aaaggtgaag atatattcct ccaattcagg acccacacga cgggaagaca 1740
   agttcatgta ctttgagttc cctcagccgt tacctgtgtg tggtgatatc aaagtagagt 1800
   tcttccacaa acagaacaag atgctaaaaa aggacaaaat gtttcacttt tgggtaaata 1860
   cattcttcat accaggacca gaggaaacct cagaaaaagt agaaaatgga agtctatgtg 1920
   atcaagaaat cgatagcatt tgcagtatag agcgtgcaga taatgacaag gaatatctag 1980
   tacttacttt aacaaaaaat gatcttgaca aagcaaataa agacaaagcc aaccgatact 2040
   tttctccaaa ttttaaggtg aagctgtact tcacaaaaac agtagaggag ccgtcaaatc 2100
   cagaggctag cagttcaact tctgtaacac cagatgttag tgacaatgaa cctgatcatt 2160
   atagatattc tgacaccact gactctgatc cagagaatga accttttgat gaagatcagc 2220
   atacacaaat tacaaaagtc tgaatttttt tttatcaaga gggataaaac accatgaaaa 2280
   taaacttgaa taaactgaaa atggaccttt ttttttttaa tggcaatagg acattgtgtc 2340
   agattaccag ttataggaac aattctcttt tcctgaccaa tcttgtttta ccctatacat 2400
   ccacagggtt ttgacacttg ttgtccagtt gaaaaaaggt tgtgtagctg tgtcatgtat 2460
   ataccttttt gtgtcaaaag gacatttaaa attcaattag gattaataaa gatggcactt 2520
   tcccgtttta ttccagtttt ataaaaagtg gagacagact gatgtgtata cgtaggaatt 2580
   ttttcctttt gtgttctgtc accaactgaa gtggctaaag agctttgtga tatactggtt 2640
   cacatcctac ccctttgcac ttgtggcaac agataagttt gcagttggct aagagaggtt 2700
   tccgaagggt tttgctacat tctaatgcat gtattcgggt taggggaatg gagggaatgc 2760
   tcagaaagga aataatttta tgctggactc tggaccatat accatctcca gctatttaca 2820
   cacacctttc tttagcatgc tacagttatt aatctggaca ttcgaggaat tggccgctgt 2880
   cactgcttgt tgtttgcgca ttttttttta aagcatattg gtgctagaaa aggcagctaa 2940
   aggaagtgaa tctgtattgg ggtacaggaa tgaaccttct gcaacatctt aagatccaca 3000
   aatgaaggga tataaaaata atgtcatagg taagaaacac agcaacaatg acttaaccat 3060
   ataaatgtgg aggctatcaa caaagaatgg gcttgaaaca ttataaaaat tgacaatgat 3120
   ttattaaata tgttttctca attgtaacga cttctccatc tcctgtgtaa tcaaggccag 3180
   tgctaaaatt cagatgctgt tagtacctac atcagtcaac aacttacact tattttacta 3240
   gttttcaatc ataatacctg ctgtggatgc ttcatgtgct gcctgcaagc ttcttttttc 3300
   tcattaaata taaaatattt tgtaatgctg cacagaaatt ttcaatttga gattctacag 3360
   taagcgtttt ttttctttga agatttatga tgcacttatt caatagctgt cagccgttcc 3420
   acccttttga ccttacacat tctattacaa tgaattttgc agttttgcac attttttaaa 3480
   tgtcattaac tgttagggaa ttttacttga atactgaata catataatgt ttatattaaa 3540
   aaggacattt gtgttaaaaa ggaaattaga gttgcagtaa actttcaatg ctgcacacaa 3600
   aaaaaagaca tttgattttt cagtagaaat tgtcctacat gtgctttatt gatttgctat 3660
   tgaaagaata gggttttttt tttttttttt tttttttttt ttaaatgtgc agtgttgaat 3720
   catttcttca tagtgctccc ccgagttggg actagggctt caatttcact tcttaaaaaa 3780
   aatcatcata tatttgatat gcccagactg catacgattt taagcggagt acaactacta 3840
   ttgtaaagct aatgtgaaga tattattaaa aaggtttttt tttccagaaa tttggtgtct 3900
   tcaaattata ccttcacctt gacatttgaa tatccagcca ttttgtttct taatggtata 3960
   aaattccatt ttcaataact tattggtgct gaaattgttc actagctgtg gtctgaccta 4020
   gttaatttac aaatacagat tgaataggac ctactagagc agcatttata gagtttgatg 4080
   gcaaatagat taggcagaac ttcatctaaa atattcttag taaataatgt tgacacgttt 4140
   tccatacctt gtcagtttca ttcaacaatt tttaaatttt taacaaagct cttaggattt 4200
   acacatttat atttaaacat tgatatatag agtattgatt gattgctcat aagttaaatt 4260
   ggtaaagtta gagacaacta ttctaacacc tcaccattga aatttatatg ccaccttgtc 4320
   tttcataaaa gctgaaaatt gttacctaaa atgaaaatca acttcatgtt ttgaagatag 4380
   ttataaatat tgttctttgt tacaatttcg ggcaccgcat attaaaacgt aactttattg 4440
   ttccaatatg taacatggag ggccaggtca taaataatga cattataatg ggcttttgca 4500
   ctgttattat ttttcctttg gaatgtgaag gtctgaatga gggttttgat tttgaatgtt 4560
   tcaatgtttt tgagaagcct tgcttacatt ttatggtgta gtcattggaa atggaaaaat 4620
   ggcattatat atattatata tataaatata tattatacat actctcctta ctttatttca 4680
   gttaccatcc ccatagaatt tgacaagaat tgctatgact gaaaggtttt cgagtcctaa 4740
   ttaaaacttt atttatggca gtattcataa ttagcctgaa atgcattctg taggtaatct 4800
   ctgagtttct ggaatatttt cttagacttt ttggatgtgc agcagcttac atgtctgaag 4860
   ttacttgaag gcatcacttt taagaaagct tacagttggg ccctgtacca tcccaagtcc 4920
   tttgtagctc ctcttgaaca tgtttgccat acttttaaaa gggtagttga ataaatagca 4980
   tcaccattct ttgctgtggc acaggttata aacttaagtg gagtttaccg gcagcatcaa 5040
   atgtttcagc tttaaaaaat aaaagtaggg tacaagttta atgtttagtt ctagaaattt 5100
   tgtgcaatat gttcataacg atggctgtgg ttgccacaaa gtgcctcgtt tacctttaaa 5160
   tactgttaat gtgtcatgca tgcagatgga aggggtggaa ctgtgcacta aagtgggggc 5220
   tttaactgta gtatttggca gagttgcctt ctacctgcca gttcaaaagt tcaacctgtt 5280
   ttcatataga atatatatac taaaaaattt cagtctgtta aacagcctta ctctgattca 5340
   gcctcttcag atactcttgt gctgtgcagc agtggctctg tgtgtaaatg ctatgcactg 5400
   aggatacaca aaaataccaa tatgatgtgt acaggataat gcctcatccc aatcagatgt 5460
   ccatttgtta ttgtgtttgt taacaaccct ttatctctta gtgttataaa ctccacttaa 5520
   aactgattaa agtctcattc ttgtcaaaaa aaaaaaaaaa aaaaaaaaaa aa         5572
<210> 15
   <211> 103886
   <212> DNA
   <213> H. sapiens
<400> 15
   cccttgcagg gaccgtccct gcatttccct ctacactgag cagcgtggtc acctggtcct 60
   tttcacctgt gcacaggtaa cctcagactc gagtcagtga cactgctcaa cgcacccatc 120
   tcagctttca tcatcagtcc tccacccccg ccccacaaca gcctaccctg cctccggctg 180
   ggtttctggg cagaggccga ggcttagctc gttatcctcg cctcgcgttg ctgcaaaagc 240
   cgcagcaagt gcagctgcag gctggcggct gggaaccggc ccgagcaagc cccaggcagc 300
   tacactgggc atgctcagta gagcctgcgg cttggggact ctgcgctcgc acccagagct 360
   accgctctgc cccctcctac cgccccctgc cctgccctgc cctcccctcg cccggcgcgg 420
   tcccgtccgc ctctcgctcg cctcccgcct cccctcggtc ttccgaggcg cccgggctcc 480
   cggcgcggcg gcggaggggg cgggcaggcc ggcgggcggt gatgtggcgg gactctttat 540
   gcgctgcggc aggatacgcg ctcggcgctg ggacgcgact gcgctcagtt ctctcctctc 600
   ggaagctgca gccatgatgg aagtttgaga gttgagccgc tgtgaggcga ggccgggctc 660
   aggcgaggga gatgagagac ggcggcggcc gcggcccgga gcccctctca gcgcctgtga 720
   gcagccgcgg gggcagcgcc ctcggggagc cggccggcct gcggcggcgg cagcggcggc 780
   gtttctcgcc tcctcttcgt cttttctaac cgtgcagcct cttcctcggc ttctcctgaa 840
   agggaaggtg gaagccgtgg gctcgggcgg gagccggctg aggcgcggcg gcggcggcgg 900
   cacctcccgc tcctggagcg ggggggagaa gcggcggcgg cggcggccgc ggcggctgca 960
   gctccaggga gggggtctga gtcgcctgtc accatttcca gggctgggaa cgccggagag 1020
   ttggtctctc cccttctact gcctccaaca cggcggcggc ggcggcggca catccaggga 1080
   cccgggccgg ttttaaacct cccgtccgcc gccgccgcac cccccgtggc ccgggctccg 1140
   gaggccgccg gcggaggcag ccgttcggag gattattcgt cttctcccca ttccgctgcc 1200
   gccgctgcca ggcctctggc tgctgaggag aagcaggccc agtcgctgca accatccagc 1260
   agccgccgca gcagccatta cccggctgcg gtccagagcc aagcggcggc agagcgaggg 1320
   gcatcagcta ccgccaagtc cagagccatt tccatcctgc agaagaagcc ccgccaccag 1380
   cagcttctgc catctctctc ctcctttttc ttcagccaca ggctcccaga catgacagcc 1440
   atcatcaaag agatcgttag cagaaacaaa aggagatatc aagaggatgg attcgactta 1500
   gacttgacct gtatccattt ctgcggctgc tcctctttac ctttctgtca ctctcttaga 1560
   acgtgggagt agacggatgc gaaaatgtcc gtagtttggg tgactataac atttaaccct 1620
   ggtcaggttg ctaggtcata tattttgtgt ttcctttctg tgtattcaac ctagggtgtg 1680
   tttggctaga cggaactctt gcctggttgc aagtgtcaag ccaccgattg ctttcttagg 1740
   ctatctatat ggtctcttcc tgagggctat tgtccgttaa tacagaatac agtacactgt 1800
   tagtggatta gcgagctcgg taatccggtc tcctaaatga acaaaaaagt agacgctttt 1860
   tgaggttgag catatttcga ttaaatcttg gcttaggccc tagatcaagg gtttagatca 1920
   gaataaaatg aaaattagtg ttgcacgtac gcatattgca tcagaatctt gcagtgattg 1980
   ttttagtttc ctgagttgca ttgatagatt cttttaaaat atgactgatt tgcataactt 2040
   tagaagcaga atcattttca gtatatatgg tgcacattga gggcaaaaag tagttttgtt 2100
   aatgtttaaa cttaagttac ctacaacttt gaactgtatg tagaagtttt gtagtttgaa 2160
   gtcaatagtg ccataatata ccttataagg cgttcttact agatctttgt tatatttacc 2220
   tttttctctc cctatggggt gatgtaggat agtgcttgaa atttgcactt cagtagcatt 2280
   taatgttcag tgctcttgtc ataaacatag aatggatatt gagtagtttc tgatcccaga 2340
   tggtaatgtg taggttcaag ggtattgtgt gtagcaagtg aagattgcag aaataaaact 2400
   tcagttcatg cttgaaattt aagtattgtt gtgatgccag aattgctgct caccgttttt 2460
   aggtttcagg tcctctgaca ccttttggta tcgttaattt tactgatttg tgtagaatgt 2520
   cagttgtatt ttaccagcta atatctagaa atgctggcaa gaggggttta ctccagcttt 2580
   agattgtagg tatgttagct tttttcatac agtgtattaa atttactgag tcagcttgct 2640
   gaataagaca gaagcccaag aattttaaca gtgtgtagct ttagttgtct aaaagttagg 2700
   ccttcgggct tcaaaagtta gtggtcatcg aaaagcatta atctttgcag tttcaggtac 2760
   aacacattgg ttttgattag ggatggggat ggggccctct ttttgcagaa tggggaaagt 2820
   attgacagga attgagagct attggtaggc cagtgtataa ggtatgtgaa aacagaatta 2880
   agttattggt ctgaagtgac tgaagcattt aggctctatc aaggcctaaa atttggtaat 2940
   atgagtttgg taatgcgaat tgtggcagtg gacaatattt agttaaaatt atgtaattgc 3000
   ataagtacta gcacagtatt tttaataaaa gttatttctt agcaaatgtc agttgcattt 3060
   tgtctaaagg tagagtgaca ctacagtgtc tatatgtcct gctaaaaatt gtggggaata 3120
   ttttttttta agacagcgtt tatatcggga gaggttttat tccgttggat tatgttagct 3180
   gcatataaat gtgcacagtt aattttgccc aagtttttgt tttgaaatga atgtaaaact 3240
   tactgaagaa gtagcttccc aaaatttagt tttctgttaa gccaaaaata ttattttaaa 3300
   agagtatttg caaattttga agttgacatt aattgagaat gttactaagg ctaaactgga 3360
   cccgcttgcc cagaagataa ttatggaaaa attcttttgt gacttccaaa gcagtctact 3420
   attagcatga attactgaca gtcatccaaa tatataggaa caaaaaatta aatgtttatg 3480
   taactttgaa aaaaaagcct ttgaagaaaa taattgaatg ctgtctggga gacagatttc 3540
   tttcagcact taaagtacat aacacactac ttttactttt cccacttgat tttaaattat 3600
   cagggttatt aagaccttaa aattatttta ccaggttttt acatgtgagc tgtgacatga 3660
   ctggcatttt ctttgatttc agcgtatgtt ggtctctaca catgaaattt gtgtgactta 3720
   aaactttctc taaaactgta cttttagtta tgatatgcat agaaagcagt atcaaatatt 3780
   gcgtcaaatg actaataaca cttaatttct agagttgtgg ttttattgag ccaaaagttg 3840
   atatgaaaaa aagtcagtaa ggaaagtcag tgaagtgctt gctttttgga taattgcact 3900
   cccaataatt ttgatattcc aacgtacttg gtttgcttgt tttcacgtta atgttttctg 3960
   tttattggag tgggaaggca ttaaaattgt cattgaagac tttgtctttg acatgttgta 4020
   gtatttattt cagtaactaa cctgtgaaaa gttaaattcc tttatgaaag tagtgattgg 4080
   agtattttta tctgataaag aaagattaat aatgaagcca tttctccgag gaaaattgag 4140
   gacaataatt cagttttaaa atattatcgc aaaattaaat tattctaaaa atttgttagt 4200
   agggttatat gcttaatatt agtctgaaat atagtgctga atttgagact atagaaaaat 4260
   taagtgtatt tagggtatgt ggaaacgtta ggcttctgtt gtatttttat tgtttggtag 4320
   atttgcctct tttcaaataa atgttcacag ggaatacttt taacttgtag agagtacagt 4380
   gactattgaa gttacctaaa ttacctcaag gtaagtgatt actgaaatta atcatgaggg 4440
   ttttaaaaag tattctattc acaacattta ttttacattg ttttgtatct gctaagttat 4500
   atttcctgaa aaacatgact ggaccaccta attgctgtat gataacttac aaacttcatt 4560
   tttcatagtg cttattcaag tgtaaagcac aactgaaagg agtaatgtac agtttatatg 4620
   aggaaaaagg aattttattg ctgccagtgt aaaagtttgc acagcagtat agtcatcaat 4680
   gcagatttac attgcttata atatactaag taaatactaa atgattaaag ataataaaat 4740
   atggtgaggt ataaccacct tcattttaaa cttagtttta gaagatagta aagaaagatt 4800
   cctttattac ctttttagaa ttttattttt aataacatgg gaaaggcaac tggtgatatt 4860
   ttaatttttg tatggaacag tgcatctgct ttctcatagc cacataaaat acataaactt 4920
   cttagtgtta tgaaatggct tactttttgg aagtgaagaa gtcttcaatt cttattttct 4980
   aatgttattt tgaaatttgc ctccatttgc tgtttgttca tttggtgata gcgcaacact 5040
   taaaaaaata ttttaagccg cttctgaagt aatcacttca gtgactttta atggaggagt 5100
   atttgttatg ggaaattcac ttcacaaagt tttaacatta attcacttga agtaaacgtg 5160
   ctatttttaa attttcatct caatctttta agtaagacga aagcttagga aatcactttt 5220
   attattgata ttgtgtgtga cttcagagtt tgtaaagaga attgtagaag tgttgcatgc 5280
   atatgacaat tttctgctta attgaaatgt gaggcctctg ccatactaca aggatttagc 5340
   ttccagaaaa tgtaatatta acatagctta agaaatgtat tttttttttt ctgtagaaac 5400
   cgttgggtta aacaaacagt tcagaagttt tattacatgg aacccattag ttcttaatct 5460
   tgttaccttt ttcttcattt tttctgttaa acttgatttt cacagtcagc attgaagaat 5520
   tcatcttgtg gcctgaattc attaagaaaa gatgttagga tttgttctga agatagtgac 5580
   ttaggaaatt tgtgagactg gggtcagtca gttctgtttt acaattgctt tctatttggt 5640
   agctttgaaa ttaatttagt tgcttatcag agagaataat gttgaggtta gactaacctt 5700
   aaattggtaa ggctttgctg agcaaactga taactgtaag tcttttatag ggtgcattac 5760
   tgccacatat acgttcttcc ataggtggtt aaaagtatat tggtctgtgt ttggtgattc 5820
   tctttgtaca tattgagtat atgcattcac taatgtaaaa taatttgcca agaaaggtga 5880
   aattagtata ttgtacttga ctattcacct ttcccttagt tttttgaatt tttttcattg 5940
   gttgcagagg aagtattagc aatttaattc ttttaaaata atttgcactg gaataaataa 6000
   gtatcggcaa atataagaag agtaacataa tttaagggtg aattaatttt atttgggaag 6060
   ttttagttct gtatagttaa ggcagattct tcatttgcaa cagttgacat tgggacatgt 6120
   gtgaacatct tcaaggtatt aggacatctt caaggcatta ctttttggca gtgttgagaa 6180
   tttttttttt tttttttttt ttttttgaga tggaatctcg ctctatcgcc cagtctgggg 6240
   tgcagtggca ggatctcggc tcactggaac ctctgcctcc caggttcagg cgattctcct 6300
   gcctgaacct cccaagcagc tgggattata ggtgcatgcc accacgccca gatagttttt 6360
   gtacttttag tagagatggg gttttaccac cttggccagg ctggtctcaa actccagacc 6420
   tcaagtgatc cgcctgcctc ggcctcccaa aatgctggga ttacaggcgt gagccactgc 6480
   gcctggccag tgttgagaat attgagagat ggatattgta gctgtacctg ccatcaaaag 6540
   aattttcttg acctccacat agtgtgaaaa agaagactgt ttacacatta tattttaagt 6600
   aattatacac ataattatta tcagtactca ccacttcaaa tatgaacagt gaatctaacc 6660
   agtgtttgat ttctctgtgt gtgtatgtgt atacaaagtt agcaaacctt ttatcttaat 6720
   atttattaaa aaacgaattt ttgtttcttt aaagaaaaga ctaccttaga gaatattgtt 6780
   ctatagtttt taaatatggt cagatctatt ttaaattatg ttaaaatttt gagtattacg 6840
   tttatctata cttttaagca tatatacatt gtttcatttt agattttagg gaggcagtgt 6900
   gggctctgga gccagactgc ttgttttgta atcctggatc ttccatttag tagctggatg 6960
   actttgagta ggttatttag attttctcaa tctattttat ctgtaaaatg gggatgataa 7020
   tggaacctac cgcatacgtt tatcttgaat agtaagtgag ataataataa gtaatttcat 7080
   ttagcatagt acctgccaca ttgtaaatac ttaaatggta gctactgctc tgaaaaactg 7140
   taatttcagg ttatgtatgt agggaaatta tttgtatttt catttatggt gtatgattgt 7200
   aactgaattt cctcagtttg ggccatgtta ggattttgtt tcaagttata agtgttttta 7260
   aaaataaggg tattccttta ggaagtctgg gtatgacatg tctgtgattt tgctggttca 7320
   tcacaaatgg gaaataaatc tctgctaact caaactgttg accaaagtaa aattaattat 7380
   gccaatcaaa aactatttgc tttaaaatat aaaaggcaaa aacttcctat tagcataatg 7440
   aagtagaatt tttaaacttt gttataatct taaattttct ttagtgttga agataggtca 7500
   acttaactat catacatttt tattcacata aagtaaactc tgcctcaaat gtaataaact 7560
   taatatgagt tatgtaaact ttggtcaata gaggtatatt ttttagcatt tccttttgaa 7620
   aatttcagcc ttttgaggga gtcttgcaac tgaatgtcaa gttacattta ttacaataaa 7680
   atggacactt aatataatct gtaatgcatt aacataatat gggaactttt aaagtattca 7740
   gtctctgtat tattgagtcc tatttccaca tttggccagg attctcaata tgatttaggc 7800
   ccaagacgtg ggaagaaaga agtaaagaac taaaggattt ttttcttcat ttttttaatt 7860
   gaatatgggg aaagatggaa taagcttatc tgtccagtaa aggccattat gtgtacatag 7920
   ggattattat ttttcccccc cttgggctgt actgatttcc cagatgtacc acagcactct 7980
   tagtagtgaa gcacttgact tctagtgagt ggattttttg tgtgtgtgtt ttatattgca 8040
   gagtgaatac actctgtctg atactatgtg actttctgat tatgtgattt ttatgcattt 8100
   tatgtgtttt gtaaactagc tgtatttttg gtccatgtct aggttgtaga attgaattgt 8160
   gcattttggc atctgagcac agctgagttt tctaaatcaa tctctctcct tgcacctagt 8220
   ttttgcttta gatcactacc taagacttac tgttgattta atattagagc acttaagcat 8280
   agctttgact tttatttcct ttgatttttg tagattttca ggctgaagta caataaggtt 8340
   ctctgttctt tactagtaat tgcaaagatt gtattctgtg aattttattt gtttaatact 8400
   tttgatcttt tgaagaggat gtaattattt aaggtattat gaaatgcatt gtgatttgaa 8460
   ttagatactc tttggagatg gagttttgct gttgttgccc aggctggagt gcaatggtgt 8520
   gatctcggct caccacagcc tccgcctcct gggttcaagc aattctcttg ccgcagcctc 8580
   ccaagtagct ggaattacag gcatgcgcca ccacgcccgg ctaattttat attttttttt 8640
   tcagtagaga tggggggttt ctccatgttg gtcaggctgg cctcgaactc ttgacctcag 8700
   gtaatctgcc tgtctcagcc tgctaaagtg ctgagattac aggcatgagc cactgcgccc 8760
   ggcctcagat actcttttaa ttagatgcgt ttaaaaattt aacccaccat tgctggcatg 8820
   aatagatgta tttttagagt gattcataaa tatcgtatac atgtttaaag ttacaaactt 8880
   tttgcttatt tcaaaatgca ggattctttt ccatttaaaa ttccctctct ttgtgagact 8940
   tctttttgag tattctggtt actctaaact gattggagat gaaattagat agaattgaaa 9000
   actgtacttt taaaatgaaa ttttggggat gtcattaagc ttgatttttt aggttttttt 9060
   tttagtgtgt attataaatt attttacact gattgtcagc gataaaatgg aatgcctggg 9120
   attttttaaa atttatttta ttcattttta taaggtaaaa acagtgtttt gctaggctta 9180
   atttgaccat gttgtaaaat ttattgtata ccttgaaaga atcatttatg aaagatactg 9240
   aattagctaa tatatactct gtcttatgta gtttttgatt aacaatacac tttttaaatc 9300
   attagctcat ttgattttgc aaagaagaac aggtaaccta agaggcagac agaacaggca 9360
   ttacttttat ttttctttct tttttatttt atttatttat ttatttattt attttttgca 9420
   gcttaggaat tgtagctcca gtggaatcag tatcttgtta atggctagtg aaagactgag 9480
   tctgaagaag gatgcaggac ttttttggca cttggtgcag tatttttccc attatgttac 9540
   atgagtggtt cttaaacttc agtgtgttag aacaacctga agggcttatt aagctatgga 9600
   ttgcttactc caccctcaga gtttctgatt cagtaggtct ggattgggac ctgagaattt 9660
   ttatttctta gaagttttca agtgatgctg atgctggtgc tctggggatc acactttgag 9720
   gaccaccaat gaacattatc tcccaccaag caaaccctta acatgttata ctcctttagg 9780
   ttattagaat ttatacatgc attatttcat ttgacctgta aactctaagt aactttgcat 9840
   ggaaaatgtt atcctgattt tatagacgag atagtgagtt tagaaaggca gtatggtgga 9900
   atggagcata gatttggagt tggctagacc taaagtccag attaaatctc tgctcaaggc 9960
   tgggcgtggt ggctcatgcc tgtaatccca gtgctttggg aggccagcgt tggcagattg 10020
   cttgagtctg gaagttcgag accagtctgg gcaacatagg cagaccctgt ctctacaaaa 10080
   aaaaatacaa aaattagtcg ggtgttatag tgcgcattgg tagtcccagc tactgaggag 10140
   gctgaggtgg gatcacctga gactgggact ttgaggctgc attgagctgt gattgggaca 10200
   ctgtactcct gcctgggtga cagagtgaga ccctctctca aaaataaata aataaataca 10260
   tccccgctca gccacttatc agttacgtag atacactgcc taaccttagt gaaccctgtt 10320
   tcgacaactc caaaatggga gtaaaaatcc taaacttgta cagtggtttt ttagttttgt 10380
   taaaagtaca ggtgaggttt ttttcagagt attggttgcc atctgagagt gatccccttt 10440
   cacctcctct aggactttta gcattttctg gagacatttt ggtggtcaca gctggggtgg 10500
   tagagtgtgc tattggctag ggcttgaagg ccagtgatgc tgcttaacat cctatatggc 10560
   acaagacccc tccccatcaa caaagaatta tctagcccaa aatgctgtgt aaaatgtctg 10620
   gtatataata agtataatat ttgatgaaaa tcagtacctt tgcccccagg tgtgatattt 10680
   aagaaggtca acttactaaa tcagtgatgg agttagtcct aacatctggg tgttctgact 10740
   gctgctaggc cagtattctt tatatgataa taagaacttt gtccacagaa gatatcccta 10800
   ataacaaaaa aggtttattt gaagaggact catgtgttct ttggctgatt gtgaaagtgt 10860
   tgctttgaac ttctgttaga aaaggttgaa gatgttttcc gtaagtgttt ttaatactgt 10920
   acgtagtatt cagaaggatg tttaattttt tttttaattt tgctagtagt ttttaaagta 10980
   atcctttttc ctttaattat gtagttgttg aactgttggg agttactttt ctcttactat 11040
   tttgttattt aatgtattct ttgaccttat gcttttttat tctaaagctg cttttattat 11100
   agtcagatat gatgaagtta aatgtacaat gtaaaattgc aaatttccaa cgagctatac 11160
   aaacttaaat atttctaagt aaagaaaata gggctgactc taaggttctt tgatccatgt 11220
   gttgcattct tttctaggcc ctaaatttgc tatgccagcc tgttgaatta aagtgcttta 11280
   tttatctaaa ttagaaactt gtattaaagt gaagttttag aaaaaaagaa acaaaatcgg 11340
   aatggagttt taggttagcc cagagatggg aagatgccaa gaaggtagct ttagtggatt 11400
   ctgaattttt tggttttgtt ttgtttttag ggcaggcaaa tgtaattaca aaagggttct 11460
   aggaatagat tgctgtgatt ttttttctgt ttgcatgatt ttacagtttg ctttgcctct 11520
   cacttttgaa tgcagaataa aatgtcaagg ccttattttt ttttaaattc ttaagaaatt 11580
   taagatttga ctgttaattc cttttgaaat atgggatatt ttgagatacc aattatttaa 11640
   gacaaatagg actcattgtt acaattcagt tgaataaggc ttatgatgtt tatttcagta 11700
   tatgaatgaa aactatgtgc ttattgtact taagaaaatt tcttttatta aaaacatgac 11760
   taaagagaat tttaaaaatc acccactgtc ctacttctct aaaacttaat gttttcatat 11820
   tagcttccag ttttgttcat atgcatatac tttaaaacct agttcatggt gaacttaaga 11880
   gggtgttctt tttaaaaaac aatttccatt gcactttgtc gttgccttaa ttaaatggtg 11940
   aaatcatcag aaatatttat tttcctatac ttatacattt attaagcttg tttccatttt 12000
   tttattttgt gattttttaa gtggatttaa gataacctaa acattagaga ggattttcat 12060
   ggttttgatt catgaaatca taatgttata caaacctaac tgaagtgtta gagccttgaa 12120
   gatttttccc ccgaattaca tatagtaact ctacttgtat ttaatactga aagcatattt 12180
   tacttattta agtgagacaa agtaaaattt agctgaatac tttagatcta tcatttcctt 12240
   ttcctgttgt aagaacatta cattgtgttg aaattaaagt ggatatagaa ggtaattaga 12300
   ataaactgcc acatcatttt tatagtaaag tggtaataac actattgctt tctgtttttt 12360
   taatcagaag gagtatgggc ttataatgat gttactgttc cctgaagcat attttgaatg 12420
   atacggttta tatttgcaca gttgcccagg taatcattgt gatattaatt gatcaatttg 12480
   ctatttattt gcgttttaaa tcagtactag tatttgtgct taaaaatttt gcatatgttt 12540
   tatcagattt aatttttaag tgtcagatac taaaacaaat aaccttaact ttattaaatt 12600
   ataatttttt atcatgaggt ggtattcatt tattcatata gttagaacaa aaaatattta 12660
   aaatattgag gtagaaacaa attagtctct ttttaattaa aagccagatt acttgttaga 12720
   gtaacatttt cccaaatgag gtaaaattgt tgcgactgtt aaacttaagg aaattttgat 12780
   ctaggtgtgg tatatacctt cttgtggggt gctaatgaaa acagggatgg caaaaatatt 12840
   ttgtttgtga gtgtatgcat ttatgctttt tgacaaccta agaaacactc ttacatctga 12900
   gtatctttca tggactagct gtaggaaatc tatataaaat agcttagtat actgaaagta 12960
   tgacatagtt ttacatatct agattgtggt tgtgattata tataatacta taaaatatgc 13020
   taacgtgctg cttaataata ctatttggat tttttttaat actgaaaagg tcacacagat 13080
   tgtgattatt gtgtagtgtc caagaactaa ggcctaccat ctgttactca aatgtatgaa 13140
   aaagttaaga taatttagtg atataagtgg ttttgacacc actgtttttg gaataatcta 13200
   attatgattt ttataaagac taatatcaaa ttttaaacgt ttgcaaaaat gaaacctaat 13260
   agttatactg ttatttatat ttttctatta caatacagat actggctgag aactaaagat 13320
   tgtgtaataa acgcctggcc ttcagtcatt tggttttttt tttccctcga ttgtttggat 13380
   agttaactgg acatcatgtt ttaacttgag aaattaagtt atacaagatt ttgatatttt 13440
   aaactagttt tcctaactgg ttgagatata taagaattta gtattacagg actcaatcag 13500
   ggaactgatt taataagaat ttcttaaaaa tttgtttaaa tatttttcaa gttcttttct 13560
   tcatcttcta caacttaatt cttgtctgta tgcaaatgag cttccccatt taaaattttg 13620
   ctgttgcatt ttaggccact atagaagttg tttctttaat tttcactcac aagaatttgg 13680
   tcttaccaaa ttgtgtaaat ctttaaaatt gtgtatttgg cttaatatta tagaatctga 13740
   ttgatttaat ctaccttgtt tcatttagta tgttgacatt ttcttgagaa atttgttatg 13800
   ccaaatgatt aacataataa tattttaagt ttagatatga ttttgaattt acattttcaa 13860
   atgcaacttt gtgtctgtgg cctttttttt tttttttttt ttacgagaaa catcttgcca 13920
   attttcagat taatctgtga ggaaagtaga ttggtttact agactcagtt tgtagacttt 13980
   ggtgagaact gaattggagg ctatgaaaaa aatacctttt gggcctttct gaatagacat 14040
   atatacataa attatatctc ttacattaag tgaggcacat atgtaggtga gatttttacc 14100
   tgaatattaa aagtttaaaa gtcgttacct attctgttta cttaatagta tttaaagggt 14160
   gtgagaggtg ttatgtgttt ctgtcccttg tttttattcc tatccctccc atctaactgt 14220
   tggtactctt atcttcccag gtattaaact tgtatgtttt aaaagcttat ttacttgttg 14280
   aaatggttaa cttaattagt tttttctttg aagtttcagc ctaaatattt tctgtttttt 14340
   tatatgtcct ttaaatatga aaattctaca gctaatcata attagtaatt gtactttttc 14400
   ccctattaca ataactggtt tcataataaa atggtatccc ttcaataaca agcatttata 14460
   gtagtttatt aaaactaagg gtgttatcta ttcaaaccaa gcaatgcaga cttactgttg 14520
   actctgttaa tatattttaa aattgcatat tactaaaatt taaaatatga ttttgactag 14580
   tattttggtg tatgttattt tagatatttt gattatgcac tacttaagaa tgaattgtca 14640
   agtatgatta taaagttgat ataatagtta accttcagtg ggaataggaa tcatttaata 14700
   ttgttagata tttgtattat tagaacaatc tcctatgatt cttactaata tagtaatgaa 14760
   tgacagacaa tatgttggct ttcatattta aaaattcaca tgcatttcta gtttatgttt 14820
   ttcttcgact aaaattctgc agcacttagg caaagctatt tttaccagtt ggaaaaaaag 14880
   taagtcattt ccaaccaatt ttcctggctt gtagtataga ataaagagac ttgattttat 14940
   tacattaaag ccaaatataa aatgatgcaa tctagcacac acttgtttgg aacttttctc 15000
   ttttaaatat tcagattaag aggacgttga aaggtaaatt tttttttttt tttgagacgg 15060
   agtctagctc tgtcacccag gctggagtgc actggcacga tttcggctta ctgcaagctc 15120
   tgcctcccag gttcatgcca ttctcctgcc tcagcctccc gagtagctgg gactacaggc 15180
   gcctaccacg gtggcgcccg gctaattttt tgtattttta gtagagacgg ggtttcaccg 15240
   tgttagccag gatggtctcg atctcctgac ctcgtgatct gcctgcctcg gcctcccaaa 15300
   gtgctgggat tacaggtaaa ttttgattgt tattagcaac tataaaagtt ttgcagttgg 15360
   cttattggaa aaagaaaacc tccttgccgg agacggagac gcatttgtat tagaactttg 15420
   ttttctgagt accttaccta tagtaggttt caaatattgg tgaattagtt gatggttagg 15480
   tctgcataat tactgcgtat ggaaattctg gaaccctatt ttttcaaaat gcagctaatg 15540
   ttgagagaat atgcactaaa tattactaga tctttgtttt tcaagatgct gatatccctt 15600
   aacatcttct gcactttacc tgtttgaata tcttttttgc tgtaaaaatt agtggcctta 15660
   tgtctttctg cataattata gagtagccaa aacctgtttt aggttaatca cctctggcaa 15720
   aataaatgat aaaagcatag cttttgtaag cagaatgata ttacagaagt taacttataa 15780
   atctaagtgt attaaagaca cttaggaaat ttatgataat gctgggtcag cattacagtt 15840
   ttaacttttt acagtttttc atatgctttt tttgtgattt tgctgtagaa aattaacagt 15900
   tggcatttgg cttagttcaa gtataatgct gttgacaagt atatctgaca cgtcattgaa 15960
   ctaataatat ttttgaaagc tgataggtaa gttatatcta ttttgtttca ttcgtcatta 16020
   gtgatcggtc ttagatgttt ttagcgagag caaaactgta gaggaatgtg tgtctgtgtg 16080
   tgtatatgtg tgtgtgtgtg tgtgtatttt aacagcagga gagttctgaa acaggaaacc 16140
   agtcttatca tattcatcca gagacctagg aagaaggtaa ttgtttggta tactcgttaa 16200
   aaccagttgg ttgggcaact taaattttta gaggatcaca gatgtaggct tgagcagttg 16260
   taatagatga tttctttttt tttctttctt ttttcttttt tttttgagat agagtctctc 16320
   tctgtcatcc aggctggagt gcggtggcgc gatcttggct tactgcaacc tctgcctccc 16380
   aggttcaggc gtttctcctg tctcagcctc ctgagtagct gggattacag gcgcatgctg 16440
   ccatgcccgg ctaatttttt gtattttagt agagacgggg tttcaccgtg ttccccaggc 16500
   tggtctcgaa ctcctgagct caggcaatct acccacctcg gcctcccaaa gtgctgggat 16560
   tacaggcgtg agccaccgtg tctggcgata gattatttca taattaacac ctgctatgaa 16620
   gaaaaattga ttaaaatagt tgagaagtct agtacactct cagctaatat actaaattat 16680
   actatggatt ttagagtatt gttaacatta tcagtgactt gatatcttcc tgaggttcta 16740
   atttgcttaa ctttaaataa ttggggttca gatcaccttg attgttccct taaagattaa 16800
   attttgtaaa actgtgtgta attttcctgt atctggtttg gatagcttta aaaatggttc 16860
   ttaagtttaa tgagttcaac tgggaaaaaa gttagttcta ttttagatgt tgtgtcactg 16920
   gaaattatgt ttccctgttt gttatatgca cattattaca aagttgtaat caatgttttc 16980
   atactgttct ctggtctgtt tttttcacaa atacactttt tatttgtcgc caggtactta 17040
   tttttaaagc tatagaggta atatttcatc aggtgagggt aactaccatg gtttgtttgc 17100
   tatactgtgt tagggttatt ttcgtttttt ttttctttta taaactatag ttgtgaatat 17160
   gtttatatag tttacttttg gtttattaga atatattgct agagtgggat tacaggatta 17220
   aagagtgtac agtattttag tttttttttt ttttttacaa gttgcagatt tgttgccaaa 17280
   tgaacgagtt tgtagtattg ctaacaagga gaagaattac tagcaagtct tgatgttact 17340
   tttgaagagt gtgatgattg catttaggaa gatatctaaa cttctgtttc aaagcaaaaa 17400
   gtatgtgcaa atttcttact catgacaaat tcatataata taaaaacatg aaagttgtga 17460
   ggtcaggttg tttggagaag tagaaaactt cagtagagtt tatagatagg cagtcttcct 17520
   ttctggtttg gcactgacag cagattaact agaaagtgtt agaaggaacc taaaatttat 17580
   actaaagtca atttaagtta attaatatac cagaattcct tcttttacaa tttatttata 17640
   aaaacaccat attgagttgc cttgtaatga gacatttaaa ctaaatttaa ataacagaat 17700
   tcatgcacca tctaataaca accccttatt tacaattata gagtcctttg caattttata 17760
   gatattttca tgtatcccat ttggtccttg aaacaattaa tgaagaagtt acagcaagtg 17820
   gtattatcat tattttacag aagaacaaaa caaaaatata tgtggcccag agattgagtg 17880
   atttacctga ggttataggc tttagattgc atagctggaa gtagaacctt gttcttctat 17940
   attaaatgac aatattcatt aagtacttag cacaggattt ggtacctagt aaatatttaa 18000
   atgttcctgt gttattcctg actattcctt ctttattctt aaaacgccat tttttgagca 18060
   ctcttaatat ttatagttca aactttgtac ctatgtacct ttttctcttt agaaaataag 18120
   atttcaggct gcattaattt gatctgtaca ggaatgatta tatgttttac atattgggac 18180
   aaattgctct ttttttatat accttaagct ctagggtaca tgtgcacaac atacagattt 18240
   gttacatatg tatacatgtg ccatgttagt gtgctgcacc cattaactca tcacttacat 18300
   taggtatatc tcctaatgct atccctcccc cctccccata ccccatgaca ggccctggtg 18360
   tgtgatgttc cccaccctgt gtccaagtgt tctcattgtt cagttcccac ctatgagtga 18420
   gaacacgcgg tgtttggttt tctgtccttg cgatagtttg ctcagaatga tggtttctag 18480
   cttcatccat gtccctacca aggacatgaa gctcatcctt ttttatggct gcatagtatt 18540
   ccatggtgtc tatgtgtcac attttcttaa tccagtctat cattgatgga catttgggtt 18600
   ggttccaagt ctttgctatt gtgaatagtg ctgcagtaaa catacatgtg catgtgtctt 18660
   tatagcagca tgatttatac tcctttgggt atatacccag taatgggatt gctgggtcaa 18720
   atggtatttc tagttctaga tcactgagga attgccacac tgacttgaac tagtttacag 18780
   tcccaccaac agtgtaaaag tgttcctgtt tctccacatc ctctccagca cctgttgttt 18840
   cctgactttt taatgatcac tattctaact ggtgtgagat ggtatctcat tgtggttttg 18900
   atttgcattt ctctgatggc cagtgatgat gagcattttt tcatgtgtct gttcgctgca 18960
   taaatgtatt cttttgagta gtgtctgttc atatccttcg cccacttttt gatggggttg 19020
   tttgattttt tcttggaagt ttgtttaagt tctttgtaga ttctggatat tagccctttg 19080
   tcagatgggt agattgtaaa agttttctcc cattctctag gttgcctgtt cactctcatg 19140
   gtagtttctt ttgctgtgca gaagctcttt aggacaaatt gttcttaaat aatgaacagt 19200
   tggcactttt tcaactggaa aattcaagga actgctcttt ctgctttctg ctcaatatga 19260
   atcttcaatt tagaaatgag agtccatcat taacaattca acatagctta ttaataggaa 19320
   aaaaaaacct agtaacaaat gtaaaatctt tgattaaatg agaaagtcat agaagttcat 19380
   cagatttgta tttaaagcat gatttcatta gaaaagttga taataaggat ttaactgtga 19440
   cataattgga aaatacttgt ttaaacttaa aattttgaaa agaaatgtaa atgtgatgta 19500
   acttatgaat cagtggttga gtttcttttt tgctcacaag aaccctaact gtgtgttact 19560
   tgaaagcact gatggaaatc agggaaaaag ctccagaagt tcctacgaaa taaaattaaa 19620
   tgataaagtc ctggtatctg ctaacttgcc ttccattcct gttatctttt cttcttagtc 19680
   tgacttcatt aattctttca ccctggctac tggtttagct cagtgtttta tgagccaggc 19740
   agcttcagac tttgcttttg atgctctttg ttcattacct ctaaagctgt attatcactt 19800
   tcattttatc attaatgttt catgtatatg ttatagtttc atattgttac tgcaactttt 19860
   acttagctat aatttaaaaa atatctgtga tctgtggaaa taattattct atggcagaaa 19920
   agtagttatt gcattttact ttataagttg tttaaggata agcataccta tatattaagc 19980
   actacaaaga aacttttaca atggctttat ttttagcaaa ccatcatagt taaaataaga 20040
   tttagtgtac atgtcaggaa cacagtctta tgaaataagg tttagggagc tatttttagt 20100
   tactatatcc tacttgaaaa ttgtagttaa atttctagca tataccctat taatttagat 20160
   gcaagtacag atttgagata aggtagatac attatttgga tgtcaactcg gaagttgttc 20220
   aagaaaagat attttgttat ttagatgtaa cttggaacat atttctagtg tttcaagtca 20280
   tgattgtatg cctagaacag gcaataaaaa tttacttagc tggtaaaaca gccacattat 20340
   ttcaaatata gtttagttat attatggatt aaattgattt ttgtggacag actttagaac 20400
   ttaattgcta ttaattacat tttttctttg ggacggtatt tgttctttgg tgagaaagga 20460
   ttcttgtaac acctaaatca agactgtcca aacatggcat atgcagttta ccagtcaaaa 20520
   caaactaatc acttaagatc tgtgtatttt gttttatttg aattatacct caattaaaac 20580
   aaatttagca tgtttacaca aaggtggggg gaaactgtct aatatatctg agatctgttg 20640
   gagctggggt gaccatccaa cttaggcaag atagctagta ccatgtatgc atttctttta 20700
   cctttcttat tgtatataca gtaatcactg atattatgag aaaaagaact ttttaataat 20760
   tcaggagata ttttatcgct agtatatatt gagtgcttat tatgtgcctt atatactttg 20820
   gactcattta atcctcaaca caacaaccct atgtagttca tactagaatt attcccattt 20880
   aaagatgggg aaagtgatgt ttagagaggc taagtgactt gcccatggcc aaagtattag 20940
   aactgggatt tgaacacagg tagcctgatt cctaaataaa tgaccactaa cattaaaaag 21000
   acataaacat agaggtgttt gttgcaatgt tagtcataat acctaaaaat tgtgaactag 21060
   ttaaatgtcc aatagtagta aaatggttca ttaaccatgg tacacccatc aatataatgt 21120
   tatattgtca tttaaaattg tattcccaag tttcgtatca ggaaaatata ataaattttt 21180
   gaaaaatgta atgggatata ttgtatttat aatacagtaa tcactatgta accaacattt 21240
   ttatttccat caaaaattag ttataaagaa atagaaatct aagaagttta atagatatta 21300
   ccattgaaca ctagatttgt attagtatct tttatattta tctctctcat ttcctatatt 21360
   ggccatgttt acttttaaaa tagtaaaagt cataagctct attttaaaaa taaatgttag 21420
   tttattaacc caagaataat agctaactat aaattcatat ttgataaaat aaaaagatga 21480
   cattcatcat aagggataca tacctgtgct agcatatggc atttttaaaa tcacatgagt 21540
   aatttgtaat catcatagaa atattagaaa atacaaataa gcaaacacat actgaaaatt 21600
   tagtgttccc aaatctaaaa cagagacact gtttttttct tttgggattg tattttagat 21660
   atgttcttaa gttataataa agtaaatact taaaaggcaa attgatacat taaggatttc 21720
   aaagagtaaa actttttgtt aagctttgat gttctttaga aagtttagat tattccacaa 21780
   gtcactgtcg ttgaaagaaa agtagttaca gtggggtctt atgggataag gcattaccat 21840
   ttgttcagtt gagagacagc tatcactatg ttttaagcat tgttcatata ttagctcatt 21900
   taatcctcat agcaacctta tatgataggt acctttatta gccccattct gcttaggaag 21960
   caacagaaag agtatgtatt ttgcctaagg ttgcacaata agttaagctg gggttccaat 22020
   tctagcaagt tggttctaga gtgtatgtta ttaaccatta tgccgtagtg cctgcaagta 22080
   gatctctaga tgtcagaaat actcatcttc ctctggttac ctggttgtta taaatcttta 22140
   tgcttaatac ttatgtcatt atatgtaaat ttcgtattaa acactcaact tattcagaag 22200
   aatatcaggt aagtgtaggt taaggctgtt ttctatcaga aatcattata tgtatatata 22260
   ttcctcagtt cttatgttgt ttagtttttc taaaatgtca aattttataa tatatggaga 22320
   agtataaatg tatattagaa agattttgtt tatttgtgta atttgtggca taagaaatat 22380
   ttgcctcaag atttggtgct tgtttaggta gttgctggca ttacttttgg aaatgtcagt 22440
   aaattttcat actgtcttgg aattttttca atttttacat tttattagta aatgtaatta 22500
   caggttagta aatcacttat ttgaacctgt ttcctttgaa agttttatat ttttatttgg 22560
   aaatagaaaa accttaattt cctctcgttg ggcagtatgg tgtcaaaagc ttgggctttg 22620
   gagcttcgta tataatctgg gttcaaatta taacttacta gttactaact tgggcaaatt 22680
   acccagtctt tttgactctc aatttctttg tctatgaaat gtaatactat ttaggattgt 22740
   taggattaaa tgagaatata tttggcatac tgtctggtac atgatactta acaggtacta 22800
   gttgtctaca tctttctaac ttaggatgga tgccgatgtc ttgggtaaca tctcaaactt 22860
   tatcagtaag gaaggtgaga atctgaagaa aatgaaacct taaaaagatt gaattcctgg 22920
   actccattta aaggagtaaa tagctcacga acaagacttg ctgctctgca aagtcttcca 22980
   tgttgatcct ggtctttgac tccttatctg tctgattaaa ttgaattcgc tgccgtggca 23040
   tccttaaagc tggaccttac tttgtcagtc ctgccttctc catgttgctt tgtgtgtaag 23100
   cttcactgga ctgtttgctt tttgctgatt attttatgta tttccatatg tctactttag 23160
   cctttgcttg gaatgttcta actgctcttg tttcttcctc tgtttactgg tttctgactt 23220
   aactcttaag gattatctaa tatattacct acttggtgaa ggtttatctg tgtccccaca 23280
   gaattaatcc ttccctcttt aactcttaag ctatcttatt ttttatctaa tcgggtcttt 23340
   ggcacaatta taggtctgtg tgtctgtctt ccctaataga ataggaaagc cttgaggaca 23400
   gtagtctttg cttacttagc cttatattct cagtgcccct tgtgcaaaac gttcaatata 23460
   tgtttgaatg attatgtgaa tgaaggaggg ggccagctga atttacttta atgattatgt 23520
   aacacccatt tatgtatagt tatagacttg tctgaaatga gttaaatcct ttgcaacgtt 23580
   tgctgctatg ctttgaatgt ctcttacaaa actcatgttg aaatttaatt gccattgtaa 23640
   tggtattaag agatggaacc tttaagaggt gattagataa tggtatctct atccttatga 23700
   atggattaat gctgtttgaa tggtagtgga tgagttatct tgggagtttg gcctcctccg 23760
   tctcacatgc ttccttacct tctgccatgt ggtaactcaa cacaaagatc cttgccagat 23820
   gctgatgcca tgctcttgga cttcccagtc tccagaaccg tgagccaaat acatttctgt 23880
   tcattctaaa ctacctgttt tgtggtattc tagtaaagca acataaaatt tactaagaaa 23940
   actggtacca agagtgtgat tgttgccata acaaatacct gaaaatgttc aaatggcttg 24000
   ggttctggct agagaaggat ggaagagttt ggatgaacag gctagaaaaa gcctgtattg 24060
   ctgagaatag agcattaagg acaattctga tgaggattca gaagaagaga gctgtaggga 24120
   aaatctggaa cttcttagag agttgtcatc agttggtaga actataagtg gtaaaggtct 24180
   ttctgatgat atctcagaaa tgaagaacaa gatactggac actggagtaa aggccatcct 24240
   tgttaaatag ttgcaaagaa cttggcgaaa ttatgttcat atcctaagac tttatggaat 24300
   gcagaattta agagtgatga actaggatat gctgcagaag aaataactca gcagcagagc 24360
   atttaggtta ctggatggct acttttaacc acttaaacta agctggggga agggaatgac 24420
   ttgaagacag aatttataat taaaagagag gcagaatgga aatacttgga aaatttgcag 24480
   cctggccata gtaaagaatg caaaaggtat gtttaggaga gcaaaccaag ggtgtggtcc 24540
   aggaaccatt tgctgaagag attaatattc ctagaggaga cccaagggct atttatcaag 24600
   acagtggaaa aagaccccag aggcattttg gagatctttg aggctgcctg ccccatcaca 24660
   ggcccagagc tctaggaggg cagaatggtt tgtggctcag gtggtccttc acaagcttgc 24720
   tgcccagggc tacctcagct ccccatattt caacccagtg ggccttggct gtcctaggtc 24780
   tggttcagag gggcccaggt gtggcttagg ctactgctga gtactcaaat ggtaaacctt 24840
   ggcagcgtct atatggtgct aattctgcag gctcacagga tgaaagagct gtgggagaca 24900
   tggctaccac caccaggatt tcaaaggatg atggggatag tctgggagag acttgccaca 24960
   ggcttggagc ctctgaaggg tggaaatgtg ggttggagtc actacagaga gtccctacta 25020
   gggcattgca taatggagcc atggcagcag gcccaccacc aaagcttcag aactgtagag 25080
   ctacaagtat acagtgccag cctgggagaa cttcaggctt gagaccctaa cctgtgaaag 25140
   ctgcatgggc taagtacagc aaagccatgg aggtggggct tcccagggtc tattgggatg 25200
   aaacgaatgt attttgtagg tgagaaggac atgagttttg aggcccaggg gcagaatgct 25260
   atggtttggt tgtttccttc aaaactcatg ttgaaacttc attgccatgt ggcattatta 25320
   tgaggtggaa cctttcagaa gtggttacat tataagggct ttgccttcat taatggatta 25380
   atgccattat tgcaggtgtg ggttagttat ctctggagtt tggccccctt tttctctatc 25440
   atgtgctcat gccctctttt gccatgtgat gccttctacc atgttatgat gcatccagaa 25500
   gactctcacc acatgcagcc ccttgatctt agacttctca gcttctagaa ctgtgagtga 25560
   aataaacttc ttttctttat aaattacccc gtctgtggta ttgtatagca gcagaaaata 25620
   gacatcagcc tgaagttccc ccaggctggc attaaatact aattattaat tagtatttat 25680
   agtgcaagga taaattcaag tttagccctg gttagaatga ccacatttca agggaggggc 25740
   tttgtacttc tgtgcatatc tgtaaggata aaaatcttaa tactattctc actgaaatta 25800
   atggtttagg ttaggtaagg ttgttagtgc taataattat ttcttttaat aaaatattct 25860
   tagttgcgtt gttcaaaaaa catagatgat ttgaatttat attttttggc caaaatatat 25920
   ttataatttt gagtaggaat tccagagtat tggtagctat aaccactttg ggttccctgc 25980
   cattgcttct ggtgcctcat tttttctgac gtcttccatt ttcttacatt tgtcttctaa 26040
   ggtggagtta agattactca gttaagatta tttcacttta ggcctctgct gtcttctgct 26100
   ttttttttta aaatgaatgg atataatatc ccaatacatt ttgataattg aacaacagct 26160
   acatttttaa gtgaggctac tttcttctaa ttttttaaat ttatttttct cagtttttaa 26220
   aaaaaatgtc agattggcta agagttgggg cagctttctt atgtgagagt agtagatgac 26280
   agcaaatatt tgtgatgtta aaatgataat cctaatagtt ttcttttaga atctttataa 26340
   taaaaaccct ttgaggctga gggtgaattt gtatgttcct aaagtgacaa aaaatgttct 26400
   tggggcatag taatttaaat cttagatgct tttattagta taattttttg gtagaaattt 26460
   ggcattaaaa aatgcataca gagctttttc tacatacagg gcaagacagc attttgtcat 26520
   ggcaattagt aaatagataa ttataaaaca tctaatttta agcaatttgt tacagaaacg 26580
   atacaggtac attgtggcaa aaatagaaga tacaaaacaa gcaaatatga gaaaaaatat 26640
   acatgccacc acctatatat gacttttagt actttataat cttttgtcta tatatgtcta 26700
   tatggatata tacgtattct ttttatccaa atggtattac attgtacatt ctgttttgaa 26760
   acctgccttt tttagtcatt tacatctact tttccatctc agtaactttt catcttgtgt 26820
   aatgcccgga tcacattaaa atgtttccaa ttagctcaaa aatgtccttt atggctggtt 26880
   tggctaaaac agtatccagg ccagcattgc acctatgaaa ttggctgtta ggaatcttgt 26940
   atctttaaaa atcaagggca gcaacccatc ctcccgcttc ccccacctcc caccaccccc 27000
   ccaccttttt tttttcttaa agatactggc ttgttgaaga gagaatgggt catgtcctac 27060
   aaactgtctg aatttgtcca gtttgctgtc tcatagtgtc atttagcttg ttttatccta 27120
   tgtatttcct gcaaattaaa atttgtatct gaatccttgg tggatttgag ttgaagatcc 27180
   ttaaccatca tagatgatgc tgtgtccttt atattgcatg tcagaagtta catgatcttt 27240
   acttgattca tgatgaggag atggccactg aaattggaca gtgacagtct tttctgccca 27300
   ttgttcaatt atattcgtct ctttacatta gaaagtattt tgggtagtag tattttggtg 27360
   ctgtaagaaa gttcattttc tcatcaacca ctcacctatt ggtttaacac catttgttga 27420
   tctttgagta tatcagtaat ttcatcaggg tttgcaaaat aagactttaa attctattgt 27480
   tttacattat taattgatgt tttttgataa ggtagaactt gtgaaatggg actatttgtt 27540
   tgtctttaaa tacagtctct ataggaaaga caaaataaat acttaaatct cactctttac 27600
   catttttcaa agtgaagaac tattccgtta accacctcaa aagatgataa ataaaaaggg 27660
   tatttttagt tgtttcaact tttttttttt ttttgagatg gggtcttact ctgttgccca 27720
   ggctggtgtg ctgtggtgca atcatggtac accgaagcct cagtctccct gggctcagat 27780
   gattctccca cctcagcctg ggactaaagg tgtacaccac catggccagc caattttttt 27840
   gtattttttg tagagatggg gttttgccat attgcccagc ctagcctcaa actcctgggc 27900
   tgaaggaatc cacccatctc agcctcccaa agtgctagga ttacagacgt gaccaacaat 27960
   atccggcctt aacttttttc ttttgagtgt ctttatagac tcaaggactt ttatttaatt 28020
   cagggtgtta gtaccattta aatgttttct ttgatgctca gattatcaca gctagtcatt 28080
   tggaccttta taccacctcc tatgtccatt tgatataggc cattaatctc tataagcctt 28140
   cctccttctc ttggaatgaa aaggtatcct aggctcacct gtaccttccc tactccagac 28200
   ctggcattaa gtctttttcc aaggagtttg gtacctttta gtttattatg atattagaga 28260
   tgaaaatctg tgttctagga atgtttatta ctgctagagt gatgttgctt ttaggccatt 28320
   tcagagaaaa gacctagaaa acagattttt acaaacatga attcatactg atatttttag 28380
   ttttttacat gatttcttga ttttacaata ttatctgctt tcttaactta aaattatgaa 28440
   ccttaaagtc attagcataa cttctttgct tatttctaca acataaagaa aatagtcctg 28500
   gtgcggtggc tcacactgta atcccagcac tttgggagat tgaggcaggt ggattgcttg 28560
   agctcaggag ttcaagacca acctgggcaa catgatgaaa ccttgtcttt acaaaaaatt 28620
   agctgggcat ggtggcatgt gcttgtattc ccagctactc aggaggctga ggtgggagga 28680
   tcacctgagc ccaggaggtc aaggctctag tgagccatga tcatgccacc acactccagc 28740
   ctgggtgaca aagtgagacc ctgtttcagg gggaaaaaaa agataaaata gtttgaggag 28800
   gctggatgta gtggctcatg catgtcatcc tggcactttg ggaggtcaag gtgggaggat 28860
   ggcttgagcc caggagtttg agaccagcct gtgccacatc atgagacctg gtctctattt 28920
   aaaaaaaaaa aaaaaagaaa atagtttgag gatatcaata atgatattac tagaatcagt 28980
   aaaactacca aaagaagttt aaagtttctt cctagtgttt ttttgttctt agaatacttc 29040
   ctaccaagaa gtgcagtaaa agtgcagtgt ccaaatagcc cttgtaacaa aacctttctc 29100
   tttctcctgg gtgccaattt gacatttaat cagttttgtt tctagcagtg ttcaatttat 29160
   tagattataa gtcttttttt tctttatatt attctaagat caaaaatata taaagatata 29220
   cacaggagtc ctgctgctac ctgttcttgc tatgcttttc cccttttctt ccctttctct 29280
   gtgaagcagc catttttatt agtttcttgt ttatcactca tgcatgcata tgtttattga 29340
   ggatgttgac attcaagcaa atatatgggt taacattctt tttgtcatcc ctatacgaaa 29400
   gatataccca gtatactcta ttgggtgggt ttttttcctt aaaatattca gtagatctct 29460
   ccagttagca catagttatc ttatagatag aacatataca tataaccttt tcttaaacta 29520
   tgctattaaa ataatagctt tcagtaactt gataattatt tttggattga aaatactact 29580
   gaaatcaact caatcatgtg aaagctgcag aaagaaaaag acctagaaaa agggcattgg 29640
   attaggtcaa ctttgaattt tatttggaag ataaatgagt ccagaagtga gtgggcagag 29700
   attattggag ttggtcttga aatgaggcgt taggcagatt gactgggctg gtgtgaaagg 29760
   tctgtcagaa aatcatgaga ttagattgag gtacctcaaa aaatgagagc tggtatgatg 29820
   agtgggtaag aatcataaaa gcgtagagtg ttgatgattt ttatagttta taaatggttc 29880
   ttgtgtgtag agttttgttt ttatgctagc tatagtctgt aacataattc actataatgg 29940
   gcatgctaaa tatccatgac agttgaccct tgaacaacac agagggtagg ggcgcctacc 30000
   cctgtgcagt tgaaaattca catgtaactt ttgactcccc aaaacttaat atttagccta 30060
   tacttgacta gaagtcttac tgatgacata atgttcgtta atacatattt tatatatgtg 30120
   tcagatagca tatttgtata ataaagtaag ctgcaggaaa aatattaaaa tcataaagaa 30180
   gagaaaatat acttactatt cattaagtgg aagtggatcc tcataaaggt cttcatcctc 30240
   actgccttca ctttgagtag gccgaggagt aggagagaga ggaaaggtca gacttgctgt 30300
   ctcatgggtg gcagaggtag aagaaggtcc acatacaagt ggtccgacac agctcaaacc 30360
   ggttttgttc attggccaac tgtagtttga ttgaaagtaa taataaatga agtttctgcc 30420
   tcagttcagt attatcaagt catagatagc aagggctgga agaaacctta gtagtaatct 30480
   ctttgagtct aattatcatg tagaatagga aattgcggtc tagaaaggtt aagtgacttg 30540
   tccaaattac acaactagtt agagacatag ccagctctta aatctgactt ccagattttc 30600
   actgtgtctt cttttttctg taacgtgttg ccttttttag ccatgaaaaa ttagaagttg 30660
   aactcttgtc ttttcaggca ggtgtcaatt ttggggtttt gttttgattt ttggtttttg 30720
   acataaagta ctttagttct gtgatgtata aaccgtgagt ttctgttttt ctcatatacc 30780
   tgaatactgt ccatgtggaa gttacctttt atctttacca gtattaacac ataaatggtt 30840
   atacataaat acattgacca ccttttatta ctccagctat agtggggaaa actttctttt 30900
   cataactagc taatgtttta aaaagtattc ttttagtttg attgctgcat atttcagata 30960
   tttctttcct taactaaagt actcagatat ttatccaaac attattgcta tgggatttcc 31020
   tgcagaaaga cttgaaggcg tatacaggaa caatattgat gatgtagtaa ggtaagaatg 31080
   ctttgatttt ctatttcaaa tattgatgtt tatattcatg ttgtgttttc atttagaaaa 31140
   gatttctaag ccacagaaaa agatactttg tgatgtaaac tattattgta gtgctctata 31200
   atcatttttt ggcttaccgt acctaatgga cttcaggggg atacagttca tttgataaga 31260
   actgacctta tacattacat aatcaggtac ttatgtgata tcatttcctg gactccataa 31320
   aatgctggtc accaggttta atacctggat tccattacag tgtgattttt gtcttatttc 31380
   atagttgggg attaggctta aaatcctaga gtggatttat tcagttaaat ttattcacac 31440
   taagatgtag atgactaata ctgtatattt ttatgtagac caaattttaa ggtaccactg 31500
   tgcatatgta taccaactac ctgaagaagt atttggttgg tacaagagat atagaaagga 31560
   atcgctgggt gtaccaaggc taatcagttt tataattttg cataattttc taactgcgat 31620
   tatcatttag tttagaacaa tttatttctc aaggcccatg taaatattat ttttaaaata 31680
   tacagtctta agaattcatg gcatatttta tgaaaggagg aattcatgtc tgatgtgcaa 31740
   atagtcttaa gatattttct aatttcagag caaaaatata tatgtatgaa taaattaact 31800
   gtaaattgtc agtaggaacc ttaagaattc gtggcgtatt ttatgaaagg aattcatgtc 31860
   tgatgtgcaa atagtcttaa catatttgct aatttcagag caaaaatata tgtgtattaa 31920
   taaatcaact gtaaattgtc agtaagaacc ttaatggctt taaaagttaa aatttcaggt 31980
   caagcattgt ggtgtgctcc tgtagtccca gctacttggg aggctgaggt gggaggatca 32040
   cttggcttga acccccaggt agagggtaga ggccagtctg ggtaacacag cgagaaccca 32100
   tctcttaaaa aaaaagttta agttgtggat tatttccttt acactctttc attagtatct 32160
   ttcctggaga ctttcaattt aaatacttgg tgcttatgac aattagatgt taaaatggat 32220
   gggaaagtac tttgtaactt ataaagcatt atgcagatgt agactccttt tataatagtt 32280
   gtgtaagtat ataagacaac ctacattctt catgagctag ccataagttt tagcaacttg 32340
   ctttgaacca cggtagattt acaattttct gtagtattga gttgtgttca tttagaattt 32400
   tgtaatattt atattgaaaa tcaaattttt gtacctacaa aaactacaaa aaaatccccc 32460
   tagtttttat agtttctatt aaaattatag ctggtacata gggatgccag aaggactgtt 32520
   taagaagctg aaaatagaga aatgaattta tcttctcata gttaggcagg gcacagtaga 32580
   aggatgctta acattgcaag ctgatgggaa cagcaggttg atatagcttg tgataacact 32640
   tctaaagaaa aagcaatgag ccatagaaaa aagaaaaaga tacattttga attaaggaag 32700
   atggtgaatc tgggaagtga gcagtacagt caccagacgt gtatcctctc ctatggtaca 32760
   gaagtgttta ttgggtctct ttatggcctg catgatatat cccacaagat gacctacttc 32820
   acattatttt aattctgtat tcaactaagc actaattcaa cccagccaga ttagtactca 32880
   taccaaaaaa gagtgaatac tctgaataga gggcaggttt tctgattatg gtgagaatat 32940
   ctttgtggta aattaatctg gtgtgctagt ttttacgttg gtctcttctc agtgtcgtta 33000
   gtcactgagg ctgattgatc atcttttagg ttactgataa agttcctgta cagctgattt 33060
   tcagacctta gattgcaata acttcaccaa gaaaatactt cattgggaag cattttggtc 33120
   cttccatttg attcataact cttaccttta tgcctctgaa ggaaaagatt tatacattca 33180
   gcttgtaatt agtaatcaag actgaggttt agtctatcta gcttcacaat ctatctagtt 33240
   tgttttgtct agccatatga tttcttcaaa tatgccattt cttaaaaaaa aatgttttat 33300
   gtatcccgat taatatttag ccagtggttc ttttagccga tggatcttgt cacctcttat 33360
   gatactatta atagcatgtc aacatgaaga attatctgct gaatataata gctatgctgt 33420
   ccttgtttcc ttttgtctca ttcttttttg attgggggat aattggccaa taaagctttg 33480
   atagcctcta ttgcccaggc ccctcctctt cttttatgag agaaaggatg aacagtgacc 33540
   agaaataaag gtattgtttt tttctatcaa ctaaaatgga aataaataat tcctaagtaa 33600
   tttgcctgtt aggattaaag tctccaagag aatggctgtg cctagtacct aagtgattaa 33660
   tttccttgat tggttcacat tatattgagg atattagtaa tcagtagtga ttcctttttt 33720
   ggttcaaaga tgatagtgtc acagtgaaaa atgtttttaa aatttttgta tacttaattt 33780
   ttctgttaac gaaagtattt tcagttggat ttttgtttgc cctctctatt agaatgccca 33840
   aagaatattt aaaattttcc ttttctctta tactgcatat ttttcctgtg atttttcccc 33900
   aaacggaaaa tactctgcag agattagact ttgttattgt tgtactacat cattgctttg 33960
   actaaaataa actcagattg caaatacctt caagcttaca ttgctcagta tttttttttt 34020
   tttttttttt tgagacggag tctcactctt gtcgcccagg ctggagtgca gtggtgccat 34080
   ctcagctcac tgcaacctct gcctcctggg ttcaagcgat tctcctgcct cagcctgcgg 34140
   agcagctggg attatagatg cccgccccca cgcccagctg atttttgtat ttgtagtaga 34200
   gatggggttt taccttgttg gccagtctgg tctcaaactc ctgacctcgg gtgatccatc 34260
   tgtctcggcc tctggaatta caggtgtgag ccgccacgcc tggctaaatt gatcagtatt 34320
   atttaacttt gagggatatg atttgttatg gaatgcgaag ttttatactt gaggtactca 34380
   gagtcctttt gagacaaata tttaacttct ccttttgagg ttaccgccta cgattgggaa 34440
   ttaatgtaaa aaataagcca aaagaaagtg agggaaaagt gaaccaagct gtaatttttt 34500
   tactcttttt tattgttgtt gttattgttg ctgtttttta ctatcttgat tgcaacagtt 34560
   tggcttatat atatagcatt tggaattgac agtaagaaag ccacatctca tagaagctaa 34620
   ctattcccaa attgtttttt tcttcttttc ctcttactac tgctgttttc ctcctttctt 34680
   gctgctaagc tcttgtcctg acatgctggt aatatgaaac agtgttttat tcagataatt 34740
   gattattctg taatatgtat gttaatcttt tttattacac tttaagtaat agggtacata 34800
   tgcacaactt acagattcgt tacatatgta tacatgtgcc gtgttggttt gctgcaccca 34860
   ttaactcgtc atttacatta ggtatttctc ctaatgttat ccctctccca accccccacc 34920
   ccaggacagg ccccggtgtg tgatgttccc cgccctgtgt ccaagtgttc tcgttgttca 34980
   gttgccacct gtgagtgaga acatgcggtg tttggttttc tgtccttgcg atagtttgct 35040
   cagaatgatg gtttccagct tcatctatgt ccctacaaag gacgtgaagc tcatcctttt 35100
   ttatggctgc atactactcc gtggtgtata tgtgccacat tttcttaatc cagtcagtca 35160
   ttgatggaca tttgggttgg ttctaattct ttgctattgt gaatagtgct gcagtaaaca 35220
   tacgtatgca tgtgtcttta tagtagcatg atttataatc ctttggatat atacccagta 35280
   atggaattgc tgggtcaaat ggtatttcta gttctagatc cctgaggaat tgccacactg 35340
   tcttccacaa tggttgaact agtttacagt cccaccaaca gtgtaaaaat gttcctgttc 35400
   ctccacatcc tctccagcac ctgttgtttc ctgacttttt aatgatcgcc attctaactg 35460
   gtgtgagatg gtatctcatt gtggttttga tttgcatttc tctgatggcc agtgatgatg 35520
   agcatttttt catgtgtctg ttggctgcat aaatgtctat aaatgtcttc ttttggaaag 35580
   tgtctgttca tatcctttgc ccactttttg atggggttgt ttgatttttt tcctgtaaat 35640
   ttgtttaagt tctttgtaga ttctggatat tagccatttg tcagatgggt agattgcaga 35700
   aattttcttc cattctatag gttgcctgtc cactctgatg gtagtttctt ttgctgtgca 35760
   gaagctcttt agtttaatta gatcccattt gactattttg gcttttgttg ccattgcttt 35820
   tggtgtttta gtcatgaagt ccttgcccat gcctatgtcc tgaatggtat tgcctaggtt 35880
   tgcttctagg gtttttatgg ttttaggtct acatttaagt ctttaacatt taagtcttta 35940
   atccatcttg aattaatttt tgtataaggt gtaaggaaat gatccaattt cagctttcta 36000
   catatgacta gccagttttc ccagcaccat ttattaacta gggaaccctt tccccatttc 36060
   ctgtttttgt caggtttgtc aaagatcaga tggttgtaga tgtgtcatgt tatttctgag 36120
   ggctctgttc tgttccattg gtctatatct ctgttttggt accagtacca tgctgttttg 36180
   gttactgtag ccttgtagta tagtttgaag tcaggtagtg tgatgcctcc agcttttttc 36240
   tttctgctta ggattgtctt ggcagtgcgg gctctttttt ggctccatat gaactttaaa 36300
   gtagtttttt ccaattctgt gaagaaattt attggtagct tgatggggat ggcattgttt 36360
   ctataaatta ccttgggcag tgtggccatt ttcacgatat tgattcttcc tacccatgag 36420
   catggaatgt tcttccattt gtttgtgtca tcttttattt cgttgagcag tggtttgtag 36480
   ttcttgaaca ggtccttcac atcccttgta agttggattc ctaggtattt tattctcttt 36540
   gtagcagttg tgagtgggag ttcactcatg atttggctct ctgtctgtct gttattggtg 36600
   tataagaatg cttgtgattt ttgcacattg attttgtatc ctgagacttt gctgaagttg 36660
   cttatcagct gaaggagatt ttgggctgag acagtggggc tttctaaata tacaatcatg 36720
   tcatctgcaa acagggacaa tttgacttcc tcttttccta attgaatacc ctttatttct 36780
   ttctcttgcc tgattgccct ggccagaact tccaacactg tgttgaatag gagtggtgag 36840
   agagggcgtc cctgtcttgt gccagttttc aaagggaatg cttccagttt ttgcccattc 36900
   agtatgatac tggctgtggg tttgtcataa atagctctta ttattttgag atacgttcca 36960
   tcaataccta atttattgag agtttttagc atgaagggct gttgaatttt gtcaaaggcc 37020
   ttttctgcat ctattgagat aatcatgtgg ttttttgtct ttggttctct ttatgtgatg 37080
   gattatgttt attgatttgc gtatgttgaa ccagccttgc atcacaggga tgaagccaac 37140
   ttgatcttgg tggataagct ttttgatgtg ctgctggatt cggtttgcca atattttatt 37200
   gaggattttt gcattgatgt tcatcagggg tgttggtcta aaattctctt tttttgttgt 37260
   gtctctgcca ggctttggta tcgggatggt gctggcctcc taaaatgagt tagggaggat 37320
   tccctctttt tctatgaatt ggaatagttt cagaaggaat ggtaccagct cgtcttttta 37380
   cctctggtag aattcggctg tgaatctgtc tggtcctgga cttttttcgg ttggtaggct 37440
   attaattatt gcctcaattt cagagcctgt tactggtcta ttcagggatt caacttcttc 37500
   ctggtttagt cttgggaggg tgtatatgtc caggaattta tccatttctt ctagattttc 37560
   tagtttattt gcatagaggt gtttatagta ttctctgatg gtagtttgta tttctgtggg 37620
   atcagtggtg atatcccctt tatcattttt tattgcatct atttgattct tctctctttt 37680
   cttccttatt agtcttgcta gcagtctatc aattttgttt tttaaaaaaa ccagctcctg 37740
   gattcattga tttttttttt gaagggtttt ttgtgtccta tctccttcaa ttctgctctg 37800
   atcttagtta tttcttgcct tctgctagct tttgaatttg tttgctcttg catctctagt 37860
   tgttttaatt gtgatattag ggtgttgatt ttagatcttt cctgctttct cttgtgggca 37920
   tttagtgcta taaatttccc tgtatacact gctttaaatg tgtcccagag attctggtac 37980
   gttgtgtctt tgttctcatt ggtttcaaag aacatcttta tttctgcctt cattttgtta 38040
   tttacccagt agtcattcag gagcaggttg atcagtttcc atgtagttgt gcagttttga 38100
   gtgagtttct taatcctgag ttctaatttg attttactgt ggtctgagag acagtttgtt 38160
   gtgattttta ttcttttaca tttgctgagg agtgagtgct ttacttccaa ctatgtggtc 38220
   aattttggaa taagtgtgat gtggtgctga taagaatgta tattctgttg atttgggatg 38280
   gagagttctg tagatgtcta ttaggtctgc ttggtgcaga gctgagttca aatcctggat 38340
   atccttgtta accttctgtc tcgttgatct gtctcatatt gacagtgggg tgttaaaatc 38400
   tcccgatatt aactgtgtgg gagtctaagt ctctttgtag gtcactcagg acttgcttta 38460
   tgaatctagg tgctcctgta ttgggtgtat atatatttag gatagttagc tcttcttgtt 38520
   gaattgatcc ctttaccatt atgtaatgcc cttctttgtc tcttttgatc tttgttggtt 38580
   tacagtttgt tttattagag actaggattg caacccctgc tttttcttgc tttccatttg 38640
   cttggtagat cttcctccat ccctttattt tgagcctgtg tgtgtgtctg catgtgagat 38700
   acatctcctg aatacagcac actgatgggt cttgactctt tatccaattt gccagtcttt 38760
   gtcttttaat tggggcattt accccattta catttaaggt taatattgtt atgtgtgaat 38820
   ttgatcctgt cattgtgatg ttagctggtt attttgccca ttagttgatg tagtttcttc 38880
   ctagcatcaa tggtctttac aatttggcat gtttttgcag tggctgatac cagttgttcc 38940
   tttccatgtt tagtgcttcc ttcaggagct cttgtaaggc aggcctggta gtgacaaaat 39000
   ctctcagcat ttgcttgtct gtaaaggttt ttatttctcc ttcccttatg aagcttagtt 39060
   tggctggata tgaaattctg ggttgaaaat tcttttcttt aagaatgtag actattggcc 39120
   cccactctct tctggcttgt agagtttcag cggagagatc tgctgttagt ctgatgggct 39180
   tccctttgtg ggtaacccga cctttctctc tggctgccct ttacattttt tcctgcattt 39240
   ccaccttggt gaatctaaca attatgtgtc ttggggttgc tcttctctag gagtatcttt 39300
   gtggtggtct ctgtattccc tgaatttgaa tgttggcgtg ccttgctatg ttggggaagt 39360
   tctcctggat aatatcctga agagtgtttt ccagcttggt tccattctcc ccgtcactgt 39420
   caagtacacc aatcaaacgt agatttggtc ttttcacata gtcccatatt tcttggaggc 39480
   tttgttcatt tctttttact gttttttctc taaacttctc ttcttgcttc atttcattca 39540
   tttgatctgc aattactgat accctttctt ccacttgatc gaatcggctg ctgaagcttg 39600
   tgcatgcgtc atgtagttct cgagccatga ttttcagctc catcaggtca tttatggtct 39660
   tctgtacact gtttattcta gttagccatt tgtctaatct tttttcaagg tttttagctt 39720
   ccttgcgatg ggtttgaaca tcctccttta gctcggagaa gtttgttact actgaccttc 39780
   tgaagcctac ttctgtcaac tcgtcaaagt cattcttcat ccagctttgt tccattgctg 39840
   gtgaggagct gtgatccttt ggaggagaag aggcactctg ggttttagaa ttttcagctt 39900
   ttctgctctg ttttctcccc atctttgtgg ttttatctac ctttggtctt tgattatggt 39960
   gacctacaga tggggttttg gtgtggatgt cctttttgtt aatgttgatg ctattccttt 40020
   ctgtttgtta gttttccttc taacagtcag gtccctcagc tgtaggtctg ttggagtttg 40080
   ctggaggtcc actccagaca ctgtctggat atcaccagtg gaggctgcag aacagcaaat 40140
   attgcagaac agcaaatatt gctgccggag ccttcctctg gaagcttcgt cttgggggca 40200
   cccggctgta tgaggtgtca gtcggcccct actgggaggt gtctcccagt taggctacaa 40260
   gggggtcagg gacccacttg aggaggcagt ctgtccgttc tcagagctca aacactgtgc 40320
   tggtagaact actgctctct tcagagctgt cagagaggga tgtttaagtc tgaggaagtt 40380
   tctgctgcct tttgttcagc tatgccctgc ctccagaggt ggagtctaca gaggcaggca 40440
   ggcctccttg agctgtggtg ggctccaccc agttggagct tcccaaccac tttacctact 40500
   caagcctcag caatgatgga cgcccctccc ccagccaggc tgctgccttg aagttcaatt 40560
   tggaactgct acgctagcag tgagcaaggc tctgtgggcg taggacctgc tgagccaggc 40620
   acgggatata atctcctgtt gtgccatttg ctaagaccgt tggaaaagcg cagtatttgg 40680
   gtggcagtgt cccaattttc ccggtatagt gtgtcacagc ttcccttggc ttggaaaggg 40740
   acatcccccg accccttgtg cttcctgggt gaggcaatgc cccgccctgc ttcagctcac 40800
   cctccgtggg ctgcacccac tttccaacca gtcccagtga gaagaaccag gtacctcagt 40860
   tggaaatgca gaaatcacct gtcttccgcg tggatcatgc tgggagctgc agacaggagc 40920
   tgttcctatt tgaccatctt ggaatgccac cttttttttt tttttttttt ttttaaggca 40980
   gtttcttgct ctgtcaccca ggctggggtg cagaggcatg atcacggctc actgcaacct 41040
   ctgccttctg ggctcaagtg atcctcccac ctcagcctcc caagttgctg ggaccacagc 41100
   cacgcatcac caggcctggc taatttttgt gttttttgta gagatagggt ttcgctgtgt 41160
   ttcccaggct ggtctcaaac tcctgcgctc aagcgatccg cctgcctcag cctcccaaag 41220
   tgctgggatt acaggcatga gccactgcac ccggccaata tgtatgttaa tctcatccct 41280
   caagctgata ctgaagtttt tcaatttatg ttatttggtg taaatctagg cagtctttaa 41340
   caaaattggt gcttcatgtg tttaagaggc ataacttaag aattgtttgt ttcttataaa 41400
   tcaggagaat ggaggtttaa tagaggtgaa ctgtctttct cactgcagaa cctttaatat 41460
   gccactatgc attgtaaatc tcccaagagt gagattctag tatgatgctt ttcttttcct 41520
   tttctgttct ttccctttcc ctctacctcc tttttctttt ctttgttggt ggcatgagtc 41580
   ctatattata aggaaatgct tttagagtac agtcttctga tatatagtga tttttgaaaa 41640
   agatttattt attgtcttgt tcactgtgag ctttttcccc catgtataag cagctgtgta 41700
   atagattcaa gagcaccccc tcgccccttt ttttttgaga cagagtctcg ctcggtcact 41760
   caggctggag tacggtggtg ctgtgatcat ggttcacctc gacttctggg ctccagcgat 41820
   cctcccacct catcctctca agtagctggg accacaggcg tgtgtcaccg tacatggcta 41880
   atttttctat ttttagtaga ggcagagttt cgccatgttt tccaggctgg tctcgaactc 41940
   ctgaactcaa gcagtccacc tgtctcagcc tcccaaagtg ctgggattac aggcgtgagc 42000
   ctccactccc agtctcaaat attcttttga aatatttgaa atatgttgat ctctcagtct 42060
   ttcaacctta gttgtatgtt gattttcaat aaaaaggaag tatttgttgc cctaacatca 42120
   gtattggcta ttcagtttaa aaaaggagtt aaagagatgt tatttatagg caggcttcaa 42180
   aagaggaaag aatgatcagt ttcattctct gtttctagca tattctgact ccttctctca 42240
   tattacctcg tttttcccac atttttcttt taataaagtg aaattcacat aacagctaac 42300
   cattttaacc acggaaagtg tacattccgt ggcatttatt accttcacag tgttacctct 42360
   acctttatca agtttcaaaa cattttatca ccccaaaaga aagccctgtt cttattgggt 42420
   gcctcttgct tttttttttt tttttttttt taaatcttga gacggggtct tggtttgttt 42480
   cccaggttgt agtgcaatgg ggcgatctca tctcattgca acctctgcct cccgagttca 42540
   agcaattctc ctgcctcagc ctcccgtagc tgggactaca ggcacgcgcc acatgcctgg 42600
   ctaatttttg tatttttagt agaaacgtgg tttcaccatg ttggccaggc tagccttgaa 42660
   ctcctgacct taggtaatct gcctgccttg gcctcccaaa gtgctgggat tataggcgtg 42720
   agccaccgtt ctggccacct cctacttctt tcattagtct taattcctta gtggatttga 42780
   cagtgtttat attatctaca ccaatgcatt tttttgtatg ttaataatag gagatattta 42840
   ttgggcattt atttacatac ttatttgcat gtgtaactgt tgtatagcca gtttatgtat 42900
   ataatcttac taaatctcta cagtaaatct atccccattt catagatggt ttaaaagaag 42960
   ttaacttccc caagcattac ttttagtaag tagtgagact gaaggttgag ttctggtctg 43020
   tcagattccg aagttgtttc cttaggaacc atattatctt gtgtacaact cttgggctaa 43080
   tcttgttaat attctttatt tgacctcaca ctgttgattc ataccatgtt taaaattgaa 43140
   atacattacc tatatttaaa aattgagacc tcacataaaa agctatattt ctagctgctt 43200
   ttgaaatttc gaaggatctt ccaacacttg gctgacattc ctgcgtgaca aaaatcagct 43260
   ggagctgtgt aatgtccgac ctgtctctgt caatgaacag attattcatt gtcatttctc 43320
   atctgttttt gagattgtaa ctttgattct gtataactca tagaattagt agttagacct 43380
   atatatgtta gttattacat tatttgtata tgtacagact gctttagaca atattgtagt 43440
   gttatatgtt aattttatca attaaaatgt gctataggat cattgtagag atcttgtctc 43500
   taattaacag gattcataaa gagaaaacaa aggagagaaa catccagatt gaaaagacct 43560
   acatagtgcc aagcacaata aaagaaaccc tatactaggc aaattattat gaaatttgtt 43620
   ttaggacacc agaaataaag ataatatcta aaaacttttt agaatcgcct aggagggatt 43680
   aggaatatga atagcatcta acttgttggc agtagaattg aaggtagaag gtggtaaaac 43740
   atcaccttga aagttctgca ttcagcctag aattctgtat ccagttaaac catcaatcaa 43800
   gtgtgaaagt agggggaaaa actcaaggac taaaaaaatg tgctcatgaa gaacattttt 43860
   tttggacatt acccagaaga tgtggttcaa caaaacaagg gaataaacca agaaaggaaa 43920
   taaaagagct tcagtaaaca gagaatccca ctcagaagca acaaagaaga aagtcccagg 43980
   atgacagctg tgacacaagc tgaaaagtta ccagttttgc ttggagcagg agattagaac 44040
   ttccgggaaa ataatcaaat tgatagatgg atgttgaaat atttggagaa aaatgtaatg 44100
   gattcttgca aaactgagca aattagaaaa aggaaacaat tattagcatt ggtggtttga 44160
   gttaacccaa aattgtgatg ttgctatttt aggggaatta agataagtga aacacgtatg 44220
   gaatactgct agttttgtaa gtctccttta ccatggcagg acatctgtag ttaataaatc 44280
   tgtaagaagc agtattaaga ataatatttt aaaataccta attaaatagg aggaaaaaga 44340
   atccgagtag ttgagggtga ttgcatctgg ggagaagcag gaataaaggt ttgaagataa 44400
   atagggccag agatgagtaa ttttgttact gtgataatat attttatata tatgtgtatg 44460
   tgtgtgcgtg tgtatatata tatatgagat atatctcata tatatgagat atatatgata 44520
   tatatatgat atatatgata tatatatata aaacatacag ttcagtagca ttaacatcta 44580
   gcattcagta catttacatt gttgtgcaat tgtcaccact gtccatctct agaacttttt 44640
   cattatccca cactgacata ccacacccat taaataataa ctcctcattg ctcctcctgt 44700
   tagtaccaac cattgttcta ctttcatctc tatgtatttg actattctag gtacctcgta 44760
   taagcggaat cacgtgatat ctttttgtaa ctggcttatt tcactaacta tcttcgaggt 44820
   tcatccatgt tgtagcagtt gttagcattt ccttcctttt aaaaggccga ataatattcc 44880
   attgttatgt atataccata ttttgtttat ccatttattc atcaatagac acttttggct 44940
   gttgtgaata atgctgctat gaatatgggt atgtaatacc tgtttgagta tctgctttca 45000
   tttcttttgg atatgtaccc aaaagtgaga ttgctggatt gaatggtaat tctatattta 45060
   aatttttgag aaaccaccat actgtttgat atactggctg cccaatttta cattaccacc 45120
   agcaatacac tagggttccg aatttgccac atcctcacca tcgtgttgtt ttgtttatgt 45180
   ttttttttaa taatagccat cctaatgggt gtgaagtctc attgtggttt taatttgcat 45240
   tttcctaatg atcagcgata ttgaacattt gcacatgctt atttggtcat ttgtatatca 45300
   gctttggagc aatgatgtct cttgaagcct tttgcccatt tatgaattga gtagtttggg 45360
   atttttaaat tgtgttttag aagttctttg tatactctgg ctgggcacgg tggctcgtgc 45420
   ctttgggagg ccgaggcagg tggatcacga ggttaggagt tcaagaccag actggctggt 45480
   atagtgaaac cccatctcta ctaaaaatac aaaaattagc tggtgtggtc gggcgtgatg 45540
   gtgcacacct gtagtcccag ctgttgggga ggctgaggca ggagacttgc ttgaacccgg 45600
   aaggtggggg ggttgcagtg agacaggatt gtgccactgc actcagcctg ggtgacagag 45660
   cgagactctg tctcaaaaaa aaagaaaaag aagttctttg tattctctga atattaatcc 45720
   cttattggat atgttatttg caaatatttt ctcccataaa gaatgggtta ctttttcact 45780
   ctgttgattg tttcctttgc tgtgcaggag ctatttagct tgaaaaaatc caacttgtct 45840
   gttttctttt gttgcctgta cctttggtgt cacattcaag aaataattgc caaattcata 45900
   ccatgaaact ttcccccatg ttttctcctg aaggttttta tagttttagc tctcacattt 45960
   aggtgtttga tccattttga gttaaatttt gtatataatg ttatgtaaga gtccagcttc 46020
   acacttttgg atgtgaatat ctagttttcc cagcattatt tgttgaaaag agtgtctttt 46080
   tccccattga atagtcttgg cactcttgtt gaaaattatt tgacaataga tgcaagggtt 46140
   tatttttggg ctctctcgac tattctgtta gactatatgt ttgttttttt atgtcaggac 46200
   caccctaatt ttagtactgt agctttatag taaaatttga aaccaggaag tatatgtctt 46260
   gtgtatttat ttacttattt tttgaaatag catctggctc tgttgcccag gctggagtgc 46320
   agtggcacaa tcttagctca ctgcaacctc cacatctgag gttcaagcaa tcctcccacc 46380
   tcagcctcct gagtagctgg gattgtagac acataccacc atgctcagct agtttttgta 46440
   tttcttgtag agacagggtt ttgccatatt gcccaggtgg gtctcgaact cctgagccca 46500
   agcagtctgc cctcctcagt gtcccaaagt gttgcgatta caggtatgag ccaccgtgca 46560
   tggccccaac ttcttatatt tcaagatggt tttggccctt cagggccctt tgtgagtttt 46620
   aggatggatt ttttttttaa cttttaagtt taggggtgca tgtgcaggtt tattacatag 46680
   gtaaatttgt gtcaaggtgt tctgttgtat agattatttc atcacccagg tattaagcct 46740
   agtacccatt agttattttt cctgagctcg cctcctccca cctggatttt tttttttatt 46800
   tctaccagaa acattgttgg gattttggta gggattgtat tagtctgtag attgcattga 46860
   atagtactga catcttaaca atattaagtc tttaaagcca tgaacaccag atgtctttcc 46920
   atttatttac gtattctttc ttttctttca gcaatgtttt gtaattttca gtgtacaagt 46980
   attttacctc cttggttaag ttaattccta agtattttat tcattctgat gatcttataa 47040
   atctgttttc ttaatttcct ttcctaattg ttcattctta gggtatagaa acacaactga 47100
   ttcttcgcac attaaatttg tgccctgctt cttcgcgggt ttgtttattc tttttttgtg 47160
   tttgaaatcc ttgaggtttt ctgcatataa gattatatca tctgcaaatg agataatttt 47220
   acttgttcct ttccaatttg agatgatttt tattcatttt cttaatgctc tctcatacat 47280
   tcaatactat gttgaatgga agtggtgaaa gcaggcatcc tgtcttgttt ctgaccttat 47340
   aggaaaagct ttcaattctt tgccattgac tatcatgtta gctatgggat tttttttttc 47400
   cccccagata gagtctcgct gtgtcgccca ggctggagtg cagtggtgcg atctcggttc 47460
   actgcaccct cctcctcccg ccaggttcaa gtgattctcc tgcttcagcc tcccaagtag 47520
   ctgggattac aggtgtccac cactatgccc agctaatttt cgtattttta gtagaaacat 47580
   ggtttcacca tgttggccaa gctggtctcg aactcttggc ctcaagtgat tcacctgcct 47640
   cggcctccca tagtgctggg attatagtca gccaccatgc ctggccactg tgggattttt 47700
   atatatggcc ttcattatgt tgtggtaatt tctttttatt cttagtttat tgagtgtttt 47760
   tatcataaaa tcttgttgaa ttttttcaaa tattttttct gtgctagttg agatgaccat 47820
   gtgatttgtt ttcttctttc tattaacatg atatattgtt tttcatatat tgagccattt 47880
   ttgcatccca ggaataaatt ttacttggtc ttcgtgtata atccatttaa taagctgtgg 47940
   aattcagttt gttggttctg tgttgaggac tttatatcaa tgttcctaag ggctactggt 48000
   ctatagtttt cttttgtagt ttctttgact ttgctatcag ggcaatgctg gcctcattga 48060
   atgtgttagg aagtgtttcc tcatccattt ttggcaaaac tttgggaaaa aacgatgttc 48120
   tttaaatgtt tgatagaatt cacagataaa aaaatcacat ctagggcttt tgtctggaat 48180
   ttttttattg ttattattga ttcagtcttg ttactagtta taggtctatt cagattttct 48240
   ttttgtgtgt gtgattcagt attagtacat tttgtacttc tcgttatttc tccatttaat 48300
   ctatattatc taatttgttg gcatacaatt gttcatagta ctgtcttctc ttttttttaa 48360
   acttctgtgc agttgatact aatgtcccta cttttatttc agattttagt aatttgaatc 48420
   ttctttatct taatacaggt aaagctgggt caattgttaa aattttttca aagaaccagt 48480
   ttttggtttc attgattttt ctctgttatt tttctattat ttatatcctc tctaagcttt 48540
   gttatttcct tcatcctgct agctttgggt ttattagttt gttctttttc tagttcctta 48600
   agatttgaag ttggattatt gatttgagat cgttttcaat taaatgtgta caactacaaa 48660
   tttccctctt aggactgctt ttgctgttct gtaatttttg gcatgttgtg tttttttgtt 48720
   ttaatttatt tctaagtatt ttctaagttc ccttgtgatt tcttctgcgt ttaaccgtgt 48780
   attttttaat ttccacagtt ggtgaatttt ctactttttc ttcagttatt gattttattg 48840
   attttcagtg gcatcctgtt gtgatagaag atactttatt tggttccctg tttttttttt 48900
   aaaacagagt gttgctctgt cacccaggct ggagtgcagt ggtgcgatct tggctcactg 48960
   caacatccac ctcctgggtt cgagcaattc tcctggtctc agcctcccca gtaggtagga 49020
   ttacaggcac atgccaccat gcccagctaa tttttgtatt tttagtagag acagggtttc 49080
   gccatgttgg ccaggatgat ctcgaactcc tgacttcaag tgatccaccc gccttggcct 49140
   cccgaagtgc taggattata ggcgttagcc accttgtctg gcccagatcc tctattttct 49200
   tgcctatatt ctgactgggt gtttttgtcc attattgaga gtagggtatc gaagtgtcca 49260
   gctgttattt cagaactgtc tgtttacctt caattctgtc aattttttgc ttcatataat 49320
   tgggtggtct cttattaggc atgtaaatgt ttttgattat tatatcttct tgctatattg 49380
   acacttattg atgtgtaata tcttttttgt ctcaaccttg ttttgattta gtttgtctaa 49440
   tattaatgta gctacctgca ctctcatttg gttattactt gtatagaatg tctttttaca 49500
   tcccttattt gtgtctttgg atctgaaatg agtctcttgt agacagcata tacaatctct 49560
   tgtagattgt gtttttctta tacattctgt caaactctgc cttttgattg aagagtttta 49620
   tcatttacaa ttaaagtaat tattgataag gatttaactg ctgccatttt gctgtttatt 49680
   ttctatatgc cttacagctt ttttgtccct catttcctgc cttactggca cttgtgttta 49740
   gttgatattt tgtggtgaag tgttttaatt tccttgttat ttccttttgt ttatattctg 49800
   ctattttctg tgtgtgtgtg tgtgtggttg ccattggggg tcacatttaa tgccctaaag 49860
   ttataacact gcaatttcaa tttataccaa tttactttca atagcataca aaaattcagc 49920
   tcataagatt cagtccctgc tcccttccag ttattgatgt cataaaatta catttttatg 49980
   tattgtgtgt ctaaaagcgt agactaataa ttgtttttta atgcagtagt gtcttaattt 50040
   gtggaaaaca aaaagtggag ttgtaaacca atgttacaat aatgctagct ttggtaattg 50100
   ctcatgtatt tatcatttct cagatcttta tttcttaata cagcttcaag ttactgtcta 50160
   gtctcctttt atttcagcct gaaagactca cttcagcgtt tcttgcagga caggtctggt 50220
   gataatgaac tctctcagat tttgttaatc tggaaatgtc ttaatgtctt catttttaag 50280
   gacatttttg ctggatatag tattctcagt tgacaggtat ttgtgtttat ttgtttgttt 50340
   cctttcagga ttttaaatat atcatcccac tgccttctgt ccttcaagag ttctgatgag 50400
   aaatctgctg atattgagga tccctttgta tgttacaagt tgcttctctc attccatgtt 50460
   caggattctc ttttttcata gtttgattat aatctatctc agtttttcct acttggatct 50520
   ctgagttttt cctacttgga gttaattgag cttcttgaat atttatattc atgtctcatc 50580
   aaatttggga agtttttgat taatatttct tcacataatc ttttttgccc cttttctctc 50640
   tttttattct gggattccca gagtgtgtat gtgttggtcc acttgatgat ggtgttccac 50700
   aggtgtctta ggctcttgtc ttcaattttc cttcagtttt ttttttctgt tcctcagaca 50760
   cgtggtattt tcagctgttc tgccttccaa gtttactgat tcttctgcct gcccaaattg 50820
   gcttttgaat tcctctagta aatttttatt tcagtttttg tacttttcag ctccagcatt 50880
   tattttttga tttttttatg ttttctcttt attgatattt caattttgtt ttttgacatt 50940
   atccatatct tcctttagct ttttgagcac ctttcaacat ttgttttaaa gtctatgtct 51000
   agtaagtgtc tgccatctga tcttctcagg cacagtttct gttaatttat ttttttcctt 51060
   ttggcctata cttaatgttt tgcttggggt agtttttttt tttggtatgc tttgtgattt 51120
   ttgttgttgt tgtcaaaaac tggaatttga atcttaaaga gtggtaactt tggaaattag 51180
   atttttctct tttctctaag atgtgctatt ttgtttggtt tttttatttg ttgtagagta 51240
   tttctatgct gggtgtaatc ttaagatctt ctcggcctgt gtttttccct gggcatgtgt 51300
   agtgactttc taaattgccc tatgtatgca gttcttttgc agtagtatcc tccttaaatg 51360
   tttggctcct aaaaggcaaa ataaataaat aaataaataa aaattaaaaa ttaaaaataa 51420
   atcaaagggg tgaaatagct ctggatcttt aaatccctgg agcaattttt tcagccaatg 51480
   gcagttaaat aatgatagtc tgcctctgtg tcacattttg atcagaagca gcaattagca 51540
   atcagaacac agattcctga tatttggagg gcaaggtctt tgttgccaac cttgactctt 51600
   acaaactgtg tgcagggtgc tctgggaaca tgtgcatggt tgcctgcttt gagagtgggt 51660
   gatgggtagc cgcgacggca caaagagctg aaattgactc aaactaactg atttaccatt 51720
   caagtctttc cttagaaact gaaaacctga atagactcca gagttccaga atcacagatt 51780
   ctgcttacag tcgtctaggt ggggagatgg gttcctggta cttctgattc tgccatcttt 51840
   ctttgactaa tttttttttt ttgttcttga gatggagtct tactctgttg cccgcccagg 51900
   ctggagtaca gtagcatgac ctcggctcac tgcaacctct gcctcccggg ttcaggcaat 51960
   tctcctgcct cagcttccca agtagctgga attacaggcg tgcaccacca tgcctggcta 52020
   attttttgta tttttagtag agacagggtt tcaccatgtt ggccaggcta gtctcgaact 52080
   cctgacctca ggtgatcaac ccgcctcagc ctcccaaagt gctaggatta caggtgtgag 52140
   ccactgcacc cagctcttag acaaattttt tattccaaac tttttttatt ttatcatttg 52200
   aaaggtatat gtttattatt ttgtcaaaaa taattttaaa acgtattctt gaagcttatt 52260
   tagatctgtt tcataggaac tgtgaagaaa gtaaagaatt taaaaaatga agacagattt 52320
   tctcaccctg cttatgggtg cttctcgtgc tagccttttg caagtgtcgg gaagtgtaac 52380
   ctgcaggagg catcagggct ttgggcctgc atggtctgag tgctgccctg tgagtttcag 52440
   aaggcgcagc aacctgtata cctgaaagcc atctctgctg gggcaggtac ctagtgtccc 52500
   cacctacctg ggtcgtagtc aggccctggg caagcctgct atgcttttcc ttccctaatc 52560
   cctcaggggt gggatagaga gcacagtggc ctcccaggga ggtagaagct gctccagact 52620
   aacaatcaga gctgccagtt cttaatcccc aagaccgcca gacttcacaa agacataccg 52680
   aggtctgtgc tgtcagtgcc ccactactac actcccttaa gtagccccac attcttgtgc 52740
   ttgtttcttt tttctgctct ctttccttgc ccaggtaaga ggtctgccca taagggatat 52800
   tttgcagcat gtgaagcttt ttaaaaagtt aggcttattg aagtataatt tacacacaaa 52860
   gtacaaaaaa aaaaagactg tgttctcaaa tctgtgagtc attaatgggt ttagatgttt 52920
   atatattgaa attattggaa gtaaggtatg tttatattag aaagatttgt agtctagatt 52980
   atccaagttt tgggagtatt acctctctgc ttttgtttat ctactttttt agtctctact 53040
   ttccaagtat ctataggcaa attttcccat ttccctttgg aaagtgctgt tttcttgctt 53100
   tttttccgcc tttccattgt gtcagactta taaggcaatc agccaactgt gggcatgaaa 53160
   tccttgggag gaaagagaag gaagtgggag gggcagccat ggtgaatgtt tccctaagtt 53220
   atagtcaagt tctttgagag aacataacct catccccttt ttaaactgtt gtaatacttt 53280
   cttttaaata gattgtttat tctcctgcaa gtctcacagt tgttcacagt ggtaggtaag 53340
   aaatcataaa gttcaaatat taaagggagc tcacaaaaga gcatggtttc accagccctc 53400
   actaaaaaca aaattatggg aaaatgctgt aaaagaaacc agaattcttg gttgcaaatg 53460
   atagaaagtg actctgattt acctaatcag aaaggaattt ttaaaaaagt attaggtagg 53520
   tggaagctaa gggaagcaag acatggcccc aaggtttcaa caggagcaat ctgcttagga 53580
   ctttgctgct tggacacttg gtgtaatagc tgctgccact atgcctcgaa actggtgact 53640
   ctgctcatta actcacctcc tctggtgatc tctaggaata atctctgact ctcctgtacc 53700
   ttgtcctcac taggattcgg tatccacggc aaaaagatct attaatagtt ggtatcaggc 53760
   ctgtacatgt gttaagagaa agatgaggaa agaagtatct gcttctaatc tcttgaaatt 53820
   atctccaaat tgaaatggta ttttggttgc ctaacagcct gaagatgaca aatatcccct 53880
   acaaatttct cctattttac cctcttccta actatatctg taatttaaag tttcacatat 53940
   tcttttgaaa attgttttca ttgtttaccc actttttaag aaaagcaaat gggaacatac 54000
   taccactgtt tggccccttt caaaaatttt atatctgagg aatcttccat attgttgtgg 54060
   acatctacct acctgattct tttattaact accttttatt tcattttatg atcatgctat 54120
   cattaatagg cccctatgat gaatatataa gttgtttcca gttttttttt tgtcattgag 54180
   aacagttcac atatgtatct tgttgtcttt tccaagtata tctttaaggc aaattcttag 54240
   cagtggagtt gctaggtcca ttgcgtctat ggtttaaact agtgccttca aaaatggtat 54300
   tttatatact cactgtttaa gagtgcttct tccttctatc cccactaact ttggcaaact 54360
   gaatagtttc aaactttaac tttttatagc ttgttggcaa aaaatggtat cttgttattt 54420
   gatcgtgttt ttttaattgt gaggtttagc gactttttga tgtattggtc ttatgtactt 54480
   tctggtgtgt gtgtatgtat atactgaccc tattttcaac ttatttttct tttagtttgt 54540
   ttgtattttc cttaatgatt ttcaggaaac caattttatt ctttcttcag acagttgtct 54600
   aatgttctgc ttctcttgcc atttgaattt tgtgactaca aaattcagat gaaacaataa 54660
   tagcataaag aacttggtgg gttatctttt gttttgcatt attgattgtt tattaagaaa 54720
   tattctttaa aagtcacctt gcttaaatta gcaagtagga aatgctttca ataaagagaa 54780
   ctgtcatgta cccactactc cttacttact gaatcatctt cttttggata gagaagataa 54840
   aagtgaaaag ggaatttaag agttcctgcc tttttccttg tctttagcat tatatagctg 54900
   tttaatgtgt gggagtctaa tttctttttt ctttcttgag acaaagtctc actctgttgc 54960
   ccaggctgga gtgcagtggc acagtcttgg ctcactgcaa cctctgcctc ccaggttcaa 55020
   gcaattctcc tgccttagcc tcctgagtag ctgggactac aggcatgtgc caccatgccc 55080
   ggctaatttt tgtattttta gtagatatgg gacttcacca tgttggccag gctggtcttg 55140
   aactcctgac ctctagtgat ctgcctgctt tggcctccca aagtgctggg attacaggca 55200
   tgagccactg cacctggcct aattttttta ttgttctttt tggtgtgaac attctcccct 55260
   cctccaagcc ttttgttttt actattttca tgttccttta tatgtgctgc tgttttgttt 55320
   catctgtaat tatctctcat cccctttttt ggctattata atatatatat gtacgttttg 55380
   aatctgagct ttgaaggtaa attcactgca gctgtgttgg ttgattttag ataatttgtg 55440
   tatttcctcc tttgtctttt ttaaactgga gtcatttgta gttgtttata cagaatttta 55500
   gtttttaaaa ccacaagtct ttcattatag gttgagttat gaattcatag cctgttattt 55560
   aaatgaagct tttgaaatct gttttactga tctgtatcat atctaactac gccagtattt 55620
   ccttccttgt ctgacgtgaa ctctaaaatt atgtgaacac tttctccctg tttcctggca 55680
   tttccactca aacttgttcc tcattcttag ttagaaatat atccagaatt gtagtttctt 55740
   tctaatctaa tgacagaagc aaattaatca agcatggcaa gaatttattg gaaaactgca 55800
   tgtagttgaa aatatgttta gtatatattt tgacagctgt gaagtctcta atttttactg 55860
   taccttttct ctgttccaat tttatgctct attctaagga tgtacccatt tctactacct 55920
   gactagggag catgtgtatt gtatcccagc agattttttt tttcatagat agatatcctt 55980
   tagatatctg ttatccagtg taggtagcca ctagccacat gtagctatca ttatgtttaa 56040
   atgtaaataa aataaaataa atttactgag ttgtttttgc tagctacatt tcttgtgctc 56100
   agtagctaca tgtggcttgt gattactgta ttaagacagc acagatacag aacattttca 56160
   ttattgcaaa agttctgtta gacagtgctg ttctatacag tgtcattctg cctctcattc 56220
   taaaaagttc taattcctga agttgatgta ctctttctgt tgctgtcctc tagcttaatc 56280
   aaaataaatt tgagtctttt taaaggtagg ttgcatttta catactgata tttctaaatc 56340
   agaggctatt tatattactt tttttatatt acttttaaaa attagcttta ttggagtata 56400
   atttacatgc aataaaatct acccatttta aatgtacggt tcattgactt ttgagaaata 56460
   cacacacaca cacacacaca cacacacctt cttgtaaaca cacaccttct tgtaaacaca 56520
   acaaccaaga tttagaacac tcgctttatg aaaagtttcc ctcatgccca tttgtagtca 56580
   gtccccaaac ctggtttcag gcaatctctg atctgctttc tatatgcttt gcctatacta 56640
   ggattacata taaatacagt catatagcat gtattccttt ttgtgtctgg cttctttctt 56700
   ttagtataat atttttgaaa tttatccctg ttgttactag tatcaataat ttgttctttt 56760
   ttattgctga ctaatattac attgtatgga tatgacattt ctttattagt gtggtgggca 56820
   tttgagttgt tttcagtttg ggtctgttat gaacaaagct gctgtaagca ttcatgtgca 56880
   agacttttgt ggacatatat ttttgtttct gtttattcaa tacctttgag tagaattgtt 56940
   gggtcacatg atgtagatca gttgaacaga gtagattcca gaaaagttca catacacatt 57000
   tcttgacaaa ggtgctgaga ttattcatgg gaaaaggata atcttttaaa caaataatac 57060
   tggaacaata gagaaaacaa agtgaacctt gacttttatg tcttatcata tacaaaaatt 57120
   aatttgaagt ggattgttga cctaaatgta aaagtaaaat ttaaaaatat aaaacttcta 57180
   gatgaaaaca taggagaaaa tctctgtgac ttttggttta aagatttctt agacagtaca 57240
   tataaaatta actatataag gaaaaaatgg acaaatttga ctttatcaac attaaaaatt 57300
   tctgctcatt gaaagactca aaatgaaaag gcaagcagtt ttggagaaaa tatttgcaat 57360
   acatatatct gaaaaaggac ttgaatgtat aatatataca taaagatgct cttacaactt 57420
   cataatgaga aaataacccc ataaagagaa gggcaggccg ggtgcagtgg ctcatgccta 57480
   taatgccagc acttttggag gctgaggtgg gtgaattgct tgagcccagg agtttgagac 57540
   cagcctgggc aacatggtga aacccagtct ctacaaaata aaaaaataca agaaattagc 57600
   tgggcatgat ggcatgcacc tgtagtccta gctgtttggg aggctgaggt gggaggatag 57660
   cttgagcctg ggaggcggag gctgcagtga gctgtgatcg caccgctgca cgcttgcctg 57720
   agcaacacag tgagatcctg tctcaaaaca aacaaacaaa aaaaaaaaca aaaaatggaa 57780
   acagaaattt tacaaaagaa gatatataga tggccagtag gcatatgaaa agatgtttaa 57840
   aatcagtcat cagggaaatg aaaatttaaa cgtaatgaga tagctcatat ttactggaat 57900
   ggctcaaaaa gggcttacag gaattggcaa agacatagat taactggaac tcttatgcat 57960
   gttggttaga gcacaaaatg atatgatttc ttgggagaaa tatttggcag tttttaagat 58020
   tatttttgat agccttctga atttcttagt gagttatagg tcagttctgc cactgtttct 58080
   ttcttttctt tctttctttc tttccttcct tcccttcctt cccttccttg cctgtcttgc 58140
   ctgccttcct tgccttgcct gccttgcctt cctttcttcc tttcttccct ttcttttctt 58200
   tcttttcttt ttttttttaa aggagtctcg ttttgttgcc caggctggag tgcagtggca 58260
   cgatcttggc tcactgcaac ctccacctcc cgggttcaag caattctccc tgcctcagct 58320
   tccccaatag ctgggattac aggcgcgttc caccatactt ggctaatttt tttaattttg 58380
   gcagaggcag ggtttcactg tgttggccag gctagtctcg aacacctgac ctcaagtgat 58440
   ctgcccgcct tggcctccca gagtactggg attacaggtg tgagccactg cgcctggcct 58500
   ggcactgttt atttcttttc cctccagttt tatacctatt tagagagatt agattttctt 58560
   gagtactagg aatcactatt tttgagcaga attattcaaa actgttatta ttttttcttt 58620
   aacttgaggc aatgtaggag aaagcagtac tgtgcaggtg aaagttacaa acaagaacat 58680
   tttaaacaag atagttactt tccatgtatt ggatacgtaa cagaattaat tctaataacc 58740
   atcctgaaga tggtcaggag gcattagtta agaattgaaa tgtttggagc ttgcctgtgt 58800
   tgatgggatt aaggcaggga tgatttatgt gtaaatttat gcgttagtaa cagcagtaac 58860
   cgctgtagtt acactagggt tctaagagca aatgttgatt aaacatgaat gtagcaggag 58920
   tgataaggtt tggctctgtg tccccaccca aatctcatgt ggagttgtga tcctcagtgt 58980
   tggaggaggg gcttggtagg aggtgattgg atcatgggag tggtttgtaa tggttttagc 59040
   actatcaccc tagagctgtc tcgcgaaaga gttctcctga gatctgcttg tatataagtg 59100
   tgtagcacct cccctctttg ctctctctct tcctcctact cctgccgcgg ggacgtgctt 59160
   gctttccctt ggccttctgc catgattgta agtttcctga ggcctctgat taaacctttc 59220
   ttcttctaaa agattaccca gtctcaggta gttctttata gcagtgtgag aatggactaa 59280
   tacaagggga aatatatatg gttaccaaat agcgaattag ccatgggaaa aagtagcaaa 59340
   taaataatta ttttactttt tcagatgcta atttttcttt tcgtttattt taggattggt 59400
   gggagctgtc caatgtcctt aggctgtttt ccaaatgaga taccaaaagc tagttctcca 59460
   tcgggtttct caggctgcta gaagcattca ttattatggt tgtcattact tcgagttctg 59520
   ttgccgctat gcccacagta gtatttgtta cataacaggt gcttgataaa tatttgctaa 59580
   atgaattttt ggaaaataca atctgccaca cctttcttct acagtttaca atcttctgtt 59640
   gagatcatcc gatagatttt ttttcttaga tattgtactt ttgaggcctc aaattgctgt 59700
   cttttgtatt ttctatgtct gcagagactt tccatctttc actcattgta ttcattgttt 59760
   tttaacatct ttgtacatat ttatagtaac tgttttaaag tcactgtctg ttaattcaaa 59820
   catctggttc atcttggagt ctgattctat tgcctgctct tttttctttg taataggtca 59880
   tgtttttctg ctttgcctgt ctagtaaatt ttaatcgtat gttgaaatgt agggagtttg 59940
   gattgttact tcctttaagg gtgctgagtt tcattttgtc aggcatttaa attgatagtt 60000
   gatttagtat tgtcaggttt ggttctcttt gttaaagcag gcatttttca gatttgtctt 60060
   ttgtcctagg gcatggtttt taacttcaag gttgcccttt ccaatgtctc agctaagtat 60120
   ctggggtgtt ccatgaggtc tcttccactt tgcctaggcc agaactccag cttctcccag 60180
   tattatattt cgttacctct ggcgtcatct ccgttatgct ttcagatcct gcgcatagac 60240
   agcccagccc ccagccaagg acctgagatg aaatccatac aaaattctta gtcccttgct 60300
   ccacaaactc caacagcctt agcagtctaa tctcttcctg tttacctcag tgaaatctgt 60360
   gttccacttg agttccattt ccttctgtat cagagaagag ccaccatgct gaaagcaagg 60420
   ggcactatgt ttctttgttc ttaaggatgg tagcctatct gcaacaactg tagtgtgata 60480
   taaaaatata taatttatgt tgctgacagt tacaaatact gcttgcagta ctttgtaaca 60540
   taatttttca gattcaagtt catatactct ttttttccac atcaccacac acatattttc 60600
   agacttcctc ctcatccttc ttcttgccag tagttgtatt ataattcctg ccagtagtta 60660
   cattataatt ttggttatat caatattgag tttttatggg attataacta gataaatgcc 60720
   attcatagtt aagtgataga gtatattgtg acttttttcc tgcatgtttt attttttctg 60780
   gacttcacag ttgtctctct tttttttaaa aaaattagtt ttcaatgttc ttagctttaa 60840
   ttcataaact cacccctaat tgtataaatc tctcaacatg tttaagcaca tttggcatta 60900
   tatcaatttt atcttttcca ggtgccttct aatctgtccc agtctggact aattgttctt 60960
   cctggcttgc tgtatggctg tctactcaag atgtcccttc accatcattc tagggattcc 61020
   cttttcctct cttgtgggtt agattcttca gttcttggag actgtcatct tctttcatgg 61080
   tttcccactc ttgttttggc ggagcacatc tttagtaact tcctgacaaa gtgtatggtt 61140
   tgagatttcg ctgattttaa aatgccctta ttatatagtc acacttgatt tatagtctgt 61200
   cttggtatag aattctaggc tgagaagagt tttccctcaa aatcagaagg ttttgcccaa 61260
   ttgtttttta gctgctagta ttgctgttaa aaaggataat gtcattttga ttctagattc 61320
   ttttatgaaa cctgtttctt ctctggcagc ttttaggatc ttctgtttct ttggtattca 61380
   gaaatttcat gagatatgtg tgcttctatt ttggtcttat tttcatctgt tttgccaggt 61440
   actcatgcaa ctttccagtt tgaaaactca catccttcac ttttgagtat ttttcttgag 61500
   ttatgtcttc ggtttcttct cagtgttctc tgtttctgga actcctaaaa tatatttaac 61560
   atcctgaact cttagttttt gtttagtctt ctgattttca tttgtctttt tattctgtat 61620
   attctgttaa ttcctcggct tcatggtctt ctagcccttc ttttgcctat cttatggggt 61680
   tgaggattaa acagtttata tacctgaggt gcttaggata tgtctgtcat atagtaagtg 61740
   cttgtgttag ctgtaattgt tgtttacttt cataactgtc ttgagggaaa ggtctttggt 61800
   cttgattctt tgacttcttg gctgtacatg accttggacg agttatgtaa tctctttgag 61860
   acctacctcc ctcttctgta tagtgttaat aagctctagc tctcagatgt ttgtgagggt 61920
   cgaatggagt atatatgtga aaatgtttaa tacctttgta cagaattaat agttagtacg 61980
   tggatctttc aaatatcaaa agttttcagt ttgatgggaa aatgatgtct gaattttcag 62040
   ggttattttt aagagtactt gattatgact gtcttgtaaa tctctatgag ctaggtatac 62100
   ttgcactaaa tgctaatgct ttttaaagaa gttatgtctt aatattcagt ctcattatgt 62160
   taggttgaag atagaagatt atgaaaatat tctctgaaaa gctctggttt tacttcagat 62220
   tgtataaatc tgtgtaatgt aataattatt taagaatgac atgattacta ctctaaaccc 62280
   atagaagggg tatttgttgg attatttatt ttcacttaaa tggtatttga gattaggaaa 62340
   aagaaaatct gtcttttggt ttttcttgat agtattaatg taatttcaaa tgttagctca 62400
   tttttgttaa tggtggcttt ttgtttgttt gttttgtttt aaggtttttg gattcaaagc 62460
   ataaaaacca ttacaagata tacaatctgt aagtatgttt tcttatttgt atgcttgcaa 62520
   atatcttcta aaacaactat taagtgaaag ttatctgctt gttagagtga ggtagagtta 62580
   aagatacatt ttaacagaat tgtattccta aaccgattaa gtcaagaagt ccaagagcat 62640
   tgttagatca tttagaaagt gtagtgatga ggtaaaacat tgttggcaca gattcatgtt 62700
   acttgatctg ctttaaatga cttggcatct agcccatatt tgagcccata accgtgtggt 62760
   aatttgaagt gtaattcaca gtagagcttc tgttaaagca ctaatagcat cttccatgga 62820
   ggtatacttc agagtgaata taattttgtt tatcctgtgt ctctagagct attgactgaa 62880
   aaagctgtta gggcattctc taactgtaca tcacctaagt tatttaaaat tgctgaatta 62940
   ggtggcttgt cttgtctagg acagagtttt aaggactgcc cacctgattg atagagctag 63000
   ttgaccttat ctttaacttt ttgtttttct tttgactttg ggagtagaga tgtgaaaagg 63060
   taaaaaggaa ggaaggaaga gaaaacttaa ctctttttgc ccatgaagac tgtttttcct 63120
   tctcaaaata ttgactattt tctgatttgt aaaaatcggc acataaaacg tgttattttt 63180
   tacttgactt ttatctttcc catgtgatat ctataaatta tagataggaa aaatttatct 63240
   gtaatttagt gatctttcta gtgtgataaa acgtcagaag tactgagagt ggagtggaca 63300
   ttgatattgt tactctcagt aagttttcac tgatttttct cagagtcatg aaggaacaaa 63360
   cgtttgttaa gtccttatca cttattagat aacacaaaac atgttggggg ggtgtgtaca 63420
   gaggtgagta agatgtagct cccattctca agtcgcttac attctaatgt aaaaggtaga 63480
   caaagcatta cagaagaagt aactctgcta tagaaggttg caatgaagag aacattggaa 63540
   acactaattt taccttataa agaaggtttc ataaaggaag gcaagtttga gctggggtga 63600
   aaaggaccag taagggttga ctttcaagcc aaggagagga ggggaagtga tgttacaggc 63660
   caaaggaatg gcattgtaag aagcttgttg gcataaaagt gtttagaata tggcagcgaa 63720
   ttcattatca tcagattgtg gtgtctgtat gttgggggtg ggagagaatt gtggtggcaa 63780
   taggcaacaa gataaaagaa agtaaaaggt gttatggaaa cttaatgggt ccagcttaca 63840
   aatgatctat gcatttaggg gtctttctct tttcctgata aacctctcct acaaagagcc 63900
   ttgttgcgga taccatagtg tttctttgga ggaaaataaa aactacaaag ctttgtattt 63960
   tttgcacaac tggattcaga atataagtaa taaaaaagga caagaacttt caaaagctag 64020
   aagccattaa actgagtcac ttcagggtta gactatcaga actggggatt tagaaagtct 64080
   cagaatggaa atcgaaggac accaaagaca aattcggcct ttttcaaaat tttattctag 64140
   tttaacatat tcaaagaaag ggaaggaaat tcttttcatt cctgtgtgta gtgacttcct 64200
   gctttaagaa cttaggactt cagctgtact atcagtattg taggtcactt aacattatta 64260
   tggttaaagt tggcattgga gagagcctag gaacctaact gcctgtttgt ttttatattt 64320
   ccaaccattg gattcccaag ttaatgaagt ctgtttatta gttgagggta gctcttaatg 64380
   catatatttt aatgcccctt ccccacatgg aatcataagc tttcagaact ggagagtacc 64440
   tgaaagagat catttagtcc aaccttctca ttttacagat ggggaatctg aggcctagag 64500
   aagttaagtg agttgaacaa ggtcacacag gtacatatgg tagccgacca tccactgttt 64560
   atgccaatat tccctttacg ttttgctttt ttgcttgttc gttttaacct ctccaaattt 64620
   tactgacttc agaagtttct agaactaagt tatagcatgt tttgagttct aatgtcactt 64680
   tccgatcttc tttacctttt ttctacctct gtttgtattt ctggttctgg ttaagtgagt 64740
   ctggtaagca gcaggtgttc tattttattt cttttatttt taggatagta ttacatgtga 64800
   tatatatgtc tttgcaaaca tacataattt gaagatctta aaatatttgc actaggcata 64860
   cccacattta atagtatgtt aaatctttta tagcaattat gatatacatg ggtgaagaag 64920
   agttcctaat atggcctttc tgattaactg tatctgttta tatctgtgtt ttcttcaggc 64980
   attcataaca ttaagcaaat tcaggtgtac tgttacttaa ttgaattaat cagtttgttt 65040
   tgtacaagta tattttattt ttgttccttg ttgtataatc tggtaggaat ggggaagggg 65100
   agatagtgaa taaagagatg tatacttctt gcctttgagg aatttaagtt ttcactgtat 65160
   accaattttt taaaggtatt tactatattt cagtgcatat tttatttgac atactttatc 65220
   attttgtggt aaacctttag ctttactaat tttcatctat taagttttct tttgtaagat 65280
   ggtgatagct tcatcaaaga gagtaaagaa gagacctgcc tacctagctg attctatggc 65340
   aaatctcact tctctggaag cttttcctgt taatcttatt ccttcagttt ctgcctcttg 65400
   tttcataaaa actcattctt taaatgctta ttcatttctc ttgtctcata taaaccaata 65460
   tgaggtactg gtatcttttg agttttagtt ataaggaagc ataaatggtt aaatttaaat 65520
   ggctaaaccc catttgccat ttgtgtatct ttaattttag tttgttgaga gacttatcac 65580
   taccaaacca caaagaattt aaaagaaact gtcagtaggt ataggtggaa ggagggcatt 65640
   tatcagagat tttaatttaa gaagaaagtc ttcatcctta tcctaccaac ccccattccc 65700
   tgagcatatt tatcattact agtcccagca tatttgctcc catatttcct atgcttacct 65760
   gtgaagattt tcataacttt ttccttgctt tttactgtca ctgttggttc tgtgatttat 65820
   gacagatact gctcttgtag gaatgctggc tttgactgaa atttgttact gcttttgtat 65880
   ttaaaacttt ttttttatta taagtagaat tatggaacag tagtagaaaa agtttgactt 65940
   ttgtaatcag agatactgag cttgagttct ggctctttca tttgtatact gttatttggg 66000
   gcaagttttt taatgctctt aagtcttagc tttctcatat ataaaatgga gataataaca 66060
   gttatcacgt gattgtgagg atgaaacaaa aaaaagtgga aactctttgt aaggtgtgtt 66120
   catctggttg acacttagta gtcattactt ccactttccg tccatatagt cctcttaaca 66180
   gtaatatttg agaggcattt ttattaaagc agtcttaagg agtgttcgtc aaaccacatg 66240
   ttctgggatc ctgagaaagt aggggaagtt tagagaactg aagctgcaca aaactaatgt 66300
   ttattttctg ttgtgttgtc ctgagaccag cttcttagat tgtgtttcct agtcctacat 66360
   ctctgattcc ttataaaata ttccattatg aattcttcac tattgacaat ttctcccctt 66420
   ttatcttaaa agtaccaaag aaagtgtaaa atgtgactgt cttgtcagtc ctctttttcc 66480
   tgtttttcat gtcagtgggt atgaaattac tagcaaggat gcatatatgt gcatatgtca 66540
   ttactaaatg cattttcttt ctagaaaaac tcaatatact aaattgtact aaaaaggaaa 66600
   agcttgtttt gttttgagtg gtagtatgaa agttgtttta ttttaggtct gaccagttag 66660
   aaaccaatgg attgtagttt atttataatt agttaaacct tcatgtgaat ttggttttga 66720
   attaccttta aggtagagaa gaaactatat agatgttttt cagggtttct aaatgtacaa 66780
   tacaggttca caatcactta tttgaaactc ttggggccaa gtatgtttcc attttcagaa 66840
   attttagttt tcaaaaggta gcacagataa atatactttt acataaacac cccagtgggg 66900
   tgtgggtcag tacctgaaat gaaatgtttt actcttcgct ctaagtgtat taaatattat 66960
   gtacaatctt attacttcag atcaggattt gctgtagttg agtttgccat aaaacttaag 67020
   agaaaatttt agatgttttg aacttttggg atattgaaat tgcaggttaa ggagctatgg 67080
   acctttattt gttttaaaat gctaagagtt tattttaagt aatttttaaa aaatttgttt 67140
   tgcatagtag ttggagttac cagggtactg ctaaccacac tgatatgtaa gatctctttc 67200
   tgagcctttt attgtttgta aacatggcct gttaatcatt agaaagccag tacatactaa 67260
   catatcactg ctattaagac aaatattagc atactctagt aatgacaagt cagcatttta 67320
   ctattctgta ttgattttac ttattctttc attactctca tactgtaatt aaaacttgca 67380
   atctgagaga ctgttgaaaa aggtgatcgt tggcttttca acagggagta aggtctggtt 67440
   taaaaaaaaa ttagtaagca tttggccaag tagattaaca acattcagtt tttctttact 67500
   gtccttatgc ttttactatt tttaacatat atctttttga agaatagttt gagaattatg 67560
   tatgcttaac tatgagatac agatactatt gaaactagtc agttgtttat aggtacttgt 67620
   aaaattaaaa atatattcca atagcatgca gatttttcat agaggaaatt tgaaagcatg 67680
   gaagcacctg aatttacagt actctgtatt agtggcatca caagttttta agcaaatgta 67740
   ttagctctaa ttgcatacac ttaatctttt aagctttggt tttattatta taatatgggg 67800
   gtgataacag tatctactta atagaattct tgttattaca tgaaataatt aatgttaaac 67860
   acagcataat atgtgtcaca ttataaagat tcaggcaatg tttgttagta ttagtacttt 67920
   tttttcttcc taagtgcaaa agataacttt atatcacttt taaacttttc ttttagttgt 67980
   gctgaaagac attatgacac cgccaaattt aattgcagag gtaggtatga atgtactgta 68040
   ctatgttgta taacttaaac ccgatagact gtatcttact gtcataacaa taatgagtca 68100
   tccagattat cgagtgagat acatatttaa gaattatctt taaaaatttc aaaaatttta 68160
   attttactgt tgtgttttag gaaaaagtat tgcataaagc tattaatatt gtcaggaaga 68220
   ctaaagtgca gcatagacta agaattagga aaattcctag actaaaaata gtataaggag 68280
   agggtttacc tactatttga ggcagttggt ctaatagtaa gcaatcacag ggagaaagca 68340
   gaactactta actcttctgt gttgaggaat gacataaaag gtaggaaagg atataacaaa 68400
   tgttgataag aggagtctga tggatgagag gagggaactg ctttaaatga gtttctactt 68460
   cagacataag ttaattctca gagcccacaa aaactttcac ttttatttgt gaaatacaac 68520
   tcagttctca tggcttaaca ctttaaacca tgagaaaact gaagagttga gaagcttggc 68580
   agatgctgct gtgatagtca aaaagaaagt gggtgccatg agctactatt gatgtatttg 68640
   ccattgatcc ctcctgaaaa tctagaatgg actttcagac aaatggtttg aaaattctaa 68700
   atcactaatg attgagattt agtataggtt tactaagaac gggttttttt tgtttttgtt 68760
   tttggtggat ttaggctgtt gcttactaag caaagcaggc tttagttgag gtttatcttg 68820
   ctttaaacag atatttaaca gattttcctg gaggtttttg tgtaccactg ggaaaatgaa 68880
   gttaggcaga tgactaagtg aaagctgtcc tgctgactcc ttataatgat agtcattgtc 68940
   taccagaaga tctctcctgt cacaccaaag gataattgat tatatcctgt accatattat 69000
   gagtcacctg attggagata taagacatac ttctcacata tttagatgac acaggttagt 69060
   acattgaata tcagccaggg tttttaagga tcttaataga gtggaactaa ggtagaaact 69120
   attaagagca attaatagtg atatatctat agtcctgttt ctaaacaagt ttttttaaaa 69180
   acctcaactc tgactatagt gaacagagaa gtcttggact cttacaattc atgtgagaag 69240
   acctgaaact ttgataacaa ttatatacat tttgtgagta atttctttgg tgtatgcctt 69300
   cacatatctc tggtatgtga cctatgctgc agtccattga gcatagattc ccagaatgta 69360
   ttctcctgca gaaaatggag gaaaataata cttggcttcc ctaatgatta catgtgtata 69420
   caacactaac atttgcaaga ccacctttaa ataacacact tagcattttt attttatgaa 69480
   atgtaatatg tagttctttg catagtttat cctattagta atctattctg tctttggaat 69540
   atgttttgtg atgatgaaat aaatactata aatagtatta ttccttttgc attgagagtc 69600
   ctgacgaaat gtccatgtga cagttcattt tgggtttagc tctacctcta atatgtgacc 69660
   tatgctacca gtccgtatag cgtaaattcc cagaatatat cctcctgaat aaaatggggg 69720
   aaaataatac ctggcttcct taatgattat atttaagact tatcaagaga ctattttcta 69780
   tttaacaatt agaaagttaa gcaatacatt atttttctct ggaatccagt gtttctttta 69840
   aatacctgtt aagtttgtat gcaacatttc taaagttacc tacttgttaa ttaaaaattc 69900
   aagagttttt ttttcttatt ctgaggttat ctttttacca cagttgcaca atatcctttt 69960
   gaagaccata acccaccaca gctagaactt atcaaaccct tttgtgaaga tcttgaccaa 70020
   tggctaagtg aagatgacaa tcatgttgca gcaattcact gtaaagctgg aaagggacga 70080
   actggtgtaa tgatatgtgc atatttatta catcggggca aatttttaaa ggcacaagag 70140
   gccctagatt tctatgggga agtaaggacc agagacaaaa aggtaagtta ttttttgatg 70200
   tttttccttt cctcttcctg gatctgagaa tttattggaa aacagatttt gggtttcttt 70260
   ttttccttca gttttattga ggtgtaattg acaagtaaaa attatatata aatacaatgt 70320
   ataatatgat gttttgatgt atgtgtatat acattgtgaa atgattacta cagtcaaact 70380
   acttaacata ttcatcacct cacataatta ttattctccc cccagggtga aagcatttaa 70440
   gatctacaag ctacaatttt caattataca atgttattat taactatagt cactatgctg 70500
   tccagtagag cttcagatct tgttcatctt gtgttcctcc ctccccaccc tcagtccctg 70560
   gaaaacaggt tttaaagata gttgctaatc cttatttctt ctaaattttt aaatcagttg 70620
   ctgcctcaat ttctatatga gaaatgactg attgatttca tttttctgtt cacgctacca 70680
   ttttcatatc atactagcac atgttaccca ttaactgtat tgcagatttg gtctcacaaa 70740
   attcttctaa aataacattt ttaaaaagca tattaatcaa aaataagctt tatatttctg 70800
   aagcttgttt gagcatagaa tgcctttgga taaaatacca ttacctagta aagtgtgaac 70860
   ttttataatc cataaaaatt attcttttat aagaatattc ataaatgtag ttagattaat 70920
   agaagattct cgattctttg atcagaaaac taaggactat attgaaaaat cagtgacaaa 70980
   tttaattctt atagtacatc tgaaagaaaa aagaaaactc ttgggagaac ttttacagtg 71040
   atttaatttt gctgttgata tatttctttg ggtggtaagt atggcaaaac atgttaaaat 71100
   ttaatgcaaa gagattttgt acatttttcc atctctaaga aggacaaagc ctaagcccct 71160
   ccagatagat agaaaaactc atttagagag ttctccttca tgttaatcta atttcttctt 71220
   aattcagctg taaaacagaa atagaatgat cgtattaatc atttaaagct gtgtaattgc 71280
   atagattcct tgttccttta ccccctctta tatcttgttt cctatccttt gtgacttttt 71340
   ttgcattata tataaggatg ccgaaatact gtttattgtt gatagtttac aaaattgaat 71400
   cttacattag tgcataattt tggtgaatgt tgaagattat ggtagattgc cttacatttc 71460
   tgcatattgt ttgcaccttg gaatgatagc actggcatga attatagagc tgaggatcta 71520
   aagattttta ctttgattta tcccattatc atctgcaggg aaacaattgc ttttactgat 71580
   taaaaatgca ggctgggcac agcggctcac gcctgtaatc ccagtacttt gggaggccga 71640
   ggcgggcgga tcacaaggtc aagagatcga gaccatcctg gccaaccaac atggtgaaac 71700
   ctcatctcta ctaaaaatac aaaaattagc tgggtgtggt ggcgcgtgcc tgtaatccca 71760
   gctactcagg atgctgaggc aggagaatcg cttgaacccg ggaggtggag gttgcagtga 71820
   gccgagactg tgccactgca ctccagcctg gtgatagagg gagactccat ctcaaaaaaa 71880
   aaaaaaaatg cagtagcaaa agcgatggta gaaatttaaa acagagttga tgagcagcat 71940
   atattttggt agtggaaaaa aaggtaaaaa attttttgta ataaaataga aaaattttgt 72000
   aatgtggagg cgcagaacac tagatttaag ccagggggtc ttaaattgtg ttacattcct 72060
   tttaaagtct gatggaaggt ataaatgttc tcccctcaaa aaatgtgcat agtgtacata 72120
   aaattttgca gtttttatta cattgaaata tattctttta gacagaatgt aaaagaacct 72180
   tcatgaaaac tatgtcactt ttttatgcaa aaaccagtgg ctactacatg agagcaatga 72240
   ataaatctaa gtggtacaaa ttaaccaaaa ttaagcttta gttctgttca atactaaatt 72300
   ttaatgaaaa gactgctatt taacttttaa aataacaagt tgaaactatg ctctttgact 72360
   ttgactttgc aacttttata tgatctttga tatccaatca gtgttgactt tggtaaaaag 72420
   tgctgaaaat gctattttac aaaagaaaga agagtaaatg gaatctgtag attctattgc 72480
   ctgatgaaag tagacgtgtc aagaaataag aattctccaa ggctcttcag ataaattcat 72540
   gtttcatcat tttctttgcc ttcaagttac tgagatcatt tttggcaaga tctgtatcat 72600
   taatgctgtg ttaggaaaga aaagattatg actccacatt ttactttcaa ggttgaagag 72660
   ttaaactgtt taaaaagagt gtatgttatc ctgtaaacag cagtatcagg ctgtagaatt 72720
   tgtcttctga aagcagggaa cttatatata gcaaagaact tcatagtgct cccatttctt 72780
   gacaaaacct ctcgagaagc tcttgattga aagtcttggc tttcatgaat ctggcagctt 72840
   tcacaatagt ggatttttca tgacaaatca tcttacacag ggaattattc aagggttggc 72900
   acttgaaaca gtagaatact ttcacaacaa gagataagat ttctttcagg attgatgaca 72960
   gtcttgcacc ctagcgcata ctgatgaaga gagcagtggg tgaccatgac atggagagct 73020
   tctgtcttta ccagtgcccc aatatcagat gtgttgtctg gcagtaaggt gtactgtctg 73080
   cctacagaat actgaggttt cttcaggaga agttttttgg taaagaaact ttaccatttt 73140
   gaaagtgtta atgttttctg aagcttccaa aaagattcca aaatgggaat gtttccttga 73200
   ttgtgtcacc atgcttgcat ttgatgaaaa cttgtagccg gctatactga gaaatcatat 73260
   ctgaagaaag gtggtacttc caatcttttt gtgacctact ttattattgt ttttttaatg 73320
   tcagggtttt ttttggaatg gagaaaagta tttgatagag gtattgcaac agtcttattc 73380
   ttcttcatgc tacaagtata tttgactctt tctaagatac ttgccttcac tgttcaactg 73440
   tgtgactttt tgtttgttta gcattacaat caatatccta gtaggatgat ttaatcaatg 73500
   atttttaatt ggaacaaata gtttttgtaa tggtctaggc ttttccaact taactgtgct 73560
   ctcacatgtg gtctcttttt ctccctcttt cctccttctt atacactctc acccacacac 73620
   atatgcatac ataccctgtc tgatgtatct gcttcttcag aatagttggc tgtgctctgc 73680
   tgatgatgag aacttgccat ttaagaagga cttgggatag tccatgtcat catgttcagg 73740
   gataaaagta aaacccaagg gcatttaaac tttattgtat tttattttct gtttccagtc 73800
   caaattaaat ccaagagaag gctccataat caaaaagtaa ggacatattt taaatttgcc 73860
   aatgggaaga tattctagtc attacagtct ggtaatacta tcaattctgt ttctcttcag 73920
   aggtgagggg agactatttg atgaaatcgt aagtcctgta gggtgttgtg aaatagggcc 73980
   agaatgaaag atagcaagaa tagtgttatg aaaataaaat gcaaagttta taatatcatg 74040
   tggtaaaatg taatagtatt tacttcatca gtagaactgc tctagtagct gtatattctc 74100
   catccttgca taggttggaa tatcccccaa gtgaaaagag attgatgggc taatagttaa 74160
   tagaaaatgg agatctgtac atacagtgtt aagaatgtag atattaaaat tgttatattt 74220
   agctgttaca taatattaag actcagagtt aagtaatttc actgaaattg attgcttttt 74280
   gtgtcttgga gtcaaaataa ataactgaaa tctactatac ttggctcatg cttaattaat 74340
   atacttagac catatttcgg atgaattatt cacagaatct aaaggagtat cctcgtgttc 74400
   ttaccttctt tatccctgtg tttatttaaa aaggcaaaaa aaatggagca gatgctgttg 74460
   gttgaccata ttttactgaa cagtagcatt tgtgtttagg ttgaaacagc attagaaaac 74520
   tagatacgga ttaaagtcag tggtaggttt tttttttttt ttcttccagg aatgtttctt 74580
   atagatgatc aaacaggcac aggaagggga agtgttgtga tcaatattat ccagttaata 74640
   ttagcattca gaggaaaatt tgagtcctct gatacactgt taaatttctt tctatactat 74700
   caagtccaca aatcctggaa ctgcaaaaga attttgagac tgttcaaaat aattaatctc 74760
   tgtataggct caggctttcc tgcaaggtta tgaaatgctg ataaaattgg tcttattttg 74820
   aaaggctcct cagcttatac ctttccttac aaatgcttcc ttacaaatgc taaagcattt 74880
   aatgactcct gacttaaagg gaatttggac agattgaggt tgttggtctt ggaaatataa 74940
   tactgcaggc ttctgtaaaa tacttgaaat gtaattgttt taaaactttc aaagatacca 75000
   cttgtttgcc tgttggttag aatactggtg aaataatttt taatctttta tgaataacta 75060
   atttcatcat aagaaaactt agctaagcat ggtaaagctg ttgttataca actgtggaat 75120
   tcttcctgag gagtaactat cttataataa atgtagttga ttatctaaag tagttttatt 75180
   cttggaatat ctcataatag gtttattctc ttcttgtcag tatttccttg tagattgagc 75240
   ctgtggattt gcatttttgt aattgtgaat caccattata ggagatacat gcattttatc 75300
   tacttttcag tttgtatggg gttaacttta ttagaattat ctttaatgtt attttgctta 75360
   tatacttaat tttaattata gacaaacatt aagaagctgg agaaaattat gttctagtga 75420
   catttatata gaagaagaat cttttttccc cctttctttt ttgaagggag atgaggcagt 75480
   catattttgg taaagaattt gtagactttg cagaggtctc ttcaaaataa tctggctcag 75540
   agtcttgaca tatcctcagc agacatggtg caaattagat ggcagagtgg tgggtacaag 75600
   ttgaccataa aataacgcat taggttagta atgcccaaat aatactttgg gttttcagtg 75660
   ttgcagagaa gtcagacaac tgatagttat tataaagaaa aatgttctga gagtgaggta 75720
   accgcttaag ggaaggaagc ctccttctgt cttattcact aatttacaag aagataattg 75780
   tgttacactt ccttaggagt cattcatttg tatatttgac acttttgctt tatgaacatg 75840
   tgaagattat tcaaaagtaa gctgttggtg atttttttct tccaagaaag catgccacag 75900
   ggcaacttct agggttggtt ctcatctagt cctgtgctcc acactatctg catctgcact 75960
   taagtttcaa tattagataa ctcacatgtt taaactatga agaaagagtt aaaacatcct 76020
   gagaatgcta gtaagtatgt atttttgaaa ggacttccaa aatttgagtt taaagaggta 76080
   aactcctttt acatgacaaa gttacttaga aacactactg ctgtttccct ctcccttgcc 76140
   ttctccctgt cccatgcata cccccagctg tgttccagaa tgatggcaca taaagtaaac 76200
   attcatattt atttcccttt ttttgttttt tttttttttt ttttttgctt gtttgttttg 76260
   tttttttgtt tgagacagtc tcactcaatc acccaggctg gagtgcagtg gcaacatctc 76320
   agctcactgc aacctctacc tcctgagttc aagcgattct cctgcctcag cctcccgagc 76380
   agctgggatt acaggcgcct gcccccacgc ctggctaatt tttgtatttt tagtagagat 76440
   ggggtttcgc catgttggcc agactggtct tgaactcttg acctcaagtg atccgcccac 76500
   ctcggcctcc caaagtgctg agattacagg catgagtcac tgtgcctggc ctcttatttt 76560
   ttctttggtt aaacttttag ggaaaaagtt tgagctgctt ttaattttct ttttgttttt 76620
   aaataaatta ttaaagtttc tctatgttag gaactcttgt gtacatgagt tcattgagct 76680
   tattcttaat aaagacaaat cttctagaaa taatagttgt atctttaaat gatctcaagg 76740
   aaaatgtttg gtttctctgg ggaatgaatt ttcatgacct aatcttaaat caggttattt 76800
   tttctagcct gtttactaaa tttctacatg ttataaccta atgaaatttt cttacttcct 76860
   ctttatttaa aacaaactat aattactgtc tttttaaaaa tcttccaatg tggcgttctt 76920
   atttttctta acatttgaat tttcctgggc caaaccatgt tactatgata cacattattt 76980
   aaggctgtta tataatacag taaaattgta gaactttcat accttgaagg atcttagcaa 77040
   ttatttaatt caaacccatt ctaacataga tgataaaaca gatttgcagg gttgggcacg 77100
   gtggctcata cctatattcc cagcactttg ggagactcaa gcgggaagat tgcttgagcc 77160
   caggagttca agaccagctt gggcaacata gtgagaggct gtctctacaa aaaaatattt 77220
   aaaaaatagc cgggcatggt gtcacgtgcc tgtagttcca gctgcttggg aggctgaggt 77280
   gggaggattg ccagagcctg ggaggttgag gctgcagtga gccatgatca caccacagca 77340
   ctctagccta gagcctccct gtgtggcagg ctctacactt cagataggca acagatcgag 77400
   accttgtttc acaaaacgaa cagatctgca aagatcaacc tgtcctaagt catataatct 77460
   ctttgtgtaa taataaaaac cccatcttct aaccttaaac ctggtatttt tttctacgaa 77520
   actatgttct gcagtccaaa ttatttttct ttattatttt gaatcctaaa gtagaaatag 77580
   aaacttagaa aaataaaaag caactccttt atgacatatg aggacttttt cagttttaaa 77640
   taagaaaaac ccaactcaaa gtagcttaaa taaaaggaga cattttttga cttacataac 77700
   ctaaaagctc tggcctggat ctaggtgctc aacagatctc aagagtatct ctcttattct 77760
   ctctccctcc cttaccctcc tcctcttttc tctcttctgt atatatgtta ttttcaaaca 77820
   ggctctcaca agtagtggca agatatagtt cctaatagct tcaggtttgc attctaccta 77880
   ctttagcaac tatgatgaga agagagctac aatttgagca aaaatttgta gggtgagttc 77940
   tgatttccct ggattgaggc acatgcctat tattcctgaa ccaaatattc tgtccaggga 78000
   atggaattct ctggggcatg tatctaaagc tgaagtgtag agcctgccac acagggataa 78060
   taatagtttt ccaaagaaaa atcaaggagg ggaaagaatg ttggaaagag aaaaaaatat 78120
   cagttgtctg cttcacattt gttctcaata attagtttca tagaagtgaa atactgtgta 78180
   acaccctaaa ctttagagat tcttcgtaga caggaaaaat aagaactcaa tgaagttgtg 78240
   acttcattca aatcacgtag tttatataca tgctattagt aaaacccagg acagctgagt 78300
   acaagtttta cccttatatt cacattgagg tccagatcct ggttttgaat gagataatta 78360
   cgtgcagtcg gactgttttc tgatcctaaa aatagagaca ataatatcta tcttgtaaag 78420
   ttatggtagt gttaaagata tataaaatgt tggcaagtac cttaatatac aataactact 78480
   gctatatgtt gtcattgtaa taataatcat atttcttcct ttgttgaatt gctttcctgt 78540
   agtaatctta ttgtgatcat cctgaaacat agatttccga gcttcaagca aacactatta 78600
   tgttgaaaaa tctacattat ttctaagttt agcagtgcca gtggaaagtt tattgaaata 78660
   gaaaattact tttttaactg aggagtgtag attgtgaatt cgtgattcat cttcttagga 78720
   gatgatcgga atattgataa atattgatgc atagaatatg aacaaaacat tacatatctt 78780
   gtgctgtgat attaaagtag tattctgttc tggtagtagt atggcagtat tttaggtctg 78840
   aaagatgtac ataatctgta ctttgaagtc tgttttttaa gagattaatc acaagagatt 78900
   tacataaaac aactaaggtt aaaaataaat ggtggattag agatacatca ggcaaatttc 78960
   aatttcaaaa gcagatgaca gaatctcagt atcagggtag catttaaagc aaaaagcatt 79020
   aaactggatc aaaaatgtca ttttacatca acacagggta cactccaggt aaagacttaa 79080
   cagttatgaa cgaatgtgct aaacatcaaa atgtattaag taaaagctga aagaaatgaa 79140
   gaataattgg tagaaaagca atgataggaa tccttaatgt acttcaaaag gcacgaagta 79200
   agaaggtaca gtcatgtacc acataatgat gtttgagtca acacctgacc tggtatacga 79260
   caatggtcct ataaaattat aatggagcta aaaagcttct atcacctagt gatgttgaag 79320
   ccgttgcaat gcaatgtatc actcacatgt ttgtggtaat gctggtgtaa gcaaacttac 79380
   tgtactgtca gttgtataaa agcatagcac agttatgttc agcacataat actttataat 79440
   gataaacgaa tatgttacta gtttacgtgt ttacagtact attattttag catgtgcttc 79500
   tgcttattaa aaaatgttaa ctataaaata atctttaggc agatcctaca ggaggtattc 79560
   cagataaagg cattattgtc ataggagatg gcagctccat ggatgttatt gccccttaag 79620
   accttccagt gggacaagat gtataggtgg aagacagtga tattgatgat cctgaccttg 79680
   tagaggccaa ggctaaagta tgtatttatt agtttttaac aagagtttaa aaagtaaaaa 79740
   aaaaaaataa atttaaaaat agaaaaaatc ttaataagga tacaaagaaa aaatgttttt 79800
   gtgtagctgt gtaatatgtt tttgttttaa gttaaatgtt acaaaagagt cagagttaaa 79860
   tttttttata tttataaagt gaaaaagtta taaaatgcta aggttaattt actgaagaaa 79920
   gaaaaaattt ttaaacagat ttagagtagc atgtttataa aatctacagt agtttacagt 79980
   aatatcctag gccttcacat taactcacca ctcacccact gactcaccca gagcagcttc 80040
   caatcctgta agctccattc gtggtaagtg ccctatacag gtgtacaatt tttatatcct 80100
   ttataccatt tttactgaac cttttctgtc ttttagatac acaaataaca ttgtgttaca 80160
   gttgcctatg atattgaata cagtaactta ctgtacaagt ttgtagcata ggagctgtat 80220
   catatagcct gggtgtgtag tgaactatac catctaggct tgtgtaagta tattttatga 80280
   cacagtgatg aaatcatcca gatacagttt ctcttaagca acacataact gtatatatat 80340
   atatataggt ggctttaata ataaaataat gaaatatatt tctttttttc tgtcattgac 80400
   aaaacaatta ccagtcttac taattagatc aactgcaaaa caaaatctca ctcaaaaata 80460
   aaaatatgta agtcccattc cttgattaca attcattaga actggagatt tttaaaaatg 80520
   tttaaattta tatggaaata taaaatcatg ttttaaataa tttttgattc aatgaggaaa 80580
   tgtaaattta gaactaaaga ttaataacta gagatttata aaggcaaaaa tgttaatgaa 80640
   ttaggaaaca gtaaaccatt tgaactaatc taaatactga ttgcatgaaa atgccaataa 80700
   aatcaataca tatttttaaa agccagtcaa ggataaaaga aaagagggaa actaaaagtg 80760
   agacattaag tatgagaaag gagataaaac taaagtgtgg aggtgatgtt aaaaattata 80820
   ttcaggtctg tgctaatcat tttggaaatt tcagtgagaa gtgggcaatt ttcagtgaat 80880
   atatcattgt aaatgctttg aggagagaca aaatctgaat agaccctgca ataatgaaaa 80940
   tgtctaggag ccctcaagaa atacctggtt taaattgcat ttcaggtggc tggttatcac 81000
   accttgaagt atcaagtaat tatgttatct aaattggacc aggtgttaga aaaatatgtg 81060
   agacttcaca ggtggctgct tatgtagccc ttatacctta atctgataaa ggtagcatat 81120
   atttaaaaag agagaaaacc agaaggtaca atttagggaa aattaaatac ttctatttgg 81180
   ccaaagtaag agtacattca agggaattgt gagaggtaag agtggaaaca gaggttggag 81240
   ctgtattttt atgctgtgat tgaattacag tgtgtgataa atattgctct tatttgggta 81300
   gattaccatt taacattttg aattaattgg taattggatt aatcttaact tttaaaaaac 81360
   taatctgaga gtggtataaa ggatagatta cagaatggat aaagggtgat aaatcagttg 81420
   gctatggcaa aattgcagga agaaactgaa ataggccaca gaaaactgaa actgacattt 81480
   tgagtagtat ttcagaatca agatctggat tttggcaact gaatagatgc gtaggaatca 81540
   aagatgagta tactaaatga acttaatcta tgatttgtct gtcattttat tgtgtaccat 81600
   tagtgtgtat gcatgtatgt atgtgccagg tagttaatag gctgactgtg tctcttagct 81660
   ccactctgct gcctgggcct ttgccatagt gctggagttc cctcacttct cttttctgta 81720
   accctattat attactgctg tctctcagct gtgtttcatt cctcaagcag aaagaagatg 81780
   ggaggatcat atagtagttg actagaagct gtggagtttg agtgctggga ttatatctag 81840
   ttccattact tatgaagatt atatctagtt ccattacttc acctcatcta taaaatggtt 81900
   taccaatagt acctacctta catggtttta tgagtattaa attatgtatt tctaaagaca 81960
   tttagaacaa tacaaagaat atagtgtggg ctcaataagt gacgatggtg ttagttatta 82020
   gaaggccatc gagtgctgga gaaaataatt gaatatcatt gatggaaata aaggaagttt 82080
   tccacgttaa aaagcttcgg tttttggaaa tgtgtgtttt cagtatttct gagattacca 82140
   ggtagaaatt ccagccagat gttggaattc tgcactggca gttgggaata aagtcatgtt 82200
   aaaggagata agttttggag ttattgtgta aaattggtaa agcactggaa tagattggtt 82260
   tgacagaggg gaaactcttt ttgagatagg cctatattta gggacaagag aagagacaac 82320
   caataagggt tgaaagaaac cttgagagag taagtcctat gacagaagca gggaagtttc 82380
   agaatgattg cagaaagatc agccaggctc ttcctgttct ccatcgtggt gcaggatcaa 82440
   ggtgaaaagg ataaccccat gcaggaattt tgcatctgca ggctccgctt caacatctgt 82500
   ttaggagaaa gtggagacag actgacctga gtaactaagg tcttggaaca gcttacaggt 82560
   cagaaccagg tgttttccaa agctagatat actgttagcg cctttggcaa cagaagaaat 82620
   gaaaagactg ttgtctgctg cacagttcga ggggccaagg cagacgaaat cctggagaat 82680
   gatctaaagg tgcaggagtg tgagttaaga aaaaataact tctcagatac tggaaacttt 82740
   ggttttggga cccaggaaca cattgatctg ggtatcagat atgacccaag cattgatgtc 82800
   tacagcctgg acttctatgt ggtgctggaa aagccaggtt tcatcattgc aggtaagaag 82860
   tgcgggacag gcttcattgg tgccaaatag aatcagcaaa gaggaggcca tgcgctggtt 82920
   ccagcagaag tatgatggga tcatccttcc tggcaaataa attctcattt ctacccaaaa 82980
   gggtaataaa aagttttcag tgaaatgttt aaaaaaaaaa taaaaaaaag atcagccagc 83040
   agccaggatg ggattgtgaa aacagcagga aattagttgt tgacaaagca ttaatgacca 83100
   ttaagaaatc agcctcggct gggcatggtg gctcatccct gtaatcgtaa cactttggga 83160
   ggccaaggca gatttcttga gtccaggagt tgagaccagc ctaggcaaca tggcaaaacc 83220
   ctcgttcctc cttaataaat aaataaatga ataaataaac aagcaagcaa gcagggcttg 83280
   gtgttaggcg cctgtactcc tagctactcg ggaggctgag ggggttgaac ctgggaggca 83340
   aaggttgcag tgagccaaga ttgcactact gcactccagc ctgggtgaca gagtgagacc 83400
   atggcacccc cctccccttc aaaaagaaat cagcctcata atcaatttct ctggattaag 83460
   gagtaagagc gtctcaagat ttgctgttta tagagaggga ggctaatagt ttgagagaga 83520
   tacagaatct agggagagtg tgggtttttg gtctttcagt tgtttgccag ccttggataa 83580
   agaatgaaga ttacttgagc atattattta gagacaagtg gagagaataa aggcacatgc 83640
   cagataggag ataattaata aagcacttgt ccaaaataga aacttgttga acaggaagag 83700
   acgtcaagta taaggagatt ttaagatggg agaagggaat tttgagtgtt tgtattggat 83760
   gacctcaggg ttcccagtaa agcaggagct gaattcatcg aaggtgatgt gttggtcagg 83820
   atcaagagag aggttgggag aacaaagtgc taaaatcgtt gtggtcaaga gtttaaaaag 83880
   tgtataccag aagagttatt gagtgatagg ggtttgaaat aggcaaagct gtaggaaagg 83940
   gggctggaag gaatattagg aggaacacta aatatacttc tgaggtctac ctcctggtct 84000
   gtgaacataa aggagctgaa agagtaatgg ctgaagttct ttagtttagc taaagttttt 84060
   tagctaaagc tagaattgtt gaaagttgta tttgaggaaa aaaagttaag gatacagttg 84120
   accgttcgat aatgtagccc actgttgacc agaagcccta cccacaacat aaacaggcaa 84180
   taacacatat tttgtatgtg tattatatag tatattctta acaataaagt aaactagaga 84240
   aaagaacatg tatcaagaaa atcataagga agagaaaaca catttacagt actgtactgt 84300
   atttattggt accatacatt tatgttgctg tttacaagat gaagcatctg tctgaaatgg 84360
   ccagcagcta cagctgtacc tatctactgt acatatcaag caagtcactt tattcttata 84420
   atgtctatga cttctttctt tgaaagcgct tccatcatca ctgttggcac ttcatatggg 84480
   tctcatggtg ttaaggttta cggcattgca ctagacacaa tgaaaactac acaagagggc 84540
   cgggcacggt ggctcacgcc tgtaatccca gcactttggg aggccgaggc gggcggatca 84600
   tgaggtcagg agattgagac catcctggct aacacagtga aaccctgtct ctattaaaaa 84660
   taaaaaaatt agccaggcat ggtggcacgt gcctgtaatc ccagctaatc gggaggctga 84720
   ggcaggagaa tcgctttttc ccagaaggcg taggttgcag tgagccgaga tcgtgccact 84780
   gcactccagc ctggatgata gagggagact ctgtcgcaaa aaaaaagaaa agaaaaagaa 84840
   aagaaaagaa aaacacacaa gagccgtgag agagatagct tttgattgca atacacaatt 84900
   tactggagag atgagctgat catacagaga tgattagtgt cacacagtgt tttaaacaga 84960
   ttcttgcaac cctggagttc actgcagtag caacagaagt tagctatgag attttaacag 85020
   tagtatagta tgtactacag ttaatattag gtagctatga tttaatgctg catctttgca 85080
   tttgtttaca tttatcttga ctacaagtgg tattatgtct ggtcttaagg tttgtgtgca 85140
   tatgttttga tgaattttaa ctttttataa taggtttgtg tatattttat ggcagtaaat 85200
   gataaaacag actaatctac atatatttta tgtagtcatg acataaacct aactttttct 85260
   taactttttg atatttctag tctatgtgtt tcatctgcag gttttttcaa attgttgaaa 85320
   tctctgaaaa attttattga aaaaaaatcc atatatgtaa gtggacccac acatttcaaa 85380
   cctgtgttca agggtcagct gtgtaaataa ttttcctcaa aattaaagtg gaaaaggaga 85440
   gttactacta gtagaaagta gaactgtacc cttgggcagg ggtgtgtgtg tgtagttaaa 85500
   gatcaattta acttaaaagg tcttggttag agaataaaaa ctggccctta ttagctttaa 85560
   tttacatgaa aaatgaaaaa ttttaggcca ggcacagtgg ctcaggcctg taatccctta 85620
   actttgggag accaagggga gtggatcact tgaggtcagg agttcaagac agcctggcca 85680
   acatgctgac tcacccttcc ctactgaaaa tacaaaagtt agccaggcgc agtggccatc 85740
   cctacagtgc tagctactcg ggaggctgag gcaggagaat tgcttgaacc cgggaggcaa 85800
   ggttgcggtg agctgagatc gcaccactgc actccagact gggtgacaga gcgagactcc 85860
   atctcaaaaa aaaaaaaaaa aaaaaaagat ttgaaaacag agtatttttt aatctgcaag 85920
   agctttacag ccttttattc atatgtataa gcttttaaag atgactaaaa ttttagtgtg 85980
   gactttccac tcattggaaa tcctatattg caggtgttaa ttcaatttta gtgagtgtgc 86040
   atcatggctc agagagatag gactagaatg aggaggtcac attggagact ctgaaacaga 86100
   tacatgtgag cctcccaact ttttaatatt tgttaatcta gaagtgttga attttgggtg 86160
   ctgacaaggc agcaggtaga taagaattgc aaagttaaga aaatagactg taatattgat 86220
   ggtaaactaa ttgattaaat tttaaaatgt acttttccat gtttttcttt gaattgtcag 86280
   taattttgtt tcaactggta ttcatacata gattattcac ccaatgttga caactagtag 86340
   atttatatat tttttatgtt gcctatcctt ttttgggtaa ggattaacag aatgtataat 86400
   cacctacatt ataggtacac tactaatcac ttgctacttg aaaaaaccta aagctttgaa 86460
   atctttttat tattgcacac aaacttatgc caaaaatgga gataaagaga aaaatgtcat 86520
   ccactaaacc ccaacaaata atgttgacaa tgtggtctac tcgtagactc gcattgactt 86580
   aattttttta aatcttattg catattttga ctagataata aatgcatatg gttaaaaaat 86640
   tcacatggtt caaaaaagta cacctcccac tcatcttcca tgtgatattt cctttctgct 86700
   tagcaattct gtatttatct tgctaaacat gaatgacagt tgtttgctga aattacatta 86760
   aatgtgacgt aataaaatca ttgtaagttt acatttttta actttaataa tttttaatgt 86820
   tttaatgaag agtatgaaga gtagtagtac tgctcttcaa agtactacta ctttacctta 86880
   ccttttactg ttttgttaag aaaattaggc cgggcgcagt ggctcacgcc tgtaatccca 86940
   gcactttggg aggccgagac gggcggatca cgaggtcagg agatcgagac catcctggct 87000
   aacacggtga aaccccgtct ctactaaaaa tacaaaaatt agccgggcat ggtggtgcgc 87060
   gcctgtagtc ccagctacac gggaggctga ggcaggagaa tggcgtgaac ccgggaggca 87120
   gagcttgcag tgagccgaga tcgcaccact gcactccagc ctgggcgaca gagcgaaact 87180
   ccgtctcaaa aaaaaaaaaa aaaaaaagaa aaaagaaaat tatatagaaa taaaattcca 87240
   gctattccaa aactgcacct tgaatacagg tacagaattg ctaaaaccgt gtaccatttt 87300
   gtagttttag catgcttttg tgtaactgca tctggtgttt gatcctcatg agagccctgt 87360
   taaggaaggg tacatattat tgtcctcatt ttccttcgaa aacacatcag agtttgtatt 87420
   ttgactgtca gcattcaaat acaagtcttt tatttataaa attttggtct ttatactgtg 87480
   gctaaaaatc ttaaatcact tgtcatgatt tgaaatggtt tataccgatt ttttttgaca 87540
   tttatacaca catacacata tttttaaatt gtctataata aaatcatgct catctttgaa 87600
   aaaatattag gagtactaca gtggatacct acatacttgc tattcagcat acctggtttt 87660
   ttgtttgttt tttgagacag tcttctctgt ggtccaggtt ggagtgcagt ggcacgatct 87720
   cagctcattg caacctccgt ctcccaggct caagtgattg tcctgcctca gcctcccaag 87780
   tagctgggac tgcaggtaca catcaccacg cccagctaat tttttgtatt tttggtagag 87840
   acggggtttc accatgttgg ccaggctggt ctccaactcc tgacctcaag tgatcagccc 87900
   acctcggcct cccagagtgc tgggattaca ggttgtgagc cactgcacct ggcctgtttt 87960
   taaattcaca taaatatgtt ttatattttt cattagggag aagaaggttg tgtctacaat 88020
   ttttaagaca ttggggagat ttagatgcca gtagtaactt aaaagagaaa taattgcaaa 88080
   ttctttttcc tcttgagtat actttcattt aaggtacagt gttctgtaag ttacttttac 88140
   cgttaaactt cttaatgttg cttattgttt gtcttacatt tttaggttgg atttttctta 88200
   agtcacatgt ctaataaaaa aaacccttaa atacctcatt tattcgtctt cgttagtgaa 88260
   tgcattgttg tacatattag atttttctct ttagataact cagcttcccc tattaagtgc 88320
   cacatgtatt acaaaatttt atttatgttt tattgtttaa taaactcttg agaactagat 88380
   acattttaat catttgtaat acttacattt tctaaaacac ttcatttttc cctggtttct 88440
   tcaacaaaga gatgcatgta gtacaaggat agctttacct gtgttagaag attgtttcac 88500
   acatttacat caactgcata gtcctgtttt tgttgggccc taatgccagc atcacttttt 88560
   gctactgctg tttctgcctt aaaggcaata tgcctctgtc tagtttgctg attctgatac 88620
   tctttcccct ggaaagtagg taatcaagtt tgtgaggagc tgtgtgttta aggagtccat 88680
   aaatccttgt ggggagccct aggtgtatag agcatagctg tagggcagag gcctttgaca 88740
   cttattctgg atatgcagtg gcctttgcct atggggttca tgggtcagag cgctgttgtg 88800
   acctttgaat aaatgggttg ttatgataat tgttttaagg gaggagagtt attctgatat 88860
   cctttgtatt gatattgctc ttatttatta ttgagctgga tttaagtatt aatcatttaa 88920
   ggtcaaattt ctaatgtata tatgttctta aatggctacg acccagttac catagcaatt 88980
   tagtgaaata actataatgg aacatttttt ttcaatttgg cttctctttt ttttctgtcc 89040
   accagggagt aactattccc agtcagaggc gctatgtgta ttattatagc tacctgttaa 89100
   agaatcatct ggattataga ccagtggcac tgttgtttca caagatgatg tttgaaacta 89160
   ttccaatgtt cagtggcgga acttgcagta agtgcttgaa attctcatcc ttccatgtat 89220
   tggaacagtt ttcttaacca tatctagaag tttacataaa aatttagaaa gaaatttacc 89280
   acatttgaaa tttatgcagg agactatatt tctgaagcat ttgaacaaat taattagctt 89340
   tgttgttcaa ctcattgggc taaagaagcc aaaagcaatg ggttttaatg tagtcgaagc 89400
   caaattatat ttatgaaaga aatattctgt gttataacca ccaaatacag cccaattctg 89460
   actagatgat ggaagaacct gtcccatcag aggtccagca tgaggtccag cagaggtcca 89520
   ccagaggagt tcagcaattt gctgctctta gggcagggat caattcctta atatcttagg 89580
   aagactaggt attgacagta atggtgacaa agcaatgaaa aggaaaggaa gaagtgataa 89640
   gacgtggcag caagctgaag tatgatgagt aaagaatagg aatcaaagta tgtggagtgt 89700
   tagagaaaac ctggatttag atccagattc tagtcctatc tctgtcatta atctattgcg 89760
   taaccctgag catatcatct acctctcttt gagtttgctt gtcaataaaa tgaagagact 89820
   ttgaaatctg agacttcctg gataagtact aaatacagat tatgtcactg atgtctgcct 89880
   ctatttattt ctccctttta ccctaatctc tataagtcta cctcagtcat cctgatccta 89940
   ttctacttct ctgatgttgt tgtcagatag gtgtgatcat cctcatcaga tcttttctgt 90000
   attcttagag acagataact ttatcaaaga ccacagattt attagtatag catgttaaag 90060
   tcttctaaag agtctcattg atgctctttt catctcagta caatttttaa aactgctgaa 90120
   tgcaaggtac tgagctgttg gaagtgactg acagatgaat gtaacagatt catagagaag 90180
   gaaaaaggaa gaaaaactca tgctcttcct atagtattga tatcagtgta agagccaaga 90240
   gaaaggtata aagtatcatg cagatattaa gggaaagaaa acattcactt tagtaatctt 90300
   tcctcatttt ctagtttcct cttatgtact atgatttaat actgtagtaa agttttaata 90360
   aaatatgagc tatatgtaat taagtgggag gttgtggggc taggcacgag gctcacacgt 90420
   gtaaccccag cactttggga ggctgaggca ggcggatcgc ttgatctcag gagttcgaga 90480
   ccagcctgga caacaaggtg aaaccccatc tctactaaaa acacaaaaat tagctgggca 90540
   tagtggcaca cacctgtagt cccagcttct tgggaggctg aggcaggaga atcgcttgaa 90600
   tccaggaggc agaggttgca gttagccgag atcatgccac tgcactgcag cctggacatc 90660
   ggagcaagac tttgtcttag aaataaataa ataaatataa aataaaataa atgggaagtt 90720
   gtgtatataa attataaatg ctacattcag aaaagctttt gaaggttgtc agacagtttc 90780
   ttaaaggaag ttcaccagtt ctttattgaa cattgaagaa aacatacagt ttagactggc 90840
   attaaaactg aaagaagtgg ccagacgcag tggtagacgc agtggttcac gcctgtaatc 90900
   ccagcacttt gggaggtcaa ggtggatgga tcacctgagg tcaggagttt gagatcaggc 90960
   tggccgacat ggtgaaaccc tgtctctact aaaaatacaa aaattagcca ggcatggtga 91020
   tgcgtgcctg tagtcccagc tacttgggag gctgaggcag gagaattgct tgaagccgaa 91080
   ggtggaggtt gcagtgagcc gagattgcgt cattgcactc cagccagggc ggtaagagtg 91140
   aggctccgtc ttaaaaaaaa aataagtaaa taaattaaaa actactgaaa gaagtattac 91200
   aggcaatggg aaatagcttg agtggaagtg cagcagaagg aaaaagctgg acaagaatgt 91260
   agtgtcagag aataggtatg gaacgtgtga gtgactgtta gaggatcttg aatggggata 91320
   acagacttga tttcataaat actgagatgt catgataata cttgaggact aaaccatgtt 91380
   ttaaggacag ttgtatgcaa agttgtaatc gcaagaggaa aaaatagtgg aaaagaaacc 91440
   agtaataaaa cttgccttaa tgcaggtatg ctaagacaat caaatgggat ttcattaatt 91500
   ttttatttgc catttatagc caaagatttt gtaaaagttt tgagcccagt caggtgaaat 91560
   agtctcagaa agaaagaaaa gtgaatctga gacttggaga cattaatgtt gatattttgg 91620
   ttttaaacgt gttttaaatc cggtaaaagt gagcttctca catgacaata ttcagtgggt 91680
   acttgggagt atgggttcga atctaggtag gagatatatc gatattttgg gcatcatcag 91740
   gaaagggaga gtagttaagc ctttcatata aataatggtg tggcgtttgg gcatgggaag 91800
   tcttggagga aaggaagaaa aggagagggt gaggactgag ataagaatgg caacttgggt 91860
   ttaggaagaa gaagaggaat caatgtagag aacagatagt gctgaaaaat acagcatctt 91920
   ctgtagggat tggcagcttt ttcttgattt ttgtcttaat atttctaaga gatggaaaaa 91980
   gctactatat tctagacatt taacagggtt aaaaatgtta ctaaaagatg atcaatgtgg 92040
   ttttcattca agactataac aatatgtata tatccaagga aatttaattc tgacttaaaa 92100
   aaattgtttt gcttgtatag atttagggac acaagtgtaa ttttgttaca tgcatagagt 92160
   gtatagtttc aagtcagggc ttttaggttg tccatcatct gaataatata cattgtaccc 92220
   attaagtaat ttctcatctt ctactcaccg tttcaagtct ccactattta tcattccatt 92280
   ctttacattc tgattttcat ttactaggtg tattagtctg tttttgcatt gctttaaaga 92340
   aatacctgag actgagtatt aaacaggttt aacctgtttt ctttatgaat tcttctttaa 92400
   ttggctcatg gttctgcacg ctgtacagaa agcatagcag catctgcttc tggggaggcc 92460
   tcaggaagcc tccaatcatg gctgaaggca aaaggggagc atggtgagaa tgggagcaag 92520
   aaagagaagg agtggtgggg agaaggtgcc acccactttt aaatgccagc tcacttacca 92580
   ccaagaggat ggcccaagcc attcatgtgg gatctgcccc catgattcaa gcttcttcca 92640
   ccaggcccca cctctagcac tggggattac aattcaacct gagatttggg agggaaagat 92700
   atccaaacta tatcactagg tctggatctt gttatttatt ttttggaaca tagtcatata 92760
   tatccaagga tatatattgt agaagtccac agaaccatac taatattgga cttctgctta 92820
   gttaggtctt atctatctga aacatgatat tcatattgca gagaagatta ttttctttag 92880
   tgattgagga aatctttact acttatacat ttttaatata atactataat atttgaagat 92940
   gcacatttta gatgtagttt aattgaaacc tggaaatact attaatttgc tttttaaagt 93000
   cctaaaatca ggattatcag attctgaatt aatggagttt aaatcaaaaa gattacaagg 93060
   cagtttttca gttttattct ggttaatttt atcacagctt tggaatccta ctttgtttat 93120
   ttgcttcttg aagttagatt tcccagtgaa atttcagtat cacataaagt cttatgaaat 93180
   ggctcattgc actttgaact ttgagtcaag gaagtgaaat ttattgatag attgttggtg 93240
   taatatttat cctgtttgtg gtagcttttt tgaataataa gtgtcttaga agaccatgtt 93300
   ggagtagcct gcatgctttt atcaaacata ttaattatgt gatggctgat actgctttag 93360
   atattacata gaaatagtag taggtgttta ctaaactgga aatttcattt aacttggttt 93420
   agctttgcct tgttctcagt cacattgata aaaatgtaag acttttgttt atcttttaga 93480
   ataatgacac cttttggtgc tgagaatttt ttgttttata tatatatata tatatacgta 93540
   atataaatac aaaatatatt taaatatgta taatatttct catacacttt atgtaacttt 93600
   gtgttcctgt ttctctatta tcttggcatg ttttcttcaa atggcacttc ttaacctcct 93660
   aaggttaata aatttctttg taatggactt ttgttttcta attcctcagc gtatgacaaa 93720
   tgaattatac tttgtcaaat tatttaggta actttcagtt tttgaagtcc tgggatcata 93780
   acattcatca gtctttaatt tctgtcatta aggtcattag ctataaatga atttatgagt 93840
   agatttaaaa aataaaacat acaatccttc ccttaacaca ctttcccacc atttggttca 93900
   actgctagtg taaaagcatg atgaattttg agaagttata ttttaccagt tactttattt 93960
   tttaccagtt atttaaaaca gacatgagcc aaagccagaa tacttgttaa tgaaaatgag 94020
   gtgttttgga ggaaaggaag gttgtgctgc agtttttact tgaaatctgt tacatttctt 94080
   tacagaaatt tcaaatctct tgtttcctgt tatgatggtg gcattatata cctttaaaat 94140
   gtgagctata ggaaaatgaa tgatggttaa ttttttaata aatatttaga cttgtgtttt 94200
   tgaaattttt tataacattg ttataggttt tatcctcttt ctcttgtgaa catgtagtga 94260
   tttgtatttt gtgatctttg ccgcatgcta gagacttaag aatactatag caaatatctg 94320
   tcttctttac atttaaaaat ttttcgtgac tactccctgt tgatatctgt cttaaaagtt 94380
   acttttgatg tagttcacaa atgtaccaga taattatttc atcgttttta atgcttaaag 94440
   tttttatttg tattaggatt tttagtatga ttttaatgtt aaagttttga agttactctg 94500
   ccactagaag tctaattttg ggacttacta ttcatgaaat aggaattgac ttttatataa 94560
   gtaataggac cttattttga aggttcaaac tggagaaaat cttacattgt ttatattttt 94620
   atttcattta tttcagttga tttgcttgag atcaagattg cagatacaga atccatattt 94680
   cgtgtatatt gctgatatta atcattaaaa tcgtttttga cagtttgaca gttaaaggca 94740
   tttcctgtga aataatactg gtatgtattt aaccatgcag atcctcagtt tgtggtctgc 94800
   cagctaaagg tgaagatata ttcctccaat tcaggaccca cacgacggga agacaagttc 94860
   atgtactttg agttccctca gccgttacct gtgtgtggtg atatcaaagt agagttcttc 94920
   cacaaacaga acaagatgct aaaaaaggtt tgtactttac tttcattggg agaaatatcc 94980
   aaaataagga cagattaaaa gctatatttt attttatgac atgtaaggaa ctataatttg 95040
   ttttctatta gatctgcagg tgttttgctt actctggcat tggtgagaca ttataagggt 95100
   aaataatcct gtttgaagga aaaggcctta tggcattgta acatgagagg aatttttctt 95160
   aacaaggatg gttaactgag aagaaattag catgggacca atattttaaa aattttggtc 95220
   tataggtaga aatgagatct gttctgtggt cttatgtagt gacacaaacc actttttctc 95280
   cattttggct tatgtttctt tttctttcct tttttttttt tttccttttt gttagagaca 95340
   gggtcttgtt ctattgccca ggctgagtag ctaagactac aagcatgtgc caccacaccc 95400
   agctaatttt ttttattttt atttttgtag ggacagggtc tcactatgtt gcccaggctg 95460
   gtctcaaact cctgggcaca agcagtcctc acgctttggc atcccaaaga gttggaatta 95520
   caggtgtgcg ccatcatgcc tggccttaac gtttcttaag acttgattat tttctattta 95580
   gcttctgtgg atttactgat taatttttta actaggagag aaatcagtat gaagaggaag 95640
   taataaagaa tgaaaacatg gtatttaaat gtgcaggttt agaaagttaa tgaagtttga 95700
   atttgattga tctgtattta gagaaggcaa cgtcttatta ttttaaaacc aactatccgc 95760
   cctgtgcggt ggctcacgcc tgtaattcca gcactttggg aggctgaggt gggcagatca 95820
   gctgaggtca ggagttcgag accagcctgg ccaacatggt taaaccccat ctctactaaa 95880
   aatacaaaaa aattagccgg gtgtggtggc aggcgcctgt tttcccagct actcaggagg 95940
   cttgaggcag gataattgct gaacccgaga ggcggaggtt gcagtaagcc aagaatgcac 96000
   cattgtactc cagcctgggc aacaagagtg aaactccatc tcaaaaaaaa agaaaaaaaa 96060
   aacaacaact atcttcattt aaaatattaa atgtgaatat ttaaagtgag actaaggtgc 96120
   aacattttta gatagtaatg aagaaaagga ctaactttgt agtgttgctg ccttgttaaa 96180
   catactagat agcatattgc caatctttaa acattctcaa tgataggatt tatttacttt 96240
   ttctgatttt tagcttttct tttgaaagaa aataagagga agtttcattt actgcaaaat 96300
   tttaaatgct gctttgatgt atcagtagag atataatttt cctttatcca gaatccaagt 96360
   agctggaaaa aaaaatcaaa atatgctgaa cttttttttt tttagccaga aacccatttc 96420
   ctatcgtctg tacaaataaa agttaaatat atctcaataa cttagaaaaa ttattttttg 96480
   ataatccagg aagtattagc aactgtttta aaattaagat aactagtaag ttttatttag 96540
   ctttcaaaaa taggcatcta catcatcatc tctgcatacc tttaggaatt tcctaattct 96600
   tatttccctt catctgtact ttaacacatg caaaattgaa ggttagatta aatatttatg 96660
   atttatttgt ttatccttga ctacataaat ttccatttta ttgattttcc ctgccttatt 96720
   taagaatatg ctatgattaa aacacaaaaa attttagtat aacccatata tatatagaat 96780
   tcaccttttt gttatttaaa tattattggc ttattttctt ctaagtaaaa tacaattact 96840
   ggctaaaata attgaaataa gcaaaaaaaa aattttaaag accttgtata caagattact 96900
   ttgccaggta ctgttaaaag atgcaatgac atttaagacg taacatcctt aaggatctta 96960
   ttttctgggg gataaaaaac tttaagataa attagaataa aagatttaaa tggcatttta 97020
   aggtaccagg taccagataa gatgtcacaa ggctgtatat cattaattgc caaatgattt 97080
   atacaggcca gatttctttg ttggtcaata gaggtttaaa gtgatgaact tctgttgtgt 97140
   ttttttatta agaaggtatt atcttattag taagaagtga ttttttttaa gaacaagcat 97200
   tttataacat caaaagaaat cagtagtact ctttcctacc ccctcatatt tattctgaaa 97260
   gtattcaagc attatattgt catgtaagaa actggagctt ctcatgtttg tattgctgta 97320
   gaagtaaaca tgtatttgcc atgcgtcatc agggaagttg cactcaccgt ccaagaactt 97380
   ttgttaaagt aaatcttgga ataggtagct catttgaaat gtagaaaaaa ttaaatccat 97440
   atctgaattt tgtttatatg tatgtacacg taaactaaaa acgtatttaa agctagtatt 97500
   agatgagaaa agaggttttt ttacttaaaa ttttaaggca aaagtagttt atcttagatc 97560
   ttgtgagatt gtatttttgg tttaaaattt gagaatttga gtgaagaaaa atcatgtgaa 97620
   tgaaaatgca acagataact cagattgcct tataatagtc tttgtgttta cctttattca 97680
   gaatatcaaa tgatagttta ttttgttgac tttttgcaaa tgtttaacat aggtgacaga 97740
   ttttcttttt taaaaaaata aaacatcatt aattaaatat gtcatttcat ttctttttct 97800
   tttctttttt ttttttttta ggacaaaatg tttcactttt gggtaaatac attcttcata 97860
   ccaggaccag aggaaacctc agaaaaagta gaaaatggaa gtctatgtga tcaagaaatc 97920
   gatagcattt gcagtataga gcgtgcagat aatgacaagg aatatctagt acttacttta 97980
   acaaaaaatg atcttgacaa agcaaataaa gacaaagcca accgatactt ttctccaaat 98040
   tttaaggtca gttaaattaa acattttgtg ggggttgttg acttgtatgt atgtgatgtg 98100
   tgtttaattc taggagtaca gctgatgaag aacttgcttg acaagttttt aacttatgta 98160
   ttatttcgaa gcagtgttta cgtagcagta acatgaaagt ttctaataaa atacccaatg 98220
   tacacagcgt caaaaaagct gcatttttcc ttttcctaat tcttcgttgt ttgctgaaat 98280
   ctggggcaaa ggtgcgggag ggggctaaat gactgggata tgaagtagga atgggagagg 98340
   aaagaaatag atgggaactc agtcatttgg gaatgattca tatggaatgt ttttactgct 98400
   tccactcctg tctgccttcc aatttattct caatccctca gagtgatctt aaaaatagac 98460
   ttgattgtgt cacttctgtt tacactttat aaggaccttg tgtttttttt tttaccatga 98520
   cctacaaggc ccagcataat ttagcacagg gctacctcct acatcagcac tagtcacctt 98580
   ctctccttgt ttcttgagat tcagtcatac tggtctttct tcagttcttc aaaatgctaa 98640
   gcttctgcct cttctagtct ttccagttat tttccttctc cctgtacctt ttcatctcag 98700
   ccttttcccc tgaccttcca tagctatctt catatttcca gccttagctt caatctcata 98760
   ttctctgaag tcctttgatt gtcctcccgt tattcttttt ttaaaaatcc tatttcctta 98820
   tattgtatct tagaattatt tggtttgttt catttttgcc tatgtgtgat atatgtattt 98880
   ctacataggt atatatatct acttatagac aagaattctt cagattaaaa aaatctgatt 98940
   tgtaaacatt cccaagtggt tgtttaccat ttttttcttc ccccttccta tttcttattc 99000
   tacctgattt tcccctgttc attcaccaca ctcgtttctt tctctttttt actctctctt 99060
   aatttttcat tcaattttta taacatgtaa taaatctaac tgtagcgtct gagtattaag 99120
   aatattgcta gtaatacttc acctgtaatc ccagcacttt gggaggctaa ggcaggcgga 99180
   tcacttgagg cccaggagtt taagaccagc cggccaatat ggcgaaaccc tatctgcact 99240
   acaaatacaa aaattagctg ggcatggtgt cgcacacctg taatcccagc tacttgggag 99300
   gctgaggcac aagaattgct tgagcctgtg agatggaggt tgcagtgagc cgagatcaca 99360
   ccagtgcacg tgcacttcag cctgggcaac agagcaagac tctgtcttaa aaaaaaaaaa 99420
   aaaaaaaaaa tatatacaca cacacacaca cacacacaca cacacacaca ctattactac 99480
   caatatacat acatatatgt atgtatgtat gtatgtatat tggtagtaat agtaatactt 99540
   gggcccctgc acgttttaag tgaaaataga tctaatatta aatgtcttta gcccttaaat 99600
   tttttttaag tgttcagaag tttcccttta aaaaaatttt taatatataa taattgtaca 99660
   tatttatggg atacagagtg atattttcat gtatgcagtg tgtgatgatc aaatcaggat 99720
   aattagcata tggatcacct caaacatttg tcatttcttt gtgttaggaa cattcaaaat 99780
   tctgtcttct agctatttga aaatatacag taaattattg ttgactagtt acagttctat 99840
   agaacactat aatttattcc tcctgtgtgt aattttttat cttttaacca acatctccct 99900
   atcctcccct cccactccct ttcccggcct ctaataacca cactcttatg agctcaactt 99960
   ttttagcttc catatatgag tgagaacata cggtatttat ctttctgtac ctgacttatt 100020
   ttacttaaca tcatgtcctc cgggctagac attctcttta gaatccacag gtttcctttc 100080
   ttttctctaa atctgcattt tgctcagcca ttaactttta aaatgtcttt ttccctttag 100140
   ttttattgtt ttctatttta atattgcaag atgttttata tttgtgatta caaataaaaa 100200
   ctccattatt agtaaacaaa tacaatgtca tatagtagta agtgctataa aaaatagaca 100260
   ggatagaaag taatcttggt ttgtatgttt tttgtttttt agcaaagatg attagagaag 100320
   gcccaaccaa gcagataaca tttaagcaga ggcctaaatc atataagtga gttatacaaa 100380
   tatctgggaa aagagttaag agtacagatg caaaagccct tagacaagag aatgagcttg 100440
   gtatatctga agagtggata agtcattttg actgaaacag agtggacaag aaaaccagtc 100500
   caagtgtaaa gacactagtg tgtgttcagc ataggaagga tgtaatctga attttgtgtt 100560
   taatattccc tgtgttcatg ctttcaaaat acagatgagt gaggaaagta gggagaaggg 100620
   gtaataaagg aagctgagag atcagttaag aggtacttga atagtttagt aaagatgaga 100680
   gaagatgttt gcttcttgtt gcccctcact gcttagaata gtggcagtga agggtaacaa 100740
   gaagctgtca gattaactta aagagtttac tgatgcagtg gatgttggtt gtaagagaag 100800
   aattgataat gactcttgga taatagggga gggaggggct gtcaatataa tataatgaag 100860
   aagggatttg aagtcatttc tgatttaaat ctcacatcca ctacctactt ttaatagata 100920
   tgtagccttt aacaagttcc ctaacctttc tgggccttag ctacctcccc ttggaaatgg 100980
   aaatacctaa catgtaaggt tgttttgaca gttattttca ctaggcatgt aaaggcactt 101040
   gactctctgt tatagaccac tgtattatgt taatgtccct ctccttcctc cctttaggta 101100
   aagtttttag ggctaataaa tcccaaatat caatgttgat cagtagtttg tgtttgtgta 101160
   gtgttgttta tatcaaaaac tacattgaag ccgggcacag tggttcacgc ctaaaatcgc 101220
   aacactttgg gaggccaagg tgggcctccc accttgaact aaggagtttg agaccagcct 101280
   gggcaacatg gtgaaatccc atctctacaa aaaatataaa agctagctgg gtgtggtggc 101340
   atgcacctgt agtcctagct acttgggagg ctgaggttga tcctgggagt ttgagcctgc 101400
   agtgagctgt gaagatgcca ctgcactcta gtctgggtga cagagcaaga ccctgtctca 101460
   aaaacacaca cacacacaca cacacacaca aagaaataca ttgatttttc acataggtag 101520
   taagagaaac attctttttg aactcagctg tttgtgaatt gaattttgta attcaaatgc 101580
   tatattatgt aaactattga tgactttcaa tctgcattta ttttgtataa ttatttagtt 101640
   aatatttgcc acttatattc cttaaaaaat aaaattgagg ttgggcgtgg tggctcacac 101700
   ttgtaatccc agcactttgg gaggctgagg caggcagatt gcctgagctc aggagtttga 101760
   gatcagcctg ggcaacatca tgaaccccat ttctactaaa atacaaaaaa ttatctgggc 101820
   atggtggtgt acacctgtag ccctagctgt ttgggaggct aaggcacgag aattgcttga 101880
   acccgggagg cagaggttgc agtgagccaa gatcatgcca ctgcactcca gcttggcaac 101940
   agagcaagac tcttgtctcc agaaataaaa ataaataaat tgtattaaca tcctgatagt 102000
   ttatctgttt agtacctagc aagaaagaaa atgttgaaca tcttaagaag agggtcattt 102060
   aaaaggcctc ttaaagatca tgtttgttac agtgcttaaa aattaatatg ttcatctgca 102120
   aaatggaata aaaaatctgt taaaaatata tttcactaaa tagtttaaga tgagtcatat 102180
   ttgtgggttt tcattttaaa ttttctttct ctaggtgaag ctgtacttca caaaaacagt 102240
   agaggagccg tcaaatccag aggctagcag ttcaacttct gtaacaccag atgttagtga 102300
   caatgaacct gatcattata gatattctga caccactgac tctgatccag agaatgaacc 102360
   ttttgatgaa gatcagcata cacaaattac aaaagtctga attttttttt atcaagaggg 102420
   ataaaacacc atgaaaataa acttgaataa actgaaaatg gacctttttt tttttaatgg 102480
   caataggaca ttgtgtcaga ttaccagtta taggaacaat tctcttttcc tgaccaatct 102540
   tgttttaccc tatacatcca cagggttttg acacttgttg tccagttgaa aaaaggttgt 102600
   gtagctgtgt catgtatata cctttttgtg tcaaaaggac atttaaaatt caattaggat 102660
   taataaagat ggcactttcc cgttttattc cagttttata aaaagtggag acagactgat 102720
   gtgtatacgt aggaattttt tccttttgtg ttctgtcacc aactgaagtg gctaaagagc 102780
   tttgtgatat actggttcac atcctacccc tttgcacttg tggcaacaga taagtttgca 102840
   gttggctaag agaggtttcc gaagggtttt gctacattct aatgcatgta ttcgggttag 102900
   gggaatggag ggaatgctca gaaaggaaat aattttatgc tggactctgg accatatacc 102960
   atctccagct atttacacac acctttcttt agcatgctac agttattaat ctggacattc 103020
   gaggaattgg ccgctgtcac tgcttgttgt ttgcgcattt ttttttaaag catattggtg 103080
   ctagaaaagg cagctaaagg aagtgaatct gtattggggt acaggaatga accttctgca 103140
   acatcttaag atccacaaat gaagggatat aaaaataatg tcataggtaa gaaacacagc 103200
   aacaatgact taaccatata aatgtggagg ctatcaacaa agaatgggct tgaaacatta 103260
   taaaaattga caatgattta ttaaatatgt tttctcaatt gtaacgactt ctccatctcc 103320
   tgtgtaatca aggccagtgc taaaattcag atgctgttag tacctacatc agtcaacaac 103380
   ttacacttat tttactagtt ttcaatcata atacctgctg tggatgcttc atgtgctgcc 103440
   tgcaagcttc ttttttctca ttaaatataa aatattttgt aatgctgcac agaaattttc 103500
   aatttgagat tctacagtaa gcgttttttt tctttgaaga tttatgatgc acttattcaa 103560
   tagctgtcag ccgttccacc cttttgacct tacacattct attacaatga attttgcagt 103620
   tttgcacatt ttttaaatgt cattaactgt tagggaattt tacttgaata ctgaatacat 103680
   ataatgttta tattaaaaag gacatttgtg ttaaaaagga aattagagtt gcagtaaact 103740
   ttcaatgctg cacacaaaaa aaagacattt gatttttcag tagaaattgt cctacatgtg 103800
   ctttattgat ttgctattga aagaataggg tttttttttt tttttttttt ttttttttta 103860
   aatgtgcagt gttgaatcat ttcttc 103886
<210> 16
   <211> 16
   <212> DNA
   <213> Artificial Sequence
<220>
   <223> Synthetic Oligonucleotide
<400> 16
   ccggaggtgc ttgaat 16
<210> 17
   <211> 14
   <212> DNA
   <213> Artificial Sequence
<220>
   <223> Synthetic Oligonucleotide
<400> 17
   cggaggtgct tgaa 14
<210> 18
   <211> 16
   <212> DNA
   <213> Artificial Sequence
<220>
   <223> Synthetic Oligonucleotide
<400> 18
   gaagccatac acctct 16
<210> 19
   <211> 14
   <212> DNA
   <213> Artificial Sequence
<220>
   <223> Synthetic Oligonucleotide
<400> 19
   aagccataca cctc 14
<210> 20
   <211> 16
   <212> DNA
   <213> Artificial Sequence
<220>
   <223> Synthetic Oligonucleotide
<400> 20
   gttgaagcca tacacc 16
<210> 21
   <211> 14
   <212> DNA
   <213> Artificial Sequence
<220>
   <223> Synthetic Oligonucleotide
<400> 21
   ttgaagccat acac 14
<210> 22
   <211> 16
   <212> DNA
   <213> Artificial Sequence
<220>
   <223> Synthetic Oligonucleotide
<400> 22
   cctcagggtt gaagcc 16
<210> 23
   <211> 14
   <212> DNA
   <213> Artificial Sequence
<220>
   <223> Synthetic Oligonucleotide
<400> 23
   ctcagggttg aagc 14
<210> 24
   <211> 16
   <212> DNA
   <213> Artificial Sequence
<220>
   <223> Synthetic Oligonucleotide
<400> 24
   tttgccctca gggttg 16
<210> 25
   <211> 14
   <212> DNA
   <213> Artificial Sequence
<220>
   <223> Synthetic Oligonucleotide
<400> 25
   ttgccctcag ggtt 14
<210> 26
   <211> 16
   <212> DNA
   <213> Artificial Sequence
<220>
   <223> Synthetic Oligonucleotide
<400> 26
   agttcttggt tttctt 16
<210> 27
   <211> 14
   <212> DNA
   <213> Artificial Sequence
<220>
   <223> Synthetic Oligonucleotide
<400> 27
   gttcttggtt ttct 14
<210> 28
   <211> 16
   <212> DNA
   <213> Artificial Sequence
<220>
   <223> Synthetic Oligonucleotide
<400> 28
   cctcttgatg ttcagg 16
<210> 29
   <211> 14
   <212> DNA
   <213> Artificial Sequence
<220>
   <223> Synthetic Oligonucleotide
<400> 29
   ctcttgatgt tcag 14
<210> 30
   <211> 16
   <212> DNA
   <213> Artificial Sequence
<220>
   <223> Synthetic Oligonucleotide
<400> 30
   atgcccctct tgatgt 16
<210> 31
   <211> 14
   <212> DNA
   <213> Artificial Sequence
<220>
   <223> Synthetic Oligonucleotide
<400> 31
   tgcccctctt gatg 14
<210> 32
   <211> 14
   <212> DNA
   <213> Artificial Sequence
<220>
   <223> Synthetic Oligonucleotide
<400> 32
   tgccacattg ccct 14
<210> 33
   <211> 14
   <212> DNA
   <213> Artificial Sequence
<220>
   <223> Synthetic Oligonucleotide
<400> 33
   ttgccacatt gccc 14
<210> 34
   <211> 14
   <212> DNA
   <213> Artificial Sequence
<220>
   <223> Synthetic Oligonucleotide
<400> 34
   gttgccacat tgcc 14
<210> 35
   <211> 14
   <212> DNA
   <213> Artificial Sequence
<220>
   <223> Synthetic Oligonucleotide
<400> 35
   tgttgccaca ttgc 14
<210> 36
   <211> 14
   <212> DNA
   <213> Artificial Sequence
<220>
   <223> Synthetic Oligonucleotide
<400> 36
   ctgttgccac attg 14
<210> 37
   <211> 14
   <212> DNA
   <213> Artificial Sequence
<220>
   <223> Synthetic Oligonucleotide
<400> 37
   tctgttgcca catt 14
<210> 38
   <211> 14
   <212> DNA
   <213> Artificial Sequence
<220>
   <223> Synthetic Oligonucleotide
<400> 38
   ttctgttgcc acat 14
<210> 39
   <211> 14
   <212> DNA
   <213> Artificial Sequence
<220>
   <223> Synthetic Oligonucleotide
<400> 39
   tttctgttgc caca 14
<210> 40
   <211> 14
   <212> DNA
   <213> Artificial Sequence
<220>
   <223> Synthetic Oligonucleotide
<400> 40
   atttctgttg ccac 14
<210> 41
   <211> 16
   <212> DNA
   <213> Artificial Sequence
<220>
   <223> Synthetic Oligonucleotide
<400> 41
   gtaggagaaa ggcagg 16
<210> 42
   <211> 14
   <212> DNA
   <213> Artificial Sequence
<220>
   <223> Synthetic Oligonucleotide
<400> 42
   taggagaaag gcag 14
<210> 43
   <211> 16
   <212> DNA
   <213> Artificial Sequence
<220>
   <223> Synthetic Oligonucleotide
<400> 43
   ggcttgtaaa gtgatg 16
<210> 44
   <211> 14
   <212> DNA
   <213> Artificial Sequence
<220>
   <223> Synthetic Oligonucleotide
<400> 44
   gcttgtaaag tgat 14
<210> 45
   <211> 16
   <212> DNA
   <213> Artificial Sequence
<220>
   <223> Synthetic Oligonucleotide
<400> 45
   ccactggagg atgtga 16
<210> 46
   <211> 14
   <212> DNA
   <213> Artificial Sequence
<220>
   <223> Synthetic Oligonucleotide
<400> 46
   cactggagga tgtg 14
<210> 47
   <211> 16
   <212> DNA
   <213> Artificial Sequence
<220>
   <223> Synthetic Oligonucleotide
<400> 47
   tttcagcatg ctttct 16
<210> 48
   <211> 14
   <212> DNA
   <213> Artificial Sequence
<220>
   <223> Synthetic Oligonucleotide
<400> 48
   ttcagcatgc tttc 14
<210> 49
   <211> 16
   <212> DNA
   <213> Artificial Sequence
<220>
   <223> Synthetic Oligonucleotide
<400> 49
   catatttgtc acaaac 16
<210> 50
   <211> 14
   <212> DNA
   <213> Artificial Sequence
<220>
   <223> Synthetic Oligonucleotide
<400> 50
   atatttgtca caaa 14
<210> 51
   <211> 16
   <212> DNA
   <213> Artificial Sequence
<220>
   <223> Synthetic Oligonucleotide
<400> 51
   atgcccatat ttgtca 16
<210> 52
   <211> 14
   <212> DNA
   <213> Artificial Sequence
<220>
   <223> Synthetic Oligonucleotide
<400> 52
   tgcccatatt tgtc 14
<210> 53
   <211> 16
   <212> DNA
   <213> Artificial Sequence
<220>
   <223> Synthetic Oligonucleotide
<400> 53
   ttttggtggt agagac 16
<210> 54
   <211> 14
   <212> DNA
   <213> Artificial Sequence
<220>
   <223> Synthetic Oligonucleotide
<400> 54
   tttggtggta gaga 14
<210> 55
   <211> 14
   <212> DNA
   <213> Artificial Sequence
<220>
   <223> Synthetic Oligonucleotide
<400> 55
   tctgcttcgc acct 14
<210> 56
   <211> 14
   <212> DNA
   <213> Artificial Sequence
<220>
   <223> Synthetic Oligonucleotide
<400> 56
   gtctgcttcg cacc 14
<210> 57
   <211> 14
   <212> DNA
   <213> Artificial Sequence
<220>
   <223> Synthetic Oligonucleotide
<400> 57
   agtctgcttc gcac 14
<210> 58
   <211> 14
   <212> DNA
   <213> Artificial Sequence
<220>
   <223> Synthetic Oligonucleotide
<400> 58
   cagtctgctt cgca 14
<210> 59
   <211> 14
   <212> DNA
   <213> Artificial Sequence
<220>
   <223> Synthetic Oligonucleotide
<400> 59
   tcagtctgct tcgc 14
<210> 60
   <211> 14
   <212> DNA
   <213> Artificial Sequence
<220>
   <223> Synthetic Oligonucleotide
<400> 60
   ctcagtctgc ttcg 14
<210> 61
   <211> 14
   <212> DNA
   <213> Artificial Sequence
<220>
   <223> Synthetic Oligonucleotide
<400> 61
   cctcagtctg cttc 14
<210> 62
   <211> 14
   <212> DNA
   <213> Artificial Sequence
<220>
   <223> Synthetic Oligonucleotide
<400> 62
   gcctcagtct gctt 14
<210> 63
   <211> 14
   <212> DNA
   <213> Artificial Sequence
<220>
   <223> Synthetic Oligonucleotide
<400> 63
   agcctcagtc tgct 14
<210> 64
   <211> 16
   <212> DNA
   <213> Artificial Sequence
<220>
   <223> Synthetic Oligonucleotide
<400> 64
   aactctgagg attgtt 16
<210> 65
   <211> 14
   <212> DNA
   <213> Artificial Sequence
<220>
   <223> Synthetic Oligonucleotide
<400> 65
   actctgagga ttgt 14
<210> 66
   <211> 16
   <212> DNA
   <213> Artificial Sequence
<220>
   <223> Synthetic Oligonucleotide
<400> 66
   tcattaactc tgagga 16
<210> 67
   <211> 14
   <212> DNA
   <213> Artificial Sequence
<220>
   <223> Synthetic Oligonucleotide
<400> 67
   cattaactct gagg 14
<210> 68
   <211> 16
   <212> DNA
   <213> Artificial Sequence
<220>
   <223> Synthetic Oligonucleotide
<400> 68
   attcatcatt aactct 16
<210> 69
   <211> 14
   <212> DNA
   <213> Artificial Sequence
<220>
   <223> Synthetic Oligonucleotide
<400> 69
   ttcatcatta actc 14
<210> 70
   <211> 16
   <212> DNA
   <213> Artificial Sequence
<220>
   <223> Synthetic Oligonucleotide
<400> 70
   ttgttctgaa tgtcca 16
<210> 71
   <211> 4
   <212> DNA
   <213> Artificial Sequence
<220>
   <223> Synthetic Oligonucleotide
<400> 71
   actg 4
<210> 72
   <211> 4
   <212> DNA
   <213> Artificial Sequence
<220>
   <223> Synthetic Oligonucleotide
<400> 72
   actg 4
<210> 73
   <211> 14
   <212> DNA
   <213> Artificial Sequence
<220>
   <223> Synthetic Oligonucleotide
<400> 73
   tgttctgaat gtcc 14
<210> 74
   <211> 16
   <212> DNA
   <213> Artificial Sequence
<220>
   <223> Synthetic Oligonucleotide
<400> 74
   cagatgagtc catttg 16
<210> 75
   <211> 14
   <212> DNA
   <213> Artificial Sequence
<220>
   <223> Synthetic Oligonucleotide
<400> 75
   agatgagtcc attt 14
<210> 76
   <211> 16
   <212> DNA
   <213> Artificial Sequence
<220>
   <223> Synthetic Oligonucleotide
<400> 76
   atccacaggg aaattg 16
<210> 77
   <211> 14
   <212> DNA
   <213> Artificial Sequence
<220>
   <223> Synthetic Oligonucleotide
<400> 77
   tccacaggga aatt 14
<210> 78
   <211> 16
   <212> DNA
   <213> Artificial Sequence
<220>
   <223> Synthetic Oligonucleotide
<400> 78
   cagttgtaca agttgc 16
<210> 79
   <211> 14
   <212> DNA
   <213> Artificial Sequence
<220>
   <223> Synthetic Oligonucleotide
<400> 79
   agttgtacaa gttg 14
<210> 80
   <211> 16
   <212> DNA
   <213> Artificial Sequence
<220>
   <223> Synthetic Oligonucleotide
<400> 80
   cacagagtca gccttc 16
<210> 81
   <211> 14
   <212> DNA
   <213> Artificial Sequence
<220>
   <223> Synthetic Oligonucleotide
<400> 81
   acagagtcag cctt 14
<210> 82
   <211> 16
   <212> DNA
   <213> Artificial Sequence
<220>
   <223> Synthetic Oligonucleotide
<400> 82
   ggtcaaccac agagtc 16
<210> 83
   <211> 14
   <212> DNA
   <213> Artificial Sequence
<220>
   <223> Synthetic Oligonucleotide
<400> 83
   gtcaaccaca gagt 14
<210> 84
   <211> 14
   <212> DNA
   <213> Artificial Sequence
<220>
   <223> Synthetic Oligonucleotide
<400> 84
   cagccacatg cagc 14
<210> 85
   <211> 14
   <212> DNA
   <213> Artificial Sequence
<220>
   <223> Synthetic Oligonucleotide
<400> 85
   ccagccacat gcag 14
<210> 86
   <211> 14
   <212> DNA
   <213> Artificial Sequence
<220>
   <223> Synthetic Oligonucleotide
<400> 86
   accagccaca tgca 14
<210> 87
   <211> 14
   <212> DNA
   <213> Artificial Sequence
<220>
   <223> Synthetic Oligonucleotide
<400> 87
   taccagccac atgc 14
<210> 88
   <211> 14
   <212> DNA
   <213> Artificial Sequence
<220>
   <223> Synthetic Oligonucleotide
<400> 88
   ttaccagcca catg 14
<210> 89
   <211> 14
   <212> DNA
   <213> Artificial Sequence
<220>
   <223> Synthetic Oligonucleotide
<400> 89
   gttaccagcc acat 14
<210> 90
   <211> 14
   <212> DNA
   <213> Artificial Sequence
<220>
   <223> Synthetic Oligonucleotide
<400> 90
   ggttaccagc caca 14
<210> 91
   <211> 14
   <212> DNA
   <213> Artificial Sequence
<220>
   <223> Synthetic Oligonucleotide
<400> 91
   aggttaccag ccac 14
<210> 92
   <211> 14
   <212> DNA
   <213> Artificial Sequence
<220>
   <223> Synthetic Oligonucleotide
<400> 92
   taggttacca gcca 14
<210> 93
   <211> 16
   <212> DNA
   <213> Artificial Sequence
<220>
   <223> Synthetic Oligonucleotide
<400> 93
   aggttctgct ttcaac 16
<210> 94
   <211> 14
   <212> DNA
   <213> Artificial Sequence
<220>
   <223> Synthetic Oligonucleotide
<400> 94
   ggttctgctt tcaa 14
<210> 95
   <211> 16
   <212> DNA
   <213> Artificial Sequence
<220>
   <223> Synthetic Oligonucleotide
<400> 95
   tactgatcaa attgta 16
<210> 96
   <211> 14
   <212> DNA
   <213> Artificial Sequence
<220>
   <223> Synthetic Oligonucleotide
<400> 96
   actgatcaaa ttgt 14
<210> 97
   <211> 16
   <212> DNA
   <213> Artificial Sequence
<220>
   <223> Synthetic Oligonucleotide
<400> 97
   tttttcttgt atctgg 16
<210> 98
   <211> 14
   <212> DNA
   <213> Artificial Sequence
<220>
   <223> Synthetic Oligonucleotide
<400> 98
   ttttcttgta tctg 14
<210> 99
   <211> 16
   <212> DNA
   <213> Artificial Sequence
<220>
   <223> Synthetic Oligonucleotide
<400> 99
   atccattaaa acctgg 16
<210> 100
   <211> 14
   <212> DNA
   <213> Artificial Sequence
<220>
   <223> Synthetic Oligonucleotide
<400> 100
   tccattaaaa cctg 14
<210> 101
   <211> 16
   <212> DNA
   <213> Artificial Sequence
<220>
   <223> Synthetic Oligonucleotide
<400> 101
   atattgctct gcaaag 16
<210> 102
   <211> 14
   <212> DNA
   <213> Artificial Sequence
<220>
   <223> Synthetic Oligonucleotide
<400> 102
   tattgctctg caaa 14
<210> 103
   <211> 4
   <212> DNA
   <213> Artificial Sequence
<220>
   <223> Synthetic Oligonucleotide
<400> 103
   actg 4
<210> 104
   <211> 14
   <212> DNA
   <213> Artificial Sequence
<220>
   <223> Synthetic Oligonucleotide
<400> 104
   atattgctct gcaa 14
<210> 105
   <211> 14
   <212> DNA
   <213> Artificial Sequence
<220>
   <223> Synthetic Oligonucleotide
<400> 105
   aatattgctc tgca 14
<210> 106
   <211> 14
   <212> DNA
   <213> Artificial Sequence
<220>
   <223> Synthetic Oligonucleotide
<400> 106
   gaatattgct ctgc 14
<210> 107
   <211> 14
   <212> DNA
   <213> Artificial Sequence
<220>
   <223> Synthetic Oligonucleotide
<400> 107
   agaatattgc tctg 14
<210> 108
   <211> 14
   <212> DNA
   <213> Artificial Sequence
<220>
   <223> Synthetic Oligonucleotide
<400> 108
   tagaatattg ctct 14
<210> 109
   <211> 14
   <212> DNA
   <213> Artificial Sequence
<220>
   <223> Synthetic Oligonucleotide
<400> 109
   atagaatatt gctc 14
<210> 110
   <211> 14
   <212> DNA
   <213> Artificial Sequence
<220>
   <223> Synthetic Oligonucleotide
<400> 110
   gatagaatat tgct 14
<210> 111
   <211> 14
   <212> DNA
   <213> Artificial Sequence
<220>
   <223> Synthetic Oligonucleotide
<400> 111
   ggatagaata ttgc 14
<210> 112
   <211> 16
   <212> DNA
   <213> Artificial Sequence
<220>
   <223> Synthetic Oligonucleotide
<400> 112
   atggaatcct caaatc 16
<210> 113
   <211> 14
   <212> DNA
   <213> Artificial Sequence
<220>
   <223> Synthetic Oligonucleotide
<400> 113
   tggaatcctc aaat 14
<210> 114
   <211> 16
   <212> DNA
   <213> Artificial Sequence
<220>
   <223> Synthetic Oligonucleotide
<400> 114
   gaattctggt atgtga 16
<210> 115
   <211> 14
   <212> DNA
   <213> Artificial Sequence
<220>
   <223> Synthetic Oligonucleotide
<400> 115
   aattctggta tgtg 14
<210> 116
   <211> 16
   <212> DNA
   <213> Artificial Sequence
<220>
   <223> Synthetic Oligonucleotide
<400> 116
   agctggaatt ctggta 16
<210> 117
   <211> 14
   <212> DNA
   <213> Artificial Sequence
<220>
   <223> Synthetic Oligonucleotide
<400> 117
   gctggaattc tggt 14
<210> 118
   <211> 16
   <212> DNA
   <213> Artificial Sequence
<220>
   <223> Synthetic Oligonucleotide
<400> 118
   tgaaaatcaa aattga 16
<210> 119
   <211> 14
   <212> DNA
   <213> Artificial Sequence
<220>
   <223> Synthetic Oligonucleotide
<400> 119
   gaaaatcaaa attg 14
<210> 120
   <211> 16
   <212> DNA
   <213> Artificial Sequence
<220>
   <223> Synthetic Oligonucleotide
<400> 120
   aaacagtgca tagtta 16
<210> 121
   <211> 14
   <212> DNA
   <213> Artificial Sequence
<220>
   <223> Synthetic Oligonucleotide
<400> 121
   aacagtgcat agtt 14
<210> 122
   <211> 16
   <212> DNA
   <213> Artificial Sequence
<220>
   <223> Synthetic Oligonucleotide
<400> 122
   ttcaggaatt gttaaa 16
<210> 123
   <211> 14
   <212> DNA
   <213> Artificial Sequence
<220>
   <223> Synthetic Oligonucleotide
<400> 123
   tcaggaattg ttaa 14
<210> 124
   <211> 16
   <212> DNA
   <213> Artificial Sequence
<220>
   <223> Synthetic Oligonucleotide
<400> 124
   ttttgtttca ttatag 16
<210> 125
   <211> 14
   <212> DNA
   <213> Artificial Sequence
<220>
   <223> Synthetic Oligonucleotide
<400> 125
   tttgtttcat tata 14
<210> 126
   <211> 16
   <212> DNA
   <213> Artificial Sequence
<220>
   <223> Synthetic Oligonucleotide
<400> 126
   gatgacactt gattta 16
<210> 127
   <211> 14
   <212> DNA
   <213> Artificial Sequence
<220>
   <223> Synthetic Oligonucleotide
<400> 127
   atgacacttg attt 14
<210> 128
   <211> 16
   <212> DNA
   <213> Artificial Sequence
<220>
   <223> Synthetic Oligonucleotide
<400> 128
   gtgtgatgac acttga 16
<210> 129
   <211> 14
   <212> DNA
   <213> Artificial Sequence
<220>
   <223> Synthetic Oligonucleotide
<400> 129
   tgtgatgaca cttg 14
<210> 130
   <211> 16
   <212> DNA
   <213> Artificial Sequence
<220>
   <223> Synthetic Oligonucleotide
<400> 130
   tattcagtgt gatgac 16
<210> 131
   <211> 14
   <212> DNA
   <213> Artificial Sequence
<220>
   <223> Synthetic Oligonucleotide
<400> 131
   attcagtgtg atga 14
<210> 132
   <211> 16
   <212> DNA
   <213> Artificial Sequence
<220>
   <223> Synthetic Oligonucleotide
<400> 132
   attggtattc agtgtg 16
<210> 133
   <211> 14
   <212> DNA
   <213> Artificial Sequence
<220>
   <223> Synthetic Oligonucleotide
<400> 133
   ttggtattca gtgt 14
<210> 134
   <211> 14
   <212> DNA
   <213> Artificial Sequence
<220>
   <223> Synthetic Oligonucleotide
<400> 134
   cctctagctg taag 14
<210> 135
   <211> 14
   <212> DNA
   <213> Artificial Sequence
<220>
   <223> Synthetic Oligonucleotide
<400> 135
   ccctctagct gtaa 14
<210> 136
   <211> 14
   <212> DNA
   <213> Artificial Sequence
<220>
   <223> Synthetic Oligonucleotide
<400> 136
   gccctctagc tgta 14
<210> 137
   <211> 14
   <212> DNA
   <213> Artificial Sequence
<220>
   <223> Synthetic Oligonucleotide
<400> 137
   ggccctctag ctgt 14
<210> 138
   <211> 14
   <212> DNA
   <213> Artificial Sequence
<220>
   <223> Synthetic Oligonucleotide
<400> 138
   aggccctcta gctg 14
<210> 139
   <211> 14
   <212> DNA
   <213> Artificial Sequence
<220>
   <223> Synthetic Oligonucleotide
<400> 139
   gaggccctct agct 14
<210> 140
   <211> 14
   <212> DNA
   <213> Artificial Sequence
<220>
   <223> Synthetic Oligonucleotide
<400> 140
   agaggccctc tagc 14
<210> 141
   <211> 14
   <212> DNA
   <213> Artificial Sequence
<220>
   <223> Synthetic Oligonucleotide
<400> 141
   aagaggccct ctag 14
<210> 142
   <211> 14
   <212> DNA
   <213> Artificial Sequence
<220>
   <223> Synthetic Oligonucleotide
<400> 142
   aaagaggccc tcta 14
<210> 143
   <211> 16
   <212> DNA
   <213> Artificial Sequence
<220>
   <223> Synthetic Oligonucleotide
<400> 143
   gaatggacag gtcaat 16
<210> 144
   <211> 14
   <212> DNA
   <213> Artificial Sequence
<220>
   <223> Synthetic Oligonucleotide
<400> 144
   aatggacagg tcaa 14
<210> 145
   <211> 16
   <212> DNA
   <213> Artificial Sequence
<220>
   <223> Synthetic Oligonucleotide
<400> 145
   gttttgaatg gacagg 16
<210> 146
   <211> 14
   <212> DNA
   <213> Artificial Sequence
<220>
   <223> Synthetic Oligonucleotide
<400> 146
   ttttgaatgg acag 14
<210> 147
   <211> 16
   <212> DNA
   <213> Artificial Sequence
<220>
   <223> Synthetic Oligonucleotide
<400> 147
   tggtagtttt gaatgg 16
<210> 148
   <211> 14
   <212> DNA
   <213> Artificial Sequence
<220>
   <223> Synthetic Oligonucleotide
<400> 148
   ggtagttttg aatg 14
<210> 149
   <211> 14
   <212> DNA
   <213> Artificial Sequence
<220>
   <223> Synthetic Oligonucleotide
<400> 149
   tcactgtatg gttt 14
<210> 150
   <211> 14
   <212> DNA
   <213> Artificial Sequence
<220>
   <223> Synthetic Oligonucleotide
<400> 150
   ctcactgtat ggtt 14
<210> 151
   <211> 14
   <212> DNA
   <213> Artificial Sequence
<220>
   <223> Synthetic Oligonucleotide
<400> 151
   gctcactgta tggt 14
<210> 152
   <211> 14
   <212> DNA
   <213> Artificial Sequence
<220>
   <223> Synthetic Oligonucleotide
<400> 152
   ggctcactgt atgg 14
<210> 153
   <211> 14
   <212> DNA
   <213> Artificial Sequence
<220>
   <223> Synthetic Oligonucleotide
<400> 153
   tggctcactg tatg 14
<210> 154
   <211> 14
   <212> DNA
   <213> Artificial Sequence
<220>
   <223> Synthetic Oligonucleotide
<400> 154
   ctggctcact gtat 14
<210> 155
   <211> 14
   <212> DNA
   <213> Artificial Sequence
<220>
   <223> Synthetic Oligonucleotide
<400> 155
   gctggctcac tgta 14
<210> 156
   <211> 14
   <212> DNA
   <213> Artificial Sequence
<220>
   <223> Synthetic Oligonucleotide
<400> 156
   ggctggctca ctgt 14
<210> 157
   <211> 14
   <212> DNA
   <213> Artificial Sequence
<220>
   <223> Synthetic Oligonucleotide
<400> 157
   aggctggctc actg 14
<210> 158
   <211> 14
   <212> DNA
   <213> Artificial Sequence
<220>
   <223> Synthetic Oligonucleotide
<400> 158
   caggtccagt tcat 14
<210> 159
   <211> 14
   <212> DNA
   <213> Artificial Sequence
<220>
   <223> Synthetic Oligonucleotide
<400> 159
   gcaggtccag ttca 14
<210> 160
   <211> 14
   <212> DNA
   <213> Artificial Sequence
<220>
   <223> Synthetic Oligonucleotide
<400> 160
   tgcaggtcca gttc 14
<210> 161
   <211> 14
   <212> DNA
   <213> Artificial Sequence
<220>
   <223> Synthetic Oligonucleotide
<400> 161
   gtgcaggtcc agtt 14
<210> 162
   <211> 14
   <212> DNA
   <213> Artificial Sequence
<220>
   <223> Synthetic Oligonucleotide
<400> 162
   ggtgcaggtc cagt 14
<210> 163
   <211> 14
   <212> DNA
   <213> Artificial Sequence
<220>
   <223> Synthetic Oligonucleotide
<400> 163
   tggtgcaggt ccag 14
<210> 164
   <211> 14
   <212> DNA
   <213> Artificial Sequence
<220>
   <223> Synthetic Oligonucleotide
<400> 164
   ttggtgcagg tcca 14
<210> 165
   <211> 14
   <212> DNA
   <213> Artificial Sequence
<220>
   <223> Synthetic Oligonucleotide
<400> 165
   tttggtgcag gtcc 14
<210> 166
   <211> 14
   <212> DNA
   <213> Artificial Sequence
<220>
   <223> Synthetic Oligonucleotide
<400> 166
   ctttggtgca ggtc 14
<210> 167
   <211> 14
   <212> DNA
   <213> Artificial Sequence
<220>
   <223> Synthetic Oligonucleotide
<400> 167
   taactcagat cctg 14
<210> 168
   <211> 14
   <212> DNA
   <213> Artificial Sequence
<220>
   <223> Synthetic Oligonucleotide
<400> 168
   ataactcaga tcct 14
<210> 169
   <211> 14
   <212> DNA
   <213> Artificial Sequence
<220>
   <223> Synthetic Oligonucleotide
<400> 169
   aataactcag atcc 14
<210> 170
   <211> 14
   <212> DNA
   <213> Artificial Sequence
<220>
   <223> Synthetic Oligonucleotide
<400> 170
   aaataactca gatc 14
<210> 171
   <211> 14
   <212> DNA
   <213> Artificial Sequence
<220>
   <223> Synthetic Oligonucleotide
<400> 171
   aaaataactc agat 14
<210> 172
   <211> 14
   <212> DNA
   <213> Artificial Sequence
<220>
   <223> Synthetic Oligonucleotide
<400> 172
   caaaataact caga 14
<210> 173
   <211> 14
   <212> DNA
   <213> Artificial Sequence
<220>
   <223> Synthetic Oligonucleotide
<400> 173
   gcaaaataac tcag 14
<210> 174
   <211> 14
   <212> DNA
   <213> Artificial Sequence
<220>
   <223> Synthetic Oligonucleotide
<400> 174
   agcaaaataa ctca 14
<210> 175
   <211> 14
   <212> DNA
   <213> Artificial Sequence
<220>
   <223> Synthetic Oligonucleotide
<400> 175
   tagcaaaata actc 14
<210> 176
   <211> 14
   <212> DNA
   <213> Artificial Sequence
<220>
   <223> Synthetic Oligonucleotide
<400> 176
   cagggcctgg agag 14
<210> 177
   <211> 16
   <212> DNA
   <213> Artificial Sequence
<220>
   <223> Synthetic Oligonucleotide
<400> 177
   tctgaagtcc atgatc 16
<210> 178
   <211> 14
   <212> DNA
   <213> Artificial Sequence
<220>
   <223> Synthetic Oligonucleotide
<400> 178
   ctgaagtcca tgat 14
<210> 179
   <211> 16
   <212> DNA
   <213> Artificial Sequence
<220>
   <223> Synthetic Oligonucleotide
<400> 179
   tgggcatgat tccatt 16
<210> 180
   <211> 14
   <212> DNA
   <213> Artificial Sequence
<220>
   <223> Synthetic Oligonucleotide
<400> 180
   gggcatgatt ccat 14
<210> 181
   <211> 14
   <212> DNA
   <213> Artificial Sequence
<220>
   <223> Synthetic Oligonucleotide
<400> 181
   tggagcccac gtgc 14
<210> 182
   <211> 16
   <212> DNA
   <213> Artificial Sequence
<220>
   <223> Synthetic Oligonucleotide
<400> 182
   ttgaagttga gggctg 16
<210> 183
   <211> 14
   <212> DNA
   <213> Artificial Sequence
<220>
   <223> Synthetic Oligonucleotide
<400> 183
   tgaagttgag ggct 14
<210> 184
   <211> 16
   <212> DNA
   <213> Artificial Sequence
<220>
   <223> Synthetic Oligonucleotide
<400> 184
   accagtatta attttg 16
<210> 185
   <211> 14
   <212> DNA
   <213> Artificial Sequence
<220>
   <223> Synthetic Oligonucleotide
<400> 185
   ccagtattaa tttt 14
<210> 186
   <211> 16
   <212> DNA
   <213> Artificial Sequence
<220>
   <223> Synthetic Oligonucleotide
<400> 186
   gtgttctttg aagcgg 16
<210> 187
   <211> 14
   <212> DNA
   <213> Artificial Sequence
<220>
   <223> Synthetic Oligonucleotide
<400> 187
   tgttctttga agcg 14
<210> 188
   <211> 14
   <212> DNA
   <213> Artificial Sequence
<220>
   <223> Synthetic Oligonucleotide
<400> 188
   agttactttg gtgt 14
<210> 189
   <211> 16
   <212> DNA
   <213> Artificial Sequence
<220>
   <223> Synthetic Oligonucleotide
<400> 189
   tggtacatgg aagtct 16
<210> 190
   <211> 14
   <212> DNA
   <213> Artificial Sequence
<220>
   <223> Synthetic Oligonucleotide
<400> 190
   ggtacatgga agtc 14
<210> 191
   <211> 4
   <212> DNA
   <213> Artificial Sequence
<220>
   <223> Synthetic Oligonucleotide
<400> 191
   actg 4
<210> 192
   <211> 4
   <212> DNA
   <213> Artificial Sequence
<220>
   <223> Synthetic Oligonucleotide
<400> 192
   actg 4
<210> 193
   <211> 4
   <212> DNA
   <213> Artificial Sequence
<220>
   <223> Synthetic Oligonucleotide
<400> 193
   actg 4
<210> 194
   <211> 4
   <212> DNA
   <213> Artificial Sequence
<220>
   <223> Synthetic Oligonucleotide
<400> 194
   actg 4
<210> 195
   <211> 4
   <212> DNA
   <213> Artificial Sequence
<220>
   <223> Synthetic Oligonucleotide
<400> 195
   actg 4
<210> 196
   <211> 4
   <212> DNA
   <213> Artificial Sequence
<220>
   <223> Synthetic Oligonucleotide
<400> 196
   actg 4
<210> 197
   <211> 4
   <212> DNA
   <213> Artificial Sequence
<220>
   <223> Synthetic Oligonucleotide
<400> 197
   actg 4
<210> 198
   <211> 4
   <212> DNA
   <213> Artificial Sequence
<220>
   <223> Synthetic Oligonucleotide
<400> 198
   actg 4
<210> 199
   <211> 4
   <212> DNA
   <213> Artificial Sequence
<220>
   <223> Synthetic Oligonucleotide
<400> 199
   actg 4
<210> 200
   <211> 14
   <212> DNA
   <213> Artificial Sequence
<220>
   <223> Synthetic Oligonucleotide
<400> 200
   tccatgccat atgt 14
<210> 201
   <211> 16
   <212> DNA
   <213> Artificial Sequence
<220>
   <223> Synthetic Oligonucleotide
<400> 201
   ccctgaagaa gtccat 16
<210> 202
   <211> 14
   <212> DNA
   <213> Artificial Sequence
<220>
   <223> Synthetic Oligonucleotide
<400> 202
   cctgaagaag tcca 14
<210> 203
   <211> 16
   <212> DNA
   <213> Artificial Sequence
<220>
   <223> Synthetic Oligonucleotide
<400> 203
   gcccagttcc atgacc 16
<210> 204
   <211> 14
   <212> DNA
   <213> Artificial Sequence
<220>
   <223> Synthetic Oligonucleotide
<400> 204
   cccagttcca tgac 14
<210> 205
   <211> 16
   <212> DNA
   <213> Artificial Sequence
<220>
   <223> Synthetic Oligonucleotide
<400> 205
   ttgaggaagc cagatt 16
<210> 206
   <211> 14
   <212> DNA
   <213> Artificial Sequence
<220>
   <223> Synthetic Oligonucleotide
<400> 206
   tgaggaagcc agat 14
<210> 207
   <211> 16
   <212> DNA
   <213> Artificial Sequence
<220>
   <223> Synthetic Oligonucleotide
<400> 207
   tggatgcagt aatctc 16
<210> 208
   <211> 14
   <212> DNA
   <213> Artificial Sequence
<220>
   <223> Synthetic Oligonucleotide
<400> 208
   ggatgcagta atct 14
<210> 209
   <211> 16
   <212> DNA
   <213> Artificial Sequence
<220>
   <223> Synthetic Oligonucleotide
<400> 209
   tataaagtcc agcatt 16
<210> 210
   <211> 14
   <212> DNA
   <213> Artificial Sequence
<220>
   <223> Synthetic Oligonucleotide
<400> 210
   ataaagtcca gcat 14
<210> 211
   <211> 16
   <212> DNA
   <213> Artificial Sequence
<220>
   <223> Synthetic Oligonucleotide
<400> 211
   aagttcctgc ttgaag 16
<210> 212
   <211> 14
   <212> DNA
   <213> Artificial Sequence
<220>
   <223> Synthetic Oligonucleotide
<400> 212
   agttcctgct tgaa 14
<210> 213
   <211> 14
   <212> DNA
   <213> Artificial Sequence
<220>
   <223> Synthetic Oligonucleotide
<400> 213
   aatggtgaag tact 14
<210> 214
   <211> 12
   <212> DNA
   <213> Artificial Sequence
<220>
   <223> Synthetic Oligonucleotide
<400> 214
   tgtcagcagg at 12
<210> 215
   <211> 12
   <212> DNA
   <213> Artificial Sequence
<220>
   <223> Synthetic Oligonucleotide
<400> 215
   cagcaggaaa ta 12
<210> 216
   <211> 12
   <212> DNA
   <213> Artificial Sequence
<220>
   <223> Synthetic Oligonucleotide
<400> 216
   ccagcaggaa at 12
<210> 217
   <211> 12
   <212> DNA
   <213> Artificial Sequence
<220>
   <223> Synthetic Oligonucleotide
<400> 217
   accagcagga aa 12
<210> 218
   <211> 12
   <212> DNA
   <213> Artificial Sequence
<220>
   <223> Synthetic Oligonucleotide
<400> 218
   gaccagcagg aa 12
<210> 219
   <211> 12
   <212> DNA
   <213> Artificial Sequence
<220>
   <223> Synthetic Oligonucleotide
<400> 219
   tgaccagcag ga 12
<210> 220
   <211> 4
   <212> DNA
   <213> Artificial Sequence
<220>
   <223> Synthetic Oligonucleotide
<400> 220
   actg 4
<210> 221
   <211> 12
   <212> DNA
   <213> Artificial Sequence
<220>
   <223> Synthetic Oligonucleotide
<400> 221
   atgaccagca gg 12
<210> 222
   <211> 12
   <212> DNA
   <213> Artificial Sequence
<220>
   <223> Synthetic Oligonucleotide
<400> 222
   aatgaccagc ag 12
<210> 223
   <211> 12
   <212> DNA
   <213> Artificial Sequence
<220>
   <223> Synthetic Oligonucleotide
<400> 223
   caatgaccag ca 12
<210> 224
   <211> 12
   <212> DNA
   <213> Artificial Sequence
<220>
   <223> Synthetic Oligonucleotide
<400> 224
   ccaatgacca gc 12
<210> 225
   <211> 12
   <212> DNA
   <213> Artificial Sequence
<220>
   <223> Synthetic Oligonucleotide
<400> 225
   accacaagcc aa 12
<210> 226
   <211> 12
   <212> DNA
   <213> Artificial Sequence
<220>
   <223> Synthetic Oligonucleotide
<400> 226
   tagccgccca ca 12
<210> 227
   <211> 4
   <212> DNA
   <213> Artificial Sequence
<220>
   <223> Synthetic Oligonucleotide
<400> 227
   actg 4
<210> 228
   <211> 12
   <212> DNA
   <213> Artificial Sequence
<220>
   <223> Synthetic Oligonucleotide
<400> 228
   ccggccacca ca 12
<210> 229
   <211> 12
   <212> DNA
   <213> Artificial Sequence
<220>
   <223> Synthetic Oligonucleotide
<400> 229
   accggccacc ac 12
<210> 230
   <211> 12
   <212> DNA
   <213> Artificial Sequence
<220>
   <223> Synthetic Oligonucleotide
<400> 230
   gatgttgctg gc 12
<210> 231
   <211> 4
   <212> DNA
   <213> Artificial Sequence
<220>
   <223> Synthetic Oligonucleotide
<400> 231
   actg 4
<210> 232
   <211> 12
   <212> DNA
   <213> Artificial Sequence
<220>
   <223> Synthetic Oligonucleotide
<400> 232
   cgatgttgct gg 12
<210> 233
   <211> 12
   <212> DNA
   <213> Artificial Sequence
<220>
   <223> Synthetic Oligonucleotide
<400> 233
   ccgatgttgc tg 12
<210> 234
   <211> 12
   <212> DNA
   <213> Artificial Sequence
<220>
   <223> Synthetic Oligonucleotide
<400> 234
   gccgatgttg ct 12
<210> 235
   <211> 12
   <212> DNA
   <213> Artificial Sequence
<220>
   <223> Synthetic Oligonucleotide
<400> 235
   tgccgatgtt gc 12
<210> 236
   <211> 12
   <212> DNA
   <213> Artificial Sequence
<220>
   <223> Synthetic Oligonucleotide
<400> 236
   ctgccgatgt tg 12
<210> 237
   <211> 12
   <212> DNA
   <213> Artificial Sequence
<220>
   <223> Synthetic Oligonucleotide
<400> 237
   caggcccaca aa 12
<210> 238
   <211> 12
   <212> DNA
   <213> Artificial Sequence
<220>
   <223> Synthetic Oligonucleotide
<400> 238
   ccaggcccac aa 12
<210> 239
   <211> 12
   <212> DNA
   <213> Artificial Sequence
<220>
   <223> Synthetic Oligonucleotide
<400> 239
   gccaggccca ca 12
<210> 240
   <211> 12
   <212> DNA
   <213> Artificial Sequence
<220>
   <223> Synthetic Oligonucleotide
<400> 240
   ccaagccact tg 12
<210> 241
   <211> 12
   <212> DNA
   <213> Artificial Sequence
<220>
   <223> Synthetic Oligonucleotide
<400> 241
   agagcgcatt cc 12
<210> 242
   <211> 12
   <212> DNA
   <213> Artificial Sequence
<220>
   <223> Synthetic Oligonucleotide
<400> 242
   acaggtagag gc 12
<210> 243
   <211> 12
   <212> DNA
   <213> Artificial Sequence
<220>
   <223> Synthetic Oligonucleotide
<400> 243
   agatcttggt ga 12
<210> 244
   <211> 4
   <212> DNA
   <213> Artificial Sequence
<220>
   <223> Synthetic Oligonucleotide
<400> 244
   actg 4
<210> 245
   <211> 13
   <212> DNA
   <213> Artificial Sequence
<220>
   <223> Synthetic Oligonucleotide
<400> 245
   tgttccagcc cag 13
<210> 246
   <211> 12
   <212> DNA
   <213> Artificial Sequence
<220>
   <223> Synthetic Oligonucleotide
<400> 246
   tgttccagcc ca 12
<210> 247
   <211> 4
   <212> DNA
   <213> Artificial Sequence
<220>
   <223> Synthetic Oligonucleotide
<400> 247
   actg 4
<210> 248
   <211> 4
   <212> DNA
   <213> Artificial Sequence
<220>
   <223> Synthetic Oligonucleotide
<400> 248
   actg 4
<210> 249
   <211> 4
   <212> DNA
   <213> Artificial Sequence
<220>
   <223> Synthetic Oligonucleotide
<400> 249
   actg 4
<210> 250
   <211> 4
   <212> DNA
   <213> Artificial Sequence
<220>
   <223> Synthetic Oligonucleotide
<400> 250
   actg 4
<210> 251
   <211> 12
   <212> DNA
   <213> Artificial Sequence
<220>
   <223> Synthetic Oligonucleotide
<400> 251
   atgttccagc cc 12
<210> 252
   <211> 12
   <212> DNA
   <213> Artificial Sequence
<220>
   <223> Synthetic Oligonucleotide
<400> 252
   tggtgatgcc ca 12
<210> 253
   <211> 12
   <212> DNA
   <213> Artificial Sequence
<220>
   <223> Synthetic Oligonucleotide
<400> 253
   atggtgatgc cc 12
<210> 254
   <211> 12
   <212> DNA
   <213> Artificial Sequence
<220>
   <223> Synthetic Oligonucleotide
<400> 254
   catggtgatg cc 12
<210> 255
   <211> 4
   <212> DNA
   <213> Artificial Sequence
<220>
   <223> Synthetic Oligonucleotide
<400> 255
   actg 4
<210> 256
   <211> 12
   <212> DNA
   <213> Artificial Sequence
<220>
   <223> Synthetic Oligonucleotide
<400> 256
   tcatggtgat gc 12
<210> 257
   <211> 12
   <212> DNA
   <213> Artificial Sequence
<220>
   <223> Synthetic Oligonucleotide
<400> 257
   atcatggtga tg 12
<210> 258
   <211> 12
   <212> DNA
   <213> Artificial Sequence
<220>
   <223> Synthetic Oligonucleotide
<400> 258
   ccctcctgtc ac 12
<210> 259
   <211> 12
   <212> DNA
   <213> Artificial Sequence
<220>
   <223> Synthetic Oligonucleotide
<400> 259
   gccctcctgt ca 12
<210> 260
   <211> 12
   <212> DNA
   <213> Artificial Sequence
<220>
   <223> Synthetic Oligonucleotide
<400> 260
   agccctcctg tc 12
<210> 261
   <211> 12
   <212> DNA
   <213> Artificial Sequence
<220>
   <223> Synthetic Oligonucleotide
<400> 261
   cagccctcct gt 12
<210> 262
   <211> 12
   <212> DNA
   <213> Artificial Sequence
<220>
   <223> Synthetic Oligonucleotide
<400> 262
   ccagccctcc tg 12
<210> 263
   <211> 12
   <212> DNA
   <213> Artificial Sequence
<220>
   <223> Synthetic Oligonucleotide
<400> 263
   gccagccctc ct 12
<210> 264
   <211> 12
   <212> DNA
   <213> Artificial Sequence
<220>
   <223> Synthetic Oligonucleotide
<400> 264
   gacgaaggtc tg 12
<210> 265
   <211> 12
   <212> DNA
   <213> Artificial Sequence
<220>
   <223> Synthetic Oligonucleotide
<400> 265
   gtatttgtcg aa 12
<210> 266
   <211> 12
   <212> DNA
   <213> Artificial Sequence
<220>
   <223> Synthetic Oligonucleotide
<400> 266
   ggacaccgtc ag 12
<210> 267
   <211> 12
   <212> DNA
   <213> Artificial Sequence
<220>
   <223> Synthetic Oligonucleotide
<400> 267
   cagcttcagg ta 12
<210> 268
   <211> 12
   <212> DNA
   <213> Artificial Sequence
<220>
   <223> Synthetic Oligonucleotide
<400> 268
   catgaccatg ag 12
<210> 269
   <211> 12
   <212> DNA
   <213> Artificial Sequence
<220>
   <223> Synthetic Oligonucleotide
<400> 269
   gcatgaccat ga 12
<210> 270
   <211> 12
   <212> DNA
   <213> Artificial Sequence
<220>
   <223> Synthetic Oligonucleotide
<400> 270
   ggcatgacca tg 12
<210> 271
   <211> 12
   <212> DNA
   <213> Artificial Sequence
<220>
   <223> Synthetic Oligonucleotide
<400> 271
   tggcatgacc at 12
<210> 272
   <211> 12
   <212> DNA
   <213> Artificial Sequence
<220>
   <223> Synthetic Oligonucleotide
<400> 272
   ctggcatgac ca 12
<210> 273
   <211> 4
   <212> DNA
   <213> Artificial Sequence
<220>
   <223> Synthetic Oligonucleotide
<400> 273
   actg 4
<210> 274
   <211> 12
   <212> DNA
   <213> Artificial Sequence
<220>
   <223> Synthetic Oligonucleotide
<400> 274
   cctggcatga cc 12
<210> 275
   <211> 12
   <212> DNA
   <213> Artificial Sequence
<220>
   <223> Synthetic Oligonucleotide
<400> 275
   gcagcccacc tc 12
<210> 276
   <211> 12
   <212> DNA
   <213> Artificial Sequence
<220>
   <223> Synthetic Oligonucleotide
<400> 276
   agcagcccac ct 12
<210> 277
   <211> 12
   <212> DNA
   <213> Artificial Sequence
<220>
   <223> Synthetic Oligonucleotide
<400> 277
   gagcagccca cc 12
<210> 278
   <211> 12
   <212> DNA
   <213> Artificial Sequence
<220>
   <223> Synthetic Oligonucleotide
<400> 278
   catgagcttc ac 12
<210> 279
   <211> 12
   <212> DNA
   <213> Artificial Sequence
<220>
   <223> Synthetic Oligonucleotide
<400> 279
   gcatgagctt ca 12
<210> 280
   <211> 13
   <212> DNA
   <213> Artificial Sequence
<220>
   <223> Synthetic Oligonucleotide
<400> 280
   ggcatgagct tca 13
<210> 281
   <211> 12
   <212> DNA
   <213> Artificial Sequence
<220>
   <223> Synthetic Oligonucleotide
<400> 281
   ggcatgagct tc 12
<210> 282
   <211> 4
   <212> DNA
   <213> Artificial Sequence
<220>
   <223> Synthetic Oligonucleotide
<400> 282
   actg 4
<210> 283
   <211> 12
   <212> DNA
   <213> Artificial Sequence
<220>
   <223> Synthetic Oligonucleotide
<400> 283
   gggcatgagc tt 12
<210> 284
   <211> 12
   <212> DNA
   <213> Artificial Sequence
<220>
   <223> Synthetic Oligonucleotide
<400> 284
   cagcatgagt cc 12
<210> 285
   <211> 12
   <212> DNA
   <213> Artificial Sequence
<220>
   <223> Synthetic Oligonucleotide
<400> 285
   ccagcatgag tc 12
<210> 286
   <211> 4
   <212> DNA
   <213> Artificial Sequence
<220>
   <223> Synthetic Oligonucleotide
<400> 286
   actg 4
<210> 287
   <211> 12
   <212> DNA
   <213> Artificial Sequence
<220>
   <223> Synthetic Oligonucleotide
<400> 287
   gccagcatga gt 12
<210> 288
   <211> 12
   <212> DNA
   <213> Artificial Sequence
<220>
   <223> Synthetic Oligonucleotide
<400> 288
   ccatggtgaa ga 12
<210> 289
   <211> 12
   <212> DNA
   <213> Artificial Sequence
<220>
   <223> Synthetic Oligonucleotide
<400> 289
   cacagctgcc cg 12
<210> 290
   <211> 12
   <212> DNA
   <213> Artificial Sequence
<220>
   <223> Synthetic Oligonucleotide
<400> 290
   acacagctgc cc 12
<210> 291
   <211> 12
   <212> DNA
   <213> Artificial Sequence
<220>
   <223> Synthetic Oligonucleotide
<400> 291
   cacacagctg cc 12
<210> 292
   <211> 13
   <212> DNA
   <213> Artificial Sequence
<220>
   <223> Synthetic Oligonucleotide
<400> 292
   gcacacagct gcc 13
<210> 293
   <211> 12
   <212> DNA
   <213> Artificial Sequence
<220>
   <223> Synthetic Oligonucleotide
<400> 293
   gcacacagct gc 12
<210> 294
   <211> 4
   <212> DNA
   <213> Artificial Sequence
<220>
   <223> Synthetic Oligonucleotide
<400> 294
   actg 4
<210> 295
   <211> 12
   <212> DNA
   <213> Artificial Sequence
<220>
   <223> Synthetic Oligonucleotide
<400> 295
   gccggagact ga 12
<210> 296
   <211> 12
   <212> DNA
   <213> Artificial Sequence
<220>
   <223> Synthetic Oligonucleotide
<400> 296
   attgaggttg ac 12
<210> 297
   <211> 12
   <212> DNA
   <213> Artificial Sequence
<220>
   <223> Synthetic Oligonucleotide
<400> 297
   cattgaggtt ga 12
<210> 298
   <211> 12
   <212> DNA
   <213> Artificial Sequence
<220>
   <223> Synthetic Oligonucleotide
<400> 298
   gcattgaggt tg 12
<210> 299
   <211> 12
   <212> DNA
   <213> Artificial Sequence
<220>
   <223> Synthetic Oligonucleotide
<400> 299
   ggcattgagg tt 12
<210> 300
   <211> 12
   <212> DNA
   <213> Artificial Sequence
<220>
   <223> Synthetic Oligonucleotide
<400> 300
   gggcattgag gt 12
<210> 301
   <211> 4
   <212> DNA
   <213> Artificial Sequence
<220>
   <223> Synthetic Oligonucleotide
<400> 301
   actg 4
<210> 302
   <211> 4
   <212> DNA
   <213> Artificial Sequence
<220>
   <223> Synthetic Oligonucleotide
<400> 302
   actg 4
<210> 303
   <211> 4
   <212> DNA
   <213> Artificial Sequence
<220>
   <223> Synthetic Oligonucleotide
<400> 303
   actg 4
<210> 304
   <211> 4
   <212> DNA
   <213> Artificial Sequence
<220>
   <223> Synthetic Oligonucleotide
<400> 304
   actg 4
<210> 305
   <211> 4
   <212> DNA
   <213> Artificial Sequence
<220>
   <223> Synthetic Oligonucleotide
<400> 305
   actg 4
<210> 306
   <211> 4
   <212> DNA
   <213> Artificial Sequence
<220>
   <223> Synthetic Oligonucleotide
<400> 306
   actg 4
<210> 307
   <211> 4
   <212> DNA
   <213> Artificial Sequence
<220>
   <223> Synthetic Oligonucleotide
<400> 307
   actg 4
<210> 308
   <211> 4
   <212> DNA
   <213> Artificial Sequence
<220>
   <223> Synthetic Oligonucleotide
<400> 308
   actg 4
<210> 309
   <211> 4
   <212> DNA
   <213> Artificial Sequence
<220>
   <223> Synthetic Oligonucleotide
<400> 309
   actg 4
<210> 310
   <211> 4
   <212> DNA
   <213> Artificial Sequence
<220>
   <223> Synthetic Oligonucleotide
<400> 310
   actg 4
<210> 311
   <211> 4
   <212> DNA
   <213> Artificial Sequence
<220>
   <223> Synthetic Oligonucleotide
<400> 311
   actg 4
<210> 312
   <211> 4
   <212> DNA
   <213> Artificial Sequence
<220>
   <223> Synthetic Oligonucleotide
<400> 312
   actg 4
<210> 313
   <211> 4
   <212> DNA
   <213> Artificial Sequence
<220>
   <223> Synthetic Oligonucleotide
<400> 313
   actg 4
<210> 314
   <211> 4
   <212> DNA
   <213> Artificial Sequence
<220>
   <223> Synthetic Oligonucleotide
<400> 314
   actg 4
<210> 315
   <211> 4
   <212> DNA
   <213> Artificial Sequence
<220>
   <223> Synthetic Oligonucleotide
<400> 315
   actg 4
<210> 316
   <211> 4
   <212> DNA
   <213> Artificial Sequence
<220>
   <223> Synthetic Oligonucleotide
<400> 316
   actg 4
<210> 317
   <211> 4
   <212> DNA
   <213> Artificial Sequence
<220>
   <223> Synthetic Oligonucleotide
<400> 317
   actg 4
<210> 318
   <211> 4
   <212> DNA
   <213> Artificial Sequence
<220>
   <223> Synthetic Oligonucleotide
<400> 318
   actg 4
<210> 319
   <211> 4
   <212> DNA
   <213> Artificial Sequence
<220>
   <223> Synthetic Oligonucleotide
<400> 319
   actg 4
<210> 320
   <211> 4
   <212> DNA
   <213> Artificial Sequence
<220>
   <223> Synthetic Oligonucleotide
<400> 320
   actg 4
<210> 321
   <211> 4
   <212> DNA
   <213> Artificial Sequence
<220>
   <223> Synthetic Oligonucleotide
<400> 321
   actg 4
<210> 322
   <211> 4
   <212> DNA
   <213> Artificial Sequence
<220>
   <223> Synthetic Oligonucleotide
<400> 322
   actg 4
<210> 323
   <211> 4
   <212> DNA
   <213> Artificial Sequence
<220>
   <223> Synthetic Oligonucleotide
<400> 323
   actg 4
<210> 324
   <211> 4
   <212> DNA
   <213> Artificial Sequence
<220>
   <223> Synthetic Oligonucleotide
<400> 324
   actg 4
<210> 325
   <211> 4
   <212> DNA
   <213> Artificial Sequence
<220>
   <223> Synthetic Oligonucleotide
<400> 325
   actg 4
<210> 326
   <211> 4
   <212> DNA
   <213> Artificial Sequence
<220>
   <223> Synthetic Oligonucleotide
<400> 326
   actg 4
<210> 327
   <211> 4
   <212> DNA
   <213> Artificial Sequence
<220>
   <223> Synthetic Oligonucleotide
<400> 327
   actg 4
<210> 328
   <211> 4
   <212> DNA
   <213> Artificial Sequence
<220>
   <223> Synthetic Oligonucleotide
<400> 328
   actg 4
<210> 329
   <211> 14
   <212> DNA
   <213> Artificial Sequence
<220>
   <223> Synthetic Oligonucleotide
<400> 329
   atggggcaac ttca 14
<210> 330
   <211> 14
   <212> DNA
   <213> Artificial Sequence
<220>
   <223> Synthetic Oligonucleotide
<400> 330
   catggggcaa cttc 14
<210> 331
   <211> 14
   <212> DNA
   <213> Artificial Sequence
<220>
   <223> Synthetic Oligonucleotide
<400> 331
   acatggggca actt 14
<210> 332
   <211> 14
   <212> DNA
   <213> Artificial Sequence
<220>
   <223> Synthetic Oligonucleotide
<400> 332
   gggatgctct gggc 14
<210> 333
   <211> 14
   <212> DNA
   <213> Artificial Sequence
<220>
   <223> Synthetic Oligonucleotide
<400> 333
   cgctccaggt tcca 14
<210> 334
   <211> 14
   <212> DNA
   <213> Artificial Sequence
<220>
   <223> Synthetic Oligonucleotide
<400> 334
   gatacacctc cacc 14
<210> 335
   <211> 14
   <212> DNA
   <213> Artificial Sequence
<220>
   <223> Synthetic Oligonucleotide
<400> 335
   agatacacct ccac 14
<210> 336
   <211> 14
   <212> DNA
   <213> Artificial Sequence
<220>
   <223> Synthetic Oligonucleotide
<400> 336
   gagatacacc tcca 14
<210> 337
   <211> 14
   <212> DNA
   <213> Artificial Sequence
<220>
   <223> Synthetic Oligonucleotide
<400> 337
   gcctgtctgt ggaa 14
<210> 338
   <211> 14
   <212> DNA
   <213> Artificial Sequence
<220>
   <223> Synthetic Oligonucleotide
<400> 338
   ctgtcacact tgct 14
<210> 339
   <211> 14
   <212> DNA
   <213> Artificial Sequence
<220>
   <223> Synthetic Oligonucleotide
<400> 339
   cggccgctga ccac 14
<210> 340
   <211> 14
   <212> DNA
   <213> Artificial Sequence
<220>
   <223> Synthetic Oligonucleotide
<400> 340
   ccggccgctg acca 14
<210> 341
   <211> 14
   <212> DNA
   <213> Artificial Sequence
<220>
   <223> Synthetic Oligonucleotide
<400> 341
   cccggccgct gacc 14
<210> 342
   <211> 14
   <212> DNA
   <213> Artificial Sequence
<220>
   <223> Synthetic Oligonucleotide
<400> 342
   tcccggccgc tgac 14
<210> 343
   <211> 14
   <212> DNA
   <213> Artificial Sequence
<220>
   <223> Synthetic Oligonucleotide
<400> 343
   atcccggccg ctga 14
<210> 344
   <211> 14
   <212> DNA
   <213> Artificial Sequence
<220>
   <223> Synthetic Oligonucleotide
<400> 344
   catcccggcc gctg 14
<210> 345
   <211> 14
   <212> DNA
   <213> Artificial Sequence
<220>
   <223> Synthetic Oligonucleotide
<400> 345
   gcatcccggc cgct 14
<210> 346
   <211> 14
   <212> DNA
   <213> Artificial Sequence
<220>
   <223> Synthetic Oligonucleotide
<400> 346
   gtgcccttcc cttg **14**
<210> 347
   <211> 14
   <212> DNA
   <213> Artificial Sequence
<220>
   <223> Synthetic Oligonucleotide
<400> 347
   atgagggtgc cgct 14
<210> 348
   <211> 14
   <212> DNA
   <213> Artificial Sequence
<220>
   <223> Synthetic Oligonucleotide
<400> 348
   tatgagggtg ccgc 14
<210> 349
   <211> 14
   <212> DNA
   <213> Artificial Sequence
<220>
   <223> Synthetic Oligonucleotide
<400> 349
   ctatgagggt gccg 14
<210> 350
   <211> 14
   <212> DNA
   <213> Artificial Sequence
<220>
   <223> Synthetic Oligonucleotide
<400> 350
   cctatgaggg tgcc 14
<210> 351
   <211> 14
   <212> DNA
   <213> Artificial Sequence
<220>
   <223> Synthetic Oligonucleotide
<400> 351
   cgaataaact ccag 14
<210> 352
   <211> 14
   <212> DNA
   <213> Artificial Sequence
<220>
   <223> Synthetic Oligonucleotide
<400> 352
   ccgaataaac tcca 14
<210> 353
   <211> 14
   <212> DNA
   <213> Artificial Sequence
<220>
   <223> Synthetic Oligonucleotide
<400> 353
   tccgaataaa ctcc 14
<210> 354
   <211> 14
   <212> DNA
   <213> Artificial Sequence
<220>
   <223> Synthetic Oligonucleotide
<400> 354
   ttccgaataa actc 14
<210> 355
   <211> 14
   <212> DNA
   <213> Artificial Sequence
<220>
   <223> Synthetic Oligonucleotide
<400> 355
   gccaggggca gcag 14
<210> 356
   <211> 14
   <212> DNA
   <213> Artificial Sequence
<220>
   <223> Synthetic Oligonucleotide
<400> 356
   gagtagaggc aggc 14
<210> 357
   <211> 14
   <212> DNA
   <213> Artificial Sequence
<220>
   <223> Synthetic Oligonucleotide
<400> 357
   ccccaaagtc ccca 14
<210> 358
   <211> 14
   <212> DNA
   <213> Artificial Sequence
<220>
   <223> Synthetic Oligonucleotide
<400> 358
   tccccaaagt cccc 14
<210> 359
   <211> 14
   <212> DNA
   <213> Artificial Sequence
<220>
   <223> Synthetic Oligonucleotide
<400> 359
   gtccccaaag tccc 14
<210> 360
   <211> 14
   <212> DNA
   <213> Artificial Sequence
<220>
   <223> Synthetic Oligonucleotide
<400> 360
   acgtgggcag cagc 14
<210> 361
   <211> 14
   <212> DNA
   <213> Artificial Sequence
<220>
   <223> Synthetic Oligonucleotide
<400> 361
   cacgtgggca gcag 14
<210> 362
   <211> 14
   <212> DNA
   <213> Artificial Sequence
<220>
   <223> Synthetic Oligonucleotide
<400> 362
   ccacgtgggc agca 14
<210> 363
   <211> 14
   <212> DNA
   <213> Artificial Sequence
<220>
   <223> Synthetic Oligonucleotide
<400> 363
   gccacgtggg cagc 14
<210> 364
   <211> 14
   <212> DNA
   <213> Artificial Sequence
<220>
   <223> Synthetic Oligonucleotide
<400> 364
   cagggaacca ggcc 14
<210> 365
   <211> 14
   <212> DNA
   <213> Artificial Sequence
<220>
   <223> Synthetic Oligonucleotide
<400> 365
   tcagggaacc aggc 14
<210> 366
   <211> 14
   <212> DNA
   <213> Artificial Sequence
<220>
   <223> Synthetic Oligonucleotide
<400> 366
   ctcagggaac cagg 14
<210> 367
   <211> 14
   <212> DNA
   <213> Artificial Sequence
<220>
   <223> Synthetic Oligonucleotide
<400> 367
   cctcagggaa ccag 14
<210> 368
   <211> 14
   <212> DNA
   <213> Artificial Sequence
<220>
   <223> Synthetic Oligonucleotide
<400> 368
   tcctcaggga acca 14
<210> 369
   <211> 14
   <212> DNA
   <213> Artificial Sequence
<220>
   <223> Synthetic Oligonucleotide
<400> 369
   gtcctcaggg aacc 14
<210> 370
   <211> 14
   <212> DNA
   <213> Artificial Sequence
<220>
   <223> Synthetic Oligonucleotide
<400> 370
   ggtcctcagg gaac 14
<210> 371
   <211> 14
   <212> DNA
   <213> Artificial Sequence
<220>
   <223> Synthetic Oligonucleotide
<400> 371
   tggtcctcag ggaa 14
<210> 372
   <211> 14
   <212> DNA
   <213> Artificial Sequence
<220>
   <223> Synthetic Oligonucleotide
<400> 372
   ctggtcctca ggga 14
<210> 373
   <211> 14
   <212> DNA
   <213> Artificial Sequence
<220>
   <223> Synthetic Oligonucleotide
<400> 373
   gctggtcctc aggg 14
<210> 374
   <211> 14
   <212> DNA
   <213> Artificial Sequence
<220>
   <223> Synthetic Oligonucleotide
<400> 374
   gtgctggggg gcag 14
<210> 375
   <211> 14
   <212> DNA
   <213> Artificial Sequence
<220>
   <223> Synthetic Oligonucleotide
<400> 375
   ggtgctgggg ggca 14
<210> 376
   <211> 14
   <212> DNA
   <213> Artificial Sequence
<220>
   <223> Synthetic Oligonucleotide
<400> 376
   gggtgctggg gggc 14
<210> 377
   <211> 14
   <212> DNA
   <213> Artificial Sequence
<220>
   <223> Synthetic Oligonucleotide
<400> 377
   tgggtgctgg gggg 14
<210> 378
   <211> 14
   <212> DNA
   <213> Artificial Sequence
<220>
   <223> Synthetic Oligonucleotide
<400> 378
   gagcagctca gcag 14
<210> 379
   <211> 14
   <212> DNA
   <213> Artificial Sequence
<220>
   <223> Synthetic Oligonucleotide
<400> 379
   ggagcagctc agca 14
<210> 380
   <211> 14
   <212> DNA
   <213> Artificial Sequence
<220>
   <223> Synthetic Oligonucleotide
<400> 380
   tggagcagct cagc 14
<210> 381
   <211> 14
   <212> DNA
   <213> Artificial Sequence
<220>
   <223> Synthetic Oligonucleotide
<400> 381
   ctggagcagc tcag 14
<210> 382
   <211> 14
   <212> DNA
   <213> Artificial Sequence
<220>
   <223> Synthetic Oligonucleotide
<400> 382
   ccctcacccc caaa 14
<210> 383
   <211> 14
   <212> DNA
   <213> Artificial Sequence
<220>
   <223> Synthetic Oligonucleotide
<400> 383
   accctcaccc ccaa 14
<210> 384
   <211> 14
   <212> DNA
   <213> Artificial Sequence
<220>
   <223> Synthetic Oligonucleotide
<400> 384
   caccctcacc ccca 14
<210> 385
   <211> 14
   <212> DNA
   <213> Artificial Sequence
<220>
   <223> Synthetic Oligonucleotide
<400> 385
   acaccctcac cccc 14
<210> 386
   <211> 14
   <212> DNA
   <213> Artificial Sequence
<220>
   <223> Synthetic Oligonucleotide
<400> 386
   gacaccctca cccc 14
<210> 387
   <211> 14
   <212> DNA
   <213> Artificial Sequence
<220>
   <223> Synthetic Oligonucleotide
<400> 387
   agacaccctc accc 14
<210> 388
   <211> 14
   <212> DNA
   <213> Artificial Sequence
<220>
   <223> Synthetic Oligonucleotide
<400> 388
   tagacaccct cacc 14
<210> 389
   <211> 14
   <212> DNA
   <213> Artificial Sequence
<220>
   <223> Synthetic Oligonucleotide
<400> 389
   tggcagcagg aagc 14
<210> 390
   <211> 14
   <212> DNA
   <213> Artificial Sequence
<220>
   <223> Synthetic Oligonucleotide
<400> 390
   atggcagcag gaag 14
<210> 391
   <211> 14
   <212> DNA
   <213> Artificial Sequence
<220>
   <223> Synthetic Oligonucleotide
<400> 391
   catggcagca ggaa 14
<210> 392
   <211> 14
   <212> DNA
   <213> Artificial Sequence
<220>
   <223> Synthetic Oligonucleotide
<400> 392
   gcatggcagc agga 14
<210> 393
   <211> 14
   <212> DNA
   <213> Artificial Sequence
<220>
   <223> Synthetic Oligonucleotide
<400> 393
   ggcagcagat ggca 14
<210> 394
   <211> 14
   <212> DNA
   <213> Artificial Sequence
<220>
   <223> Synthetic Oligonucleotide
<400> 394
   cggcagcaga tggc 14
<210> 395
   <211> 14
   <212> DNA
   <213> Artificial Sequence
<220>
   <223> Synthetic Oligonucleotide
<400> 395
   ccggcagcag atgg 14
<210> 396
   <211> 14
   <212> DNA
   <213> Artificial Sequence
<220>
   <223> Synthetic Oligonucleotide
<400> 396
   tccggcagca gatg 14
<210> 397
   <211> 14
   <212> DNA
   <213> Artificial Sequence
<220>
   <223> Synthetic Oligonucleotide
<400> 397
   ctccggcagc agat 14
<210> 398
   <211> 14
   <212> DNA
   <213> Artificial Sequence
<220>
   <223> Synthetic Oligonucleotide
<400> 398
   gctccggcag caga 14
<210> 399
   <211> 14
   <212> DNA
   <213> Artificial Sequence
<220>
   <223> Synthetic Oligonucleotide
<400> 399
   ggctccggca gcag 14
<210> 400
   <211> 14
   <212> DNA
   <213> Artificial Sequence
<220>
   <223> Synthetic Oligonucleotide
<400> 400
   cggctccggc agca 14
<210> 401
   <211> 14
   <212> DNA
   <213> Artificial Sequence
<220>
   <223> Synthetic Oligonucleotide
<400> 401
   ccggctccgg cagc 14
<210> 402
   <211> 14
   <212> DNA
   <213> Artificial Sequence
<220>
   <223> Synthetic Oligonucleotide
<400> 402
   gccggctccg gcag 14
<210> 403
   <211> 14
   <212> DNA
   <213> Artificial Sequence
<220>
   <223> Synthetic Oligonucleotide
<400> 403
   agttacaaaa gcaa 14
<210> 404
   <211> 4
   <212> DNA
   <213> Artificial Sequence
<220>
   <223> Synthetic Oligonucleotide
<400> 404
   actg 4
<210> 405
   <211> 4
   <212> DNA
   <213> Artificial Sequence
<220>
   <223> Synthetic Oligonucleotide
<400> 405
   actg 4
<210> 406
   <211> 16
   <212> DNA
   <213> Artificial Sequence
<220>
   <223> Synthetic Oligonucleotide
<400> 406
   gtcgcccttc agcacg 16
<210> 407
   <211> 14
   <212> DNA
   <213> Artificial Sequence
<220>
   <223> Synthetic Oligonucleotide
<400> 407
   tcgcccttca gcac 14
<210> 408
   <211> 12
   <212> DNA
   <213> Artificial Sequence
<220>
   <223> Synthetic Oligonucleotide
<400> 408
   cgcccttcag ca 12
<210> 409
   <211> 16
   <212> DNA
   <213> Artificial Sequence
<220>
   <223> Synthetic Oligonucleotide
<400> 409
   actctggacc caaacc 16
<210> 410
   <211> 14
   <212> DNA
   <213> Artificial Sequence
<220>
   <223> Synthetic Oligonucleotide
<400> 410
   ctctggaccc aaac 14
<210> 411
   <211> 16
   <212> DNA
   <213> Artificial Sequence
<220>
   <223> Synthetic Oligonucleotide
<400> 411
   ccatttcagg agacct 16
<210> 412
   <211> 14
   <212> DNA
   <213> Artificial Sequence
<220>
   <223> Synthetic Oligonucleotide
<400> 412
   catttcagga gacc 14
<210> 413
   <211> 14
   <212> DNA
   <213> Artificial Sequence
<220>
   <223> Synthetic Oligonucleotide
<400> 413
   tttgggaggt ggtc 14
<210> 414
   <211> 14
   <212> DNA
   <213> Artificial Sequence
<220>
   <223> Synthetic Oligonucleotide
<400> 414
   cacaccaggc agag 14
<210> 415
   <211> 14
   <212> DNA
   <213> Artificial Sequence
<220>
   <223> Synthetic Oligonucleotide
<400> 415
   ctttacagct tcca 14
<210> 416
   <211> 14
   <212> DNA
   <213> Artificial Sequence
<220>
   <223> Synthetic Oligonucleotide
<400> 416
   cactaccttc cact 14
<210> 417
   <211> 14
   <212> DNA
   <213> Artificial Sequence
<220>
   <223> Synthetic Oligonucleotide
<400> 417
   aacacacact acct 14
<210> 418
   <211> 14
   <212> DNA
   <213> Artificial Sequence
<220>
   <223> Synthetic Oligonucleotide
<400> 418
   ctcttcaaaa caca 14
<210> 419
   <211> 14
   <212> DNA
   <213> Artificial Sequence
<220>
   <223> Synthetic Oligonucleotide
<400> 419
   gtaattgtgc tgtc 14
<210> 420
   <211> 14
   <212> DNA
   <213> Artificial Sequence
<220>
   <223> Synthetic Oligonucleotide
<400> 420
   tttttcttcg aatt 14
<210> 421
   <211> 14
   <212> DNA
   <213> Artificial Sequence
<220>
   <223> Synthetic Oligonucleotide
<400> 421
   cattttcgat agcg 14
<210> 422
   <211> 14
   <212> DNA
   <213> Artificial Sequence
<220>
   <223> Synthetic Oligonucleotide
<400> 422
   accttccagg ttca 14
<210> 423
   <211> 14
   <212> DNA
   <213> Artificial Sequence
<220>
   <223> Synthetic Oligonucleotide
<400> 423
   ataggaagca taaa 14
<210> 424
   <211> 14
   <212> DNA
   <213> Artificial Sequence
<220>
   <223> Synthetic Oligonucleotide
<400> 424
   tcttttaaag aaga 14
<210> 425
   <211> 14
   <212> DNA
   <213> Artificial Sequence
<220>
   <223> Synthetic Oligonucleotide
<400> 425
   aaggatattt taaa 14
<210> 426
   <211> 4
   <212> DNA
   <213> Artificial Sequence
<220>
   <223> Synthetic Oligonucleotide
<400> 426
   actg 4
<210> 427
   <211> 14
   <212> DNA
   <213> Artificial Sequence
<220>
   <223> Synthetic Oligonucleotide
<400> 427
   gaacaaaaat taaa 14
<210> 428
   <211> 14
   <212> DNA
   <213> Artificial Sequence
<220>
   <223> Synthetic Oligonucleotide
<400> 428
   ttccacagat ctgt 14
<210> 429
   <211> 14
   <212> DNA
   <213> Artificial Sequence
<220>
   <223> Synthetic Oligonucleotide
<400> 429
   tttataaagt aaag 14
<210> 430
   <211> 4
   <212> DNA
   <213> Artificial Sequence
<220>
   <223> Synthetic Oligonucleotide
<400> 430
   actg 4
<210> 431
   <211> 4
   <212> DNA
   <213> Artificial Sequence
<220>
   <223> Synthetic Oligonucleotide
<400> 431
   actg 4
<210> 432
   <211> 4
   <212> DNA
   <213> Artificial Sequence
<220>
   <223> Synthetic Oligonucleotide
<400> 432
   actg 4
<210> 433
   <211> 14
   <212> DNA
   <213> Artificial Sequence
<220>
   <223> Synthetic Oligonucleotide
<400> 433
   tactgtgaga aata 14
<210> 434
   <211> 4
   <212> DNA
   <213> Artificial Sequence
<220>
   <223> Synthetic Oligonucleotide
<400> 434
   actg 4
<210> 435
   <211> 14
   <212> DNA
   <213> Artificial Sequence
<220>
   <223> Synthetic Oligonucleotide
<400> 435
   ttccagcttg aaga 14
<210> 436
   <211> 14
   <212> DNA
   <213> Artificial Sequence
<220>
   <223> Synthetic Oligonucleotide
<400> 436
   gatcagttct catg 14
<210> 437
   <211> 4
   <212> DNA
   <213> Artificial Sequence
<220>
   <223> Synthetic Oligonucleotide
<400> 437
   actg 4
<210> 438
   <211> 4
   <212> DNA
   <213> Artificial Sequence
<220>
   <223> Synthetic Oligonucleotide
<400> 438
   actg 4
<210> 439
   <211> 14
   <212> DNA
   <213> Artificial Sequence
<220>
   <223> Synthetic Oligonucleotide
<400> 439
   ttatcaatga tgca 14
<210> 440
   <211> 14
   <212> DNA
   <213> Artificial Sequence
<220>
   <223> Synthetic Oligonucleotide
<400> 440
   gcatgctgga cagt 14
<210> 441
   <211> 4
   <212> DNA
   <213> Artificial Sequence
<220>
   <223> Synthetic Oligonucleotide
<400> 441
   actg 4
<210> 442
   <211> 4
   <212> DNA
   <213> Artificial Sequence
<220>
   <223> Synthetic Oligonucleotide
<400> 442
   actg 4
<210> 443
   <211> 14
   <212> DNA
   <213> Artificial Sequence
<220>
   <223> Synthetic Oligonucleotide
<400> 443
   ttgcacctga acta 14
<210> 444
   <211> 14
   <212> DNA
   <213> Artificial Sequence
<220>
   <223> Synthetic Oligonucleotide
<400> 444
   cagaatatat ttct 14
<210> 445
   <211> 4
   <212> DNA
   <213> Artificial Sequence
<220>
   <223> Synthetic Oligonucleotide
<400> 445
   actg 4
<210> 446
   <211> 4
   <212> DNA
   <213> Artificial Sequence
<220>
   <223> Synthetic Oligonucleotide
<400> 446
   actg 4
<210> 447
   <211> 4
   <212> DNA
   <213> Artificial Sequence
<220>
   <223> Synthetic Oligonucleotide
<400> 447
   actg 4
<210> 448
   <211> 4
   <212> DNA
   <213> Artificial Sequence
<220>
   <223> Synthetic Oligonucleotide
<400> 448
   actg 4
<210> 449
   <211> 4
   <212> DNA
   <213> Artificial Sequence
<220>
   <223> Synthetic Oligonucleotide
<400> 449
   actg 4
<210> 450
   <211> 14
   <212> DNA
   <213> Artificial Sequence
<220>
   <223> Synthetic Oligonucleotide
<400> 450
   ataagagatt aaaa 14
<210> 451
   <211> 14
   <212> DNA
   <213> Artificial Sequence
<220>
   <223> Synthetic Oligonucleotide
<400> 451
   tcccccttct catt 14
<210> 452
   <211> 14
   <212> DNA
   <213> Artificial Sequence
<220>
   <223> Synthetic Oligonucleotide
<400> 452
   gggcattgtt aaaa 14
<210> 453
   <211> 4
   <212> DNA
   <213> Artificial Sequence
<220>
   <223> Synthetic Oligonucleotide
<400> 453
   actg 4
<210> 454
   <211> 4
   <212> DNA
   <213> Artificial Sequence
<220>
   <223> Synthetic Oligonucleotide
<400> 454
   actg 4
<210> 455
   <211> 14
   <212> DNA
   <213> Artificial Sequence
<220>
   <223> Synthetic Oligonucleotide
<400> 455
   agaactcaca tctg 14
<210> 456
   <211> 14
   <212> DNA
   <213> Artificial Sequence
<220>
   <223> Synthetic Oligonucleotide
<400> 456
   gagctggacg gagg 14
<210> 457
   <211> 14
   <212> DNA
   <213> Artificial Sequence
<220>
   <223> Synthetic Oligonucleotide
<400> 457
   aagcttcatc ggag 14
<210> 458
   <211> 14
   <212> DNA
   <213> Artificial Sequence
<220>
   <223> Synthetic Oligonucleotide
<400> 458
   ataatggcat cccg 14
<210> 459
   <211> 4
   <212> DNA
   <213> Artificial Sequence
<220>
   <223> Synthetic Oligonucleotide
<400> 459
   actg 4
<210> 460
   <211> 14
   <212> DNA
   <213> Artificial Sequence
<220>
   <223> Synthetic Oligonucleotide
<400> 460
   tgctgtccta taag 14
<210> 461
   <211> 14
   <212> DNA
   <213> Artificial Sequence
<220>
   <223> Synthetic Oligonucleotide
<400> 461
   cacaaaggta attg 14
<210> 462
   <211> 14
   <212> DNA
   <213> Artificial Sequence
<220>
   <223> Synthetic Oligonucleotide
<400> 462
   atcatttctt ccag 14
<210> 463
   <211> 4
   <212> DNA
   <213> Artificial Sequence
<220>
   <223> Synthetic Oligonucleotide
<400> 463
   actg 4
<210> 464
   <211> 4
   <212> DNA
   <213> Artificial Sequence
<220>
   <223> Synthetic Oligonucleotide
<400> 464
   actg 4
<210> 465
   <211> 4
   <212> DNA
   <213> Artificial Sequence
<220>
   <223> Synthetic Oligonucleotide
<400> 465
   actg 4
<210> 466
   <211> 14
   <212> DNA
   <213> Artificial Sequence
<220>
   <223> Synthetic Oligonucleotide
<400> 466
   ccattacctt ccag 14
<210> 467
   <211> 14
   <212> DNA
   <213> Artificial Sequence
<220>
   <223> Synthetic Oligonucleotide
<400> 467
   gcataaacag ggtt 14
<210> 468
   <211> 4
   <212> DNA
   <213> Artificial Sequence
<220>
   <223> Synthetic Oligonucleotide
<400> 468
   actg 4
<210> 469
   <211> 4
   <212> DNA
   <213> Artificial Sequence
<220>
   <223> Synthetic Oligonucleotide
<400> 469
   actg 4
<210> 470
   <211> 4
   <212> DNA
   <213> Artificial Sequence
<220>
   <223> Synthetic Oligonucleotide
<400> 470
   actg 4
<210> 471
   <211> 4
   <212> DNA
   <213> Artificial Sequence
<220>
   <223> Synthetic Oligonucleotide
<400> 471
   actg 4
<210> 472
   <211> 14
   <212> DNA
   <213> Artificial Sequence
<220>
   <223> Synthetic Oligonucleotide
<400> 472
   tatgaaagga atgt 14
<210> 473
   <211> 4
   <212> DNA
   <213> Artificial Sequence
<220>
   <223> Synthetic Oligonucleotide
<400> 473
   actg 4
<210> 474
   <211> 14
   <212> DNA
   <213> Artificial Sequence
<220>
   <223> Synthetic Oligonucleotide
<400> 474
   ttccttaagc ttcc 14
<210> 475
   <211> 4
   <212> DNA
   <213> Artificial Sequence
<220>
   <223> Synthetic Oligonucleotide
<400> 475
   actg 4
<210> 476
   <211> 4
   <212> DNA
   <213> Artificial Sequence
<220>
   <223> Synthetic Oligonucleotide
<400> 476
   actg 4
<210> 477
   <211> 4
   <212> DNA
   <213> Artificial Sequence
<220>
   <223> Synthetic Oligonucleotide
<400> 477
   actg 4
<210> 478
   <211> 4
   <212> DNA
   <213> Artificial Sequence
<220>
   <223> Synthetic Oligonucleotide
<400> 478
   actg 4
<210> 479
   <211> 4
   <212> DNA
   <213> Artificial Sequence
<220>
   <223> Synthetic Oligonucleotide
<400> 479
   actg 4
<210> 480
   <211> 4
   <212> DNA
   <213> Artificial Sequence
<220>
   <223> Synthetic Oligonucleotide
<400> 480
   actg 4
<210> 481
   <211> 4
   <212> DNA
   <213> Artificial Sequence
<220>
   <223> Synthetic Oligonucleotide
<400> 481
   actg 4
<210> 482
   <211> 4
   <212> DNA
   <213> Artificial Sequence
<220>
   <223> Synthetic Oligonucleotide
<400> 482
   actg 4
<210> 483
   <211> 4
   <212> DNA
   <213> Artificial Sequence
<220>
   <223> Synthetic Oligonucleotide
<400> 483
   actg 4
<210> 484
   <211> 4
   <212> DNA
   <213> Artificial Sequence
<220>
   <223> Synthetic Oligonucleotide
<400> 484
   actg 4
<210> 485
   <211> 14
   <212> DNA
   <213> Artificial Sequence
<220>
   <223> Synthetic Oligonucleotide
<400> 485
   gaacagttaa acat 14
<210> 486
   <211> 16
   <212> DNA
   <213> Artificial Sequence
<220>
   <223> Synthetic Oligonucleotide
<400> 486
   tagagcttcc acttct 16
<210> 487
   <211> 14
   <212> DNA
   <213> Artificial Sequence
<220>
   <223> Synthetic Oligonucleotide
<400> 487
   gagcttccac ttct 14
<210> 488
   <211> 14
   <212> DNA
   <213> Artificial Sequence
<220>
   <223> Synthetic Oligonucleotide
<400> 488
   tgttggccgt ggta 14
<210> 489
   <211> 14
   <212> DNA
   <213> Artificial Sequence
<220>
   <223> Synthetic Oligonucleotide
<400> 489
   atgttggccg tggt 14
<210> 490
   <211> 14
   <212> DNA
   <213> Artificial Sequence
<220>
   <223> Synthetic Oligonucleotide
<400> 490
   gatgttggcc gtgg 14
<210> 491
   <211> 14
   <212> DNA
   <213> Artificial Sequence
<220>
   <223> Synthetic Oligonucleotide
<400> 491
   agatgttggc cgtg 14
<210> 492
   <211> 14
   <212> DNA
   <213> Artificial Sequence
<220>
   <223> Synthetic Oligonucleotide
<400> 492
   gagatgttgg ccgt 14
<210> 493
   <211> 14
   <212> DNA
   <213> Artificial Sequence
<220>
   <223> Synthetic Oligonucleotide
<400> 493
   ggagatgttg gccg 14
<210> 494
   <211> 14
   <212> DNA
   <213> Artificial Sequence
<220>
   <223> Synthetic Oligonucleotide
<400> 494
   aggagatgtt ggcc 14
<210> 495
   <211> 14
   <212> DNA
   <213> Artificial Sequence
<220>
   <223> Synthetic Oligonucleotide
<400> 495
   caggagatgt tggc 14
<210> 496
   <211> 14
   <212> DNA
   <213> Artificial Sequence
<220>
   <223> Synthetic Oligonucleotide
<400> 496
   gcaggagatg ttgg 14
<210> 497
   <211> 14
   <212> DNA
   <213> Artificial Sequence
<220>
   <223> Synthetic Oligonucleotide
<400> 497
   ggcaggagat gttg 14
<210> 498
   <211> 14
   <212> DNA
   <213> Artificial Sequence
<220>
   <223> Synthetic Oligonucleotide
<400> 498
   gtggtgccaa ggca 14
<210> 499
   <211> 14
   <212> DNA
   <213> Artificial Sequence
<220>
   <223> Synthetic Oligonucleotide
<400> 499
   tgtggtgcca aggc 14
<210> 500
   <211> 14
   <212> DNA
   <213> Artificial Sequence
<220>
   <223> Synthetic Oligonucleotide
<400> 500
   ttgtggtgcc aagg 14
<210> 501
   <211> 14
   <212> DNA
   <213> Artificial Sequence
<220>
   <223> Synthetic Oligonucleotide
<400> 501
   tttgtggtgc caag 14
<210> 502
   <211> 14
   <212> DNA
   <213> Artificial Sequence
<220>
   <223> Synthetic Oligonucleotide
<400> 502
   ctttgtggtg ccaa 14
<210> 503
   <211> 14
   <212> DNA
   <213> Artificial Sequence
<220>
   <223> Synthetic Oligonucleotide
<400> 503
   actttgtggt gcca 14
<210> 504
   <211> 14
   <212> DNA
   <213> Artificial Sequence
<220>
   <223> Synthetic Oligonucleotide
<400> 504
   cactttgtgg tgcc 14
<210> 505
   <211> 14
   <212> DNA
   <213> Artificial Sequence
<220>
   <223> Synthetic Oligonucleotide
<400> 505
   gcactttgtg gtgc 14
<210> 506
   <211> 14
   <212> DNA
   <213> Artificial Sequence
<220>
   <223> Synthetic Oligonucleotide
<400> 506
   tgcactttgt ggtg 14
<210> 507
   <211> 14
   <212> DNA
   <213> Artificial Sequence
<220>
   <223> Synthetic Oligonucleotide
<400> 507
   ttgcactttg tggt 14
<210> 508
   <211> 14
   <212> DNA
   <213> Artificial Sequence
<220>
   <223> Synthetic Oligonucleotide
<400> 508
   cggtgttgca cttt 14
<210> 509
   <211> 14
   <212> DNA
   <213> Artificial Sequence
<220>
   <223> Synthetic Oligonucleotide
<400> 509
   gcggtgttgc actt 14
<210> 510
   <211> 14
   <212> DNA
   <213> Artificial Sequence
<220>
   <223> Synthetic Oligonucleotide
<400> 510
   agcggtgttg cact 14
<210> 511
   <211> 14
   <212> DNA
   <213> Artificial Sequence
<220>
   <223> Synthetic Oligonucleotide
<400> 511
   aagcggtgtt gcac 14
<210> 512
   <211> 14
   <212> DNA
   <213> Artificial Sequence
<220>
   <223> Synthetic Oligonucleotide
<400> 512
   gaagcggtgt tgca 14
<210> 513
   <211> 14
   <212> DNA
   <213> Artificial Sequence
<220>
   <223> Synthetic Oligonucleotide
<400> 513
   cgaagcggtg ttgc 14
<210> 514
   <211> 14
   <212> DNA
   <213> Artificial Sequence
<220>
   <223> Synthetic Oligonucleotide
<400> 514
   acgaagcggt gttg 14
<210> 515
   <211> 14
   <212> DNA
   <213> Artificial Sequence
<220>
   <223> Synthetic Oligonucleotide
<400> 515
   cacgaagcgg tgtt 14
<210> 516
   <211> 14
   <212> DNA
   <213> Artificial Sequence
<220>
   <223> Synthetic Oligonucleotide
<400> 516
   acacgaagcg gtgt 14
<210> 517
   <211> 14
   <212> DNA
   <213> Artificial Sequence
<220>
   <223> Synthetic Oligonucleotide
<400> 517
   aacacgaagc ggtg 14
<210> 518
   <211> 14
   <212> DNA
   <213> Artificial Sequence
<220>
   <223> Synthetic Oligonucleotide
<400> 518
   gctgctgtac atct 14
<210> 519
   <211> 4
   <212> DNA
   <213> Artificial Sequence
<220>
   <223> Synthetic Oligonucleotide
<400> 519
   actg 4
<210> 520
   <211> 14
   <212> DNA
   <213> Artificial Sequence
<220>
   <223> Synthetic Oligonucleotide
<400> 520
   agctgctgta catc 14
<210> 521
   <211> 14
   <212> DNA
   <213> Artificial Sequence
<220>
   <223> Synthetic Oligonucleotide
<400> 521
   aagctgctgt acat 14
<210> 522
   <211> 14
   <212> DNA
   <213> Artificial Sequence
<220>
   <223> Synthetic Oligonucleotide
<400> 522
   gaagctgctg taca 14
<210> 523
   <211> 14
   <212> DNA
   <213> Artificial Sequence
<220>
   <223> Synthetic Oligonucleotide
<400> 523
   ggaagctgct gtac 14
<210> 524
   <211> 14
   <212> DNA
   <213> Artificial Sequence
<220>
   <223> Synthetic Oligonucleotide
<400> 524
   tggaagctgc tgta 14
<210> 525
   <211> 14
   <212> DNA
   <213> Artificial Sequence
<220>
   <223> Synthetic Oligonucleotide
<400> 525
   ctggaagctg ctgt 14
<210> 526
   <211> 14
   <212> DNA
   <213> Artificial Sequence
<220>
   <223> Synthetic Oligonucleotide
<400> 526
   cctggaagct gctg 14
<210> 527
   <211> 14
   <212> DNA
   <213> Artificial Sequence
<220>
   <223> Synthetic Oligonucleotide
<400> 527
   acctggaagc tgct 14
<210> 528
   <211> 14
   <212> DNA
   <213> Artificial Sequence
<220>
   <223> Synthetic Oligonucleotide
<400> 528
   cacctggaag ctgc 14
<210> 529
   <211> 14
   <212> DNA
   <213> Artificial Sequence
<220>
   <223> Synthetic Oligonucleotide
<400> 529
   tcacctggaa gctg 14
<210> 530
   <211> 14
   <212> DNA
   <213> Artificial Sequence
<220>
   <223> Synthetic Oligonucleotide
<400> 530
   atcacctgga agct 14
<210> 531
   <211> 14
   <212> DNA
   <213> Artificial Sequence
<220>
   <223> Synthetic Oligonucleotide
<400> 531
   catcacctgg aagc 14
<210> 532
   <211> 14
   <212> DNA
   <213> Artificial Sequence
<220>
   <223> Synthetic Oligonucleotide
<400> 532
   acatcacctg gaag 14
<210> 533
   <211> 14
   <212> DNA
   <213> Artificial Sequence
<220>
   <223> Synthetic Oligonucleotide
<400> 533
   tacatcacct ggaa 14
<210> 534
   <211> 14
   <212> DNA
   <213> Artificial Sequence
<220>
   <223> Synthetic Oligonucleotide
<400> 534
   gtacatcacc tgga 14
<210> 535
   <211> 14
   <212> DNA
   <213> Artificial Sequence
<220>
   <223> Synthetic Oligonucleotide
<400> 535
   tgtacatcac ctgg 14
<210> 536
   <211> 14
   <212> DNA
   <213> Artificial Sequence
<220>
   <223> Synthetic Oligonucleotide
<400> 536
   gtgtacatca cctg 14
<210> 537
   <211> 14
   <212> DNA
   <213> Artificial Sequence
<220>
   <223> Synthetic Oligonucleotide
<400> 537
   tagcgggtcc tgag 14
<210> 538
   <211> 14
   <212> DNA
   <213> Artificial Sequence
<220>
   <223> Synthetic Oligonucleotide
<400> 538
   gtagcgggtc ctga 14
<210> 539
   <211> 14
   <212> DNA
   <213> Artificial Sequence
<220>
   <223> Synthetic Oligonucleotide
<400> 539
   tgtagcgggt cctg 14
<210> 540
   <211> 14
   <212> DNA
   <213> Artificial Sequence
<220>
   <223> Synthetic Oligonucleotide
<400> 540
   ctgtagcggg tcct 14
<210> 541
   <211> 14
   <212> DNA
   <213> Artificial Sequence
<220>
   <223> Synthetic Oligonucleotide
<400> 541
   gctgtagcgg gtcc 14
<210> 542
   <211> 14
   <212> DNA
   <213> Artificial Sequence
<220>
   <223> Synthetic Oligonucleotide
<400> 542
   ggctgtagcg ggtc 14
<210> 543
   <211> 14
   <212> DNA
   <213> Artificial Sequence
<220>
   <223> Synthetic Oligonucleotide
<400> 543
   tggctgtagc gggt 14
<210> 544
   <211> 14
   <212> DNA
   <213> Artificial Sequence
<220>
   <223> Synthetic Oligonucleotide
<400> 544
   ctggctgtag cggg 14
<210> 545
   <211> 14
   <212> DNA
   <213> Artificial Sequence
<220>
   <223> Synthetic Oligonucleotide
<400> 545
   tctggctgta gcgg 14
<210> 546
   <211> 14
   <212> DNA
   <213> Artificial Sequence
<220>
   <223> Synthetic Oligonucleotide
<400> 546
   ttctggctgt agcg 14
<210> 547
   <211> 14
   <212> DNA
   <213> Artificial Sequence
<220>
   <223> Synthetic Oligonucleotide
<400> 547
   tgaacaccgc ggcc 14
<210> 548
   <211> 14
   <212> DNA
   <213> Artificial Sequence
<220>
   <223> Synthetic Oligonucleotide
<400> 548
   atgaacaccg cggc 14
<210> 549
   <211> 14
   <212> DNA
   <213> Artificial Sequence
<220>
   <223> Synthetic Oligonucleotide
<400> 549
   catgaacacc gcgg 14
<210> 550
   <211> 14
   <212> DNA
   <213> Artificial Sequence
<220>
   <223> Synthetic Oligonucleotide
<400> 550
   gcatgaacac cgcg 14
<210> 551
   <211> 14
   <212> DNA
   <213> Artificial Sequence
<220>
   <223> Synthetic Oligonucleotide
<400> 551
   tgcatgaaca ccgc 14
<210> 552
   <211> 14
   <212> DNA
   <213> Artificial Sequence
<220>
   <223> Synthetic Oligonucleotide
<400> 552
   ttgcatgaac accg 14
<210> 553
   <211> 14
   <212> DNA
   <213> Artificial Sequence
<220>
   <223> Synthetic Oligonucleotide
<400> 553
   attgcatgaa cacc 14
<210> 554
   <211> 14
   <212> DNA
   <213> Artificial Sequence
<220>
   <223> Synthetic Oligonucleotide
<400> 554
   tattgcatga acac 14
<210> 555
   <211> 14
   <212> DNA
   <213> Artificial Sequence
<220>
   <223> Synthetic Oligonucleotide
<400> 555
   atattgcatg aaca 14
<210> 556
   <211> 14
   <212> DNA
   <213> Artificial Sequence
<220>
   <223> Synthetic Oligonucleotide
<400> 556
   catattgcat gaac 14
<210> 557
   <211> 14
   <212> DNA
   <213> Artificial Sequence
<220>
   <223> Synthetic Oligonucleotide
<400> 557
   aggttgtgca ggta 14
<210> 558
   <211> 14
   <212> DNA
   <213> Artificial Sequence
<220>
   <223> Synthetic Oligonucleotide
<400> 558
   caggttgtgc aggt 14
<210> 559
   <211> 14
   <212> DNA
   <213> Artificial Sequence
<220>
   <223> Synthetic Oligonucleotide
<400> 559
   gcaggttgtg cagg 14
<210> 560
   <211> 14
   <212> DNA
   <213> Artificial Sequence
<220>
   <223> Synthetic Oligonucleotide
<400> 560
   agcaggttgt gcag 14
<210> 561
   <211> 14
   <212> DNA
   <213> Artificial Sequence
<220>
   <223> Synthetic Oligonucleotide
<400> 561
   cagcaggttg tgca 14
<210> 562
   <211> 14
   <212> DNA
   <213> Artificial Sequence
<220>
   <223> Synthetic Oligonucleotide
<400> 562
   ccagcaggtt gtgc 14
<210> 563
   <211> 14
   <212> DNA
   <213> Artificial Sequence
<220>
   <223> Synthetic Oligonucleotide
<400> 563
   cccagcaggt tgtg 14
<210> 564
   <211> 14
   <212> DNA
   <213> Artificial Sequence
<220>
   <223> Synthetic Oligonucleotide
<400> 564
   gcccagcagg ttgt 14
<210> 565
   <211> 14
   <212> DNA
   <213> Artificial Sequence
<220>
   <223> Synthetic Oligonucleotide
<400> 565
   ggcccagcag gttg 14
<210> 566
   <211> 14
   <212> DNA
   <213> Artificial Sequence
<220>
   <223> Synthetic Oligonucleotide
<400> 566
   aggcccagca ggtt 14
<210> 567
   <211> 16
   <212> DNA
   <213> Artificial Sequence
<220>
   <223> Synthetic Oligonucleotide
<400> 567
   tgtcattgct ggtcca 16
<210> 568
   <211> 14
   <212> DNA
   <213> Artificial Sequence
<220>
   <223> Synthetic Oligonucleotide
<400> 568
   tcattgctgg tcca 14
<210> 569
   <211> 16
   <212> DNA
   <213> Artificial Sequence
<220>
   <223> Synthetic Oligonucleotide
<400> 569
   tggccagccg gaactt 16
<210> 570
   <211> 14
   <212> DNA
   <213> Artificial Sequence
<220>
   <223> Synthetic Oligonucleotide
<400> 570
   gccagccgga actt 14
<210> 571
   <211> 16
   <212> DNA
   <213> Artificial Sequence
<220>
   <223> Synthetic Oligonucleotide
<400> 571
   gggatgaggg tcagcg 16
<210> 572
   <211> 14
   <212> DNA
   <213> Artificial Sequence
<220>
   <223> Synthetic Oligonucleotide
<400> 572
   gatgagggtc agcg 14
<210> 573
   <211> 14
   <212> DNA
   <213> Artificial Sequence
<220>
   <223> Synthetic Oligonucleotide
<400> 573
   aaggcaaaga ccac 14
<210> 574
   <211> 14
   <212> DNA
   <213> Artificial Sequence
<220>
   <223> Synthetic Oligonucleotide
<400> 574
   ggagcgcagg gtgc 14
<210> 575
   <211> 14
   <212> DNA
   <213> Artificial Sequence
<220>
   <223> Synthetic Oligonucleotide
<400> 575
   tgcacctcct tgtt 14
<210> 576
   <211> 14
   <212> DNA
   <213> Artificial Sequence
<220>
   <223> Synthetic Oligonucleotide
<400> 576
   cagcagaccc tgga 14
<210> 577
   <211> 16
   <212> DNA
   <213> Artificial Sequence
<220>
   <223> Synthetic Oligonucleotide
<400> 577
   acatctggca gaggtt 16
<210> 578
   <211> 14
   <212> DNA
   <213> Artificial Sequence
<220>
   <223> Synthetic Oligonucleotide
<400> 578
   atctggcaga ggtt 14
<210> 579
   <211> 16
   <212> DNA
   <213> Artificial Sequence
<220>
   <223> Synthetic Oligonucleotide
<400> 579
   caggccagca ggagta 16
<210> 580
   <211> 14
   <212> DNA
   <213> Artificial Sequence
<220>
   <223> Synthetic Oligonucleotide
<400> 580
   ggccagcagg agta 14
<210> 581
   <211> 16
   <212> DNA
   <213> Artificial Sequence
<220>
   <223> Synthetic Oligonucleotide
<400> 581
   ctcaaacaaa aagtcc 16
<210> 582
   <211> 14
   <212> DNA
   <213> Artificial Sequence
<220>
   <223> Synthetic Oligonucleotide
<400> 582
   caaacaaaaa gtcc 14
<210> 583
   <211> 16
   <212> DNA
   <213> Artificial Sequence
<220>
   <223> Synthetic Oligonucleotide
<400> 583
   gtggtgacat tggtc 16
<210> 584
   <211> 14
   <212> DNA
   <213> Artificial Sequence
<220>
   <223> Synthetic Oligonucleotide
<400> 584
   ggtgacattg gtca 14
<210> 585
   <211> 4
   <212> DNA
   <213> Artificial Sequence
<220>
   <223> Synthetic Oligonucleotide
<400> 585
   actg 4
<210> 586
   <211> 4
   <212> DNA
   <213> Artificial Sequence
<220>
   <223> Synthetic Oligonucleotide
<400> 586
   actg 4
<210> 587
   <211> 14
   <212> DNA
   <213> Artificial Sequence
<220>
   <223> Synthetic Oligonucleotide
<400> 587
   ttggcagtgg tgtt 14
<210> 588
   <211> 14
   <212> DNA
   <213> Artificial Sequence
<220>
   <223> Synthetic Oligonucleotide
<400> 588
   atgttggcag tggt 14
<210> 589
   <211> 16
   <212> DNA
   <213> Artificial Sequence
<220>
   <223> Synthetic Oligonucleotide
<400> 589
   aggaaatgtt ggcagt 16
<210> 590
   <211> 14
   <212> DNA
   <213> Artificial Sequence
<220>
   <223> Synthetic Oligonucleotide
<400> 590
   gaaatgttgg cagt 14
<210> 591
   <211> 14
   <212> DNA
   <213> Artificial Sequence
<220>
   <223> Synthetic Oligonucleotide
<400> 591
   caggaaatgt tggc 14
<210> 592
   <211> 14
   <212> DNA
   <213> Artificial Sequence
<220>
   <223> Synthetic Oligonucleotide
<400> 592
   gggcaggaaa tgtt 14
<210> 593
   <211> 14
   <212> DNA
   <213> Artificial Sequence
<220>
   <223> Synthetic Oligonucleotide
<400> 593
   aaggtaggta ccag 14
<210> 594
   <211> 14
   <212> DNA
   <213> Artificial Sequence
<220>
   <223> Synthetic Oligonucleotide
<400> 594
   accaaggtag gtac 14
<210> 595
   <211> 14
   <212> DNA
   <213> Artificial Sequence
<220>
   <223> Synthetic Oligonucleotide
<400> 595
   ctttgtggca ccaa 14
<210> 596
   <211> 14
   <212> DNA
   <213> Artificial Sequence
<220>
   <223> Synthetic Oligonucleotide
<400> 596
   gcactttgtg gcac 14
<210> 597
   <211> 14
   <212> DNA
   <213> Artificial Sequence
<220>
   <223> Synthetic Oligonucleotide
<400> 597
   tgcactttgt ggca 14
<210> 598
   <211> 14
   <212> DNA
   <213> Artificial Sequence
<220>
   <223> Synthetic Oligonucleotide
<400> 598
   gctgcacttt gtgg 14
<210> 599
   <211> 14
   <212> DNA
   <213> Artificial Sequence
<220>
   <223> Synthetic Oligonucleotide
<400> 599
   ggtgctgcac tttg 14
<210> 600
   <211> 14
   <212> DNA
   <213> Artificial Sequence
<220>
   <223> Synthetic Oligonucleotide
<400> 600
   ggcggtgctg cact 14
<210> 601
   <211> 14
   <212> DNA
   <213> Artificial Sequence
<220>
   <223> Synthetic Oligonucleotide
<400> 601
   tgaacactag gcgg 14
<210> 602
   <211> 14
   <212> DNA
   <213> Artificial Sequence
<220>
   <223> Synthetic Oligonucleotide
<400> 602
   tcttgaacac tagg 14
<210> 603
   <211> 16
   <212> DNA
   <213> Artificial Sequence
<220>
   <223> Synthetic Oligonucleotide
<400> 603
   cacctcttga acacta 16
<210> 604
   <211> 14
   <212> DNA
   <213> Artificial Sequence
<220>
   <223> Synthetic Oligonucleotide
<400> 604
   cctcttgaac acta 14
<210> 605
   <211> 14
   <212> DNA
   <213> Artificial Sequence
<220>
   <223> Synthetic Oligonucleotide
<400> 605
   acctcttgaa cact 14
<210> 606
   <211> 14
   <212> DNA
   <213> Artificial Sequence
<220>
   <223> Synthetic Oligonucleotide
<400> 606
   cacacctctt gaac 14
<210> 607
   <211> 14
   <212> DNA
   <213> Artificial Sequence
<220>
   <223> Synthetic Oligonucleotide
<400> 607
   cctcgaaccc actg 14
<210> 608
   <211> 16
   <212> DNA
   <213> Artificial Sequence
<220>
   <223> Synthetic Oligonucleotide
<400> 608
   cttctggacc tcgatc 16
<210> 609
   <211> 14
   <212> DNA
   <213> Artificial Sequence
<220>
   <223> Synthetic Oligonucleotide
<400> 609
   tctggacctc gatc 14
<210> 610
   <211> 16
   <212> DNA
   <213> Artificial Sequence
<220>
   <223> Synthetic Oligonucleotide
<400> 610
   ctgctataca tcttgg 16
<210> 611
   <211> 14
   <212> DNA
   <213> Artificial Sequence
<220>
   <223> Synthetic Oligonucleotide
<400> 611
   gctatacatc ttgg 14
<210> 612
   <211> 16
   <212> DNA
   <213> Artificial Sequence
<220>
   <223> Synthetic Oligonucleotide
<400> 612
   cacggtgtac atcacc 16
<210> 613
   <211> 14
   <212> DNA
   <213> Artificial Sequence
<220>
   <223> Synthetic Oligonucleotide
<400> 613
   cggtgtacat cacc 14
<210> 614
   <211> 16
   <212> DNA
   <213> Artificial Sequence
<220>
   <223> Synthetic Oligonucleotide
<400> 614
   ggacagactg tagccc 16
<210> 615
   <211> 14
   <212> DNA
   <213> Artificial Sequence
<220>
   <223> Synthetic Oligonucleotide
<400> 615
   acagactgta gccc 14
<210> 616
   <211> 16
   <212> DNA
   <213> Artificial Sequence
<220>
   <223> Synthetic Oligonucleotide
<400> 616
   gtcatcgcca atcttc 16
<210> 617
   <211> 14
   <212> DNA
   <213> Artificial Sequence
<220>
   <223> Synthetic Oligonucleotide
<400> 617
   catcgccaat cttc 14
<210> 618
   <211> 16
   <212> DNA
   <213> Artificial Sequence
<220>
   <223> Synthetic Oligonucleotide
<400> 618
   tcacactgag gtcatc 16
<210> 619
   <211> 14
   <212> DNA
   <213> Artificial Sequence
<220>
   <223> Synthetic Oligonucleotide
<400> 619
   acactgaggt catc 14
<210> 620
   <211> 14
   <212> DNA
   <213> Artificial Sequence
<220>
   <223> Synthetic Oligonucleotide
<400> 620
   cgccccgtca ctga 14
<210> 621
   <211> 16
   <212> DNA
   <213> Artificial Sequence
<220>
   <223> Synthetic Oligonucleotide
<400> 621
   ccagcaacca gcaata 16
<210> 622
   <211> 14
   <212> DNA
   <213> Artificial Sequence
<220>
   <223> Synthetic Oligonucleotide
<400> 622
   agcaaccagc aata 14
<210> 623
   <211> 16
   <212> DNA
   <213> Artificial Sequence
<220>
   <223> Synthetic Oligonucleotide
<400> 623
   caggctgtac aggtac 16
<210> 624
   <211> 14
   <212> DNA
   <213> Artificial Sequence
<220>
   <223> Synthetic Oligonucleotide
<400> 624
   ggctgtacag gtac 14
<210> 625
   <211> 16
   <212> DNA
   <213> Artificial Sequence
<220>
   <223> Synthetic Oligonucleotide
<400> 625
   gaagctcctc tcagag 16
<210> 626
   <211> 14
   <212> DNA
   <213> Artificial Sequence
<220>
   <223> Synthetic Oligonucleotide
<400> 626
   agctcctctc agag 14
<210> 627
   <211> 16
   <212> DNA
   <213> Artificial Sequence
<220>
   <223> Synthetic Oligonucleotide
<400> 627
   cccagccaat gcccag 16
<210> 628
   <211> 14
   <212> DNA
   <213> Artificial Sequence
<220>
   <223> Synthetic Oligonucleotide
<400> 628
   cagccaatgc ccag 14
<210> 629
   <211> 16
   <212> DNA
   <213> Artificial Sequence
<220>
   <223> Synthetic Oligonucleotide
<400> 629
   tcaaacagac acttga 16
<210> 630
   <211> 14
   <212> DNA
   <213> Artificial Sequence
<220>
   <223> Synthetic Oligonucleotide
<400> 630
   aaacagacac ttga 14
<210> 631
   <211> 16
   <212> DNA
   <213> Artificial Sequence
<220>
   <223> Synthetic Oligonucleotide
<400> 631
   agcactgaac attctc 16
<210> 632
   <211> 14
   <212> DNA
   <213> Artificial Sequence
<220>
   <223> Synthetic Oligonucleotide
<400> 632
   cactgaacat tctc 14
<210> 633
   <211> 16
   <212> DNA
   <213> Artificial Sequence
<220>
   <223> Synthetic Oligonucleotide
<400> 633
   ggatccacca gaatcc 16
<210> 634
   <211> 14
   <212> DNA
   <213> Artificial Sequence
<220>
   <223> Synthetic Oligonucleotide
<400> 634
   atccaccaga atcc 14
<210> 635
   <211> 14
   <212> DNA
   <213> Artificial Sequence
<220>
   <223> Synthetic Oligonucleotide
<400> 635
   tgatcagtaa ggcc 14
<210> 636
   <211> 16
   <212> DNA
   <213> Artificial Sequence
<220>
   <223> Synthetic Oligonucleotide
<400> 636
   ggacaaagat gaaaaa 16
<210> 637
   <211> 14
   <212> DNA
   <213> Artificial Sequence
<220>
   <223> Synthetic Oligonucleotide
<400> 637
   acaaagatga aaaa 14
<210> 638
   <211> 16
   <212> DNA
   <213> Artificial Sequence
<220>
   <223> Synthetic Oligonucleotide
<400> 638
   ggcacgcagc ttggcc 16
<210> 639
   <211> 14
   <212> DNA
   <213> Artificial Sequence
<220>
   <223> Synthetic Oligonucleotide
<400> 639
   cacgcagctt ggcc 14
<210> 640
   <211> 16
   <212> DNA
   <213> Artificial Sequence
<220>
   <223> Synthetic Oligonucleotide
<400> 640
   tggaccccca gcagag 16
<210> 641
   <211> 14
   <212> DNA
   <213> Artificial Sequence
<220>
   <223> Synthetic Oligonucleotide
<400> 641
   gacccccagc agag 14
<210> 642
   <211> 14
   <212> DNA
   <213> Artificial Sequence
<220>
   <223> Synthetic Oligonucleotide
<400> 642
   caaaggcaaa gacc 14
<210> 643
   <211> 14
   <212> DNA
   <213> Artificial Sequence
<220>
   <223> Synthetic Oligonucleotide
<400> 643
   tcacaaaggc aaag 14
<210> 644
   <211> 14
   <212> DNA
   <213> Artificial Sequence
<220>
   <223> Synthetic Oligonucleotide
<400> 644
   cagtcacaaa ggca 14
<210> 645
   <211> 14
   <212> DNA
   <213> Artificial Sequence
<220>
   <223> Synthetic Oligonucleotide
<400> 645
   cgtcagtcac aaag 14
<210> 646
   <211> 14
   <212> DNA
   <213> Artificial Sequence
<220>
   <223> Synthetic Oligonucleotide
<400> 646
   gctcgtcagt caca 14
<210> 647
   <211> 14
   <212> DNA
   <213> Artificial Sequence
<220>
   <223> Synthetic Oligonucleotide
<400> 647
   catgctcgtc agtc 14
<210> 648
   <211> 14
   <212> DNA
   <213> Artificial Sequence
<220>
   <223> Synthetic Oligonucleotide
<400> 648
   gggcatgctc gtca 14
<210> 649
   <211> 4
   <212> DNA
   <213> Artificial Sequence
<220>
   <223> Synthetic Oligonucleotide
<400> 649
   actg 4
<210> 650
   <211> 4
   <212> DNA
   <213> Artificial Sequence
<220>
   <223> Synthetic Oligonucleotide
<400> 650
   actg 4
<210> 651
   <211> 4
   <212> DNA
   <213> Artificial Sequence
<220>
   <223> Synthetic Oligonucleotide
<400> 651
   actg 4
<210> 652
   <211> 4
   <212> DNA
   <213> Artificial Sequence
<220>
   <223> Synthetic Oligonucleotide
<400> 652
   actg 4
<210> 653
   <211> 4
   <212> DNA
   <213> Artificial Sequence
<220>
   <223> Synthetic Oligonucleotide
<400> 653
   actg 4
<210> 654
   <211> 14
   <212> DNA
   <213> Artificial Sequence
<220>
   <223> Synthetic Oligonucleotide
<400> 654
   cttgggcatg ctcg 14
<210> 655
   <211> 4
   <212> DNA
   <213> Artificial Sequence
<220>
   <223> Synthetic Oligonucleotide
<400> 655
   actg 4
<210> 656
   <211> 4
   <212> DNA
   <213> Artificial Sequence
<220>
   <223> Synthetic Oligonucleotide
<400> 656
   actg 4
<210> 657
   <211> 14
   <212> DNA
   <213> Artificial Sequence
<220>
   <223> Synthetic Oligonucleotide
<400> 657
   tgccttgggc atgc 14
<210> 658
   <211> 14
   <212> DNA
   <213> Artificial Sequence
<220>
   <223> Synthetic Oligonucleotide
<400> 658
   gggtgccttg ggca 14
<210> 659
   <211> 14
   <212> DNA
   <213> Artificial Sequence
<220>
   <223> Synthetic Oligonucleotide
<400> 659
   gcagggtgcc ttgg 14
<210> 660
   <211> 14
   <212> DNA
   <213> Artificial Sequence
<220>
   <223> Synthetic Oligonucleotide
<400> 660
   agcgcagggt gcct 14
<210> 661
   <211> 16
   <212> DNA
   <213> Artificial Sequence
<220>
   <223> Synthetic Oligonucleotide
<400> 661
   gtggagcgca gggtgc 16
<210> 662
   <211> 14
   <212> DNA
   <213> Artificial Sequence
<220>
   <223> Synthetic Oligonucleotide
<400> 662
   tggagcgcag ggtg 14
<210> 663
   <211> 14
   <212> DNA
   <213> Artificial Sequence
<220>
   <223> Synthetic Oligonucleotide
<400> 663
   tggtggagcg cagg 14
<210> 664
   <211> 14
   <212> DNA
   <213> Artificial Sequence
<220>
   <223> Synthetic Oligonucleotide
<400> 664
   gcttggtgga gcgc 14
<210> 665
   <211> 14
   <212> DNA
   <213> Artificial Sequence
<220>
   <223> Synthetic Oligonucleotide
<400> 665
   agagcttggt ggag 14
<210> 666
   <211> 16
   <212> DNA
   <213> Artificial Sequence
<220>
   <223> Synthetic Oligonucleotide
<400> 666
   aaaagagctt ggtgga 16
<210> 667
   <211> 14
   <212> DNA
   <213> Artificial Sequence
<220>
   <223> Synthetic Oligonucleotide
<400> 667
   aagagcttgg tgga 14
<210> 668
   <211> 14
   <212> DNA
   <213> Artificial Sequence
<220>
   <223> Synthetic Oligonucleotide
<400> 668
   aaaagagctt ggtg 14
<210> 669
   <211> 14
   <212> DNA
   <213> Artificial Sequence
<220>
   <223> Synthetic Oligonucleotide
<400> 669
   caaaaaagag cttg 14
<210> 670
   <211> 16
   <212> DNA
   <213> Artificial Sequence
<220>
   <223> Synthetic Oligonucleotide
<400> 670
   aaggagctga ggaaca 16
<210> 671
   <211> 14
   <212> DNA
   <213> Artificial Sequence
<220>
   <223> Synthetic Oligonucleotide
<400> 671
   ggagctgagg aaca 14
<210> 672
   <211> 16
   <212> DNA
   <213> Artificial Sequence
<220>
   <223> Synthetic Oligonucleotide
<400> 672
   gcagaccctg gaagga 16
<210> 673
   <211> 14
   <212> DNA
   <213> Artificial Sequence
<220>
   <223> Synthetic Oligonucleotide
<400> 673
   agaccctgga agga 14
<210> 674
   <211> 16
   <212> DNA
   <213> Artificial Sequence
<220>
   <223> Synthetic Oligonucleotide
<400> 674
   accagcagac cctgga 16
<210> 675
   <211> 4
   <212> DNA
   <213> Artificial Sequence
<220>
   <223> Synthetic Oligonucleotide
<400> 675
   actg 4
<210> 676
   <211> 16
   <212> DNA
   <213> Artificial Sequence
<220>
   <223> Synthetic Oligonucleotide
<400> 676
   cagtagagaa cagcca 16
<210> 677
   <211> 14
   <212> DNA
   <213> Artificial Sequence
<220>
   <223> Synthetic Oligonucleotide
<400> 677
   gtagagaaca gcca 14
<210> 678
   <211> 16
   <212> DNA
   <213> Artificial Sequence
<220>
   <223> Synthetic Oligonucleotide
<400> 678
   tgttgaggaa acagta 16
<210> 679
   <211> 14
   <212> DNA
   <213> Artificial Sequence
<220>
   <223> Synthetic Oligonucleotide
<400> 679
   ttgaggaaac agta 14
<210> 680
   <211> 16
   <212> DNA
   <213> Artificial Sequence
<220>
   <223> Synthetic Oligonucleotide
<400> 680
   cctgcacctc cttgtt 16
<210> 681
   <211> 14
   <212> DNA
   <213> Artificial Sequence
<220>
   <223> Synthetic Oligonucleotide
<400> 681
   atgagggtct tcat 14
<210> 682
   <211> 14
   <212> DNA
   <213> Artificial Sequence
<220>
   <223> Synthetic Oligonucleotide
<400> 682
   accccggagt aggc 14
<210> 683
   <211> 12
   <212> DNA
   <213> Artificial Sequence
<220>
   <223> Synthetic Oligonucleotide
<400> 683
   cccggagtag gc 12
<210> 684
   <211> 16
   <212> DNA
   <213> Artificial Sequence
<220>
   <223> Synthetic Oligonucleotide
<400> 684
   caggaccccg gagtag 16
<210> 685
   <211> 16
   <212> DNA
   <213> Artificial Sequence
<220>
   <223> Synthetic Oligonucleotide
<400> 685
   gcaggacccc ggagta 16
<210> 686
   <211> 14
   <212> DNA
   <213> Artificial Sequence
<220>
   <223> Synthetic Oligonucleotide
<400> 686
   aggaccccgg agta 14
<210> 687
   <211> 14
   <212> DNA
   <213> Artificial Sequence
<220>
   <223> Synthetic Oligonucleotide
<400> 687
   caggaccccg gagt 14
<210> 688
   <211> 12
   <212> DNA
   <213> Artificial Sequence
<220>
   <223> Synthetic Oligonucleotide
<400> 688
   cagacccctc gc 12
<210> 689
   <211> 12
   <212> DNA
   <213> Artificial Sequence
<220>
   <223> Synthetic Oligonucleotide
<400> 689
   agaggatgct gg 12
<210> 690
   <211> 16
   <212> DNA
   <213> Artificial Sequence
<220>
   <223> Synthetic Oligonucleotide
<400> 690
   gagccaggtg acagag 16
<210> 691
   <211> 14
   <212> DNA
   <213> Artificial Sequence
<220>
   <223> Synthetic Oligonucleotide
<400> 691
   agccaggtga caga 14
<210> 692
   <211> 12
   <212> DNA
   <213> Artificial Sequence
<220>
   <223> Synthetic Oligonucleotide
<400> 692
   tgagccaggt ga 12
<210> 693
   <211> 14
   <212> DNA
   <213> Artificial Sequence
<220>
   <223> Synthetic Oligonucleotide
<400> 693
   ttttccacct tgga 14
<210> 694
   <211> 12
   <212> DNA
   <213> Artificial Sequence
<220>
   <223> Synthetic Oligonucleotide
<400> 694
   ctgcaggcca ct 12
<210> 695
   <211> 16
   <212> DNA
   <213> Artificial Sequence
<220>
   <223> Synthetic Oligonucleotide
<400> 695
   tcaccagctg gatggg 16
<210> 696
   <211> 16
   <212> DNA
   <213> Artificial Sequence
<220>
   <223> Synthetic Oligonucleotide
<400> 696
   ttcaccagct ggatgg 16
<210> 697
   <211> 14
   <212> DNA
   <213> Artificial Sequence
<220>
   <223> Synthetic Oligonucleotide
<400> 697
   caccagctgg atgg 14
<210> 698
   <211> 14
   <212> DNA
   <213> Artificial Sequence
<220>
   <223> Synthetic Oligonucleotide
<400> 698
   tcaccagctg gatg 14
<210> 699
   <211> 12
   <212> DNA
   <213> Artificial Sequence
<220>
   <223> Synthetic Oligonucleotide
<400> 699
   tcaccagctg ga 12
<210> 700
   <211> 16
   <212> DNA
   <213> Artificial Sequence
<220>
   <223> Synthetic Oligonucleotide
<400> 700
   tgtcttcacc agctgg 16
<210> 701
   <211> 14
   <212> DNA
   <213> Artificial Sequence
<220>
   <223> Synthetic Oligonucleotide
<400> 701
   gtcttcacca gctg 14
<210> 702
   <211> 16
   <212> DNA
   <213> Artificial Sequence
<220>
   <223> Synthetic Oligonucleotide
<400> 702
   gtgtgtcttc accagc 16
<210> 703
   <211> 14
   <212> DNA
   <213> Artificial Sequence
<220>
   <223> Synthetic Oligonucleotide
<400> 703
   tgtgtcttca ccag 14
<210> 704
   <211> 16
   <212> DNA
   <213> Artificial Sequence
<220>
   <223> Synthetic Oligonucleotide
<400> 704
   ttgtgtgtct tcacca 16
<210> 705
   <211> 14
   <212> DNA
   <213> Artificial Sequence
<220>
   <223> Synthetic Oligonucleotide
<400> 705
   tgtgtgtctt cacc 14
<210> 706
   <211> 16
   <212> DNA
   <213> Artificial Sequence
<220>
   <223> Synthetic Oligonucleotide
<400> 706
   aggttgtgtg tcttca 16
<210> 707
   <211> 14
   <212> DNA
   <213> Artificial Sequence
<220>
   <223> Synthetic Oligonucleotide
<400> 707
   ggttgtgtgt cttc 14
<210> 708
   <211> 16
   <212> DNA
   <213> Artificial Sequence
<220>
   <223> Synthetic Oligonucleotide
<400> 708
   gcaggttgtg tgtctt 16
<210> 709
   <211> 14
   <212> DNA
   <213> Artificial Sequence
<220>
   <223> Synthetic Oligonucleotide
<400> 709
   caggttgtgt gtct 14
<210> 710
   <211> 16
   <212> DNA
   <213> Artificial Sequence
<220>
   <223> Synthetic Oligonucleotide
<400> 710
   tcagcaggtt gtgtgt 16
<210> 711
   <211> 14
   <212> DNA
   <213> Artificial Sequence
<220>
   <223> Synthetic Oligonucleotide
<400> 711
   cagcaggttg tgtg 14
<210> 712
   <211> 16
   <212> DNA
   <213> Artificial Sequence
<220>
   <223> Synthetic Oligonucleotide
<400> 712
   ggtcagcagg ttgtgt 16
<210> 713
   <211> 16
   <212> DNA
   <213> Artificial Sequence
<220>
   <223> Synthetic Oligonucleotide
<400> 713
   tggtcagcag gttgtg 16
<210> 714
   <211> 14
   <212> DNA
   <213> Artificial Sequence
<220>
   <223> Synthetic Oligonucleotide
<400> 714
   gtcagcaggt tgtg 14
<210> 715
   <211> 14
   <212> DNA
   <213> Artificial Sequence
<220>
   <223> Synthetic Oligonucleotide
<400> 715
   ggtcagcagg ttgt 14
<210> 716
   <211> 16
   <212> DNA
   <213> Artificial Sequence
<220>
   <223> Synthetic Oligonucleotide
<400> 716
   ctggtggtca gcaggt 16
<210> 717
   <211> **14**
   <212> DNA
   <213> Artificial Sequence
<220>
   <223> Synthetic Oligonucleotide
<400> 717
   tggtggtcag cagg 14
<210> 718
   <211> 12
   <212> DNA
   <213> Artificial Sequence
<220>
   <223> Synthetic Oligonucleotide
<400> 718
   ctggtggtca gc 12
<210> 719
   <211> 16
   <212> DNA
   <213> Artificial Sequence
<220>
   <223> Synthetic Oligonucleotide
<400> 719
   agttcctggt ggtcag 16
<210> 720
   <211> 14
   <212> DNA
   <213> Artificial Sequence
<220>
   <223> Synthetic Oligonucleotide
<400> 720
   gttcctggtg gtca 14
<210> 721
   <211> 16
   <212> DNA
   <213> Artificial Sequence
<220>
   <223> Synthetic Oligonucleotide
<400> 721
   tatagttcct ggtggt 16
<210> 722
   <211> 14
   <212> DNA
   <213> Artificial Sequence
<220>
   <223> Synthetic Oligonucleotide
<400> 722
   atagttcctg gtgg 14
<210> 723
   <211> 16
   <212> DNA
   <213> Artificial Sequence
<220>
   <223> Synthetic Oligonucleotide
<400> 723
   gatatagttc ctggtg 16
<210> 724
   <211> 14
   <212> DNA
   <213> Artificial Sequence
<220>
   <223> Synthetic Oligonucleotide
<400> 724
   atatagttcc tggt 14
<210> 725
   <211> 16
   <212> DNA
   <213> Artificial Sequence
<220>
   <223> Synthetic Oligonucleotide
<400> 725
   ccaaagatat agttcc 16
<210> 726
   <211> 16
   <212> DNA
   <213> Artificial Sequence
<220>
   <223> Synthetic Oligonucleotide
<400> 726
   tccaaagata tagttc 16
<210> 727
   <211> 14
   <212> DNA
   <213> Artificial Sequence
<220>
   <223> Synthetic Oligonucleotide
<400> 727
   caaagatata gttc 14
<210> 728
   <211> 14
   <212> DNA
   <213> Artificial Sequence
<220>
   <223> Synthetic Oligonucleotide
<400> 728
   ccaaagatat agtt 14
<210> 729
   <211> 12
   <212> DNA
   <213> Artificial Sequence
<220>
   <223> Synthetic Oligonucleotide
<400> 729
   ccaggcccat ga 12
<210> 730
   <211> 16
   <212> DNA
   <213> Artificial Sequence
<220>
   <223> Synthetic Oligonucleotide
<400> 730
   ggcacccagg cccatg 16
<210> 731
   <211> 14
   <212> DNA
   <213> Artificial Sequence
<220>
   <223> Synthetic Oligonucleotide
<400> 731
   gcacccaggc ccat 14
<210> 732
   <211> 16
   <212> DNA
   <213> Artificial Sequence
<220>
   <223> Synthetic Oligonucleotide
<400> 732
   cagaaggcac ccaggc 16
<210> 733
   <211> 14
   <212> DNA
   <213> Artificial Sequence
<220>
   <223> Synthetic Oligonucleotide
<400> 733
   agaaggcacc cagg 14
<210> 734
   <211> 12
   <212> DNA
   <213> Artificial Sequence
<220>
   <223> Synthetic Oligonucleotide
<400> 734
   ccagacatca gg 12
<210> 735
   <211> 12
   <212> DNA
   <213> Artificial Sequence
<220>
   <223> Synthetic Oligonucleotide
<400> 735
   gacagggcag at 12
<210> 736
   <211> 12
   <212> DNA
   <213> Artificial Sequence
<220>
   <223> Synthetic Oligonucleotide
<400> 736
   tgacagggca ga 12
<210> 737
   <211> 12
   <212> DNA
   <213> Artificial Sequence
<220>
   <223> Synthetic Oligonucleotide
<400> 737
   ccactcccat tc 12
<210> 738
   <211> 16
   <212> DNA
   <213> Artificial Sequence
<220>
   <223> Synthetic Oligonucleotide
<400> 738
   gccaggcatg gagctc 16
<210> 739
   <211> 14
   <212> DNA
   <213> Artificial Sequence
<220>
   <223> Synthetic Oligonucleotide
<400> 739
   ccaggcatgg agct 14
<210> 740
   <211> 12
   <212> DNA
   <213> Artificial Sequence
<220>
   <223> Synthetic Oligonucleotide
<400> 740
   ccaggcatgg ag 12
<210> 741
   <211> 12
   <212> DNA
   <213> Artificial Sequence
<220>
   <223> Synthetic Oligonucleotide
<400> 741
   cagggtgact gc 12
<210> 742
   <211> 16
   <212> DNA
   <213> Artificial Sequence
<220>
   <223> Synthetic Oligonucleotide
<400> 742
   gttccgcagg gtgact 16
<210> 743
   <211> 14
   <212> DNA
   <213> Artificial Sequence
<220>
   <223> Synthetic Oligonucleotide
<400> 743
   ttccgcaggg tgac 14
<210> 744
   <211> 16
   <212> DNA
   <213> Artificial Sequence
<220>
   <223> Synthetic Oligonucleotide
<400> 744
   gcggttccgc agggtg 16
<210> 745
   <211> 14
   <212> DNA
   <213> Artificial Sequence
<220>
   <223> Synthetic Oligonucleotide
<400> 745
   cggttccgca gggt 14
<210> 746
   <211> 16
   <212> DNA
   <213> Artificial Sequence
<220>
   <223> Synthetic Oligonucleotide
<400> 746
   tcggccccag gagccc 16
<210> 747
   <211> 14
   <212> DNA
   <213> Artificial Sequence
<220>
   <223> Synthetic Oligonucleotide
<400> 747
   cggccccagg agcc 14
<210> 748
   <211> 16
   <212> DNA
   <213> Artificial Sequence
<220>
   <223> Synthetic Oligonucleotide
<400> 748
   ccatcggccc caggag 16
<210> 749
   <211> 14
   <212> DNA
   <213> Artificial Sequence
<220>
   <223> Synthetic Oligonucleotide
<400> 749
   catcggcccc agga 14
<210> 750
   <211> 16
   <212> DNA
   <213> Artificial Sequence
<220>
   <223> Synthetic Oligonucleotide
<400> 750
   gacccatcgg ccccag 16
<210> 751
   <211> 14
   <212> DNA
   <213> Artificial Sequence
<220>
   <223> Synthetic Oligonucleotide
<400> 751
   acccatcggc ccca 14
<210> 752
   <211> 16
   <212> DNA
   <213> Artificial Sequence
<220>
   <223> Synthetic Oligonucleotide
<400> 752
   ttctggaccc atcggc 16
<210> 753
   <211> 12
   <212> DNA
   <213> Artificial Sequence
<220>
   <223> Synthetic Oligonucleotide
<400> 753
   ggacccatcg gc 12
<210> 754
   <211> 14
   <212> DNA
   <213> Artificial Sequence
<220>
   <223> Synthetic Oligonucleotide
<400> 754
   tctggaccca tcgg 14
<210> 755
   <211> 16
   <212> DNA
   <213> Artificial Sequence
<220>
   <223> Synthetic Oligonucleotide
<400> 755
   caccagcccc caggtg 16
<210> 756
   <211> 14
   <212> DNA
   <213> Artificial Sequence
<220>
   <223> Synthetic Oligonucleotide
<400> 756
   accagccccc aggt 14
<210> 757
   <211> 16
   <212> DNA
   <213> Artificial Sequence
<220>
   <223> Synthetic Oligonucleotide
<400> 757
   tagggcacca gccccc 16
<210> 758
   <211> 14
   <212> DNA
   <213> Artificial Sequence
<220>
   <223> Synthetic Oligonucleotide
<400> 758
   agggcaccag cccc 14
<210> 759
   <211> 16
   <212> DNA
   <213> Artificial Sequence
<220>
   <223> Synthetic Oligonucleotide
<400> 759
   tggagtaggg caccag 16
<210> 760
   <211> 14
   <212> DNA
   <213> Artificial Sequence
<220>
   <223> Synthetic Oligonucleotide
<400> 760
   ggagtagggc acca 14
<210> 761
   <211> 12
   <212> DNA
   <213> Artificial Sequence
<220>
   <223> Synthetic Oligonucleotide
<400> 761
   cttggagtag gg 12
<210> 762
   <211> 16
   <212> DNA
   <213> Artificial Sequence
<220>
   <223> Synthetic Oligonucleotide
<400> 762
   tgatgggctt ggagta 16
<210> 763
   <211> 14
   <212> DNA
   <213> Artificial Sequence
<220>
   <223> Synthetic Oligonucleotide
<400> 763
   gatgggcttg gagt 14
<210> 764
   <211> 16
   <212> DNA
   <213> Artificial Sequence
<220>
   <223> Synthetic Oligonucleotide
<400> 764
   tgtggtacag gtcgat 16
<210> 765
   <211> 14
   <212> DNA
   <213> Artificial Sequence
<220>
   <223> Synthetic Oligonucleotide
<400> 765
   gtggtacagg tcga 14
<210> 766
   <211> 12
   <212> DNA
   <213> Artificial Sequence
<220>
   <223> Synthetic Oligonucleotide
<400> 766
   ggtacaggtc ga 12
<210> 767
   <211> 16
   <212> DNA
   <213> Artificial Sequence
<220>
   <223> Synthetic Oligonucleotide
<400> 767
   ggtgtggtac aggtcg 16
<210> 768
   <211> 14
   <212> DNA
   <213> Artificial Sequence
<220>
   <223> Synthetic Oligonucleotide
<400> 768
   gtgtggtaca ggtc 14
<210> 769
   <211> 16
   <212> DNA
   <213> Artificial Sequence
<220>
   <223> Synthetic Oligonucleotide
<400> 769
   catggtgtgg tacagg 16
<210> 770
   <211> 14
   <212> DNA
   <213> Artificial Sequence
<220>
   <223> Synthetic Oligonucleotide
<400> 770
   atggtgtggt acag 14
<210> 771
   <211> 16
   <212> DNA
   <213> Artificial Sequence
<220>
   <223> Synthetic Oligonucleotide
<400> 771
   tacatggtgt ggtaca 16
<210> 772
   <211> 14
   <212> DNA
   <213> Artificial Sequence
<220>
   <223> Synthetic Oligonucleotide
<400> 772
   acatggtgtg gtac 14
<210> 773
   <211> 16
   <212> DNA
   <213> Artificial Sequence
<220>
   <223> Synthetic Oligonucleotide
<400> 773
   atgtacatgg tgtggt 16
<210> 774
   <211> 14
   <212> DNA
   <213> Artificial Sequence
<220>
   <223> Synthetic Oligonucleotide
<400> 774
   tgtacatggt gtgg 14
<210> 775
   <211> 12
   <212> DNA
   <213> Artificial Sequence
<220>
   <223> Synthetic Oligonucleotide
<400> 775
   gcctccatgt ac 12
<210> 776
   <211> 12
   <212> DNA
   <213> Artificial Sequence
<220>
   <223> Synthetic Oligonucleotide
<400> 776
   agcttcacca gg 12
<210> 777
   <211> 12
   <212> DNA
   <213> Artificial Sequence
<220>
   <223> Synthetic Oligonucleotide
<400> 777
   gttcacctcc ag 12
<210> 778
   <211> 16
   <212> DNA
   <213> Artificial Sequence
<220>
   <223> Synthetic Oligonucleotide
<400> 778
   catgccccag ccgccg 16
<210> 779
   <211> 14
   <212> DNA
   <213> Artificial Sequence
<220>
   <223> Synthetic Oligonucleotide
<400> 779
   atgccccagc cgcc 14
<210> 780
   <211> 16
   <212> DNA
   <213> Artificial Sequence
<220>
   <223> Synthetic Oligonucleotide
<400> 780
   gatgagggtc ttcatg 16
<210> 781
   <211> 16
   <212> DNA
   <213> Artificial Sequence
<220>
   <223> Synthetic Oligonucleotide
<400> 781
   gaccccggag taggca 16
<210> 782
   <211> 16
   <212> DNA
   <213> Artificial Sequence
<220>
   <223> Synthetic Oligonucleotide
<400> 782
   atgctggagc cagtgc 16
<210> 783
   <211> 14
   <212> DNA
   <213> Artificial Sequence
<220>
   <223> Synthetic Oligonucleotide
<400> 783
   tgctggagcc agtg 14
<210> 784
   <211> 14
   <212> DNA
   <213> Artificial Sequence
<220>
   <223> Synthetic Oligonucleotide
<400> 784
   gtcttggagg gccg 14
<210> 785
   <211> 14
   <212> DNA
   <213> Artificial Sequence
<220>
   <223> Synthetic Oligonucleotide
<400> 785
   cccaggtgtc agag 14
<210> 786
   <211> 16
   <212> DNA
   <213> Artificial Sequence
<220>
   <223> Synthetic Oligonucleotide
<400> 786
   ttttccacct tggatc 16
<210> 787
   <211> 14
   <212> DNA
   <213> Artificial Sequence
<220>
   <223> Synthetic Oligonucleotide
<400> 787
   tttccacctt ggat 14
<210> 788
   <211> 16
   <212> DNA
   <213> Artificial Sequence
<220>
   <223> Synthetic Oligonucleotide
<400> 788
   tttttccacc ttggat 16
<210> 789
   <211> 16
   <212> DNA
   <213> Artificial Sequence
<220>
   <223> Synthetic Oligonucleotide
<400> 789
   aggtgttttt ccacct 16
<210> 790
   <211> 14
   <212> DNA
   <213> Artificial Sequence
<220>
   <223> Synthetic Oligonucleotide
<400> 790
   ggtgtttttc cacc 14
<210> 791
   <211> 16
   <212> DNA
   <213> Artificial Sequence
<220>
   <223> Synthetic Oligonucleotide
<400> 791
   ccaggaagga taggac 16
<210> 792
   <211> 14
   <212> DNA
   <213> Artificial Sequence
<220>
   <223> Synthetic Oligonucleotide
<400> 792
   caggaaggat agga 14
<210> 793
   <211> 16
   <212> DNA
   <213> Artificial Sequence
<220>
   <223> Synthetic Oligonucleotide
<400> 793
   tgacactgca ggccac 16
<210> 794
   <211> 14
   <212> DNA
   <213> Artificial Sequence
<220>
   <223> Synthetic Oligonucleotide
<400> 794
   gacactgcag gcca 14
<210> 795
   <211> 16
   <212> DNA
   <213> Artificial Sequence
<220>
   <223> Synthetic Oligonucleotide
<400> 795
   acatgaggat gacact 16
<210> 796
   <211> 14
   <212> DNA
   <213> Artificial Sequence
<220>
   <223> Synthetic Oligonucleotide
<400> 796
   catgaggatg acac 14
<210> 797
   <211> 16
   <212> DNA
   <213> Artificial Sequence
<220>
   <223> Synthetic Oligonucleotide
<400> 797
   gcagaaggtg tacatg 16
<210> 798
   <211> 14
   <212> DNA
   <213> Artificial Sequence
<220>
   <223> Synthetic Oligonucleotide
<400> 798
   cagaaggtgt acat 14
<210> 799
   <211> 16
   <212> DNA
   <213> Artificial Sequence
<220>
   <223> Synthetic Oligonucleotide
<400> 799
   gcagtcagtg cagaag 16
<210> 800
   <211> 14
   <212> DNA
   <213> Artificial Sequence
<220>
   <223> Synthetic Oligonucleotide
<400> 800
   cagtcagtgc agaa 14
<210> 801
   <211> 16
   <212> DNA
   <213> Artificial Sequence
<220>
   <223> Synthetic Oligonucleotide
<400> 801
   acacggccca gtttcg 16
<210> 802
   <211> 14
   <212> DNA
   <213> Artificial Sequence
<220>
   <223> Synthetic Oligonucleotide
<400> 802
   cacggcccag tttc 14
<210> 803
   <211> 16
   <212> DNA
   <213> Artificial Sequence
<220>
   <223> Synthetic Oligonucleotide
<400> 803
   ggcccatgat gccatg 16
<210> 804
   <211> 14
   <212> DNA
   <213> Artificial Sequence
<220>
   <223> Synthetic Oligonucleotide
<400> 804
   gcccatgatg ccat 14
<210> 805
   <211> 16
   <212> DNA
   <213> Artificial Sequence
<220>
   <223> Synthetic Oligonucleotide
<400> 805
   tacagaaggc acccag 16
<210> 806
   <211> 14
   <212> DNA
   <213> Artificial Sequence
<220>
   <223> Synthetic Oligonucleotide
<400> 806
   acagaaggca ccca 14
<210> 807
   <211> 16
   <212> DNA
   <213> Artificial Sequence
<220>
   <223> Synthetic Oligonucleotide
<400> 807
   gaagttgcca gccaat 16
<210> 808
   <211> 14
   <212> DNA
   <213> Artificial Sequence
<220>
   <223> Synthetic Oligonucleotide
<400> 808
   aagttgccag ccaa 14
<210> 809
   <211> 14
   <212> DNA
   <213> Artificial Sequence
<220>
   <223> Synthetic Oligonucleotide
<400> 809
   cagggcagat cctt 14
<210> 810
   <211> 16
   <212> DNA
   <213> Artificial Sequence
<220>
   <223> Synthetic Oligonucleotide
<400> 810
   caagtagtct atggtg 16
<210> 811
   <211> 14
   <212> DNA
   <213> Artificial Sequence
<220>
   <223> Synthetic Oligonucleotide
<400> 811
   aagtagtcta tggt 14
<210> 812
   <211> 16
   <212> DNA
   <213> Artificial Sequence
<220>
   <223> Synthetic Oligonucleotide
<400> 812
   tggaaagcaa gtagtc 16
<210> 813
   <211> 14
   <212> DNA
   <213> Artificial Sequence
<220>
   <223> Synthetic Oligonucleotide
<400> 813
   ggaaagcaag tagt 14
<210> 814
   <211> 16
   <212> DNA
   <213> Artificial Sequence
<220>
   <223> Synthetic Oligonucleotide
<400> 814
   ggccagcttt acaaag 16
<210> 815
   <211> 14
   <212> DNA
   <213> Artificial Sequence
<220>
   <223> Synthetic Oligonucleotide
<400> 815
   gccagcttta caaa 14
<210> 816
   <211> 14
   <212> DNA
   <213> Artificial Sequence
<220>
   <223> Synthetic Oligonucleotide
<400> 816
   cgcagggcca gctt 14
<210> 817
   <211> 16
   <212> DNA
   <213> Artificial Sequence
<220>
   <223> Synthetic Oligonucleotide
<400> 817
   aaaggaatag gtggga 16
<210> 818
   <211> 14
   <212> DNA
   <213> Artificial Sequence
<220>
   <223> Synthetic Oligonucleotide
<400> 818
   aaggaatagg tggg 14
<210> 819
   <211> 16
   <212> DNA
   <213> Artificial Sequence
<220>
   <223> Synthetic Oligonucleotide
<400> 819
   gcgaaaccaa tatact 16
<210> 820
   <211> 14
   <212> DNA
   <213> Artificial Sequence
<220>
   <223> Synthetic Oligonucleotide
<400> 820
   cgaaaccaat atac 14
<210> 821
   <211> 16
   <212> DNA
   <213> Artificial Sequence
<220>
   <223> Synthetic Oligonucleotide
<400> 821
   ccccaggtgt cagagg 16
<210> 822
   <211> 4
   <212> DNA
   <213> Artificial Sequence
<220>
   <223> Synthetic Oligonucleotide
<400> 822
   actg 4
<210> 823
   <211> 16
   <212> DNA
   <213> Artificial Sequence
<220>
   <223> Synthetic Oligonucleotide
<400> 823
   gattgtcaaa gagctt 16
<210> 824
   <211> 14
   <212> DNA
   <213> Artificial Sequence
<220>
   <223> Synthetic Oligonucleotide
<400> 824
   attgtcaaag agct 14
<210> 825
   <211> 16
   <212> DNA
   <213> Artificial Sequence
<220>
   <223> Synthetic Oligonucleotide
<400> 825
   ttggtcttgt gattgt 16
<210> 826
   <211> 14
   <212> DNA
   <213> Artificial Sequence
<220>
   <223> Synthetic Oligonucleotide
<400> 826
   tggtcttgtg attg 14
<210> 827
   <211> 16
   <212> DNA
   <213> Artificial Sequence
<220>
   <223> Synthetic Oligonucleotide
<400> 827
   aaggccgaat ttggtc 16
<210> 828
   <211> 14
   <212> DNA
   <213> Artificial Sequence
<220>
   <223> Synthetic Oligonucleotide
<400> 828
   aggccgaatt tggt 14
<210> 829
   <211> 12
   <212> DNA
   <213> Artificial Sequence
<220>
   <223> Synthetic Oligonucleotide
<400> 829
   aatccgactg tg 12
<210> 830
   <211> 12
   <212> DNA
   <213> Artificial Sequence
<220>
   <223> Synthetic Oligonucleotide
<400> 830
   aatttaatcc ga 12
<210> 831
   <211> 14
   <212> DNA
   <213> Artificial Sequence
<220>
   <223> Synthetic Oligonucleotide
<400> 831
   accttcgatc acag 14
<210> 832
   <211> 12
   <212> DNA
   <213> Artificial Sequence
<220>
   <223> Synthetic Oligonucleotide
<400> 832
   actgatcctg ca 12
<210> 833
   <211> 12
   <212> DNA
   <213> Artificial Sequence
<220>
   <223> Synthetic Oligonucleotide
<400> 833
   actgtggtca aa 12
<210> 834
   <211> 12
   <212> DNA
   <213> Artificial Sequence
<220>
   <223> Synthetic Oligonucleotide
<400> 834
   agccgcccag tt 12
<210> 835
   <211> 12
   <212> DNA
   <213> Artificial Sequence
<220>
   <223> Synthetic Oligonucleotide
<400> 835
   agccttgtcg at 12
<210> 836
   <211> 12
   <212> DNA
   <213> Artificial Sequence
<220>
   <223> Synthetic Oligonucleotide
<400> 836
   agttcccagc ct 12
<210> 837
   <211> 12
   <212> DNA
   <213> Artificial Sequence
<220>
   <223> Synthetic Oligonucleotide
<400> 837
   atccgactgt gg 12
<210> 838
   <211> 12
   <212> DNA
   <213> Artificial Sequence
<220>
   <223> Synthetic Oligonucleotide
<400> 838
   atcctgcact ga 12
<210> 839
   <211> 12
   <212> DNA
   <213> Artificial Sequence
<220>
   <223> Synthetic Oligonucleotide
<400> 839
   atttaatccg ac 12
<210> 840
   <211> 12
   <212> DNA
   <213> Artificial Sequence
<220>
   <223> Synthetic Oligonucleotide
<400> 840
   caatttaatc cg 12
<210> 841
   <211> 12
   <212> DNA
   <213> Artificial Sequence
<220>
   <223> Synthetic Oligonucleotide
<400> 841
   cactgacgag tc 12
<210> 842
   <211> 12
   <212> DNA
   <213> Artificial Sequence
<220>
   <223> Synthetic Oligonucleotide
<400> 842
   cactgatcct gc 12
<210> 843
   <211> 12
   <212> DNA
   <213> Artificial Sequence
<220>
   <223> Synthetic Oligonucleotide
<400> 843
   cagccttgtc ga 12
<210> 844
   <211> 12
   <212> DNA
   <213> Artificial Sequence
<220>
   <223> Synthetic Oligonucleotide
<400> 844
   cagttcccag cc 12
<210> 845
   <211> 12
   <212> DNA
   <213> Artificial Sequence
<220>
   <223> Synthetic Oligonucleotide
<400> 845
   ccactgatcc tg 12
<210> 846
   <211> 12
   <212> DNA
   <213> Artificial Sequence
<220>
   <223> Synthetic Oligonucleotide
<400> 846
   ccagccttgt cg 12
<210> 847
   <211> 12
   <212> DNA
   <213> Artificial Sequence
<220>
   <223> Synthetic Oligonucleotide
<400> 847
   ccagttccca gc 12
<210> 848
   <211> 12
   <212> DNA
   <213> Artificial Sequence
<220>
   <223> Synthetic Oligonucleotide
<400> 848
   cccagccttg tc 12
<210> 849
   <211> 12
   <212> DNA
   <213> Artificial Sequence
<220>
   <223> Synthetic Oligonucleotide
<400> 849
   cccagttccc ag 12
<210> 850
   <211> 12
   <212> DNA
   <213> Artificial Sequence
<220>
   <223> Synthetic Oligonucleotide
<400> 850
   ccgcccagtt cc 12
<210> 851
   <211> 12
   <212> DNA
   <213> Artificial Sequence
<220>
   <223> Synthetic Oligonucleotide
<400> 851
   cctgcactga cg 12
<210> 852
   <211> 14
   <212> DNA
   <213> Artificial Sequence
<220>
   <223> Synthetic Oligonucleotide
<400> 852
   ccttggaatg tctg 14
<210> 853
   <211> 12
   <212> DNA
   <213> Artificial Sequence
<220>
   <223> Synthetic Oligonucleotide
<400> 853
   ccttgtcgat ct 12
<210> 854
   <211> 12
   <212> DNA
   <213> Artificial Sequence
<220>
   <223> Synthetic Oligonucleotide
<400> 854
   cgactgtggt ca 12
<210> 855
   <211> 12
   <212> DNA
   <213> Artificial Sequence
<220>
   <223> Synthetic Oligonucleotide
<400> 855
   cgcccagttc cc 12
<210> 856
   <211> 12
   <212> DNA
   <213> Artificial Sequence
<220>
   <223> Synthetic Oligonucleotide
<400> 856
   ctgatcctgc ac 12
<210> 857
   <211> 12
   <212> DNA
   <213> Artificial Sequence
<220>
   <223> Synthetic Oligonucleotide
<400> 857
   ctgcactgac ga 12
<210> 858
   <211> 12
   <212> DNA
   <213> Artificial Sequence
<220>
   <223> Synthetic Oligonucleotide
<400> 858
   ctgtggtcaa aa 12
<210> 859
   <211> 12
   <212> DNA
   <213> Artificial Sequence
<220>
   <223> Synthetic Oligonucleotide
<400> 859
   cttgtcgatc tc 12
<210> 860
   <211> 12
   <212> DNA
   <213> Artificial Sequence
<220>
   <223> Synthetic Oligonucleotide
<400> 860
   gatcctgcac tg 12
<210> 861
   <211> 12
   <212> DNA
   <213> Artificial Sequence
<220>
   <223> Synthetic Oligonucleotide
<400> 861
   gcaatttaat cc 12
<210> 862
   <211> 12
   <212> DNA
   <213> Artificial Sequence
<220>
   <223> Synthetic Oligonucleotide
<400> 862
   gcactgacga gt 12
<210> 863
   <211> 12
   <212> DNA
   <213> Artificial Sequence
<220>
   <223> Synthetic Oligonucleotide
<400> 863
   gcccagttcc ca 12
<210> 864
   <211> 12
   <212> DNA
   <213> Artificial Sequence
<220>
   <223> Synthetic Oligonucleotide
<400> 864
   gccgcccagt tc 12
<210> 865
   <211> 12
   <212> DNA
   <213> Artificial Sequence
<220>
   <223> Synthetic Oligonucleotide
<400> 865
   gccttgtcga tc 12
<210> 866
   <211> 12
   <212> DNA
   <213> Artificial Sequence
<220>
   <223> Synthetic Oligonucleotide
<400> 866
   ggtcaaaagg gc 12
<210> 867
   <211> 14
   <212> DNA
   <213> Artificial Sequence
<220>
   <223> Synthetic Oligonucleotide
<400> 867
   ggtcatgcac aggc 14
<210> 868
   <211> 12
   <212> DNA
   <213> Artificial Sequence
<220>
   <223> Synthetic Oligonucleotide
<400> 868
   gtcgatctcc tc 12
<210> 869
   <211> 12
   <212> DNA
   <213> Artificial Sequence
<220>
   <223> Synthetic Oligonucleotide
<400> 869
   gtggtcaaaa gg 12
<210> 870
   <211> 12
   <212> DNA
   <213> Artificial Sequence
<220>
   <223> Synthetic Oligonucleotide
<400> 870
   gttcccagcc tt 12
<210> 871
   <211> 12
   <212> DNA
   <213> Artificial Sequence
<220>
   <223> Synthetic Oligonucleotide
<400> 871
   taatccgact gt 12
<210> 872
   <211> 14
   <212> DNA
   <213> Artificial Sequence
<220>
   <223> Synthetic Oligonucleotide
<400> 872
   tattccatgg ccat 14
<210> 873
   <211> 14
   <212> DNA
   <213> Artificial Sequence
<220>
   <223> Synthetic Oligonucleotide
<400> 873
   tcaggtcatg caca 14
<210> 874
   <211> 12
   <212> DNA
   <213> Artificial Sequence
<220>
   <223> Synthetic Oligonucleotide
<400> 874
   tcccagcctt gt 12
<210> 875
   <211> 12
   <212> DNA
   <213> Artificial Sequence
<220>
   <223> Synthetic Oligonucleotide
<400> 875
   tccgactgtg gt 12
<210> 876
   <211> 12
   <212> DNA
   <213> Artificial Sequence
<220>
   <223> Synthetic Oligonucleotide
<400> 876
   tcctgcactg ac 12
<210> 877
   <211> 12
   <212> DNA
   <213> Artificial Sequence
<220>
   <223> Synthetic Oligonucleotide
<400> 877
   tcgatctcct cg 12
<210> 878
   <211> 12
   <212> DNA
   <213> Artificial Sequence
<220>
   <223> Synthetic Oligonucleotide
<400> 878
   tgatcctgca ct 12
<210> 879
   <211> 12
   <212> DNA
   <213> Artificial Sequence
<220>
   <223> Synthetic Oligonucleotide
<400> 879
   tgcaatttaa tc 12
<210> 880
   <211> 12
   <212> DNA
   <213> Artificial Sequence
<220>
   <223> Synthetic Oligonucleotide
<400> 880
   tgcactgacg ag 12
<210> 881
   <211> 12
   <212> DNA
   <213> Artificial Sequence
<220>
   <223> Synthetic Oligonucleotide
<400> 881
   tggtcaaaag gg 12
<210> 882
   <211> 12
   <212> DNA
   <213> Artificial Sequence
<220>
   <223> Synthetic Oligonucleotide
<400> 882
   tgtcgatctc ct 12
<210> 883
   <211> 12
   <212> DNA
   <213> Artificial Sequence
<220>
   <223> Synthetic Oligonucleotide
<400> 883
   tgtggtcaaa ag 12
<210> 884
   <211> 12
   <212> DNA
   <213> Artificial Sequence
<220>
   <223> Synthetic Oligonucleotide
<400> 884
   ttaatccgac tg 12
<210> 885
   <211> 12
   <212> DNA
   <213> Artificial Sequence
<220>
   <223> Synthetic Oligonucleotide
<400> 885
   ttcccagcct tg 12
<210> 886
   <211> 12
   <212> DNA
   <213> Artificial Sequence
<220>
   <223> Synthetic Oligonucleotide
<400> 886
   ttgtcgatct cc 12
<210> 887
   <211> 12
   <212> DNA
   <213> Artificial Sequence
<220>
   <223> Synthetic Oligonucleotide
<400> 887
   tttaatccga ct 12
<210> 888
   <211> 12
   <212> DNA
   <213> Artificial Sequence
<220>
   <223> Synthetic Oligonucleotide
<400> 888
   gactgtggtc aa 12
<210> 889
   <211> 12
   <212> DNA
   <213> Artificial Sequence
<220>
   <223> Synthetic Oligonucleotide
<400> 889
   ccgactgtgg tc 12
<210> 890
   <211> 14
   <212> DNA
   <213> Artificial Sequence
<220>
   <223> Synthetic Oligonucleotide
<400> 890
   cccagtgggt ttga 14
<210> 891
   <211> 12
   <212> DNA
   <213> Artificial Sequence
<220>
   <223> Synthetic Oligonucleotide
<400> 891
   ttcttgatgt cc 12
<210> 892
   <211> 12
   <212> DNA
   <213> Artificial Sequence
<220>
   <223> Synthetic Oligonucleotide
<400> 892
   gactccaaag tc 12
<210> 893
   <211> 16
   <212> DNA
   <213> Artificial Sequence
<220>
   <223> Synthetic Oligonucleotide
<400> 893
   tcctgcactg acgagt 16
<210> 894
   <211> 16
   <212> DNA
   <213> Artificial Sequence
<220>
   <223> Synthetic Oligonucleotide
<400> 894
   cactgatcct gcactg 16
<210> 895
   <211> 16
   <212> DNA
   <213> Artificial Sequence
<220>
   <223> Synthetic Oligonucleotide
<400> 895
   cttccactga tcctta 16
<210> 896
   <211> 16
   <212> DNA
   <213> Artificial Sequence
<220>
   <223> Synthetic Oligonucleotide
<400> 896
   gaaagctcct tccact 16
<210> 897
   <211> 14
   <212> DNA
   <213> Artificial Sequence
<220>
   <223> Synthetic Oligonucleotide
<400> 897
   ggcagtcttt atcc 14
<210> 898
   <211> 12
   <212> DNA
   <213> Artificial Sequence
<220>
   <223> Synthetic Oligonucleotide
<400> 898
   ctggtaaata gc 12
<210> 899
   <211> 14
   <212> DNA
   <213> Artificial Sequence
<220>
   <223> Synthetic Oligonucleotide
<400> 899
   ctacctgagg attt 14
<210> 900
   <211> 14
   <212> DNA
   <213> Artificial Sequence
<220>
   <223> Synthetic Oligonucleotide
<400> 900
   cccagtacta cctg 14
<210> 901
   <211> 12
   <212> DNA
   <213> Artificial Sequence
<220>
   <223> Synthetic Oligonucleotide
<400> 901
   tgttccctct ac 12
<210> 902
   <211> 14
   <212> DNA
   <213> Artificial Sequence
<220>
   <223> Synthetic Oligonucleotide
<400> 902
   tattcctgga aaac 14
<210> 903
   <211> 14
   <212> DNA
   <213> Artificial Sequence
<220>
   <223> Synthetic Oligonucleotide
<400> 903
   caagaagtgt ggtt 14
<210> 904
   <211> 14
   <212> DNA
   <213> Artificial Sequence
<220>
   <223> Synthetic Oligonucleotide
<400> 904
   gtgaaaatgc tggc 14
<210> 905
   <211> 14
   <212> DNA
   <213> Artificial Sequence
<220>
   <223> Synthetic Oligonucleotide
<400> 905
   aggaggttaa acca 14
<210> 906
   <211> 12
   <212> DNA
   <213> Artificial Sequence
<220>
   <223> Synthetic Oligonucleotide
<400> 906
   tggatgattg gc 12
<210> 907
   <211> 12
   <212> DNA
   <213> Artificial Sequence
<220>
   <223> Synthetic Oligonucleotide
<400> 907
   caaaaggatc cc 12
<210> 908
   <211> 12
   <212> DNA
   <213> Artificial Sequence
<220>
   <223> Synthetic Oligonucleotide
<400> 908
   atgtcaaccg gc 12
<210> 909
   <211> 12
   <212> DNA
   <213> Artificial Sequence
<220>
   <223> Synthetic Oligonucleotide
<400> 909
   tcagccagac ag 12
<210> 910
   <211> 14
   <212> DNA
   <213> Artificial Sequence
<220>
   <223> Synthetic Oligonucleotide
<400> 910
   taagtgtccc tttg 14
<210> 911
   <211> 14
   <212> DNA
   <213> Artificial Sequence
<220>
   <223> Synthetic Oligonucleotide
<400> 911
   ctaaatttag ttca 14
<210> 912
   <211> 14
   <212> DNA
   <213> Artificial Sequence
<220>
   <223> Synthetic Oligonucleotide
<400> 912
   gactacattt taca 14
<210> 913
   <211> 14
   <212> DNA
   <213> Artificial Sequence
<220>
   <223> Synthetic Oligonucleotide
<400> 913
   cagtaaggaa tttt 14
<210> 914
   <211> 14
   <212> DNA
   <213> Artificial Sequence
<220>
   <223> Synthetic Oligonucleotide
<400> 914
   tgtgtccctc agtc 14
<210> 915
   <211> 12
   <212> DNA
   <213> Artificial Sequence
<220>
   <223> Synthetic Oligonucleotide
<400> 915
   ccgttggacc cc 12
<210> 916
   <211> 14
   <212> DNA
   <213> Artificial Sequence
<220>
   <223> Synthetic Oligonucleotide
<400> 916
   gacagcttct ataa 14
<210> 917
   <211> 14
   <212> DNA
   <213> Artificial Sequence
<220>
   <223> Synthetic Oligonucleotide
<400> 917
   agtgactgac caca 14
<210> 918
   <211> 14
   <212> DNA
   <213> Artificial Sequence
<220>
   <223> Synthetic Oligonucleotide
<400> 918
   gtagcataga gcct 14
<210> 919
   <211> 14
   <212> DNA
   <213> Artificial Sequence
<220>
   <223> Synthetic Oligonucleotide
<400> 919
   cagatcttgt caag 14
<210> 920
   <211> 12
   <212> DNA
   <213> Artificial Sequence
<220>
   <223> Synthetic Oligonucleotide
<400> 920
   gtaagaggca gg 12
<210> 921
   <211> 12
   <212> DNA
   <213> Artificial Sequence
<220>
   <223> Synthetic Oligonucleotide
<400> 921
   ccatggcggg ac 12
<210> 922
   <211> 12
   <212> DNA
   <213> Artificial Sequence
<220>
   <223> Synthetic Oligonucleotide
<400> 922
   gcttacgatt gt 12
<210> 923
   <211> 14
   <212> DNA
   <213> Artificial Sequence
<220>
   <223> Synthetic Oligonucleotide
<400> 923
   ccagcacact ggaa 14
<210> 924
   <211> 14
   <212> DNA
   <213> Artificial Sequence
<220>
   <223> Synthetic Oligonucleotide
<400> 924
   gtcagtccca gcta 14
<210> 925
   <211> 14
   <212> DNA
   <213> Artificial Sequence
<220>
   <223> Synthetic Oligonucleotide
<400> 925
   ttagtatgac agct 14
<210> 926
   <211> 14
   <212> DNA
   <213> Artificial Sequence
<220>
   <223> Synthetic Oligonucleotide
<400> 926
   tcagtgtagg aaga 14
<210> 927
   <211> 14
   <212> DNA
   <213> Artificial Sequence
<220>
   <223> Synthetic Oligonucleotide
<400> 927
   tgaatataca gatg 14
<210> 928
   <211> 12
   <212> DNA
   <213> Artificial Sequence
<220>
   <223> Synthetic Oligonucleotide
<400> 928
   cccgccacca cc 12
<210> 929
   <211> 12
   <212> DNA
   <213> Artificial Sequence
<220>
   <223> Synthetic Oligonucleotide
<400> 929
   ttgttccctc ta 12
<210> 930
   <211> 14
   <212> DNA
   <213> Artificial Sequence
<220>
   <223> Synthetic Oligonucleotide
<400> 930
   tctacctgag tcca 14
<210> 931
   <211> 14
   <212> DNA
   <213> Artificial Sequence
<220>
   <223> Synthetic Oligonucleotide
<400> 931
   aacatcaagc ttga 14
<210> 932
   <211> 14
   <212> DNA
   <213> Artificial Sequence
<220>
   <223> Synthetic Oligonucleotide
<400> 932
   gatcaccttc agag 14
<210> 933
   <211> 14
   <212> DNA
   <213> Artificial Sequence
<220>
   <223> Synthetic Oligonucleotide
<400> 933
   tgaacacatc acta 14
<210> 934
   <211> 12
   <212> DNA
   <213> Artificial Sequence
<220>
   <223> Synthetic Oligonucleotide
<400> 934
   tttcctcttg tc 12
<210> 935
   <211> 14
   <212> DNA
   <213> Artificial Sequence
<220>
   <223> Synthetic Oligonucleotide
<400> 935
   taattgatgt caat 14
<210> 936
   <211> 14
   <212> DNA
   <213> Artificial Sequence
<220>
   <223> Synthetic Oligonucleotide
<400> 936
   agtaattgat gtca 14
<210> 937
   <211> 14
   <212> DNA
   <213> Artificial Sequence
<220>
   <223> Synthetic Oligonucleotide
<400> 937
   acagtaattg atgt 14
<210> 938
   <211> 14
   <212> DNA
   <213> Artificial Sequence
<220>
   <223> Synthetic Oligonucleotide
<400> 938
   ttacagtaat tgat 14
<210> 939
   <211> 14
   <212> DNA
   <213> Artificial Sequence
<220>
   <223> Synthetic Oligonucleotide
<400> 939
   acttacagta attg 14
<210> 940
   <211> 14
   <212> DNA
   <213> Artificial Sequence
<220>
   <223> Synthetic Oligonucleotide
<400> 940
   agacttacag taat 14
<210> 941
   <211> 14
   <212> DNA
   <213> Artificial Sequence
<220>
   <223> Synthetic Oligonucleotide
<400> 941
   tcagacttac agta 14
<210> 942
   <211> 14
   <212> DNA
   <213> Artificial Sequence
<220>
   <223> Synthetic Oligonucleotide
<400> 942
   aatcagactt acag 14
<210> 943
   <211> 14
   <212> DNA
   <213> Artificial Sequence
<220>
   <223> Synthetic Oligonucleotide
<400> 943
   tgaatcagac ttac 14
<210> 944
   <211> 14
   <212> DNA
   <213> Artificial Sequence
<220>
   <223> Synthetic Oligonucleotide
<400> 944
   aatgaatcag actt 14
<210> 945
   <211> 14
   <212> DNA
   <213> Artificial Sequence
<220>
   <223> Synthetic Oligonucleotide
<400> 945
   tgcaggatgt tgag 14
<210> 946
   <211> 14
   <212> DNA
   <213> Artificial Sequence
<220>
   <223> Synthetic Oligonucleotide
<400> 946
   ctggtcagca ttga 14
<210> 947
   <211> 14
   <212> DNA
   <213> Artificial Sequence
<220>
   <223> Synthetic Oligonucleotide
<400> 947
   caaagtccct tagc 14
<210> 948
   <211> 14
   <212> DNA
   <213> Artificial Sequence
<220>
   <223> Synthetic Oligonucleotide
<400> 948
   atttctctta cagg 14
<210> 949
   <211> 12
   <212> DNA
   <213> Artificial Sequence
<220>
   <223> Synthetic Oligonucleotide
<400> 949
   ttaacgagcc tt 12
<210> 950
   <211> 12
   <212> DNA
   <213> Artificial Sequence
<220>
   <223> Synthetic Oligonucleotide
<400> 950
   cgacacggga ac 12
<210> 951
   <211> 12
   <212> DNA
   <213> Artificial Sequence
<220>
   <223> Synthetic Oligonucleotide
<400> 951
   caagtaggat gt 12
<210> 952
   <211> 14
   <212> DNA
   <213> Artificial Sequence
<220>
   <223> Synthetic Oligonucleotide
<400> 952
   gttccctttg cagg 14
<210> 953
   <211> 14
   <212> DNA
   <213> Artificial Sequence
<220>
   <223> Synthetic Oligonucleotide
<400> 953
   attagccata tctc 14
<210> 954
   <211> 14
   <212> DNA
   <213> Artificial Sequence
<220>
   <223> Synthetic Oligonucleotide
<400> 954
   agcattcagc agtg 14
<210> 955
   <211> 12
   <212> DNA
   <213> Artificial Sequence
<220>
   <223> Synthetic Oligonucleotide
<400> 955
   ttccctctac ac 12
<210> 956
   <211> 12
   <212> DNA
   <213> Artificial Sequence
<220>
   <223> Synthetic Oligonucleotide
<400> 956
   tccctctaca cc 12
<210> 957
   <211> 14
   <212> DNA
   <213> Artificial Sequence
<220>
   <223> Synthetic Oligonucleotide
<400> 957
   ctacaggaca atac 14
<210> 958
   <211> 14
   <212> DNA
   <213> Artificial Sequence
<220>
   <223> Synthetic Oligonucleotide
<400> 958
   aactgggtta agta 14
<210> 959
   <211> 14
   <212> DNA
   <213> Artificial Sequence
<220>
   <223> Synthetic Oligonucleotide
<400> 959
   tgaacacgct atcc 14
<210> 960
   <211> 14
   <212> DNA
   <213> Artificial Sequence
<220>
   <223> Synthetic Oligonucleotide
<400> 960
   tttccacttg ggtg 14
<210> 961
   <211> 12
   <212> DNA
   <213> Artificial Sequence
<220>
   <223> Synthetic Oligonucleotide
<400> 961
   tctacaccag gt 12
<210> 962
   <211> 14
   <212> DNA
   <213> Artificial Sequence
<220>
   <223> Synthetic Oligonucleotide
<400> 962
   gaaagctcct tcca 14
<210> 963
   <211> 12
   <212> DNA
   <213> Artificial Sequence
<220>
   <223> Synthetic Oligonucleotide
<400> 963
   aggtaggaga ag 12
<210> 964
   <211> 12
   <212> DNA
   <213> Artificial Sequence
<220>
   <223> Synthetic Oligonucleotide
<400> 964
   acccagtcag gg 12
<210> 965
   <211> 12
   <212> DNA
   <213> Artificial Sequence
<220>
   <223> Synthetic Oligonucleotide
<400> 965
   atgtcattaa ac 12
<210> 966
   <211> 12
   <212> DNA
   <213> Artificial Sequence
<220>
   <223> Synthetic Oligonucleotide
<400> 966
   gaggtgggaa aa 12
<210> 967
   <211> 14
   <212> DNA
   <213> Artificial Sequence
<220>
   <223> Synthetic Oligonucleotide
<400> 967
   tttcctagga ggtg 14
<210> 968
   <211> 12
   <212> DNA
   <213> Artificial Sequence
<220>
   <223> Synthetic Oligonucleotide
<400> 968
   gttcttagga ag 12
<210> 969
   <211> 12
   <212> DNA
   <213> Artificial Sequence
<220>
   <223> Synthetic Oligonucleotide
<400> 969
   ctctacacca gg 12
<210> 970
   <211> 14
   <212> DNA
   <213> Artificial Sequence
<220>
   <223> Synthetic Oligonucleotide
<400> 970
   tgtttttaca caga 14
<210> 971
   <211> 12
   <212> DNA
   <213> Artificial Sequence
<220>
   <223> Synthetic Oligonucleotide
<400> 971
   tggcttcatg tc 12
<210> 972
   <211> 14
   <212> DNA
   <213> Artificial Sequence
<220>
   <223> Synthetic Oligonucleotide
<400> 972
   ttgttcttag gaag 14
<210> 973
   <211> 12
   <212> DNA
   <213> Artificial Sequence
<220>
   <223> Synthetic Oligonucleotide
<400> 973
   tacaccaggt ca 12
<210> 974
   <211> 14
   <212> DNA
   <213> Artificial Sequence
<220>
   <223> Synthetic Oligonucleotide
<400> 974
   tgagctacag tagg 14
<210> 975
   <211> 12
   <212> DNA
   <213> Artificial Sequence
<220>
   <223> Synthetic Oligonucleotide
<400> 975
   ggctgacatt ca 12
<210> 976
   <211> 14
   <212> DNA
   <213> Artificial Sequence
<220>
   <223> Synthetic Oligonucleotide
<400> 976
   tggtcaactg aaag 14
<210> 977
   <211> 12
   <212> DNA
   <213> Artificial Sequence
<220>
   <223> Synthetic Oligonucleotide
<400> 977
   tagtcattat ct 12
<210> 978
   <211> 12
   <212> DNA
   <213> Artificial Sequence
<220>
   <223> Synthetic Oligonucleotide
<400> 978
   ccatttttat ca 12
<210> 979
   <211> 14
   <212> DNA
   <213> Artificial Sequence
<220>
   <223> Synthetic Oligonucleotide
<400> 979
   gggcttcttc catt 14
<210> 980
   <211> 14
   <212> DNA
   <213> Artificial Sequence
<220>
   <223> Synthetic Oligonucleotide
<400> 980
   ggtgtggata acag 14
<210> 981
   <211> 14
   <212> DNA
   <213> Artificial Sequence
<220>
   <223> Synthetic Oligonucleotide
<400> 981
   tcataactat taag 14
<210> 982
   <211> 12
   <212> DNA
   <213> Artificial Sequence
<220>
   <223> Synthetic Oligonucleotide
<400> 982
   cctctacacc ag 12
<210> 983
   <211> 14
   <212> DNA
   <213> Artificial Sequence
<220>
   <223> Synthetic Oligonucleotide
<400> 983
   cagcttaggc agag 14
<210> 984
   <211> 12
   <212> DNA
   <213> Artificial Sequence
<220>
   <223> Synthetic Oligonucleotide
<400> 984
   tcattcccca ct 12
<210> 985
   <211> 12
   <212> DNA
   <213> Artificial Sequence
<220>
   <223> Synthetic Oligonucleotide
<400> 985
   ctcccacacc at 12
<210> 986
   <211> 12
   <212> DNA
   <213> Artificial Sequence
<220>
   <223> Synthetic Oligonucleotide
<400> 986
   ttctgctccc ac 12
<210> 987
   <211> 12
   <212> DNA
   <213> Artificial Sequence
<220>
   <223> Synthetic Oligonucleotide
<400> 987
   cagagaaggt ct 12
<210> 988
   <211> 12
   <212> DNA
   <213> Artificial Sequence
<220>
   <223> Synthetic Oligonucleotide
<400> 988
   gtatgcactg ct 12
<210> 989
   <211> 14
   <212> DNA
   <213> Artificial Sequence
<220>
   <223> Synthetic Oligonucleotide
<400> 989
   caatgaagca cagg 14
<210> 990
   <211> 12
   <212> DNA
   <213> Artificial Sequence
<220>
   <223> Synthetic Oligonucleotide
<400> 990
   tcccaaacaa at 12
<210> 991
   <211> 14
   <212> DNA
   <213> Artificial Sequence
<220>
   <223> Synthetic Oligonucleotide
<400> 991
   attcttaaca caga 14
<210> 992
   <211> 12
   <212> DNA
   <213> Artificial Sequence
<220>
   <223> Synthetic Oligonucleotide
<400> 992
   cgggtactat gg 12
<210> 993
   <211> 12
   <212> DNA
   <213> Artificial Sequence
<220>
   <223> Synthetic Oligonucleotide
<400> 993
   ctacaccagg tc 12
<210> 994
   <211> 12
   <212> DNA
   <213> Artificial Sequence
<220>
   <223> Synthetic Oligonucleotide
<400> 994
   tatagctcct ct 12
<210> 995
   <211> 14
   <212> DNA
   <213> Artificial Sequence
<220>
   <223> Synthetic Oligonucleotide
<400> 995
   catttagggt ctaa 14
<210> 996
   <211> 12
   <212> DNA
   <213> Artificial Sequence
<220>
   <223> Synthetic Oligonucleotide
<400> 996
   atcttcagag at 12
<210> 997
   <211> 12
   <212> DNA
   <213> Artificial Sequence
<220>
   <223> Synthetic Oligonucleotide
<400> 997
   ttgatatagt ca 12
<210> 998
   <211> 12
   <212> DNA
   <213> Artificial Sequence
<220>
   <223> Synthetic Oligonucleotide
<400> 998
   taatatgact tg 12
<210> 999
   <211> 14
   <212> DNA
   <213> Artificial Sequence
<220>
   <223> Synthetic Oligonucleotide
<400> 999
   agccgcctga agtg 14
<210> 1000
   <211> 12
   <212> DNA
   <213> Artificial Sequence
<220>
   <223> Synthetic Oligonucleotide
<400> 1000
   ccccagcagc gg 12
<210> 1001
   <211> 12
   <212> DNA
   <213> Artificial Sequence
<220>
   <223> Synthetic Oligonucleotide
<400> 1001
   tccttccact ga 12
<210> 1002
   <211> 12
   <212> DNA
   <213> Artificial Sequence
<220>
   <223> Synthetic Oligonucleotide
<400> 1002
   cggtttttgt tc 12
<210> 1003
   <211> 14
   <212> DNA
   <213> Artificial Sequence
<220>
   <223> Synthetic Oligonucleotide
<400> 1003
   taaatcctct agca 14
<210> 1004
   <211> 12
   <212> DNA
   <213> Artificial Sequence
<220>
   <223> Synthetic Oligonucleotide
<400> 1004
   gttccctcta ca 12
<210> 1005
   <211> 12
   <212> DNA
   <213> Artificial Sequence
<220>
   <223> Synthetic Oligonucleotide
<400> 1005
   acaccatctc cc 12
<210> 1006
   <211> 12
   <212> DNA
   <213> Artificial Sequence
<220>
   <223> Synthetic Oligonucleotide
<400> 1006
   gctccttcca ct 12
<210> 1007
   <211> 14
   <212> DNA
   <213> Artificial Sequence
<220>
   <223> Synthetic Oligonucleotide
<400> 1007
   cactgatcct gcac 14
<210> 1008
   <211> 12
   <212> DNA
   <213> Artificial Sequence
<220>
   <223> Synthetic Oligonucleotide
<400> 1008
   tcggactttg aa 12
<210> 1009
   <211> 14
   <212> DNA
   <213> Artificial Sequence
<220>
   <223> Synthetic Oligonucleotide
<400> 1009
   tcggactttg aaaa 14
<210> 1010
   <211> 14
   <212> DNA
   <213> Artificial Sequence
<220>
   <223> Synthetic Oligonucleotide
<400> 1010
   atcagccaga caga 14
<210> 1011
   <211> 14
   <212> DNA
   <213> Artificial Sequence
<220>
   <223> Synthetic Oligonucleotide
<400> 1011
   catcagcaag aggc 14
<210> 1012
   <211> 12
   <212> DNA
   <213> Artificial Sequence
<220>
   <223> Synthetic Oligonucleotide
<400> 1012
   ttgttcttag ga 12
<210> 1013
   <211> 12
   <212> DNA
   <213> Artificial Sequence
<220>
   <223> Synthetic Oligonucleotide
<400> 1013
   gccagacaga ag 12
<210> 1014
   <211> 12
   <212> DNA
   <213> Artificial Sequence
<220>
   <223> Synthetic Oligonucleotide
<400> 1014
   ttgtcgatct gc 12
<210> 1015
   <211> 14
   <212> DNA
   <213> Artificial Sequence
<220>
   <223> Synthetic Oligonucleotide
<400> 1015
   ggagaagcgc agct 14
<210> 1016
   <211> 12
   <212> DNA
   <213> Artificial Sequence
<220>
   <223> Synthetic Oligonucleotide
<400> 1016
   ggagaagcgc ag 12
<210> 1017
   <211> 14
   <212> DNA
   <213> Artificial Sequence
<220>
   <223> Synthetic Oligonucleotide
<400> 1017
   ctgcactgac gagt 14
<210> 1018
   <211> 12
   <212> DNA
   <213> Artificial Sequence
<220>
   <223> Synthetic Oligonucleotide
<400> 1018
   ctgcaacatg at 12
<210> 1019
   <211> 14
   <212> DNA
   <213> Artificial Sequence
<220>
   <223> Synthetic Oligonucleotide
<400> 1019
   ctgatcctta gaag 14
<210> 1020
   <211> 16
   <212> DNA
   <213> Artificial Sequence
<220>
   <223> Synthetic Oligonucleotide
<400> 1020
   ccttccactg atcctg 16
<210> 1021
   <211> 12
   <212> DNA
   <213> Artificial Sequence
<220>
   <223> Synthetic Oligonucleotide
<400> 1021
   ctccttccac tg 12
<210> 1022
   <211> 14
   <212> DNA
   <213> Artificial Sequence
<220>
   <223> Synthetic Oligonucleotide
<400> 1022
   tgcagccatg tact 14
<210> 1023
   <211> 12
   <212> DNA
   <213> Artificial Sequence
<220>
   <223> Synthetic Oligonucleotide
<400> 1023
   cgtttgggtg gc 12
<210> 1024
   <211> 14
   <212> DNA
   <213> Artificial Sequence
<220>
   <223> Synthetic Oligonucleotide
<400> 1024
   tccactgatc ctgc 14
<210> 1025
   <211> 14
   <212> DNA
   <213> Artificial Sequence
<220>
   <223> Synthetic Oligonucleotide
<400> 1025
   ttccactgat cctg 14
<210> 1026
   <211> 12
   <212> DNA
   <213> Artificial Sequence
<220>
   <223> Synthetic Oligonucleotide
<400> 1026
   tccactgatc ct 12
<210> 1027
   <211> 14
   <212> DNA
   <213> Artificial Sequence
<220>
   <223> Synthetic Oligonucleotide
<400> 1027
   cttccactga tcct 14
<210> 1028
   <211> 16
   <212> DNA
   <213> Artificial Sequence
<220>
   <223> Synthetic Oligonucleotide
<400> 1028
   tccttccact gatcct 16
<210> 1029
   <211> 12
   <212> DNA
   <213> Artificial Sequence
<220>
   <223> Synthetic Oligonucleotide
<400> 1029
   ttccactgat cc 12
<210> 1030
   <211> 16
   <212> DNA
   <213> Artificial Sequence
<220>
   <223> Synthetic Oligonucleotide
<400> 1030
   ctccttccac tgatcc 16
<210> 1031
   <211> 14
   <212> DNA
   <213> Artificial Sequence
<220>
   <223> Synthetic Oligonucleotide
<400> 1031
   tccttccact gatc 14
<210> 1032
   <211> 16
   <212> DNA
   <213> Artificial Sequence
<220>
   <223> Synthetic Oligonucleotide
<400> 1032
   gctccttcca ctgatc 16
<210> 1033
   <211> 14
   <212> DNA
   <213> Artificial Sequence
<220>
   <223> Synthetic Oligonucleotide
<400> 1033
   gctccttcca ctga 14
<210> 1034
   <211> 16
   <212> DNA
   <213> Artificial Sequence
<220>
   <223> Synthetic Oligonucleotide
<400> 1034
   aagctccttc cactga 16
<210> 1035
   <211> 14
   <212> DNA
   <213> Artificial Sequence
<220>
   <223> Synthetic Oligonucleotide
<400> 1035
   aagctccttc cact 14
<210> 1036
   <211> 12
   <212> DNA
   <213> Artificial Sequence
<220>
   <223> Synthetic Oligonucleotide
<400> 1036
   agctccttcc ac 12
<210> 1037
   <211> 16
   <212> DNA
   <213> Artificial Sequence
<220>
   <223> Synthetic Oligonucleotide
<400> 1037
   gggaaagctc cttcca 16
<210> 1038
   <211> 14
   <212> DNA
   <213> Artificial Sequence
<220>
   <223> Synthetic Oligonucleotide
<400> 1038
   ttgcaatgtc tggc 14
<210> 1039
   <211> 14
   <212> DNA
   <213> Artificial Sequence
<220>
   <223> Synthetic Oligonucleotide
<400> 1039
   gatttatctg gctg 14
<210> 1040
   <211> 12
   <212> DNA
   <213> Artificial Sequence
<220>
   <223> Synthetic Oligonucleotide
<400> 1040
   aagggccctg gg 12
<210> 1041
   <211> 12
   <212> DNA
   <213> Artificial Sequence
<220>
   <223> Synthetic Oligonucleotide
<400> 1041
   aacttcagtg tc 12
<210> 1042
   <211> 12
   <212> DNA
   <213> Artificial Sequence
<220>
   <223> Synthetic Oligonucleotide
<400> 1042
   agggcttcca gt 12
<210> 1043
   <211> 12
   <212> DNA
   <213> Artificial Sequence
<220>
   <223> Synthetic Oligonucleotide
<400> 1043
   aggaagggct tc 12
<210> 1044
   <211> 12
   <212> DNA
   <213> Artificial Sequence
<220>
   <223> Synthetic Oligonucleotide
<400> 1044
   ccttccactg at 12
<210> 1045
   <211> 14
   <212> DNA
   <213> Artificial Sequence
<220>
   <223> Synthetic Oligonucleotide
<400> 1045
   ggaaacatac cctg 14
<210> 1046
   <211> 14
   <212> DNA
   <213> Artificial Sequence
<220>
   <223> Synthetic Oligonucleotide
<400> 1046
   ccttccactg atcc 14
<210> 1047
   <211> 12
   <212> DNA
   <213> Artificial Sequence
<220>
   <223> Synthetic Oligonucleotide
<400> 1047
   cttccactga tc 12
<210> 1048
   <211> 14
   <212> DNA
   <213> Artificial Sequence
<220>
   <223> Synthetic Oligonucleotide
<400> 1048
   gaataggtta aggc 14
<210> 1049
   <211> 14
   <212> DNA
   <213> Artificial Sequence
<220>
   <223> Synthetic Oligonucleotide
<400> 1049
   aacaatgtgt tgta 14
<210> 1050
   <211> 12
   <212> DNA
   <213> Artificial Sequence
<220>
   <223> Synthetic Oligonucleotide
<400> 1050
   ccctctacac ca 12
<210> 1051
   <211> 14
   <212> DNA
   <213> Artificial Sequence
<220>
   <223> Synthetic Oligonucleotide
<400> 1051
   gcacacagct gagg 14
<210> 1052
   <211> 14
   <212> DNA
   <213> Artificial Sequence
<220>
   <223> Synthetic Oligonucleotide
<400> 1052
   gtgcacacag ctga 14
<210> 1053
   <211> 14
   <212> DNA
   <213> Artificial Sequence
<220>
   <223> Synthetic Oligonucleotide
<400> 1053
   cagtgtgcac acag 14
<210> 1054
   <211> 14
   <212> DNA
   <213> Artificial Sequence
<220>
   <223> Synthetic Oligonucleotide
<400> 1054
   ctcagtgtgc acac 14
<210> 1055
   <211> 12
   <212> DNA
   <213> Artificial Sequence
<220>
   <223> Synthetic Oligonucleotide
<400> 1055
   cacccactgg tg 12
<210> 1056
   <211> 14
   <212> DNA
   <213> Artificial Sequence
<220>
   <223> Synthetic Oligonucleotide
<400> 1056
   catttccatg gcca 14
<210> 1057
   <211> 14
   <212> DNA
   <213> Artificial Sequence
<220>
   <223> Synthetic Oligonucleotide
<400> 1057
   accaaacagt tcag 14
<210> 1058
   <211> 12
   <212> DNA
   <213> Artificial Sequence
<220>
   <223> Synthetic Oligonucleotide
<400> 1058
   ttgaccagga ag 12
<210> 1059
   <211> 12
   <212> DNA
   <213> Artificial Sequence
<220>
   <223> Synthetic Oligonucleotide
<400> 1059
   caggccatgt gg 12
<210> 1060
   <211> 14
   <212> DNA
   <213> Artificial Sequence
<220>
   <223> Synthetic Oligonucleotide
<400> 1060
   agtcaggcca tgtg 14
<210> 1061
   <211> 12
   <212> DNA
   <213> Artificial Sequence
<220>
   <223> Synthetic Oligonucleotide
<400> 1061
   gcgcgagccc ga 12
<210> 1062
   <211> 12
   <212> DNA
   <213> Artificial Sequence
<220>
   <223> Synthetic Oligonucleotide
<400> 1062
   tgtgaggctc ca 12
<210> 1063
   <211> 12
   <212> DNA
   <213> Artificial Sequence
<220>
   <223> Synthetic Oligonucleotide
<400> 1063
   ccctgaaggt tc 12
<210> 1064
   <211> 14
   <212> DNA
   <213> Artificial Sequence
<220>
   <223> Synthetic Oligonucleotide
<400> 1064
   tttgataaag ccct 14
<210> 1065
   <211> 14
   <212> DNA
   <213> Artificial Sequence
<220>
   <223> Synthetic Oligonucleotide
<400> 1065
   caagaagacc ttac 14
<210> 1066
   <211> 14
   <212> DNA
   <213> Artificial Sequence
<220>
   <223> Synthetic Oligonucleotide
<400> 1066
   tgcacaggca ggtt 14
<210> 1067
   <211> 12
   <212> DNA
   <213> Artificial Sequence
<220>
   <223> Synthetic Oligonucleotide
<400> 1067
   acagccaggt ag 12
<210> 1068
   <211> 14
   <212> DNA
   <213> Artificial Sequence
<220>
   <223> Synthetic Oligonucleotide
<400> 1068
   tggaaaactg cacc 14
<210> 1069
   <211> 12
   <212> DNA
   <213> Artificial Sequence
<220>
   <223> Synthetic Oligonucleotide
<400> 1069
   tgggtggccg gg 12
<210> 1070
   <211> 12
   <212> DNA
   <213> Artificial Sequence
<220>
   <223> Synthetic Oligonucleotide
<400> 1070
   gggcttcttc ca 12
<210> 1071
   <211> 12
   <212> DNA
   <213> Artificial Sequence
<220>
   <223> Synthetic Oligonucleotide
<400> 1071
   gctgacatct cg 12
<210> 1072
   <211> 14
   <212> DNA
   <213> Artificial Sequence
<220>
   <223> Synthetic Oligonucleotide
<400> 1072
   cagcctggca ccta 14
<210> 1073
   <211> 12
   <212> DNA
   <213> Artificial Sequence
<220>
   <223> Synthetic Oligonucleotide
<400> 1073
   taaaaacaac aa 12
<210> 1074
   <211> 12
   <212> DNA
   <213> Artificial Sequence
<220>
   <223> Synthetic Oligonucleotide
<400> 1074
   tttataaaac tg 12
<210> 1075
   <211> 4
   <212> DNA
   <213> Artificial Sequence
<220>
   <223> Synthetic Oligonucleotide
<400> 1075
   actg 4
<210> 1076
   <211> 14
   <212> DNA
   <213> Artificial Sequence
<220>
   <223> Synthetic Oligonucleotide
<400> 1076
   agtaaatatt ggct 14
<210> 1077
   <211> 12
   <212> DNA
   <213> Artificial Sequence
<220>
   <223> Synthetic Oligonucleotide
<400> 1077
   gttgagcatg ac 12
<210> 1078
   <211> 12
   <212> DNA
   <213> Artificial Sequence
<220>
   <223> Synthetic Oligonucleotide
<400> 1078
   gtgcgctccc at 12
<210> 1079
   <211> 14
   <212> DNA
   <213> Artificial Sequence
<220>
   <223> Synthetic Oligonucleotide
<400> 1079
   tcctgcactg acga 14
<210> 1080
   <211> 16
   <212> DNA
   <213> Artificial Sequence
<220>
   <223> Synthetic Oligonucleotide
<400> 1080
   gatcctgcac tgacga 16
<210> 1081
   <211> 14
   <212> DNA
   <213> Artificial Sequence
<220>
   <223> Synthetic Oligonucleotide
<400> 1081
   gatcctgcac tgac 14
<210> 1082
   <211> 16
   <212> DNA
   <213> Artificial Sequence
<220>
   <223> Synthetic Oligonucleotide
<400> 1082
   ctgatcctgc actgac 16
<210> 1083
   <211> 16
   <212> DNA
   <213> Artificial Sequence
<220>
   <223> Synthetic Oligonucleotide
<400> 1083
   tccactgatc ctgcac 16
<210> 1084
   <211> 14
   <212> DNA
   <213> Artificial Sequence
<220>
   <223> Synthetic Oligonucleotide
<400> 1084
   cgcgagatat ctaa 14
<210> 1085
   <211> 14
   <212> DNA
   <213> Artificial Sequence
<220>
   <223> Synthetic Oligonucleotide
<400> 1085
   cgcacctggt aaat 14
<210> 1086
   <211> 14
   <212> DNA
   <213> Artificial Sequence
<220>
   <223> Synthetic Oligonucleotide
<400> 1086
   gttcaagcgg ccta 14
<210> 1087
   <211> 14
   <212> DNA
   <213> Artificial Sequence
<220>
   <223> Synthetic Oligonucleotide
<400> 1087
   ccctttacac aagt 14
<210> 1088
   <211> 14
   <212> DNA
   <213> Artificial Sequence
<220>
   <223> Synthetic Oligonucleotide
<400> 1088
   ctcaaaatag attt 14
<210> 1089
   <211> 14
   <212> DNA
   <213> Artificial Sequence
<220>
   <223> Synthetic Oligonucleotide
<400> 1089
   cacatgagct attc 14
<210> 1090
   <211> 14
   <212> DNA
   <213> Artificial Sequence
<220>
   <223> Synthetic Oligonucleotide
<400> 1090
   gtgaagtgag tcat 14
<210> 1091
   <211> 14
   <212> DNA
   <213> Artificial Sequence
<220>
   <223> Synthetic Oligonucleotide
<400> 1091
   ggtcactcaa gatg 14
<210> 1092
   <211> 14
   <212> DNA
   <213> Artificial Sequence
<220>
   <223> Synthetic Oligonucleotide
<400> 1092
   ggactcactc agca 14
<210> 1093
   <211> 14
   <212> DNA
   <213> Artificial Sequence
<220>
   <223> Synthetic Oligonucleotide
<400> 1093
   tcagggctac tcat 14
<210> 1094
   <211> 14
   <212> DNA
   <213> Artificial Sequence
<220>
   <223> Synthetic Oligonucleotide
<400> 1094
   cagcactaga ttca 14
<210> 1095
   <211> 14
   <212> DNA
   <213> Artificial Sequence
<220>
   <223> Synthetic Oligonucleotide
<400> 1095
   tagcttaatg taac 14
<210> 1096
   <211> 14
   <212> DNA
   <213> Artificial Sequence
<220>
   <223> Synthetic Oligonucleotide
<400> 1096
   ttgtgacatc tagg 14
<210> 1097
   <211> 14
   <212> DNA
   <213> Artificial Sequence
<220>
   <223> Synthetic Oligonucleotide
<400> 1097
   gtgcctagca caga 14
<210> 1098
   <211> 12
   <212> DNA
   <213> Artificial Sequence
<220>
   <223> Synthetic Oligonucleotide
<400> 1098
   cagcctacca gt 12
<210> 1099
   <211> 14
   <212> DNA
   <213> Artificial Sequence
<220>
   <223> Synthetic Oligonucleotide
<400> 1099
   acatgtcagt aatt 14
<210> 1100
   <211> 14
   <212> DNA
   <213> Artificial Sequence
<220>
   <223> Synthetic Oligonucleotide
<400> 1100
   ctcatggaca caaa 14
<210> 1101
   <211> 14
   <212> DNA
   <213> Artificial Sequence
<220>
   <223> Synthetic Oligonucleotide
<400> 1101
   ggaagttttc aagt 14
<210> 1102
   <211> 14
   <212> DNA
   <213> Artificial Sequence
<220>
   <223> Synthetic Oligonucleotide
<400> 1102
   gaatctggag gtaa 14
<210> 1103
   <211> 14
   <212> DNA
   <213> Artificial Sequence
<220>
   <223> Synthetic Oligonucleotide
<400> 1103
   agccccttgg ccgt 14
<210> 1104
   <211> 14
   <212> DNA
   <213> Artificial Sequence
<220>
   <223> Synthetic Oligonucleotide
<400> 1104
   gaatacttca aatc 14
<210> 1105
   <211> 14
   <212> DNA
   <213> Artificial Sequence
<220>
   <223> Synthetic Oligonucleotide
<400> 1105
   ctgatcctgc actg 14
<210> 1106
   <211> 14
   <212> DNA
   <213> Artificial Sequence
<220>
   <223> Synthetic Oligonucleotide
<400> 1106
   tttgaggagc tatt 14
<210> 1107
   <211> 12
   <212> DNA
   <213> Artificial Sequence
<220>
   <223> Synthetic Oligonucleotide
<400> 1107
   ttgttgttcc ct 12
<210> 1108
   <211> 12
   <212> DNA
   <213> Artificial Sequence
<220>
   <223> Synthetic Oligonucleotide
<400> 1108
   gagttgttgt tc 12
<210> 1109
   <211> 12
   <212> DNA
   <213> Artificial Sequence
<220>
   <223> Synthetic Oligonucleotide
<400> 1109
   cgagttgttg tt 12
<210> 1110
   <211> 12
   <212> DNA
   <213> Artificial Sequence
<220>
   <223> Synthetic Oligonucleotide
<400> 1110
   gcgctaggcc gc 12
<210> 1111
   <211> 12
   <212> DNA
   <213> Artificial Sequence
<220>
   <223> Synthetic Oligonucleotide
<400> 1111
   gttgttgttc cc 12
<210> 1112
   <211> 12
   <212> DNA
   <213> Artificial Sequence
<220>
   <223> Synthetic Oligonucleotide
<400> 1112
   agttgttgtt cc 12
<210> 1113
   <211> 12
   <212> DNA
   <213> Artificial Sequence
<220>
   <223> Synthetic Oligonucleotide
<400> 1113
   ctcaccttca tg 12
<210> 1114
   <211> 14
   <212> DNA
   <213> Artificial Sequence
<220>
   <223> Synthetic Oligonucleotide
<400> 1114
   cttagaaggc agca 14
<210> 1115
   <211> 12
   <212> DNA
   <213> Artificial Sequence
<220>
   <223> Synthetic Oligonucleotide
<400> 1115
   gttgttccct ct 12
<210> 1116
   <211> 12
   <212> DNA
   <213> Artificial Sequence
<220>
   <223> Synthetic Oligonucleotide
<400> 1116
   ccaactccaa ct 12
<210> 1117
   <211> 12
   <212> DNA
   <213> Artificial Sequence
<220>
   <223> Synthetic Oligonucleotide
<400> 1117
   gatctctcga gt 12
<210> 1118
   <211> 12
   <212> DNA
   <213> Artificial Sequence
<220>
   <223> Synthetic Oligonucleotide
<400> 1118
   aatgcaggat ct 12
<210> 1119
   <211> 12
   <212> DNA
   <213> Artificial Sequence
<220>
   <223> Synthetic Oligonucleotide
<400> 1119
   ctcggacttt ga 12
<210> 1120
   <211> 12
   <212> DNA
   <213> Artificial Sequence
<220>
   <223> Synthetic Oligonucleotide
<400> 1120
   tgactctcgg ac 12
<210> 1121
   <211> 12
   <212> DNA
   <213> Artificial Sequence
<220>
   <223> Synthetic Oligonucleotide
<400> 1121
   cttgtccatc ag 12
<210> 1122
   <211> 12
   <212> DNA
   <213> Artificial Sequence
<220>
   <223> Synthetic Oligonucleotide
<400> 1122
   gggtctttcc tc 12
<210> 1123
   <211> 16
   <212> DNA
   <213> Artificial Sequence
<220>
   <223> Synthetic Oligonucleotide
<400> 1123
   cactgatcct tagaag 16
<210> 1124
   <211> 14
   <212> DNA
   <213> Artificial Sequence
<220>
   <223> Synthetic Oligonucleotide
<400> 1124
   cactgatcct taga 14
<210> 1125
   <211> 16
   <212> DNA
   <213> Artificial Sequence
<220>
   <223> Synthetic Oligonucleotide
<400> 1125
   tccactgatc cttaga 16
<210> 1126
   <211> 16
   <212> DNA
   <213> Artificial Sequence
<220>
   <223> Synthetic Oligonucleotide
<400> 1126
   cttccactga tcctgc 16
<210> 1127
   <211> 14
   <212> DNA
   <213> Artificial Sequence
<220>
   <223> Synthetic Oligonucleotide
<400> 1127
   tccactgatc ctta 14
<210> 1128
   <211> 12
   <212> DNA
   <213> Artificial Sequence
<220>
   <223> Synthetic Oligonucleotide
<400> 1128
   cagtggacca ca 12
<210> 1129
   <211> 12
   <212> DNA
   <213> Artificial Sequence
<220>
   <223> Synthetic Oligonucleotide
<400> 1129
   tgcgagttgt tg 12
<210> 1130
   <211> 12
   <212> DNA
   <213> Artificial Sequence
<220>
   <223> Synthetic Oligonucleotide
<400> 1130
   aaagtcaggc ca 12
<210> 1131
   <211> 14
   <212> DNA
   <213> Artificial Sequence
<220>
   <223> Synthetic Oligonucleotide
<400> 1131
   taacttcagt gtct 14
<210> 1132
   <211> 14
   <212> DNA
   <213> Artificial Sequence
<220>
   <223> Synthetic Oligonucleotide
<400> 1132
   gaagggcttc cagt 14
<210> 1133
   <211> 14
   <212> DNA
   <213> Artificial Sequence
<220>
   <223> Synthetic Oligonucleotide
<400> 1133
   tgaccaggaa gggc 14
<210> 1134
   <211> 14
   <212> DNA
   <213> Artificial Sequence
<220>
   <223> Synthetic Oligonucleotide
<400> 1134
   aggccctgag atta 14
<210> 1135
   <211> 14
   <212> DNA
   <213> Artificial Sequence
<220>
   <223> Synthetic Oligonucleotide
<400> 1135
   ggttaaggcc ctga 14
<210> 1136
   <211> 14
   <212> DNA
   <213> Artificial Sequence
<220>
   <223> Synthetic Oligonucleotide
<400> 1136
   ggaatgtctg agtt 14
<210> 1137
   <211> 14
   <212> DNA
   <213> Artificial Sequence
<220>
   <223> Synthetic Oligonucleotide
<400> 1137
   taatgacctg atga 14
<210> 1138
   <211> 12
   <212> DNA
   <213> Artificial Sequence
<220>
   <223> Synthetic Oligonucleotide
<400> 1138
   tgaagttaat tc 12
<210> 1139
   <211> 12
   <212> DNA
   <213> Artificial Sequence
<220>
   <223> Synthetic Oligonucleotide
<400> 1139
   gaaattgagg aa 12
<210> 1140
   <211> 12
   <212> DNA
   <213> Artificial Sequence
<220>
   <223> Synthetic Oligonucleotide
<400> 1140
   gtattcaagt aa 12
<210> 1141
   <211> 12
   <212> DNA
   <213> Artificial Sequence
<220>
   <223> Synthetic Oligonucleotide
<400> 1141
   acaattatgg ca 12
<210> 1142
   <211> 12
   <212> DNA
   <213> Artificial Sequence
<220>
   <223> Synthetic Oligonucleotide
<400> 1142
   cagtttattc aa 12
<210> 1143
   <211> 12
   <212> DNA
   <213> Artificial Sequence
<220>
   <223> Synthetic Oligonucleotide
<400> 1143
   gaagctgctg gt 12
<210> 1144
   <211> 12
   <212> DNA
   <213> Artificial Sequence
<220>
   <223> Synthetic Oligonucleotide
<400> 1144
   gtgaaacatt tt 12
<210> 1145
   <211> 14
   <212> DNA
   <213> Artificial Sequence
<220>
   <223> Synthetic Oligonucleotide
<400> 1145
   aaaagtgaaa catt 14
<210> 1146
   <211> 12
   <212> DNA
   <213> Artificial Sequence
<220>
   <223> Synthetic Oligonucleotide
<400> 1146
   ttcttaaagg tg 12
<210> 1147
   <211> 12
   <212> DNA
   <213> Artificial Sequence
<220>
   <223> Synthetic Oligonucleotide
<400> 1147
   gcacaaattt tc 12
<210> 1148
   <211> 12
   <212> DNA
   <213> Artificial Sequence
<220>
   <223> Synthetic Oligonucleotide
<400> 1148
   gtttagtgca ca 12
<210> 1149
   <211> 12
   <212> DNA
   <213> Artificial Sequence
<220>
   <223> Synthetic Oligonucleotide
<400> 1149
   tctggatcag ag 12
<210> 1150
   <211> 12
   <212> DNA
   <213> Artificial Sequence
<220>
   <223> Synthetic Oligonucleotide
<400> 1150
   tgctgcacat cc 12
<210> 1151
   <211> 12
   <212> DNA
   <213> Artificial Sequence
<220>
   <223> Synthetic Oligonucleotide
<400> 1151
   tctgactggg aa 12
<210> 1152
   <211> 12
   <212> DNA
   <213> Artificial Sequence
<220>
   <223> Synthetic Oligonucleotide
<400> 1152
   acaaagagtc cc 12
<210> 1153
   <211> 12
   <212> DNA
   <213> Artificial Sequence
<220>
   <223> Synthetic Oligonucleotide
<400> 1153
   agagagctga gc 12
<210> 1154
   <211> 12
   <212> DNA
   <213> Artificial Sequence
<220>
   <223> Synthetic Oligonucleotide
<400> 1154
   tggcctcaca gc 12
<210> 1155
   <211> 12
   <212> DNA
   <213> Artificial Sequence
<220>
   <223> Synthetic Oligonucleotide
<400> 1155
   ggaccgcagc cg 12
<210> 1156
   <211> 12
   <212> DNA
   <213> Artificial Sequence
<220>
   <223> Synthetic Oligonucleotide
<400> 1156
   gttagaacag ac 12
<210> 1157
   <211> 12
   <212> DNA
   <213> Artificial Sequence
<220>
   <223> Synthetic Oligonucleotide
<400> 1157
   cctgaaactg ca 12
<210> 1158
   <211> 12
   <212> DNA
   <213> Artificial Sequence
<220>
   <223> Synthetic Oligonucleotide
<400> 1158
   tgctcacagg cg 12
<210> 1159
   <211> 12
   <212> DNA
   <213> Artificial Sequence
<220>
   <223> Synthetic Oligonucleotide
<400> 1159
   agagagcaac tc 12
<210> 1160
   <211> 12
   <212> DNA
   <213> Artificial Sequence
<220>
   <223> Synthetic Oligonucleotide
<400> 1160
   atggctgctg cg 12
<210> 1161
   <211> 12
   <212> DNA
   <213> Artificial Sequence
<220>
   <223> Synthetic Oligonucleotide
<400> 1161
   catggctgca gc 12
<210> 1162
   <211> 14
   <212> DNA
   <213> Artificial Sequence
<220>
   <223> Synthetic Oligonucleotide
<400> 1162
   cttggccctg gacc 14
<210> 1163
   <211> 13
   <212> DNA
   <213> Artificial Sequence
<220>
   <223> Synthetic Oligonucleotide
<400> 1163
   gctagcctct gga 13
<210> 1164
   <211> 12
   <212> DNA
   <213> Artificial Sequence
<220>
   <223> Synthetic Oligonucleotide
<400> 1164
   tggccctgga cc 12
<210> 1165
   <211> 12
   <212> DNA
   <213> Artificial Sequence
<220>
   <223> Synthetic Oligonucleotide
<400> 1165
   tttctgcagg aa 12
<210> 1166
   <211> 14
   <212> DNA
   <213> Artificial Sequence
<220>
   <223> Synthetic Oligonucleotide
<400> 1166
   tacaggtcaa gtct 14
<210> 1167
   <211> 12
   <212> DNA
   <213> Artificial Sequence
<220>
   <223> Synthetic Oligonucleotide
<400> 1167
   acaggtcaag tc 12
<210> 1168
   <211> 14
   <212> DNA
   <213> Artificial Sequence
<220>
   <223> Synthetic Oligonucleotide
<400> 1168
   ttggataaat atct 14
<210> 1169
   <211> 12
   <212> DNA
   <213> Artificial Sequence
<220>
   <223> Synthetic Oligonucleotide
<400> 1169
   tggataaata tc 12
<210> 1170
   <211> 12
   <212> DNA
   <213> Artificial Sequence
<220>
   <223> Synthetic Oligonucleotide
<400> 1170
   ttctgcagga tg 12
<210> 1171
   <211> 12
   <212> DNA
   <213> Artificial Sequence
<220>
   <223> Synthetic Oligonucleotide
<400> 1171
   caccttcaag tc 12
<210> 1172
   <211> 12
   <212> DNA
   <213> Artificial Sequence
<220>
   <223> Synthetic Oligonucleotide
<400> 1172
   catagattgt at 12
<210> 1173
   <211> 12
   <212> DNA
   <213> Artificial Sequence
<220>
   <223> Synthetic Oligonucleotide
<400> 1173
   gtcagaatat ct 12
<210> 1174
   <211> 12
   <212> DNA
   <213> Artificial Sequence
<220>
   <223> Synthetic Oligonucleotide
<400> 1174
   tctgcagtta aa 12
<210> 1175
   <211> 12
   <212> DNA
   <213> Artificial Sequence
<220>
   <223> Synthetic Oligonucleotide
<400> 1175
   atcttgtgaa ac 12
<210> 1176
   <211> 12
   <212> DNA
   <213> Artificial Sequence
<220>
   <223> Synthetic Oligonucleotide
<400> 1176
   tcatcaaaag gt 12
<210> 1177
   <211> 12
   <212> DNA
   <213> Artificial Sequence
<220>
   <223> Synthetic Oligonucleotide
<400> 1177
   ctttgtcaag at 12
<210> 1178
   <211> 12
   <212> DNA
   <213> Artificial Sequence
<220>
   <223> Synthetic Oligonucleotide
<400> 1178
   tatgcacaaa tc 12
<210> 1179
   <211> 12
   <212> DNA
   <213> Artificial Sequence
<220>
   <223> Synthetic Oligonucleotide
<400> 1179
   taatccaggt ga 12
<210> 1180
   <211> 12
   <212> DNA
   <213> Artificial Sequence
<220>
   <223> Synthetic Oligonucleotide
<400> 1180
   cacacaggca at 12
<210> 1181
   <211> 12
   <212> DNA
   <213> Artificial Sequence
<220>
   <223> Synthetic Oligonucleotide
<400> 1181
   ttgagcatct tg 12
<210> 1182
   <211> 14
   <212> DNA
   <213> Artificial Sequence
<220>
   <223> Synthetic Oligonucleotide
<400> 1182
   cattttgtcc tttt 14
<210> 1183
   <211> 12
   <212> DNA
   <213> Artificial Sequence
<220>
   <223> Synthetic Oligonucleotide
<400> 1183
   gatttcctga tc 12
<210> 1184
   <211> 15
   <212> DNA
   <213> Artificial Sequence
<220>
   <223> Synthetic Oligonucleotide
<400> 1184
   ctggatttga tggct 15
<210> 1185
   <211> 15
   <212> DNA
   <213> Artificial Sequence
<220>
   <223> Synthetic Oligonucleotide
<400> 1185
   tctggatttg atggc 15
<210> 1186
   <211> 12
   <212> DNA
   <213> Artificial Sequence
<220>
   <223> Synthetic Oligonucleotide
<400> 1186
   tggacttggc gg 12
<210> 1187
   <211> 15
   <212> DNA
   <213> Artificial Sequence
<220>
   <223> Synthetic Oligonucleotide
<400> 1187
   ctctggattt gatgg 15
<210> 1188
   <211> 15
   <212> DNA
   <213> Artificial Sequence
<220>
   <223> Synthetic Oligonucleotide
<400> 1188
   cctctggatt tgatg 15
<210> 1189
   <211> 15
   <212> DNA
   <213> Artificial Sequence
<220>
   <223> Synthetic Oligonucleotide
<400> 1189
   gcctctggat ttgat 15
<210> 1190
   <211> 16
   <212> DNA
   <213> Artificial Sequence
<220>
   <223> Synthetic Oligonucleotide
<400> 1190
   tttagcctct ggattt 16
<210> 1191
   <211> 16
   <212> DNA
   <213> Artificial Sequence
<220>
   <223> Synthetic Oligonucleotide
<400> 1191
   tctagcctct ggattt 16
<210> 1192
   <211> 12
   <212> DNA
   <213> Artificial Sequence
<220>
   <223> Synthetic Oligonucleotide
<400> 1192
   agtagttgta ct 12
<210> 1193
   <211> 12
   <212> DNA
   <213> Artificial Sequence
<220>
   <223> Synthetic Oligonucleotide
<400> 1193
   tctggagtca ca 12
<210> 1194
   <211> 12
   <212> DNA
   <213> Artificial Sequence
<220>
   <223> Synthetic Oligonucleotide
<400> 1194
   gtcataatgt ct 12
<210> 1195
   <211> 14
   <212> DNA
   <213> Artificial Sequence
<220>
   <223> Synthetic Oligonucleotide
<400> 1195
   gtgtcataat gtct 14
<210> 1196
   <211> 12
   <212> DNA
   <213> Artificial Sequence
<220>
   <223> Synthetic Oligonucleotide
<400> 1196
   cttcaaaagg at 12
<210> 1197
   <211> 12
   <212> DNA
   <213> Artificial Sequence
<220>
   <223> Synthetic Oligonucleotide
<400> 1197
   gtcttcaaaa gg 12
<210> 1198
   <211> 12
   <212> DNA
   <213> Artificial Sequence
<220>
   <223> Synthetic Oligonucleotide
<400> 1198
   atcctcttga ta 12
<210> 1199
   <211> 12
   <212> DNA
   <213> Artificial Sequence
<220>
   <223> Synthetic Oligonucleotide
<400> 1199
   gtgcctttaa aa 12
<210> 1200
   <211> 12
   <212> DNA
   <213> Artificial Sequence
<220>
   <223> Synthetic Oligonucleotide
<400> 1200
   tgtcataatg tc 12
<210> 1201
   <211> 12
   <212> DNA
   <213> Artificial Sequence
<220>
   <223> Synthetic Oligonucleotide
<400> 1201
   caatctgaca ca 12
<210> 1202
   <211> 12
   <212> DNA
   <213> Artificial Sequence
<220>
   <223> Synthetic Oligonucleotide
<400> 1202
   aatattgttc ct 12
<210> 1203
   <211> 12
   <212> DNA
   <213> Artificial Sequence
<220>
   <223> Synthetic Oligonucleotide
<400> 1203
   gtcaggagaa ga 12
<210> 1204
   <211> 12
   <212> DNA
   <213> Artificial Sequence
<220>
   <223> Synthetic Oligonucleotide
<400> 1204
   tgtcaaaacc ac 12
<210> 1205
   <211> 12
   <212> DNA
   <213> Artificial Sequence
<220>
   <223> Synthetic Oligonucleotide
<400> 1205
   gtcctattgc ca 12
<210> 1206
   <211> 12
   <212> DNA
   <213> Artificial Sequence
<220>
   <223> Synthetic Oligonucleotide
<400> 1206
   aggtatatac at 12
<210> 1207
   <211> 12
   <212> DNA
   <213> Artificial Sequence
<220>
   <223> Synthetic Oligonucleotide
<400> 1207
   cagctggtga ca 12
<210> 1208
   <211> 12
   <212> DNA
   <213> Artificial Sequence
<220>
   <223> Synthetic Oligonucleotide
<400> 1208
   ttcacttagc ca 12
<210> 1209
   <211> 12
   <212> DNA
   <213> Artificial Sequence
<220>
   <223> Synthetic Oligonucleotide
<400> 1209
   aaccctgcag aa 12
<210> 1210
   <211> 12
   <212> DNA
   <213> Artificial Sequence
<220>
   <223> Synthetic Oligonucleotide
<400> 1210
   tgtcaaaacc ct 12
<210> 1211
   <211> 12
   <212> DNA
   <213> Artificial Sequence
<220>
   <223> Synthetic Oligonucleotide
<400> 1211
   gtgtcaaaac cc 12
<210> 1212
   <211> 14
   <212> DNA
   <213> Artificial Sequence
<220>
   <223> Synthetic Oligonucleotide
<400> 1212
   atggtccaga gccc 14
<210> 1213
   <211> 12
   <212> DNA
   <213> Artificial Sequence
<220>
   <223> Synthetic Oligonucleotide
<400> 1213
   tggtccagag cc 12
<210> 1214
   <211> 12
   <212> DNA
   <213> Artificial Sequence
<220>
   <223> Synthetic Oligonucleotide
<400> 1214
   tgggttatgg tc 12
<210> 1215
   <211> 12
   <212> DNA
   <213> Artificial Sequence
<220>
   <223> Synthetic Oligonucleotide
<400> 1215
   caaagaatgg tg 12
<210> 1216
   <211> 12
   <212> DNA
   <213> Artificial Sequence
<220>
   <223> Synthetic Oligonucleotide
<400> 1216
   gtttagcatg ct 12
<210> 1217
   <211> 12
   <212> DNA
   <213> Artificial Sequence
<220>
   <223> Synthetic Oligonucleotide
<400> 1217
   tgcctttaaa aa 12
<210> 1218
   <211> 12
   <212> DNA
   <213> Artificial Sequence
<220>
   <223> Synthetic Oligonucleotide
<400> 1218
   accaatatgc tc 12
<210> 1219
   <211> 12
   <212> DNA
   <213> Artificial Sequence
<220>
   <223> Synthetic Oligonucleotide
<400> 1219
   caccaataag tt 12
<210> 1220
   <211> 12
   <212> DNA
   <213> Artificial Sequence
<220>
   <223> Synthetic Oligonucleotide
<400> 1220
   cctttagctg gc 12
<210> 1221
   <211> 12
   <212> DNA
   <213> Artificial Sequence
<220>
   <223> Synthetic Oligonucleotide
<400> 1221
   gttgaaagcc tc 12
<210> 1222
   <211> 12
   <212> DNA
   <213> Artificial Sequence
<220>
   <223> Synthetic Oligonucleotide
<400> 1222
   tttgatgatg gc 12
<210> 1223
   <211> 12
   <212> DNA
   <213> Artificial Sequence
<220>
   <223> Synthetic Oligonucleotide
<400> 1223
   tcattgtcaa at 12
<210> 1224
   <211> 12
   <212> DNA
   <213> Artificial Sequence
<220>
   <223> Synthetic Oligonucleotide
<400> 1224
   ttacaactga ga 12
<210> 1225
   <211> 12
   <212> DNA
   <213> Artificial Sequence
<220>
   <223> Synthetic Oligonucleotide
<400> 1225
   agatggagca gt 12
<210> 1226
   <211> 14
   <212> DNA
   <213> Artificial Sequence
<220>
   <223> Synthetic Oligonucleotide
<400> 1226
   cttagcactg gcct 14
<210> 1227
   <211> 12
   <212> DNA
   <213> Artificial Sequence
<220>
   <223> Synthetic Oligonucleotide
<400> 1227
   agagtatctg aa 12
<210> 1228
   <211> 12
   <212> DNA
   <213> Artificial Sequence
<220>
   <223> Synthetic Oligonucleotide
<400> 1228
   actgatgtag gg 12
<210> 1229
   <211> 12
   <212> DNA
   <213> Artificial Sequence
<220>
   <223> Synthetic Oligonucleotide
<400> 1229
   ccacagtagg ta 12
<210> 1230
   <211> 14
   <212> DNA
   <213> Artificial Sequence
<220>
   <223> Synthetic Oligonucleotide
<400> 1230
   ctggcagcca gcac 14
<210> 1231
   <211> 12
   <212> DNA
   <213> Artificial Sequence
<220>
   <223> Synthetic Oligonucleotide
<400> 1231
   tggcagccag ca 12
<210> 1232
   <211> 12
   <212> DNA
   <213> Artificial Sequence
<220>
   <223> Synthetic Oligonucleotide
<400> 1232
   gcatcatcaa tc 12
<210> 1233
   <211> 12
   <212> DNA
   <213> Artificial Sequence
<220>
   <223> Synthetic Oligonucleotide
<400> 1233
   aaatcattgt ca 12
<210> 1234
   <211> 12
   <212> DNA
   <213> Artificial Sequence
<220>
   <223> Synthetic Oligonucleotide
<400> 1234
   gcaaaactca tt 12
<210> 1235
   <211> 14
   <212> DNA
   <213> Artificial Sequence
<220>
   <223> Synthetic Oligonucleotide
<400> 1235
   tgtgcaactc tgca 14
<210> 1236
   <211> 12
   <212> DNA
   <213> Artificial Sequence
<220>
   <223> Synthetic Oligonucleotide
<400> 1236
   gtgcaactct gc 12
<210> 1237
   <211> 12
   <212> DNA
   <213> Artificial Sequence
<220>
   <223> Synthetic Oligonucleotide
<400> 1237
   aatattcatg ta 12
<210> 1238
   <211> 12
   <212> DNA
   <213> Artificial Sequence
<220>
   <223> Synthetic Oligonucleotide
<400> 1238
   ggattgcaag tt 12
<210> 1239
   <211> 12
   <212> DNA
   <213> Artificial Sequence
<220>
   <223> Synthetic Oligonucleotide
<400> 1239
   ataaagcatc tg 12
<210> 1240
   <211> 12
   <212> DNA
   <213> Artificial Sequence
<220>
   <223> Synthetic Oligonucleotide
<400> 1240
   ccactgaaca tt 12
<210> 1241
   <211> 12
   <212> DNA
   <213> Artificial Sequence
<220>
   <223> Synthetic Oligonucleotide
<400> 1241
   gaagccctaa tc 12
<210> 1242
   <211> 12
   <212> DNA
   <213> Artificial Sequence
<220>
   <223> Synthetic Oligonucleotide
<400> 1242
   taaattgtat gc 12
<210> 1243
   <211> 12
   <212> DNA
   <213> Artificial Sequence
<220>
   <223> Synthetic Oligonucleotide
<400> 1243
   aagatcttca ca 12
<210> 1244
   <211> 12
   <212> DNA
   <213> Artificial Sequence
<220>
   <223> Synthetic Oligonucleotide
<400> 1244
   caagatcttc ac 12
<210> 1245
   <211> 12
   <212> DNA
   <213> Artificial Sequence
<220>
   <223> Synthetic Oligonucleotide
<400> 1245
   atgtcaaggt ca 12
<210> 1246
   <211> 12
   <212> DNA
   <213> Artificial Sequence
<220>
   <223> Synthetic Oligonucleotide
<400> 1246
   ataatggctg ga 12
<210> 1247
   <211> 14
   <212> DNA
   <213> Artificial Sequence
<220>
   <223> Synthetic Oligonucleotide
<400> 1247
   agcaccaata tgct 14
<210> 1248
   <211> 12
   <212> DNA
   <213> Artificial Sequence
<220>
   <223> Synthetic Oligonucleotide
<400> 1248
   gcaccaatat gc 12
<210> 1249
   <211> 12
   <212> DNA
   <213> Artificial Sequence
<220>
   <223> Synthetic Oligonucleotide
<400> 1249
   tggcagacca ca 12
<210> 1250
   <211> 12
   <212> DNA
   <213> Artificial Sequence
<220>
   <223> Synthetic Oligonucleotide
<400> 1250
   tcctatgcaa tc 12
<210> 1251
   <211> 12
   <212> DNA
   <213> Artificial Sequence
<220>
   <223> Synthetic Oligonucleotide
<400> 1251
   tataaatgct tc 12
<210> 1252
   <211> 12
   <212> DNA
   <213> Artificial Sequence
<220>
   <223> Synthetic Oligonucleotide
<400> 1252
   gtcaaattct at 12
<210> 1253
   <211> 12
   <212> DNA
   <213> Artificial Sequence
<220>
   <223> Synthetic Oligonucleotide
<400> 1253
   ttcaggctaa tc 12
<210> 1254
   <211> 12
   <212> DNA
   <213> Artificial Sequence
<220>
   <223> Synthetic Oligonucleotide
<400> 1254
   ctgatgaggt at 12
<210> 1255
   <211> 12
   <212> DNA
   <213> Artificial Sequence
<220>
   <223> Synthetic Oligonucleotide
<400> 1255
   ggtgtcagaa ta 12
<210> 1256
   <211> 12
   <212> DNA
   <213> Artificial Sequence
<220>
   <223> Synthetic Oligonucleotide
<400> 1256
   tttgaaagac ac 12
<210> 1257
   <211> 4
   <212> DNA
   <213> Artificial Sequence
<220>
   <223> Synthetic Oligonucleotide
<400> 1257
   actg 4
<210> 1258
   <211> 12
   <212> DNA
   <213> Artificial Sequence
<220>
   <223> Synthetic Oligonucleotide
<400> 1258
   tgcaacagcc ca 12
<210> 1259
   <211> 14
   <212> DNA
   <213> Artificial Sequence
<220>
   <223> Synthetic Oligonucleotide
<400> 1259
   aaagagtccc gcca 14
<210> 1260
   <211> 12
   <212> DNA
   <213> Artificial Sequence
<220>
   <223> Synthetic Oligonucleotide
<400> 1260
   aagagtcccg cc 12
<210> 1261
   <211> 14
   <212> DNA
   <213> Artificial Sequence
<220>
   <223> Synthetic Oligonucleotide
<400> 1261
   tccgagagga gaga 14
<210> 1262
   <211> 12
   <212> DNA
   <213> Artificial Sequence
<220>
   <223> Synthetic Oligonucleotide
<400> 1262
   ccgagaggag ag 12
<210> 1263
   <211> 14
   <212> DNA
   <213> Artificial Sequence
<220>
   <223> Synthetic Oligonucleotide
<400> 1263
   tcatggctgc agct 14
<210> 1264
   <211> 14
   <212> DNA
   <213> Artificial Sequence
<220>
   <223> Synthetic Oligonucleotide
<400> 1264
   acagcggctc aact 14
<210> 1265
   <211> 12
   <212> DNA
   <213> Artificial Sequence
<220>
   <223> Synthetic Oligonucleotide
<400> 1265
   cagcggctca ac 12
<210> 1266
   <211> 14
   <212> DNA
   <213> Artificial Sequence
<220>
   <223> Synthetic Oligonucleotide
<400> 1266
   ggtgacaggc gact 14
<210> 1267
   <211> 12
   <212> DNA
   <213> Artificial Sequence
<220>
   <223> Synthetic Oligonucleotide
<400> 1267
   gtgacaggcg ac 12
<210> 1268
   <211> 12
   <212> DNA
   <213> Artificial Sequence
<220>
   <223> Synthetic Oligonucleotide
<400> 1268
   atggtgacag gc 12
<210> 1269
   <211> 12
   <212> DNA
   <213> Artificial Sequence
<220>
   <223> Synthetic Oligonucleotide
<400> 1269
   agaggcctgg ca 12
<210> 1270
   <211> 12
   <212> DNA
   <213> Artificial Sequence
<220>
   <223> Synthetic Oligonucleotide
<400> 1270
   ctggatggtt gc 12
<210> 1271
   <211> 14
   <212> DNA
   <213> Artificial Sequence
<220>
   <223> Synthetic Oligonucleotide
<400> 1271
   aatggctgct gcgg 14
<210> 1272
   <211> 12
   <212> DNA
   <213> Artificial Sequence
<220>
   <223> Synthetic Oligonucleotide
<400> 1272
   ccgggtaatg gc 12
<210> 1273
   <211> 14
   <212> DNA
   <213> Artificial Sequence
<220>
   <223> Synthetic Oligonucleotide
<400> 1273
   tggaccgcag ccgg 14
<210> 1274
   <211> 14
   <212> DNA
   <213> Artificial Sequence
<220>
   <223> Synthetic Oligonucleotide
<400> 1274
   tgatgcccct cgct 14
<210> 1275
   <211> 12
   <212> DNA
   <213> Artificial Sequence
<220>
   <223> Synthetic Oligonucleotide
<400> 1275
   gatgcccctc gc 12
<210> 1276
   <211> 14
   <212> DNA
   <213> Artificial Sequence
<220>
   <223> Synthetic Oligonucleotide
<400> 1276
   ctggacttgg cggt 14
<210> 1277
   <211> 14
   <212> DNA
   <213> Artificial Sequence
<220>
   <223> Synthetic Oligonucleotide
<400> 1277
   ggaaatggct ctgg 14
<210> 1278
   <211> 12
   <212> DNA
   <213> Artificial Sequence
<220>
   <223> Synthetic Oligonucleotide
<400> 1278
   gaaatggctc tg 12
<210> 1279
   <211> 14
   <212> DNA
   <213> Artificial Sequence
<220>
   <223> Synthetic Oligonucleotide
<400> 1279
   agaagctgct ggtg 14
<210> 1280
   <211> 12
   <212> DNA
   <213> Artificial Sequence
<220>
   <223> Synthetic Oligonucleotide
<400> 1280
   gatggcagaa gc 12
<210> 1281
   <211> 14
   <212> DNA
   <213> Artificial Sequence
<220>
   <223> Synthetic Oligonucleotide
<400> 1281
   agagagatgg caga 14
<210> 1282
   <211> 12
   <212> DNA
   <213> Artificial Sequence
<220>
   <223> Synthetic Oligonucleotide
<400> 1282
   gagagatggc ag 12
<210> 1283
   <211> 14
   <212> DNA
   <213> Artificial Sequence
<220>
   <223> Synthetic Oligonucleotide
<400> 1283
   gtggctgaag aaaa 14
<210> 1284
   <211> 12
   <212> DNA
   <213> Artificial Sequence
<220>
   <223> Synthetic Oligonucleotide
<400> 1284
   tggctgaaga aa 12
<210> 1285
   <211> 14
   <212> DNA
   <213> Artificial Sequence
<220>
   <223> Synthetic Oligonucleotide
<400> 1285
   atgtctggga gcct 14
<210> 1286
   <211> 12
   <212> DNA
   <213> Artificial Sequence
<220>
   <223> Synthetic Oligonucleotide
<400> 1286
   tgtctgggag cc 12
<210> 1287
   <211> 14
   <212> DNA
   <213> Artificial Sequence
<220>
   <223> Synthetic Oligonucleotide
<400> 1287
   atgatggctg tcat 14
<210> 1288
   <211> 12
   <212> DNA
   <213> Artificial Sequence
<220>
   <223> Synthetic Oligonucleotide
<400> 1288
   tgatgatggc tg 12
<210> 1289
   <211> 14
   <212> DNA
   <213> Artificial Sequence
<220>
   <223> Synthetic Oligonucleotide
<400> 1289
   ctttgatgat ggct 14
<210> 1290
   <211> 14
   <212> DNA
   <213> Artificial Sequence
<220>
   <223> Synthetic Oligonucleotide
<400> 1290
   acgatctctt tgat 14
<210> 1291
   <211> 14
   <212> DNA
   <213> Artificial Sequence
<220>
   <223> Synthetic Oligonucleotide
<400> 1291
   tgtttctgct aacg 14
<210> 1292
   <211> 12
   <212> DNA
   <213> Artificial Sequence
<220>
   <223> Synthetic Oligonucleotide
<400> 1292
   gtttctgcta ac 12
<210> 1293
   <211> 14
   <212> DNA
   <213> Artificial Sequence
<220>
   <223> Synthetic Oligonucleotide
<400> 1293
   tttgtttctg ctaa 14
<210> 1294
   <211> 14
   <212> DNA
   <213> Artificial Sequence
<220>
   <223> Synthetic Oligonucleotide
<400> 1294
   cttgatatct cctt 14
<210> 1295
   <211> 14
   <212> DNA
   <213> Artificial Sequence
<220>
   <223> Synthetic Oligonucleotide
<400> 1295
   catcctcttg atat 14
<210> 1296
   <211> 14
   <212> DNA
   <213> Artificial Sequence
<220>
   <223> Synthetic Oligonucleotide
<400> 1296
   aatccatcct cttg 14
<210> 1297
   <211> 12
   <212> DNA
   <213> Artificial Sequence
<220>
   <223> Synthetic Oligonucleotide
<400> 1297
   gaatccatcc tc 12
<210> 1298
   <211> 14
   <212> DNA
   <213> Artificial Sequence
<220>
   <223> Synthetic Oligonucleotide
<400> 1298
   gtctaagtcg aatc 14
<210> 1299
   <211> 12
   <212> DNA
   <213> Artificial Sequence
<220>
   <223> Synthetic Oligonucleotide
<400> 1299
   caagtctaag tc 12
<210> 1300
   <211> 12
   <212> DNA
   <213> Artificial Sequence
<220>
   <223> Synthetic Oligonucleotide
<400> 1300
   aggtcaagtc ta 12
<210> 1301
   <211> 14
   <212> DNA
   <213> Artificial Sequence
<220>
   <223> Synthetic Oligonucleotide
<400> 1301
   cgcagaaatg gata 14
<210> 1302
   <211> 12
   <212> DNA
   <213> Artificial Sequence
<220>
   <223> Synthetic Oligonucleotide
<400> 1302
   gcagaaatgg at 12
<210> 1303
   <211> 14
   <212> DNA
   <213> Artificial Sequence
<220>
   <223> Synthetic Oligonucleotide
<400> 1303
   ttcgcatccg tcta 14
<210> 1304
   <211> 12
   <212> DNA
   <213> Artificial Sequence
<220>
   <223> Synthetic Oligonucleotide
<400> 1304
   tcgcatccgt ct 12
<210> 1305
   <211> 14
   <212> DNA
   <213> Artificial Sequence
<220>
   <223> Synthetic Oligonucleotide
<400> 1305
   ccctaggttg aata 14
<210> 1306
   <211> 12
   <212> DNA
   <213> Artificial Sequence
<220>
   <223> Synthetic Oligonucleotide
<400> 1306
   cctaggttga at 12
<210> 1307
   <211> 14
   <212> DNA
   <213> Artificial Sequence
<220>
   <223> Synthetic Oligonucleotide
<400> 1307
   gttatgcaaa tcag 14
<210> 1308
   <211> 12
   <212> DNA
   <213> Artificial Sequence
<220>
   <223> Synthetic Oligonucleotide
<400> 1308
   ttatgcaaat ca 12
<210> 1309
   <211> 14
   <212> DNA
   <213> Artificial Sequence
<220>
   <223> Synthetic Oligonucleotide
<400> 1309
   tgactcagta aatt 14
<210> 1310
   <211> 12
   <212> DNA
   <213> Artificial Sequence
<220>
   <223> Synthetic Oligonucleotide
<400> 1310
   gactcagtaa at 12
<210> 1311
   <211> 14
   <212> DNA
   <213> Artificial Sequence
<220>
   <223> Synthetic Oligonucleotide
<400> 1311
   ttaaaattct tggg 14
<210> 1312
   <211> 12
   <212> DNA
   <213> Artificial Sequence
<220>
   <223> Synthetic Oligonucleotide
<400> 1312
   taaaattctt gg 12
<210> 1313
   <211> 14
   <212> DNA
   <213> Artificial Sequence
<220>
   <223> Synthetic Oligonucleotide
<400> 1313
   cctaactttt agac 14
<210> 1314
   <211> 12
   <212> DNA
   <213> Artificial Sequence
<220>
   <223> Synthetic Oligonucleotide
<400> 1314
   ctaactttta ga 12
<210> 1315
   <211> 14
   <212> DNA
   <213> Artificial Sequence
<220>
   <223> Synthetic Oligonucleotide
<400> 1315
   acctgaaact gcaa 14
<210> 1316
   <211> 14
   <212> DNA
   <213> Artificial Sequence
<220>
   <223> Synthetic Oligonucleotide
<400> 1316
   gtgtcaaaac cact 14
<210> 1317
   <211> 14
   <212> DNA
   <213> Artificial Sequence
<220>
   <223> Synthetic Oligonucleotide
<400> 1317
   cctattccca ctga 14
<210> 1318
   <211> 12
   <212> DNA
   <213> Artificial Sequence
<220>
   <223> Synthetic Oligonucleotide
<400> 1318
   ctattcccac tg 12
<210> 1319
   <211> 14
   <212> DNA
   <213> Artificial Sequence
<220>
   <223> Synthetic Oligonucleotide
<400> 1319
   cccatagcaa taat 14
<210> 1320
   <211> 12
   <212> DNA
   <213> Artificial Sequence
<220>
   <223> Synthetic Oligonucleotide
<400> 1320
   ccatagcaat aa 12
<210> 1321
   <211> 12
   <212> DNA
   <213> Artificial Sequence
<220>
   <223> Synthetic Oligonucleotide
<400> 1321
   atcccatagc aa 12
<210> 1322
   <211> 14
   <212> DNA
   <213> Artificial Sequence
<220>
   <223> Synthetic Oligonucleotide
<400> 1322
   tctgcaggaa atcc 14
<210> 1323
   <211> 12
   <212> DNA
   <213> Artificial Sequence
<220>
   <223> Synthetic Oligonucleotide
<400> 1323
   ctgcaggaaa tc 12
<210> 1324
   <211> 14
   <212> DNA
   <213> Artificial Sequence
<220>
   <223> Synthetic Oligonucleotide
<400> 1324
   ttctgcagga aatc 14
<210> 1325
   <211> 14
   <212> DNA
   <213> Artificial Sequence
<220>
   <223> Synthetic Oligonucleotide
<400> 1325
   ccttcaagtc tttc 14
<210> 1326
   <211> 14
   <212> DNA
   <213> Artificial Sequence
<220>
   <223> Synthetic Oligonucleotide
<400> 1326
   ttgttcctgt atac 14
<210> 1327
   <211> 14
   <212> DNA
   <213> Artificial Sequence
<220>
   <223> Synthetic Oligonucleotide
<400> 1327
   caatattgtt cctg 14
<210> 1328
   <211> 12
   <212> DNA
   <213> Artificial Sequence
<220>
   <223> Synthetic Oligonucleotide
<400> 1328
   caatattgtt cc 12
<210> 1329
   <211> 14
   <212> DNA
   <213> Artificial Sequence
<220>
   <223> Synthetic Oligonucleotide
<400> 1329
   acatcatcaa tatt 14
<210> 1330
   <211> 14
   <212> DNA
   <213> Artificial Sequence
<220>
   <223> Synthetic Oligonucleotide
<400> 1330
   ctttgaatcc aaaa 14
<210> 1331
   <211> 12
   <212> DNA
   <213> Artificial Sequence
<220>
   <223> Synthetic Oligonucleotide
<400> 1331
   tttgaatcca aa 12
<210> 1332
   <211> 14
   <212> DNA
   <213> Artificial Sequence
<220>
   <223> Synthetic Oligonucleotide
<400> 1332
   tatgctttga atcc 14
<210> 1333
   <211> 14
   <212> DNA
   <213> Artificial Sequence
<220>
   <223> Synthetic Oligonucleotide
<400> 1333
   ttgtaatggt tttt 14
<210> 1334
   <211> 12
   <212> DNA
   <213> Artificial Sequence
<220>
   <223> Synthetic Oligonucleotide
<400> 1334
   atcttgtaat gg 12
<210> 1335
   <211> 14
   <212> DNA
   <213> Artificial Sequence
<220>
   <223> Synthetic Oligonucleotide
<400> 1335
   agattgtata tctt 14
<210> 1336
   <211> 14
   <212> DNA
   <213> Artificial Sequence
<220>
   <223> Synthetic Oligonucleotide
<400> 1336
   cggtgtcata atgt 14
<210> 1337
   <211> 14
   <212> DNA
   <213> Artificial Sequence
<220>
   <223> Synthetic Oligonucleotide
<400> 1337
   aaatttggcg gtgt 14
<210> 1338
   <211> 14
   <212> DNA
   <213> Artificial Sequence
<220>
   <223> Synthetic Oligonucleotide
<400> 1338
   ttaaatttgg cggt 14
<210> 1339
   <211> 12
   <212> DNA
   <213> Artificial Sequence
<220>
   <223> Synthetic Oligonucleotide
<400> 1339
   taaatttggc gg 12
<210> 1340
   <211> 14
   <212> DNA
   <213> Artificial Sequence
<220>
   <223> Synthetic Oligonucleotide
<400> 1340
   tcttcaaaag gata 14
<210> 1341
   <211> 12
   <212> DNA
   <213> Artificial Sequence
<220>
   <223> Synthetic Oligonucleotide
<400> 1341
   tggtcttcaa aa 12
<210> 1342
   <211> 14
   <212> DNA
   <213> Artificial Sequence
<220>
   <223> Synthetic Oligonucleotide
<400> 1342
   gtgggttatg gtct 14
<210> 1343
   <211> 14
   <212> DNA
   <213> Artificial Sequence
<220>
   <223> Synthetic Oligonucleotide
<400> 1343
   aagttctagc tgtg 14
<210> 1344
   <211> 12
   <212> DNA
   <213> Artificial Sequence
<220>
   <223> Synthetic Oligonucleotide
<400> 1344
   ataagttcta gc 12
<210> 1345
   <211> 14
   <212> DNA
   <213> Artificial Sequence
<220>
   <223> Synthetic Oligonucleotide
<400> 1345
   aagggtttga taag 14
<210> 1346
   <211> 14
   <212> DNA
   <213> Artificial Sequence
<220>
   <223> Synthetic Oligonucleotide
<400> 1346
   tcaagatctt caca 14
<210> 1347
   <211> 14
   <212> DNA
   <213> Artificial Sequence
<220>
   <223> Synthetic Oligonucleotide
<400> 1347
   agccattggt caag 14
<210> 1348
   <211> 14
   <212> DNA
   <213> Artificial Sequence
<220>
   <223> Synthetic Oligonucleotide
<400> 1348
   tcttcactta gcca 14
<210> 1349
   <211> 14
   <212> DNA
   <213> Artificial Sequence
<220>
   <223> Synthetic Oligonucleotide
<400> 1349
   tgctgcaaca tgat 14
<210> 1350
   <211> 12
   <212> DNA
   <213> Artificial Sequence
<220>
   <223> Synthetic Oligonucleotide
<400> 1350
   gctgcaacat ga 12
<210> 1351
   <211> 14
   <212> DNA
   <213> Artificial Sequence
<220>
   <223> Synthetic Oligonucleotide
<400> 1351
   ttacagtgaa ttgc 14
<210> 1352
   <211> 12
   <212> DNA
   <213> Artificial Sequence
<220>
   <223> Synthetic Oligonucleotide
<400> 1352
   ccagctttac ag 12
<210> 1353
   <211> 12
   <212> DNA
   <213> Artificial Sequence
<220>
   <223> Synthetic Oligonucleotide
<400> 1353
   cattacacca gt 12
<210> 1354
   <211> 14
   <212> DNA
   <213> Artificial Sequence
<220>
   <223> Synthetic Oligonucleotide
<400> 1354
   atcattacac cagt 14
<210> 1355
   <211> 12
   <212> DNA
   <213> Artificial Sequence
<220>
   <223> Synthetic Oligonucleotide
<400> 1355
   tcattacacc ag 12
<210> 1356
   <211> 14
   <212> DNA
   <213> Artificial Sequence
<220>
   <223> Synthetic Oligonucleotide
<400> 1356
   aataaatatg caca 14
<210> 1357
   <211> 14
   <212> DNA
   <213> Artificial Sequence
<220>
   <223> Synthetic Oligonucleotide
<400> 1357
   tgtgccttta aaaa 14
<210> 1358
   <211> 14
   <212> DNA
   <213> Artificial Sequence
<220>
   <223> Synthetic Oligonucleotide
<400> 1358
   ttgtgccttt aaaa 14
<210> 1359
   <211> 14
   <212> DNA
   <213> Artificial Sequence
<220>
   <223> Synthetic Oligonucleotide
<400> 1359
   gggcctcttg tgcc 14
<210> 1360
   <211> 14
   <212> DNA
   <213> Artificial Sequence
<220>
   <223> Synthetic Oligonucleotide
<400> 1360
   ccttacttcc ccat 14
<210> 1361
   <211> 14
   <212> DNA
   <213> Artificial Sequence
<220>
   <223> Synthetic Oligonucleotide
<400> 1361
   ctctggtcct tact 14
<210> 1362
   <211> 16
   <212> DNA
   <213> Artificial Sequence
<220>
   <223> Synthetic Oligonucleotide
<400> 1362
   gtctctggtc cttact 16
<210> 1363
   <211> 14
   <212> DNA
   <213> Artificial Sequence
<220>
   <223> Synthetic Oligonucleotide
<400> 1363
   tctctggtcc ttac 14
<210> 1364
   <211> 14
   <212> DNA
   <213> Artificial Sequence
<220>
   <223> Synthetic Oligonucleotide
<400> 1364
   cctctgactg ggaa 14
<210> 1365
   <211> 14
   <212> DNA
   <213> Artificial Sequence
<220>
   <223> Synthetic Oligonucleotide
<400> 1365
   catagcgcct ctga 14
<210> 1366
   <211> 14
   <212> DNA
   <213> Artificial Sequence
<220>
   <223> Synthetic Oligonucleotide
<400> 1366
   caggtagcta taat 14
<210> 1367
   <211> 12
   <212> DNA
   <213> Artificial Sequence
<220>
   <223> Synthetic Oligonucleotide
<400> 1367
   caggtagcta ta 12
<210> 1368
   <211> 14
   <212> DNA
   <213> Artificial Sequence
<220>
   <223> Synthetic Oligonucleotide
<400> 1368
   tcatcttgtg aaac 14
<210> 1369
   <211> 12
   <212> DNA
   <213> Artificial Sequence
<220>
   <223> Synthetic Oligonucleotide
<400> 1369
   catcttgtga aa 12
<210> 1370
   <211> 14
   <212> DNA
   <213> Artificial Sequence
<220>
   <223> Synthetic Oligonucleotide
<400> 1370
   gtttcaaaca tcat 14
<210> 1371
   <211> 14
   <212> DNA
   <213> Artificial Sequence
<220>
   <223> Synthetic Oligonucleotide
<400> 1371
   tagtttcaaa catc 14
<210> 1372
   <211> 12
   <212> DNA
   <213> Artificial Sequence
<220>
   <223> Synthetic Oligonucleotide
<400> 1372
   agtttcaaac at 12
<210> 1373
   <211> 12
   <212> DNA
   <213> Artificial Sequence
<220>
   <223> Synthetic Oligonucleotide
<400> 1373
   gaatagtttc aa 12
<210> 1374
   <211> 14
   <212> DNA
   <213> Artificial Sequence
<220>
   <223> Synthetic Oligonucleotide
<400> 1374
   cattggaata gttt 14
<210> 1375
   <211> 14
   <212> DNA
   <213> Artificial Sequence
<220>
   <223> Synthetic Oligonucleotide
<400> 1375
   cactgaacat tgga 14
<210> 1376
   <211> 12
   <212> DNA
   <213> Artificial Sequence
<220>
   <223> Synthetic Oligonucleotide
<400> 1376
   actgaacatt gg 12
<210> 1377
   <211> 14
   <212> DNA
   <213> Artificial Sequence
<220>
   <223> Synthetic Oligonucleotide
<400> 1377
   ccgccactga acat 14
<210> 1378
   <211> 14
   <212> DNA
   <213> Artificial Sequence
<220>
   <223> Synthetic Oligonucleotide
<400> 1378
   cagaccacaa actg 14
<210> 1379
   <211> 12
   <212> DNA
   <213> Artificial Sequence
<220>
   <223> Synthetic Oligonucleotide
<400> 1379
   agaccacaaa ct 12
<210> 1380
   <211> 14
   <212> DNA
   <213> Artificial Sequence
<220>
   <223> Synthetic Oligonucleotide
<400> 1380
   ctggcagacc acaa 14
<210> 1381
   <211> 14
   <212> DNA
   <213> Artificial Sequence
<220>
   <223> Synthetic Oligonucleotide
<400> 1381
   agctggcaga ccac 14
<210> 1382
   <211> 14
   <212> DNA
   <213> Artificial Sequence
<220>
   <223> Synthetic Oligonucleotide
<400> 1382
   acctttagct ggca 14
<210> 1383
   <211> 14
   <212> DNA
   <213> Artificial Sequence
<220>
   <223> Synthetic Oligonucleotide
<400> 1383
   tcttcacctt tagc 14
<210> 1384
   <211> 12
   <212> DNA
   <213> Artificial Sequence
<220>
   <223> Synthetic Oligonucleotide
<400> 1384
   tcaaagtaca tg 12
<210> 1385
   <211> 14
   <212> DNA
   <213> Artificial Sequence
<220>
   <223> Synthetic Oligonucleotide
<400> 1385
   gaactcaaag taca 14
<210> 1386
   <211> 12
   <212> DNA
   <213> Artificial Sequence
<220>
   <223> Synthetic Oligonucleotide
<400> 1386
   ggaactcaaa gt 12
<210> 1387
   <211> 14
   <212> DNA
   <213> Artificial Sequence
<220>
   <223> Synthetic Oligonucleotide
<400> 1387
   agggaactca aagt 14
<210> 1388
   <211> 12
   <212> DNA
   <213> Artificial Sequence
<220>
   <223> Synthetic Oligonucleotide
<400> 1388
   gggaactcaa ag 12
<210> 1389
   <211> 14
   <212> DNA
   <213> Artificial Sequence
<220>
   <223> Synthetic Oligonucleotide
<400> 1389
   gctgagggaa ctca 14
<210> 1390
   <211> 14
   <212> DNA
   <213> Artificial Sequence
<220>
   <223> Synthetic Oligonucleotide
<400> 1390
   tcaccacaca cagg 14
<210> 1391
   <211> 14
   <212> DNA
   <213> Artificial Sequence
<220>
   <223> Synthetic Oligonucleotide
<400> 1391
   gaactctact ttga 14
<210> 1392
   <211> 12
   <212> DNA
   <213> Artificial Sequence
<220>
   <223> Synthetic Oligonucleotide
<400> 1392
   gaagaactct ac 12
<210> 1393
   <211> 14
   <212> DNA
   <213> Artificial Sequence
<220>
   <223> Synthetic Oligonucleotide
<400> 1393
   gtggaagaac tcta 14
<210> 1394
   <211> 12
   <212> DNA
   <213> Artificial Sequence
<220>
   <223> Synthetic Oligonucleotide
<400> 1394
   tggaagaact ct 12
<210> 1395
   <211> 14
   <212> DNA
   <213> Artificial Sequence
<220>
   <223> Synthetic Oligonucleotide
<400> 1395
   tgtttgtgga agaa 14
<210> 1396
   <211> 14
   <212> DNA
   <213> Artificial Sequence
<220>
   <223> Synthetic Oligonucleotide
<400> 1396
   catcttgttc tgtt 14
<210> 1397
   <211> 14
   <212> DNA
   <213> Artificial Sequence
<220>
   <223> Synthetic Oligonucleotide
<400> 1397
   agtgaaacat tttg 14
<210> 1398
   <211> 12
   <212> DNA
   <213> Artificial Sequence
<220>
   <223> Synthetic Oligonucleotide
<400> 1398
   ttacccaaaa gt 12
<210> 1399
   <211> 14
   <212> DNA
   <213> Artificial Sequence
<220>
   <223> Synthetic Oligonucleotide
<400> 1399
   tatttaccca aaag 14
<210> 1400
   <211> 14
   <212> DNA
   <213> Artificial Sequence
<220>
   <223> Synthetic Oligonucleotide
<400> 1400
   gtatttaccc aaaa 14
<210> 1401
   <211> 12
   <212> DNA
   <213> Artificial Sequence
<220>
   <223> Synthetic Oligonucleotide
<400> 1401
   tatttaccca aa 12
<210> 1402
   <211> 14
   <212> DNA
   <213> Artificial Sequence
<220>
   <223> Synthetic Oligonucleotide
<400> 1402
   tcctggtatg aaga 14
<210> 1403
   <211> 12
   <212> DNA
   <213> Artificial Sequence
<220>
   <223> Synthetic Oligonucleotide
<400> 1403
   cctggtatga ag 12
<210> 1404
   <211> 12
   <212> DNA
   <213> Artificial Sequence
<220>
   <223> Synthetic Oligonucleotide
<400> 1404
   ggtcctggta tg 12
<210> 1405
   <211> 14
   <212> DNA
   <213> Artificial Sequence
<220>
   <223> Synthetic Oligonucleotide
<400> 1405
   ttcctctggt cctg 14
<210> 1406
   <211> 14
   <212> DNA
   <213> Artificial Sequence
<220>
   <223> Synthetic Oligonucleotide
<400> 1406
   aggtttcctc tggt 14
<210> 1407
   <211> 12
   <212> DNA
   <213> Artificial Sequence
<220>
   <223> Synthetic Oligonucleotide
<400> 1407
   ggtttcctct gg 12
<210> 1408
   <211> 14
   <212> DNA
   <213> Artificial Sequence
<220>
   <223> Synthetic Oligonucleotide
<400> 1408
   ttttctgagg tttc 14
<210> 1409
   <211> 14
   <212> DNA
   <213> Artificial Sequence
<220>
   <223> Synthetic Oligonucleotide
<400> 1409
   agacttccat tttc 14
<210> 1410
   <211> 12
   <212> DNA
   <213> Artificial Sequence
<220>
   <223> Synthetic Oligonucleotide
<400> 1410
   agacttccat tt 12
<210> 1411
   <211> 14
   <212> DNA
   <213> Artificial Sequence
<220>
   <223> Synthetic Oligonucleotide
<400> 1411
   caaatgctat cgat 14
<210> 1412
   <211> 14
   <212> DNA
   <213> Artificial Sequence
<220>
   <223> Synthetic Oligonucleotide
<400> 1412
   gcacgctcta tact 14
<210> 1413
   <211> 12
   <212> DNA
   <213> Artificial Sequence
<220>
   <223> Synthetic Oligonucleotide
<400> 1413
   cacgctctat ac 12
<210> 1414
   <211> 14
   <212> DNA
   <213> Artificial Sequence
<220>
   <223> Synthetic Oligonucleotide
<400> 1414
   tccttgtcat tatc 14
<210> 1415
   <211> 14
   <212> DNA
   <213> Artificial Sequence
<220>
   <223> Synthetic Oligonucleotide
<400> 1415
   gctttgtcaa gatc 14
<210> 1416
   <211> 14
   <212> DNA
   <213> Artificial Sequence
<220>
   <223> Synthetic Oligonucleotide
<400> 1416
   tgctttgtca agat 14
<210> 1417
   <211> 14
   <212> DNA
   <213> Artificial Sequence
<220>
   <223> Synthetic Oligonucleotide
<400> 1417
   aagtatcggt tggc 14
<210> 1418
   <211> 12
   <212> DNA
   <213> Artificial Sequence
<220>
   <223> Synthetic Oligonucleotide
<400> 1418
   agtatcggtt gg 12
<210> 1419
   <211> 14
   <212> DNA
   <213> Artificial Sequence
<220>
   <223> Synthetic Oligonucleotide
<400> 1419
   ttaaaatttg gaga **14**
<210> 1420
   <211> 14
   <212> DNA
   <213> Artificial Sequence
<220>
   <223> Synthetic Oligonucleotide
<400> 1420
   ccttaaaatt tgga 14
<210> 1421
   <211> 12
   <212> DNA
   <213> Artificial Sequence
<220>
   <223> Synthetic Oligonucleotide
<400> 1421
   cttaaaattt gg 12
<210> 1422
   <211> 14
   <212> DNA
   <213> Artificial Sequence
<220>
   <223> Synthetic Oligonucleotide
<400> 1422
   tctactgttt ttgt 14
<210> 1423
   <211> 14
   <212> DNA
   <213> Artificial Sequence
<220>
   <223> Synthetic Oligonucleotide
<400> 1423
   ggctcctcta ctgt 14
<210> 1424
   <211> 15
   <212> DNA
   <213> Artificial Sequence
<220>
   <223> Synthetic Oligonucleotide
<400> 1424
   agcctctgga tttga 15
<210> 1425
   <211> 14
   <212> DNA
   <213> Artificial Sequence
<220>
   <223> Synthetic Oligonucleotide
<400> 1425
   agcctctgga tttg 14
<210> 1426
   <211> 15
   <212> DNA
   <213> Artificial Sequence
<220>
   <223> Synthetic Oligonucleotide
<400> 1426
   tagcctctgg atttg 15
<210> 1427
   <211> 14
   <212> DNA
   <213> Artificial Sequence
<220>
   <223> Synthetic Oligonucleotide
<400> 1427
   tagcctctgg attt 14
<210> 1428
   <211> 16
   <212> DNA
   <213> Artificial Sequence
<220>
   <223> Synthetic Oligonucleotide
<400> 1428
   gctagcctct ggattt 16
<210> 1429
   <211> 15
   <212> DNA
   <213> Artificial Sequence
<220>
   <223> Synthetic Oligonucleotide
<400> 1429
   ctagcctctg gattt 15
<210> 1430
   <211> 15
   <212> DNA
   <213> Artificial Sequence
<220>
   <223> Synthetic Oligonucleotide
<400> 1430
   gctagcctct ggatt 15
<210> 1431
   <211> 14
   <212> DNA
   <213> Artificial Sequence
<220>
   <223> Synthetic Oligonucleotide
<400> 1431
   ctagcctctg gatt 14
<210> 1432
   <211> 13
   <212> DNA
   <213> Artificial Sequence
<220>
   <223> Synthetic Oligonucleotide
<400> 1432
   tagcctctgg att 13
<210> 1433
   <211> 15
   <212> DNA
   <213> Artificial Sequence
<220>
   <223> Synthetic Oligonucleotide
<400> 1433
   tgctagcctc tggat 15
<210> 1434
   <211> 12
   <212> DNA
   <213> Artificial Sequence
<220>
   <223> Synthetic Oligonucleotide
<400> 1434
   tagcctctgg at 12
<210> 1435
   <211> 15
   <212> DNA
   <213> Artificial Sequence
<220>
   <223> Synthetic Oligonucleotide
<400> 1435
   ctgctagcct ctgga 15
<210> 1436
   <211> 15
   <212> DNA
   <213> Artificial Sequence
<220>
   <223> Synthetic Oligonucleotide
<400> 1436
   actgctagcc tctgg 15
<210> 1437
   <211> 15
   <212> DNA
   <213> Artificial Sequence
<220>
   <223> Synthetic Oligonucleotide
<400> 1437
   aactgctagc ctctg 15
<210> 1438
   <211> 15
   <212> DNA
   <213> Artificial Sequence
<220>
   <223> Synthetic Oligonucleotide
<400> 1438
   gaactgctag cctct 15
<210> 1439
   <211> 15
   <212> DNA
   <213> Artificial Sequence
<220>
   <223> Synthetic Oligonucleotide
<400> 1439
   tgaactgcta gcctc 15
<210> 1440
   <211> 15
   <212> DNA
   <213> Artificial Sequence
<220>
   <223> Synthetic Oligonucleotide
<400> 1440
   ttgaactgct agcct 15
<210> 1441
   <211> 14
   <212> DNA
   <213> Artificial Sequence
<220>
   <223> Synthetic Oligonucleotide
<400> 1441
   tgaactgcta gcct 14
<210> 1442
   <211> 14
   <212> DNA
   <213> Artificial Sequence
<220>
   <223> Synthetic Oligonucleotide
<400> 1442
   agttgaactg ctag 14
<210> 1443
   <211> 12
   <212> DNA
   <213> Artificial Sequence
<220>
   <223> Synthetic Oligonucleotide
<400> 1443
   gttgaactgc ta 12
<210> 1444
   <211> 12
   <212> DNA
   <213> Artificial Sequence
<220>
   <223> Synthetic Oligonucleotide
<400> 1444
   gaagttgaac tg 12
<210> 1445
   <211> 14
   <212> DNA
   <213> Artificial Sequence
<220>
   <223> Synthetic Oligonucleotide
<400> 1445
   acagaagttg aact 14
<210> 1446
   <211> 14
   <212> DNA
   <213> Artificial Sequence
<220>
   <223> Synthetic Oligonucleotide
<400> 1446
   tcattgtcac taac 14
<210> 1447
   <211> 12
   <212> DNA
   <213> Artificial Sequence
<220>
   <223> Synthetic Oligonucleotide
<400> 1447
   cattgtcact aa 12
<210> 1448
   <211> 12
   <212> DNA
   <213> Artificial Sequence
<220>
   <223> Synthetic Oligonucleotide
<400> 1448
   tcaggttcat tg 12
<210> 1449
   <211> 14
   <212> DNA
   <213> Artificial Sequence
<220>
   <223> Synthetic Oligonucleotide
<400> 1449
   atgatcaggt tcat 14
<210> 1450
   <211> 14
   <212> DNA
   <213> Artificial Sequence
<220>
   <223> Synthetic Oligonucleotide
<400> 1450
   tataatgatc aggt 14
<210> 1451
   <211> 12
   <212> DNA
   <213> Artificial Sequence
<220>
   <223> Synthetic Oligonucleotide
<400> 1451
   ataatgatca gg 12
<210> 1452
   <211> 14
   <212> DNA
   <213> Artificial Sequence
<220>
   <223> Synthetic Oligonucleotide
<400> 1452
   tggtgtcaga atat 14
<210> 1453
   <211> 14
   <212> DNA
   <213> Artificial Sequence
<220>
   <223> Synthetic Oligonucleotide
<400> 1453
   tcagtggtgt caga 14
<210> 1454
   <211> 14
   <212> DNA
   <213> Artificial Sequence
<220>
   <223> Synthetic Oligonucleotide
<400> 1454
   ctctggatca gagt 14
<210> 1455
   <211> 14
   <212> DNA
   <213> Artificial Sequence
<220>
   <223> Synthetic Oligonucleotide
<400> 1455
   aaggttcatt ctct 14
<210> 1456
   <211> 14
   <212> DNA
   <213> Artificial Sequence
<220>
   <223> Synthetic Oligonucleotide
<400> 1456
   ttcatcaaaa ggtt 14
<210> 1457
   <211> 14
   <212> DNA
   <213> Artificial Sequence
<220>
   <223> Synthetic Oligonucleotide
<400> 1457
   cttcatcaaa aggt 14
<210> 1458
   <211> 12
   <212> DNA
   <213> Artificial Sequence
<220>
   <223> Synthetic Oligonucleotide
<400> 1458
   cttcatcaaa ag 12
<210> 1459
   <211> 14
   <212> DNA
   <213> Artificial Sequence
<220>
   <223> Synthetic Oligonucleotide
<400> 1459
   atgctgatct tcat 14
<210> 1460
   <211> 14
   <212> DNA
   <213> Artificial Sequence
<220>
   <223> Synthetic Oligonucleotide
<400> 1460
   ttttgtaatt tgtg 14
<210> 1461
   <211> 14
   <212> DNA
   <213> Artificial Sequence
<220>
   <223> Synthetic Oligonucleotide
<400> 1461
   tcagactttt gtaa 14
<210> 1462
   <211> 14
   <212> DNA
   <213> Artificial Sequence
<220>
   <223> Synthetic Oligonucleotide
<400> 1462
   tgtcctattg ccat 14
<210> 1463
   <211> 14
   <212> DNA
   <213> Artificial Sequence
<220>
   <223> Synthetic Oligonucleotide
<400> 1463
   tctgacacaa tgtc 14
<210> 1464
   <211> 12
   <212> DNA
   <213> Artificial Sequence
<220>
   <223> Synthetic Oligonucleotide
<400> 1464
   ctgacacaat gt 12
<210> 1465
   <211> 14
   <212> DNA
   <213> Artificial Sequence
<220>
   <223> Synthetic Oligonucleotide
<400> 1465
   tgttcctata actg 14
<210> 1466
   <211> 12
   <212> DNA
   <213> Artificial Sequence
<220>
   <223> Synthetic Oligonucleotide
<400> 1466
   gttcctataa ct 12
<210> 1467
   <211> 14
   <212> DNA
   <213> Artificial Sequence
<220>
   <223> Synthetic Oligonucleotide
<400> 1467
   aagattggtc agga 14
<210> 1468
   <211> 12
   <212> DNA
   <213> Artificial Sequence
<220>
   <223> Synthetic Oligonucleotide
<400> 1468
   agattggtca gg 12
<210> 1469
   <211> 14
   <212> DNA
   <213> Artificial Sequence
<220>
   <223> Synthetic Oligonucleotide
<400> 1469
   gtgtcaaaac cctg 14
<210> 1470
   <211> 12
   <212> DNA
   <213> Artificial Sequence
<220>
   <223> Synthetic Oligonucleotide
<400> 1470
   agctacacaa cc 12
<210> 1471
   <211> 14
   <212> DNA
   <213> Artificial Sequence
<220>
   <223> Synthetic Oligonucleotide
<400> 1471
   cacagctaca caac 14
<210> 1472
   <211> 12
   <212> DNA
   <213> Artificial Sequence
<220>
   <223> Synthetic Oligonucleotide
<400> 1472
   acagctacac aa 12
<210> 1473
   <211> 14
   <212> DNA
   <213> Artificial Sequence
<220>
   <223> Synthetic Oligonucleotide
<400> 1473
   tatatacatg acac 14
<210> 1474
   <211> 12
   <212> DNA
   <213> Artificial Sequence
<220>
   <223> Synthetic Oligonucleotide
<400> 1474
   atatacatga ca 12
<210> 1475
   <211> 14
   <212> DNA
   <213> Artificial Sequence
<220>
   <223> Synthetic Oligonucleotide
<400> 1475
   aattttaaat gtcc 14
<210> 1476
   <211> 12
   <212> DNA
   <213> Artificial Sequence
<220>
   <223> Synthetic Oligonucleotide
<400> 1476
   attttaaatg tc 12
<210> 1477
   <211> 12
   <212> DNA
   <213> Artificial Sequence
<220>
   <223> Synthetic Oligonucleotide
<400> 1477
   tcctaattga at 12
<210> 1478
   <211> 12
   <212> DNA
   <213> Artificial Sequence
<220>
   <223> Synthetic Oligonucleotide
<400> 1478
   aaagtgccat ct 12
<210> 1479
   <211> 14
   <212> DNA
   <213> Artificial Sequence
<220>
   <223> Synthetic Oligonucleotide
<400> 1479
   tttataaaac tgga 14
<210> 1480
   <211> 12
   <212> DNA
   <213> Artificial Sequence
<220>
   <223> Synthetic Oligonucleotide
<400> 1480
   ttataaaact gg 12
<210> 1481
   <211> 14
   <212> DNA
   <213> Artificial Sequence
<220>
   <223> Synthetic Oligonucleotide
<400> 1481
   tgcaaactta tctg 14
<210> 1482
   <211> 12
   <212> DNA
   <213> Artificial Sequence
<220>
   <223> Synthetic Oligonucleotide
<400> 1482
   gcaaacttat ct 12
<210> 1483
   <211> 12
   <212> DNA
   <213> Artificial Sequence
<220>
   <223> Synthetic Oligonucleotide
<400> 1483
   agccaactgc aa 12
<210> 1484
   <211> 14
   <212> DNA
   <213> Artificial Sequence
<220>
   <223> Synthetic Oligonucleotide
<400> 1484
   cttagccaac tgca 14
<210> 1485
   <211> 12
   <212> DNA
   <213> Artificial Sequence
<220>
   <223> Synthetic Oligonucleotide
<400> 1485
   ttagccaact gc 12
<210> 1486
   <211> 14
   <212> DNA
   <213> Artificial Sequence
<220>
   <223> Synthetic Oligonucleotide
<400> 1486
   taaatcattg tcaa 14
<210> 1487
   <211> 14
   <212> DNA
   <213> Artificial Sequence
<220>
   <223> Synthetic Oligonucleotide
<400> 1487
   gcactggcct tgat 14
<210> 1488
   <211> 12
   <212> DNA
   <213> Artificial Sequence
<220>
   <223> Synthetic Oligonucleotide
<400> 1488
   cactggcctt ga 12
<210> 1489
   <211> 12
   <212> DNA
   <213> Artificial Sequence
<220>
   <223> Synthetic Oligonucleotide
<400> 1489
   ttagcactgg cc 12
<210> 1490
   <211> 12
   <212> DNA
   <213> Artificial Sequence
<220>
   <223> Synthetic Oligonucleotide
<400> 1490
   tgtgtaaggt ca 12
<210> 1491
   <211> 12
   <212> DNA
   <213> Artificial Sequence
<220>
   <223> Synthetic Oligonucleotide
<400> 1491
   gttaatgaca tt 12
<210> 1492
   <211> 12
   <212> DNA
   <213> Artificial Sequence
<220>
   <223> Synthetic Oligonucleotide
<400> 1492
   gacaatttct ac 12
<210> 1493
   <211> 12
   <212> DNA
   <213> Artificial Sequence
<220>
   <223> Synthetic Oligonucleotide
<400> 1493
   aacactgcac at 12
<210> 1494
   <211> 14
   <212> DNA
   <213> Artificial Sequence
<220>
   <223> Synthetic Oligonucleotide
<400> 1494
   taggtcaagt ctaa 14
<210> 1495
   <211> 14
   <212> DNA
   <213> Artificial Sequence
<220>
   <223> Synthetic Oligonucleotide
<400> 1495
   tataggtcaa gtct 14
<210> 1496
   <211> 14
   <212> DNA
   <213> Artificial Sequence
<220>
   <223> Synthetic Oligonucleotide
<400> 1496
   tttggataaa tata 14
<210> 1497
   <211> 14
   <212> DNA
   <213> Artificial Sequence
<220>
   <223> Synthetic Oligonucleotide
<400> 1497
   tgggacttca cacc 14
<210> 1498
   <211> 12
   <212> DNA
   <213> Artificial Sequence
<220>
   <223> Synthetic Oligonucleotide
<400> 1498
   accacagcta gt 12
<210> 1499
   <211> 12
   <212> DNA
   <213> Artificial Sequence
<220>
   <223> Synthetic Oligonucleotide
<400> 1499
   gggacttcac ac 12
<210> 1500
   <211> 14
   <212> DNA
   <213> Artificial Sequence
<220>
   <223> Synthetic Oligonucleotide
<400> 1500
   ccaaaaacct tact 14
<210> 1501
   <211> 12
   <212> DNA
   <213> Artificial Sequence
<220>
   <223> Synthetic Oligonucleotide
<400> 1501
   gtggcaacca ca 12
<210> 1502
   <211> 12
   <212> DNA
   <213> Artificial Sequence
<220>
   <223> Synthetic Oligonucleotide
<400> 1502
   aaggcttaaa gt 12
<210> 1503
   <211> 12
   <212> DNA
   <213> Artificial Sequence
<220>
   <223> Synthetic Oligonucleotide
<400> 1503
   aggacttggg at 12
<210> 1504
   <211> 12
   <212> DNA
   <213> Artificial Sequence
<220>
   <223> Synthetic Oligonucleotide
<400> 1504
   gggaatcaga gc 12
<210> 1505
   <211> 12
   <212> DNA
   <213> Artificial Sequence
<220>
   <223> Synthetic Oligonucleotide
<400> 1505
   atttgatgct gc 12
<210> 1506
   <211> 12
   <212> DNA
   <213> Artificial Sequence
<220>
   <223> Synthetic Oligonucleotide
<400> 1506
   ttccaatgac ta 12
<210> 1507
   <211> 12
   <212> DNA
   <213> Artificial Sequence
<220>
   <223> Synthetic Oligonucleotide
<400> 1507
   atcaacagct gc 12
<210> 1508
   <211> 14
   <212> DNA
   <213> Artificial Sequence
<220>
   <223> Synthetic Oligonucleotide
<400> 1508
   gattgcaagt tccg 14
<210> 1509
   <211> 12
   <212> DNA
   <213> Artificial Sequence
<220>
   <223> Synthetic Oligonucleotide
<400> 1509
   tgcttggaag ga 12
<210> 1510
   <211> 14
   <212> DNA
   <213> Artificial Sequence
<220>
   <223> Synthetic Oligonucleotide
<400> 1510
   ctgctgcaca tcca 14
<210> 1511
   <211> 12
   <212> DNA
   <213> Artificial Sequence
<220>
   <223> Synthetic Oligonucleotide
<400> 1511
   cacattaaca gt 12
<210> 1512
   <211> 12
   <212> DNA
   <213> Artificial Sequence
<220>
   <223> Synthetic Oligonucleotide
<400> 1512
   agttgaaatt tc 12
<210> 1513
   <211> 12
   <212> DNA
   <213> Artificial Sequence
<220>
   <223> Synthetic Oligonucleotide
<400> 1513
   accctcattc ag 12
<210> 1514
   <211> 12
   <212> DNA
   <213> Artificial Sequence
<220>
   <223> Synthetic Oligonucleotide
<400> 1514
   agaatgagac tt 12
<210> 1515
   <211> 12
   <212> DNA
   <213> Artificial Sequence
<220>
   <223> Synthetic Oligonucleotide
<400> 1515
   gtaaatccta ag 12
<210> 1516
   <211> 12
   <212> DNA
   <213> Artificial Sequence
<220>
   <223> Synthetic Oligonucleotide
<400> 1516
   taaatatagg tc 12
<210> 1517
   <211> 12
   <212> DNA
   <213> Artificial Sequence
<220>
   <223> Synthetic Oligonucleotide
<400> 1517
   aatgccttat ta 12
<210> 1518
   <211> 14
   <212> DNA
   <213> Artificial Sequence
<220>
   <223> Synthetic Oligonucleotide
<400> 1518
   caccttaaaa tttg 14
<210> 1519
   <211> 12
   <212> DNA
   <213> Artificial Sequence
<220>
   <223> Synthetic Oligonucleotide
<400> 1519
   gtgtacagat tt 12
<210> 1520
   <211> 12
   <212> DNA
   <213> Artificial Sequence
<220>
   <223> Synthetic Oligonucleotide
<400> 1520
   ctttcagtca ta 12
<210> 1521
   <211> 12
   <212> DNA
   <213> Artificial Sequence
<220>
   <223> Synthetic Oligonucleotide
<400> 1521
   gggcatatca aa 12
<210> 1522
   <211> 12
   <212> DNA
   <213> Artificial Sequence
<220>
   <223> Synthetic Oligonucleotide
<400> 1522
   tgaggcatta tc 12
<210> 1523
   <211> 4
   <212> DNA
   <213> Artificial Sequence
<220>
   <223> Synthetic Oligonucleotide
<400> 1523
   actg 4
<210> 1524
   <211> 19
   <212> DNA
   <213> Artificial Sequence
<220>
   <223> Primer
<400> 1524
   cgtgggctcc agcattcta 19
<210> 1525
   <211> 21
   <212> DNA
   <213> Artificial Sequence
<220>
   <223> Primer
<400> 1525
   agtcatttct gcctttgcgt c 21
<210> 1526
   <211> 22
   <212> DNA
   <213> Artificial Sequence
<220>
   <223> Probe
<400> 1526
   ccaatggtcg ggcactgctc aa 22
<210> 1527
   <211> 30
   <212> DNA
   <213> Artificial Sequence
<220>
   <223> Primer
<400> 1527
   gaaaatagac ttcctgaata actatgcatt 30
<210> 1528
   <211> 18
   <212> DNA
   <213> Artificial Sequence
<220>
   <223> Primer
<400> 1528
   actcgcttgc cagcttgc 18
<210> 1529
   <211> 23
   <212> DNA
   <213> Artificial Sequence
<220>
   <223> Probe
<400> 1529
   tttctgagtc cccgtgccca aca 23
<210> 1530
   <211> 20
   <212> DNA
   <213> Artificial Sequence
<220>
   <223> Primer
<400> 1530
   tgagccttgc caccttctct 20
<210> 1531
   <211> 15
   <212> DNA
   <213> Artificial Sequence
<220>
   <223> Primer
<400> 1531
   gcgcacccca gccaa 15
<210> 1532
   <211> 29
   <212> DNA
   <213> Artificial Sequence
<220>
   <223> Probe
<400> 1532
   agaggagctt cttttccctc tacctgggc 29
<210> 1533
   <211> 20
   <212> DNA
   <213> Artificial Sequence
<220>
   <223> Primer
<400> 1533
   atttcctgcc cctggtacct 20
<210> 1534
   <211> 17
   <212> DNA
   <213> Artificial Sequence
<220>
   <223> Primer
<400> 1534
   cgggcccaca cctcttg 17
<210> 1535
   <211> 26
   <212> DNA
   <213> Artificial Sequence
<220>
   <223> Probe
<400> 1535
   ccacaaagtg cagcaccgcc tagtgt 26
<210> 1536
   <211> 19
   <212> DNA
   <213> Artificial Sequence
<220>
   <223> Primer
<400> 1536
   gccacaggct cccagacat 19
<210> 1537
   <211> 25
   <212> DNA
   <213> Artificial Sequence
<220>
   <223> Primer
<400> 1537
   tccatcctct tgatatctcc ttttg 25
<210> 1538
   <211> 31
   <212> DNA
   <213> Artificial Sequence
<220>
   <223> Probe
<400> 1538
   acagccatca tcaaagagat cgttagcaga a 31
<210> 1539
   <211> 24
   <212> DNA
   <213> Artificial Sequence
<220>
   <223> Primer
<400> 1539
   atgacaatca tgttgcagca attc 24
<210> 1540
   <211> 25
   <212> DNA
   <213> Artificial Sequence
<220>
   <223> Primer
<400> 1540
   cgatgcaata aatatgcaca aatca 25
<210> 1541
   <211> 28
   <212> DNA
   <213> Artificial Sequence
<220>
   <223> Probe
<400> 1541
   ctgtaaagct ggaaagggac ggactggt 28
<210> 1542
   <211> 14
   <212> DNA
   <213> Artificial Sequence
<220>
   <223> Synthetic Oligonucleotide
<400> 1542
   gtgtgcacac agct 14
<210> 1543
   <211> 12
   <212> DNA
   <213> Artificial Sequence
<220>
   <223> Synthetic Oligonucleotide
<400> 1543
   cagcctgggc ac 12
<210> 1544
   <211> 14
   <212> DNA
   <213> Artificial Sequence
<220>
   <223> Synthetic Oligonucleotide
<400> 1544
   tgctcgaact cctt 14
<210> 1545
   <211> 14
   <212> DNA
   <213> Artificial Sequence
<220>
   <223> Synthetic Oligonucleotide
<400> 1545
   gaagtcactg gctt 14
<210> 1546
   <211> 14
   <212> DNA
   <213> Artificial Sequence
<220>
   <223> Synthetic Oligonucleotide
<400> 1546
   gggaagtcac tggc 14
<210> 1547
   <211> 14
   <212> DNA
   <213> Artificial Sequence
<220>
   <223> Synthetic Oligonucleotide
<400> 1547
   gttaggcaaa gggc 14
<210> 1548
   <211> 14
   <212> DNA
   <213> Artificial Sequence
<220>
   <223> Synthetic Oligonucleotide
<400> 1548
   gggctgagtg accc 14
<210> 1549
   <211> 14
   <212> DNA
   <213> Artificial Sequence
<220>
   <223> Synthetic Oligonucleotide
<400> 1549
   atgctagtgc acta 14
<210> 1550
   <211> 14
   <212> DNA
   <213> Artificial Sequence
<220>
   <223> Synthetic Oligonucleotide
<400> 1550
   agctcgctac ctct 14
<210> 1551
   <211> 14
   <212> DNA
   <213> Artificial Sequence
<220>
   <223> Synthetic Oligonucleotide
<400> 1551
   gaggtatccc atct 14
<210> 1552
   <211> 14
   <212> DNA
   <213> Artificial Sequence
<220>
   <223> Synthetic Oligonucleotide
<400> 1552
   ggcaacttca acct 14
<210> 1553
   <211> 20
   <212> DNA
   <213> Artificial Sequence
<220>
   <223> Synthetic Oligonucleotide
<400> 1553
   gaagtagcca ccaactgtgc 20
<210> 1554
   <211> 12
   <212> DNA
   <213> Artificial Sequence
<220>
   <223> Synthetic Oligonucleotide
<400> 1554
   tagccaccaa ct 12
<210> 1555
   <211> 4
   <212> DNA
   <213> Artificial Sequence
<220>
   <223> Synthetic Oligonucleotide
<400> 1555
   actg 4
<210> 1556
   <211> 4
   <212> DNA
   <213> Artificial Sequence
<220>
   <223> Synthetic Oligonucleotide
<400> 1556
   actg 4
<210> 1557
   <211> 10
   <212> DNA
   <213> Artificial Sequence
<220>
   <223> Synthetic Oligonucleotide
<400> 1557
   agccaccaac 10
<210> 1558
   <211> 8
   <212> DNA
   <213> Artificial Sequence
<220>
   <223> Synthetic Oligonucleotide
<400> 1558
   gccaccaa 8
<210> 1559
   <211> 13
   <212> DNA
   <213> Artificial Sequence
<220>
   <223> Synthetic Oligonucleotide
<400> 1559
   tagccaccaa ctg 13
<210> 1560
   <211> 12
   <212> DNA
   <213> Artificial Sequence
<220>
   <223> Synthetic Oligonucleotide
<400> 1560
   taccgaacac ct 12
<210> 1561
   <211> 20
   <212> DNA
   <213> Artificial Sequence
<220>
   <223> Synthetic Oligonucleotide
<400> 1561
   gtccctgaag atgtcaatgc 20
<210> 1562
   <211> 20
   <212> DNA
   <213> Artificial Sequence
<220>
   <223> Synthetic Oligonucleotide
<400> 1562
   tgcactttgt ggtaccaagg 20
<210> 1563
   <211> **14**
   <212> DNA
   <213> Artificial Sequence
<220>
   <223> Synthetic Oligonucleotide
<400> 1563
   gcttctccat cata 14
<210> 1564
   <211> 14
   <212> DNA
   <213> Artificial Sequence
<220>
   <223> Synthetic Oligonucleotide
<400> 1564
   tgtcttgctg cttt 14
<210> 1565
   <211> 4
   <212> DNA
   <213> Artificial Sequence
<220>
   <223> Synthetic Oligonucleotide
<400> 1565
   actg 4
<210> 1566
   <211> 4
   <212> DNA
   <213> Artificial Sequence
<220>
   <223> Synthetic Oligonucleotide
<400> 1566
   actg 4
<210> 1567
   <211> 4
   <212> DNA
   <213> Artificial Sequence
<220>
   <223> Synthetic Oligonucleotide
<400> 1567
   actg 4
<210> 1568
   <211> 20
   <212> DNA
   <213> Artificial Sequence
<220>
   <223> Synthetic Oligonucleotide
<400> 1568
   atggcttctc catcatatcc 20
<210> 1569
   <211> 20
   <212> DNA
   <213> Artificial Sequence
<220>
   <223> Synthetic Oligonucleotide
<400> 1569
   cttgggcatg ctcgtcagtc 20
<210> 1570
   <211> 20
   <212> DNA
   <213> Artificial Sequence
<220>
   <223> Synthetic Oligonucleotide
<400> 1570
   ccttccctga aggttcctcc 20
<210> 1571
   <211> 20
   <212> DNA
   <213> Artificial Sequence
<220>
   <223> Synthetic Oligonucleotide
<400> 1571
   ctgctagcct ctggatttga 20
<210> 1572
   <211> 4
   <212> DNA
   <213> Artificial Sequence
<220>
   <223> Synthetic Oligonucleotide
<400> 1572
   actg 4
<210> 1573
   <211> 14
   <212> DNA
   <213> Artificial Sequence
<220>
   <223> Synthetic Oligonucleotide
<400> 1573
   gaatatctat aatg 14
<210> 1574
   <211> 12
   <212> DNA
   <213> Artificial Sequence
<220>
   <223> Synthetic Oligonucleotide
<400> 1574
   gtaagcaagg ct 12
<210> 1575
   <211> 2254
   <212> DNA
   <213> Rattus norvegicus
<400> 1575
<210> 1576
   <211> 3300
   <212> DNA
   <213> Mus musculus
<400> 1576
   tacatcaatg gaatttaaat agagtcgtca tataataagg gaaacaatgt cccaactgga 60
   catcacatgg taccaaacaa aagacccagt accaggaatg agttaccatc ttatgaagtc 120
   attaaccata gagagctaat ggtagcctca atcattacaa aagctgttgc caaggttact 180
   gggtgctctc ataccctgat ggtaagaccc tatggctgaa gatggaactt attgatatcc 240
   ttgaacattg agaaattgag ctgtgtgact agaagcttca ccccatcgac gatggtcaca 300
   gtgctgcaaa gtgctatgtt caggaggaag gtatccagcc gcctccctca gccacactct 360
   gagactcaca ccagagacct gctcaaaggg catgcccact ggcccacaaa tattatggga 420
   gcaactgacc actttttggg ggtgtattta aggtccactt catgaaatgg gacccatccc 480
   tgacactgct aaaatgcctg agaacctgaa actagataga gcaagggctc taggggaaag 540
   ctcactccag ttattctaag ggcacagggt tatgacgcct aatgacatat cgctggctac 600
   atcccttggc cagcacatca ctgaaacctc accagaggag cttcttggag tagaaggtga 660
   ttaacagaac tgtccgtgac tggatgactt gcagagagtg agagacttgg gagcactcag 720
   acttcaatgg gatgcttgta tctcacccct ctgctaaaga tgcaggttct atacaggagg 780
   tgcaaagatt gtaagagtca gagttggagg ttgtcttcag gaaagcagag ttttgcagac 840
   acaacaaagc tgatgaaaat ctgaactcac agacagtgat agcatgcaca agacagatat 900
   ctgaactcac agaccgtgat aacatggcac aaagacctgc acaaagttcg aaccaaataa 960
   aatctgagca tggaaaatga ggtgtgggca caaagtccca ctcctaagta agaaactact 1020
   tgcagttgac agctactagg agagagaaat gggttttctt caatggagag acactgggtg 1080
   tatcaactgc accccaaggc aggccacacg ctcaggaaga gttggccaac acaaaacaca 1140
   ctccgtgttt ggttttctgt ttgcttgggt ttgtttttgt gcttttattc ttccttcctt 1200
   tctttctttg tttctttgtt tctctctctc tttctctctt tttctctctc cctcctcctc 1260
   ttcttctttc ttctttttct tcttcttctt cttcttcttc tttttcttct tcttcttctt 1320
   ctttttcttc ttcttcttct tcttcttctt cttcttcttc ttcttcttct tcttcttctt 1380
   cttcttcttc ttcttcttct tctgatcaga gaggggaggg gatctgggaa aagtttgggg 1440
   agaggaaaga atttgcccaa aatatattgc ataaaaactt tttaaaaata aatttaaaac 1500
   aatttttagg atagagcaaa gagaaagtag aaaatatttg ggttgggaag ggcaggagaa 1560
   tgagggaaat gtgatttttt tctccctagg ttttgtatgg cagaagtcag ggcatggcat 1620
   gcgtgacagt caggctgagg aacatgtatg tcctgctgac tgtcaggggg tgctatggag 1680
   gacttgtgcg gaggacactg tcagagttgg attcggacct tcctaatcaa agttagaggg 1740
   tgtattttca gagaacgcag gaaggaactt tgcttggaac actgggtata ggatggatcc 1800
   taaacccagg aaggagtgct cttgaattcc aaatggtcca gcgccccagg accagccttc 1860
   ggccttgata gatcctgatt cagataaata aagctggaga aggaggctga gacctggggg 1920
   acttgtcggg tcagtgctcc tgaggtaacc attaatcctt cccccagggg aatccaggga 1980
   ctagcccctt gagggacaga tggtggagag aatggagcaa cacgtagagg caggctctga 2040
   acttggggag cagaaggtcc tgattgataa tcctgctgac attctggtta tcgctgccta 2100
   tttcctgctg gtcattggtg ttggcttgtg ggtgagacat tgaggggggt tggataggga 2160
   aatgcttctg ggcttgaggg gtaaagattt agggagacct cagagaggag tgggagaaaa 2220
   gggtgcttgg atataatgag ggagaaacct agatttagta ggcaagccaa ttttaattct 2280
   ttgtcttcgt accttctgga ttgtgcaaaa gagactgggg gtatcaatag gttttttttt 2340
   aattcaagtg ttctaacaag tgctctaaga gatgtatcag ttcccacgtc tgtattatgg 2400
   ctgagcagca gcctatattt aaggtcacca ggcaagttag gctgaatcta ggcatatcta 2460
   ggttccagta gttgcgctag gattagggcc tgggttgttc tgagtgtcgg ggaaggttgg 2520
   gggtaaggag gtgcagtctg gggagtccag ggctggttaa tcttcagcct gaaacaaggc 2580
   tgaggaatgt gttgaggaag ctaaggaagt ccaaagatgt gccccaatcc cagtttcccc 2640
   ccacttctgt ttcccagtct atgttcagaa ccaatagagg cacagttggt ggctacttcc 2700
   tggcaggacg gaacatggtg tggtggccgg tgagaggggc tgggggatgg gatgggaggg 2760
   accctggagg agcaccctac ctcaccccca tttctggcca cccaggttgg agcctctctg 2820
   ttcgccagca acatcggcag cggtcatttt gtgggcctgg cagggactgg tgcagcaagt 2880
   ggcttggcgg tggctggatt tgagtggaat gtgaggtctt cttttctata ataatccacc 2940
   ccagtggaaa ctcctggaaa gatcacaact tggggaagct cctggcgtgg ggatatgaac 3000
   aagttgggac agcacagttt gctgagggtg ggtgggtgtt aggttgggct gggagcaagg 3060
   atcgcgtgct agcttacagc agtagggttc aggcaggtcc tgctcctgac tttctgtata 3120
   cgccagcatc agtcctcgtg ggccttgacc actctgaaat ggggccggca cctcactgat 3180
   gaagtacctg tctccatgtt ctttgatgct gtagtctgtg agcaactcta ggctgaactg 3240
   aagttctttg caaaaacaag gatgggttgg aaagatgagg gtttgagcct tcctgtaaga 3300